# EUROPEAN PATENT SPECIFICATION

(11) **EP 3 280 795 B1**
(45) Date of publication and mention of the grant of the patent: **24.03.2021**
(21) Application number: 16718786.3
(22) Date of filing: 07.04.2016
(51) Int. Cl.: A61K 31/505, A61K 31/506, A61K 45/06, A61P 35/00, A61P 35/02

(54) **COMBINATION OF CHIMERIC ANTIGEN RECEPTOR THERAPY AND AMINO PYRIMIDINE DERIVATIVES**
KOMBINATION VON CHIMÄRER ANTIGEN REZEPTOR THERAPIE UND AMINO PYRIMIDIN DERIVATEN
COMBINAISON DE TRAITEMENT À L'AIDE DU RÉCEPTEUR ANTIGÉNIQUE CHIMÉRIQUE ET DE DERIVÉS DE L'AMINO PYRIMIDINE

(30) Priority: 07.04.2015 US 201562144188 P
(43) Date of publication of application: 14.02.2018
(73) Proprietor: Novartis AG, 4056 Basel (CH); The Trustees of The University of Pennsylvania, Philadelphia, PA 19104 (US)
(72) Inventor: BYRD, John, Columbus, OH 43212 (US); DUBOVSKY, Jason, Columbus, OH 43235 (US); JOHNSON, Amy, Dublin, OH 43016 (US); JUNE, Carl, H., Merion Station, PA 19066 (US); MUTHUSAMY, Natarajan, Galloway, OH (US); PORTER, David, L., Springfield, PA 19064 (US); WASIK, Mariusz, Ardmore, PA 19003 (US); ANGST, Daniela, 4002 Basel (CH); FRAIETTA, Joseph, A., Williamstown, NJ 08094 (US); GESSIER, Francois, 4002 Basel (CH); GILL, Saar, Philadelphia, PA 19106 (US); MAUS, Marcela, Lexington, MA 02421 (US); RUELLA, Marco, Philadelphia, PA 19143 (US); VULPETTI, Anna, 4002 Basel (CH)
(74) Representative: Wilding, James Roger
(86) International application number: PCT/US2016/026437
(87) International publication number: WO 2016/164580

(56) References cited:
- WO-A1-2015/079417
- WO-A1-2015/157252
- Lauren Martz ET AL: "Overcoming ibrutinib resistance", SciBX: Science-Business eXchange, 28 August 2014 (2014-08-28), pages 1-3, XP055281271, Retrieved from the Internet: URL:http://www.nature.com/scibx/journal/v7 /n33/pdf/scibx.2014.971.pdf [retrieved on 2016-06-16]
- CLAIRE V. HUTCHINSON ET AL: "Breaking good: the inexorable rise of BTK inhibitors in the treatment of chronic lymphocytic leukaemia", BRITISH JOURNAL OF HAEMATOLOGY, vol. 166, no. 1, 18 April 2014 (2014-04-18), pages 12-22, XP055281280, GB ISSN: 0007-1048, DOI: 10.1111/bjh.12895
- M E DUDLEY: "Donor-Derived Anti-CD19 Chimeric-Antigen-Receptor-Expressing T Cells Cause Regression Of Malignancy Persisting After Allogeneic Hematopoietic Stem Cell Transplantation", BLOOD, vol. 122, no. 21, 15 November 2013 (2013-11-15), pages 151-151, XP055281313,
- HUAN SHI ET AL: "Chimeric antigen receptor for adoptive immunotherapy of cancer: latest research and future prospects", MOLECULAR CANCER, BIOMED CENTRAL, LONDON, GB, vol. 13, no. 1, 21 September 2014 (2014-09-21), page 219, XP021197368, ISSN: 1476-4598, DOI: 10.1186/1476-4598-13-219
- M RUELLA: "COMBINATION OF IBRUTINIB AND ANTI-CD19 CHIMERIC ANTIGEN RECEPTOR T CELLS FOR THE TREATMENT OF RELAPSING/REFRACTORY MANTLE CELL LYMPHOMA (MCL)", 20TH CONGRESS OF THE EUROPEAN HEMATOLOGY ASSOCIATION, JUN 11-14, 2015, VIENNA, AUSTRIA, 13 June 2015 (2015-06-13), pages 1-1, XP055281361,

## Description

### SEQUENCE LISTING

The instant application contains a Sequence Listing which has been submitted electronically in ASCII format. Said ASCII copy, created on April 1, 2016, is named N2067-7092WO_SL.txt and is 257,530 bytes in size.

### FIELD OF THE INVENTION

The present invention relates generally to the use of T cells engineered to express a Chimeric Antigen Receptor (CAR), e.g., in combination with another agent such as, e.g., a Bruton's tyrosine kinase (BTK) inhibitor, to treat a disease associated with expression of the Cluster of Differentiation 19 protein (CD19).

### BACKGROUND OF THE INVENTION

Many patients with B cell malignancies are incurable with standard therapy. In addition, traditional treatment options often have serious side effects. Attempts have been made in cancer immunotherapy, however, several obstacles render this a very difficult goal to achieve clinical effectiveness. Although hundreds of so-called tumor antigens have been identified, these are generally derived from self and thus are poorly immunogenic. Furthermore, tumors use several mechanisms to render themselves hostile to the initiation and propagation of immune attack.

Recent developments using chimeric antigen receptor (CAR) modified autologous T cell (CART) therapy, which relies on redirecting T cells to a suitable cell-surface molecule on cancer cells such as B cell malignancies, show promising results in harnessing the power of the immune system to treat B cell malignancies and other cancers (see, e.g., Sadelain et al., Cancer Discovery 3:388-398 (2013)). The clinical results of the murine derived CART19 (i.e., "CTL019") have shown promise in establishing complete remissions in patients suffering with CLL as well as in childhood ALL (see, e.g., Kalos et al., Sci Transl Med 3:95ra73 (2011), Porter et al., NEJM 365:725-733 (2011), Grupp et al., NEJM 368:1509-1518 (2013)). Besides the ability for the chimeric antigen receptor on the genetically modified T cells to recognize and destroy the targeted cells, a successful therapeutic T cell therapy needs to have the ability to proliferate and persist over time, and to further monitor for leukemic cell escapees. The variable quality of T cells, resulting from anergy, suppression or exhaustion will have effects on CAR-transformed T cells' performance, over which skilled practitioners have limited control at this time. To be effective, CAR transformed patient T cells need to persist and maintain the ability to proliferate in response to its cognate antigen. It has been shown that ALL patient T cells perform can do this with CART19 comprising a murine scFv (see, e.g., Grupp et al., NEJM 368:1509-1518 (2013)). Martz, L (Science-Business eXchange (2014); 7(33); doi:10.1038/scibx2014.971) relates to overcoming ibrutinib resistance.

### SUMMARY OF THE INVENTION

The disclosure features, at least in part, compositions and methods of treating disorders as cancer (e.g., hematological cancers or other B-cell malignancies) using immune effector cells (e.g., T cells or NK cells) that express a Chimeric Antigen Receptor (CAR) molecule, e.g., a CAR that binds to a B-cell antigen, e.g., Cluster of Differentiation 19 protein (CD19) (e.g., OMIM Acc. No. 107265, Swiss Prot. Acc No. P15391). The compositions include, and the methods include administering, immune effector cells (e.g., T cells or NK cells) expressing a B cell targeting CAR, in combination with a BTK inhibitor (e.g., a compound of formula (I) or a pharmaceutically acceptable salt thereof). In some instances, the combination maintains or has better clinical effectiveness as compared to either therapy alone. The disclosure further pertains to the use of engineered cells, e.g., immune effector cells (e.g., T cells or NK cells), to express a CAR molecule that binds to a B-cell antigen, e.g., CD19, in combination with a BTK inhibitor (e.g., a BTK inhibitor described herein) to treat a disorder associated with expression of a B-cell antigen, e.g., CD19 (e.g., a cancer, e.g., a hematological cancer).

Accordingly, in one aspect, the disclosure pertains to a method of treating a subject, e.g., a mammal, having a disease associated with expression of a B-cell antigen, e.g., CD19, comprising administering to the mammal an effective amount of a cell, e.g., an immune effector cell (e.g., a T cell or NK cell) that expresses a CAR molecule that binds the B-cell antigen, e.g., CD19, e.g., a CAR molecule that binds CD19 described herein, in combination with a BTK inhibitor, e.g., a BTK inhibitor described herein, e.g., a compound of formula (I) or a pharmaceutically acceptable salt thereof. In one instance, the CAR molecule binds to CD19, e.g., a CAR molecule that binds CD19 described herein. In other instances, the CAR molecule binds to one or more of CD20, CD22 or ROR1.

In one instance, the disease associated with expression of a B-cell antigen (e.g., expression of one or more of CD19, CD20, CD22, or ROR1) is selected from a proliferative disease such as a cancer or malignancy or a precancerous condition such as a myelodysplasia, a myelodysplastic syndrome or a preleukemia, or is a non-cancer related indication associated with expression of a B-cell antigen, e.g., one or more of CD19, CD20, CD22, or ROR1. In one instance, the disease is a solid or liquid tumor. In one instance, the cancer is pancreatic cancer. In one instance, the disease is a hematologic cancer. In one instance, the hematological cancer is leukemia. In one instance, the cancer is selected from the group consisting of one or more acute leukemias including but not limited to B-cell acute lymphoid leukemia (BALL), T-cell acute lymphoid leukemia (TALL), small lymphocytic leukemia (SLL), acute lymphoid leukemia (ALL); one or more chronic leukemias including but not limited to chronic myelogenous leukemia (CML), chronic lymphocytic leukemia (CLL). Additional hematological cancers or hematologic conditions include, but are not limited to, mantle cell lymphoma (MCL), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, and Waldenstrom macroglobulinemia. In certain instances, the disease associated with B-cell antigen (e.g., e.g., one or more of CD19, CD20, CD22, or ROR1) expression is a "preleukemia" which is a diverse collection of hematological conditions united by ineffective production (or dysplasia) of myeloid blood cells. In some instances, the disease associated with B-cell antigen (e.g., e.g., one or more of CD19, CD20, CD22 or ROR1) expression includes, but is not limited to atypical and/or non-classical cancers, malignancies, precancerous conditions or proliferative diseases expressing a B-cell antigen (e.g., e.g., one or more of CD19, CD20, CD22 or ROR1). Any combination of the diseases associated with B-cell antigen (e.g., e.g., one or more of CD19, CD20, CD22 or ROR1) expression described herein can be treated with the methods and compositions described herein.

In one instance, the disease associated with expression of a B-cell antigen (e.g., e.g., one or more of CD19, CD20, CD22, or ROR1) is a lymphoma, e.g., MCL or Hodgkin lymphoma. In one instance, the disease associated with expression of a B-cell antigen (e.g., e.g., one or more of CD19, CD20, CD22, or ROR1) is leukemia, e.g., SLL, CLL and/or ALL.

In one instance, the cell expresses a CAR molecule comprising an anti-CD 19 binding domain (e.g., a murine or humanized antibody or antibody fragment that specifically binds to CD19), a transmembrane domain, and an intracellular signaling domain (e.g., an intracellular signaling domain comprising a costimulatory domain and/or a primary signaling domain). In one instance, the CAR comprises an antibody or antibody fragment which includes an anti-CD 19 binding domain described herein (e.g., a murine or humanized antibody or antibody fragment that specifically binds to CD19 as described herein), a transmembrane domain described herein, and an intracellular signaling domain described herein (e.g., an intracellular signaling domain comprising a costimulatory domain and/or a primary signaling domain described herein).

In one instance, the CAR molecule is capable of binding CD19 (e.g., wild-type or mutant human CD19). In one instance, the CAR molecule comprises an anti-CD19 binding domain comprising one or more (e.g., all three) light chain complementary determining region 1 (LC CDR1), light chain complementary determining region 2 (LC CDR2), and light chain complementary determining region 3 (LC CDR3) of an anti-CD19 binding domain described herein, and one or more (e.g., all three) heavy chain complementary determining region 1 (HC CDR1), heavy chain complementary determining region 2 (HC CDR2), and heavy chain complementary determining region 3 (HC CDR3) of an anti-CD 19 binding domain described herein, e.g., an anti-CD19 binding domain comprising one or more, e.g., all three, LC CDRs and one or more, e.g., all three, HC CDRs. In one instance, the anti-CD19 binding domain comprises one or more (e.g., all three) heavy chain complementary determining region 1 (HC CDR1), heavy chain complementary determining region 2 (HC CDR2), and heavy chain complementary determining region 3 (HC CDR3) of an anti-CD19 binding domain described herein, e.g., the anti-CD19 binding domain has two variable heavy chain regions, each comprising a HC CDR1, a HC CDR2 and a HC CDR3 described herein. In one instance, the anti-CD19 binding domain comprises a murine light chain variable region described herein (e.g., in Table 7) and/or a murine heavy chain variable region described herein (e.g., in Table 7). In one instance, the anti-CD19 binding domain is a scFv comprising a murine light chain and a murine heavy chain of an amino acid sequence of Table 7. In an instance, the anti-CD19 binding domain (e.g., an scFv) comprises: a light chain variable region comprising an amino acid sequence having at least one, two or three modifications (e.g., substitutions) but not more than 30, 20 or 10 modifications (e.g., substitutions) of an amino acid sequence of a light chain variable region provided in Table 7, or a sequence with 95-99% identity with an amino acid sequence of Table 7; and/or a heavy chain variable region comprising an amino acid sequence having at least one, two or three modifications (e.g., substitutions) but not more than 30, 20 or 10 modifications (e.g., substitutions) of an amino acid sequence of a heavy chain variable region provided in Table 7, or a sequence with 95-99% identity to an amino acid sequence of Table 7. In one instance, the anti-CD19 binding domain comprises a sequence of SEQ ID NO:59, or a sequence with 95-99% identify thereof. In one instance, the anti-CD19 binding domain is a scFv, and a light chain variable region comprising an amino acid sequence described herein, e.g., in Table 7, is attached to a heavy chain variable region comprising an amino acid sequence described herein, e.g., in Table 7, via a linker, e.g., a linker described herein. In one instance, the anti-CD19 binding domain includes a (Gly₄-Ser)n linker, wherein n is 1, 2, 3, 4, 5, or 6, preferably 3 or 4 (SEQ ID NO: 53). The light chain variable region and heavy chain variable region of a scFv can be, e.g., in any of the following orientations: light chain variable region-linker-heavy chain variable region or heavy chain variable region-linker-light chain variable region.

In one instance, the CAR molecule comprises a humanized anti-CD 19 binding domain that includes one or more (e.g., all three) light chain complementary determining region 1 (LC CDR1), light chain complementary determining region 2 (LC CDR2), and light chain complementary determining region 3 (LC CDR3) of a humanized anti-CD19 binding domain described herein, and one or more (e.g., all three) heavy chain complementary determining region 1 (HC CDR1), heavy chain complementary determining region 2 (HC CDR2), and heavy chain complementary determining region 3 (HC CDR3) of a humanized anti-CD19 binding domain described herein, e.g., a humanized anti-CD19 binding domain comprising one or more, e.g., all three, LC CDRs and one or more, e.g., all three, HC CDRs. In one instance, the humanized anti-CD19 binding domain comprises at least HC CDR2. In one instance, the humanized anti-CD19 binding domain comprises one or more (e.g., all three) heavy chain complementary determining region 1 (HC CDR1), heavy chain complementary determining region 2 (HC CDR2), and heavy chain complementary determining region 3 (HC CDR3) of a humanized anti-CD 19 binding domain described herein, e.g., the humanized anti-CD19 binding domain has two variable heavy chain regions, each comprising a HC CDR1, a HC CDR2 and a HC CDR3 described herein. In one instance, the humanized anti-CD19 binding domain comprises at least HC CDR2. In one instance, the light chain variable region comprises one, two, three or all four framework regions of VK3_L25 germline sequence. In one instance, the light chain variable region has a modification (e.g., substitution, e.g., a substitution of one or more amino acid found in the corresponding position in the murine light chain variable region of SEQ ID NO: 58, e.g., a substitution at one or more of positions 71 and 87). In one instance, the heavy chain variable region comprises one, two, three or all four framework regions of VH4_4-59 germline sequence. In one instance, the heavy chain variable region has a modification (e.g., substitution, e.g., a substitution of one or more amino acid found in the corresponding position in the murine heavy chain variable region of SEQ ID NO: 58, e.g., a substitution at one or more of positions 71, 73 and 78). In one instance, the humanized anti-CD19 binding domain comprises a light chain variable region described herein (e.g., in Table 3) and/or a heavy chain variable region described herein (e.g., in Table 3). In one instance, the humanized anti-CD19 binding domain is a scFv comprising a light chain and a heavy chain of an amino acid sequence of Table 3. In an instance, the humanized anti-CD19 binding domain (e.g., an scFv) comprises: a light chain variable region comprising an amino acid sequence having at least one, two or three modifications (e.g., substitutions) but not more than 30, 20 or 10 modifications (e.g., substitutions) of an amino acid sequence of a light chain variable region provided in Table 3, or a sequence with 95-99% identity with an amino acid sequence of Table 3; and/or a heavy chain variable region comprising an amino acid sequence having at least one, two or three modifications (e.g., substitutions) but not more than 30, 20 or 10 modifications (e.g., substitutions) of an amino acid sequence of a heavy chain variable region provided in Table 3, or a sequence with 95-99% identity to an amino acid sequence of Table 3. In one instance, the humanized anti-CD19 binding domain comprises a sequence selected from a group consisting of SEQ ID NO:1, SEQ ID NO:2, SEQ ID NO:3, SEQ ID NO: 4, SEQ ID NO:5, SEQ ID NO:6, SEQ ID NO:7, SEQ ID NO:8, SEQ ID NO:9, SEQ ID NO:10, SEQ ID NO:11 and SEQ ID NO:12, or a sequence with 95-99% identify thereof. In one instance, the humanized anti-CD19 binding domain is a scFv, and a light chain variable region comprising an amino acid sequence described herein, e.g., in Table 3, is attached to a heavy chain variable region comprising an amino acid sequence described herein, e.g., in Table 3, via a linker, e.g., a linker described herein. In one instance, the humanized anti-CD19 binding domain includes a (Gly₄-Ser)n linker, wherein n is 1, 2, 3, 4, 5, or 6, preferably 3 or 4 (SEQ ID NO: 53). The light chain variable region and heavy chain variable region of a scFv can be, e.g., in any of the following orientations: light chain variable region-linker-heavy chain variable region or heavy chain variable region-linker-light chain variable region.

In one instance, the CAR molecule comprises a transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In one instance, the transmembrane domain comprises a sequence of SEQ ID NO: 15. In one instance, the transmembrane domain comprises an amino acid sequence having at least one, two or three modifications (e.g., substitutions) but not more than 20, 10 or 5 modifications (e.g., substitutions) of an amino acid sequence of SEQ ID NO: 15, or a sequence with 95-99% identity to an amino acid sequence of SEQ ID NO: 15.

In one instance, the anti-CD 19 binding domain is connected to the transmembrane domain by a hinge region, e.g., a hinge region described herein. In one instance, the encoded hinge region comprises SEQ ID NO:14 or SEQ ID NO:45, or a sequence with 95-99% identity thereof.

In one instance, the CAR molecule further comprises a sequence encoding a costimulatory domain, e.g., a costimulatory domain described herein. In one instance, the costimulatory domain comprises a functional signaling domain of a protein selected from the group consisting of OX40, CD2, CD27, CD28, CDS, ICAM-1, LFA-1 (CD11a/CD18) and 4-1BB (CD137). In one instance, the costimulatory domain comprises a sequence of SEQ ID NO: 16. In one instance, the costimulatory domain comprises a sequence of SEQ ID NO:51. In one instance, the costimulatory domain comprises an amino acid sequence having at least one, two or three modifications (e.g., substitutions) but not more than 20, 10 or 5 modifications (e.g., substitutions) of an amino acid sequence of SEQ ID NO: 16 or SEQ ID NO:51, or a sequence with 95-99% identity to an amino acid sequence of SEQ ID NO: 16 or SEQ ID NO:51.

In one instance, the CAR molecule further comprises a sequence encoding an intracellular signaling domain, e.g., an intracellular signaling domain described herein. In one instance, the intracellular signaling domain comprises a functional signaling domain of 4-1BB and/or a functional signaling domain of CD3 zeta. In one instance, the intracellular signaling domain comprises the sequence of SEQ ID NO: 16 and/or the sequence of SEQ ID NO:17. In one instance, the intracellular signaling domain comprises the sequence of SEQ ID NO:16 and/or the sequence of SEQ ID NO:43. In one instance, the intracellular signaling domain comprises a functional signaling domain of CD27 and/or a functional signaling domain of CD3 zeta. In one instance, the intracellular signaling domain comprises the sequence of SEQ ID NO: 51 and/or the sequence of SEQ ID NO:17. In one instance, the intracellular signaling domain comprises the sequence of SEQ ID NO:51 and/or the sequence of SEQ ID NO:43. In one instance, the intracellular signaling domain comprises an amino acid sequence having at least one, two or three modifications (e.g., substitutions) but not more than 20, 10 or 5 modifications (e.g., substitutions) of an amino acid sequence of SEQ ID NO:16 or SEQ ID NO:51 and/or an amino acid sequence of SEQ ID NO:17 or SEQ ID NO:43, or a sequence with 95-99% identity to an amino acid sequence of SEQ ID NO:16 or SEQ ID NO:51 and/or an amino acid sequence of SEQ ID NO:17 or SEQ ID NO:43. In one instance, the intracellular signaling domain comprises the sequence of SEQ ID NO:16 or SEQ ID NO:51 and the sequence of SEQ ID NO: 17 or SEQ ID NO:43, wherein the sequences comprising the intracellular signaling domain are expressed in the same frame and as a single polypeptide chain.

In one instance, the CAR molecule further comprises a leader sequence, e.g., a leader sequence described herein. In one instance, the leader sequence comprises an amino acid sequence of SEQ ID NO: 13, or a sequence with 95-99% identity to an amino acid sequence of SEQ ID NO:13.

In one instance, the CAR molecule comprises a leader sequence, e.g., a leader sequence described herein, e.g., a leader sequence of SEQ ID NO: 13, or having 95-99% identity thereof; an anti-CD19 binding domain described herein, e.g., an anti-CD19 binding domain comprising a LC CDR1, a LC CDR2, a LC CDR3, a HC CDR1, a HC CDR2 and a HC CDR3 described herein, e.g., a murine anti-CD19 binding domain described in Table 7, a humanized anti-CD19 binding domain described in Table 3, or a sequence with 95-99% identify thereof; a hinge region, e.g., a hinge region described herein, e.g., a hinge region of SEQ ID NO:14 or having 95-99% identity thereof; a transmembrane domain, e.g., a transmembrane domain described herein, e.g., a transmembrane domain having a sequence of SEQ ID NO: 15 or a sequence having 95-99% identity thereof; an intracellular signaling domain, e.g., an intracellular signaling domain described herein (e.g., an intracellular signaling domain comprising a costimulatory domain and/or a primary signaling domain). In one instance, the intracellular signaling domain comprises a costimulatory domain, e.g., a costimulatory domain described herein, e.g., a 4-1BB costimulatory domain having a sequence of SEQ ID NO:16 or SEQ ID NO:51, or having 95-99%identity thereof, and/or a primary signaling domain, e.g., a primary signaling domain described herein, e.g., a CD3 zeta stimulatory domain having a sequence of SEQ ID NO:17 or SEQ ID NO:43, or having 95-99% identity thereof.

In one instance, the CAR molecule comprises (e.g., consists of) an amino acid sequence of SEQ ID NO:58, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or SEQ ID NO:42, or an amino acid sequence having at least one, two, three, four, five, 10, 15, 20 or 30 modifications (e.g., substitutions) but not more than 60, 50 or 40 modifications (e.g., substitutions) of an amino acid sequence of SEQ ID NO:58, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or SEQ ID NO:42, or an amino acid sequence having 85%, 90%, 95%, 96%, 97%, 98% or 99% identity to an amino acid sequence of SEQ ID NO:58, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41 or SEQ ID NO:42.

In one instance, the cell expressing the CAR molecule comprises a vector that includes a nucleic acid sequence encoding the CAR molecule. In one instance, the vector is selected from the group consisting of a DNA, a RNA, a plasmid, a lentivirus vector, adenoviral vector, or a retrovirus vector. In one instance, the vector is a lentivirus vector. In one instance, the vector further comprises a promoter. In one instance, the promoter is an EF-1 promoter. In one instance, the EF-1 promoter comprises a sequence of SEQ ID NO: 100. In one instance, the vector is an *in vitro* transcribed vector, e.g., a vector that transcribes RNA of a nucleic acid molecule described herein. In one instance, the nucleic acid sequence in the *in vitro* vector further comprises a poly(A) tail, e.g., a poly A tail described herein, e.g., comprising about 150 adenosine bases (SEQ ID NO:104). In one instance, the nucleic acid sequence in the *in vitro* vector further comprises a 3'UTR, e.g., a 3' UTR described herein, e.g., comprising at least one repeat of a 3'UTR derived from human beta-globulin. In one instance, the nucleic acid sequence in the *in vitro* vector further comprises promoter, e.g., a T2A promoter.

In certain instances of the compositions and methods disclosed herein, the cell expressing the CAR molecule (also referred to herein as a "CAR-expressing cell") is a cell or population of cells as described herein, e.g., a human immune effector cell or population of cells (e.g., a human T cell or a human NK cell, e.g., a human T cell described herein or a human NK cell described herein). In one instance, the human T cell is a CD8+ T cell. In one instance, the cell is an autologous T cell. In one instance, the cell is an allogeneic T cell. In one instance, the cell is a T cell and the T cell is diaglycerol kinase (DGK) deficient. In one instance, the cell is a T cell and the T cell is Ikaros deficient. In one instance, the cell is a T cell and the T cell is both DGK and Ikaros deficient. It shall be understood that the compositions and methods disclosed herein reciting the term "cell" encompass compositions and methods comprising one or more cells, e.g., a population of cells.

In another instance, the cell expressing the CAR molecule, e.g., as described herein, can further express another agent, e.g., an agent which enhances the activity of a CAR-expressing cell.

In one instance, the method further includes administering a cell expressing the CAR molecule, as described herein, in combination with a BTK inhibitor described herein, in combination with an agent which enhances the activity of a CAR-expressing cell. In certain instances, the agent is a cytokine, e.g., IL-7, IL-15, IL-21, or a combination thereof. In one instance, the method includes administering IL-7 to the subject. The cytokine can be delivered in combination with, e.g., simultaneously or shortly after, administration of the CAR-expressing cell. Alternatively, the cytokine can be delivered after a prolonged period of time after administration of the CAR-expressing cell, e.g., after assessment of the subject's response to the CAR-expressing cell.

In other instances, the agent which enhances the activity of a CAR-expressing cell can be an agent which inhibits an immune inhibitory molecule. Examples of immune inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR beta. In one instance, the agent which inhibits an immune inhibitory molecule comprises a first polypeptide, e.g., an immune inhibitory molecule, associated with a second polypeptide that provides a positive signal to the cell, e.g., an intracellular signaling domain described herein. In one instance, the agent comprises a first polypeptide, e.g., of an inhibitory molecule such as PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGFR beta, or a fragment of any of these (e.g., at least a portion of the extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one instance, the agent comprises a first polypeptide of PD1 or a fragment thereof (e.g., at least a portion of the extracellular domain of PD1), and a second polypeptide of an intracellular signaling domain described herein (e.g., a CD28 signaling domain described herein and/or a CD3 zeta signaling domain described herein).

In one instance, lymphocyte infusion, for example allogeneic lymphocyte infusion, is used in the treatment of the cancer, wherein the lymphocyte infusion comprises at least one CAR-expressing cell that binds to a B-cell antigen e.g., CD19 (also referred to herein as CD19 CAR-expressing cell), as described herein. In one instance, autologous lymphocyte infusion is used in the treatment of the cancer, wherein the autologous lymphocyte infusion comprises at least one CD19-expressing cell.

In one instance, the CD19 CAR expressing cell, e.g., T cell, is administered to a subject that has received a previous stem cell transplantation, e.g., autologous stem cell transplantation.

In one instance, the CD19 CAR expressing cell, e.g., T cell, is administered to a subject that has received a previus dose of melphalan.

In one instance, the cell expressing the CAR molecule, e.g., a CAR molecule described herein, is administered in combination with an agent that ameliorates one or more side effect associated with administration of a cell expressing a CAR molecule, e.g., an agent described herein.

In one instance, the BTK inhibitor is administered in combination with an agent that ameliorates one or more side effect associated with administration of the BTK inhibitor, e.g., an agent described herein.

In one instance, the cell expressing the CAR molecule, e.g., a CAR molecule described herein, and the BTK inhibitor are administered in combination with an additional agent that treats the disease associated with a B-cell antigen e.g., CD19, e.g., an additional agent described herein.

In one instance, the cells expressing a CAR molecule, e.g., a CAR molecule described herein, are administered at a dose and/or dosing schedule described herein.

In one instance, the CAR molecule is introduced into T cells, e.g., using *in vitro* transcription, and the subject (e.g., human) receives an initial administration of cells comprising a CAR molecule, and one or more subsequent administrations of cells comprising a CAR molecule, wherein the one or more subsequent administrations are administered less than 15 days, e.g., 14, 13, 12, 11, 10, 9, 8, 7, 6, 5, 4, 3, or 2 days after the previous administration. In one instance, more than one administration of cells comprising a CAR molecule are administered to the subject (e.g., human) per week, e.g., 2, 3, or 4 administrations of cells comprising a CAR molecule are administered per week. In one instance, the subject (e.g., human subject) receives more than one administration of cells comprising a CAR molecule per week (e.g., 2, 3 or 4 administrations per week) (also referred to herein as a cycle), followed by a week of no administration of cells comprising a CAR molecule, and then one or more additional administration of cells comprising a CAR molecule (e.g., more than one administration of the cells comprising a CAR molecule per week) is administered to the subject. In another instance, the subject (e.g., human subject) receives more than one cycle of cells comprising a CAR molecule, and the time between each cycle is less than 10, 9, 8, 7, 6, 5, 4, or 3 days. In one instance, the cells comprising a CAR molecule are administered every other day for 3 administrations per week. In one instance, the cells comprising a CAR molecule are administered for at least two, three, four, five, six, seven, eight or more weeks.

In one instance, the combination of the BTK inhibitor and the cells expressing a CAR molecule, e.g., a CAR molecule described herein, are administered as a first line treatment for the disease, e.g., the cancer, e.g., the cancer described herein. In another instance, the combination of the BTK inhibitor and the cells expressing a CAR molecule, e.g., a CAR molecule described herein, are administered as a second, third, fourth line treatment for the disease, e.g., the cancer, e.g., the cancer described herein.

In one instance, a cell (e.g., a population of cells) described herein is administered to the subject.

In one instance, the method includes administering a population of cells, a plurality of which comprise a CAR molecule described herein. In some instances, the population of CAR-expressing cells comprises a mixture of cells expressing different CARs. For example, in one instance, the population of CAR-expressing cells can include a first cell expressing a CAR having an anti-CD 19 binding domain described herein, and a second cell expressing a CAR having a different anti- CD19 binding domain, e.g., an anti-CD 19 binding domain described herein that differs from the anti-CD 19 binding domain in the CAR expressed by the first cell. As another example, the population of CAR-expressing cells can include a first cell expressing a CAR that includes an anti- CD19 binding domain, e.g., as described herein, and a second cell expressing a CAR that includes an antigen binding domain to a target other than CD19 (e.g., CD123 or mesothelin). In one instance, the population of CAR-expressing cells includes, e.g., a first cell expressing a CAR that includes a primary intracellular signaling domain, and a second cell expressing a CAR that includes a secondary signaling domain.

In one instance, the method includes administering a population of cells wherein at least one cell in the population expresses a CAR having an anti- CD19 domain described herein, and an agent which enhances the activity of a CAR-expressing cell, e.g., a second cell expressing the agent which enhances the activity of a CAR-expressing cell. For example, in one instance, the agent can be an agent which inhibits an immune inhibitory molecule. Examples of immune inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR beta. In one instance, the agent which inhibits an immune inhibitory molecule comprises a first polypeptide, e.g., an inhibitory molecule, associated with a second polypeptide that provides a positive signal to the cell, e.g., an intracellular signaling domain described herein. In one instance, the agent comprises a first polypeptide, e.g., of an inhibitory molecule such as PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGFR beta, or a fragment of any of these (e.g., at least a portion of an extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one instance, the agent comprises a first polypeptide of PD1 or a fragment thereof (e.g., at least a portion of the extracellular domain of PD1), and a second polypeptide of an intracellular signaling domain described herein (e.g., a CD28 signaling domain described herein and/or a CD3 zeta signaling domain described herein).

In another aspect, the disclosure pertains to a cell expressing a CAR molecule described herein for use as a medicament in combination with a BTK inhibitor described herein, e.g., a compound of formula (I). In another aspect, the disclosure pertains to a BTK inhibitor described herein, e.g., a compound of formula (I) or a pharmaceutically acceptable salt thereof, for use as a medicament in combination with a cell expressing a CAR molecule described herein.

In another aspect, the disclosure pertains to a cell expressing a CAR molecule described herein for use in combination with a BTK inhibitor described herein, e.g., a compound of formula (I) or a pharmaceutically acceptable salt thereof, in the treatment of a disease expressing the B-cell antigen (e.g., CD19). In another aspect, the disclosure pertains to a BTK inhibitor described herein, e.g., a compound of formula (I) or a pharmaceutically acceptable salt thereof, for use in combination with a cell expressing a CAR molecule described herein, in the treatment of a disease expressing a B-cell antigen (e.g., CD19). The disease may be, e.g., a cancer such as a hematologic cancer. The cancer may be, e.g., a lymphoma, CLL, MCL, ALL, DLBCL, multiple myeloma, or another cancer described herein.

In another aspect, the disclosure pertains to a cell expressing a CAR molecule described herein for use as a medicament in combination with a BTK inhibitor described herein, e.g., a compound of formula (I) or a pharmaceutically acceptable salt thereof, and a cytokine, e.g., IL-7, IL-15 and/or IL-21 as described herein.

In another aspect, the disclosure pertains to a cell expressing a CAR molecule described herein for use in combination with a BTK inhibitor described herein, e.g., a compound of formula (I) or a pharmaceutically acceptable salt thereof, and a cytokine, e.g., IL-7, IL-15 and/or IL-21 as described herein, in the treatment of a disease expressing CD19. In another aspect, the disclosure pertains to a cytokine described herein for use in combination with a cell expressing a CAR molecule described herein and a BTK inhibitor described herein, e.g., a compound of formula (I) or a pharmaceutically acceptable salt thereof, in the treatment of a disease expressing CD19.

In one instance, the cell expressing a CAR molecule, e.g., a CAR molecule described herein, is administered in combination with an agent that increases the efficacy of a cell expressing a CAR molecule, e.g., an agent described herein.

In one instance, the cell expressing a CAR molecule, e.g., a CAR molecule described herein, is administered in combination with an agent that ameliorates one or more side effect associated with administration of a cell expressing a CAR molecule, e.g., an agent described herein.

In one instance, the cell expressing a CAR molecule, e.g., a CAR molecule described herein, is administered in combination with a low, immune enhancing dose of an mTOR inhibitor, e.g., an mTOR inhibitor described herein. While not wishing to be bound by theory, it is believed that treatment with a low, immune enhancing, dose (e.g., a dose that is insufficient to completely suppress the immune system but sufficient to improve immune function) is accompanied by a decrease in PD-1 positive T cells or an increase in PD-1 negative cells. PD-1 positive T cells, but not PD-1 negative T cells, can be exhausted by engagement with cells which express a PD-1 ligand, e.g., PD-L1 or PD-L2.

In an instance this approach can be used to optimize the performance of a CAR cell described herein in the subject. While not wishing to be bound by theory, it is believed that, in an instance, the performance of endogenous, non-modified immune effector cells, e.g., T cells, is improved. While not wishing to be bound by theory, it is believed that, in an instance, the performance of a CD19 CAR expressing cell is improved. In other instances, cells, e.g., T cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In an instance, administration of a low, immune enhancing, dose of an mTOR inhibitor, e.g., an allosteric inhibitor, e.g., RAD001, or a catalytic inhibitor, is initiated prior to administration of an CAR expressing cell described herein, e.g., T cells. In an instance, the mTOR inhibitor is RAD001 or rapamycin. In an instance, the CAR cells are administered after a sufficient time, or sufficient dosing, of an mTOR inhibitor, such that the level of PD1 negative immune effector cells, e.g., T cells, or the ratio of PD1 negative immune effector cells, e.g., T cells/ PD1 positive immune effector cells, e.g., T cells, has been, at least transiently, increased.

In an instance, the cell, e.g., an immune effector cell (e.g., a T cell or NK cell), to be engineered to express a CAR, is harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In instances, any of the methods described herein further comprise performing lymphodepletion on a subject, e.g., prior to administering the one or more cells that express a CAR molecule described herein, e.g., a CAR molecule that binds CD19. The lymphodepletion can comprise, e.g., administering one or more of melphalan, cytoxan, cyclophosphamide, and fludarabine.

In some instances, the CAR-expressing cell that is administered comprises a regulatable CAR (RCAR), e.g., an RCAR as described herein. The RCAR may comprise, e.g., an intracellular signaling member comprising an intracellular signaling domain and a first switch domain, an antigen binding member comprising an antigen binding domain that binds CD19 and a second switch domain; and a transmembrane domain. The method may further comprise administering a dimerization molecule, e.g., in an amount sufficient to cause dimerization of the first switch and second switch domains.

In some instances, the CAR-expressing cell and the BTK inhibitor are administered simultaneously or substantially simultaneously, e.g., as a first line of therapy. In some instances, the method comprises administering a combination of the BTK inhibitor and the CAR-expressing cell (e.g., a CAR19-expressing cell) to the subject, as a first line therapy.

In other instances, the CAR-expressing cell and the BTK inhibitor are administered sequentially. For example, the BTK inhibitor is administered before the CAR-expressing cell, or the CAR-expressing cell is administered before the BTK inhibitor.

In some instances, the disease associated with expression of CD19 is a hematological cancer (e.g., a hematological cancer described herein such as CLL, MCL, DLBCL, or ALL) and the subject is, or is identified as, a partial responder, non-responder, or relapser to one or more therapies for the hematological cancer, e.g., to a BTK inhibitor such as ibrutinib. In some instances, the subject has, or is identified as having, a BTK mutation such as C481S. The mutation may be, e.g., a point mutation, an insertion, or a deletion. The mutation may be, e.g., a mutation at the binding site for the BTK inhibitor, e.g., at or near the ATP-binding pocket. The mutation may confer a decreased response (e.g., resistance) to the BTK inhibitor.

In some instances of any of the methods disclosed herein, the method comprises administering the BTK inhibitor to the subject, reducing the amount (e.g., ceasing administration) of the BTK inhibitor, and subsequently administering the CAR-expressing cell (e.g., a CAR19-expressing cell) to the subject.

In some instances, the method comprises administering the BTK inhibitor to the subject and subsequently administering a combination of the BTK inhibitor and the CAR-expressing cell (e.g., a CAR19-expressing cell) to the subject.

In some instances, the method comprises administering a BTK inhibitor (e.g., ibrutinib, GDC-0834, RN-486, CGT-560, CGT-1764, HM-71224, CC-292, ONO-4059, CNX-774, or LFM-A13, or a combination thereof) to the subject, reducing the amount (e.g., ceasing or discontinuing administration) of the BTK inhibitor, and subsequently administering a combination of the CAR-expressing cell (e.g., a CAR19-expressing cell) and a second BTK inhibitor to the subject, wherein the second BTK inhibitor is a BTK inhibitor described herein.

In some instances, the patient has been administered a BTK inhibitor such as ibrutinib. In some instances, the patient does not have a complete response to the BTK inhibitor such as ibrutinib. For instance, in some instances, the patient has (or is identified as having) a partial response, stable disease, progressive disease, or relapse to treatment with a BTK inhibitor such as ibrutinib, GDC-0834, RN-486, CGI-560, CGI-1764, HM-71224, CC-292, ONO-4059, CNX-774, or LFM-A13, or a combination thereof. In some instances, the patient who does not have a complete response to the BTK inhibitor such as ibrutinib is treated with a combination of a CAR-expressing cell (e.g., a CAR19-expressing cell) and a second BTK inhibitor, wherein the second BTK inhibitor is a compound of formula (I). The CAR-expressing cell and the second BTK inhibitor can be administered, e.g., substantially simultaneously or sequentially, e.g., the CAR-expressing cell can be administered before the second BTK inhibitor or the second BTK inhibitor can be administered before the CAR-expressing cell.

In some instances, the disease associated with expression of the B-cell antigen (e.g., CD19) is a hematological cancer (e.g., a hematological cancer described herin, e.g., CLL, MCL, or ALL), and the method delays or decreases resistance to the BTK inhibitor described herein, the CAR-expressing cell (e.g., a CAR19-expressing cell), or both. In some instances, the disease associated with expression of the B-cell antigen (e.g., CD19) is a hematological cancer (e.g., a hematological cancer described herin, e.g., CLL, MCL, DLBCL, or ALL), and wherein the method prolongs remission or delays relapse of the hematological cancer. For example, remission can be prolonged, relapse can be delayed, resistance can be delayed, or resistance can be decreased, compared to the expected course of disease when treated with a monotherapy of the BTK inhibitor or the CAR-expressing cell.

Exemplary treatment regimens that can be used in any of the aforesaid methods include one or more of the following:
In one instance, the BTK inhibitor, e.g., a compound of formula (I), and the CAR19-expressing cell are administered to the mammal as a first line of therapy.

In another instance, the the CAR-expressing cell (e.g., the CAR19-expressing cell) is administered to the mammal after administration of the BTK inhibitor, e.g., a compound of formula (I).

In other instances, the the CAR-expressing cell (e.g., the CAR19-expressing cell) is administered after ceasing administration of the BTK inhibitor, e.g., a compound of formula (I).

In other instances, administration of the BTK inhibitor, e.g., a compound of formula (I), is begun prior to administration of the the CAR-expressing cell (e.g., the CAR19-expressing cell), and the CAR-expressing cell is administered in combination with continued administration of the BTK inhibitor.

In one instance, a subject is administered a BTK inhibitor, e.g., a compound of formula (I), e.g., as a first line therapy. After a predetermined time interval, (e.g., 1 or 2 months but also 2 weeks, 3 weeks, 1 month, 1.5 months, 2 months, 3 months, 4 months, 6 months, 9 months, 12 months, 15 months, or 18 months), a CAR-expressing cell (e.g., a CAR19-expressing cell) is administered to the subject alone, or in combination with the BTK inhibitor. In some instances, the subject's response to the treatment is assessed at predetermined time intervals, e.g., before or during treatment with the kinase inhibitor and/or CAR-expressing cell. If the assessment shows that the subject is a complete responder, the CAR-expressing cell (e.g., a CAR19-expressing cell) is not administered. If the assessment shows that the subject is a partial responder, or has stable disease in response, to the BTK inhibitor, the CAR-expressing cell (e.g., a CAR19-expressing cell) is administered in combination with the BTK inhibitor e.g., as described herein. If the assessment shows that the subject is a non-responder or relapser, the CAR-expressing cell (e.g., a CAR19-expressing cell) is administered in combination with the BTK inhibitor or a second BTK inhibitor, e.g., a second BTK inhibitor as described herein.

In other instances, the mammal is, or is identified as being, a complete or partial responder to the BTK inhibitor, e.g., a compound of formula (I), or a complete or partial responder to the CAR19-expressing cell.

In some instances, when a subject is (or is identified as being) a complete responder to the BTK inhibitor, e.g., a compound of formula (I), the subject is not administered a CAR-expressing cell (e.g., a CAR19-expressing cell) during the period of complete response. In other instances, when a subject is (or is identified as being) a complete responder (e.g., a complete responder to the BTK inhibitor), the subject is administered a CAR-expressing cell (e.g., a CAR19-expressing cell) during the period of complete response. In an instance, after the CAR-expressing cell (e.g., a CAR19-expressing cell), the subject experiences a prolonged response or delayed relapse (e.g., compared to the expected course of disease when treated without the CAR therapy).

In some instances, when a subject is (or is identified as being) a partial responder to the BTK inhibitor, e.g., a compound of formula (I), the subject is not administered a CAR-expressing cell (e.g., a CAR19-expressing cell) during the period of partial response. In other instances, when a subject is (or is identified as being) a partial responder to the BTK inhibitor, the subject is administered a CAR-expressing cell (e.g., a CAR19-expressing cell) (alone or in combination with the BTK inhibitor) during the period of partial response. In an instance, after the CAR therapy, the subject experiences a complete response and/or prolonged response or delayed relapse (e.g., compared to the expected course of disease when treated without CAR therapy).

In some instances, when a subject has (or is identified as having) stable disease after treatment with the BTK inhibitor, e.g., a compound of formula (I), the subject is not administered a CAR therapy during the period of stable disease. In other instances, when a subject has (or is identified as having) stable disease after treatment with the BTK inhibitor, the subject is administered a CAR therapy during the period of stable disease. In an instance, after the CAR therapy, the subject experiences a partial response, a complete response and/or prolonged response or delayed relapse (e.g., compared to the expected course of disease when treated without CAR therapy).

In some instances, when a subject has (or is identified as having) progressive disease after treatment with the BTK inhibitor, e.g., a compound of formula (I), the subject is not administered a CAR-expressing cell (e.g., a CAR19-expressing cell) during the period of progressive disease. In other instances, when a subject has (or is identified as having) progressive disease after treatment with the BTK inhibitor, the subject is administered a CAR-expressing cell (e.g., a CAR19-expressing cell) during the period of progressive disease. In an instance, after the CAR therapy, the subject experiences stable disease, a partial response, a complete response and/or prolonged response or delayed relapse (e.g., compared to the expected course of disease when treated without CAR therapy).

In other instances, the the CAR-expressing cell (e.g., the CAR19-expressing cell) is administered in combination a second BTK inhibitor, wherein the second kinase inhibitor is a BTK inhibitor described herein, e.g., a compound of formula (I), when the mammal is, or is identified as being, a non-responder or relapser to a first BTK inhibitor, e.g., ibrutinib, GDC-0834, RN-486, CGI-560, CGI-1764, HM-71224, CC-292, ONO-4059, CNX-774, or LFM-A13.

In other instances, the subject, e.g., the mammal, is (or is identified as being) a partial responder to the BTK inhibitor, and the mammal is administered the CAR-expressing cell (e.g., the CAR19-expressing cell), alone or in combination with the BTK inhibitor, during the period of partial response.

In other instances, the subject, e.g., the mammal, is (or has identified as being) a non-responder having progressive or stable disease after treatment with the BTK inhibitor, and the mammal is administered the the CAR-expressing cell (e.g., the CAR19-expressing cell), alone or in combination with a second BTK inhibitor, during the period of progressive or stable disease, wherein the second BTK inhibitor is a BTK inhibitor described herein, e.g., a compound of formula (I).

In another aspect, disclosed herein is a method of treating a subject, e.g., a mammal, having a disease associated with expression of the B-cell antigen (e.g., CD19). The method comprises administering to the subject an effective amount of a BTK inhibitor as described herein, e.g., a compound of formula (I) or a pharmaceutically acceptable salt thereof, and a CAR-expressing cell (e.g., a CAR19-expressing cell) in combination (e.g. simultaneously (or substantially simultaneously), or sequentially).

In some instances, the BTK inhibitor, e.g., a compound of formula (I) and the CAR-expressing cell (e.g., a CAR19 cell) are administered, in combination, e.g., as a first line of therapy,

In some instances, the BTK inhibitor, e.g., a compound of formula (I) is administered initially, e.g., a monotherapy or first line of therapy; after reducing the amount (e.g., ceasing or discontinuing administration) of the BTK inhibitor, administering the CAR-expressing cell (e.g., a CAR19-expressing cell) to the subject.

In other instances, the BTK inhibitor, e.g., a compound of formula (I) is administered initially, e.g., a monotherapy or first line of therapy; and subsequently administering a combination of the BTK inhibitor and the CAR-expressing cell (e.g., a CAR19-expressing cell) to the subject.

In other instances, the BTK inhibitor, e.g., a compound of formula (I) is administered initially, e.g., a monotherapy or first line of therapy; after reducing the amount (e.g., ceasing or discontinuing administration) of the BTK inhibitor, administering a combination of a second BTK inhibitor and the CAR-expressing cell (e.g., a CAR19-expressing cell) to the subject.

In some instances, the subject's response to the treatment is assessed at predetermined time intervals, e.g., before or during treatment with the kinase inhibitor and/or CAR-expressing cell. If the assessment shows that the subject is a complete responder, the CAR-expressing cell (e.g., a CAR19-expressing cell) is not administered. If the assessment shows that the subject is a partial responder, or has stable disease in response, to the kinase inhibitor, the CAR-expressing cell (e.g., a CAR19-expressing cell) is administered in combination with the BTK inhibitor as described herein. If the assessment shows that the subject is a non-responder or relapser, the CAR-expressing cell (e.g., a CAR19-expressing cell) is administered in combination with the BTK inhibitor or a second BTK inhibitor, e.g., a second BTK inhibitor as described herein. In some instances, the first BTK inhibitor, the second BTK inhibitor, or both, are compounds of formula (I).

In some instances, the disease associated with expression of the B-cell antigen (e.g., CD19) is a hematological cancer, leukemia, lymphoma, MCL, CLL, ALL, DLBCL, Hodgkin lymphoma, or multiple myeloma.

In another aspect, the disclosure provides a method of modulating BTK activity in a mammal, comprising administering to the mammal an effective amount of a cell (e.g., a population of cells) that expresses a CAR molecule that binds the B-cell antigen (e.g., CD19), in combination with a BTK inhibitor, wherein the BTK inhibitor comprises a compound of formula (I) or a pharmaceutically acceptable salt thereof.

In another aspect, the disclosure features a composition comprising a cell that expresses a CAR molecule that binds the B-cell antigen (e.g., one or more of CD19, CD20. CD22 or ROR1), and one or more BTK inhibitors as described herein, e.g., a compound of formula (I). The the CAR-expressing cell (e.g., the CAR19-expressing cell) and the one or more BTK inhibitors can be present in a single dose form, or as two or more dose forms.

In instances, the compositions disclosed herein are for use as a medicament.

In instances, the compositions disclosed herein are use in the treatment of a disease associated with expression of a B-cell antigen (e.g., CD19).

### Methods and compositions for producing CAR-expressing cells

The present disclosure also provides, in certain aspects, a method of making a population of immune effector cells (e.g., T cells or NK cells) that can be engineered to express a CAR (e.g., a CAR described herein), the method comprising: providing a population of immune effector cells; and contacting the immune effector cells with a BTK inhibitor described herein, e.g., a compound of formula (I), under conditions sufficient to inhibit BTK. The method can further comprise contacting, e.g., transducing, the immune effector cells with a nucleic acid encoding a CAR molecule.

In some aspects, the disclosure provides a method of making a CAR-expressing cell (e.g., a CAR-expressing immune effector cell or population of cells), comprising: contacting the cell or population of cells with a BTK inhibitor described herein, e.g., a compound of formula (I); and introducing (e.g., transducing) a nucleic acid encoding a CAR molecule into the cell or population of cells under conditions such that the CAR molecule is expressed.

In certain instances of the methods of producing CAR-expressing cells, the CAR molecule encoded by the nucleic acid is a CAR molecule that binds CD19. In instances, the method further comprises culturing the cell or cells under conditions that allow the cell or at least a sub-population of the cells to express the CAR molecule. In instances, the cell is a T cell or NK cell, or the population of cells includes T cells, NK cells, or both. In instances, the method comprises contacting the cell or cells with the BTK inhibitor and subsequently removing most or all of the BTK inhibitor from the cell or cells. In instances, the kinase inhibitor is added after the cell or cells are harvested or before the cell or cells are stimulated. In instances, the population of cells also comprises cancer cells, e.g., leukemia or lymphoma cells. The cancer cells may be, e.g., CLL, MCL, or ALL cells. In instances, the BTK inhibitor inhibits BTK in the cancer cells, e.g., reduces its activity by at least 10%, 20%, 30%, 40%, 50%, 60%, 70%, 80%, 90%, 95%, or 99%. In instances, the BTK inhibitor inhibits a target in the immune effector cells, e.g., reduces its activity by at least 10%, 20%, 30%, 40%, 50%, 60%, 70%, 80%, 90%, 95%, or 99%. In instances, the method further comprises depleting T regulatory cells (e.g., CD25+ cells) from the population of cells.

In some aspects, the present disclosure also provides a reaction mixture comprising a BTK inhibitor, e.g., a compound of formula (I), and a CAR molecule or a nucleic acid encoding a CAR molecule. In some instances, the reaction mixture further comprises a population of immune effector cells.

In some instances, one or more of the immune effector cells expresses the CAR molecule or comprises the nucleic acid encoding the CAR molecule. In instances, the reaction mixture comprises cancer cells, e.g., haematological cancer cells. The cancer cells may be, e.g., cells that were harvested from the subject when the immune effector cells were harvested from the subject.

In instances, a reaction mixture as described herein further comprises a buffer or other reagent, e.g., a PBS containing solution. In instances, the reaction mixture further comprises an agent that activates and/or expands to cells of the population, e.g., an agent that stimulates a CD3/TCR complex associated signal and/or a ligand that stimulates a costimulatory molecule on the surface of the cells. In instances, the agent is a bead conjugated with anti-CD3 antibody, or a fragment thereof, and/or anti-CD28 antibody, or a fragment thereof. In instances, the reaction mixture further comprises one or more factors for proliferation and/or viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells. In instances, the reaction mixture further comprises IL-15 and/or IL-7. In instances, a plurality of the cells of the population in the reaction mixture comprise a nucleic acid molecule, e.g., a nucleic acid molecule described herein, that comprises a CAR encoding sequence, e.g., a CD19 CAR encoding sequence, e.g., as described herein. In instances, a plurality of the cells of the population in the reaction mixture comprise a vector comprising a nucleic acid sequence encoding a CAR, e.g., a CAR described herein, e.g., a CD19 CAR described herein. In instances, the vector is a vector described herein, e.g., a vector selected from the group consisting of a DNA, a RNA, a plasmid, a lentivirus vector, adenoviral vector, or a retrovirus vector. In instances, the reaction mixture further comprises a cryoprotectant or stabilizer such as, e.g., a saccharide, an oligosaccharide, a polysaccharide and a polyol (e.g., trehalose, mannitol, sorbitol, lactose, sucrose, glucose and dextran), salts and crown ethers. In one instance, the cryoprotectant is dextran.

In some instances, the method of making discosed herein further comprises contacting the population of immune effector cells with a nucleic acid encoding a telomerase subunit, e.g., hTERT. The the nucleic acid encoding the telomerase subunit can be DNA.

In some instances, the method of making discosed herein further comprises culturing the population of immune effector cells in serum comprising 2% hAB serum.

### BTK inhibitors

In some instances of the methods, uses, and compositions herein, the BTK inhibitor is a compound of formula (I) or a pharmaceutically acceptable salt thereof; 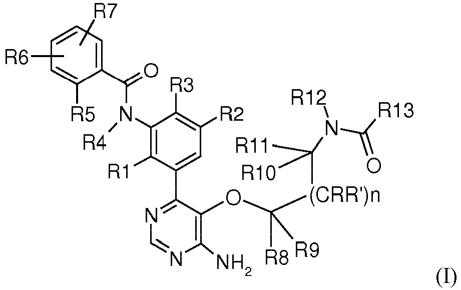 wherein,
R1 is hydrogen, C1-C6 alkyl optionally substituted by hydroxy;
R2 is hydrogen or halogen;
R3 is hydrogen or halogen;
R4 is hydrogen;
R5 is hydrogen or halogen;
or R4 and R5 are attached to each other and stand for a bond, -CH2-, -CH2-CH2-, -CH=CH-, - CH=CH-CH2-; -CH2-CH=CH-; or -CH2-CH2-CH2-;
R6 and R7 stand independently from each other for H, C1-C6 alkyl optionally substituted by hydroxyl, C3-C6 cycloalkyl optionally substituted by halogen or hydroxy, or halogen;
R8, R9, R, R', R10 and R11 independently from each other stand for H, or C1-C6 alkyl optionally substituted by C1-C6 alkoxy; or any two of R8, R9, R, R', R10 and R11 together with the carbon atom to which they are bound may form a 3 - 6 membered saturated carbocyclic ring;
R12 is hydrogen or C1-C6 alkyl optionally substituted by halogen or C1-C6 alkoxy;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl, C1-C6 alkoxy or N,N-di-C1-C6 alkyl amino; C2-C6 alkynyl optionally substituted by C1-C6 alkyl or C1-C6 alkoxy; or C2-C6 alkylenyl oxide optionally substituted by C1-C6 alkyl.

In some instances, R1 is hydrogen, or C1-C6 alkyl optionally substituted by hydroxy;
R2 is halogen;
R3 is hydrogen;
R4 is hydrogen;
R5 is halogen;
or R4 and R5 are attached to each other and stand for a bond, -CH2-, -CH2-CH2-, -CH=CH-, - CH=CH-CH2-; -CH2-CH=CH-; or -CH2-CH2-CH2-;
R6 and R7 stand independently from each other for H, C1-C6 alkyl optionally substituted by hydroxyl, C3-C6 cycloalkyl optionally substituted by halogen or hydroxy, or halogen;
R8, R9, R10 and R11 independently from each other stand for H, or C1-C6 alkyl; or any two of R8, R9, R10 and R11 together with the carbon atom to which they are bound may form a 3 - 6 membered saturated carbocyclic ring;
R and R' are hydrogen;
R12 is hydrogen or C1-C6 alkyl optionally substituted by halogen;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl or C1-C6 alkoxy; C2-C6 alkynyl optionally substituted by C1-C6 alkyl or C1-C6 alkoxy; or C2-C6 alkylenyl oxide optionally substituted by C1-C6 alkyl.

In some instances,
R1 is hydrogen, or C1-C6 alkyl optionally substituted by hydroxy;
R2 is halogen;
R3 is hydrogen;
R4 is hydrogen;
R5 is halogen;
R6 and R7 stand independently from each other for H, C1-C6 alkyl optionally substituted by hydroxyl, C3-C6 cycloalkyl optionally substituted by halogen or hydroxy, or halogen;
R8, R9, R10 and R11 independently from each other stand for H, or C1-C6 alkyl; or any two of R8, R9, R10 and R11 together with the carbon atom to which they are bound may form a 3 - 6 membered saturated carbocyclic ring;
R and R' are hydrogen;
R12 is hydrogen or C1-C6 alkyl optionally substituted by halogen;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl or C1-C6 alkoxy; C2-C6 alkynyl optionally substituted by C1-C6 alkyl or C1-C6 alkoxy; or C2-C6 alkylenyl oxide optionally substituted by C1-C6 alkyl.

In some instances,
R1 is hydrogen, C1-C6 alkyl optionally substituted by hydroxy;
R2 is hydrogen or halogen;
R3 is hydrogen or halogen;
R4 and R5 are attached to each other and stand for a bond, -CH2-, -CH2-CH2-, -CH=CH-, - CH=CH-CH2-; -CH2-CH=CH-; or -CH2-CH2-CH2-;
R6 and R7 stand independently from each other for H, C1-C6 alkyl optionally substituted by hydroxyl, C3-C6 cycloalkyl optionally substituted by halogen or hydroxy, or halogen;
R8, R9, R10 and R11 independently from each other stand for H, or C1-C6 alkyl; or any two of R8, R9, R10 and R11 together with the carbon atom to which they are bound may form a 3 - 6 membered saturated carbocyclic ring;
R and R' are hydrogen;
R12 is hydrogen or C1-C6 alkyl optionally substituted by halogen;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl or C1-C6 alkoxy; C2-C6 alkynyl optionally substituted by C1-C6 alkyl or C1-C6 alkoxy; or C2-C6 alkylenyl oxide optionally substituted by C1-C6 alkyl.

In some instances,
R1 is hydrogen, C1-C6 alkyl optionally substituted by hydroxy;
R2 is hydrogen or halogen;
R3 is hydrogen or halogen;
R4 and R5 are attached to each other and stand for a -CH2-CH2-, or -CH=CH-;
R6 and R7 stand independently from each other for H, C1-C6 alkyl optionally substituted by hydroxyl, C3-C6 cycloalkyl optionally substituted by halogen or hydroxy, or halogen;
R8, R9, R10 and R11 independently from each other stand for H, or C1-C6 alkyl; or any two of R8, R9, R10 and R11 together with the carbon atom to which they are bound may form a 3 - 6 membered saturated carbocyclic ring;
R and R' are hydrogen;
R12 is hydrogen or C1-C6 alkyl optionally substituted by halogen;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl or C1-C6 alkoxy; C2-C6 alkynyl optionally substituted by C1-C6 alkyl or C1-C6 alkoxy; or C2-C6 alkylenyl oxide optionally substituted by C1-C6 alkyl.

In some instances,
R1 is hydrogen, or C1-C6 alkyl optionally substituted by hydroxy;
R2 is halogen;
R3 is hydrogen;
R4 is hydrogen;
R5 is halogen;
R6 and R7 stand independently from each other for H, C1-C6 alkyl optionally substituted by hydroxyl, C3-C6 cycloalkyl optionally substituted by halogen or hydroxy, or halogen;
R8, R9, R10 and R11 independently from each other stand for H, or C1-C6 alkyl;
R and R' are hydrogen;
R12 is hydrogen or C1-C6 alkyl optionally substituted by halogen;
n is 0 or 1; and
R13 is C2-C6 alkenyl optionally substituted by C1-C6 alkyl or C1-C6 alkoxy; C2-C6 alkynyl optionally substituted by C1-C6 alkyl or C1-C6 alkoxy; or C2-C6 alkylenyl oxide optionally substituted by C1-C6 alkyl.

In some instances,
R1 is hydrogen, or C1-C6 alkyl optionally substituted by hydroxy;
R2 is halogen;
R3 is hydrogen;
R4 is hydrogen;
R5 is halogen;
R6 and R7 stand independently from each other for H, C1-C6 alkyl optionally substituted by hydroxyl, C3-C6 cycloalkyl optionally substituted by halogen or hydroxy, or halogen;
R8 and R9, independently from each other stand for H, or C1-C6 alkyl;
R and R' are hydrogen;
R12 and any one of R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl or C1-C6 alkoxy; C2-C6 alkynyl optionally substituted by C1-C6 alkyl or C1-C6 alkoxy; or C2-C6 alkylenyl oxide optionally substituted by C1-C6 alkyl.

In some instances,
R1 is hydrogen, or C1-C6 alkyl optionally substituted by hydroxy;
R2 is halogen;
R3 is hydrogen;
R4 is hydrogen;
R5 is halogen;
R6 and R7 stand independently from each other for H, C1-C6 alkyl optionally substituted by hydroxyl, C3-C6 cycloalkyl optionally substituted by halogen or hydroxy, or halogen;
R8, R9, R10 and R11 independently from each other stand for H, or C1-C6 alkyl;
R and R' are hydrogen;
R12 is hydrogen or C1-C6 alkyl optionally substituted by halogen;
n is 0 or 1; and
R13 is C2-C6 alkenyl optionally substituted by C1-C6 alkyl or C1-C6 alkoxy.

In some instances,
R1 is hydrogen, or C1-C6 alkyl optionally substituted by hydroxy;
R2 is fluoro;
R3 is hydrogen;
R4 is hydrogen;
R5 is halogen;
R6 and R7 stand independently from each other for H, C1-C6 alkyl optionally substituted by hydroxyl, C3-C6 cycloalkyl optionally substituted by halogen or hydroxy, or halogen;
R8 and R9 independently from each other stand for H, or C1-C6 alkyl;
R12 and any one of R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0; and
R13 is C2-C6 alkenyl optionally substituted by C1-C6 alkyl or C1-C6 alkoxy; or C2-C6 alkynyl optionally substituted by C1-C6 alkyl or C1-C6 alkoxy.

In some instances,
R1 is C1-C6 alkyl;
R2 is fluoro;
R3 is hydrogen;
R4 is hydrogen;
R5 is fluoro;
R6 and R7 stand independently from each other for H, C3-C6 cycloalkyl, or halogen;
R8, R9, R10 and R11 stand for H;
R12 is hydrogen;
n is 0; and
R13 is C2-C6 alkenyl optionally substituted by C1-C6 alkyl.

In some instances,
R1 is C1-C6 alkyl;
R2 is fluoro;
R3 is hydrogen;
R4 is hydrogen;
R5 is fluoro;
R6 and R7 stand independently from each other for H, C3-C6 cycloalkyl, or halogen;
R8, R9, R10 and R11 stand for H;
R12 is methyl;
n is 0; and
R13 is C2-C6 alkenyl optionally substituted by C1-C6 alkyl.

With regard to a compound of formula (I) the following significances represent further instances independently, collectively or in any combination or in any sub-combination thereof:
1. R1 is methyl or hydroxymethyl;
2. R2 is hydrogen or fluoro;
3. R3 is hydrogen
4. R1 is methyl or hydroxymethyl and R2 and R3 are independently hydrogen or fluoro;
5. R4 is hydrogen;
6. R4 together with R5 is -CH2-CH2- , or -CH=CH- ;
7. R5 is fluoro;
8. R6 is H and R7 is C3-C6-cycloalkyl and in particular cyclopropyl;
9. R7 is H and R6 is C3-C6-cycloalkyl and in particular cyclopropyl;
10. R8, R9, R10 and R11 stand for H;
11. R12 and any one of R8, R9, R, R',R10 or R11 together with the atoms to which they are bound may form a 4, 5, 6 or 7 membered azacyclic ring, which ring may optionally be substituted by halogen, hydroxyl, C1-C6 alkyl or C1-C6 alkoxy;
12. R8, R9, R10 and R11 independently from each other stand for H, or C1-C6 alkyl; or any two of R8, R9, R, R', R10 and R11 together with the carbon atom to which they are bound may form a 3-6 membered saturated carbocyclic ring;
13. R12 is hydrogen and R13 stands for C2-C6 alkenyl optionally substituted by C1-C6 alkyl;
14. n = 0;
15. R12 is methyl.

In some instances, the BTK inhibitor is chosen from:
N-(3-(5-((1-Acryloylazetidin-3-yl)oxy)-6-aminopyrimidin-4-yl)-5-fluoro-2-methylphenyl)-4-cyclopropyl-2-fluorobenzamide;
(E)-N-(3-(6-Amino-5-((1-(but-2-enoyl)azetidin-3-yl)oxy)pyrimidin-4-yl)-5-fluoro-2-methylphenyl)-4-cyclopropyl-2-fluorobenzamide;
N-(3-(6-Amino-5-((1-propioloylazetidin-3-yl)oxy)pyrimidin-4-yl)-5-fluoro-2-methylphenyl)-4-cyclopropyl-2-fluorobenzamide;
N-(3-(6-Amino-5-((1-(but-2-ynoyl)azetidin-3-yl)oxy)pyrimidin-4-yl)-5-fluoro-2-methylphenyl)-4-cyclopropyl-2-fluorobenzamide;
N-(3-(5-((1-Acryloylpiperidin-4-yl)oxy)-6-aminopyrimidin-4-yl)-5-fluoro-2-methylphenyl)-4-cyclopropyl-2-fluorobenzamide;
N-(3-(6-Amino-5-(2-(N-methylacrylamido)ethoxy)pyrimidin-4-yl)-5-fluoro-2-methylphenyl)-4-cyclopropyl-2-fluorobenzamide;
(E)-N-(3-(6-Amino-5-(2-(N-methylbut-2-enamido)ethoxy)pyrimidin-4-yl)-5-fluoro-2-methylphenyl)-4-cyclopropyl-2-fluorobenzamide;
N-(3-(6-Amino-5-(2-(N-methylpropiolamido)ethoxy)pyrimidin-4-yl)-5-fluoro-2-methylphenyl)-4-cyclopropyl-2-fluorobenzamide;
(E)-N-(3-(6-Amino-5-(2-(4-methoxy-N-methylbut-2-enamido)ethoxy)pyrimidin-4-yl)-5-fluoro-2-methylphenyl)-4-cyclopropyl-2-fluorobenzamide;
N-(3-(6-Amino-5-(2-(N-methylbut-2-ynamido)ethoxy)pyrimidin-4-yl)-5-fluoro-2-methylphenyl)-4-cyclopropyl-2-fluorobenzamide;
N-(2-((4-Amino-6-(3-(4-cyclopropyl-2-fluorobenzamido)-5-fluoro-2-methylphenyl)pyrimidin-5-yl)oxy)ethyl)-N-methyloxirane-2-carboxamide;
N-(2-((4-Amino-6-(3-(6-cyclopropyl-8-fluoro-1-oxoisoquinolin-2(1H)-yl)phenyl)pyrimidin-5-yl)oxy)ethyl)-N-methylacrylamide;
N-(3-(5-(2-Acrylamidoethoxy)-6-aminopyrimidin-4-yl)-5-fluoro-2-methylphenyl)-4-cyclopropyl-2-fluorobenzamide;
N-(3-(6-Amino-5-(2-(N-ethylacrylamido)ethoxy)pyrimidin-4-yl)-5-fluoro-2-methylphenyl)-4-cyclopropyl-2-fluorobenzamide;
N-(3-(6-Amino-5-(2-(N-(2-fluoroethyl)acrylamido)ethoxy)pyrimidin-4-yl)-5-fluoro-2-methylphenyl)-4-cyclopropyl-2-fluorobenzamide;
N-(3-(5-((1-Acrylamidocyclopropyl)methoxy)-6-aminopyrimidin-4-yl)-5-fluoro-2-methylphenyl)-4-cyclopropyl-2-fluorobenzamide;
(S)-N-(3-(5-(2-Acrylamidopropoxy)-6-aminopyrimidin-4-yl)-5-fluoro-2-methylphenyl)-4-cyclopropyl-2-fluorobenzamide;
(S)-N-(3-(6-Amino-5-(2-(but-2-ynamido)propoxy)pyrimidin-4-yl)-5-fluoro-2-methylphenyl)-4-cyclopropyl-2-fluorobenzamide;
(S)-N-(3-(6-Amino-5-(2-(N-methylacrylamido)propoxy)pyrimidin-4-yl)-5-fluoro-2-methylphenyl)-4-cyclopropyl-2-fluorobenzamide;
(S)-N-(3-(6-Amino-5-(2-(N-methylbut-2-ynamido)propoxy)pyrimidin-4-yl)-5-fluoro-2-methylphenyl)-4-cyclopropyl-2-fluorobenzamide;
N-(3-(6-Amino-5-(3-(N-methylacrylamido)propoxy)pyrimidin-4-yl)-5-fluoro-2-methylphenyl)-4-cyclopropyl-2-fluorobenzamide;
(S)-N-(3-(5-((1-Acryloylpyrrolidin-2-yl)methoxy)-6-aminopyrimidin-4-yl)-5-fluoro-2-methylphenyl)-4-cyclopropyl-2-fluorobenzamide;
(S)-N-(3-(6-Amino-5-((1-(but-2-ynoyl)pyrrolidin-2-yl)methoxy)pyrimidin-4-yl)-5-fluoro-2-methylphenyl)-4-cyclopropyl-2-fluorobenzamide;
(S)-2-(3-(5-((1-Acryloylpyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one;
N-(2-((4-Amino-6-(3-(6-cyclopropyl-1-oxo-3,4-dihydroisoquinolin-2(1H)-yl)-5-fluoro-2-(hydroxymethyl)phenyl)pyrimidin-5-yl)oxy)ethyl)-N-methylacrylamide;
N-(3-(5-(((2S,4R)-1-Acryloyl-4-methoxypyrrolidin-2-yl)methoxy)-6-aminopyrimidin-4-yl)-5-fluoro-2-methylphenyl)-4-cyclopropyl-2-fluorobenzamide;
N-(3-(6-Amino-5-(((2S,4R)-1-(but-2-ynoyl)-4-methoxypyrrolidin-2-yl)methoxy)pyrimidin-4-yl)-5-fluoro-2-methylphenyl)-4-cyclopropyl-2-fluorobenzamide;
2-(3-(5-(((2S,4R)-1-Acryloyl-4-methoxypyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one;
N-(3-(5-(((2S,4S)-1-Acryloyl-4-methoxypyrrolidin-2-yl)methoxy)-6-aminopyrimidin-4-yl)-5-fluoro-2-methylphenyl)-4-cyclopropyl-2-fluorobenzamide;
N-(3-(6-Amino-5-(((2S,4S)-1-(but-2-ynoyl)-4-methoxypyrrolidin-2-yl)methoxy)pyrimidin-4-yl)-5-fluoro-2-methylphenyl)-4-cyclopropyl-2-fluorobenzamide;
N-(3-(5-(((2S,4R)-1-Acryloyl-4-fluoropyrrolidin-2-yl)methoxy)-6-aminopyrimidin-4-yl)-5-fluoro-2-methylphenyl)-4-cyclopropyl-2-fluorobenzamide;
N-(3-(6-Amino-5-(((2S,4R)-1-(but-2-ynoyl)-4-fluoropyrrolidin-2-yl)methoxy)pyrimidin-4-yl)-5-fluoro-2-methylphenyl)-4-cyclopropyl-2-fluorobenzamide;
(S)-N-(3-(5-((1-Acryloylazetidin-2-yl)methoxy)-6-aminopyrimidin-4-yl)-5-fluoro-2-methylphenyl)-4-cyclopropyl-2-fluorobenzamide;
(S)-N-(3-(6-Amino-5-((1-propioloylazetidin-2-yl)methoxy)pyrimidin-4-yl)-5-fluoro-2-methylphenyl)-4-cyclopropyl-2-fluorobenzamide;
(S)-2-(3-(5-((1-Acryloylazetidin-2-yl)methoxy)-6-aminopyrimidin-4-yl)-5-fluoro-2-(hydroxymethyl)phenyl)-6-cyclopropyl-3,4-dihydroisoquinolin-1(2H)-one;
(R)-N-(3-(5-((1-Acryloylazetidin-2-yl)methoxy)-6-aminopyrimidin-4-yl)-5-fluoro-2-methylphenyl)-4-cyclopropyl-2-fluorobenzamide;
(R)-N-(3-(5-((1-Acryloylpiperidin-3-yl)methoxy)-6-aminopyrimidin-4-yl)-5-fluoro-2-methylphenyl)-4-cyclopropyl-2-fluorobenzamide;
N-(3-(5-(((2R,3S)-1-Acryloyl-3-methoxypyrrolidin-2-yl)methoxy)-6-aminopyrimidin-4-yl)-5-fluoro-2-methylphenyl)-4-cyclopropyl-2-fluorobenzamide;
N-(3-(5-(((2S,4R)-1-Acryloyl-4-cyanopyrrolidin-2-yl)methoxy)-6-aminopyrimidin-4-yl)-5-fluoro-2-methylphenyl)-4-cyclopropyl-2-fluorobenzamide;
or
N-(3-(5-(((2S,4S)-1-Acryloyl-4-cyanopyrrolidin-2-yl)methoxy)-6-aminopyrimidin-4-yl)-5-fluoro-2-methylphenyl)-4-cyclopropyl-2-fluorobenzamide.

Headings, sub-headings or numbered or lettered elements, e.g., (a), (b), (i) etc, are presented merely for ease of reading. The use of headings or numbered or lettered elements in this document does not require the steps or elements be performed in alphabetical order or that the steps or elements are necessarily discrete from one another.

The disclosure includes all combinations of any one or more of the foregoing aspects and/or embodiments/instances, as well as combinations with any one or more of the embodiments or instances set forth in the detailed description and examples.

Other features, objects, and advantages of the invention will be apparent from the description and drawings, and from the claims.

Certain instances of the present disclosure provide embodiments of the present invention. More particularly, the present invention provides the following:

1. A composition comprising a cell (e.g., a population of cells) that expresses a CAR molecule that binds CD19 (a "CAR19-expressing cell") for use, in combination with a Bruton's tyrosine kinase (BTK) inhibitor, in the treatment of a mammal having a disease associated with expression of CD19, wherein the disease is an autoimmune disease, an inflammatory disorder or a cancer, wherein the BTK inhibitor comprises a compound of formula (I) or a pharmaceutically acceptable salt thereof; 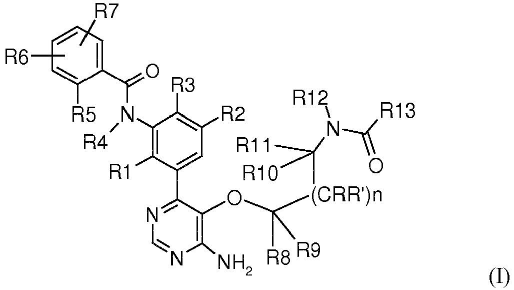 wherein,
R1 is hydrogen, C₁-C₆ alkyl optionally substituted by hydroxy;
R2 is hydrogen or halogen;
R3 is hydrogen or halogen;
R4 is hydrogen;
R5 is hydrogen or halogen;
or R4 and R5 are attached to each other and stand for a bond, -CH₂-, -CH₂-CH₂-, -CH=CH-,-CH=CH-CH₂-; -CH₂-CH=CH-; or -CH₂-CH₂-CH₂-;
R6 and R7 stand independently from each other for H, C₁-C₆ alkyl optionally substituted by hydroxyl, C₃-C₆ cycloalkyl optionally substituted by halogen or hydroxy, or halogen;
R8, R9, R, R', R10 and R11 independently from each other stand for H, or C₁-C₆ alkyl optionally substituted by C1-C6 alkoxy; or any two of R8, R9, R, R', R10 and R11 together with the carbon atom to which they are bound may form a 3 - 6 membered saturated carbocyclic ring;
R12 is hydrogen or C₁-C₆ alkyl optionally substituted by halogen or C₁-C₆ alkoxy;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C₁-C₆ alkyl or C₁-C₆ alkoxy;
n is 0 or 1; and
R13 is C₂-C₆ alkenyl optionally substituted by C₁-C₆ alkyl, C₁-C₆ alkoxy or N,N-di-C₁-C₆ alkyl amino; C₂-C₆ alkynyl optionally substituted by C₁-C₆ alkyl or C₁-C₆ alkoxy; or C₂-C₆ alkylenyl oxide optionally substituted by C₁-C₆ alkyl.
2. A composition comprising a cell that expresses a CAR molecule that binds CD19 (a "CAR19-expressing cell"), and a Bruton's tyrosine kinase (BTK) inhibitor,
   wherein the BTK inhibitor comprises a compound of formula (I) or a pharmaceutically acceptable salt thereof; 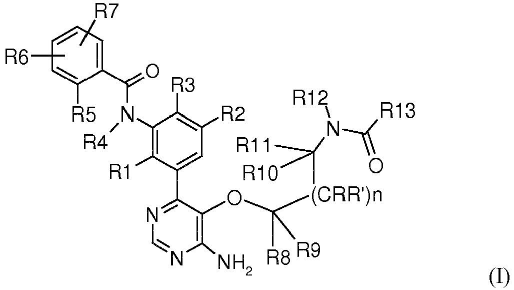 wherein,
   R1 is hydrogen, C₁-C₆ alkyl optionally substituted by hydroxy;
   R2 is hydrogen or halogen;
   R3 is hydrogen or halogen;
   R4 is hydrogen;
   R5 is hydrogen or halogen;
   or R4 and R5 are attached to each other and stand for a bond, -CH₂-, -CH₂-CH₂-, -CH=CH-,-CH=CH-CH₂-; -CH₂-CH=CH-; or -CH₂-CH₂-CH₂-;
   R6 and R7 stand independently from each other for H, C₁-C₆ alkyl optionally substituted by hydroxyl, C₃-C₆ cycloalkyl optionally substituted by halogen or hydroxy, or halogen;
   R8, R9, R, R', R10 and R11 independently from each other stand for H, or C₁-C₆ alkyl optionally substituted by C1-C6 alkoxy; or any two of R8, R9, R, R', R10 and R11 together with the carbon atom to which they are bound may form a 3 - 6 membered saturated carbocyclic ring;
   R12 is hydrogen or C₁-C₆ alkyl optionally substituted by halogen or C₁-C₆ alkoxy;
   or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C₁-C₆ alkyl or C₁-C₆ alkoxy;
   n is 0 or 1; and
      R13 is C₂-C₆ alkenyl optionally substituted by C₁-C₆ alkyl, C₁-C₆ alkoxy or N,N-di-C₁-C₆ alkyl amino; C₂-C₆ alkynyl optionally substituted by C₁-C₆ alkyl or C₁-C₆ alkoxy; or C₂-C₆ alkylenyl oxide optionally substituted by C₁-C₆ alkyl;
   optionally wherein the CAR19-expressing cell and the BTK inhibitor are present in a single dose form, or as two or more dose forms.

### BRIEF DESCRIPTION OF THE DRAWINGS

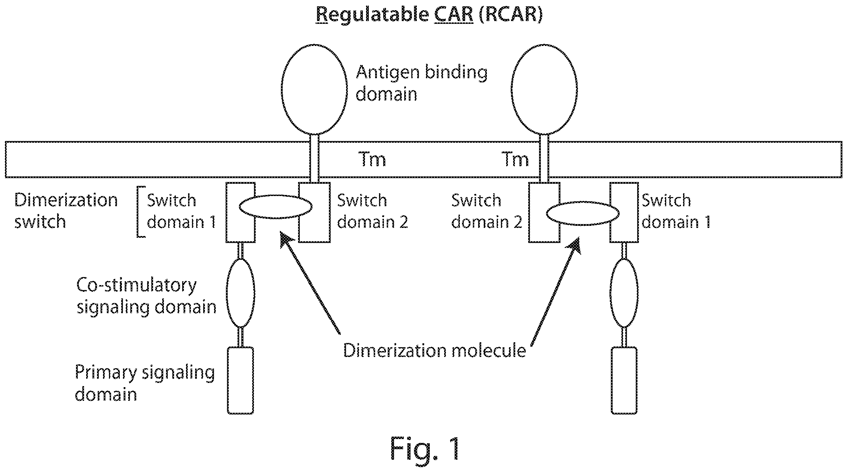

FIG. 1 depicts the structures of two exemplary RCAR configurations. The antigen binding members comprise an antigen binding domain, a transmembrane domain, and a switch domain. The intracellular binding members comprise a switch domain, a co-stimulatory signaling domain and a primary signaling domain. The two configurations demonstrate that the first and second switch domains described herein can be in different orientations with respect to the antigen binding member and the intracellular binding member. Other RCAR configurations are further described herein.
FIG. 2A and 2B are two graphs showing the cell proliferation and cell size of CART19 cells when treated with increasing concentrations of ibrutinib (10 nM, 100 nM, and 1000 nM).
FIG. 3A and 3B shows the proliferation of CART19 cells stimulated with MCL cell lines, while in the presence or absence of ibrutinib. FIG. 3A is a series of histograms showing the proliferation of CART19 cells stimulated with tumor cell lines MOLM14, JEKO-1, and RL, in the presence or absence of increasing concentrations of ibrutinib (10 nM, 100nM, and 1000 nM). Cells were stained by CFSE and analyzed by flow cytometry to determine the percentage of proliferating cells, designated by the bar in each histogram. FIG. 3B is a quantification of representative histograms in FIG. 3A.
FIG. 4A and 4B shows CD107a degranulation of CART19 cells stimulated with MCL cell lines in the presence or absence of ibrutinib. FIG. 4A is a series of flow cytometry profiles showing CD107a degranulation of CART19 cells stimulated with tumor cell lines (MOLM14, JEKO-1, and RL) in the presence or absence of increasing concentrations of ibrutinib (10 nM, 100nM, and 1000 nM). CD107a expression is measured in the y-axis. FIG. 4B is the quantification of the results from FIG. 4A.
FIG. 5 is a series of flow cytometry profiles showing intra-cytoplasmatic IL-2 production by CART19 cells stimulated with tumor cell lines (MOLM14, JEKO-1, and RL) in the presence or absence of increasing concentrations of ibrutinib (10 nM, 100nM, and 1000 nM). The y-axis represents IL-2 expression.
FIG. 6 is a series of flow cytometry profiles showing intra-cytoplasmatic TNF-a production by CART19 cells stimulated with tumor cell lines (MOLM14, JEKO-1, and RL) in the presence or absence of increasing concentrations of ibrutinib (10 nM, 100nM, and 1000 nM). The y-axis represents TNF-a expression.
FIG. 7 is a series of flow cytometry profiles showing intra-cytoplasmatic IFN-g production by CART19 cells stimulated with tumor cell lines (MOLM14, JEKO-1, and RL) in the presence or absence of increasing concentrations of ibrutinib (10 nM, 100nM, and 1000 nM). The y-axis represents IFN-g expression.
FIG. 8 is a series of graphs showing cytokine secretion from CART19 cells stimulated with tumor cell lines (MOLM14, JEKO-1, and RL) in the presence or absence of increasing concentrations of ibrutinib (10 nM, 100nM, and 1000 nM).
FIG. 9A, 9B, 9C, 9D, 9E, and 9F are graphs showing CART19 killing of tumor cells, MOLM14 (FIG. 9A and 9D), JEKO (FIG. 9B and 9E), and RL (FIG. 9C and 9F), alone or in the presence of increasing concentrations of ibrutinib. Untransduced (UTD) or CART19 cells were incubated with tumor cells at varying ratios and the total flux of cells (FIG. 9A, 9B, and 9C) and percentage of dead cells was assessed (9D, 9E, and 9F).
FIG. 10A, 10B, and 10C are graphic respresentations of CART19 killing of tumor cells after 24 hours as measured by flow cytometry to count the total number of cells. Tumor cell lines MOLM14 (FIG. 10A), JEKO (FIG. 10B), and RL (FIG. 10C) were incubated with untransduced (UTD) or CART19 cells alone (ALONE), or in combination with varying concentrations of ibrutinib.
FIG. 11A, 11B, 11C, and 11D are graphic representations of CART19 dose finding in the RL MCL mouse model. Tumor burden was monitored by bioluminescence imaging (BLI) over time (FIG. 11A and 11B). Overall survival was monitored over time (FIG. 11C).
FIG. 12A and 12B are graphic representations of CART19 dose finding in the JEKO-1 MCL mouse model. Tumor size is monitored by bioluminescence imaging (BLI) over time (FIG. 12A) and overall survival was also monitored over time (FIG. 12B).
FIG. 13 is a schematic showing the protocol for administering and assessing CART19 and ibrutinib combination therapy in *in vivo* mouse models.
FIG. 14A and 14B is a graphic representation demonstrating the sensitivity of MCL cell lines RL (FIG. 14A) and JEKO-1 (FIG. 14B) to ibrutinib treatment.
FIG. 15 is a graphic representation demonstrating the effect of ibrutinib treatment in an *in vivo* model of MCL.

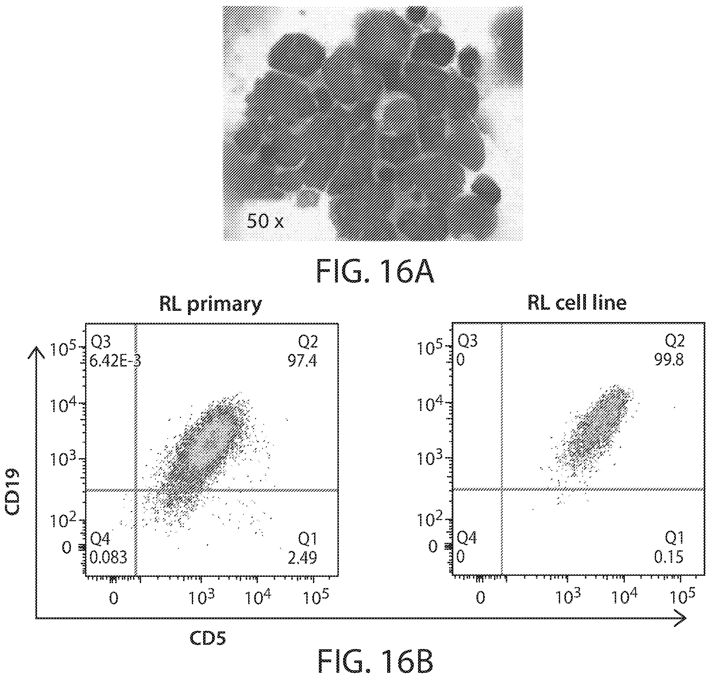

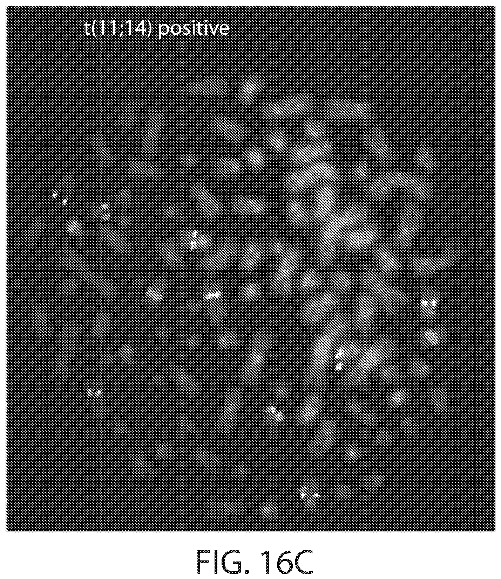

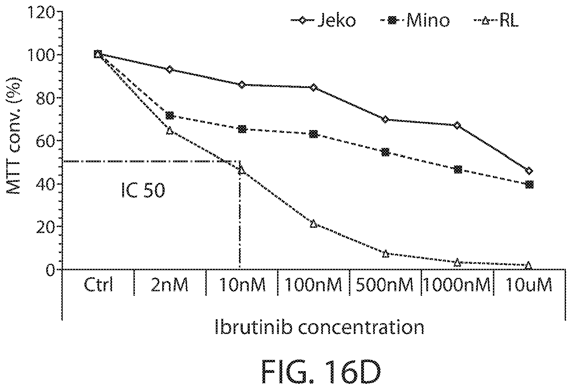

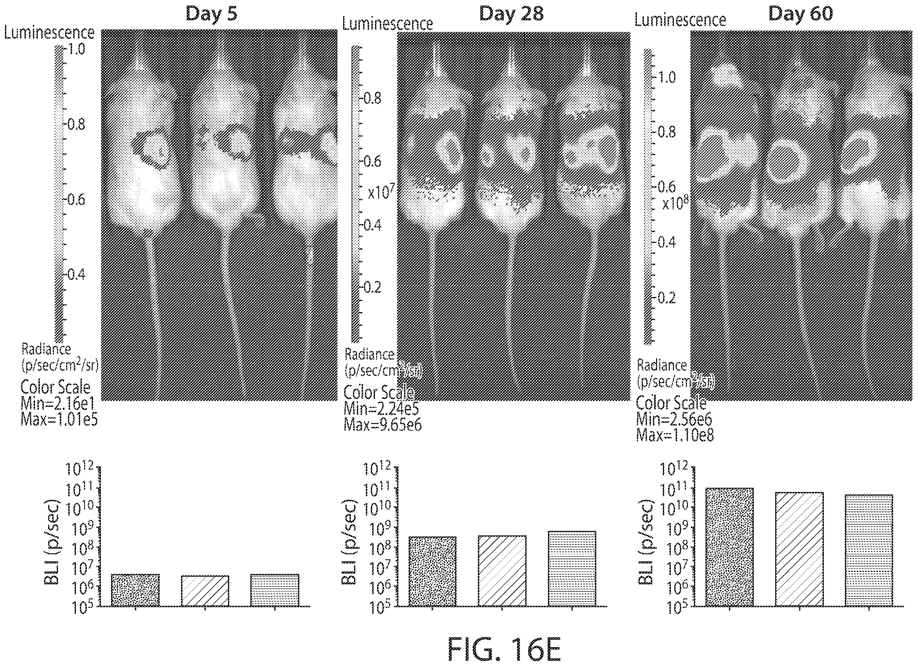

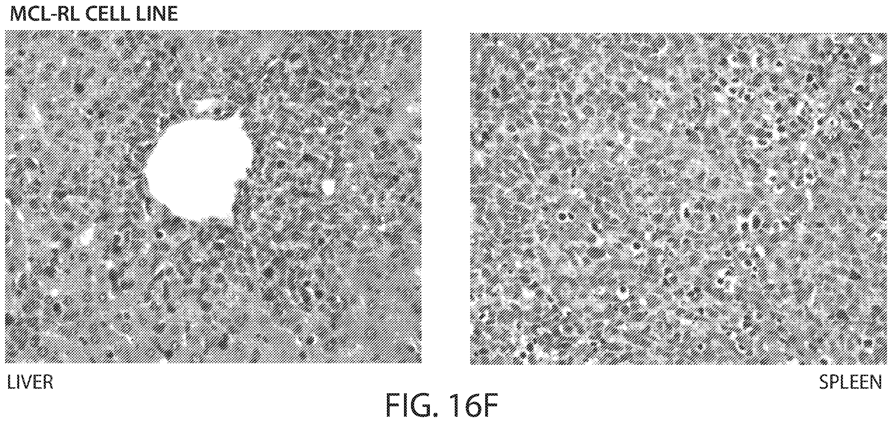

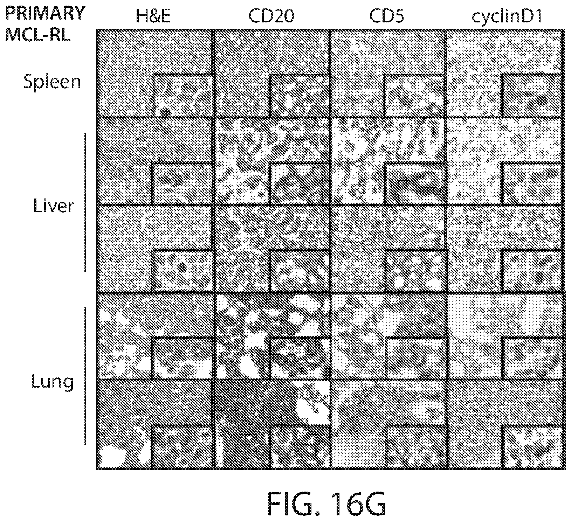

FIG. 16A, 16B, 16C, 16D, 16E, 16F, and 16G characterize cell lines used to model ibrutinib-sensitive and ibrutinib-resistant MCL. FIG. 16A is an image of a RL cell line. FIG. 16B is a set of flow cytometry scatterplots showing the expression of CD19 and CD5 in RL primary and RL cell lines. FIG. 16C is an image showing t(11;14) translocation by fluorescence in-situ hybridization (FISH). FIG. 16D is a graph showing the IC50 (by percentage MTT conversion) of ibrutinib inhibition in different cell lines. FIG. 16E is a set of images and graphs showing engraftment of RL cells in NOD-SCID-gamma chain knockout (NSG) mice and the resulting tumor burden.FIG. 16F is a set of histological images showing localization of MCL cells to various organs in mice. FIG. 16G is a set of histological images of mice that have been injected with MCL-RL cells.

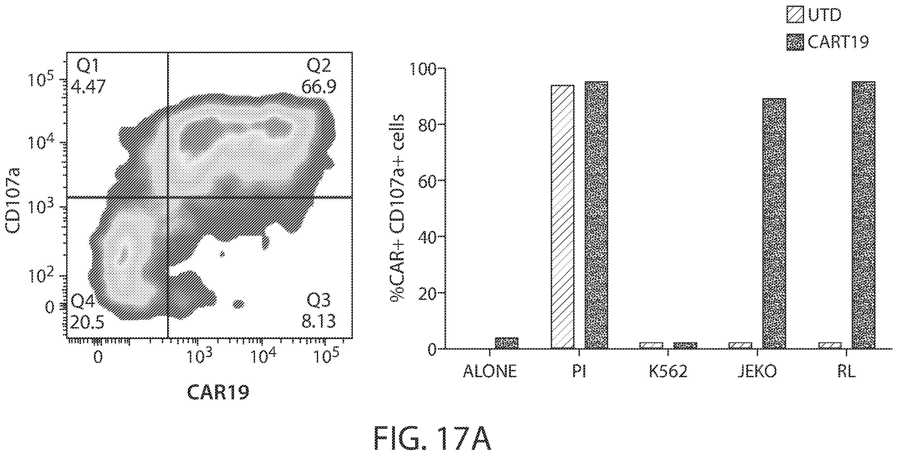

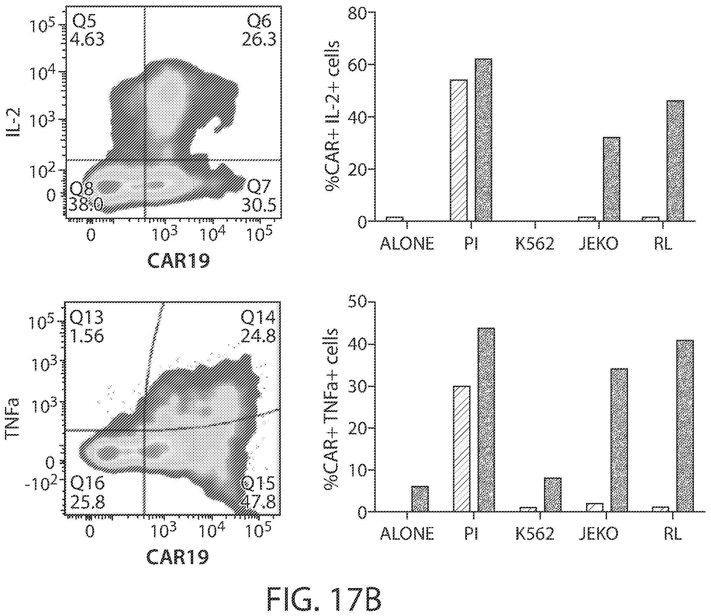

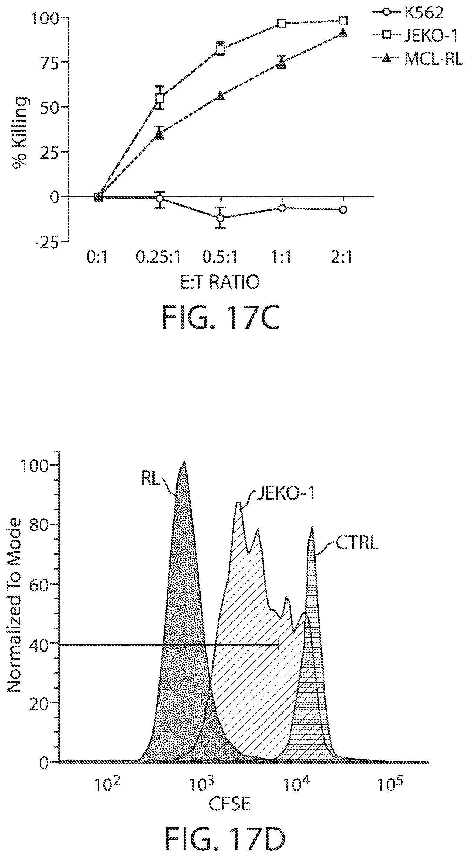

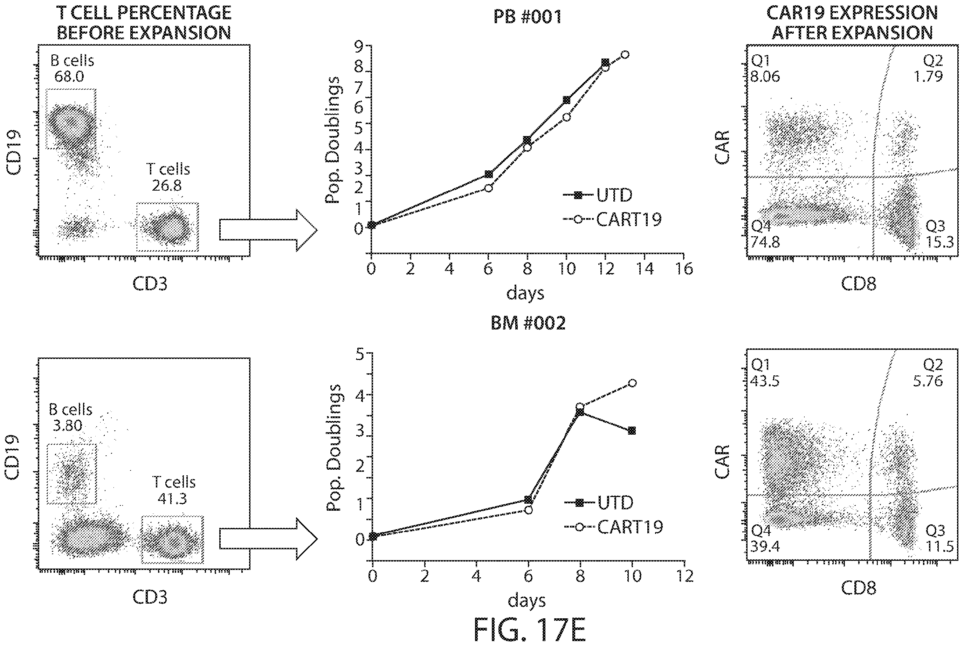

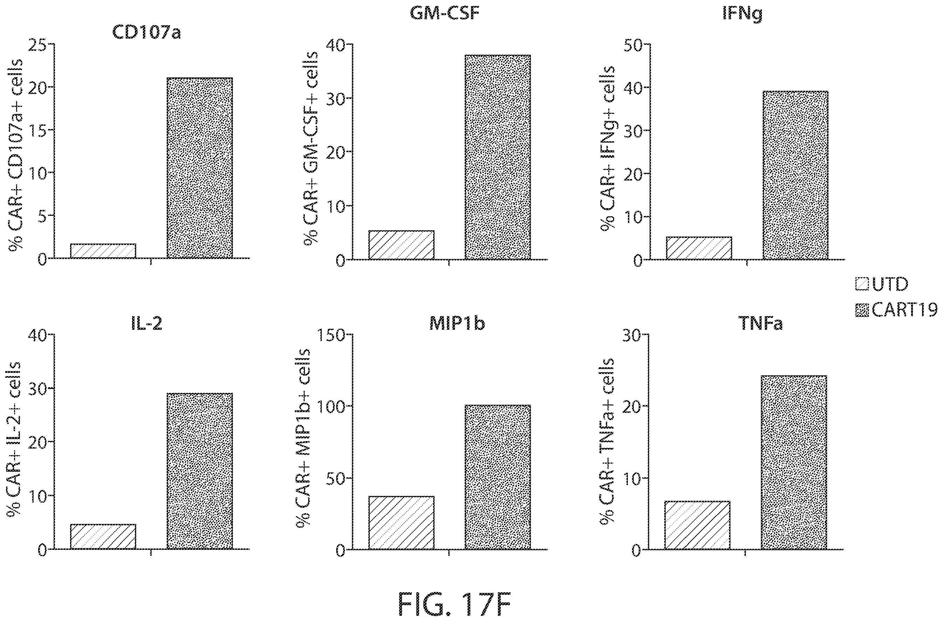

FIG. 17A, 17B, 17C, 17D, 17E, and 17F show CART19 activity against ibrutinib-sensitive and ibrutinib-resistant MCL cells. FIG. 17A is a set of graphs showing the number of CD107a+ CART19 cells when exposed to various MCL cell lines. FIG. 17B is a set of graphs showing the amount of IL-2 and TNF-alpha produced by CART19 cells when exposed to various MCL cell lines. FIG. 17C is a graph showing the percent killing of various MCL cell lines by CART19 cells at various effector:target cell ratios. FIG. 17D is a graph showing the amount of carboxyfluorescein succinimidyl ester (CFSE), a measure of proliferation, in CART19 cells exposed to various MCL cell lines. FIG. 17E is a set of graphs showing the percentage of T cells before and after expansion. FIG. 17F is a set of graphs showing the percentage of untranduced or CAR-19 transduced T cells that express or produce various biomolecules (e.g., cytokines).

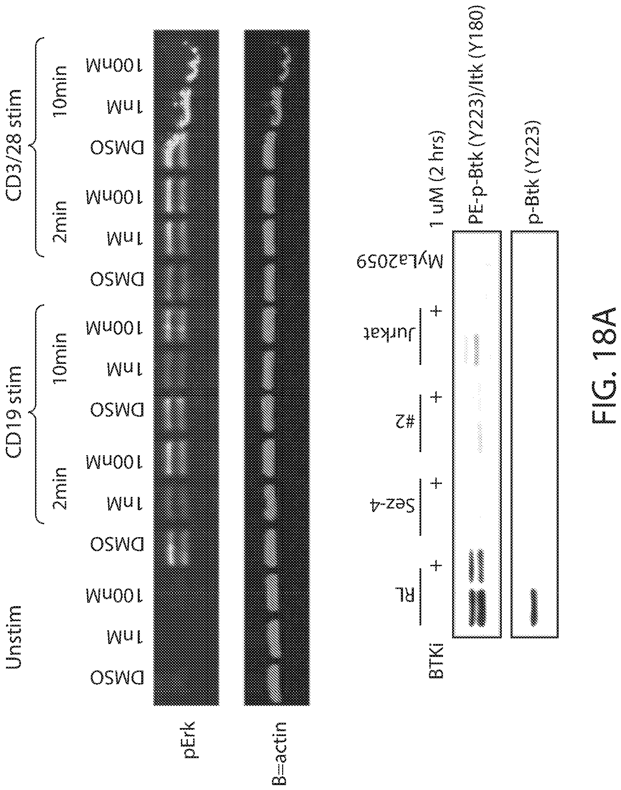

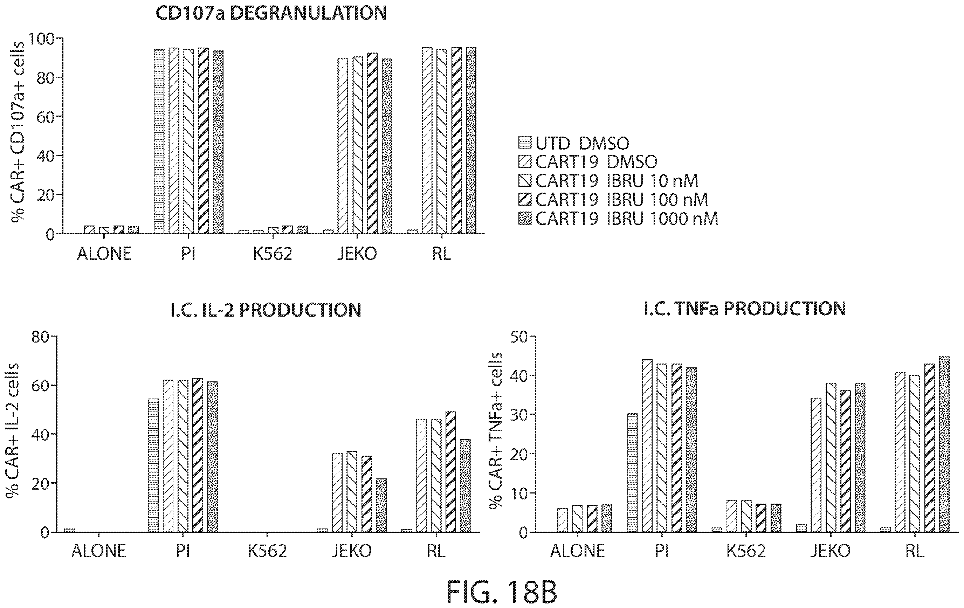

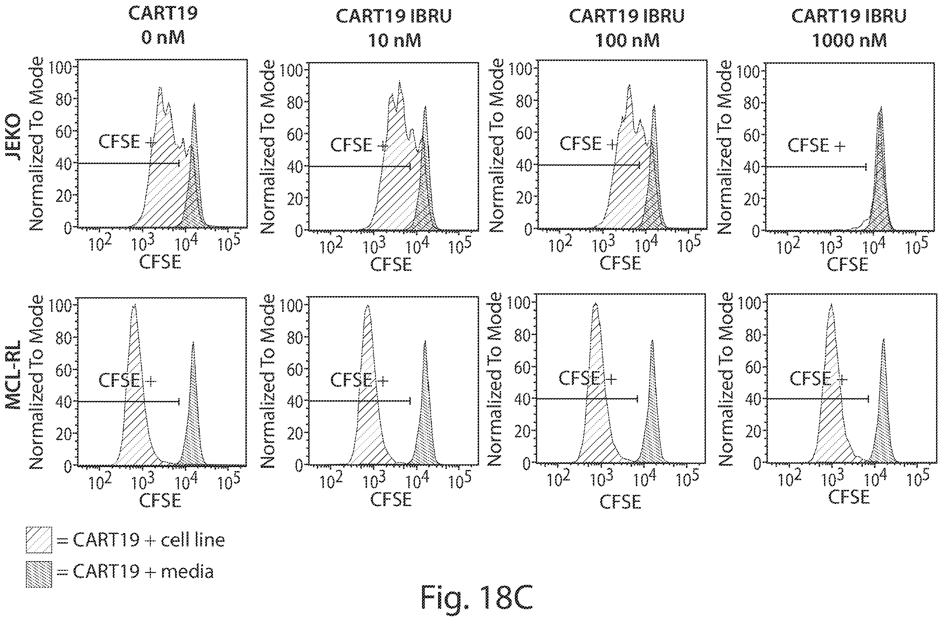

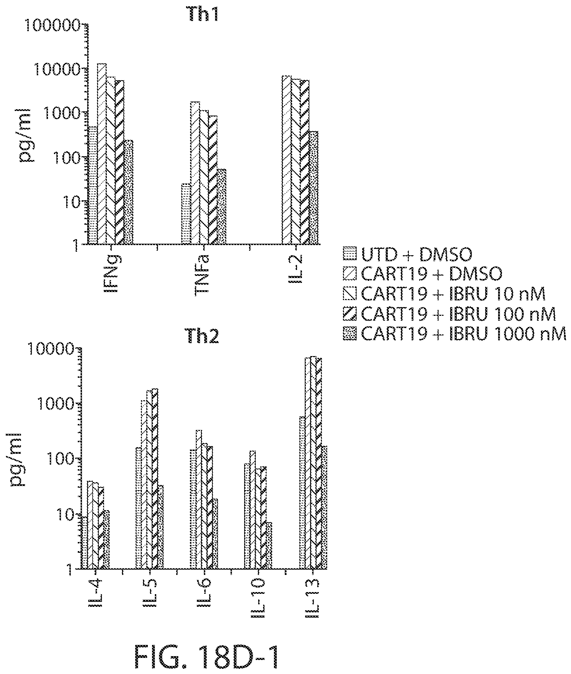

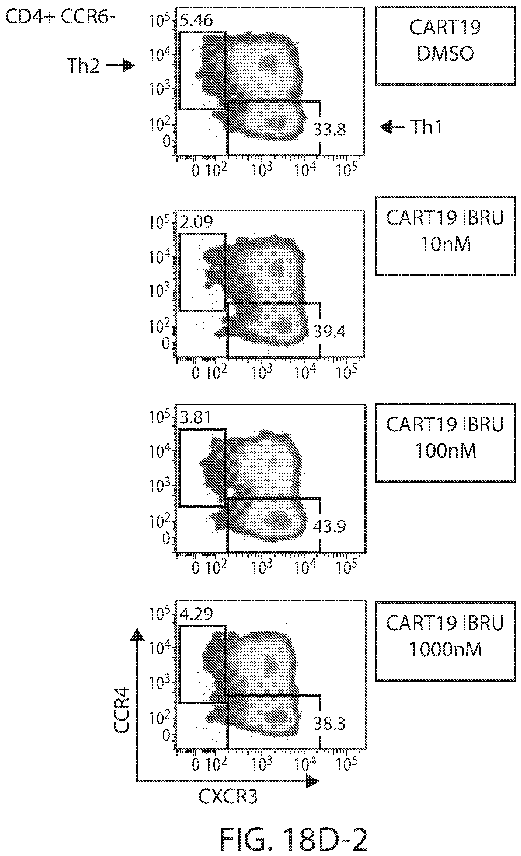

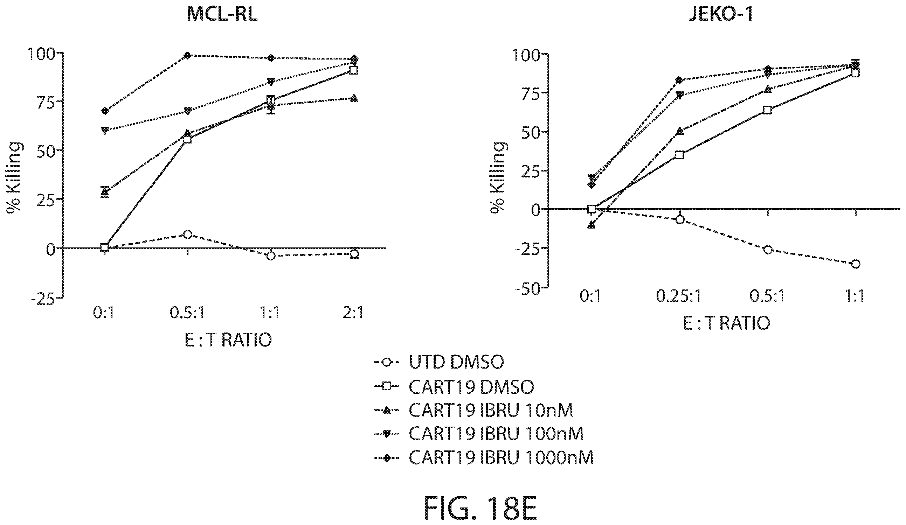

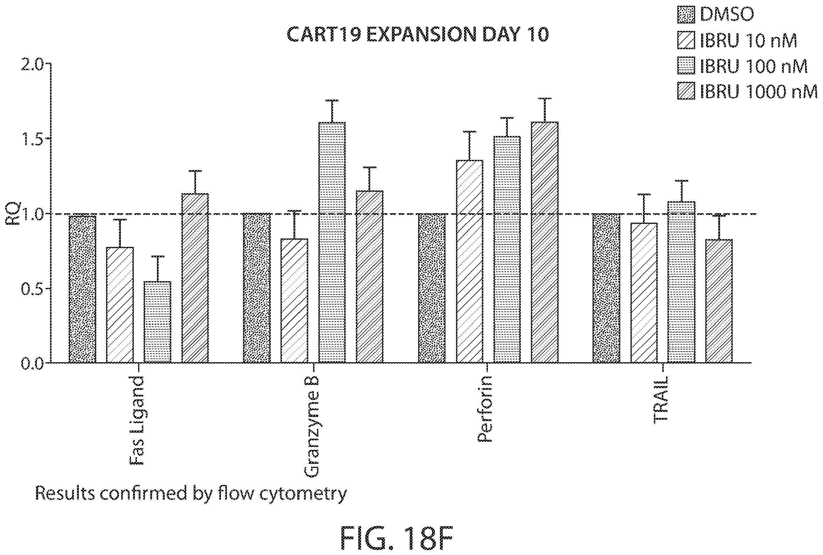

FIG. 18A, 18B, 18C, 18D, 18E, and 18F assay CART19 cells exposed to ibrutinib. FIG. 18A is a set of images showing the activation of interleukin-2-inducible T-cell kinase (ITK) when CART19 cells were stimulated specifically or non-specifically. FIG. 18B is a set of graphs showing CD107a surface expression (a measure of degranulation), IL-2 production, and TNF-alpha production by CART19 cells with various concentrations with ibrutinib. FIG. 18C is a set of histograms showing the amount of CFSE in CART19 cells with various concentrations of ibrutinib and exposed to various MCL cell lines. FIG. 18D is a set of graphs showing the expression or production of various cytokines and biomarkers as indicators of the Th1 or Th2 state of CART19 cells when combined with different concentrations of ibrutinib. FIG. 18E is a set of graphs showing the percentage killing by CART19 cells of various MCL cell lines when combined with different concentrations of ibrutinib. FIG. 18F is a bar graph showing the expression of various markers of intrinsic cytotoxic function of CART19 cells when combined with various concentrations of ibrutinib.
FIG. 19 is a schematic of an in vivo mouse model experimental setup to test the effect of CART19 and/or ibrutinib on MCL-RL-injected mice, with a readout being luminescence (a measure of the number of tumor cells).
FIG. 20 is a schematic of an in vivo mouse model experimental setup to test the effect of CART19 and/or ibrutinib on MCL-RL-injected mice, with a readout being luminescence (a measure of the number of tumor cells).
FIG. 21 is a set of graphs showing the luminescence (a measure of the measure of tumor cell number) in mice treated with ibrutinib at different concentrations and their overall survival after treatment.
FIG. 22 is a set of graphs showing the luminescence (a measure of tumor cell number) in mice treated with ibrutinib or CART19 cells as well as their overall survival after treatment.
FIG. 23 is a graph showing the luminescence (a measure of tumor cell number) in mice after treatment with ibrutinib, untransduced T cells, ibrutinib with untransduced T cells, CART19 cells, and CART19 cells with ibrutinib.
FIG. 24 is a graph showing the luminescence (a measure of tumor cell number) in mice after treatment with ibrutinib alone, CART19 cells alone, or the combination of ibrutinib with CART19 cells.
FIG. 25A is a set of graphs showing the level of Th1 cytokines produced in mice treated with ibrutinib and/or CART19 cells. FIG. 25B is a set of graphs showing the level of Th2 cytokines produced in mice treated with ibrutinib and/or CART19 cells. FIG. 25C is a graph showing the percentage of cells expressing the proliferation marker Ki67 in mice treated with CART19 cells or CART19 cells plus ibrutinib. FIG. 25D is a graph showing the percentage of cells expressing the anti-apoptotic marker BCL-2 in mice treated with CART19 cells or CART19 cells plus ibrutinib.

### DETAILED DESCRIPTION

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some embodiments, the set of polypeptides are in the same polypeptide chain (e.g., comprise a chimeric fusion protein.). In some embodiments, the set of polypeptides are not contiguous with each other, e.g., are in different polypeptide chains. In some embodiments, the set of polypeptides include a dimerization switch that, upon the presence of a dimerization molecule, can couple the polypeptides to one another, e.g., can couple an antigen binding domain to an intracellular signaling domain. In one aspect, the cytoplasmic signaling domain comprises a primary signaling domain (e.g., a primary signaling domain of CD3-zeta). In one aspect, the stimulatory molecule is the zeta chain associated with the T cell receptor complex. In one aspect, the cytoplasmic signaling domain further comprises one or more functional signaling domains derived from at least one costimulatory molecule as defined below. In one aspect, the costimulatory molecule is chosen from the costimulatory molecules described herein, e.g., 4-1BB (i.e., CD137), CD27, CD28 and/or ICOS. In one aspect, the CAR comprises a chimeric fusion protein comprising an extracellular antigen binding domain, a transmembrane domain and an intracellular signaling domain comprising a functional signaling domain derived from a stimulatory molecule. In one aspect, the CAR comprises a chimeric fusion protein comprising an extracellular antigen binding domain, a transmembrane domain and an intracellular signaling domain comprising a functional signaling domain derived from a costimulatory molecule and a functional signaling domain derived from a stimulatory molecule. In one aspect, the CAR comprises a chimeric fusion protein comprising an extracellular antigen binding domain, a transmembrane domain and an intracellular signaling domain comprising two functional signaling domains derived from one or more costimulatory molecule(s) and a functional signaling domain derived from a stimulatory molecule. In one aspect, the CAR comprises a chimeric fusion protein comprising an extracellular antigen binding domain, a transmembrane domain and an intracellular signaling domain comprising at least two functional signaling domains derived from one or more costimulatory molecule(s) and a functional signaling domain derived from a stimulatory molecule. In one aspect the CAR comprises an optional leader sequence at the amino-terminus (N-ter) of the CAR fusion protein. In one aspect, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane.

The term "signaling domain" refers to the functional portion of a protein which acts by transmitting information within the cell to regulate cellular activity via defined signaling pathways by generating second messengers or functioning as effectors by responding to such messengers.

As used herein, the term "CD19" refers to the Cluster of Differentiation 19 protein, which is an antigenic determinant detectable on leukemia precursor cells. The human and murine amino acid and nucleic acid sequences can be found in a public database, such as GenBank, UniProt and Swiss-Prot. For example, the amino acid sequence of human CD19 can be found as UniProt/Swiss-Prot Accession No. P15391 and the nucleotide sequence encoding of the human CD19 can be found at Accession No. NM_001178098. As used herein, "CD19" includes proteins comprising mutations, e.g., point mutations, fragments, insertions, deletions and splice variants of full length wild-type CD19. CD19 is expressed on most B lineage cancers, including, e.g., acute lymphoblastic leukaemia, chronic lymphocyte leukaemia and non-Hodgkin lymphoma. Other cells with express CD19 are provided below in the definition of "disease associated with expression of CD19." It is also an early marker of B cell progenitors. See, e.g., Nicholson et al. Mol. Immun. 34 (16-17): 1157-1165 (1997). In one aspect the antigen-binding portion of the CART recognizes and binds an antigen within the extracellular domain of the CD19 protein. In one aspect, the CD19 protein is expressed on a cancer cell.

As used herein, the term "CD20" refers to an antigenic determinant known to be detectable on B cells. Human CD20 is also called membrane-spanning 4-domains, subfamily A, member 1 (MS4A1). The human and murine amino acid and nucleic acid sequences can be found in a public database, such as GenBank, UniProt and Swiss-Prot. For example, the amino acid sequence of human CD20 can be found at Accession Nos. NP_690605.1 and NP_068769.2, and the nucleotide sequence encoding transcript variants 1 and 3 of the human CD20 can be found at Accession No. NM_152866.2 and NM_021950.3, respectively. In one aspect the antigen-binding portion of the CAR recognizes and binds an antigen within the extracellular domain of the CD20 protein. In one aspect, the CD20 protein is expressed on a cancer cell.

As used herein, the term "CD22," refers to an antigenic determinant known to be detectable on leukemia precursor cells. The human and murine amino acid and nucleic acid sequences can be found in a public database, such as GenBank, UniProt and Swiss-Prot. For example, the amino acid sequences of isoforms 1-5 human CD22 can be found at Accession Nos. NP 001762.2, NP 001172028.1, NP 001172029.1, NP 001172030.1, and NP 001265346.1, respectively, and the nucleotide sequence encoding variants 1-5 of the human CD22 can be found at Accession No. NM 001771.3, NM 001185099.1, NM 001185100.1, NM 001185101.1, and NM 001278417.1, respectively. In one aspect the antigen-binding portion of the CAR recognizes and binds an antigen within the extracellular domain of the CD22 protein. In one aspect, the CD22 protein is expressed on a cancer cell.

As used herein, the term "ROR1" refers to an antigenic determinant known to be detectable on leukemia precursor cells. The human and murine amino acid and nucleic acid sequences can be found in a public database, such as GenBank, UniProt and Swiss-Prot. For example, the amino acid sequences of isoforms 1 and 2 precursors of human ROR1 can be found at Accession Nos. NP_005003.2 and NP_001077061.1, respectively, and the mRNA sequences encoding them can be found at Accession Nos. NM_005012.3 and NM_001083592.1, respectively. In one aspect the antigen-binding portion of the CAR recognizes and binds an antigen within the extracellular domain of the ROR1 protein. In one aspect, the ROR1 protein is expressed on a cancer cell.

The term "antibody," as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be tetramers of immunoglobulin molecules.

The term "antibody fragment" refers to at least one portion of an antibody, that retains the ability to specifically interact with (e.g., by binding, steric hindrance, stabilizing/destabilizing, spatial distribution) an epitope of an antigen. Examples of antibody fragments include, but are not limited to, Fab, Fab', F(ab')₂, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The portion of the CAR of the disclosure comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the disclosure comprises an antibody fragment. In a further aspect, the CAR comprises an antibody fragment that comprises a scFv. The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof.

As used herein, the term "binding domain" or "antibody molecule" refers to a protein, e.g., an immunoglobulin chain or fragment thereof, comprising at least one immunoglobulin variable domain sequence. The term "binding domain" or "antibody molecule" encompasses antibodies and antibody fragments. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The portion of the CAR of the disclosure comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody, or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the disclosure comprises an antibody fragment. In a further aspect, the CAR comprises an antibody fragment that comprises a scFv.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

The term "complementarity determining region" or "CDR," as used herein, refers to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof. Under the Kabat numbering scheme, in some embodiments, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a VH, e.g., a mammalian VH, e.g., a human VH; and amino acid residues 24-34 (LCDR1), 50-56 (LCDR2), and 89-97 (LCDR3) in a VL, e.g., a mammalian VL, e.g., a human VL.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present disclosure includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated synthesized or can be derived from a biological sample, or might be macromolecule besides a polypeptide. Such a biological sample can include, but is not limited to a tissue sample, a tumor sample, a cell or a fluid with other biological components.

The term "anti-cancer effect" refers to a biological effect which can be manifested by various means, including but not limited to, e.g., a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition. An "anti-cancer effect" can also be manifested by the ability of the peptides, polynucleotides, cells and antibodies in prevention of the occurrence of cancer in the first place. The term "anti-tumor effect" refers to a biological effect which can be manifested by various means, including but not limited to, e.g., a decrease in tumor volume, a decrease in the number of tumor cells, a decrease in tumor cell proliferation, or a decrease in tumor cell survival.

The term "autologous" refers to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically

The term "xenogeneic" refers to a graft derived from an animal of a different species.

The term "cancer" refers to a disease characterized by the uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

The phrase "disease associated with expression of CD19" includes, but is not limited to, a disease associated with expression of CD19 or condition associated with cells which express, or at any time expressed, CD19 including, e.g., proliferative diseases such as a cancer or malignancy or a precancerous condition such as a myelodysplasia, a myelodysplastic syndrome or a preleukemia; or a noncancer related indication associated with cells which express CD19. For the avoidance of doubt, a disease associated with expression of CD19 may include a condition associated with cells which do not presently express CD19, e.g., because CD19 expression has been downregulated, e.g., due to treatment with a molecule targeting CD19, e.g., a CD19 CAR, but which at one time expressed CD19. In one aspect, a cancer associated with expression of CD19 is a hematological cancer. In one aspect, the hematolical cancer is a leukemia or a lymphoma. In one aspect, a cancer associated with expression of CD19 includes cancers and malignancies including, but not limited to, e.g., one or more acute leukemias including but not limited to, e.g., B-cell acute Lymphoid Leukemia (BALL), T-cell acute Lymphoid Leukemia (TALL), acute lymphoid leukemia (ALL); one or more chronic leukemias including but not limited to, e.g., chronic myelogenous leukemia (CML), Chronic Lymphoid Leukemia (CLL). Additional cancers or hematologic conditions associated with expression of CD19 comprise, but are not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma (MCL), Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further diseases associated with expression of CD19 expression include, but not limited to, e.g., atypical and/or non-classical cancers, malignancies, precancerous conditions or proliferative diseases associated with expression of CD19. Non-cancer related indications associated with expression of CD19 include, but are not limited to, e.g., autoimmune disease, (e.g., lupus), inflammatory disorders (allergy and asthma) and transplantation. In some embodiments, the tumor antigen-expressing cells express, or at any time expressed, mRNA encoding the tumor antigen. In an embodiment, the tumor antigen -expressing cells produce the tumor antigen protein (e.g., wild-type or mutant), and the tumor antigen protein may be present at normal levels or reduced levels. In an embodiment, the tumor antigen -expressing cells produced detectable levels of a tumor antigen protein at one point, and subsequently produced substantially no detectable tumor antigen protein.

The phrase "disease associated with expression of a B-cell antigen" includes, but is not limited to, a disease associated with expression of one or more of CD19, CD20, CD22 or ROR1, or a condition associated with cells which express, or at any time expressed, one or more of CD19, CD20, CD22 or ROR1, including, e.g., proliferative diseases such as a cancer or malignancy or a precancerous condition such as a myelodysplasia, a myelodysplastic syndrome or a preleukemia; or a noncancer related indication associated with cells which express one or more of CD19, CD20, CD22 or ROR1. For the avoidance of doubt, a disease associated with expression of the B-cell antigen may include a condition associated with cells which do not presently express the B-cell antigen, e.g., because the antigen expression has been downregulated, e.g., due to treatment with a molecule targeting the B-cell antigen, e.g., a B-cell targeting CAR, but which at one time expressed the antigen. The phrase "disease associated with expression of a B-cell antigen" includes a disease associated with expression of CD19, as described herein.

The term "conservative sequence modifications" refers to amino acid modifications that do not significantly affect or alter the binding characteristics of the antibody or antibody fragment containing the amino acid sequence. Such conservative modifications include amino acid substitutions, additions and deletions. Modifications can be introduced into an antibody or antibody fragment of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a CAR of the disclosure can be replaced with other amino acid residues from the same side chain family and the altered CAR can be tested using the functional assays described herein.

The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules.

The term "stimulatory molecule," refers to a molecule expressed by an immune cell (e.g., T cell, NK cell, B cell) that provides the cytoplasmic signaling sequence(s) that regulate activation of the immune cell in a stimulatory way for at least some aspect of the immune cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing cytoplasmic signaling sequence that is of particular use in the disclosure includes, but is not limited to, those derived from CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12. In a specific CAR of the disclosure, the intracellular signaling domain in any one or more CARS of the disclosure comprises an intracellular signaling sequence, e.g., a primary signaling sequence of CD3-zeta. In a specific CAR of the disclosure, the primary signaling sequence of CD3-zeta is the sequence provided as SEQ ID NO: 17 (mutant human CD3 zeta), or the equivalent residues from a non-human species, e.g., mouse, rodent, monkey, ape and the like. In a specific CAR of the disclosure, the primary signaling sequence of CD3-zeta is the sequence as provided in SEQ ID NO: 43 (wild-type human CD3 zeta), or the equivalent residues from a non-human species, e.g., mouse, rodent, monkey, ape and the like.

The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

An "intracellular signaling domain," as the term is used herein, refers to an intracellular portion of a molecule. The intracellular signaling domain generates a signal that promotes an immune effector function of the CAR containing cell, e.g., a CART cell. Examples of immune effector function, e.g., in a CART cell, include cytolytic activity and helper activity, including the secretion of cytokines. In embodiments, the intracellular signaling domain is the portion of a protein which transduces the effector function signal and directs the cell to perform a specialized function. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CART, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, FcR gamma, common FcR gamma (FCER1G), Fc gamma RIIa, FcR beta (Fc Epsilon R1b), CD3 gamma, CD3 delta, CD3 epsilon, CD22, CD79a, CD79b, CD278 ("ICOS"), FcεRI, CD66d, CD32, DAP10, and DAP12.

The term "zeta" or alternatively "zeta chain", "CD3-zeta" or "TCR-zeta" is defined as the protein provided as GenBank Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain, or functional derivatives thereof, that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO:17. In one aspect, the "zeta stimulatory domain" or a "CD3-zeta stimulatory domain" is the sequence provided as SEQ ID NO:43.

The term "costimulatory molecule" refers to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are contribute to an efficient immune response. Costimulatory molecules include, but are not limited to an MHC class I molecule, a TNF receptor protein, an Immunoglobulin-like protein, a cytokine receptor, an integrins, a signalling lymphocytic activation molecule (SLAM protein),an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

A costimulatory intracellular signaling domain refers to the intracellular portion of a costimulatory molecule. The intracellular signaling domain can comprise the entire intracellular portion, or the entire native intracellular signaling domain, of the molecule from which it is derived, or a functional fragment or derivative thereof.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc No. AAA62478.2, or the equivalent residues from a non-human species, e.g., mouse, rodent, monkey, ape and the like. In one aspect, the "4-1BB costimulatory domain" is the sequence provided as SEQ ID NO:16 or the equivalent residues from a non-human species, e.g., mouse, rodent, monkey, ape and the like.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloic-derived phagocytes.

"Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and co-stimulation are examples of immune effector function or response.

The term "effector function" refers to a specialized function of a cell. Effector function of a T cell, for example, may be cytolytic activity or helper activity including the secretion of cytokines.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "effective amount" or "therapeutically effective amount" are used interchangeably herein, and refer to an amount of a compound, formulation, material, or composition, as described herein effective to achieve a particular biological result. In one non-limiting instance, the term "a therapeutically effective amount" refers to the amount of the compound described herein that, when administered to a subject, is effective to (1) at least partially alleviate, inhibit, preventand/or ameliorate a condition, or a disorder or a disease (i) mediated by BTK, or (ii) associated with BTK activity, or (iii) characterized by activity (normal or abnormal) of BTK; or (2) reducing or inhibiting the activity of BTK; or (3) reducing or inhibiting the expression of BTK. In another non-limiting instance, the term "a therapeutically effective amount" refers to the amount of the compound described herein, that when administered to a cell, or a tissue, or a non-cellular biological material, or a medium, is effective to at least partially reducing or inhibiting the activity of BTK; or reducing or inhibiting the expression of BTK partially or completely.

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, e.g., the LENTIVECTOR® gene delivery technology from Oxford BioMedica, the LENTIMAX™ vector system from Lentigen and the like. Nonclinical types of lentiviral vectors are also available and would be known to one skilled in the art.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody or antibody fragment, where the whole molecule is of human origin or consists of an amino acid sequence identical to a human form of the antibody or immunoglobulin.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acid (DN The term "nucleic acid" includes a gene, cDNA, or an mRNA. In one embodiment, the nucleic acid molecule is synthetic (e.g., chemically synthesized) or recombinant. A) or ribonucleic acids (RNA) and polymers thereof in either single- or double-stranded form. Unless specifically limited, the term encompasses nucleic acids containing analogues or derivatives of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

The term "flexible polypeptide linker" or "linker" as used in the context of a scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser)n, where n is a positive integer equal to or greater than 1. For example, n=1, n=2, n=3, n=4, n=5, n=6, n=7, n=8, n=9 and n=10 (SEQ ID NO:105). In one embodiment, the flexible polypeptide linkers include, but are not limited to, (Gly4 Ser)4 (SEQ ID NO:106) or (Gly4 Ser)3 (SEQ ID NO:107). In another embodiment, the linkers include multiple repeats of (Gly2Ser), (GlySer) or (Gly3Ser) (SEQ ID NO: 108). In one embodiment, the linker is GSTSGSGKPGSGEGSTKG (SEQ ID NO: 142) Also included within the scope of the disclosure are linkers described in WO2012/138475.

As used herein, a 5' cap (also termed an RNA cap, an RNA 7-methylguanosine cap or an RNA m⁷G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is important for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, e.g., mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In some instances of a construct for transient expression, the polyA is between 50 and 5000 (SEQ ID NO: 109), preferably greater than 64, e.g., greater than 100, e.g., greater than 300 or 400. Poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

As used herein, the terms "treat", "treatment" and "treating" refer to the reduction or amelioration of the progression, severity and/or duration of a proliferative disorder, or the amelioration of one or more symptoms (e.g., one or more discernible symptoms) of a proliferative disorder resulting from the administration of one or more therapies (e.g., one or more therapeutic agents such as a CAR described herein). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" - refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human).

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

The term "therapeutic" as used herein means a treatment. A therapeutic effect is obtained by reduction, suppression, remission, or eradication of a disease state.

The term "prophylaxis" as used herein means the prevention of or protective treatment for a disease or disease state.

In the context of the present disclosure, "tumor antigen" or "hyperproliferative disorder antigen" or "antigen associated with a hyperproliferative disorder" refers to antigens that are common to specific hyperproliferative disorders. In certain aspects, the hyperproliferative disorder antigens of the present disclosure are derived from, cancers including but not limited to primary or metastatic melanoma, thymoma, lymphoma, sarcoma, lung cancer, liver cancer, non-Hodgkin lymphoma, Hodgkin lymphoma, leukemias, uterine cancer, cervical cancer, bladder cancer, kidney cancer and adenocarcinomas such as breast cancer, prostate cancer, ovarian cancer, pancreatic cancer, and the like.

The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The term "specifically binds," refers to an antibody, or a ligand, which recognizes and binds with a binding partner protein present in a sample, but which antibody or ligand does not substantially recognize or bind other molecules in the sample.

"Regulatable chimeric antigen receptor (RCAR),"as that term is used herein, refers to a set of polypeptides, typically two in the simplest embodiments, which when in a RCARX cell, provides the RCARX cell with specificity for a target cell, typically a cancer cell, and with regulatable intracellular signal generation or proliferation, which can optimize an immune effector property of the RCARX cell. An RCARX cell relies at least in part, on an antigen binding domain to provide specificity to a target cell that comprises the antigen bound by the antigen binding domain. In an embodiment, an RCAR includes a dimerization switch that, upon the presence of a dimerization molecule, can couple an intracellular signaling domain to the antigen binding domain.

"Membrane anchor" or "membrane tethering domain", as that term is used herein, refers to a polypeptide or moiety, e.g., a myristoyl group, sufficient to anchor an extracellular or intracellular domain to the plasma membrane.

"Switch domain," as that term is used herein, e.g., when referring to an RCAR, refers to an entity, typically a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the first and second switch domains are different from one another, e.g., they are polypeptides having different primary amino acid sequences, and are referred to collectively as a heterodimerization switch. In embodiments, the switch is intracellular. In embodiments, the switch is extracellular. In embodiments, the switch domain is a polypeptide-based entity, e.g., FKBP or FRB-based, and the dimerization molecule is small molecule, e.g., a rapalogue. In embodiments, the switch domain is a polypeptide-based entity, e.g., an scFv that binds a myc peptide, and the dimerization molecule is a polypeptide, a fragment thereof, or a multimer of a polypeptide, e.g., a myc ligand or multimers of a myc ligand that bind to one or more myc scFvs. In embodiments, the switch domain is a polypeptide-based entity, e.g., myc receptor, and the dimerization molecule is an antibody or fragments thereof, e.g., myc antibody.

"Dimerization molecule," as that term is used herein, e.g., when referring to an RCAR, refers to a molecule that promotes the association of a first switch domain with a second switch domain. In embodiments, the dimerization molecule does not naturally occur in the subject, or does not occur in concentrations that would result in significant dimerization. In embodiments, the dimerization molecule is a small molecule, e.g., rapamycin or a rapalogue, e.g, RAD001.

The term "bioequivalent" refers to an amount of an agent other than the reference compound (e.g., RAD001), required to produce an effect equivalent to the effect produced by the reference dose or reference amount of the reference compound (e.g., RAD001). In an embodiment the effect is the level of mTOR inhibition, e.g., as measured by P70 S6 kinase inhibition, e.g., as evaluated in an *in vivo* or *in vitro* assay, e.g., as measured by an assay described herein, e.g., the Boulay assay, or measurement of phosphorylated S6 levels by western blot. In an embodiment, the effect is alteration of the ratio of PD-1 positive/PD-1 negative T cells, as measured by cell sorting. In an embodiment a bioequivalent amount or dose of an mTOR inhibitor is the amount or dose that achieves the same level of P70 S6 kinase inhibition as does the reference dose or reference amount of a reference compound. In an embodiment, a bioequivalent amount or dose of an mTOR inhibitor is the amount or dose that achieves the same level of alteration in the ratio of PD-1 positive/PD-1 negative T cells as does the reference dose or reference amount of a reference compound.

The term "low, immune enhancing, dose" when used in conjuction with an mTOR inhibitor, e.g., an allosteric mTOR inhibitor, e.g., RAD001 or rapamycin, or a catalytic mTOR inhibitor, refers to a dose of mTOR inhibitor that partially, but not fully, inhibits mTOR activity, e.g., as measured by the inhibition of P70 S6 kinase activity. Methods for evaluating mTOR activity, e.g., by inhibition of P70 S6 kinase, are discussed herein. The dose is insufficient to result in complete immune suppression but is sufficient to enhance the immune response. In an embodiment, the low, immune enhancing, dose of mTOR inhibitor results in a decrease in the number of PD-1 positive T cells and/or an increase in the number of PD-1 negative T cells, or an increase in the ratio of PD-1 negative T cells/PD-1 positive T cells. In an embodiment, the low, immune enhancing, dose of mTOR inhibitor results in an increase in the number of naive T cells. In an embodiment, the low, immune enhancing, dose of mTOR inhibitor results in one or more of the following:
an increase in the expression of one or more of the following markers: CD62L^{high}, CD127^{hlgh}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{hgh}, increased CD127^{high}, increased CD27⁺, decreased KLRG1, and increased BCL2;
wherein any of the changes described above occurs, e.g., at least transiently, e.g., as compared to a non-treated subject.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become refractory during a treatment.

A "complete responder" as used herein refers to a subject having a disease, e.g., a cancer, who exhibits a complete response, e.g., a complete remission, to a treatment. A complete response may be identified, e.g., using the Cheson criteria as described herein.

A "partial responder" as used herein refers to a subject having a disease, e.g., a cancer, who exhibits a partial response, e.g., a partial remission, to a treatment. A partial response may be identified, e.g., using the Cheson criteria.

A "non-responder" as used herein refers to a subject having a disease, e.g., a cancer, who does not exhibit a response to a treatment, e.g., the patient has stable disease or progressive disease. A non-responder may be identified, e.g., using the Cheson criteria as described herein.

The term "relapse" as used herein refers to reappearance of a disease (e.g., cancer) after an initial period of responsiveness (e.g., complete response or partial response). The initial period of responsiveness may involve the level of cancer cells falling below a certain threshold, e.g., below 20%, 1%, 10%, 5%, 4%, 3%, 2%, or 1%. The reappearance may involve the level of cancer cells rising above a certain threshold, e.g., above 20%, 1%, 10%, 5%, 4%, 3%, 2%, or 1%. Relapse may be identified, e.g., using the Cheson criteria as described herein. For example, e.g., in the context of B-ALL, the reappearance may involve, e.g., a reappearance of blasts in the blood, bone marrow (> 5%), or any extramedullary site, after a complete response. A complete response, in this context, may involve < 5% BM blast. More generally, in an embodiment, a response (e.g., complete response or partial response) can involve the absence of detectable MRD (minimal residual disease). In an embodiment, the initial period of responsiveness lasts at least 1, 2, 3, 4, 5, or 6 days; at least 1, 2, 3, or 4 weeks; at least 1, 2, 3, 4, 6, 8, 10, or 12 months; or at least 1, 2, 3, 4, or 5 years.

As used herein, the term "C₁-C₆ alkyl" refers to a fully saturated branched or unbranched hydrocarbon moiety having up to 6 carbon atoms. Unless otherwise provided, it refers to hydrocarbon moieties having 1 to 6 carbon atoms, 1 to 4 carbon atoms or 1 to 2 carbon atoms. Representative examples of alkyl include, but are not limited to, methyl, ethyl, *n*-propyl, *iso*-propyl, *n*-butyl, *sec*-butyl, *iso*butyl, *tert*-butyl*, n*-pentyl, isopentyl, neopentyl, *n*-hexyl and the like.

As used herein, the term "C₂-C₆ alkenyl" refers to an unsaturated branched or unbranched hydrocarbon moiety having 2 to 6 carbon atoms. Unless otherwise provided, C2-C6 alkenyl refers to moieties having 2 to 6 carbon atoms, 2 to 5 carbon atoms, or 2 to 4 carbon atoms. Representative examples of alkenyl include, but are not limited to, ethenyl, n-propenyl, *iso*-propenyl, *n*-butenyl, *sec*-butenyl, *iso*butenyl, *tert*-butenyl, *n*-pentenyl, isopentenyl, neopentenyl, *n*-hexenyl, and the like.

As used herein, the term "C₂-C₆ alkynyl" refers to an unsaturated branched or unbranched hydrocarbon moiety having 2 to 6 carbon atoms, containing at least one triple bond, and which is attached to the rest of the molecule by a single bond. The term "C₂₋₄alkynyl" is to be construed accordingly. Examples of C₂₋₆alkynyl include, but are not limited to, ethynyl, prop-1-ynyl, but-1-ynyl, pent-1-ynyl and penta-1,4-diynyl and the like.

As used herein, the term "C₁-C₆ alkoxy" refers to alkyl-O-, wherein alkyl is defined herein above. Representative examples of alkoxy include, but are not limited to, methoxy, ethoxy, propoxy, 2-propoxy, butoxy, tert-butoxy, pentyloxy, hexyloxy, cyclopropyloxy-, cyclohexyloxy- and the like. Typically, alkoxy groups have about 1 to 6 carbon atoms, 1 to 4 carbon atoms or 1 to 2 carbon atoms.

As used herein, the term "di C₁₋₆alkylamino" refers to a moiety of the formula -N(Rₐ)-Rₐ where each Rₐ is a C₁₋₆alkyl, which may be the same or different, as defined above.

As used herein, the term "C₃-C₆ cycloalkyl" refers to saturated monocyclic hydrocarbon groups of 3-6 carbon atoms. Cycloalkyl may also be referred to as a carbocyclic ring and vice versa additionally referring to the number of carbon atoms present. Unless otherwise provided, cycloalkyl refers to cyclic hydrocarbon groups having between 3 and 6 ring carbon atoms or between 3 and 4 ring carbon atoms. Exemplary monocyclic hydrocarbon groups include, but are not limited to, cyclopropyl, cyclobutyl, cyclopentyl, and cyclohexyl.

As used herein "C₂-C₆ alkylenyl oxide" refers to a branched or unbranched hydrocarbon moiety comprising an epoxy group and having from 2 to 6 carbon atoms. Representative examples include ethylenyl oxide, propylenyl oxide, butylenyl 1,2-oxide, butylenyl 2,3-oxide, butylenyl 3,4-oxide, pentylenyl oxide, hexylenyl oxide, and the like.

As used herein, the term "azacyclic" ring refers to a saturated or unsaturated monocyclic hydrocarbon group of 3 - 7 carbon atoms as defined for "cycloalkyl", wherein one carbon atom is replaced by a nitrogen atom. It may be also referred to "azacycloalkyl" or "aza hydrocarbon". Unless otherwise provided, azacycloalkyl refers to cyclic aza-hydrocarbon groups having between 2 and 6 ring carbon atoms and one nitrogen atom, between 2 and 4 ring carbon atoms and one nitrogen atom, or between 2 and 3 ring carbon atoms and one nitrogen atom. Exemplary azacyclic groups include, but are not limited to, aziridinyl, azetidinly, pyrrolidinyl, piperidinyl, azepanyl, dihydroazepinyl and the like.

As used herein, the term "halogen" or "halo" refers to fluoro, chloro, bromo, and iodo.

As used herein, the terms "salt" or "salts" refers to an acid addition or base addition salt of a compound of the disclosure. "Salts" include in particular "pharmaceutically acceptable salts". The term "pharmaceutically acceptable salts" refers to salts that retain the biological effectiveness and properties of the compounds of this disclosure and, which typically are not biologically or otherwise undesirable. In many cases, the compounds of the present disclosure are capable of forming acid and/or base salts by virtue of the presence of amino and/or carboxyl groups or groups similar thereto.

Ranges: throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

### Description

Provided herein are compositions of matter and methods of use for the treatment of a disease such as cancer (e.g., hematological cancers or other B cell malignancies) using immune effector cells (e.g., T cells or NK cells) that express a chimeric antigen receptor (CAR) (e.g., a CAR that targets a B-cell marker, such as CD19). The methods include, inter alia, administering immune effector cells (e.g., T cells or NK cells) expressing a B cell targeting CAR described herein in combination with another agent such as a BTK inhibitor, e.g., a BTK inhibitor described herein, e.g., a compound of formula (I).

The present disclosure provides, at least in part, experiments supporting the high efficacy of a combination of a CAR therapy (e.g., a B-cell targeting CAR therapy) and a BTK inhibitor such as a compound of formula (I). The combination of BTK inhibitor such as a compound of formula (I), with a CAR therapy can increase efficacy of the combination therapy relative to a monotherapy of the BTK inhibitor, or a dose of CAR-expressing cells, or both. These beneficial effects can, for example, allow for a lower dose of the BTK inhibitor or the CAR-expressing cells, or both, while maintaining efficacy. The results herein are applicable to a wide range of cancers, e.g., hematological cancers and other B cell malignancies. For example, BTK is elevated in most lymphomas. An immune effector cell (e.g., T cell or NK cell) that expresses CAR19 targets cancers with CD19 surface expression, which is expressed in most B cell malignancies. Alternatively or in combination with CAR19, any other B-cell targeting CAR (e.g., a CAR targeting one or more of: CD20, CD22, or ROR1) can be used in the combination therapies described herein. Therefore, the combination of a CAR therapy (e.g., one or more of a CD19 CAR, CD20 CAR, CD22 CAR or ROR1 CAR therapy) with a BTK inhibitor (e.g., a compound of formula (I)) is suitable for treating a wide range of cancers involving overproliferation of B cells, including lymphomas (e.g., Hodgkin lymphoma), MCL, CLL, DLBCL, and multiple myeloma.

According to the present disclosure, BTK inhibitors can reduce tumor masses and mobilize neoplastic B cells in the peripheral blood (see e.g., Example 42 herein). Without wishing to be bound by theory, certain lymphomas, such as MCL, are characterized by masses of cancerous cells in proliferation centers in lymph nodes. CAR-expressing immune effector cells sometimes have difficulty penetrating these densely packed masses. Thus, a BTK inhibitor can reduce tumor masses and mobilize neoplastic B cells in the peripheral blood, making the lymphoma cells more vulnerable to the CAR-expressing cells.

Alternatively or in combination, BTK inhibitors, such as compounds of formula (I), can also affect the CAR-expressing cells. The present invention demonstrates that ibrutinib (a BTK inhibitor) treatment increases the level of circulating CART19 cells (see e.g., data shown in Example 42). Without wishing to be bound by theory, the increase in the level of circulating CART19 cells may be a result of, for example, increased proliferation, alteration of T cell phenotype, or other factors. For example, ibrutinib can inhibit ITK, a kinase with homology to BTK. ITK is expressed in T cells, and its inhibition may alter the T cell phenotype. Treatment with a BTK inhibitor, such as ibrutinib, can alter the T cell phenotype from a Th2 phenotype to a Th1 phenotype, and thus increase the T cell proliferative capacity. Pre-treatment, or coadministration, to a subject, of a BTK inhibitor may increase the T cell proliferative capacity in the subject, thus increasing the level of circulating CAR-expressing cells. In addition, a subject pre-treated with a BTK inhibitor can have a T cell population with a higher proliferative capacity in their apheresis for CAR manufacturing.

In one aspect, the disclosure provides a number of chimeric antigen receptors (CAR) comprising an antibody or antibody fragment engineered for specific binding to a B-cell antigen (e.g., chosen from one or more of CD19, CD20, CD22 or ROR1 protein). In one aspect, the disclosure provides a cell (e.g., T cell) engineered to express a CAR, wherein the CAR T cell ("CART") exhibits an anticancer property. In one aspect a cell is transformed with the CAR and the CAR is expressed on the cell surface. In some instances, the cell (e.g., T cell) is transduced with a viral vector encoding a CAR. In some instances, the viral vector is a retroviral vector. In some instances, the viral vector is a lentiviral vector. In some such instances, the cell may stably express the CAR. In another instance, the cell (e.g., T cell) is transfected with a nucleic acid, e.g., mRNA, cDNA, DNA, encoding a CAR. In some such instances, the cell may transiently express the CAR.

In one aspect, the anti-CD 19 protein binding portion of the CAR is a scFv antibody fragment. In one aspect such antibody fragments are functional in that they retain the equivalent binding affinity, e.g., they bind the same antigen with comparable affinity, as the IgG antibody from which it is derived. In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan. In one aspect, the anti-CD 19 antigen binding domain of the CAR is a scFv antibody fragment that is humanized compared to the murine sequence of the scFv from which it is derived. In one aspect, the parental murine scFv sequence is the CAR19 construct provided in PCT publication WO2012/079000 and provided herein as SEQ ID NO:59. In one embodiment, the anti-CD19 binding domain is a scFv described in WO2012/079000 and provided in SEQ ID NO:59.

In some aspects, the antibodies of the disclosure are incorporated into a chimeric antigen receptor (CAR). In one aspect, the CAR comprises the polypeptide sequence provided as SEQ ID NO: 12 in PCT publication WO2012/079000, and provided herein as SEQ ID NO: 58, wherein the scFv domain is substituted by one or more sequences selected from SEQ ID NOS: 1-12. In one aspect, the scFv domains of SEQ ID NOS:1-12 are humanized variants of the scFv domain of SEQ ID NO:59, which is an scFv fragment of murine origin that specifically binds to human CD19. Humanization of this mouse scFv may be desired for the clinical setting, where the mouse-specific residues may induce a human-anti-mouse antigen (HAMA) response in patients who receive CART19 treatment, e.g., treatment with T cells transduced with the CAR19 construct.

In one aspect, the anti-CD19 binding domain, e.g., humanized scFv, portion of a CAR of the disclosure is encoded by a transgene whose sequence has been codon optimized for expression in a mammalian cell. In one aspect, entire CAR construct of the disclosure is encoded by a transgene whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

In one aspect, the humanized CAR19 comprises the scFv portion provided in SEQ ID NO:1. In one aspect, the humanized CAR19 comprises the scFv portion provided in SEQ ID NO:2. In one aspect, the humanized CAR19 comprises the scFv portion provided in SEQ ID NO:3. In one aspect, the humanized CAR19 comprises the scFv portion provided in SEQ ID NO:4. In one aspect, the humanized CAR19 comprises the scFv portion provided in SEQ ID NO:5. In one aspect, the humanized CAR19 comprises the scFv portion provided in SEQ ID NO:6. In one aspect, the humanized CAR19 comprises the scFv portion provided in SEQ ID NO:7. In one aspect, the humanized CAR19 comprises the scFv portion provided in SEQ ID NO:8. In one aspect, the humanized CAR19 comprises the scFv portion provided in SEQ ID NO:9. In one aspect, the humanized CAR19 comprises the scFv portion provided in SEQ ID NO:10. In one aspect, the humanized CAR19 comprises the scFv portion provided in SEQ ID NO:11. In one aspect, the humanized CAR19 comprises the scFv portion provided in SEQ ID NO:12.

In one aspect, the CARs of the disclosure combine an antigen binding domain of a specific antibody with an intracellular signaling molecule. For example, in some aspects, the intracellular signaling molecule includes, but is not limited to, CD3-zeta chain, 4-1BB and CD28 signaling modules and combinations thereof. In one aspect, the CD19 CAR comprises a CAR selected from the sequence provided in one or more of SEQ ID NOS: 31 - 42. In one aspect, the CD19 CAR comprises the sequence provided in SEQ ID NO:31. In one aspect, the CD19 CAR comprises the sequence provided in SEQ ID NO:32. In one aspect, the CD19 CAR comprises the sequence provided in SEQ ID NO:33. In one aspect, the CD19 CAR comprises the sequence provided in SEQ ID NO:34. In one aspect, the CD19 CAR comprises the sequence provided in SEQ ID NO:35. In one aspect, the CD19 CAR comprises the sequence provided in SEQ ID NO:36. In one aspect, the CD19 CAR comprises the sequence provided in SEQ ID NO:37. In one aspect, the CD19 CAR comprises the sequence provided in SEQ ID NO:38. In one aspect, the CD19 CAR comprises the sequence provided in SEQ ID NO:39. In one aspect, the CD19 CAR comprises the sequence provided in SEQ ID NO:40. In one aspect, the CD19 CAR comprises the sequence provided in SEQ ID NO:41. In one aspect, the CD19 CAR comprises the sequence provided in SEQ ID NO:42.

Furthermore, the present invention provides CD19 CAR compositions and their use in medicaments or methods for treating, among other diseases, cancer or any malignancy or autoimmune diseases involving cells or tissues which express CD19.

In one aspect, the CAR of the disclosure can be used to eradicate CD19-expressing normal cells, thereby applicable for use as a cellular conditioning therapy prior to cell transplantation. In one aspect, the CD19-expressing normal cell is a CD19-expressing normal stem cell and the cell transplantation is a stem cell transplantation.

In one aspect, the disclosure provides a cell (e.g., T cell) engineered to express a chimeric antigen receptor (CAR), wherein the CAR-expressing cell, e.g., CAR T cell ("CART"), exhibits an anticancer property. A preferred antigen is CD19. In one aspect, the antigen binding domain of the CAR comprises a partially humanized anti-CD 19 antibody fragment. In one aspect, the antigen binding domain of the CAR comprises a partially humanized anti-CD 19 antibody fragment comprising a scFv. Accordingly, the disclosure provides a CD19-CAR that comprises a humanized anti-CD 19 binding domain and is engineered into an immune effector cell, e.g., a T cell or an NK cell, and methods of their use for adoptive therapy.

In one aspect, the CD19-CAR comprises at least one intracellular domain selected from the group of a CD137 (4-1BB) signaling domain, a CD28 signaling domain, a CD3zeta signal domain, and any combination thereof. In one aspect, the CD19-CAR comprises at least one intracellular signaling domain is from one or more co-stimulatory molecule(s) other than a CD137 (4-1BB) or CD28.

### Chimeric Antigen Receptor (CAR)

The present disclosure encompasses a recombinant DNA construct comprising sequences encoding a CAR, wherein the CAR comprises an antibody or antibody fragment that binds specifically to a B-cell antigen (e.g., CD19, e.g., human CD19), wherein the sequence of the antibody fragment is contiguous with and in the same reading frame as a nucleic acid sequence encoding an intracellular signaling domain. The intracellular signaling domain can comprise a costimulatory signaling domain and/or a primary signaling domain, e.g., a zeta chain. The costimulatory signaling domain refers to a portion of the CAR comprising at least a portion of the intracellular domain of a costimulatory molecule. In one instance, the antigen binding domain is a murine antibody or antibody fragment described herein. In one instance, the antigen binding domain is a humanized antibody or antibody fragment.

In specific aspects, a CAR construct of the disclosure comprises a scFv domain selected from the group consisting of SEQ ID NOS:1-12 or an scFV domain of SEQ ID NO:59, wherein the scFv may be preceded by an optional leader sequence such as provided in SEQ ID NO: 13, and followed by an optional hinge sequence such as provided in SEQ ID NO:14 or SEQ ID NO:45 or SEQ ID NO:47 or SEQ ID NO:49, a transmembrane region such as provided in SEQ ID NO:15, an intracellular signalling domain that includes SEQ ID NO:16 or SEQ ID NO:51 and a CD3 zeta sequence that includes SEQ ID NO:17 or SEQ ID NO:43, wherein the domains are contiguous with and in the same reading frame to form a single fusion protein. Also included in the disclosure is a nucleotide sequence that encodes the polypeptide of each of the scFv fragments selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 and SEQ ID NO:59. Also included in the disclosure is a nucleotide sequence that encodes the polypeptide of each of the scFv fragments selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 and SEQ ID NO:59, and each of the domains of SEQ ID NOS: 13-17, plus the encoded CD19CAR fusion protein of the disclosure. In one aspect an exemplary CD19CAR constructs comprise an optional leader sequence, an extracellular antigen binding domain, a hinge, a transmembrane domain, and an intracellular stimulatory domain. In one aspect an exemplary CD19CAR construct comprises an optional leader sequence, an extracellular antigen binding domain, a hinge, a transmembrane domain, an intracellular costimulatory domain and an intracellular stimulatory domain. Specific CD19 CAR constructs containing humanized scFv domains of the disclosure are provided as SEQ ID NOS: 31-42, or a murine scFv domain as provided as SEQ ID NO:59.

Full-length CAR sequences are also provided herein as SEQ ID NOS: 31-42 and 58, as shown in Table 7 and Table 3.

An exemplary leader sequence is provided as SEQ ID NO: 13. An exemplary hinge/spacer sequence is provided as SEQ ID NO: 14 or SEQ ID NO:45 or SEQ ID NO:47 or SEQ ID NO:49. An exemplary transmembrane domain sequence is provided as SEQ ID NO:15. An exemplary sequence of the intracellular signaling domain of the 4-1BB protein is provided as SEQ ID NO: 16. An exemplary sequence of the intracellular signaling domain of CD27 is provided as SEQ ID NO:51. An exemplary CD3zeta domain sequence is provided as SEQ ID NO: 17 or SEQ ID NO:43.

In one aspect, the present disclosure encompasses a recombinant nucleic acid construct comprising a nucleic acid molecule encoding a CAR, wherein the nucleic acid molecule comprises the nucleic acid sequence encoding an anti-CD19 binding domain, e.g., described herein, that is contiguous with and in the same reading frame as a nucleic acid sequence encoding an intracellular signaling domain. In one aspect, the anti-CD19 binding domain is selected from one or more of SEQ ID NOS:1-12 and 58. In one aspect, the anti-CD 19 binding domain is encoded by a nucleotide residues 64 to 813 of the sequence provided in one or more of SEQ ID NOS:61-72 and 59. In one aspect, the anti-CD19 binding domain is encoded by a nucleotide residues 64 to 813 of SEQ ID NO:61. In one aspect, the anti-CD19 binding domain is encoded by a nucleotide residues 64 to 813 of SEQ ID NO:62. In one aspect, the anti-CD 19 binding domain is encoded by a nucleotide residues 64 to 813 of SEQ ID NO:63. In one aspect, the anti-CD19 binding domain is encoded by a nucleotide residues 64 to 813 of SEQ ID NO:64. In one aspect, the anti-CD19 binding domain is encoded by a nucleotide residues 64 to 813 of SEQ ID NO:65. In one aspect, the anti-CD 19 binding domain is encoded by a nucleotide residues 64 to 813 of SEQ ID NO:66. In one aspect, the anti-CD19 binding domain is encoded by a nucleotide residues 64 to 813 of SEQ ID NO:67. In one aspect, the anti-CD19 binding domain is encoded by a nucleotide residues 64 to 813 of SEQ ID NO:68. In one aspect, the anti-CD 19 binding domain is encoded by a nucleotide residues 64 to 813 of SEQ ID NO:69. In one aspect, the anti-CD19 binding domain is encoded by a nucleotide residues 64 to 813 of SEQ ID NO:70. In one aspect, the anti-CD19 binding domain is encoded by a nucleotide residues 64 to 813 of SEQ ID NO:71. In one aspect, the anti-CD19 binding domain is encoded by a nucleotide residues 64 to 813 of SEQ ID NO:72.

In one aspect, the present disclosure encompasses a recombinant nucleic acid construct comprising a transgene encoding a CAR, wherein the nucleic acid molecule comprises a nucleic acid sequence encoding an anti-CD19 binding domain selected from one or more of SEQ ID NOS:61-72, wherein the sequence is contiguous with and in the same reading frame as the nucleic acid sequence encoding an intracellular signaling domain. An exemplary intracellular signaling domain that can be used in the CAR includes, but is not limited to, one or more intracellular signaling domains of, e.g., CD3-zeta, CD28, 4-1BB, and the like. In some instances, the CAR can comprise any combination of CD3-zeta, CD28, 4-1BB, and the like. In one aspect the nucleic acid sequence of a CAR construct of the disclosure is selected from one or more of SEQ ID NOS:85-96. In one aspect the nucleic acid sequence of a CAR construct is SEQ ID NO:85. In one aspect the nucleic acid sequence of a CAR construct is SEQ ID NO:86. In one aspect the nucleic acid sequence of a CAR construct is SEQ ID NO:87. In one aspect the nucleic acid sequence of a CAR construct is SEQ ID NO:88. In one aspect the nucleic acid sequence of a CAR construct is SEQ ID NO:89. In one aspect the nucleic acid sequence of a CAR construct is SEQ ID NO:90. In one aspect the nucleic acid sequence of a CAR construct is SEQ ID NO:91. In one aspect the nucleic acid sequence of a CAR construct is SEQ ID NO:92. In one aspect the nucleic acid sequence of a CAR construct is SEQ ID NO:93. In one aspect the nucleic acid sequence of a CAR construct is SEQ ID NO:94. In one aspect the nucleic acid sequence of a CAR construct is SEQ ID NO:95. In one aspect the nucleic acid sequence of a CAR construct is SEQ ID NO:96. In one aspect the nucleic acid sequence of a CAR construct is SEQ ID NO:97. In one aspect the nucleic acid sequence of a CAR construct is SEQ ID NO:98. In one aspect the nucleic acid sequence of a CAR construct is SEQ ID NO:99.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the nucleic acid of interest can be produced synthetically, rather than cloned.

The present disclosure includes retroviral and lentiviral vector constructs expressing a CAR that can be directly transduced into a cell.

The present disclosure also includes an RNA construct that can be directly transfected into a cell. A method for generating mRNA for use in transfection involves in vitro transcription (TVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO:131). RNA so produced can efficiently transfect different kinds of cells. In one instance, the template includes sequences for the CAR. In an instance, an RNA CAR vector is transduced into a T cell by electroporation.

### Antigen binding domain

In one aspect, the CAR of the disclosure comprises a target-specific binding element otherwise referred to as an antigen binding domain. The choice of moiety depends upon the type and number of ligands that define the surface of a target cell. For example, the antigen binding domain may be chosen to recognize a ligand that acts as a cell surface marker on target cells associated with a particular disease state. Thus examples of cell surface markers that may act as ligands for the antigen binding domain in a CAR of the disclosure include those associated with viral, bacterial and parasitic infections, autoimmune disease and cancer cells.

In one aspect, the CAR-mediated T-cell response can be directed to an antigen of interest by way of engineering an antigen binding domain that specifically binds a desired antigen into the CAR.

In one aspect, the portion of the CAR comprising the antigen binding domain comprises an antigen binding domain that targets CD19. In one aspect, the antigen binding domain targets human CD19. In one aspect, the antigen binding domain of the CAR has the same or a similar binding specificity as the FMC63 scFv fragment described in Nicholson et al. Mol. Immun. 34 (16-17): 1157-1165 (1997). In one embodiment, the antigen binding domain of the CAR includes the scFv fragment described in Nicholson et al. Mol. Immun. 34 (16-17): 1157-1165 (1997).

The antigen binding domain can be any domain that binds to the antigen including but not limited to a monoclonal antibody, a polyclonal antibody, a recombinant antibody, a murine antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, and to an alternative scaffold known in the art to function as antigen binding domain, such as a recombinant fibronectin domain, and the like.

In one embodiment, the CAR molecule comprises an anti-CD 19 binding domain comprising one or more (e.g., all three) light chain complementary determining region 1 (LC CDR1), light chain complementary determining region 2 (LC CDR2), and light chain complementary determining region 3 (LC CDR3) of an anti-CD19 binding domain described herein, and one or more (e.g., all three) heavy chain complementary determining region 1 (HC CDR1), heavy chain complementary determining region 2 (HC CDR2), and heavy chain complementary determining region 3 (HC CDR3) of an anti-CD 19 binding domain described herein, e.g., an anti-CD19 binding domain comprising one or more, e.g., all three, LC CDRs and one or more, e.g., all three, HC CDRs. In one embodiment, the anti-CD19 binding domain comprises one or more (e.g., all three) heavy chain complementary determining region 1 (HC CDR1), heavy chain complementary determining region 2 (HC CDR2), and heavy chain complementary determining region 3 (HC CDR3) of an anti-CD19 binding domain described herein, e.g., the anti-CD 19 binding domain has two variable heavy chain regions, each comprising a HC CDR1, a HC CDR2 and a HC CDR3 described herein. In one embodiment, the anti-CD19 binding domain comprises a murine light chain variable region described herein (e.g., in Table 7) and/or a murine heavy chain variable region described herein (e.g., in Table 7). In one embodiment, the anti-CD19 binding domain is a scFv comprising a murine light chain and a murine heavy chain of an amino acid sequence of Table 7. In an embodiment, the anti-CD19 binding domain (e.g., an scFv) comprises: a light chain variable region comprising an amino acid sequence having at least one, two or three modifications (e.g., substitutions) but not more than 30, 20 or 10 modifications (e.g., substitutions) of an amino acid sequence of a light chain variable region provided in Table 7, or a sequence with 95-99% identity with an amino acid sequence of Table 7; and/or a heavy chain variable region comprising an amino acid sequence having at least one, two or three modifications (e.g., substitutions) but not more than 30, 20 or 10 modifications (e.g., substitutions) of an amino acid sequence of a heavy chain variable region provided in Table 7, or a sequence with 95-99% identity to an amino acid sequence of Table 7. In one embodiment, the anti-CD19 binding domain comprises a sequence of SEQ ID NO:59, or a sequence with 95-99% identify thereof. In one embodiment, the anti-CD 19 binding domain is a scFv, and a light chain variable region comprising an amino acid sequence described herein, e.g., in Table 7, is attached to a heavy chain variable region comprising an amino acid sequence described herein, e.g., in Table 7, via a linker, e.g., a linker described herein. In one embodiment, the anti-CD19 binding domain includes a (Gly₄-Ser)n linker, wherein n is 1, 2, 3, 4, 5, or 6, preferably 3 or 4 (SEQ ID NO: 53). The light chain variable region and heavy chain variable region of a scFv can be, e.g., in any of the following orientations: light chain variable region-linker-heavy chain variable region or heavy chain variable region-linker-light chain variable region.

In some instances, it is beneficial for the antigen binding domain to be derived from the same species in which the CAR will ultimately be used in. For example, for use in humans, it may be beneficial for the antigen binding domain of the CAR to comprise human or humanized residues for the antigen binding domain of an antibody or antibody fragment.

Thus, in one aspect, the antigen binding domain comprises a humanized antibody or an antibody fragment. In one embodiment, the humanized anti-CD19 binding domain comprises one or more (e.g., all three) light chain complementary determining region 1 (LC CDR1), light chain complementary determining region 2 (LC CDR2), and light chain complementary determining region 3 (LC CDR3) of a murine or humanized anti-CD19 binding domain described herein, and/or one or more (e.g., all three) heavy chain complementary determining region 1 (HC CDR1), heavy chain complementary determining region 2 (HC CDR2), and heavy chain complementary determining region 3 (HC CDR3) of a murine or humanized anti-CD19 binding domain described herein, e.g., a humanized anti-CD19 binding domain comprising one or more, e.g., all three, LC CDRs and one or more, e.g., all three, HC CDRs. In one embodiment, the humanized anti-CD19 binding domain comprises one or more (e.g., all three) heavy chain complementary determining region 1 (HC CDR1), heavy chain complementary determining region 2 (HC CDR2), and heavy chain complementary determining region 3 (HC CDR3) of a murine or humanized anti-CD 19 binding domain described herein, e.g., the humanized anti-CD19 binding domain has two variable heavy chain regions, each comprising a HC CDR1, a HC CDR2 and a HC CDR3 described herein. In one embodiment, the humanized anti-CD 19 binding domain comprises a humanized light chain variable region described herein (e.g., in Table 3) and/or a humanized heavy chain variable region described herein (e.g., in Table 3). In one embodiment, the humanized anti-CD19 binding domain comprises a humanized heavy chain variable region described herein (e.g., in Table 3), e.g., at least two humanized heavy chain variable regions described herein (e.g., in Table 3). In one embodiment, the anti-CD19 binding domain is a scFv comprising a light chain and a heavy chain of an amino acid sequence of Table 3. In an embodiment, the anti-CD19 binding domain (e.g., an scFv) comprises: a light chain variable region comprising an amino acid sequence having at least one, two or three modifications (e.g., substitutions) but not more than 30, 20 or 10 modifications (e.g., substitutions) of an amino acid sequence of a light chain variable region provided in Table 3, or a sequence with 95-99% identity with an amino acid sequence of Table 3; and/or a heavy chain variable region comprising an amino acid sequence having at least one, two or three modifications (e.g., substitutions) but not more than 30, 20 or 10 modifications (e.g., substitutions) of an amino acid sequence of a heavy chain variable region provided in Table 3, or a sequence with 95-99% identity to an amino acid sequence of Table 3. In one embodiment, the humanized anti-CD 19 binding domain comprises a sequence selected from a group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, and SEQ ID NO:12, or a sequence with 95-99% identify thereof. In one embodiment, the nucleic acid sequence encoding the humanized anti-CD 19 binding domain comprises a sequence selected from a group consisting of SEQ ID NO:61, SEQ ID NO:62, SEQ ID NO:63, SEQ ID NO:64, SEQ ID NO:65, SEQ ID NO:66, SEQ ID NO:67, SEQ ID NO:68, SEQ ID NO:70, SEQ ID NO:71 and SEQ ID NO:72, or a sequence with 95-99% identify thereof. In one embodiment, the humanized anti-CD19 binding domain is a scFv, and a light chain variable region comprising an amino acid sequence described herein, e.g., in Table 3, is attached to a heavy chain variable region comprising an amino acid sequence described herein, e.g., in Table 3, via a linker, e.g., a linker described herein. In one embodiment, the humanized anti-CD19 binding domain includes a (Gly₄-Ser)n linker, wherein n is 1, 2, 3, 4, 5, or 6, preferably 3 or 4 (SEQ ID NO:53). The light chain variable region and heavy chain variable region of a scFv can be, e.g., in any of the following orientations: light chain variable region-linker-heavy chain variable region or heavy chain variable region-linker-light chain variable region.

In one aspect, the antigen binding domain portion comprises one or more sequence selected from SEQ ID NOS:1-12. In one aspect the humanized CAR is selected from one or more sequence selected from SEQ ID NOS: 31-42. In some aspects, a non-human antibody is humanized, where specific sequences or regions of the antibody are modified to increase similarity to an antibody naturally produced in a human or fragment thereof.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089, veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973, chain shuffling (see, e.g., U.S. Pat. No. 5,565,332), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16):10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu J S, Gene, 150(2):409-10 (1994), and Pedersen et al., J. Mol. Biol., 235(3):959-73 (1994). Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323)

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments comprise one or more CDRs from nonhuman immunoglobulin molecules and framework regions wherein the amino acid residues comprising the framework are derived completely or mostly from human germline. Multiple techniques for humanization of antibodies or antibody fragments are well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640). In such humanized antibodies and antibody fragments, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies. Humanization of antibodies and antibody fragments can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987). Another method uses a particular framework derived from the consensus sequence of all human antibodies of a particular subgroup of light or heavy chains. The same framework may be used for several different humanized antibodies (see, e.g., Nicholson et al. Mol. Immun. 34 (16-17): 1157-1165 (1997); Carter et al., Proc. Natl. Acad. Sci. USA, 89:4285 (1992); Presta et al., J. Immunol., 151:2623 (1993). In some embodiments, the framework region, e.g., all four framework regions, of the heavy chain variable region are derived from a VH4_4-59 germline sequence. In one embodiment, the framework region can comprise, one, two, three, four or five modifications, e.g., substitutions, e.g., from the amino acid at the corresponding murine sequence (e.g., of SEQ ID NO:59). In one embodiment, the framework region, e.g., all four framework regions of the light chain variable region are derived from a VK3_1.25 germline sequence. In one embodiment, the framework region can comprise, one, two, three, four or five modifications, e.g., substitutions, e.g., from the amino acid at the corresponding murine sequence (e.g., of SEQ ID NO:59).

In some aspects, the portion of a CAR composition of the disclosure that comprises an antibody fragment is humanized with retention of high affinity for the target antigen and other favorable biological properties. According to one aspect of the disclosure, humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

A humanized antibody or antibody fragment may retain a similar antigenic specificity as the original antibody, e.g., in the present disclosure, the ability to bind human CD19. In some embodiments, a humanized antibody or antibody fragment may have improved affinity and/or specificity of binding to human CD19.

In one aspect, the anti-CD 19 binding domain is characterized by particular functional features or properties of an antibody or antibody fragment. For example, in one aspect, the portion of a CAR composition of the invention that comprises an antigen binding domain specifically binds human CD19. In one aspect, the antigen binding domain has the same or a similar binding specificity to human CD19 as the FMC63 scFv described in Nicholson et al. Mol. Immun. 34 (16-17): 1157-1165 (1997). In one aspect, the invention relates to an antigen binding domain comprising an antibody or antibody fragment, wherein the antibody binding domain specifically binds to a CD19 protein or fragment thereof, wherein the antibody or antibody fragment comprises a variable light chain and/or a variable heavy chain that includes an amino acid sequence of SEQ ID NO: 1-12 or SEQ ID NO:59. In one aspect, the antigen binding domain comprises an amino acid sequence of an scFv selected from SEQ ID NOs: 1-12 or SEQ ID NO:59. In certain aspects, the scFv is contiguous with and in the same reading frame as a leader sequence. In one aspect the leader sequence is the polypeptide sequence provided as SEQ ID NO:13.

In one aspect, the anti-CD19 binding domain is a fragment, e.g., a single chain variable fragment (scFv). In one aspect, the anti-CD 19 binding domain is a Fv, a Fab, a (Fab')2, or a bi-functional (e.g. bispecific) hybrid antibody (e.g., Lanzavecchia et al., Eur. J. Immunol. 17, 105 (1987)). In one aspect, the antibodies and fragments thereof of the disclosure binds a CD19 protein with wild-type or enhanced affinity.

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715.

A scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:18). In one embodiment, the linker can be (Gly₄Ser)₄ (SEQ ID NO:106) or (Gly₄Ser)₃ (SEQ ID NO:107). Variation in the linker length may retain or enhance activity, giving rise to superior efficacy in activity studies.

In some embodiments, the amino acid sequence of the antigen binding domain (or other portions or the entire CAR) can be modified, e.g., an amino acid sequence described herein can be modified, e.g., by a conservative substitution. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Percent identity in the context of two or more nucleic acids or polypeptide sequences, refers to two or more sequences that are the same. Two sequences are "substantially identical" if two sequences have a specified percentage of amino acid residues or nucleotides that are the same (e.g., 60% identity, optionally 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'1. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

In one aspect, the present invention contemplates modifications of the starting antibody or fragment (e.g., scFv) amino acid sequence that generate functionally equivalent molecules. For example, the VH or VL of an anti-CD19 binding domain, e.g., scFv, comprised in the CAR can be modified to retain at least about 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity of the starting VH or VL framework region of the anti-CD19 binding domain, e.g., scFv. The present invention contemplates modifications of the entire CAR construct, e.g., modifications in one or more amino acid sequences of the various domains of the CAR construct in order to generate functionally equivalent molecules. The CAR construct can be modified to retain at least about 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity of the starting CAR construct.

### Bispecific CARs

In an embodiment a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an embodiment the first and second epitopes are on the same antigen, e.g., the same protein (or subunit of a multimeric protein). In an embodiment the first and second epitopes overlap. In an embodiment the first and second epitopes do not overlap. In an embodiment the first and second epitopes are on different antigens, e.g., different proteins (or different subunits of a multimeric protein). In an embodiment a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an embodiment a bispecific antibody molecule comprises a half antibody having binding specificity for a first epitope and a half antibody having binding specificity for a second epitope. In an embodiment a bispecific antibody molecule comprises a half antibody, or fragment thereof, having binding specificity for a first epitope and a half antibody, or fragment thereof, having binding specificity for a second epitope. In an embodiment a bispecific antibody molecule comprises a scFv, or fragment thereof, have binding specificity for a first epitope and a scFv, or fragment thereof, have binding specificity for a second epitope.

In certain embodiments, the antibody molecule is a multi-specific (e.g., a bispecific or a trispecific) antibody molecule. Protocols for generating bispecific or heterodimeric antibody molecules, and various configurations for bispecific antibody molecules, are described in, e.g., paragraphs 455-458 of WO2015/142675, filed March 13, 2015.

In one aspect, the bispecific antibody molecule is characterized by a first immunoglobulin variable domain sequence, e.g., a scFv, which has binding specificity for CD19, e.g., comprises a scFv as described herein, or comprises the light chain CDRs and/or heavy chain CDRs from a scFv described herein, and a second immunoglobulin variable domain sequence that has binding specificity for a second epitope on a different antigen.

### Chimeric TCR

In one aspect, the antibodies and antibody fragments of the present disclosure (e.g., CD19 antibodies and fragments) can be grafted to one or more constant domain of a T cell receptor ("TCR") chain, for example, a TCR alpha or TCR beta chain, to create a chimeric TCR. Without being bound by theory, it is believed that chimeric TCRs will signal through the TCR complex upon antigen binding. For example, an scFv as disclosed herein, can be grafted to the constant domain, e.g., at least a portion of the extracellular constant domain, the transmembrane domain and the cytoplasmic domain, of a TCR chain, for example, the TCR alpha chain and/or the TCR beta chain. As another example, an antibody fragment, for example a VL domain as described herein, can be grafted to the constant domain of a TCR alpha chain, and an antibody fragment, for example a VH domain as described herein, can be grafted to the constant domain of a TCR beta chain (or alternatively, a VL domain may be grafted to the constant domain of the TCR beta chain and a VH domain may be grafted to a TCR alpha chain). As another example, the CDRs of an antibody or antibody fragment may be grafted into a TCR alpha and/or beta chain to create a chimeric TCR. For example, the LCDRs disclosed herein may be grafted into the variable domain of a TCR alpha chain and the HCDRs disclosed herein may be grafted to the variable domain of a TCR beta chain, or vice versa. Such chimeric TCRs may be produced, e.g., by methods known in the art (For example, Willemsen RA et al, Gene Therapy 2000; 7: 1369-1377; Zhang T et al, Cancer Gene Ther 2004; 11: 487-496; Aggen et al, Gene Ther. 2012 Apr;19(4):365-74).

### Non-Antibody Scaffolds

In embodiments, the antigen binding domain comprises a non-antibody scaffold, e.g., a fibronectin, ankyrin, domain antibody, lipocalin, small modular immuno-pharmaceutical, maxybody, Protein A, or affilin. The non-antibody scaffold has the ability to bind to target antigen on a cell. In embodiments, the antigen binding domain is a polypeptide or fragment thereof of a naturally occurring protein expressed on a cell. In some embodiments, the antigen binding domain comprises a non-antibody scaffold. A wide variety of non-antibody scaffolds can be employed so long as the resulting polypeptide includes at least one binding region which specifically binds to the target antigen on a target cell.

Non-antibody scaffolds include: fibronectin (Novartis, MA), ankyrin (Molecular Partners AG, Zurich, Switzerland), domain antibodies (Domantis, Ltd., Cambridge, MA, and Ablynx nv, Zwijnaarde, Belgium), lipocalin (Pieris Proteolab AG, Freising, Germany), small modular immuno-pharmaceuticals (Trubion Pharmaceuticals Inc., Seattle, WA), maxybodies (Avidia, Inc., Mountain View, CA), Protein A (Affibody AG, Sweden), and affilin (gamma-crystallin or ubiquitin) (Scil Proteins GmbH, Halle, Germany).

In an embodiment the antigen binding domain comprises the extracellular domain, or a counterligand binding fragment thereof, of molecule that binds a counterligand on the surface of a target cell.

### Transmembrane domain

With respect to the transmembrane domain, in various embodiments, a CAR can be designed to comprise a transmembrane domain that is attached to the extracellular domain of the CAR. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR, e.g., in one embodiment, the transmembrane domain may be from the same protein that the signaling domain, costimulatory domain or the hinge domain is derived from. In another aspect, the transmembrane domain is not derived from the same protein that any other domain of the CAR is derived from. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the cell surface of a CAR-expressing cell. In a different aspect the amino acid sequence of the transmembrane domain may be modified or substituted so as to minimize interactions with the binding domains of the native binding partner present in the same CAR-expressing cell.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. A transmembrane domain of particular use in this disclosure may include at least the transmembrane region(s) of e.g., the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C, or CD19.

In some instances, the transmembrane domain can be attached to the extracellular region of the CAR, e.g., the antigen binding domain of the CAR, via a hinge, e.g., a hinge from a human protein. For example, in one embodiment, the hinge can be a human Ig (immunoglobulin) hinge, e.g., an IgG4 hinge, an IgD hinge), a GS linker (e.g., a GS linker described herein), a KIR2DS2 hinge or a CD8a hinge. In one embodiment, the hinge or spacer comprises (e.g., consists of) the amino acid sequence of SEQ ID NO:14. In one aspect, the transmembrane domain comprises (e.g., consists of) a transmembrane domain of SEQ ID NO: 15.

In one aspect, the hinge or spacer comprises an IgG4 hinge. For example, in one embodiment, the hinge or spacer comprises a hinge of the amino acid sequence ESKYGPPCPPCPAPEFLGGPSVFLFPPKPKDTLMISRTPEVTCVVVDVSQEDPEVQFNWYVDGVEV HNAKTKPREEQFNSTYRVVSVLTVLHQDWLNGKEYKCKVSNKGLPSSIEKTISKAKGQPREPQVY TLPPSQEEMTKNQVSLTCLVKGFYPSDIAVEWESNGQPENNYKTTPPVLDSDGSFFLYSRLTVDKS RWQEGNVFSCSVMHEALHNHYTQKSLSLSLGKM (SEQ ID NO:45). In some embodiments, the hinge or spacer comprises a hinge encoded by a nucleotide sequence of

In one aspect, the hinge or spacer comprises an IgD hinge. For example, in one embodiment, the hinge or spacer comprises a hinge of the amino acid sequence RWPESPKAQASSVPTAQPQAEGSLAKATTAPATTRNTGRGGEEKKKEKEKEEQEERETKTPECPS HTQPLGVYLLTPAVQDLWLRDKATFTCFVVGSDLKDAHLTWEVAGKVPTGGVEEGLLERHSNGS QSQHSRLTLPRSLWNAGTSVTCTLNHPSLPPQRLMALREPAAQAPVKLSLNLLASSDPPEAASWLL CEVSGFSPPNILLMWLEDQREVNTSGFAPARPPPQPGSTTFWAWSVLRVPAPPSPQPATYTCWSH EDSRTLLNASRSLEVSYVTDH (SEQ ID NO:47). In some embodiments, the hinge or spacer comprises a hinge encoded by a nucleotide sequence of

In one aspect, the transmembrane domain may be recombinant, in which case it will comprise predominantly hydrophobic residues such as leucine and valine. In one aspect a triplet of phenylalanine, tryptophan and valine can be found at each end of a recombinant transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO:49). In some embodiments, the linker is encoded by a nucleotide sequence of GGTGGCGGAGGTTCTGGAGGTGGAGGTTCC (SEQ ID NO:50).

In one aspect, the hinge or spacer comprises a KIR2DS2 hinge.

### Cytoplasmic domain

The cytoplasmic domain or region of the CAR includes an intracellular signaling domain. An intracellular signaling domain is generally responsible for activation of at least one of the normal effector functions of the immune cell in which the CAR has been introduced.

Examples of intracellular signaling domains for use in the CAR of the disclosure include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are insufficient for full activation of the T cell and that a secondary and/or costimulatory signal is also required. Thus, T cell activation can be said to be mediated by two distinct classes of cytoplasmic signaling sequences: those that initiate antigen-dependent primary activation through the TCR (primary intracellular signaling domains) and those that act in an antigen-independent manner to provide a secondary or costimulatory signal (secondary cytoplasmic domain, e.g., a costimulatory domain).

A primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs.

Examples of ITAM containing primary intracellular signaling domains that are of particular use in the disclosure include those of TCR zeta, FcR gamma, FcR beta, CD3 gamma, CD3 delta, CD3 epsilon, CD5, CD22, CD79a, CD79b, CD278 (also known as "ICOS"), FcεRI, DAP10, DAP12, and CD66d. In one embodiment, a CAR of the disclosure comprises an intracellular signaling domain, e.g., a primary signaling domain of CD3-zeta.

In one embodiment, a primary signaling domain comprises a modified ITAM domain, e.g., a mutated ITAM domain which has altered (e.g., increased or decreased) activity as compared to the native ITAM domain. In one embodiment, a primary signaling domain comprises a modified ITAM-containing primary intracellular signaling domain, e.g., an optimized and/or truncated ITAM-containing primary intracellular signaling domain. In an embodiment, a primary signaling domain comprises one, two, three, four or more ITAM motifs.

Further examples of molecules containing a primary intracellular signaling domain that are of particular use in the disclosure include those of DAP10, DAP12, and CD32.

### Costimulatory Signaling Domain

The intracellular signalling domain of the CAR can comprise the CD3-zeta signaling domain by itself or it can be combined with any other desired intracellular signaling domain(s) useful in the context of a CAR of the disclosure. For example, the intracellular signaling domain of the CAR can comprise a CD3 zeta chain portion and a costimulatory signaling domain. The costimulatory signaling domain refers to a portion of the CAR comprising the intracellular domain of a costimulatory molecule. In one embodiment, the intracellular domain is designed to comprise the signaling domain of CD3-zeta and the signaling domain of CD28. In one aspect, the intracellular domain is designed to comprise the signaling domain of CD3-zeta and the signaling domain of ICOS.

A costimulatory molecule can be a cell surface molecule other than an antigen receptor or its ligands that is required for an efficient response of lymphocytes to an antigen. Examples of such molecules include CD27, CD28, 4-1BB (CD137), OX40, CD30, CD40, PD1, ICOS, lymphocyte function-associated antigen-1 (LFA-1), CD2, CD7, LIGHT, NKG2C, B7-H3, and a ligand that specifically binds with CD83, and the like. For example, CD27 costimulation has been demonstrated to enhance expansion, effector function, and survival of human CART cells in vitro and augments human T cell persistence and antitumor activity in vivo (Song et al. Blood. 2012; 119(3):696-706). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), NKG2D, CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, and CD19a.

The intracellular signaling sequences within the cytoplasmic portion of the CAR of the disclosure may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequence. In one embodiment, a glycine-serine doublet can be used as a suitable linker. In one embodiment, a single amino acid, e.g., an alanine, a glycine, can be used as a suitable linker.

In one aspect, the intracellular signaling domain is designed to comprise two or more, e.g., 2, 3, 4, 5, or more, costimulatory signaling domains. In an embodiment, the two or more, e.g., 2, 3, 4, 5, or more, costimulatory signaling domains, are separated by a linker molecule, e.g., a linker molecule described herein. In one embodiment, the intracellular signaling domain comprises two costimulatory signaling domains. In some embodiments, the linker molecule is a glycine residue. In some embodiments, the linker is an alanine residue.

In one aspect, the intracellular signaling domain is designed to comprise the signaling domain of CD3-zeta and the signaling domain of CD28. In one aspect, the intracellular signaling domain is designed to comprise the signaling domain of CD3-zeta and the signaling domain of 4-1BB. In one aspect, the signaling domain of 4-1BB is a signaling domain of SEQ ID NO: 16. In one aspect, the signaling domain of CD3-zeta is a signaling domain of SEQ ID NO: 17.

In one aspect, the intracellular signaling domain is designed to comprise the signaling domain of CD3-zeta and the signaling domain of CD27. In one aspect, the signaling domain of CD27 comprises an amino acid sequence of QRRKYRSNKGESPVEPAEPCRYSCPREEEGSTIPIQEDYRKPEPACSP (SEQ ID NO:51). In one aspect, the signalling domain of CD27 is encoded by a nucleic acid sequence of

**Strategies for Regulating Chimeric Antigen Receptors** In some embodiments, a regulatable CAR (RCAR) where the CAR activity can be controlled is desirable to optimize the safety and efficacy of a CAR therapy. There are many ways CAR activities can be regulated. For example, inducible apoptosis using, e.g., a caspase fused to a dimerization domain (see, e.g., Di Stasa et al., N Engl. J. Med. 2011 Nov. 3; 365(18):1673-1683), can be used as a safety switch in the CAR therapy of the instant disclosure. In one instance, the cells (e.g., T cells or NK cells) expressing a CAR of the present disclosure further comprise an inducible apoptosis switch, wherein a human caspase (e.g., caspase 9) or a modified version is fused to a modification of the human FKB protein that allows conditional dimerization. In the presence of a small molecule, such as a rapalog (e.g., AP 1903, AP20187), the inducible caspase (e.g., caspase 9) is activated and leads to the rapid apoptosis and death of the cells (e.g., T cells or NK cells) expressing a CAR of the present disclosure. Examples of a caspase based inducible apoptosis switch (or one or more aspects of such a switch) have been described in, e.g., US2004040047; US20110286980; US20140255360; WO1997031899; WO2014151960; WO2014164348; WO2014197638; WO2014197638.

In another example, CAR-expressing cells can also express an inducible Caspase-9 (iCaspase-9) molecule that, upon administration of a dimerizer drug (e.g., rimiducid (also called AP1903 (Bellicum Pharmaceuticals) or AP20187 (Ariad)) leads to activation of the Caspase-9 and apoptosis of the cells. The iCaspase-9 molecule contains a chemical inducer of dimerization (CID) binding domain that mediates dimerization in the presence of a CID. This results in inducible and selective depletion of CAR-expressing cells. In some cases, the iCaspase-9 molecule is encoded by a nucleic acid molecule separate from the CAR-encoding vector(s). In some cases, the iCaspase-9 molecule is encoded by the same nucleic acid molecule as the CAR-encoding vector. The iCaspase-9 can provide a safety switch to avoid any toxicity of CAR-expressing cells. See, e.g., Song et al. Cancer Gene Ther. 2008; 15(10):667-75; Clinical Trial Id. No. NCT02107963; and Di Stasi et al. N. Engl. J. Med. 2011; 365:1673-83.

Alternative strategies for regulating the CAR therapy of the instant disclosure include utilizing small molecules or antibodies that deactivate or turn off CAR activity, e.g., by deleting CAR-expressing cells, e.g., by inducing antibody dependent cell-mediated cytotoxicity (ADCC). For example, CAR-expressing cells described herein may also express an antigen that is recognized by molecules capable of inducing cell death, e.g., ADCC or complement-induced cell death. For example, CAR expressing cells described herein may also express a receptor capable of being targeted by an antibody or antibody fragment. Examples of such receptors include EpCAM, VEGFR, integrins (e.g., integrins ανβ3, α4, αI¾β3, α4β7, α5β1, ανβ3, αν), members of the TNF receptor superfamily (e.g., TRAIL-R1 , TRAIL-R2), PDGF Receptor, interferon receptor, folate receptor, GPNMB, ICAM-1 , HLA-DR, CEA, CA-125, MUC1 , TAG-72, IL-6 receptor, 5T4, GD2, GD3, CD2, CD3, CD4, CD5, CD1 1, CD1 1 a/LFA-1 , CD15, CD18/ITGB2, CD19, CD20, CD22, CD23/lgE Receptor, CD25, CD28, CD30, CD33, CD38, CD40, CD41 , CD44, CD51 , CD52, CD62L, CD74, CD80, CD125, CD147/basigin, CD152/CTLA-4, CD154/CD40L, CD195/CCR5, CD319/SLAMF7, and EGFR, and truncated versions thereof (e.g., versions preserving one or more extracellular epitopes but lacking one or more regions within the cytoplasmic domain).

For example, a CAR-expressing cell described herein may also express a truncated epidermal growth factor receptor (EGFR) which lacks signaling capacity but retains the epitope that is recognized by molecules capable of inducing ADCC, e.g., cetuximab (ERBITUX®), such that administration of cetuximab induces ADCC and subsequent depletion of the CAR-expressing cells (see, e.g., WO2011/056894, and Jonnalagadda et al., Gene Ther. 2013; 20(8)853-860). Another strategy includes expressing a highly compact marker/suicide gene that combines target epitopes from both CD32 and CD20 antigens in the CAR-expressing cells described herein, which binds rituximab, resulting in selective depletion of the CAR-expressing cells, e.g., by ADCC (see, e.g., Philip et al., Blood. 2014; 124(8)1277-1287). Other methods for depleting CAR-expressing cells described herein include administration of CAMPATH, a monoclonal anti-CD52 antibody that selectively binds and targets mature lymphocytes, e.g., CAR-expressing cells, for destruction, e.g., by inducing ADCC. In other instances, the CAR-expressing cell can be selectively targeted using a CAR ligand, e.g., an anti-idiotypic antibody. In some instances, the anti-idiotypic antibody can cause effector cell activity, e.g., ADCC or ADC activities, thereby reducing the number of CAR-expressing cells. In other instances, the CAR ligand, e.g., the anti-idiotypic antibody, can be coupled to an agent that induces cell killing, e.g., a toxin, thereby reducing the number of CAR-expressing cells. Alternatively, the CAR molecules themselves can be configured such that the activity can be regulated, e.g., turned on and off, as described below.

In other instances, a CAR-expressing cell described herein may also express a target protein recognized by the T cell depleting agent. In one instance, the target protein is CD20 and the T cell depleting agent is an anti-CD20 antibody, e.g., rituximab. In such instances, the T cell depleting agent is administered once it is desirable to reduce or eliminate the CAR-expressing cell, e.g., to mitigate the CAR induced toxicity. In other instances, the T cell depleting agent is an anti-CD52 antibody, e.g., alemtuzumab.

In an aspect, a RCAR comprises a set of polypeptides, typically two in the simplest embodiments, in which the components of a standard CAR described herein, e.g., an antigen binding domain and an intracellular signaling domain, are partitioned on separate polypeptides or members. In some instances, the set of polypeptides include a dimerization switch that, upon the presence of a dimerization molecule, can couple the polypeptides to one another, e.g., can couple an antigen binding domain to an intracellular signaling domain. In one instance, the CARs of the present disclosure utilizes a dimerization switch as those described in, e.g., WO2014127261. Additional description and exemplary configurations of such regulatable CARs are provided herein and in, e.g., paragraphs 527-551 of International Publication No. WO 2015/090229 filed March 13, 2015.

In some embodiments, an RCAR involves a switch domain, e.g., a FKBP switch domain, as set out SEQ ID NO: 122, or comprise a fragment of FKBP having the ability to bind with FRB, e.g., as set out in SEQ ID NO: 123. In some embodiments, the RCAR involves a switch domain comprising a FRB sequence, e.g., as set out in SEQ ID NO: 124, or a mutant FRB sequence, e.g., as set out in any of SEQ ID Nos. 125-130.

**Table 13. Exemplary mutant FRB having increased affinity for a dimerization molecule.**

| **FRB mutant** | **Amino Acid Sequence** | **SEQ ID NO:** |
|---|---|---|
| E2032I mutant | | 125 |
| E2032L mutant | | 126 |
| T2098L mutant | | 127 |
| E2032, T2098 mutant | | 128 |
| E2032I, T2098L mutant | | 129 |
| E2032L, T2098L mutant | | 130 |

### RNA Transfection

Disclosed herein are methods for producing an in vitro transcribed RNA CAR. The present disclosure also includes a CAR encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO: 131). RNA so produced can efficiently transfect different kinds of cells. In one aspect, the template includes sequences for the CAR.

In one aspect the anti-CD19 CAR is encoded by a messenger RNA (mRNA). In one aspect, the mRNA encoding the anti-CD19 CAR is introduced into an immune effector cell, e.g., a T cell or a NK cell, for production of a CAR-expressing cell, e.g., a CART cell or a CAR NK cell.

In one instance, the in vitro transcribed RNA CAR can be introduced to a cell as a form of transient transfection. The RNA is produced by in vitro transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired temple for in vitro transcription is a CAR of the present disclosure. For example, the template for the RNA CAR comprises an extracellular region comprising a single chain variable domain of an anti-tumor antibody; a hinge region, a transmembrane domain (e.g., a transmembrane domain of CD8a); and a cytoplasmic region that includes an intracellular signaling domain, e.g., comprising the signaling domain of CD3-zeta and the signaling domain of 4-1BB.

In one instance, the DNA to be used for PCR contains an open reading frame. The DNA can be from a naturally occurring DNA sequence from the genome of an organism. In one instance, the nucleic acid can include some or all of the 5' and/or 3' untranslated regions (UTRs). The nucleic acid can include exons and introns. In one instance, the DNA to be used for PCR is a human nucleic acid sequence. In another instance, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one instance, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one instance, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one instance, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other instances the 5' UTR can be 5'UTR of an RNA virus whose RNA genome is stable in cells. In other instances various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred instance, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred instance, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (SEQ ID NO: 110) (size can be 50-5000 T (SEQ ID NO: 111)), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one instance, the poly(A) tail is between 100 and 5000 adenosines (SEQ ID NO: 112).

Poly(A) tails of RNAs can be further extended following in vitro transcription with the use of a poly(A) polymerase, such as E. coli polyA polymerase (E-PAP). In one instance, increasing the length of a poly(A) tail from 100 nucleotides to between 300 and 400 nucleotides (SEQ ID NO: 113) results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred instance, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

### Non-viral delivery methods

In some aspects, non-viral methods can be used to deliver a nucleic acid encoding a CAR described herein into a cell or tissue or a subject.

In some instances, the non-viral method includes the use of a transposon (also called a transposable element). In some instances, a transposon is a piece of DNA that can insert itself at a location in a genome, for example, a piece of DNA that is capable of self-replicating and inserting its copy into a genome, or a piece of DNA that can be spliced out of a longer nucleic acid and inserted into another place in a genome. For example, a transposon comprises a DNA sequence made up of inverted repeats flanking genes for transposition.

Exemplary methods of nucleic acid delivery using a transposon include a Sleeping Beauty transposon system (SBTS) and a piggyBac (PB) transposon system. See, e.g., Aronovich et al. Hum. Mol. Genet. 20.R1(2011):R14-20; Singh et al. Cancer Res. 15(2008):2961-2971; Huang et al. Mol. Ther. 16(2008):580-589; Grabundzija et al. Mol. Ther. 18(2010):1200-1209; Kebriaei et al. Blood. 122.21(2013):166; Williams. Molecular Therapy 16.9(2008):1515-16; Bell et al. Nat. Protoc. 2.12(2007):3153-65; and Ding et al. Cell. 122.3(2005):473-83.

The SBTS includes two components: 1) a transposon containing a transgene and 2) a source of transposase enzyme. The transposase can transpose the transposon from a carrier plasmid (or other donor DNA) to a target DNA, such as a host cell chromosome/genome. For example, the transposase binds to the carrier plasmid/donor DNA, cuts the transposon (including transgene(s)) out of the plasmid, and inserts it into the genome of the host cell. See, e.g., Aronovich et al. *supra.*

Exemplary transposons include a pT2-based transposon. See, e.g., Grabundzija et al. Nucleic Acids Res. 41.3(2013):1829-47; and Singh et al. Cancer Res. 68.8(2008): 2961-2971. Exemplary transposases include a Tcl/mariner-type transposase, e.g., the SB10 transposase or the SB11 transposase (a hyperactive transposase which can be expressed, e.g., from a cytomegalovirus promoter). See, e.g., Aronovich et al.; Kebriaei et al.; and Grabundzija et al.

Use of the SBTS permits efficient integration and expression of a transgene, e.g., a nucleic acid encoding a CAR described herein. Provided herein are methods of generating a cell, e.g., T cell or NK cell, that stably expresses a CAR described herein, e.g., using a transposon system such as SBTS.

In accordance with methods described herein, in some instances, one or more nucleic acids, e.g., plasmids, containing the SBTS components are delivered to a cell (e.g., T or NK cell). For example, the nucleic acid(s) are delivered by standard methods of nucleic acid (e.g., plasmid DNA) delivery, e.g., methods described herein, e.g., electroporation, transfection, or lipofection. In some instances, the nucleic acid contains a transposon comprising a transgene, e.g., a nucleic acid encoding a CAR described herein. In some instances, the nucleic acid contains a transposon comprising a transgene (e.g., a nucleic acid encoding a CAR described herein) as well as a nucleic acid sequence encoding a transposase enzyme. In other instances, a system with two nucleic acids is provided, e.g., a dual-plasmid system, e.g., where a first plasmid contains a transposon comprising a transgene, and a second plasmid contains a nucleic acid sequence encoding a transposase enzyme. For example, the first and the second nucleic acids are co-delivered into a host cell.

In some instances, cells, e.g., T or NK cells, are generated that express a CAR described herein by using a combination of gene insertion using the SBTS and genetic editing using a nuclease (e.g., Zinc finger nucleases (ZFNs), Transcription Activator-Like Effector Nucleases (TALENs), the CRISPR/Cas system, or engineered meganuclease re-engineered homing endonucleases).

In some instances, use of a non-viral method of delivery permits reprogramming of cells, e.g., T or NK cells, and direct infusion of the cells into a subject. Advantages of non-viral vectors include but are not limited to the ease and relatively low cost of producing sufficient amounts required to meet a patient population, stability during storage, and lack of immunogenicity.

### Nucleic Acid Constructs Encoding a CAR

The present disclosure also provides nucleic acid molecules encoding one or more CAR constructs described herein. In one aspect, the nucleic acid molecule is provided as a messenger RNA transcript. In one aspect, the nucleic acid molecule is provided as a DNA construct. The nucleic acid molecules described herein can be a DNA molecule, an RNA molecule, or a combination thereof. In other instances, the nucleic acid molecule is a vector that includes any of the aforesaid nucleic acid molecules.

Accordingly, in one aspect, the disclosure pertains to an isolated nucleic acid molecule encoding a chimeric antigen receptor (CAR), wherein the CAR comprises a anti-CD19 binding domain (e.g., a humanized anti-CD19 binding domain), a transmembrane domain, and an intracellular signaling domain comprising a stimulatory domain, e.g., a costimulatory signaling domain and/or a primary signaling domain, e.g., zeta chain. In one instance, the anti-CD19 binding domain is an anti-CD19 binding domain described herein, e.g., an anti-CD19 binding domain which comprises a sequence selected from a group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 and SEQ ID NO:59, or a sequence with 95-99% identify thereof. In one instance, the transmembrane domain is transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In one instance, the transmembrane domain comprises a sequence of SEQ ID NO: 15, or a sequence with 95-99% identity thereof. In one instance, the anti-CD19 binding domain is connected to the transmembrane domain by a hinge region, e.g., a hinge described herein. In one instance, the hinge region comprises SEQ ID NO:14 or SEQ ID NO:45 or SEQ ID NO:47 or SEQ ID NO:49, or a sequence with 95-99% identity thereof. In one instance, the isolated nucleic acid molecule further comprises a sequence encoding a costimulatory domain. In one instance, the costimulatory domain is a functional signaling domain of a protein selected from the group consisting of OX40, CD27, CD28, CDS, ICAM-1, LFA-1 (CD11a/CD18), ICOS (CD278), and 4-1BB (CD137). In one instance, the costimulatory domain comprises a sequence of SEQ ID NO:16, or a sequence with 95-99% identity thereof. In one instance, the intracellular signaling domain comprises a functional signaling domain of 4-1BB and a functional signaling domain of CD3 zeta. In one instance, the intracellular signaling domain comprises the sequence of SEQ ID NO: 16 or SEQ ID NO:51, or a sequence with 95-99% identity thereof, and the sequence of SEQ ID NO: 17 or SEQ ID NO:43, or a sequence with 95-99% identity thereof, wherein the sequences comprising the intracellular signaling domain are expressed in the same frame and as a single polypeptide chain.

In another aspect, the disclosure pertains to an isolated nucleic acid molecule encoding a CAR construct comprising a leader sequence of SEQ ID NO: 13, a scFv domain having a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, and SEQ ID NO:59, (or a sequence with 95-99% identify thereof), a hinge region of SEQ ID NO:14 or SEQ ID NO:45 or SEQ ID NO:47 or SEQ ID NO:49 (or a sequence with 95-99% identity thereof), a transmembrane domain having a sequence of SEQ ID NO: 15 (or a sequence with 95-99% identity thereof), a 4-1BB costimulatory domain having a sequence of SEQ ID NO:16 or a CD27 costimulatory domain having a sequence of SEQ ID NO:51 (or a sequence with 95-99% identity thereof), and a CD3 zeta stimulatory domain having a sequence of SEQ ID NO:17 or SEQ ID NO:43 (or a sequence with 95-99% identity thereof).

In another aspect, the disclosure pertains to an isolated polypeptide molecule encoded by the nucleic acid molecule. In one instance, the isolated polypeptide molecule comprises a sequence selected from the group consisting of SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:59 or a sequence with 95-99% identify thereof.

In another aspect, the disclosure pertains to a nucleic acid molecule encoding a chimeric antigen receptor (CAR) molecule that comprises an anti-CD 19 binding domain, a transmembrane domain, and an intracellular signaling domain comprising a stimulatory domain, and wherein said anti-CD 19 binding domain comprises a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12 and SEQ ID NO:59, or a sequence with 95-99% identify thereof.

In one instance, the encoded CAR molecule further comprises a sequence encoding a costimulatory domain. In one instance, the costimulatory domain is a functional signaling domain of a protein selected from the group consisting of OX40, CD27, CD28, CDS, ICAM-1, LFA-1 (CD11a/CD18) and 4-1BB (CD137). In one instance, the costimulatory domain comprises a sequence of SEQ ID NO:16. In one instance, the transmembrane domain is a transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In one instance, the transmembrane domain comprises a sequence of SEQ ID NO:15. In one instance, the intracellular signaling domain comprises a functional signaling domain of 4-1BB and a functional signaling domain of zeta. In one instance, the intracellular signaling domain comprises the sequence of SEQ ID NO: 16 and the sequence of SEQ ID NO: 17, wherein the sequences comprising the intracellular signaling domain are expressed in the same frame and as a single polypeptide chain. In one instance, the anti-CD19 binding domain is connected to the transmembrane domain by a hinge region. In one instance, the hinge region comprises SEQ ID NO:14. In one instance, the hinge region comprises SEQ ID NO:45 or SEQ ID NO:47 or SEQ ID NO:49.

In another aspect, the disclosure pertains to an encoded CAR molecule comprising a leader sequence of SEQ ID NO: 13, a scFv domain having a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, and SEQ ID NO:59, or a sequence with 95-99% identify thereof, a hinge region of SEQ ID NO:14 or SEQ ID NO:45 or SEQ ID NO:47 or SEQ ID NO:49, a transmembrane domain having a sequence of SEQ ID NO: 15, a 4-1BB costimulatory domain having a sequence of SEQ ID NO:16 or a CD27 costimulatory domain having a sequence of SEQ ID NO:51, and a CD3 zeta stimulatory domain having a sequence of SEQ ID NO:17 or SEQ ID NO:43. In one instance, the encoded CAR molecule comprises a sequence selected from a group consisting of SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, and SEQ ID NO:59, or a sequence with 95-99% identify thereof.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present disclosure also provides vectors in which a DNA of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. A retroviral vector may also be, e.g., a gammaretroviral vector. A gammaretroviral vector may include, e.g., a promoter, a packaging signal (ψ), a primer binding site (PBS), one or more (e.g., two) long terminal repeats (LTR), and a transgene of interest, e.g., a gene encoding a CAR. A gammaretroviral vector may lack viral structural gens such as gag, pol, and env. Exemplary gammaretroviral vectors include Murine Leukemia Virus (MLV), Spleen-Focus Forming Virus (SFFV), and Myeloproliferative Sarcoma Virus (MPSV), and vectors derived therefrom. Other gammaretroviral vectors are described, e.g., in Tobias Maetzig et al., "Gammaretroviral Vectors: Biology, Technology and Application" Viruses. 2011 Jun; 3(6): 677-713.

In another instance, the vector comprising the nucleic acid encoding the desired CAR of the disclosure is an adenoviral vector (A5/35). In another instance, the expression of nucleic acids encoding CARs can be accomplished using of transposons such as sleeping beauty, crisper, CAS9, and zinc finger nucleases. See below June et al. 2009 Nature Reviews Immunology 9.10: 704-716.

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In brief summary, the expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

The expression constructs of the present disclosure may also be used for nucleic acid immunization and gene therapy, using standard gene delivery protocols. Methods for gene delivery are known in the art. See, e.g., U.S. Pat. Nos. 5,399,346, 5,580,859, 5,589,466. In another instance, the disclosure provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals. Viruses, which are useful as vectors include, but are not limited to, retroviruses, adenoviruses, adeno- associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some instances, adenovirus vectors are used. A number of adenovirus vectors are known in the art. In one instance, lentivirus vectors are used.

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription. Exemplary promoters include the CMV IE gene, EF-1α, ubiquitin C, or phosphoglycerokinase (PGK) promoters. In an instance, the promoter is a PGK promoter, e.g., a truncated PGK promoter as described herein.

An example of a promoter that is capable of expressing a CAR transgene in a mammalian T cell is the EF1a promoter. The native EF1a promoter drives expression of the alpha subunit of the elongation factor-1 complex, which is responsible for the enzymatic delivery of aminoacyl tRNAs to the ribosome. The EF1a promoter has been extensively used in mammalian expression plasmids and has been shown to be effective in driving CAR expression from transgenes cloned into a lentiviral vector. See, e.g., Milone et al., Mol. Ther. 17(8): 1453-1464 (2009). In one aspect, the EF1a promoter comprises the sequence provided as SEQ ID NO:100.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1α promoter, the hemoglobin promoter, and the creatine kinase promoter. Further, the disclosure should not be limited to the use of constitutive promoters. Inducible promoters are also contemplated as part of the disclosure. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Another example of a promoter is the phosphoglycerate kinase (PGK) promoter. In instances, a truncated PGK promoter (e.g., a PGK promoter with one or more, e.g., 1, 2, 5, 10, 100, 200, 300, or 400, nucleotide deletions when compared to the wild-type PGK promoter sequence) may be desired. The nucleotide sequences of exemplary PGK promoters are provided below.

### WT PGK Promoter:

### Exemplary truncated PGK Promoters:

### PGK100:

### PGK200:

### PGK300:

### PGK400:

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

In instances, the vector may comprise two or more nucleic acid sequences encoding a CAR, e.g., a CAR described herein, e.g., a CD19 CAR, and a second CAR, e.g., an inhibitory CAR or a CAR that specifically binds to an antigen other than CD19. In such instances, the two or more nucleic acid sequences encoding the CAR are encoded by a single nucleic molecule in the same frame and as a single polypeptide chain. In this aspect, the two or more CARs, can, e.g., be separated by one or more peptide cleavage sites. (e.g., an auto-cleavage site or a substrate for an intracellular protease). Examples of peptide cleavage sites include T2A, P2A, E2A, or F2A sites.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1 -4, Cold Spring Harbor Press, NY). A suitable method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g. , an artificial membrane vesicle). Other methods of state-of-the-art targeted delivery of nucleic acids are available, such as delivery of polynucleotides with targeted nanoparticles or other suitable sub-micron sized delivery system.

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present disclosure, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the disclosure.

The present disclosure further provides a vector comprising a CAR encoding nucleic acid molecule. In one aspect, a CAR vector can be directly transduced into a cell, *e.g.,* a T cell. In one aspect, the vector is a cloning or expression vector, *e.g.,* a vector including, but not limited to, one or more plasmids (*e.g.,* expression plasmids, cloning vectors, minicircles, minivectors, double minute chromosomes), retroviral and lentiviral vector constructs. In one aspect, the vector is capable of expressing the CAR construct in mammalian T cells. In one aspect, the mammalian T cell is a human T cell.

### Natural Killer Cell Receptor (NKR) CARs

In an instance, the CAR molecule described herein comprises one or more components of a natural killer cell receptor (NKR), thereby forming an NKR-CAR. The NKR component can be a transmembrane domain, a hinge domain, or a cytoplasmic domain from any of the following natural killer cell receptors: killer cell immunoglobulin-like receptor (KIR), e.g., KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1, and KIR3DP1; natural cytotoxicity receptor (NCR), e.g., NKp30, NKp44, NKp46; signaling lymphocyte activation molecule (SLAM) family of immune cell receptors, e.g., CD48, CD229, 2B4, CD84, NTB-A, CRACC, BLAME, and CD2F-10; Fc receptor (FcR), e.g., CD16, and CD64; and Ly49 receptors, e.g., LY49A, LY49C. The NKR-CAR molecules described herein may interact with an adaptor molecule or intracellular signaling domain, e.g., DAP12. Exemplary configurations and sequences of CAR molecules comprising NKR components are described in International Publication No. WO2014/145252.

### Split CAR

In some embodiments, the CAR-expressing cell uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 41BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens.

### Immune Effector cells

Also described herein are cells which contain a CAR molecule described herein or a nucleic acid encoding a CAR as described herein. Also described herein are cells which have been transfected or transformed with a nucleic acid described herein, e.g., a nucleic acid encoding a CAR, e.g., as described herein. In one instance, the cell is a cell described herein, e.g., a human T cell, e.g., a human T cell described herein, or a human NK cell, e.g., a human NK cell described herein. In one instance, the human T cell is a CD8+ T cell. In some instances, the cell is autologous to the subject to be treated with the cell. In some instances, the cell is allogeneic to the subject to be treated with the cell.

In one aspect, the CAR-expressing cell described herein can further comprise a second CAR, e.g., a second CAR that includes a different antigen binding domain, e.g., to the same target or a different target (e.g., a target other than a tumor antigen described herein or a different tumor antigen described herein). In one embodiment, the second CAR includes an antigen binding domain to a target expressed the same cancer cell type as the tumor antigen. In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. While not wishing to be bound by theory, placement of a costimulatory signaling domain, e.g., 4-1BB, CD28, ICOS, CD27 or OX-40, onto the first CAR, and the primary signaling domain, e.g., CD3 zeta, on the second CAR can limit the CAR activity to cells where both targets are expressed. In one instance, the CAR expressing cell comprises a first tumor antigen CAR that includes an antigen binding domain that binds a target antigen described herein, a transmembrane domain and a costimulatory domain and a second CAR that targets a different target antigen (e.g., an antigen expressed on that same cancer cell type as the first target antigen) and includes an antigen binding domain, a transmembrane domain and a primary signaling domain. In another instance, the CAR expressing cell comprises a first CAR that includes an antigen binding domain that binds a target antigen described herein, a transmembrane domain and a primary signaling domain and a second CAR that targets an antigen other than the first target antigen (e.g., an antigen expressed on the same cancer cell type as the first target antigen) and includes an antigen binding domain to the antigen, a transmembrane domain and a costimulatory signaling domain.

In another aspect, the present disclosure provides a population of CAR-expressing cells, e.g., at least one or more of which comprises a nucleic acid molecule described herein. In some instances, the population of CAR-expressing cells comprises a mixture of cells expressing different CARs.

For example, in one instance, the population of CART cells can include a first cell expressing a CAR having an antigen binding domain to a tumor antigen described herein, and a second cell expressing a CAR having a different antigen binding domain, e.g., an antigen binding domain to a different tumor antigen described herein, e.g., an antigen binding domain to a tumor antigen described herein that differs from the tumor antigen bound by the antigen binding domain of the CAR expressed by the first cell.

As another example, the population of CAR-expressing cells can include a first cell expressing a CAR that includes an antigen binding domain to a tumor antigen described herein, and a second cell expressing a CAR that includes an antigen binding domain to a target other than a tumor antigen as described herein. In one instance, the population of CAR-expressing cells includes, e.g., a first cell expressing a CAR that includes a primary intracellular signaling domain, and a second cell expressing a CAR that includes a secondary signaling domain.

In another aspect, the present disclosure provides a population of cells wherein at least one cell in the population expresses a CAR having an antigen binding domain to a tumor antigen described herein, and a second cell expressing another agent, e.g., an agent which enhances the activity of a CAR-expressing cell. In one instance, the agent can be an agent which inhibits an inhibitory molecule. Inhibitory molecules, e.g., PD-1, can, in some instances, decrease the ability of a CAR-expressing cell to mount an immune effector response. Examples of inhibitory molecules include PD-1, PD-L1, PD-L2, CTLA4, TIM3, CEACAM (CEACAM-1, CEACAM-3, and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR (e.g., TGFRbeta). In one instance, the agent which inhibits an inhibitory molecule comprises a first polypeptide, e.g., an inhibitory molecule, associated with a second polypeptide that provides a positive signal to the cell, e.g., an intracellular signaling domain described herein. In one instance, the agent comprises a first polypeptide, e.g., of an inhibitory molecule such as PD1, PD-L1, CTLA4, TIM3, CEACAM (e.g., CEACAM-1, CEACAM-3, and/or CEACAM-5), LAG3, VISTA, BTLA, TIGIT, LAIR1, CD160, 2B4 or TGFR beta, or a fragment of any of these, and a second polypeptide which is an intracellular signaling domain described herein (e.g., comprising a costimulatory domain (e.g., 41BB, CD27, OX40 or CD28, e.g., as described herein) and/or a primary signaling domain (e.g., a CD3 zeta signaling domain described herein). In one instance, the agent comprises a first polypeptide of PD-1 or a fragment thereof, and a second polypeptide of an intracellular signaling domain described herein (e.g., a CD28 signaling domain described herein and/or a CD3 zeta signaling domain described herein).

### Co-expression of CAR with Other Molecules or Agents

### Co-expression of a Second CAR

In one aspect, the CAR-expressing cell described herein can further comprise a second CAR, e.g., a second CAR that includes a different antigen binding domain, e.g., to the same target (e.g., CD19) or a different target (e.g., a target other than CD19, e.g., a target described herein). In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. Placement of a costimulatory signaling domain, e.g., 4-1BB, CD28, CD27, OX-40 or ICOS, onto the first CAR, and the primary signaling domain, e.g., CD3 zeta, on the second CAR can limit the CAR activity to cells where both targets are expressed. In one embodiment, the CAR expressing cell comprises a first CAR that includes an antigen binding domain, a transmembrane domain and a costimulatory domain and a second CAR that targets another antigen and includes an antigen binding domain, a transmembrane domain and a primary signaling domain. In another embodiment, the CAR expressing cell comprises a first CAR that includes an antigen binding domain, a transmembrane domain and a primary signaling domain and a second CAR that targets another antigen and includes an antigen binding domain to the antigen, a transmembrane domain and a costimulatory signaling domain.

In one instance, the CAR-expressing cell comprises an XCAR described herein and an inhibitory CAR. In one instance, the inhibitory CAR comprises an antigen binding domain that binds an antigen found on normal cells but not cancer cells. In one instance, the inhibitory CAR comprises the antigen binding domain, a transmembrane domain and an intracellular domain of an inhibitory molecule. For example, the intracellular domain of the inhibitory CAR can be an intracellular domain of PD1, PD-L1, PD-L2, CTLA4, TIM3, CEACAM (e.g., CEACAM-1, CEACAM-3, and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR (e.g., TGFRbeta).

In one embodiment, when the CAR-expressing cell comprises two or more different CARs, the antigen binding domains of the different CARs can be such that the antigen binding domains do not interact with one another. For example, a cell expressing a first and second CAR can have an antigen binding domain of the first CAR, e.g., as a fragment, e.g., an scFv, that does not form an association with the antigen binding domain of the second CAR, e.g., the antigen binding domain of the second CAR is a VHH.

In some embodiments, the antigen binding domain comprises a single domain antigen binding (SDAB) molecules include molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies. SDAB molecules may be any of the art, or any future single domain molecules. SDAB molecules may be derived from any species including, but not limited to mouse, human, camel, llama, lamprey, fish, shark, goat, rabbit, and bovine. This term also includes naturally occurring single domain antibody molecules from species other than Camelidae and sharks.

In one aspect, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from Camelidae species, for example in camel, llama, dromedary, alpaca and guanaco. Other species besides Camelidae may produce heavy chain molecules naturally devoid of light chain; such VHHs are within the scope of the disclosure.

The SDAB molecules can be recombinant, CDR-grafted, humanized, camelized, de-immunized and/or in vitro generated (e.g., selected by phage display).

It has also been discovered, that cells having a plurality of chimeric membrane embedded receptors comprising an antigen binding domain that interactions between the antigen binding domain of the receptors can be undesirable, e.g., because it inhibits the ability of one or more of the antigen binding domains to bind its cognate antigen. Accordingly, disclosed herein are cells having a first and a second non-naturally occurring chimeric membrane embedded receptor comprising antigen binding domains that minimize such interactions. Also disclosed herein are nucleic acids encoding a first and a second non-naturally occurring chimeric membrane embedded receptor comprising an antigen binding domains that minimize such interactions, as well as methods of making and using such cells and nucleic acids. In an embodiment the antigen binding domain of one of the first and the second non-naturally occurring chimeric membrane embedded receptor, comprises an scFv, and the other comprises a single VH domain, e.g., a camelid, shark, or lamprey single VH domain, or a single VH domain derived from a human or mouse sequence.

In some embodiments, the cell comprises a first and second CAR, wherein the antigen binding domain of one of the first CAR and the second CAR does not comprise a variable light domain and a variable heavy domain. In some embodiments, the antigen binding domain of one of the first CAR and the second CAR is an scFv, and the other is not an scFv. In some embodiments, the antigen binding domain of one of the first CAR and the second CAR comprises a single VH domain, e.g., a camelid, shark, or lamprey single VH domain, or a single VH domain derived from a human or mouse sequence. In some embodiments, the antigen binding domain of one of the first CAR and the second CAR comprises a nanobody. In some embodiments, the antigen binding domain of one of the first CAR and the second CAR comprises a camelid VHH domain.

In some embodiments, the antigen binding domain of one of the first CAR and the second CAR comprises an scFv, and the other comprises a single VH domain, e.g., a camelid, shark, or lamprey single VH domain, or a single VH domain derived from a human or mouse sequence. In some embodiments, the antigen binding domain of one of the first CAR and the second CAR comprises an scFv, and the other comprises a nanobody. In some embodiments, the antigen binding domain of one of the first CAR and the second CAR comprises an scFv, and the other comprises a camelid VHH domain.

In some embodiments, when present on the surface of a cell, binding of the antigen binding domain of the first CAR to its cognate antigen is not substantially reduced by the presence of the second CAR. In some embodiments, binding of the antigen binding domain of the first CAR to its cognate antigen in the presence of the second CAR is 85%, 90%, 95%, 96%, 97%, 98% or 99% of binding of the antigen binding domain of the first CAR to its cognate antigen in the absence of the second CAR.

In some embodiments, when present on the surface of a cell, the antigen binding domains of the first CAR and the second CAR, associate with one another less than if both were scFv antigen binding domains. In some embodiments, the antigen binding domains of the first CAR and the second CAR, associate with one another 85%, 90%, 95%, 96%, 97%, 98% or 99% less than if both were scFv antigen binding domains.

### Co-expression of an Agent that Enhances CAR Activity

In another aspect, the CAR-expressing cell described herein can further express another agent, e.g., an agent that enhances the activity or fitness of a CAR-expressing cell.

For example, in one embodiment, the agent can be an agent which inhibits a molecule that modulates or regulates, e.g., inhibits, T cell function. In some embodiments, the molecule that modulates or regulates T cell function is an inhibitory molecule. Inhibitory molecules, e.g., PD1, can, in some embodiments, decrease the ability of a CAR-expressing cell to mount an immune effector response. Examples of inhibitory molecules include PD1, PD-L1, CTLA4, TIM3, LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, or TGFR beta.

In one embodiment, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used to inhibit expression of a molecule that modulates or regulates, e.g., inhibits, T-cell function in the CAR-expressing cell. In an embodiment the agent is an shRNA, e.g., an shRNA described herein. In an embodiment, the agent that modulates or regulates, e.g., inhibits, T-cell function is inhibited within a CAR-expressing cell. For example, a dsRNA molecule that inhibits expression of a molecule that modulates or regulates, e.g., inhibits, T-cell function is linked to the nucleic acid that encodes a component, e.g., all of the components, of the CAR.

In one embodiment, the agent which inhibits an inhibitory molecule comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD1, PD-L1, CTLA4, TIM3, LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, or TGFR beta, or a fragment of any of these (e.g., at least a portion of an extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of an extracellular domain of PD1), and a second polypeptide of an intracellular signaling domain described herein (e.g., a CD28 signaling domain described herein and/or a CD3 zeta signaling domain described herein). PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

In one embodiment, the agent comprises the extracellular domain (ECD) of an inhibitory molecule, e.g., Programmed Death 1 (PD1), can be fused to a transmembrane domain and intracellular signaling domains such as 41BB and CD3 zeta (also referred to herein as a PD1 CAR). In one embodiment, the PD1 CAR, when used incombinations with a CD19 CAR described herein, improves the persistence of the T cell. In one embodiment, the CAR is a PD1 CAR comprising the extracellular domain of PD1 indicated as underlined in SEQ ID NO: 121. In one embodiment, the PD1 CAR comprises the amino acid sequence of SEQ ID NO:121.

In one embodiment, the PD1 CAR comprises the amino acid sequence provided below (SEQ ID NO: 132).

In one embodiment, the agent comprises a nucleic acid sequence encoding the PD1 CAR, e.g., the PD1 CAR described herein. In one embodiment, the nucleic acid sequence for the PD1 CAR is shown below, with the PD1 ECD underlined below in SEQ ID NO: 120

In another example, in one embodiment, the agent which enhances the activity of a CAR-expressing cell can be a costimulatory molecule or costimulatory molecule ligand. Examples of costimulatory molecules include an MHC class I molecule, a TNF receptor protein, an Immunoglobulinlike protein, a cytokine receptor, an integrins, a signalling lymphocytic activation molecule (SLAM protein),an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83, e.g., as described herein. Examples of costimulatory molecule ligands include CD80, CD86, CD40L, ICOSL, CD70, OX40L, 4-1BBL, GITRL, and LIGHT. In embodiments, the costimulatory molecule ligand is a ligand for a costimulatory molecule different from the costimulatory molecule domain of the CAR. In embodiments, the costimulatory molecule ligand is a ligand for a costimulatory molecule that is the same as the costimulatory molecule domain of the CAR. In an embodiment, the costimulatory molecule ligand is 4-1BBL. In an embodiment, the costimulatory ligand is CD80 or CD86. In an embodiment, the costimulatory molecule ligand is CD70. In embodiments, a CAR-expressing immune effector cell described herein can be further engineered to express one or more additional costimulatory molecules or costimulatory molecule ligands.

### Sources of Cells

Prior to expansion and genetic modification or other modification, a source of cells, e.g., T cells or natural killer (NK) cells, can be obtained from a subject. Examples of subjects include humans, monkeys, chimpanzees, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors.

In certain aspects of the present disclosure, immune effector cells, e.g., T cells, can be obtained from a unit of blood collected from a subject using any number of techniques known to the skilled artisan, such as Ficoll™ separation. In one aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one instance, the cells are washed with phosphate buffered saline (PBS). In an alternative instance, the wash solution lacks calcium and may lack magnesium or may lack many if not all divalent cations.

Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi:10.1038/cti.2014.31.

In one aspect, T cells are isolated from peripheral blood lymphocytes by lysing the red blood cells and depleting the monocytes, for example, by centrifugation through a PERCOLL™ gradient or by counterflow centrifugal elutriation.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. In some instances, the population of T regulatory depleted cells contains less than 30%, 25%, 20%, 15%, 10%, 5%, 4%, 3%, 2%, 1 % of CD25+ cells.

In one instance, T regulatory cells, e.g., CD25+ T cells, are removed from the population using an anti-CD25 antibody, or fragment thereof, or a CD25-binding ligand, e.g., IL-2. In one instance, the anti-CD25 antibody, or fragment thereof, or CD25-binding ligand is conjugated to a substrate, e.g., a bead, or is otherwise coated on a substrate, e.g., a bead. In one instance, the anti-CD25 antibody, or fragment thereof, is conjugated to a substrate as described herein.

In one instance, the T regulatory cells, e.g., CD25+ T cells, are removed from the population using CD25 depletion reagent from Miltenyi™. In one instance, the ratio of cells to CD25 depletion reagent is 1e7 cells to 20 uL, or 1e7 cells to15 uL, or 1e7 cells to 10 uL, or 1e7 cells to 5 uL, or 1e7 cells to 2.5 uL, or 1e7 cells to 1.25 uL. In one instance, e.g., for T regulatory cells, e.g., CD25+ depletion, greater than 500 million cells/ml is used. In a further aspect, a concentration of cells of 600, 700, 800, or 900 million cells/ml is used.

In one instance, the population of immune effector cells to be depleted includes about 6 x 10⁹ CD25+ T cells. In other aspects, the population of immune effector cells to be depleted include about 1 x 10⁹ to 1x 10¹⁰ CD25+ T cell, and any integer value in between. In one instance, the resulting population T regulatory depleted cells has 2 x 10⁹ T regulatory cells, e.g., CD25+ cells, or less (e.g., 1 x 10⁹, 5 x 10⁸, 1 x 10⁸, 5 x 10⁷, 1 x 10⁷, or less CD25+ cells).

In one instance, the T regulatory cells, e.g., CD25+ cells, are removed from the population using the CliniMAC system with a depletion tubing set, such as, e.g., tubing 162-01. In one instance, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product can reduce the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, mTOR inhibitor, and combinations thereof.

In some instances, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In an instance, a subject is pre-treated with one or more therapies that reduce T_{REG} cells prior to collection of cells for CAR-expressing cell product manufacturing, thereby reducing the risk of subject relapse to CAR-expressing cell treatment. In an instance, methods of decreasing T_{REG} cells include, but are not limited to, administration to the subject of one or more of cyclophosphamide, anti-GITR antibody, CD25-depletion, or a combination thereof. Administration of one or more of cyclophosphamide, anti-GITR antibody, CD25-depletion, or a combination thereof, can occur before, during or after an infusion of the CAR-expressing cell product.

In an instance, a subject is pre-treated with cyclophosphamide prior to collection of cells for CAR-expressing cell product manufacturing, thereby reducing the risk of subject relapse to CAR-expressing cell treatment. In an instance, a subject is pre-treated with an anti-GITR antibody prior to collection of cells for CAR-expressing cell product manufacturing, thereby reducing the risk of subject relapse to CAR-expressing cell treatment.

In one instance, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one instance, such cells are envisioned to be removed concurrently with regulatory T cells and/or tumor cells, or following said depletion, or in another order.

In an instance, the CAR-expressing cell (e.g., T cell, NK cell) manufacturing process is modified to deplete T_{REG} cells prior to manufacturing of the CAR-expressing cell (e.g., T cell, NK cell) product (e.g., a CTL019 product). In an instance, CD25-depletion is used to deplete T_{REG} cells prior to manufacturing of the CAR-expressing cell (e.g., T cell, NK cell) product (e.g., a CTL019 product).

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a CAR, e.g., a CAR described herein. In one instance, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other instances, the removal of T regulatory cells, e.g., CD25+ cells, and the removal of the tumor antigen expressing cells is sequential, and can occur, e.g., in either order.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary check point inhibitors include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR (e.g., TGFRbeta), e.g., as described hereinIn one instance, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other instances, the removal of T regulatory cells, e.g., CD25+ cells, and the removal of the check point inhibitor expressing cells is sequential, and can occur, e.g., in either order.

Methods described herein can include a positive selection step. For example, T cells can isolated by incubation with anti-CD3/anti-CD28 (e.g., 3x28)-conjugated beads, such as DYNABEADS® M-450 CD3/CD28 T, for a time period sufficient for positive selection of the desired T cells. In one instance, the time period is about 30 minutes. In a further instance, the time period ranges from 30 minutes to 36 hours or longer and all integer values there between. In a further instance, the time period is at least 1, 2, 3, 4, 5, or 6 hours. In yet another instance, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points.

In one instance, a T cell population can be selected that expresses one or more of IFN-γ, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 10 billion cells/ml, 9 billion/ml, 8 billion/ml, 7 billion/ml, 6 billion/ml, or 5 billion/ml is used. In one aspect, a concentration of 1 billion cells/ml is used. In yet one aspect, a concentration of cells from 75, 80, 85, 90, 95, or 100 million cells/ml is used. In further aspects, concentrations of 125 or 150 million cells/ml can be used.

Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 x 10⁶/ml. In other aspects, the concentration used can be from about 1 x 10⁵/ml to 1 x 10⁶/ml, and any integer value in between.

In other aspects, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present disclosure.

Also contemplated in the context of the disclosure is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in immune effector cell therapy for any number of diseases or conditions that would benefit from immune effector cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present disclosure, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present disclosure to collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one instance, the immune effector cells expressing a CAR molecule, e.g., a CAR molecule described herein, are obtained from a subject that has received a low, immune enhancing dose of an mTOR inhibitor. In an instance, the population of immune effector cells, e.g., T cells, to be engineered to express a CAR, are harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other instances, population of immune effector cells, e.g., T cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In one instance, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one instance, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In instances, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an instance, the NK cells are obtained from the subject. In another instance, the NK cells are an NK cell line, e.g., NK-92 cell line (Conkwest).

### Allogeneic CAR

In embodiments described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

A T cell lacking a functional TCR can be, e.g., engineered such that it does not express any functional TCR on its surface, engineered such that it does not express one or more subunits that comprise a functional TCR (e.g., engineered such that it does not express (or exhibits reduced expression) of TCR alpha, TCR beta, TCR gamma, TCR delta, TCR epsilon, and/or TCR zeta) or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, is downregulated. In some embodiments, downregulation of HLA may be accomplished by reducing or eliminating expression of beta-2 microglobulin (B2M).

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not express or expresses at low levels an inhibitory molecule, e.g. by any mehod described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR (e.g., TGFR beta). Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some embodiments, TCR expression and/or HLA expression can be inhibited using siRNA or shRNA that targets a nucleic acid encoding a TCR and/or HLA and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta), in a cell, e.g., T cell.
Expression systems for siRNA and shRNAs, and exemplary shRNAs, are described, e.g., in paragraphs 649 and 650 of International Application WO2015/142675, filed March 13, 2015*.**CRISPR to inhibit TCR or HLA***

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta), in a cell, e.g., T cell.
The CRISPR/Cas system, and uses thereof, are described, e.g., in paragraphs 651-658 of International Application WO2015/142675, filed March 13, 2015

### TALEN to inhibit TCR and/or HLA

"TALEN" or "TALEN to HLA and/or TCR" or "TALEN to inhibit HLA and/or TCR" refers to a transcription activator-like effector nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta), in a cell, e.g., T cell.

TALENs, and uses thereof, are described, e.g., in paragraphs 659-665 of International Application WO2015/142675, filed March 13, 2015. ***Zinc finger nuclease to inhibit HLA and*/*or TCR***

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta), in a cell, e.g., T cell.
ZFNs, and uses thereof, are described, e.g., in paragraphs 666-671 of International Application WO2015/142675, filed March 13, 2015.

### Telomerase expression

While not wishing to be bound by any particular theory, in some instances, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an instance, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell,
comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

In one aspect, the disclosure features a method of making a population of immune effector cells (e.g., T cells or NK cells). In an instance, the method comprises: providing a population of immune effector cells (e.g., T cells or NK cells), contacting the population of immune effector cells with a nucleic acid encoding a CAR; and contacting the population of immune effector cells with a nucleic acid encoding a telomerase subunit, e.g., hTERT, under conditions that allow for CAR and telomerase expression.

In an instance, the nucleic acid encoding the telomerase subunit is DNA. In an instance, the nucleic acid encoding the telomerase subunit comprises a promoter capable of driving expression of the telomerase subunit.

In an instance, hTERT has the amino acid sequence of GenBank Protein ID AAC51724.1 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795) as follows:

In an instance, the hTERT has a sequence at least 80%, 85%, 90%, 95%, 96^, 97%, 98%, or 99% identical to the sequence of SEQ ID NO: 118. In an instance, the hTERT has a sequence of SEQ ID NO: 118. In an instance, the hTERT comprises a deletion (e.g., of no more than 5, 10, 15, 20, or 30 amino acids) at the N-terminus, the C-terminus, or both. In an instance, the hTERT comprises a transgenic amino acid sequence (e.g., of no more than 5, 10, 15, 20, or 30 amino acids) at the N-terminus, the C-terminus, or both.

In an instance, the hTERT is encoded by the nucleic acid sequence of GenBank Accession No. AF018167 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795):

In an instance, the hTERT is encoded by a nucleic acid having a sequence at least 80%, 85%, 90%, 95%, 96, 97%, 98%, or 99% identical to the sequence of SEQ ID NO: 119. In an instance, the hTERT is encoded by a nucleic acid of SEQ ID NO: 119.

### Activation and Expansion of Immune Effector Cells (e.g., T Cells)

Immune effector cells such as T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

The procedure for *ex vivo* expansion of hematopoietic stem and progenitor cells is described in U.S. Pat. No. 5,199,942, can be applied to the cells of the present disclosure. Other suitable methods are known in the art, therefore the present disclosure is not limited to any particular method of *ex vivo* expansion of the cells. Briefly, *ex vivo* culture and expansion of T cells can comprise: (1) collecting CD34+ hematopoietic stem and progenitor cells from a mammal from peripheral blood harvest or bone marrow explants; and (2) expanding such cells *ex vivo.* In addition to the cellular growth factors described in U.S. Pat. No. 5,199,942, other factors such as flt3-L, IL-1, IL-3 and c-kit ligand, can be used for culturing and expansion of the cells.

Generally, a population of immune effector cells e.g., T regulatory cell depleted cells, may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody can be used. Examples of an anti-CD28 antibody include 9.3, B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present disclosure.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present disclosure, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3:CD28 antibody bound to the beads ranges from 100:1 to 1:100 and all integer values there between. In one aspect, more anti-CD28 antibody is bound to the particles than anti-CD3 antibody, i.e., the ratio of CD3:CD28 is less than one.
In certain aspects, the ratio of anti CD28 antibody to anti CD3 antibody bound to the beads is greater than 2:1. In one particular aspect, a 1:100 CD3:CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one aspect, a 1:10 CD3:CD28 ratio of antibody bound to beads is used. In one aspect, a 1:3 CD3:CD28 ratio of antibody bound to the beads is used. In yet one aspect, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain suitablevalues include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one aspect, a ratio of particles to cells of 1:1 or less is used. In one particular aspect, a suitable particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present disclosure. In particular, ratios will vary depending on particle size and on cell size and type. In one aspect, the most typical ratios for use are in the neighborhood of 1:1, 2:1 and 3:1 on the first day.

In further aspects, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS® M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01 % of the sample or the entire sample (i.e., 100%) may comprise the target cell of interest. Accordingly, any cell number is within the context of the present disclosure. In certain aspects, it may be desirable to significantly decrease the volume in which particles and cells are mixed together (i.e., increase the concentration of cells), to ensure maximum contact of cells and particles. For example, in one aspect, a concentration of about 10 billion cells/ml, 9 billion/ml, 8 billion/ml, 7 billion/ml, 6 billion/ml, 5 billion/ml, or 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+ T cells that normally have weaker CD28 expression.

In one instance, cells transduced with a nucleic acid encoding a CAR, e.g., a CAR described herein, are expanded, e.g., by a method described herein. In one instance, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one instance, the cells are expanded for a period of 4 to 9 days. In one instance, the cells are expanded for a period of 8 days or less, e.g., 7, 6 or 5 days. In one instance, the cells, e.g., a CD19 CAR cell described herein, are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one instance, the cells, e.g., a CD19 CAR cell described herein, expanded for 5 days show at least a one, two, three or four fold increase in cells doublings upon antigen stimulation as compared to the same cells expanded in culture for 9 days under the same culture
conditions. In one instance, the cells, e.g., the cells expressing a CD19 CAR described herein, are expanded in culture for 5 days, and the resulting cells exhibit higher proinflammatory cytokine production, e.g., IFN-γ and/or GM-CSF levels, as compared to the same cells expanded in culture for 9 days under the same culture conditions. In one instance, the cells, e.g., a CD19 CAR cell described herein, expanded for 5 days show at least a one, two, three, four, five, ten fold or more increase in pg/ml of proinflammatory cytokine production, e.g., IFN-γ and/or GM-CSF levels, as compared to the same cells expanded in culture for 9 days under the same culture conditions.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one instance, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one instance, the cells are expanded in the presence of IL-15 and/or IL-7 (e.g., IL-15 and IL-7).

In instances, methods described herein, e.g., CAR-expressing cell manufacturing methods, comprise removing T regulatory cells, e.g., CD25+ T cells, from a cell population, e.g., using an anti-CD25 antibody, or fragment thereof, or a CD25-binding ligand, IL-2. Methods of removing T regulatory cells, e.g., CD25+ T cells, from a cell population are described herein. In instances, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with
IL-15 and/or IL-7. For example, the cell population (e.g., that has previously contacted an anti-CD25 antibody, fragment thereof, or CD25-binding ligand) is expanded in the presence of IL-15 and/or IL-7.

In some instances a CAR-expressing cell described herein is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15, during the manufacturing of the CAR-expressing cell, e.g., ex vivo. In instances, a CAR-expressing cell described herein is contacted with a composition comprising a IL-15 polypeptide during the manufacturing of the CAR-expressing cell, e.g., ex vivo. In instances, a CAR-expressing cell described herein is contacted with a composition comprising a combination of both a IL-15 polypeptide and a IL-15 Ra polypeptide during the manufacturing of the CAR-expressing cell, e.g., ex vivo. In instances, a CAR-expressing cell described herein is contacted with a composition comprising hetIL-15 during the manufacturing of the CAR-expressing cell, e.g., ex vivo.

In one instance the CAR-expressing cell described herein is contacted with a composition comprising hetIL-15 during ex vivo expansion. In an instance, the CAR-expressing cell described herein is contacted with a composition comprising an IL-15 polypeptide during ex vivo expansion. In an instance, the CAR-expressing cell described herein is contacted with a composition comprising both an IL-15 polypeptide and an IL-15Ra polypeptide during ex vivo expansion. In one instance the contacting results in the survival and proliferation of a lymphocyte subpopulation, e.g., CD8+ T cells.

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

In other instances, the method of making disclosed herein further comprises contacting the population of immune effector cells with a nucleic acid encoding a telomerase subunit, e.g., hTERT. The the nucleic acid encoding the telomerase subunit can be DNA.

In some instances, a BTK inhibitor as described hereing, e.g., a compound of formula (I) is added during the CAR cell manufacturing process. According to the non-limiting theory herein, the BTK inhibitor can improve the quality of the population of cells produced. For instance, CAR-expressing cells are often produced from a cancer patient's own plasma apheresis sample, which can contain cancer cells, and the BTK inhibitor can alter signalling in those cancer cells (e.g., a BTK-expresssing cancer such as CLL or MCL), e.g., reducing their proliferation or increasing levels of apoptosis. As another example, the BTK inhibitor may alter signalling in the CAR-expressing cells (or immune effector cells before they express CAR), e.g., by inhibiting ITK in T cells. The BTK inhibitor may shift the balance of T cells from TH2 cells towards TH1 cells.

The BTK inhibitor such as a compound of formula (I) can be added to the reaction mixture in a level sufficient to inhibit its target, e.g., BTK. In some instances, the BTK inhibitor is added at a comcentration of about 0.1-0.2, 0.2-0.5, 0.5-1, 1-2, 2-5, or 5-10 µM. In some instances, the BTK inhibitor is a covalent inhibitor and a short pulse is sufficient to irreversibly inactivate the target while avoiding nonspecific toxicity. Consequently, the BTK inhibitor may be added for, e.g., 10-20, 20-30, 30-40, 40-60, or 60-120 minutes. The BTK inhibitor may also be added for longer periods of time, for instance if the BTK inhibitor has a noncovalent mode of action. Thus, the BTK inhibitor may be added for, e.g., 2-4, 4-6, 6-8, 8-12, 12-18, or 18-24 hours, or for 1-2, 2-3, 3-4, 4-6, 6-8, 8-10 days, or for the entire length of time the cells are being cultured. The BTK inhibitor may be added at various points during the manufacturing process, for example, after harvesting the cells, before stimulating with beads, after stimulating with beads, before transduction, after transduction, or before administration of the cells to the patient. In some instances, the BTK inhibitor is added after harvesting the cells or before stimulating, e.g., with beads. Before and after, in this context, can refer to, e.g., about 1, 5, 15, 30, 45, or 60 minutes before or after, or 1, 2, 3, 4, 5, or 6 hours before or after.

Once a CD 19 CAR is constructed, various assays can be used to evaluate the activity of the molecule, such as but not limited to, the ability to expand T cells following antigen stimulation, sustain T cell expansion in the absence of re-stimulation, and anti-cancer activities in appropriate in vitro and animal models. Assays to evaluate the effects of a CD 19 CAR are described in further detail below

Western blot analysis of CAR expression in primary T cells can be used to detect the presence of monomers and dimers. See, *e.g.,* Milone et al., Molecular Therapy 17(8): 1453-1464 (2009). Very briefly, T cells (1:1 mixture of CD4⁺ and CD8⁺ T cells) expressing the CARs are expanded *in vitro* for more than 10 days followed by lysis and SDS-PAGE under reducing conditions. CARs containing the full length TCR-ζ cytoplasmic domain and the endogenous TCR-ζ chain are detected by western blotting using an antibody to the TCR-ζ chain. The same T cell subsets are used for SDS-PAGE analysis under nonreducing conditions to permit evaluation of covalent dimer formation.

*In vitro* expansion of CAR⁺ T cells following antigen stimulation can be measured by flow cytometry. For example, a mixture of CD4⁺ and CD8⁺ T cells are stimulated with αCD3/αCD28 beads followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, *e.g.,* Milone et al., Molecular Therapy 17(8): 1453-1464 (2009). Alternatively, a mixture of CD4⁺ and CD8⁺ T cells are stimulated with αCD3/αCD28 coated magnetic beads on day 0, and transduced with CAR on day 1 using a bicistronic lentiviral vector expressing CAR along with eGFP using a 2A ribosomal skipping sequence. Cultures are re-stimulated with either CD19⁺ K562 cells (K562-CD19), wild-type K562 cells (K562 wild type) or K562 cells expressing hCD32 and 4-1BBL in the presence of anti-CD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, *e.g.,* Milone et al., Molecular Therapy 17(8): 1453-1464 (2009).

Sustained CAR⁺ T cell expansion in the absence of re-stimulation can also be measured. See, e.g., Milone et al., Molecular Therapy 17(8): 1453-1464 (2009). Briefly, mean T cell volume (fl) is measured on day 8 of culture using a Coulter Multisizer particle counter, a Nexcelom Cellometer Vision or Millipore Scepter, following stimulation with αCD3/αCD28 coated magnetic beads on day 0, and transduction with the indicated CAR on day 1.

Animal models can also be used to measure a CAR-expressing cell activity, e.g., as described in paragraph 698 of International Application WO2015/142675, filed March 13, 2015.Dose dependent CAR treatment response can be evaluated, e.g., as described in paragraph 699 of International Application WO2015/142675, filed March 13, 2015.

Assessment of cell proliferation and cytokine production has been previously described, e.g., as described in paragraph 700 of International Application WO2015/142675, filed March 13, 2015.

Cytotoxicity can be assessed by a standard ⁵¹Cr-release assay, e.g., as described in paragraph 701 of International Application WO2015/142675, filed March 13, 2015.

Imaging technologies can be used to evaluate specific trafficking and proliferation of CARs in tumor-bearing animal models, e.g., as described in paragraph 702 of International Application WO2015/142675, filed March 13, 2015.

Other assays, including those described in the Example section herein as well as those that are known in the art can also be used to evaluate the CD 19 CAR constructs of the disclosure.

Alternatively, or in combination to the methods disclosed herein, methods and compositions for one or more of: detection and/or quantification of CAR-expressing cells (e.g., in vitro or in vivo (e.g., clinical monitoring)); immune cell expansion and/or activation; and/or CAR-specific selection, that involve the use of a CAR ligand, are disclosed. In one exemplary instance, the CAR ligand is an antibody that binds to the CAR molecule, e.g., binds to the extracellular antigen binding domain of CAR (e.g., an antibody that binds to the antigen binding domain, e.g., an anti-idiotypic antibody; or an antibody that binds to a constant region of the extracellular binding domain). In other instances, the CAR ligand is a CAR antigen molecule (e.g., a CAR antigen molecule as described herein).

In one aspect, a method for detecting and/or quantifying CAR-expressing cells is disclosed. For example, the CAR ligand can be used to detect and/or quantify CAR-expressing cells in vitro or in vivo (e.g., clinical monitoring of CAR-expressing cells in a patient, or dosing a patient). The method includes:
providing the CAR ligand (optionally, a labelled CAR ligand, e.g., a CAR ligand that includes a tag, a bead, a radioactive or fluorescent label);
acquiring the CAR-expressing cell (e.g., acquiring a sample containing CAR-expressing cells, such as a manufacturing sample or a clinical sample);
contacting the CAR-expressing cell with the CAR ligand under conditions where binding occurs, thereby detecting the level (e.g., amount) of the CAR-expressing cells present. Binding of the CAR-expressing cell with the CAR ligand can be detected using standard techniques such as FACS, ELISA and the like.

In another aspect, a method of expanding and/or activating cells (e.g., immune effector cells) is disclosed. The method includes:
providing a CAR-expressing cell (e.g., a first CAR-expressing cell or a transiently expressing CAR cell);
contacting said CAR-expressing cell with a CAR ligand, e.g., a CAR ligand as described herein), under conditions where immune cell expansion and/or proliferation occurs, thereby producing the activated and/or expanded cell population.

In certain instances, the CAR ligand is present on a substrate (e.g., is immobilized or attached to a substrate, e.g., a non-naturally occurring substrate). In some instances, the substrate is a non-cellular substrate. The non-cellular substrate can be a solid support chosen from, e.g., a plate (e.g., a microtiter plate), a membrane (e.g., a nitrocellulose membrane), a matrix, a chip or a bead. In instances, the CAR ligand is present in the substrate (e.g., on the substrate surface). The CAR ligand can be immobilized, attached, or associated covalently or non-covalently (e.g., cross-linked) to the substrate. In one instance, the CAR ligand is attached (e.g., covalently attached) to a bead. In the aforesaid instances, the immune cell population can be expanded in vitro or ex vivo. The method can further include culturing the population of immune cells in the presence of the ligand of the CAR molecule, e.g., using any of the methods described herein.

In other instances, the method of expanding and/or activating the cells further comprises addition of a second stimulatory molecule, e.g., CD28. For example, the CAR ligand and the second stimulatory molecule can be immobilized to a substrate, e.g., one or more beads, thereby providing increased cell expansion and/or activation.

In yet another aspect, a method for selecting or enriching for a CAR expressing cell is provided. The method includes contacting the CAR expressing cell with a CAR ligand as described herein; and selecting the cell on the basis of binding of the CAR ligand.

In yet other instances, a method for depleting, reducing and/or killing a CAR expressing cell is provided. The method includes contacting the CAR expressing cell with a CAR ligand as described herein; and targeting the cell on the basis of binding of the CAR ligand, thereby reducing the number, and/or killing, the CAR-expressing cell. In one instance, the CAR ligand is coupled to a toxic agent (e.g., a toxin or a cell ablative drug). In another instance, the anti-idiotypic antibody can cause effector cell activity, e.g., ADCC or ADC activities.

Exemplary anti-CAR antibodies that can be used in the methods disclosed herein are described, e.g., in WO 2014/190273 and by Jena et al., "Chimeric Antigen Receptor (CAR)-Specific Monoclonal Antibody to Detect CD19-Specific T cells in Clinical Trials", PLOS March 2013 8:3 e57838.

In some aspects and instances, the compositions and methods herein are optimized for a specific subset of T cells, e.g., as described in US Serial No. PCT/US2015/043219 filed July 31, 2015 . In some instances, the optimized subsets of T cells display an enhanced persistence compared to a control T cell, e.g., a T cell of a different type (e.g., CD8+ or CD4+) expressing the same construct.

In some instances, a CD4+ T cell comprises a CAR described herein, which CAR comprises an intracellular signaling domain suitable for (e.g., optimized for, e.g., leading to enhanced persistence in) a CD4+ T cell, e.g., an ICOS domain. In some instances, a CD8+ T cell comprises a CAR described herein, which CAR comprises an intracellular signaling domain suitable for (e.g., optimized for, e.g., leading to enhanced persistence of) a CD8+ T cell, e.g., a 4-1BB domain, a CD28 domain, or another costimulatory domain other than an ICOS domain. In some instances, the CAR described herein comprises an antigen binding domain described herein, e.g., a CAR comprising an antigen binding domain.

In an aspect, described herein is a method of treating a subject, e.g., a subject having cancer. The method includes administering to said subject, an effective amount of:
1) a CD4+ T cell comprising a CAR (the CARCD4+) comprising:
   an antigen binding domain, e.g., an antigen binding domain described herein;
   a transmembrane domain; and
   an intracellular signaling domain, e.g., a first costimulatory domain, e.g., an ICOS domain; and
2) a CD8+ T cell comprising a CAR (the CARCD8+) comprising:
   an antigen binding domain, e.g., an antigen binding domain described herein;
   a transmembrane domain; and
   an intracellular signaling domain, e.g., a second costimulatory domain, e.g., a 4-1BB domain, a CD28 domain, or another costimulatory domain other than an ICOS domain;
   wherein the CARCD4+ and the CARCD8+ differ from one another.

Optionally, the method further includes administering:
3) a second CD8+ T cell comprising a CAR (the second CARCD8+) comprising:
an antigen binding domain, e.g., an antigen binding domain described herein;
a transmembrane domain; and
an intracellular signaling domain, wherein the second CARCD8+ comprises an intracellular signaling domain, e.g., a costimulatory signaling domain, not present on the CARCD8+, and, optionally, does not comprise an ICOS signaling domain.

Any of the methods described herein can further include administration of a BTK inhibitor as described herein.

### Biopolymer delivery methods

In some instances, one or more CAR-expressing cells as disclosed herein, optionally in combination with a BTK inhibitor, e.g., a compound of formula (I), can be administered or delivered to the subject via a biopolymer scaffold, e.g., a biopolymer implant. Biopolymer scaffolds can support or enhance the delivery, expansion, and/or dispersion of the CAR-expressing cells described herein. A biopolymer scaffold comprises a biocompatible (e.g., does not substantially induce an inflammatory or immune response) and/or a biodegradable polymer that can be naturally occurring or synthetic. Exemplary biopolymers are described, e.g., in paragraphs 1004-1006 of International Application WO2015/142675, filed March 13,2015.

### Methods of Manufacture/Production

In some instances, the methods disclosed herein further include administering a T cell depleting agent after treatment with the cell (e.g., an immune effector cell as described herein, e.g., an immune effector cell expressing CAR described herein), thereby reducing (e.g., depleting) the CAR-expressing cells (e.g., the CD19CAR-expressing cells). Such T cell depleting agents can be used to effectively deplete CAR-expressing cells (e.g., CD19CAR-expressing cells) to mitigate toxicity. In some instances, the CAR-expressing cells were manufactured according to a method herein, e.g., assayed (e.g., before or after transfection or transduction) according to a method herein.

In some instances, the T cell depleting agent is administered one, two, three, four, or five weeks after administration of the cell, e.g., the population of immune effector cells, described herein.

In one instance, the T cell depleting agent is an agent that depletes CAR-expressing cells, e.g., by inducing antibody dependent cell-mediated cytotoxicity (ADCC) and/or complement-induced cell death. For example, CAR-expressing cells described herein may also express an antigen (e.g., a target antigen) that is recognized by molecules capable of inducing cell death, e.g., ADCC or complement-induced cell death. For example, CAR expressing cells described herein may also express a target protein (e.g., a receptor) capable of being targeted by an antibody or antibody fragment. Examples of such target proteins include, but are not limited to, EpCAM, VEGFR, integrins (e.g., integrins ανββ, α4, αI3/4β3, α4β7, α5β1, ανβ3, αv), members of the TNF receptor superfamily (e.g., TRAIL-R1 , TRAIL-R2), PDGF Receptor, interferon receptor, folate receptor, GPNMB, ICAM-1, HLA-DR, CEA, CA-125, MUC1, TAG-72, IL-6 receptor, 5T4, GD2, GD3, CD2, CD3, CD4, CD5, CD11 , CD11a/LFA-1, CD15, CD18/ITGB2, CD19, CD20, CD22, CD23/lgE Receptor, CD25, CD28, CD30, CD33, CD38, CD40, CD41 , CD44, CD51 , CD52, CD62L, CD74, CD80, CD125, CD147/basigin, CD152/CTLA-4, CD154/CD40L, CD195/CCR5, CD319/SLAMF7, and EGFR, and truncated versions thereof (e.g., versions preserving one or more extracellular epitopes but lacking one or more regions within the cytoplasmic domain).

In some instances, the CAR expressing cell co-expresses the CAR and the target protein, e.g., naturally expresses the target protein or is engineered to express the target protein. For example, the cell, e.g., the population of immune effector cells, can include a nucleic acid (e.g., vector) comprising the CAR nucleic acid (e.g., a CAR nucleic acid as described herein) and a nucleic acid encoding the target protein.

In one instance, the T cell depleting agent is a CD52 inhibitor, e.g., an anti-CD52 antibody molecule, e.g., alemtuzumab.

In other instances, the cell, e.g., the population of immune effector cells, expresses a CAR molecule as described herein (e.g., CD19CAR) and the target protein recognized by the T cell depleting agent. In one instance, the target protein is CD20. In instances where the target protein is CD20, the T cell depleting agent is an anti-CD20 antibody, e.g., rituximab.

In further instances of any of the aforesaid methods, the methods further include transplanting a cell, e.g., a hematopoietic stem cell, or a bone marrow, into the mammal.
In another aspect, the disclosure features a method of conditioning a mammal prior to cell transplantation. The method includes administering to the mammal an effective amount of the cell comprising a CAR nucleic acid or polypeptide, e.g., a CD19 CAR nucleic acid or polypeptide. In some instances, the cell transplantation is a stem cell transplantation, e.g., a hematopoietic stem cell transplantation, or a bone marrow transplantation. In other instances, conditioning a subject prior to cell transplantation includes reducing the number of target-expressing cells in a subject, e.g., CD19-expressing normal cells or CD19-expressing cancer cells.

### BTK Inhibitor

In some embodiments, the BTK inhibitor is a compound of formula (I) as described herein, or a pharmaceutically acceptable salt thereof.

Although many of the compounds herein, e.g., compounds of formula (I), are often referred to as BTK inhibitors, it is understood that in some contexts a compound of formula (I) can have one or more activities other than inhibition of BTK. For instance, in some cases the relevant activity is inhibition of a kinase with homology to BTK, such as ITK. This non-BTK inhibition activity may be in addition to or in place of a BTK inhibition activity.

### Methods of synthesizing Amino-pyrimidines

Agents of the disclosure, i.e. compounds in accordance to the definition of formula (I), may be prepared by a reaction sequence involving an alkylation of 4-amino-6-chloro-pyrimidin-5-ol 1 with an alkyl halide (2) using an appropriate base, Suzuki coupling with a boronic ester (4) using an appropriate palladium catalyst, such as bis(triphenylphosphine)-palladium(II) dichloride, deprotection using an appropriate acid, such as TFA or HCl to form intermediate (6), followed by amide formation of the ammonium salt or the free amine with an acid using an appropriate coupling reagent, such as T3P, and an appropriate base, such as DIPEA, or with an acid chloride using an appropriate base, such as DIPEA, to yield compound (7) as shown in **Scheme 1** below:

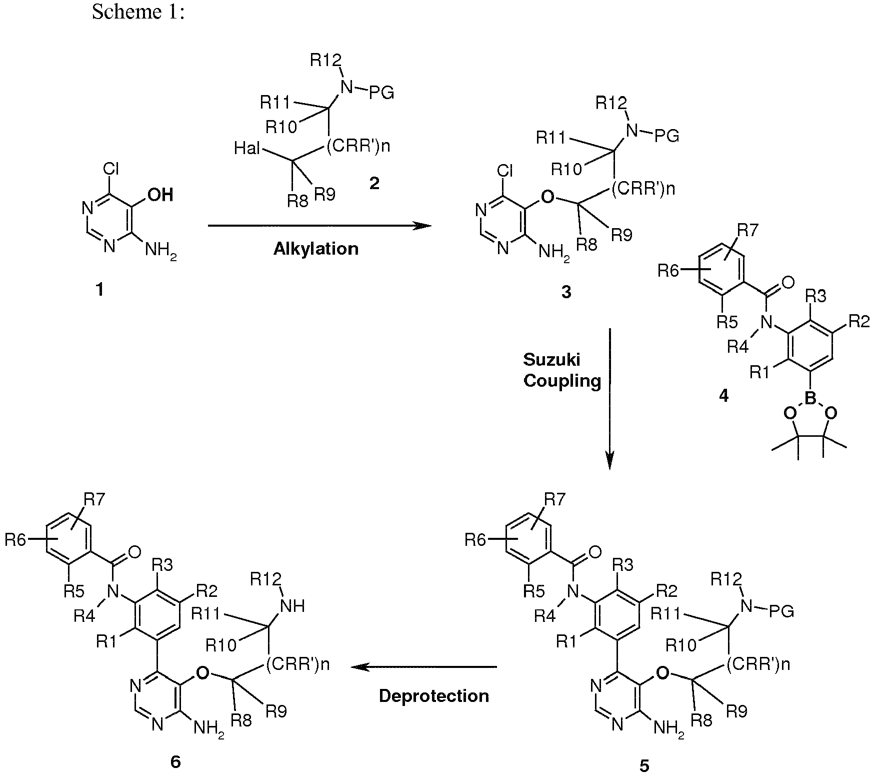

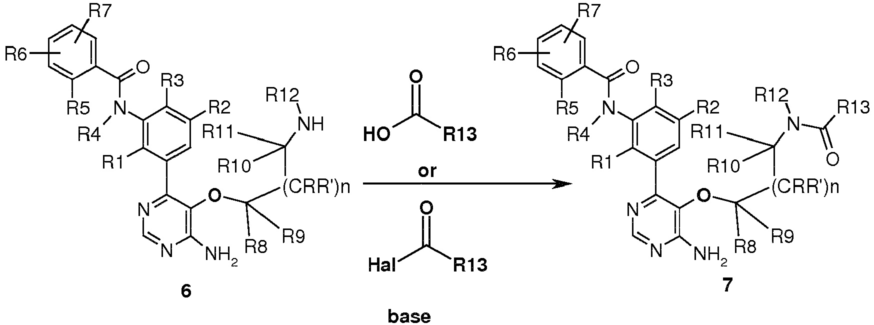

Compounds of the disclosure may also be prepared by an alternative reaction sequence (shown below) comprising the steps of reacting the amino hydroxypyrimidine 1 with the hydroxyl amino-alkyl-derivative 2' in a Mitsunobu reaction to furnish intermediate 3, which intermediate 3 is then reacted via a Suzuki-coupling to yield intermediate 5, which is then deprotected to yield intermediate 6, which is then amidated with an acid or acid chloride to yield the final product 7 as already described in scheme 1.

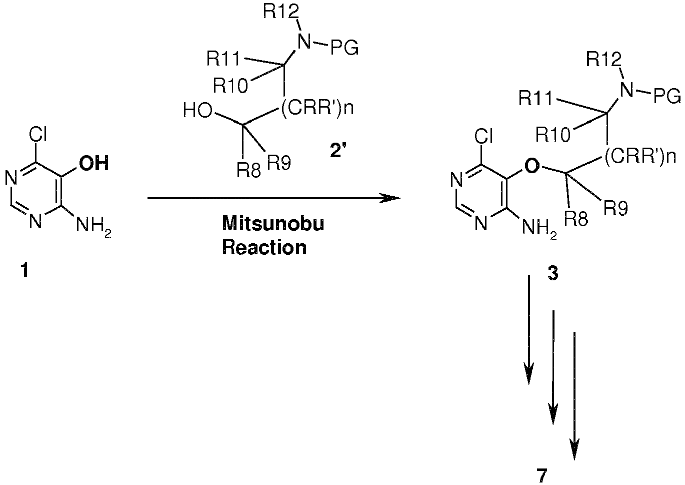

### Abbreviations:

- BISPIN:: Bis(pinacolato)diboron
- Boc: t-Butyloxycarbonyl
- DCE:: Dichloroethane
- DCM:: Dichloromethane
- DIAD:: Diisopropyl azodicarboxylate
- DIPEA:: *N*-Diisopropylethylamine
- DME:: 1,2-Dimethoxyethane
- DMF:: *N*,*N*-Dimethylformamide
- DMSO:: Dimethyl sulfoxide
- EtOAc:: Ethyl acetate
- EtOH:: Ethanol
- hr:: Hour
- M:: Molar
- MeOH:: Methanol
- min:: Minute
- NaHMDS:: Sodium bis(trimethylsilyl)amide
- rt:: Retention time
- RT:: Room temperature
- SFC:: Supercritical fluid chromatography
- Smopex-301:: Polymer supported triphenylphosphine
- SPE:: Solid phase extraction
- TBAF:: Tetrabutylammonium fluoride
- TBDPS:: *tert*-Butyldiphylsilyl
- TBHP:: *tert*-Butyl hydroperoxide
- TBME:: *tert*-Butyl methyl ether
- TEA:: Triethylamine
- TFA:: Trifluoroacetic acid
- THF:: Tetrahydrofuran
- T3P:: Propylphosphonic anhydride
- XPhos:: 2-Dicyclohexylphosphino-2',4',6'-triisopropylbiphenyl

¹H NMR spectra were recorded on a Bruker 400 MHz NMR spectrometer. Significant peaks are tabulated in the order: multiplicity (s, singlet; d, doublet; t, triplet; q, quartet; m, multiplet; br, broad; v, very) and number of protons. Electron Spray Ionization (ESI) mass spectra were recorded on a Waters Acquity SQD mass spectrometer. Mass spectrometry results are reported as the ratio of mass over charge.

### UPLC-MS Method:

Waters Acquity UPLC instrument equipped with PDA detector, Waters Acquity SQD mass spectrometer and Waters Acquity HSS T3 1.8 µm 2.1 x 50 mm column. Peak detection is reported at full scan 210 - 450 nM. Mass spectrometry results are reported as the ratio of mass over charge.
Eluent A: Water + 0.05% formic acid + 3.75 mM ammonium acetate.
Eluent B: Acetonitrile + 0.04% formic acid.
Flow: 1 mL/min

The gradient may be, for example: At time 0.00 minutes, percent Eluant A = 95% and percent Eluent B = 5%; at time 1.40 minutes, percent Eluant A = 2% and percent Eluent B = 98%; at time 1.80 minutes, percent Eluant A = 2% and percent Eluent B = 98%; at time 1.90 minutes, percent Eluant A = 95% and percent Eluent B = 5%; and at time 2.00 minutes, percent Eluant A = 95% and percent Eluent B = 5%.

### Isotopically labeled forms

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, phosphorous, fluorine, and chlorine, such as ²H, ³H, ¹¹C, ¹³C, ¹⁴C, ¹⁵N, ¹⁸F ³¹P, ³²P, ³⁵S, ³⁶Cl, ¹²⁵I respectively. The disclosure includes various isotopically labeled compounds as defined herein, for example those into which radioactive isotopes, such as ³H and ¹⁴C, or those into which non-radioactive isotopes, such as ²H and ¹³C are present. Such isotopically labeled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F or labeled compound may be particularly desirable for PET or SPECT studies. Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of a compound of the formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this disclosure is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

Pharmaceutically acceptable solvates in accordance with the invention include those wherein the solvent of crystallization may be isotopically substituted, *e.g.* D₂O, d₆-acetone, d₆-DMSO.

### Solvates, Hydrates, Polymorphs, Crystallization

The compounds of the present disclosure, including their salts, can also be obtained in the form of their hydrates, or include other solvents used for their crystallization. The compounds of the present disclosure may inherently or by design form solvates with pharmaceutically acceptable solvents (including water); therefore, it is intended that the disclosure embrace both solvated and unsolvated forms. The term "solvate" refers to a molecular complex of a compound of the present disclosure (including pharmaceutically acceptable salts thereof) with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water.

The compounds of the present disclosure, including salts, hydrates and solvates thereof, may inherently or by design form polymorphs.

Compounds of the disclosure, i.e. compounds of formula (I) that contain groups capable of acting as donors and/or acceptors for hydrogen bonds may be capable of forming co-crystals with suitable co-crystal formers. These co-crystals may be prepared from compounds of formula (I) by known co-crystal forming procedures. Such procedures include grinding, heating, co-subliming, co-melting, or contacting in solution compounds of formula (I) with the co-crystal former under crystallization conditions and isolating co-crystals thereby formed. Suitable co-crystal formers include those described in WO 2004/078163. Hence the disclosure further provides co-crystals comprising a compound of formula (I).

### Chirality

Any asymmetric atom (e.g., carbon or the like) of the compound(s) of the present disclosure can be present in racemic or enantiomerically enriched, for example the (*R*)-, (*S*)- or (*R,S*)- configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)- configuration. Substituents at atoms with unsaturated double bonds may, if possible, be present in *cis-*(Z)- or *trans-* (*E*)- form.

Accordingly, as used herein a compound of the present disclosure can be in the form of one of the possible isomers, rotamers, atropisomers, tautomers or mixtures thereof, for example, as substantially pure geometric (*cis* or *trans*) isomers, diastereomers, optical isomers (antipodes), racemates or mixtures thereof.

Any resulting mixtures of isomers can be separated on the basis of the physicochemical differences of the constituents, into the pure or substantially pure geometric or optical isomers, diastereomers, racemates, for example, by chromatography and/or fractional crystallization.

Any resulting racemates of final products or intermediates can be resolved into the optical antipodes by known methods, *e.g.,* by separation of the diastereomeric salts thereof, obtained with an optically active acid or base, and liberating the optically active acidic or basic compound. In particular, a basic moiety may thus be employed to resolve the compounds of the present disclosure into their optical antipodes, *e.g.,* by fractional crystallization of a salt formed with an optically active acid, *e.g.,* tartaric acid, dibenzoyl tartaric acid, diacetyl tartaric acid, di-*O*, *O*'-*p*-toluoyl tartaric acid, mandelic acid, malic acid or camphor-10-sulfonic acid. Racemic products can also be resolved by chiral chromatography, *e.g.*, high pressure liquid chromatography (HPLC) using a chiral adsorbent.

### Salts

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present disclosure can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

In one embodiment, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In an embodiment, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In one embodiment, the BTK inhibitor is ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of ibrutinib are administered.

The structure of ibrutinib (1-[(3*R*)-3-[4-Amino-3-(4-phenoxyphenyl)-1*H*-pyrazolo[3,4-d]pyrimidin-1-yl]piperidin-1-yl]prop-2-en-1-one) is shown below.

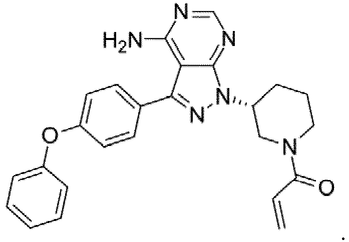

In instances, a CAR-expressing cell described herein, in combination with a BTK inhibitor such as a compound of formula (I), is administered to a subject in combination with a phosphoinositide 3-kinase (PI3K) inhibitor (e.g., a PI3K inhibitor described herein, e.g., idelalisib or duvelisib) and/or rituximab. In instances, a CAR-expressing cell described herein is administered to a subject in combination with idelalisib and rituximab. In instances, a CAR-expressing cell described herein is administered to a subject in combination with duvelisib and rituximab. Idelalisib (also called GS-1101 or CAL-101; Gilead) is a small molecule that blocks the delta isoform of PI3K. The structure of idelalisib (5-Fluoro-3-phenyl-2-[(1*S*)-1-(7*H*-purin-6-ylamino)propyl]-4(3*H*)-quinazolinone) is shown below.

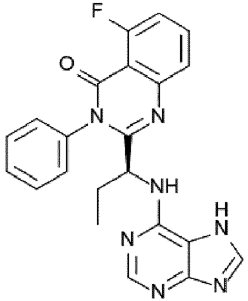

Duvelisib is a small molecule that blocks PI3K-δ,γ. The structure of duvelisib (8-Chloro-2-phenyl-3-[(1S)-1-(9H-purin-6-ylamino)ethyl]-1(2H)-isoquinolinone) is shown below.

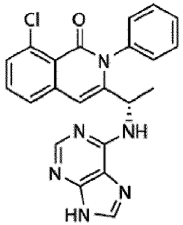

In embodiments, the subject has CLL. In embodiments, the subject has relapsed CLL, e.g., the subject has previously been administered a cancer therapy (e.g., previously been administered an anti-CD20 antibody or previously been administered ibrutinib). For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region *(IgV_{H})* gene. In other embodiments, the subject does not comprise a leukemic cell comprising a mutation in the immunoglobulin heavy-chain variable-region *(IgV_{H})* gene. In embodiments, the subject has a deletion in the long arm of chromosome 11 (del(11q)). In other embodiments, the subject does not have a del(11q). In embodiments, idelalisib is administered at a dosage of about 100-400 mg (e.g., 100-125, 125-150, 150-175, 175-200, 200-225, 225-250, 250-275, 275-300, 325-350, 350-375, or 375-400 mg), e.g., BID. In embodiments, duvelisib is administered at a dosage of about 15-100 mg (e.g., about 15-25, 25-50, 50-75, or 75-100 mg), e.g., twice a day. In embodiments, rituximab is administered at a dosage of about 350-550 mg/m² (e.g., 350-375, 375-400, 400-425, 425-450, 450-475, or 475-500 mg/m²), e.g., intravenously.

In instances, a CAR-expressing cell described herein, in combination with a BTK inhibitor such as a compound of formula (I), is administered to a subject in combination with an anaplastic lymphoma kinase (ALK) inhibitor. Exemplary ALK kinase inhibitors include but are not limited to crizotinib (Pfizer), ceritinib (Novartis), alectinib (Chugai), brigatinib (also called AP26113; Ariad), entrectinib (Ignyta), PF-06463922 (Pfizer), TSR-011 (Tesaro) (see, e.g., Clinical Trial Identifier No. NCT02048488), CEP-37440 (Teva), and X-396 (Xcovery). In some instances, the subject has a solid cancer, e.g., a solid cancer described herein, e.g., lung cancer.

The chemical name of crizotinib is 3-[(1*R*)-1-(2,6-dichloro-3-fluorophenyl)ethoxy]-5-(1-piperidin-4-ylpyrazol-4-yl)pyridin-2-amine. The chemical name of ceritinib is 5-Chloro-*N*²-[2-isopropoxy-5-methyl-4-(4-piperidinyl)phenyl]-*N*⁴-[2-(isopropylsulfonyl)phenyl]-2,4-pyrimidinediamine. The chemical name of alectinib is 9-ethyl-6,6-dimethyl-8-(4-morpholinopiperidin-1-yl)-11-oxo-6,11-dihydro-5H-benzo[b]carbazole-3-carbonitrile. The chemical name of brigatinib is 5-Chloro-N²-{4-[4-(dimethylamino)-1-piperidinyl]-2-methoxyphenyl}-N⁴-[2-(dimethylphosphoryl)phenyl]-2,4-pyrimidinediamine. The chemical name of entrectinib is N-(5-(3,5-difluorobenzyl)-1H-indazol-3-yl)-4-(4-methylpiperazin-1-yl)-2-((tetrahydro-2H-pyran-4-yl)amino)benzamide. The chemical name of PF-06463922 is (10R)-7-Amino-12-fluoro-2,10,16-trimethyl-15-oxo-10,15,16,17-tetrahydro-2H-8,4-(metheno)pyrazolo[4,3-h][2,5,11]-benzoxadiazacyclotetradecine-3-carbonitrile. The chemical structure of CEP-37440 is (S)-2-((5-chloro-2-((6-(4-(2-hydroxyethyl)piperazin-1-yl)-1-methoxy-6,7,8,9-tetrahydro-5H-benzo[7]annulen-2-yl)amino)pyrimidin-4-yl)amino)-N-methylbenzamide. The chemical name of X-396 is (R)-6-amino-5-(1-(2,6-dichloro-3-fluorophenyl)ethoxy)-N-(4-(4-methylpiperazine-1-carbonyl)phenyl)pyridazine-3-carboxamide.

In instances, a CAR-expressing cell described herein, in combination with a BTK inhibitor such as a compound of formula (I), is administered to a subject in combination with an indoleamine 2,3-dioxygenase (IDO) inhibitor. IDO is an enzyme that catalyzes the degradation of the amino acid, L-tryptophan, to kynurenine. Many cancers overexpress IDO, e.g., prostatic, colorectal, pancreatic, cervical, gastric, ovarian, head, and lung cancer. pDCs, macrophages, and dendritic cells (DCs) can express IDO. Without being bound by theory, it is thought that a decrease in L-tryptophan (e.g., catalyzed by IDO) results in an immunosuppressive milieu by inducing T-cell anergy and apoptosis. Thus, without being bound by theory, it is thought that an IDO inhibitor can enhance the efficacy of a CAR-expressing cell described herein, e.g., by decreasing the suppression or death of a CAR-expressing immune cell. In instances, the subject has a solid tumor, e.g., a solid tumor described herein, e.g., prostatic, colorectal, pancreatic, cervical, gastric, ovarian, head, or lung cancer. Exemplary inhibitors of IDO include but are not limited to 1-methyl-tryptophan, indoximod (NewLink Genetics) (see, e.g., Clinical Trial Identifier Nos. NCT01191216; NCT01792050), and INCB024360 (Incyte Corp.) (see, e.g., Clinical Trial Identifier Nos. NCT01604889; NCT01685255)

In instances, a CAR-expressing cell described herein, optionally in combination with a BTK inhibitor such as a compound of formula (I), is administered to a subject in combination with a modulator of myeloid-derived suppressor cells (MDSCs). MDSCs accumulate in the periphery and at the tumor site of many solid tumors. These cells suppress T cell responses, thereby hindering the efficacy of CAR-expressing cell therapy. Without being bound by theory, it is thought that administration of a MDSC modulator enhances the efficacy of a CAR-expressing cell described herein. In an instance, the subject has a solid tumor, e.g., a solid tumor described herein, e.g., glioblastoma. Exemplary modulators of MDSCs include but are not limited to MCS110 and BLZ945. MCS110 is a monoclonal antibody (mAb) against macrophage colony-stimulating factor (M-CSF). See, e.g., Clinical Trial Identifier No. NCT00757757. BLZ945 is a small molecule inhibitor of colony stimulating factor 1 receptor (CSF1R). See, e.g., Pyonteck et al. Nat. Med. 19(2013):1264-72. The structure of BLZ945 is shown below.

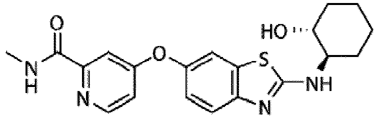

In some instances , a CAR-expressing cell described herein, in combination with a BTK inhibitor such as a compound of formula (I), is administered to a subject in combination with a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15 (Admune Therapeutics, LLC). hetIL-15 is a heterodimeric non-covalent complex of IL-15 and IL-15Ra. hetIL-15 is described in, e.g., U.S. 8,124,084, U.S. 2012/0177598, U.S. 2009/0082299, U.S. 2012/0141413, and U.S. 2011/0081311. In instances, het-IL-15 is administered subcutaneously. In instances, the subject has a cancer, e.g., solid cancer, e.g., melanoma or colon cancer. In instances, the subject has a metastatic cancer.

### Therapeutic Application

### CD19 Associated Diseases and/or Disorders

In embodiments, the BTK inhibitor, e.g., a compound of formula (I), is administered to a subject that has CLL, mantle cell lymphoma (MCL), or small lymphocytic lymphoma (SLL). For example, the subject to whom the BTK inhibitor is administered has a deletion in the short arm of chromosome 17 (del(17p), e.g., in a leukemic cell). In other examples, the subject to whom the BTK inhibitor is administered does not have a del(17p). In embodiments, the subject to whom the BTK inhibitor is administered has relapsed CLL or SLL, e.g., the subject has previously been administered a cancer therapy (e.g., previously been administered one, two, three, or four prior cancer therapies). In embodiments, the subject to whom the BTK inhibitor is administered has refractory CLL or SLL. In other embodiments, the subject to whom the BTK inhibitor is administered has follicular lymphoma, e.g., relapse or refractory follicular lymphoma.

In one aspect, the invention relates to treating a disease associated with CD19 expression. In one aspect, the invention relates to treating a disease wherein part of the tumor is negative for CD 19 and part of the tumor is positive for CD19. For example, the CAR of the disclosure is useful for treating subjects that have undergone treatment for a disease associated with elevated expression of CD19, wherein the subject that has undergone treatment for elevated levels of CD19 exhibits a disease associated with elevated levels of CD19.

The therapies described herein can be used to treat, e.g., subjects who respond to a BTK inhibitor such as a compound of formula (I) (e.g., partial response or complete response) or subjects who do not (e.g., non-responders or relapsers). Without wishing to be bound by theory, a number of patients undergoing treatment with BTK inhibitors such as ibrutinib may show a reduced response to the treatment (e.g., are partial or non-responders to the treatment, or relapse during treatment). According, administration of the CAR-therapies disclosed herein, in combination with BTK inhibitors such as compounds of formula (I), can result in beneficial effects.

Exemplary therapeutic regimens for these subjects are described below.

In some cases, when the subject is a non-responder or relapser to a BTK inhibitor such as a compound of formula (I), the BTK inhibitor is withdrawn and CAR therapy is administered. In other cases, when the subject does not respond to a BTK inhibitor such as a compound of formula (I), the BTK inhibitor therapy is continued and CAR therapy is added to the regimen. This use is supported, e.g., by experiments in Example 42 herein which indicate that CAR therapy is effective as a monotherapy in ibrutinib-resistant cells. Without wishing to be bound by theory, continuing BTK inhibitor therapy can improve the efficacy of the CAR therapy, e.g., by increasing the number of CAR-expressing cells in the bloodstream (see Example 42 herein).

Without being bound by theory, a subject who is a non-responder or relapser to a BTK inhibitor (e.g., ibrutinib or a compound of formula (I)) can be non-responsive for at least two reasons: the subjects may have a mutation in the drug target (e.g., BTK, e.g., a C481S mutation) that prevents target inhibition, or can have alterations in other pathways that can drive proliferation even when the target is adequately inhibited (e.g., a mutation in PLCy, such as an activating mutation in PLCγ resulting in constitutive BTK-independent cell signaling). The treatment can be altered depending on the reason for non-responsiveness. For instance, in the first situation (in some instances), if the subjects has (or is identified as having) a mutation that prevents the BTK inhibitor from inhibiting its target, a second BTK inhibitor can be substituted for (or administered in combination with) the first BTK inhibitor. More specifically, in some instances where the patient has (or is identified as having) a mutation that prevents the first BTK inhibitor (e.g., ibrutinib, GDC-0834, RN-486, CGI-560, CGI-1764, HM-71224, CC-292, ONO-4059, CNX-774, or LFM-A13) from inhibiting BTK, a second BTK inhibitor, e.g., a BTK inhibitor described herein such as a compound of formula (I) can be substituted for the first BTK inhibitor. Without wishing to be bound by theory, the second kinase inhibitor may act on, e.g., bind to, a region of BTK that is not disrupted by the mutation, and therefore the subject is sensitive to the second BTK inhibitor. In other instances, the original BTK inhibitor such as ibrutinib is maintained. According to the non-limiting theory here, the original kinase inhibitor may have useful activity on the CAR-expressing cells, e.g., promoting a TH1 phenotype, promoting proliferation, or otherwise increasing levels or activity of the cells.

As noted above, in some cases a subject is non-responsive because the subject has an alteration (e.g., a mutation) in another pathway that can drive proliferation even when the target is adequately inhibited. Accordingly, if the subject has (or is identified has having) an alteration in a pathway that makes the first BTK inhibitor's activity ineffectual, the BTK inhibitor therapy can be maintained. Without wishing to be bound by theory, the BTK inhibitor such as ibrutinib or a compound of formula (I) can promote useful biological changes in the cancer cells even if the BTK inhibitor alone is not sufficient to slow proliferation. For instance, the kinase inhibitor can be sufficient to mobilize cancer cells out of the lymph nodes, making them more vulnerable to the CAR therapy.

Turning to subjects who respond to a BTK inhibitor such as a compound of formula (I), various therapeutic regimens are now described. In some instances, when a subject is (or is identified as being) a complete responder to the BTK inhibitor, the subject is not administered a CAR therapy during the period of complete response. In other instances, when a subject is (or is identified as being) a complete responder to the BTK inhibitor, the subject is administered a CAR therapy during the period of complete response. In an instance, after the CAR therapy, the subject experiences a prolonged response or delayed relapse (e.g., compared to the expected course of disease when treated without CAR therapy). For instance, MCL treated with ibrutinib monotherapy has a median duration of response of about 17.5 months.

In some instances, when a subject is (or is identified as being) a partial responder to the BTK inhibitor such as a compound of formula (I), the subject is not administered a CAR therapy during the period of partial response. In other instances, when a subject is (or is identified as being) a partial responder to the BTK inhibitor, the subject is administered a CAR therapy during the period of partial response. In an instance, after the CAR therapy, the subject experiences a complete response and/or prolonged response or delayed relapse (e.g., compared to the expected course of disease when treated without CAR therapy).

In some instances, when a subject has (or is identified as having) stable disease after the beginning of treatment with the BTK inhibitor such as a compound of formula (I), the subject is not administered a CAR therapy during the period of stable disease. In other instances, when a subject has (or is identified as having) stable disease after the beginning of treatment with the BTK inhibitor, the subject is administered a CAR therapy during the period of stable disease. In an instance, after the CAR therapy, the subject experiences a partial response, a complete response and/or prolonged response or delayed relapse (e.g., compared to the expected course of disease when treated without CAR therapy).

In some instances, when a subject has (or is identified as having) progressive disease after the beginning of treatment with the BTK inhibitor such as a compound of formula (I), the subject is administered a CAR therapy during the period of progressive disease. In an instance, after the CAR therapy, the subject experiences stable disease, a partial response, a complete response and/or prolonged response or delayed relapse (e.g., compared to the expected course of disease when treated without CAR therapy).

Thus, one or more disease assessment steps can be performed before or during treatment, to determine which course of treatment is suitable for a given patient. For instance, a subject can be administered a BTK inhibitor such as a compound of formula (I) as a first line therapy. Then, after a period of time (e.g., 1 or 2 months but also 2 weeks, 3 weeks, 1 month, 1.5 months, 2 months, 3 months, 4 months, 6 months, 9 months, 12 months, 15 months, or 18 months) the patient's response can be assessed. If the assessment shows that the subject is a complete responder, in some instances CAR therapy is not administered, e.g., as described above. If the assessment shows that the subject is a partial responder or has stable disease, in some instances CAR therapy is administered in combination with the kinase inhibitor e.g., as described above. If the assessment shows that the subject is a non-responder or relapser, in some instances CAR therapy is administered in combination with the BTK inhibitor or a second BTK inhibitor, e.g., as described above. In some instances, the BTK inhibitor controls the disease while a CAR-expressing cell is being manufactured, e.g., while the patient's own T cells are being engineered to express a CAR and/or other factors.

Clinical standards for classifying a patient's responder status or relapser status are known in the art. As an example, for malignant lymphoma, standardized response criteria are described in Cheson et al, J Clin Oncol 17:1244 (1999) and Cheson et al., "Revised Response Criteria for Malignant Lymphoma", J Clin Oncol 25:579-586 (2007). Accordingly, in some instances, a subject is considered a complete responder, partial responder, having stable disease, a non-responder, or a relapser according to Cheson criteria or modified Cheson criteria. Criteria for classifying other hematological malignancies are known in the art.

According to the criteria in Table 2 of Cheson 2007, a complete responder has disappearance of all evidence of disease; a partial responder has regression of measurable disease and no new sites; a patient with stable disease has a failure to attain CR/PR or PD; and a patient with relapsed disease or progressive disease has any new lesion or increase by greater than or equal to 50% of previously involved sites from nadir. The assessment can involve a determination of whether the disease is FDG-avid, PET positive or negative, whether nodules are present e.g., palpable in the liver or spleen, and whether bone marrow is cleared or shows involvement.

The CAR therapy and the BTK inhibitor such as a compound of formula (I) can be administered, e.g., simultaneously or sequentially. In some embodiments, the CAR therapy is begun at substantially the same time as BTK inhibitor therapy begins. In some embodiments, the CAR therapy is begun before the BTK inhibitor therapy begins. In some embodiments, the CAR therapy is begun after the BTK inhibitor therapy begins. For instance, the CAR therapy can be begun, e.g., at least 1, 2, 3, or 4 weeks, or 1, 2, 3, 4, 6, 9, 12, 15, 18, or 24 months after the BTK inhibitor therapy begins. In some embodiments, the CAR therapy is begun while a patient has physiologically relevant levels of the BTK inhibitor in their body.

When administered in combination, the CAR therapy and the BTK inhibitor such as a compound of formula (I), or both, can be administered in an amount or dose that is higher, lower or the same than the amount or dosage of each agent used individually, e.g., as a monotherapy. In certain embodiments, the administered amount or dosage of the CAR therapy, the BTK inhibitor, or both, is lower (e.g., at least 20%, at least 30%, at least 40%, or at least 50%) than the amount or dosage of each agent used individually, e.g., as a monotherapy. In other embodiments, the amount or dosage of the CAR therapy, the BTK inhibitor, or both, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

When administered in combination, the CAR therapy and the BTK inhibitor such as a compound of formula (I), or both, can be administered with a duration that is longer, shorter, or the same than the duration of each agent used individually, e.g., as a monotherapy. In certain embodiments, the duration of administration of the CAR therapy, the BTK inhibitor, or both, is shorter (e.g., at least 20%, at least 30%, at least 40%, or at least 50%) than the duration of each agent used individually, e.g., as a monotherapy. In other embodiments, the duration of administration of the CAR therapy, the BTK inhibitor, or both, that results in a desired effect (e.g., treatment of cancer) is shorter (e.g., at least 20%, at least 30%, at least 40%, or at least 50% shorter) than the duration of each agent used individually, e.g., as a monotherapy, required to achieve the same therapeutic effect. In some embodiment, the patient is administered an abbreviated course of the BTK inhibitor. For instance, the abbreviated course of the BTK inhibitor may last about 0-2, 2-4, 4-6, 6-8, 8-10, 10-12, 12-15, 15-18, 18-21, or 21-24 months total or may last about 0-2, 2-4, 4-6, 6-8, 8-10, 10-12, 12-15, 15-18, 18-21, or 21-24 months after administration of the CAR therapy. In embodiments, the abbreviated course of the BTK inhibitor ends before relapse. In embodiments, the BTK inhibitor is administered at normal (e.g., monotherapy) levels during the abbreviated course.

In embodiments, a single dose of CAR-expressing cells comprises about 5 x 10⁸ CD19 CART cells. A dose of CAR-expressing cells may also comprise about 5 x 10⁶, 1 x 10⁷,2 x 10⁷, 5 x 10⁷, 1 x 10⁸, 2 x 10⁸, 5 x 10⁸, 1 x 10⁹, 2 x 10⁹, or 5 x 10⁹ cells, e.g., CD19 CAR cells, e.g., CD19 CART cells.

In one aspect, the disclosure pertains to a vector comprising CD19 CAR operably linked to promoter for expression in mammalian cells, e.g., T cells. In one aspect, the invention provides a recombinant cell, e.g., a T cell, expressing the CD19 CAR for use in treating CD19-expressing tumors, wherein the recombinant T cell expressing the CD19 CAR is termed a CD19 CART. In one aspect, the CD19 CART described herein, is capable of contacting a tumor cell with at least one CD19 CAR expressed on its surface such that the CART targets the tumor cell and growth of the tumor is inhibited.

In one aspect, the invention relates to a method of inhibiting growth of a CD19-expressing tumor cell, comprising contacting the tumor cell with a CD19 CAR expressing cell, e.g., a CD19 CART cell, described herein such that the CART is activated in response to the antigen and targets the cancer cell, wherein the growth of the tumor is inhibited. The CD19 CAR-expressing cell, e.g., T cell, is administered in combination with a BTK inhibitor, e.g., a compound of formula (I).

Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered. In one embodiment, the CAR-expressing cell is administered at a dose and/or dosing schedule described herein, and the BTK inhibitor or agent that enhances the activity of the CAR-expressing cell is administered at a dose and/or dosing schedule described herein.

The invention includes a type of cellular therapy where T cells are genetically modified to express a chimeric antigen receptor (CAR) and the CAR T cell is infused to a recipient in need thereof. The infused cell is able to kill tumor cells in the recipient. Unlike antibody therapies, CAR-modified T cells are able to replicate in vivo resulting in long-term persistence that can lead to sustained tumor control. In various aspects, the T cells administered to the patient, or their progeny, persist in the patient for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years after administration of the T cell to the patient.

The invention also includes a type of cellular therapy where T cells are modified, e.g., by *in vitro* transcribed RNA, to transiently express a chimeric antigen receptor (CAR) and the CAR T cell is infused to a recipient in need thereof. The infused cell is able to kill tumor cells in the recipient. Thus, in various aspects, the T cells administered to the patient, is present for less than one month, e.g., three weeks, two weeks, one week, after administration of the T cell to the patient.

Without wishing to be bound by any particular theory, the anti-tumor immunity response elicited by the CAR-modified T cells may be an active or a passive immune response, or alternatively may be due to a direct vs indirect immune response. In one aspect, the CAR transduced T cells exhibit specific proinflammatory cytokine secretion and potent cytolytic activity in response to human cancer cells expressing the CD19, resist soluble CD19 inhibition, mediate bystander killing and mediate regression of an established human tumor. For example, antigen-less tumor cells within a heterogeneous field of CD19-expressing tumor may be susceptible to indirect destruction by CD19-redirected T cells that has previously reacted against adjacent antigen-positive cancer cells.

In one aspect, the fully-human CAR-modified T cells of the disclosure may be a type of vaccine for *ex vivo* immunization and/or in vivo therapy in a mammal. In one aspect, the mammal is a human.

With respect to *ex vivo* immunization, at least one of the following occurs *in vitro* prior to administering the cell into a mammal: i) expansion of the cells, ii) introducing a nucleic acid encoding a CAR to the cells or iii) cryopreservation of the cells.

*Ex vivo* procedures are well known in the art and are discussed more fully below. Briefly, cells are isolated from a mammal (e.g., a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient. In addition to using a cell-based vaccine in terms of *ex vivo* immunization, also included in the methods described herein are compositions and methods for *in vivo* immunization to elicit an immune response directed against an antigen in a patient.

Generally, the cells activated and expanded as described herein may be utilized in the treatment and prevention of diseases that arise in individuals who are immunocompromised. In particular, the CAR-expressing cells described herein are used in the treatment of diseases, disorders and conditions associated with expression of CD19. In certain aspects, the cells are used in the treatment of patients at risk for developing diseases, disorders and conditions associated with expression of CD19. Thus, the present disclosure provides methods for the treatment or prevention of diseases, disorders and conditions associated with expression of CD19 comprising administering to a subject in need thereof, a therapeutically effective amount of the CAR-expressing cells described herein, in combination with a kinase inhibitor, e.g., a kinase inhibitor described herein.

The present disclosure also provides methods for inhibiting the proliferation or reducing a CD 19-expressing cell population, the methods comprising contacting a population of cells comprising a CD 19-expressing cell with an anti-CD 19 CAR-expressing cell described herein that binds to the CD19-expressing cell, and contacting the population of CD19-expressing cells with a BTK inhibitor, e.g., a compound of formula (I). In a specific aspect, the present disclosure provides methods for inhibiting the proliferation or reducing the population of cancer cells expressing CD19, the methods comprising contacting the CD19-expressing cancer cell population with an anti-CD 19 CAR-expressing cell described herein that binds to the CD19-expressing cell, and contacting the CD19-expressing cell with a BTK inhibitor, e.g., a compound of formula (I). In one aspect, the present disclosure provides methods for inhibiting the proliferation or reducing the population of cancer cells expressing CD19, the methods comprising contacting the CD19-expressing cancer cell population with an anti-CD 19 CAR-expressing cell described herein that binds to the CD19-expressing cell and contacting the CD19-expressing cell with a BTK inhibitor, e.g., a compound of formula (I). In certain aspects, the combination of the anti-CD 19 CAR-expressing cell described herein and the BTK inhibitor, e.g., a compound of formula (I), reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for a hematological cancer or another cancer associated with CD19-expressing cells relative to a negative control. In one aspect, the subject is a human.

The present disclosure also provides methods for preventing, treating and/or managing a disease associated with CD19-expressing cells (e.g., a hematologic cancer or atypical cancer expessing CD19), the methods comprising administering to a subject in need an anti-CD 19 CAR-expressing cell that binds to the CD19-expressing cell and administering a BTK inhibitor, e.g., a compound of formula (I). In one aspect, the subject is a human. Non-limiting examples of disorders associated with CD19-expressing cells include autoimmune disorders (such as lupus), inflammatory disorders (such as allergies and asthma) and cancers (such as hematological cancers or atypical cancers expessing CD19).

The present disclosure also provides methods for preventing, treating and/or managing a disease associated with CD19-expressing cells, the methods comprising administering to a subject in need an anti-CD19 CART cell of the disclosure that binds to the CD19-expressing cell. In one aspect, the subject is a human.

The present disclosure provides methods for preventing relapse of cancer associated with CD19-expressing cells, the methods comprising administering to a subject in need thereof an anti-CD19 expressing cell (such as an anti-CD19 CART cell) of the disclosure that binds to the CD19-expressing cell. In one aspect, the methods comprise administering to the subject in need thereof an effective amount of an anti-CD19 expressing cell (such as an anti-CD19 CART cell) described herein that binds to the CD19-expressing cell in combination with an effective amount of another therapy.

In one aspect, the disclosure pertains to a method of treating cancer in a subject. The method comprises administering to the subject a cell (e.g., an immune effector cell) expressing a B-cell targeting CAR, e.g., a T cell or NK cell, described herein, in combination with a BTK inhibitor, e.g., a compound of formula (I), such that the cancer is treated in the subject. An example of a cancer that is treatable by the methods described herein is a cancer associated with expression of the B-cell antigen, e.g., CD19. In one instance, the disease is a solid or liquid tumor. In one instance, the disease is a hematologic cancer. In one instance, the hematologic cancer is leukemia. In one instance, the hematologic cancer is a mature B cell neoplasm, e.g., according to WHO classification. In one instance, the hematologic cancer is a CD19+ B-lymphocyte-derived malignancy. In one instance, the cancer is selected from the group consisting of one or more acute leukemias including but not limited to B-cell acute lymphoid leukemia (BALL), T-cell acute lymphoid leukemia (TALL), small lymphocytic leukemia (SLL), acute lymphoid leukemia (ALL); one or more chronic leukemias including but not limited to chronic myelogenous leukemia (CML), chronic lymphocytic leukemia (CLL); additional hematologic cancers or hematologic conditions including, but not limited to mantle cell lymphoma (MCL), B cell prolymphocytic leukemia, blastic plasmacytoid dendritic cell neoplasm, Burkitt's lymphoma, diffuse large B cell lymphoma (DLBCL) (e.g., T-cell/histiocyte rich large B-cell lymphoma, primary DLCBL of the CNS, primary cutaneous DLBCL leg type, or EBV+ DLBCL of the elderly), DLBCL associated with chronic inflammation, follicular lymphoma, pediatric follicular lymphoma, hairy cell leukemia, small cell- or a large cell-follicular lymphoma, malignant lymphoproliferative conditions, MALT lymphoma (extranodal marginal zone lymphoma of mucosa-associated lymphoid tissue), Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, splenic marginal zone lymphoma, splenic lymphoma/leukemia (e.g., unclassifiable), splenic diffuse red pulp small B-cell lymphoma, hairy cell leukemia-variant, lymphoplasmacytic lymphoma, a heavy chain disease (e.g., alpha heavy chain disease, gamma heavy chain disease, or mu heavy chain disease), plasma cell myeloma, solitary plasmocytoma of bone, extraosseous plasmocytoma, nodal marginal zone lymphoma, pediatric nodal marginal zone lymphoma, primary cutaneous follicle center lymphoma, lymphomatoid granulomatosis, primary mediastinal (theymic) large B-cell lymphoma, intravascular large B-cell lymphoma, ALK+ large B-cell lymphoma, large B-cell lymphoma arising in HHV8-associated multicenric Castleman disease, primary effusion lymphoma, B-cell lymphoma, unclassifiable (e.g., with features intermediate between DLBCL and Burkitt lymphoma or intermediate between DLBCL and classical Hodgkin lymphoma), and "preleukemia" which are a diverse collection of hematological conditions united by ineffective production (or dysplasia) of myeloid blood cells, and to disease associated with with B-cell antigen (e.g., CD19) expression include, but not limited to atypical and/or non-classical cancers, malignancies, precancerous conditions or proliferative diseases expressing B-cell antigen (e.g., CD19); and any combination thereof.

In some instances, the cancer is Hodgkin lymphoma, and the patient is treated with CAR expressing cells, e.g., as a monotherapy, or in combination with one or more additional therapeutics. In instances, the Hodgkin lymphoma is stage I, II, III, or IV. The additional therapeutic may comprise, e.g., a kinase inhibitor such as a BTK inhibitor like ibrutinib. The additional therapeutic may comprise a treatment for Hodgkin lymphoma. The additional therapeutic may comprise, e.g., radiation therapy, MOPP (Mustargen, Oncovin, Prednisone, and Procarbazine), ABVD (Adriamycin, bleomycin, vinblastine, and dacarbazine), Stanford V (a regimen with chemotherapy and radiation treatment), or BEACOPP (Bleomycin, Etoposide, Adriamycin, Cyclophosphamide, Oncovin, Procarbazine, Prednisone). In some instances, the subject has previously been treated with, or is resistant to, or is refractory to, one or more of radiation therapy, MOPP, Stanford V, or BEACOPP.

Non-cancer related indications associated with expression of B-cell antigen, e.g., one or more of CD19, CD20, CD22 or ROR1, include, but are not limited to, e.g., autoimmune disease, (e.g., lupus), inflammatory disorders (allergy and asthma) and transplantation.

In some instances, a cancer that can be treated with the combination described herein is multiple myeloma. Multiple myeloma is a cancer of the blood, characterized by accumulation of a plasma cell clone in the bone marrow. Current therapies for multiple myeloma include, but are not limited to, treatment with lenalidomide, which is an analog of thalidomide. Lenalidomide has activities which include anti-tumor activity, angiogenesis inhibition, and immunomodulation. In some instances, a CD19 CAR, e.g., as described herein, may be used to target myeloma cells. In some instances, the combination described herein can be used with one or more additional therapies, e.g., lenalidomide treatment.

The CAR-expressing cells described herein may be administered either alone, or as a pharmaceutical composition in combination with diluents and/or with other components such as IL-2 or other cytokines or cell populations.

In instances, a lymphodepleting chemotherapy is administered to the subject prior to, concurrently with, or after administration (e.g., infusion) of CAR cells, e.g., CAR-expressing cells described herein. In an example, the lymphodepleting chemotherapy is administered to the subject prior to administration of CAR cells. For example, the lymphodepleting chemotherapy ends 1-4 days (e.g,. 1, 2, 3, or 4 days) prior to CAR cell infusion. In instances, multiple doses of CAR cells are administered, e.g., as described herein. For example, a single dose comprises about 5 x 10⁸ CAR cells. In instances, a lymphodepleting chemotherapy is administered to the subject prior to, concurrently with, or after administration (e.g., infusion) of a CAR-expressing cell described herein.

### Hematologic Cancer

Hematological cancer conditions are the types of cancer such as leukemia, lymphoma and malignant lymphoproliferative conditions that affect blood, bone marrow and the lymphatic system.

Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

Lymphoma is a group of blood cell tumors that develop from lymphocytes. Exemplary lymphomas include non-Hodgkin lymphoma and Hodgkin lymphoma.

The present invention provides for compositions for use in methods for treating cancer. In one aspect, the cancer is a hematologic cancer including but is not limited to hematological cancer is a leukemia or a lymphoma. In one aspect, the CART cells of the disclosure may be used to treat cancers and malignancies such as, but not limited to, e.g., acute leukemias including but not limited to, e.g., B-cell acute lymphoid leukemia ("BALL"), T-cell acute lymphoid leukemia ("TALL"), acute lymphoid leukemia (ALL); one or more chronic leukemias including but not limited to, e.g., chronic myelogenous leukemia (CML), chronic lymphocytic leukemia (CLL); additional hematologic cancers or hematologic conditions including, but not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further a disease associated with a cancer associate antigen as described herein expression includes, but not limited to, e.g., atypical and/or non-classical cancers, malignancies, precancerous conditions or proliferative diseases expressing a cancer associate antigen as described herein.

The present disclosure also provides methods for inhibiting the proliferation or reducing a tumor antigen -expressing cell population, the methods comprising contacting a population of cells comprising a tumor antigen with a CAR-expressing cell or NK cell of the disclosure that binds to the tumor antigen. In a specific aspect, the present disclosure provides methods for inhibiting the proliferation or reducing the population of cancer cells expressing a tumor antigen as described herein, the methods comprising contacting a tumor antigen -expressing cancer cell population with a CAR-expressing T cell or NK cell of the disclosure that binds to the tumor antigen. In one aspect, the present disclosure provides methods for inhibiting the proliferation or reducing the population of cancer cells expressing a tumor antigen, e.g., described herein, the methods comprising contacting a tumor antigen -expressing cancer cell population with a CAR-expressing T cell or NK cell of the disclosure that binds to a tumor antigen. In certain aspects, a CAR-expressing T cell or NK cell of the disclosure reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for myeloid leukemia or another cancer associated with a tumor antigen-expressing cells relative to a negative control. In one aspect, the subject is a human.

The present disclosure also provides methods for preventing, treating and/or managing a disease associated with a tumor antigen -expressing cells (e.g., a hematologic cancer or atypical cancer expressing a tumor antigen, e.g., described herein), the methods comprising administering to a subject in need a CAR T cell or NK cell of the disclosure that binds to a tumor antigen -expressing cell. In one aspect, the subject is a human. Non-limiting examples of disorders associated with a tumor antigen-expressing cells include autoimmune disorders (such as lupus), inflammatory disorders (such as allergies and asthma) and cancers (such as hematological cancers or atypical cancers expressing a tumor antigen as described herein).

The present disclosure also provides methods for preventing, treating and/or managing a disease associated with a tumor antigen -expressing cells, the methods comprising administering to a subject in need a CAR T cell or NK cell of the disclosure that binds to a tumor antigen -expressing cell. In one aspect, the subject is a human.

The present disclosure provides methods for preventing relapse of cancer associated with a tumor antigen -expressing cells, the methods comprising administering to a subject in need thereof a CAR T cell or NK cell of the disclosure that binds to a tumor antigen -expressing cell. In one aspect, the methods comprise administering to the subject in need thereof an effective amount of a CAR-expressing T cell or NK cell described herein that binds to a tumor antigen -expressing cell in combination with an effective amount of another therapy.

### Combination Therapies

The combination of a CAR-expressing cell described herein (e.g., and a BTK inhibitor, e.g., a compound of formula (I)) may be used in combination with other known agents and therapies.

A CAR-expressing cell described herein, the BTK inhibitor and/or the at least one additional therapeutic agent can be administered simultaneously, in the same or in separate compositions, or sequentially. For sequential administration, the CAR-expressing cell described herein can be administered first, and the additional agent can be administered second, or the order of administration can be reversed.

The CAR therapy and/or other therapeutic agents, procedures or modalities can be administered during periods of active disorder, or during a period of remission or less active disease. The CAR therapy can be administered before another treatment, concurrently with the treatment, post-treatment, or during remission of the disorder.

When administered in combination, the CAR therapy and one or more additional agent (e.g., BTK inhibitor and/or a third agent), or all, can be administered in an amount or dose that is higher, lower or the same than the amount or dosage of each agent used individually, e.g., as a monotherapy. In certain embodiments, the administered amount or dosage of the CAR therapy, the additional agent (e.g., BTK inhibitor and/or third agent), or all, is lower (e.g., at least 20%, at least 30%, at least 40%, or at least 50%) than the amount or dosage of each agent used individually, e.g., as a monotherapy. In other embodiments, the amount or dosage of the CAR therapy, the additional agent (e.g., BTK inhibitor and/or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

In further aspects, the combination of the CAR-expressing cell described herein (e.g., and the BTK inhibitor) may be used in a treatment regimen in combination with surgery, chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation, peptide vaccine, such as that described in Izumoto et al. 2008 J Neurosurg 108:963-971.

In one instance, the combination of a CAR-expressing cell described herein (e.g., and a BTK inhibitor, e.g., a compound of formula (I)) can be used in combination with another chemotherapeutic agent. Exemplary chemotherapeutic agents include an anthracycline (e.g., doxorubicin (e.g., liposomal doxorubicin)); a vinca alkaloid (e.g., vinblastine, vincristine, vindesine, vinorelbine); an alkylating agent (e.g., cyclophosphamide, decarbazine, melphalan, ifosfamide, temozolomide); an immune cell antibody (e.g., alemtuzamab, gemtuzumab, rituximab, ofatumumab, tositumomab, brentuximab); an antimetabolite (including, e.g., folic acid antagonists, pyrimidine analogs, purine analogs and adenosine deaminase inhibitors (e.g., fludarabine)); a TNFR glucocorticoid induced TNFR related protein (GITR) agonist; a proteasome inhibitor (e.g., aclacinomycin A, gliotoxin or bortezomib); an immunomodulator such as thalidomide or a thalidomide derivative (e.g., lenalidomide).

General Chemotherapeutic agents considered for use in combination therapies include anastrozole (Arimidex®), bicalutamide (Casodex®), bleomycin sulfate (Blenoxane®), busulfan (Myleran®), busulfan injection (Busulfex®), capecitabine (Xeloda®), N4-pentoxycarbonyl-5-deoxy-5-fluorocytidine, carboplatin (Paraplatin®), carmustine (BiCNU®), chlorambucil (Leukeran®), cisplatin (Platinol®), cladribine (Leustatin®), cyclophosphamide (Cytoxan® or Neosar®), cytarabine, cytosine arabinoside (Cytosar-U®), cytarabine liposome injection (DepoCyt®), dacarbazine (DTIC-Dome®), dactinomycin (Actinomycin D, Cosmegan), daunorubicin hydrochloride (Cerubidine®), daunorubicin citrate liposome injection (DaunoXome®), dexamethasone, docetaxel (Taxotere®), doxorubicin hydrochloride (Adriamycin®, Rubex®), etoposide (Vepesid®), fludarabine phosphate (Fludara®), 5-fluorouracil (Adrucil®, Efudex®), flutamide (Eulexin®), tezacitibine, gemcitabine (difluorodeoxycitidine), hydroxyurea (Hydrea®), Idarubicin (Idamycin®), ifosfamide (IFEX®), irinotecan (Camptosar®), L-asparaginase (ELSPAR®), leucovorin calcium, melphalan (Alkeran®), 6-mercaptopurine (Purinethol®), methotrexate (Folex®), mitoxantrone (Novantrone®), mylotarg, paclitaxel (Taxol®), phoenix (Yttrium90/MX-DTPA), pentostatin, polifeprosan 20 with carmustine implant (Gliadel®), tamoxifen citrate (Nolvadex®), teniposide (Vumon®), 6-thioguanine, thiotepa, tirapazamine (Tirazone®), topotecan hydrochloride for injection (Hycamptin®), vinblastine (Velban®), vincristine (Oncovin®), and vinorelbine (Navelbine®).

Exemplary alkylating agents include, without limitation, nitrogen mustards, ethylenimine derivatives, alkyl sulfonates, nitrosoureas and triazenes): uracil mustard (Aminouracil Mustard®, Chlorethaminacil®, Demethyldopan®, Desmethyldopan®, Haemanthamine®, Nordopan®, Uracil nitrogen mustard®, Uracillost®, Uracilmostaza®, Uramustin®, Uramustine®), chlormethine (Mustargen®), cyclophosphamide (Cytoxan®, Neosar®, Clafen®, Endoxan®, Procytox®, Revimmune™), ifosfamide (Mitoxana®), melphalan (Alkeran®), Chlorambucil (Leukeran®), pipobroman (Amedel®, Vercyte®), triethylenemelamine (Hemel®, Hexalen®, Hexastat®), triethylenethiophosphoramine, Temozolomide (Temodar®), thiotepa (Thioplex®), busulfan (Busilvex®, Myleran®), carmustine (BiCNU®), lomustine (CeeNU®), streptozocin (Zanosar®), and Dacarbazine (DTIC-Dome®). Additional exemplary alkylating agents include, without limitation, Oxaliplatin (Eloxatin®); Temozolomide (Temodar® and Temodal®); Dactinomycin (also known as actinomycin-D, Cosmegen®); Melphalan (also known as L-PAM, L-sarcolysin, and phenylalanine mustard, Alkeran®); Altretamine (also known as hexamethylmelamine (HMM), Hexalen®); Carmustine (BiCNU®); Bendamustine (Treanda®); Busulfan (Busulfex® and Myleran®); Carboplatin (Paraplatin®); Lomustine (also known as CCNU, CeeNU®); Cisplatin (also known as CDDP, Platinol® and Platinol®-AQ); Chlorambucil (Leukeran®); Cyclophosphamide (Cytoxan® and Neosar®); Dacarbazine (also known as DTIC, DIC and imidazole carboxamide, DTIC-Dome®); Altretamine (also known as hexamethylmelamine (HMM), Hexalen®); Ifosfamide (Ifex®); Prednumustine; Procarbazine (Matulane®); Mechlorethamine (also known as nitrogen mustard, mustine and mechloroethamine hydrochloride, Mustargen®); Streptozocin (Zanosar®); Thiotepa (also known as thiophosphoamide, TESPA and TSPA, Thioplex®); Cyclophosphamide (Endoxan®, Cytoxan®, Neosar®, Procytox®, Revimmune®); and Bendamustine HC1 (Treanda®).

In instances, a CAR-expressing cell described herein, optionally in combination with a kinase inhibitor e.g., a BTK inhibitor such as ibrutinib, is administered to a subject in combination with fludarabine, cyclophosphamide, and/or rituximab. In instances, a CAR-expressing cell described herein is administered to a subject in combination with fludarabine, cyclophosphamide, and rituximab (FCR). In instances, the subject has CLL. For example, the subject has a deletion in the short arm of chromosome 17 (del(17p), e.g., in a leukemic cell). In other examples, the subject does not have a del(17p). In instances, the subject comprises a leukemic cell comprising a mutation in the immunoglobulin heavy-chain variable-region *(IgV_{H})* gene. In other instances, the subject does not comprise a leukemic cell comprising a mutation in the immunoglobulin heavy-chain variable-region (*IgV_{H}*) gene. In instances, the fludarabine is administered at a dosage of about 10-50 mg/m² (e.g., about 10-15, 15-20, 20-25, 25-30, 30-35, 35-40, 40-45, or 45-50 mg/m²), e.g., intravenously. In instances, the cyclophosphamide is administered at a dosage of about 200-300 mg/m² (e.g., about 200-225, 225-250, 250-275, or 275-300 mg/m²), e.g., intravenously. In instances, the rituximab is administered at a dosage of about 400-600 mg/m² (e.g., 400-450, 450-500, 500-550, or 550-600 mg/m²), e.g., intravenously.

In instances, a CAR-expressing cell described herein, optionally in combination with a BTK inhibitor, e.g., a compound of formula (I), is administered to a subject in combination with bendamustine and rituximab. In instances, the subject has CLL. For example, the subject has a deletion in the short arm of chromosome 17 (del(17p), e.g., in a leukemic cell). In other examples, the subject does not have a del(17p). In instances, the subject comprises a leukemic cell comprising a mutation in the immunoglobulin heavy-chain variable-region (*IgV_{H}*) gene. In other instances, the subject does not comprise a leukemic cell comprising a mutation in the immunoglobulin heavy-chain variable-region (*IgV_{H}*) gene. In instances, the bendamustine is administered at a dosage of about 70-110 mg/m² (e.g., 70-80, 80-90, 90-100, or 100-110 mg/m²), e.g., intravenously. In instances, the rituximab is administered at a dosage of about 400-600 mg/m² (e.g., 400-450, 450-500, 500-550, or 550-600 mg/m²), e.g., intravenously.

In instances, a CAR-expressing cell described herein, optionally in combination with a BTK inhibitor, e.g., a compound of formula (I), is administered to a subject in combination with rituximab, cyclophosphamide, doxorubicine, vincristine, and/or a corticosteroid (e.g., prednisone). In instances, a CAR-expressing cell described herein is administered to a subject in combination with rituximab, cyclophosphamide, doxorubicine, vincristine, and prednisone (R-CHOP). In instances, the subject has diffuse large B-cell lymphoma (DLBCL). In instances, the subject has nonbulky limited-stage DLBCL (e.g., comprises a tumor having a size/diameter of less than 7 cm). In instances, the subject is treated with radiation in combination with the R-CHOP. For example, the subject is administered R-CHOP (e.g., 1-6 cycles, e.g., 1, 2, 3, 4, 5, or 6 cycles of R-CHOP), followed by radiation. In some cases, the subject is administered R-CHOP (e.g., 1-6 cycles, e.g., 1, 2, 3, 4, 5, or 6 cycles of R-CHOP) following radiation.

In instances, a CAR-expressing cell described herein, optionally in combination with a BTK inhibitor, e.g., a compound of formula (I), is administered to a subject in combination with etoposide, prednisone, vincristine, cyclophosphamide, doxorubicin, and/or rituximab. In instances, a CAR-expressing cell described herein is administered to a subject in combination with etoposide, prednisone, vincristine, cyclophosphamide, doxorubicin, and rituximab (EPOCH-R). In instances, a CAR-expressing cell described herein is administered to a subject in combination with dose-adjusted EPOCH-R (DA-EPOCH-R). In instances, the subject has a B cell lymphoma, e.g., a Myc-rearranged aggressive B cell lymphoma.

In instances, a CAR-expressing cell described herein, optionally in combination with a BTK inhibitor, e.g., a compound of formula (I), is administered to a subject in combination with rituximab and/or lenalidomide. Lenalidomide ((*RS*)-3-(4-Amino-1-oxo 1,3-dihydro-2*H*-isoindol-2-yl)piperidine-2,6-dione) is an immunomodulator. In instances, a CAR-expressing cell described herein is administered to a subject in combination with rituximab and lenalidomide. In instances, the subject has follicular lymphoma (FL) or mantle cell lymphoma (MCL). In instances, the subject has FL and has not previously been treated with a cancer therapy. In instances, lenalidomide is administered at a dosage of about 10-20 mg (e.g., 10-15 or 15-20 mg), e.g., daily. In instances, rituximab is administered at a dosage of about 350-550 mg/m² (e.g., 350-375, 375-400, 400-425, 425-450, 450-475, or 475-500 mg/m²), e.g., intravenously.

Treatment with a combination of a chemotherapeutic agent and a cell expressing a CAR molecule described herein, optionally in combination with a BTK inhibitor, e.g., a compound of formula (I), can be used to treat a hematologic cancer described herein, e.g., AML. In instances, the combination of a chemotherapeutic agent and a CAR-expressing cell is useful for targeting, e.g., killing, cancer stem cells, e.g., leukemic stem cells, e.g., in subjects with AML. In instances, the combination of a chemotherapeutic agent and a CAR-expressing cell is useful for treating minimal residual disease (MRD). MRD refers to the small number of cancer cells that remain in a subject during treatment, e.g., chemotherapy, or after treatment. MRD is often a major cause for relapse. The present disclosure provides a method for treating cancer, e.g., MRD, comprising administering a chemotherapeutic agent in combination with a CAR-expressing cell, e.g., as described herein.

In an instance, the chemotherapeutic agent is administered prior to administration of the cell expressing a CAR molecule, e.g., a CAR molecule described herein. In chemotherapeutic regimens where more than one administration of the chemotherapeutic agent is desired, the chemotherapeutic regimen is initiated or completed prior to administration of a cell expressing a CAR molecule, e.g., a CAR molecule described herein. In instances, the chemotherapeutic agent is administered at least 1 day, 2 days, 3 days, 4 days, 5 days, 6 days, 7 days, 8 days, 9 days, 10 days, 11 days, 12 days, 13 days, 14 days, 15 days, 20 days, 25 days, or 30 days prior to administration of the cell expressing the CAR molecule. In instances, the chemotherapeutic regimen is initiated or completed at least 1 day, 2 days, 3 days, 4 days, 5 days, 6 days, 7 days, 8 days, 9 days, 10 days, 11 days, 12 days, 13 days, 14 days, 15 days, 20 days, 25 days, or 30 days prior to administration of the cell expressing the CAR molecule. In instances, the chemotherapeutic agent is a chemotherapeutic agent that increases expression of CD19, CD20, or CD22 on the cancer cells, e.g., the tumor cells, e.g., as compared to expression on normal or non-cancer cells. Expression can be determined, for example, by immunohistochemical staining or flow cytometry analysis. For example, the chemotherapeutic agent is cytarabine (Ara-C).

Anti-cancer agents of particular interest for combinations with the compounds of the present disclosure include: antimetabolites; drugs that inhibit either the calcium dependent phosphatase calcineurin or the p70S6 kinase FK506) or inhibit the p70S6 kinase; alkylating agents; mTOR inhibitors; immunomodulators; anthracyclines; vinca alkaloids; proteosome inhibitors; GITR agonists; protein tyrosine phosphatase inhibitors; a CDK4 kinase inhibitor; a BTK kinase inhibitor; a MKN kinase inhibitor; a DGK kinase inhibitor; or an oncolytic virus.

Exemplary antimetabolites include, without limitation, folic acid antagonists (also referred to herein as antifolates), pyrimidine analogs, purine analogs and adenosine deaminase inhibitors): methotrexate (Rheumatrex®, Trexall®), 5-fluorouracil (Adrucil®, Efudex®, Fluoroplex®), floxuridine (FUDF®), cytarabine (Cytosar-U®, Tarabine PFS), 6-mercaptopurine (Puri-Nethol®)), 6-thioguanine (Thioguanine Tabloid®), fludarabine phosphate (Fludara®), pentostatin (Nipent®), pemetrexed (Alimta®), raltitrexed (Tomudex®), cladribine (Leustatin®), clofarabine (Clofarex®, Clolar®), mercaptopurine (Puri-Nethol®), capecitabine (Xeloda®), nelarabine (Arranon®), azacitidine (Vidaza®) and gemcitabine (Gemzar®). Preferred antimetabolites include, e.g., 5-fluorouracil (Adrucil®, Efudex®, Fluoroplex®), floxuridine (FUDF®), capecitabine (Xeloda®), pemetrexed (Alimta®), raltitrexed (Tomudex®) and gemcitabine (Gemzar®).

In instances, a CAR-expressing cell described herein, optionally in combination with a BTK inhibitor, e.g., a compound of formula (I), is administered to a subject in combination with fludarabine, cyclophosphamide, and/or rituximab. In instances, a CAR-expressing cell described herein is administered to a subject in combination with fludarabine, cyclophosphamide, and rituximab (FCR). In instances, the subject has CLL. For example, the subject has a deletion in the short arm of chromosome 17 (del(17p), e.g., in a leukemic cell). In other examples, the subject does not have a del(17p). In instances, the subject comprises a leukemic cell comprising a mutation in the immunoglobulin heavy-chain variable-region *(IgV_{H})* gene. In other instances, the subject does not comprise a leukemic cell comprising a mutation in the immunoglobulin heavy-chain variable-region (*IgV_{H}*) gene. In instances, the fludarabine is administered at a dosage of about 10-50 mg/m² (e.g., about 10-15, 15-20, 20-25, 25-30, 30-35, 35-40, 40-45, or 45-50 mg/m²), e.g., intravenously. In instances, the cyclophosphamide is administered at a dosage of about 200-300 mg/m² (e.g., about 200-225, 225-250, 250-275, or 275-300 mg/m²), e.g., intravenously. In instances, the rituximab is administered at a dosage of about 400-600 mg/m2 (e.g., 400-450, 450-500, 500-550, or 550-600 mg/m²), e.g., intravenously.

In instances, a CAR-expressing cell described herein, optionally in combination with a BTK inhibitor, e.g., a compound of formula (I), is administered to a subject in combination with bendamustine and rituximab. In instances, the subject has CLL. For example, the subject has a deletion in the short arm of chromosome 17 (del(17p), e.g., in a leukemic cell). In other examples, the subject does not have a del(17p). In instances, the subject comprises a leukemic cell comprising a mutation in the immunoglobulin heavy-chain variable-region (*IgV_{H}*) gene. In other instances, the subject does not comprise a leukemic cell comprising a mutation in the immunoglobulin heavy-chain variable-region (*IgV_{H}*) gene. In instances, the bendamustine is administered at a dosage of about 70-110 mg/m2 (e.g., 70-80, 80-90, 90-100, or 100-110 mg/m2), e.g., intravenously. In instances, the rituximab is administered at a dosage of about 400-600 mg/m2 (e.g., 400-450, 450-500, 500-550, or 550-600 mg/m²), e.g., intravenously.

In instances, a CAR-expressing cell described herein, optionally in combination with a BTK inhibitor, e.g., a compound of formula (I), is administered to a subject in combination with rituximab, cyclophosphamide, doxorubicine, vincristine, and/or a corticosteroid (e.g., prednisone). In instances, a CAR-expressing cell described herein is administered to a subject in combination with rituximab, cyclophosphamide, doxorubicine, vincristine, and prednisone (R-CHOP). In embodiments, the subject has diffuse large B-cell lymphoma (DLBCL). In instances, the subject has nonbulky limited-stage DLBCL (e.g., comprises a tumor having a size/diameter of less than 7 cm). In instances, the subject is treated with radiation in combination with the R-CHOP. For example, the subject is administered R-CHOP (e.g., 1-6 cycles, e.g., 1, 2, 3, 4, 5, or 6 cycles of R-CHOP), followed by radiation. In some cases, the subject is administered R-CHOP (e.g., 1-6 cycles, e.g., 1, 2, 3, 4, 5, or 6 cycles of R-CHOP) following radiation.

In instances, a CAR-expressing cell described herein, optionally in combination with a BTK inhibitor, e.g., a compound of formula (I), is administered to a subject in combination with etoposide, prednisone, vincristine, cyclophosphamide, doxorubicin, and/or rituximab. In instances, a CAR-expressing cell described herein is administered to a subject in combination with etoposide, prednisone, vincristine, cyclophosphamide, doxorubicin, and rituximab (EPOCH-R). In instances, a CAR-expressing cell described herein is administered to a subject in combination with dose-adjusted EPOCH-R (DA-EPOCH-R). In instances, the subject has a B cell lymphoma, e.g., a Myc-rearranged aggressive B cell lymphoma.

In instances, a CAR-expressing cell described herein, optionally in combination with a BTK inhibitor, e.g., a compound of formula (I), is administered to a subject in combination with rituximab and/or lenalidomide. Lenalidomide ((*RS*)-3-(4-Amino-1-oxo 1,3-dihydro-2*H*-isoindol- 2-yl)piperidine-2,6-dione) is an immunomodulator. In instances, a CAR-expressing cell described herein is administered to a subject in combination with rituximab and lenalidomide. In instances, the subject has follicular lymphoma (FL) or mantle cell lymphoma (MCL). In instances, the subject has FL and has not previously been treated with a cancer therapy. In instances, lenalidomide is administered at a dosage of about 10-20 mg (e.g., 10-15 or 15-20 mg), e.g., daily. In instances, rituximab is administered at a dosage of about 350-550 mg/m² (e.g., 350-375, 375-400, 400-425, 425-450, 450-475, or 475-500 mg/m²), e.g., intravenously.

Exemplary mTOR inhibitors include, e.g., temsirolimus; ridaforolimus (formally known as deferolimus, (1*R*,2*R*,4*S*)-4-[(2*R*)-2[(1*R*,9*S*,12*S*,15*R*,16*E*,18*R*,19*R*,21*R*,23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); everolimus (Afinitor® or RAD001); rapamycin (AY22989, Sirolimus®); simapimod (CAS 164301-51-3); emsirolimus, (5-{2,4-Bis[(3*S*)-3-methylmorpholin-4-yl]pyrido[2,3-*d*]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502, CAS 1013101-36-4); and *N*²-[1,4-dioxo-4-[[4-(4-oxo-8-phenyl-4*H*-1-benzopyran-2-yl)morpholinium-4-yl]methoxy]butyl]-L-arginylglycyl-L-a-aspartylL-serine-, inner salt (SF1126, CAS 936487-67-1) (SEQ ID NO: 134), and XL765.

Exemplary immunomodulators include, e.g., afutuzumab (available from Roche®); pegfilgrastim (Neulasta®); lenalidomide (CC-5013, Revlimid®); thalidomide (Thalomid®), pomelidomide, actimid (CC4047); and IRX-2 (mixture of human cytokines including interleukin 1, interleukin 2, and interferon γ, CAS 951209-71-5, available from IRX Therapeutics).

Exemplary anthracyclines include, e.g., doxorubicin (Adriamycin® and Rubex®); bleomycin (lenoxane®); daunorubicin (dauorubicin hydrochloride, daunomycin, and rubidomycin hydrochloride, Cerubidine®); daunorubicin liposomal (daunorubicin citrate liposome, DaunoXome®); mitoxantrone (DHAD, Novantrone®); epirubicin (Ellence™); idarubicin (Idamycin®, Idamycin PFS®); mitomycin C (Mutamycin®); geldanamycin; herbimycin; ravidomycin; and desacetylravidomycin.

Exemplary vinca alkaloids include, e.g., vinorelbine tartrate (Navelbine®), Vincristine (Oncovin®), and Vindesine (Eldisine®)); vinblastine (also known as vinblastine sulfate, vincaleukoblastine and VLB, Alkaban-AQ® and Velban®); and vinorelbine (Navelbine®).

Exemplary proteosome inhibitors include bortezomib (Velcade®); carfilzomib (PX-171-007, (S)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((S)-2-(2-morpholinoacetamido)-4-phenylbutanamido)-pentanamide); marizomib (NPI-0052); ixazomib citrate (MLN-9708); delanzomib (CEP-18770); and *O*-Methyl-*N*-[(2-methyl-5-thiazolyl)carbonyl]-L-seryl-*O*-methyl-N-[(1*S*)-2-[(2*R*)-2-methyl-2-oxiranyl]-2-oxo-1-(phenylmethyl)ethyl]-L-serinamide (ONX-0912).

In instances, a CAR-expressing cell described herein, optionally in combination with a BTK inhibitor, e.g., a compound of formula (I), is administered to a subject in combination with brentuximab. Brentuximab is an antibody-drug conjugate of anti-CD30 antibody and monomethyl auristatin E. In instances, the subject has Hodgkin lymphoma (HL), e.g., relapsed or refractory HL. In instances, the subject comprises CD30+ HL. In instances, the subject has undergone an autologous stem cell transplant (ASCT). In instances, the subject has not undergone an ASCT. In instances, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In instances, a CAR-expressing cell described herein, optionally in combination with a BTK inhibitor, e.g., a compound of formula (I), is administered to a subject in combination with brentuximab and dacarbazine or in combination with brentuximab and bendamustine. Dacarbazine is an alkylating agent with a chemical name of 5-(3,3-Dimethyl-1-triazenyl)imidazole-4-carboxamide. Bendamustine is an alkylating agent with a chemical name of 4-[5-[Bis(2-chloroethyl)amino]-1-methylbenzimidazol-2-yl]butanoic acid. In instances, the subject has Hodgkin lymphoma (HL). In instances, the subject has not previously been treated with a cancer therapy. In instances, the subject is at least 60 years of age, e.g., 60, 65, 70, 75, 80, 85, or older. In instances, dacarbazine is administered at a dosage of about 300-450 mg/m² (e.g., about 300-325, 325-350, 350-375, 375-400, 400-425, or 425-450 mg/m²), e.g., intravenously. In instances, bendamustine is administered at a dosage of about 75-125 mg/m² (e.g., 75-100 or 100-125 mg/m², e.g., about 90 mg/m²), e.g., intravenously. In instances, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In some instances, a CAR-expressing cell described herein, optionally in combination with a BTK inhibitor, e.g., a compound of formula (I), is administered to a subject in combination with a CD20 inhibitor, e.g., an anti-CD20 antibody (e.g., an anti-CD20 mono- or bispecific antibody) or a fragment thereof. Exemplary anti-CD20 antibodies include but are not limited to rituximab, ofatumumab, ocrelizumab, veltuzumab, obinutuzumab, TRU-015 (Trubion Pharmaceuticals), ocaratuzumab, and Pro131921 (Genentech). See, e.g., Lim et al. Haematologica. 95.1(2010):135-43.

In some instances, the anti-CD20 antibody comprises rituximab. Rituximab is a chimeric mouse/human monoclonal antibody IgG1 kappa that binds to CD20 and causes cytolysis of a CD20 expressing cell, e.g., as described in www.accessdata.fda.gov/drugsatfda_docs/label/2010/103705s531 11bl.pdf. In instances, a CAR-expressing cell described herein is administered to a subject in combination with rituximab. In instances, the subject has CLL or SLL.

In some instances, rituximab is administered intravenously, e.g., as an intravenous infusion. For example, each infusion provides about 500-2000 mg (e.g., about 500-550, 550-600, 600-650, 650-700, 700-750, 750-800, 800-850, 850-900, 900-950, 950-1000, 1000-1100, 1100-1200, 1200-1300, 1300-1400, 1400-1500, 1500-1600, 1600-1700, 1700-1800, 1800-1900, or 1900-2000 mg) of rituximab. In some instances, rituximab is administered at a dose of 150 mg/m² to 750 mg/m², e.g., about 150-175 mg/m², 175-200 mg/m², 200-225 mg/m², 225-250 mg/m², 250-300 mg/m², 300-325 mg/m², 325-350 mg/m², 350-375 mg/m², 375-400 mg/m², 400-425 mg/m², 425-450 mg/m², 450-475 mg/m², 475-500 mg/m², 500-525 mg/m², 525-550 mg/m², 550-575 mg/m², 575-600 mg/m², 600-625 mg/m², 625-650 mg/m², 650-675 mg/m², or 675-700 mg/m², where m² indicates the body surface area of the subject. In some instances, rituximab is administered at a dosing interval of at least 4 days, e.g., 4, 7, 14, 21, 28, 35 days, or more. For example, rituximab is administered at a dosing interval of at least 0.5 weeks, e.g., 0.5, 1, 2, 3, 4, 5, 6, 7, 8 weeks, or more. In some instances, rituximab is administered at a dose and dosing interval described herein for a period of time, e.g., at least 2 weeks, e.g., at least 2, 3, 4, 5, 6, 7, 8, 9, 10, 11, 12, 13, 14, 15, 16, 17, 18, 19, 20 weeks, or greater. For example, rituximab is administered at a dose and dosing interval described herein for a total of at least 4 doses per treatment cycle (e.g., at least 4, 5, 6, 7, 8, 9, 10, 11, 12, 13, 14, 15, 16, or more doses per treatment cycle).

In some instances, the anti-CD20 antibody comprises ofatumumab. Ofatumumab is an anti-CD20 IgG1κ human monoclonal antibody with a molecular weight of approximately 149 kDa. For example, ofatumumab is generated using transgenic mouse and hybridoma technology and is expressed and purified from a recombinant murine cell line (NS0). See, e.g., www.accessdata.fda.gov/drugsatfda_docs/label/2009/1253261bl.pdf; and Clinical Trial Identifier number NCT01363128, NCT01515176, NCT01626352, and NCT01397591. In instances, a CAR-expressing cell described herein is administered to a subject in combination with ofatumumab. In instances, the subject has CLL or SLL.

In some instances, ofatumumab is administered as an intravenous infusion. For example, each infusion provides about 150-3000 mg (e.g., about 150-200, 200-250, 250-300, 300-350, 350-400, 400-450, 450-500, 500-550, 550-600, 600-650, 650-700, 700-750, 750-800, 800-850, 850-900, 900-950, 950-1000, 1000-1200, 1200-1400, 1400-1600, 1600-1800, 1800-2000, 2000-2200, 2200-2400, 2400-2600, 2600-2800, or 2800-3000 mg) of ofatumumab. In instances, ofatumumab is administered at a starting dosage of about 300 mg, followed by 2000 mg, e.g., for about 11 doses, e.g., for 24 weeks. In some instances, ofatumumab is administered at a dosing interval of at least 4 days, e.g., 4, 7, 14, 21, 28, 35 days, or more. For example, ofatumumab is administered at a dosing interval of at least 1 week, e.g., 1, 2, 3, 4, 5, 6, 7, 8, 9, 10, 11, 12, 24, 26, 28, 20, 22, 24, 26, 28, 30 weeks, or more. In some instances, ofatumumab is administered at a dose and dosing interval described herein for a period of time, e.g., at least 1 week, e.g., 1, 2, 3, 4, 5, 6, 7, 8, 9, 10, 11, 12, 13, 14, 15, 16, 17, 18, 19, 20, 22, 24, 26, 28, 30, 40, 50, 60 weeks or greater, or 1, 2, 3, 4, 5, 6, 7, 8, 9, 10, 11, 12 months or greater, or 1, 2, 3, 4, 5 years or greater. For example, ofatumumab is administered at a dose and dosing interval described herein for a total of at least 2 doses per treatment cycle (e.g., at least 2, 3, 4, 5, 6, 7, 8, 9, 10, 11, 12, 13, 14, 15, 16, 18, 20, or more doses per treatment cycle).

In some cases, the anti-CD20 antibody comprises ocrelizumab. Ocrelizumab is a humanized anti-CD20 monoclonal antibody, e.g., as described in Clinical Trials Identifier Nos. NCT00077870, NCT01412333, NCT00779220, NCT00673920, NCT01194570, and Kappos et al. Lancet. 19.378(2011):1779-87.

In some cases, the anti-CD20 antibody comprises veltuzumab. Veltuzumab is a humanized monoclonal antibody against CD20. See, e.g., Clinical Trial Identifier No. NCT00547066, NCT00546793, NCT01101581, and Goldenberg et al. Leuk Lymphoma. 51(5)(2010):747-55.

In some cases, the anti-CD20 antibody comprises GA101. GA101 (also called obinutuzumab or RO5072759) is a humanized and glyco-engineered anti-CD20 monoclonal antibody. See, e.g., Robak. Curr. Opin. Investig. Drugs. 10.6(2009):588-96; Clinical Trial Identifier Numbers: NCT01995669, NCT01889797, NCT02229422, and NCT01414205; and www.accessdata.fda.gov/drugsatfda_docs/label/2013/125486s0001b1.pdf.

In some cases, the anti-CD20 antibody comprises AME-133v. AME-133v (also called LY2469298 or ocaratuzumab) is a humanized IgG1 monoclonal antibody against CD20 with increased affinity for the FcyRIIIa receptor and an enhanced antibody dependent cellular cytotoxicity (ADCC) activity compared with rituximab. See, e.g., Robak et al. BioDrugs 25.1(2011):13-25; and Forero-Torres et al. Clin Cancer Res. 18.5(2012):1395-403.

In some cases, the anti-CD20 antibody comprises PRO131921. PRO131921 is a humanized anti-CD20 monoclonal antibody engineered to have better binding to FcγRIIIa and enhanced ADCC compared with rituximab. See, e.g., Robak et al. BioDrugs 25.1(2011):13-25; and Casulo et al. Clin Immunol. 154.1(2014):37-46; and Clinical Trial Identifier No. NCT00452127.

In some cases, the anti-CD20 antibody comprises TRU-015. TRU-015 is an anti-CD20 fusion protein derived from domains of an antibody against CD20. TRU-015 is smaller than monoclonal antibodies, but retains Fc-mediated effector functions. See, e.g., Robak et al. BioDrugs 25.1(2011):13-25. TRU-015 contains an anti-CD20 single-chain variable fragment (scFv) linked to human IgG1 hinge, CH2, and CH3 domains but lacks CH1 and CL domains.

In some instances, an anti-CD20 antibody described herein is conjugated or otherwise bound to a therapeutic agent, e.g., a chemotherapeutic agent (e.g., cytoxan, fludarabine, histone deacetylase inhibitor, demethylating agent, peptide vaccine, anti-tumor antibiotic, tyrosine kinase inhibitor, alkylating agent, anti-microtubule or anti-mitotic agent), anti-allergic agent, anti-nausea agent (or anti-emetic), pain reliever, or cytoprotective agent described herein.

In instances, a CAR-expressing cell described herein, optionally in combination with a BTK inhibitor, e.g., a compound of formula (I), is administered to a subject in combination with a B-cell lymphoma 2 (BCL-2) inhibitor (e.g., venetoclax, also called ABT-199 or GDC-0199) and/or rituximab. In instances, a CAR-expressing cell described herein is administered to a subject in combination with venetoclax and rituximab. Venetoclax is a small molecule that inhibits the anti-apoptotic protein, BCL-2. The structure of venetoclax (4-(4-{[2-(4-chlorophenyl)-4,4-dimethylcyclohex-1-en-1-yl]methyl}piperazin-1-yl)-*N*-({3-nitro-4-[(tetrahydro-2*H*-pyran-4-ylmethyl)amino]phenyl}sulfonyl)-2-(1*H*-pyrrolo[2,3-*b*]pyridin-5-yloxy)benzamide) is shown below.

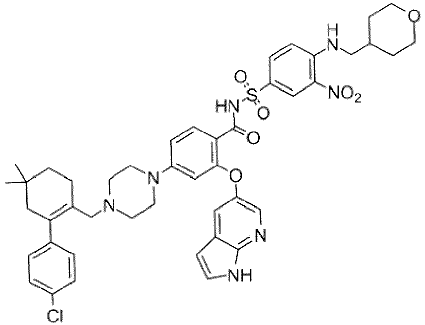

In instances, the subject has CLL. In instances, the subject has relapsed CLL, e.g., the subject has previously been administered a cancer therapy. In instances, venetoclax is administered at a dosage of about 15-600 mg (e.g., 15-20, 20-50, 50-75, 75-100, 100-200, 200-300, 300-400, 400-500, or 500-600 mg), e.g., daily. In instances, rituximab is administered at a dosage of about 350-550 mg/m2 (e.g., 350-375, 375-400, 400-425, 425-450, 450-475, or 475-500 mg/m2), e.g., intravenously, e.g., monthly.

In some instances, one or more CAR-expressing cells described herein, optionally in combination with a BTK inhibitor, e.g., a compound of formula (I), is administered in combination with an oncolytic virus. In instances, oncolytic viruses are capable of selectively replicating in and triggering the death of or slowing the growth of a cancer cell. In some cases, oncolytic viruses have no effect or a minimal effect on non-cancer cells. An oncolytic virus includes but is not limited to an oncolytic adenovirus, oncolytic Herpes Simplex Viruses, oncolytic retrovirus, oncolytic parvovirus, oncolytic vaccinia virus, oncolytic Sinbis virus, oncolytic influenza virus, or oncolytic RNA virus (e.g., oncolytic reovirus, oncolytic Newcastle Disease Virus (NDV), oncolytic measles virus, or oncolytic vesicular stomatitis virus (VSV)).

In some instances, the oncolytic virus is a virus, e.g., recombinant oncolytic virus, described in US2010/0178684 A1. In some instances, a recombinant oncolytic virus comprises a nucleic acid sequence (e.g., heterologous nucleic acid sequence) encoding an inhibitor of an immune or inflammatory response, e.g., as described in US2010/0178684 A1. In instances, the recombinant oncolytic virus, e.g., oncolytic NDV, comprises a pro-apoptotic protein (e.g., apoptin), a cytokine (e.g., GM-CSF, interferon-gamma, interleukin-2 (IL-2), tumor necrosis factor-alpha), an immunoglobulin (e.g., an antibody against ED-B firbonectin), tumor associated antigen, a bispecific adapter protein (e.g., bispecific antibody or antibody fragment directed against NDV HN protein and a T cell co-stimulatory receptor, such as CD3 or CD28; or fusion protein between human IL-2 and single chain antibody directed against NDV HN protein). See, e.g., Zamarin et al. Future Microbiol. 7.3(2012):347-67. In some instances, the oncolytic virus is a chimeric oncolytic NDV described in US 8591881 B2, US 2012/0122185 A1, or US 2014/0271677 A1.

In some instances, the oncolytic virus comprises a conditionally replicative adenovirus (CRAd), which is designed to replicate exclusively in cancer cells. See, e.g., Alemany et al. Nature Biotechnol. 18(2000):723-27. In some instances, an oncolytic adenovirus comprises one described in Table 1 on page 725 of Alemany et al.

Exemplary oncolytic viruses include but are not limited to the following:
Group B Oncolytic Adenovirus (ColoAd1) (PsiOxus Therapeutics Ltd.) (see, e.g., Clinical Trial Identifier: NCT02053220);
ONCOS-102 (previously called CGTG-102), which is an adenovirus comprising granulocyte-macrophage colony stimulating factor (GM-CSF) (Oncos Therapeutics) (see, e.g., Clinical Trial Identifier: NCT01598129);
VCN-01, which is a genetically modified oncolytic human adenovirus encoding human PH20 hyaluronidase (VCN Biosciences, S.L.) (see, e.g., Clinical Trial Identifiers: NCT02045602 and NCT02045589);
Conditionally Replicative Adenovirus ICOVIR-5, which is a virus derived from wild-type human adenovirus serotype 5 (Had5) that has been modified to selectively replicate in cancer cells with a deregulated retinoblastoma/E2F pathway (Institut Català d'Oncologia) (see, e.g., Clinical Trial Identifier: NCT01864759);
Celyvir, which comprises bone marrow-derived autologous mesenchymal stem cells (MSCs) infected with ICOVIR5, an oncolytic adenovirus (Hospital Infantil Universitario Niño Jesus, Madrid, Spain/ Ramon Alemany) (see, e.g., Clinical Trial Identifier: NCT01844661);
CG0070, which is a conditionally replicating oncolytic serotype 5 adenovirus (Ad5) in which human E2F-1 promoter drives expression of the essential E1a viral genes, thereby restricting viral replication and cytotoxicity to Rb pathway-defective tumor cells (Cold Genesys, Inc.) (see, e.g., Clinical Trial Identifier: NCT02143804); or
DNX-2401 (formerly named Delta-24-RGD), which is an adenovirus that has been engineered to replicate selectively in retinoblastoma (Rb)-pathway deficient cells and to infect cells that express certain RGD-binding integrins more efficiently (Clinica Universidad de Navarra, Universidad de Navarra/ DNAtrix, Inc.) (see, e.g., Clinical Trial Identifier: NCT01956734).

In some instances, an oncolytic virus described herein is administering by injection, e.g., subcutaneous, intra-arterial, intravenous, intramuscular, intrathecal, or intraperitoneal injection. In instances, an oncolytic virus described herein is administered intratumorally, transdermally, transmucosally, orally, intranasally, or via pulmonary administration.

In an instance, cells expressing a CAR described herein, in combination with a BTK inhibitor, e.g., a compound of formula (I), are administered to a subject in combination with a molecule that decreases the Treg cell population. Methods that decrease the number of (e.g., deplete) Treg cells are known in the art and include, e.g., CD25 depletion, cyclophosphamide administration, modulating GITR function. Without wishing to be bound by theory, it is believed that reducing the number of Treg cells in a subject prior to apheresis or prior to administration of a CAR-expressing cell described herein reduces the number of unwanted immune cells (e.g., Tregs) in the tumor microenvironment and reduces the subject's risk of relapse. In one instance, cells expressing a CAR described herein, in combination with a BTK inhibitor, e.g., a compound of formula (I), are administered to a subject in combination with a molecule targeting GITR and/or modulating GITR functions, such as a GITR agonist and/or a GITR antibody that depletes regulatory T cells (Tregs). In instances, cells expressing a CAR described herein, in combination with a BTK inhibitor, e.g., a compound of formula (I), are administered to a subject in combination with cyclophosphamide. In one instance, the GITR binding molecules and/or molecules modulating GITR functions (e.g., GITR agonist and/or Treg depleting GITR antibodies) are administered prior to administration of the CAR-expressing cell. For example, in one instance, the GITR agonist can be administered prior to apheresis of the cells. In instances, cyclophosphamide is administered to the subject prior to administration (e.g., infusion or re-infusion) of the CAR-expressing cell or prior to apheresis of the cells. In instances, cyclophosphamide and an anti-GITR antibody are administered to the subject prior to administration (e.g., infusion or re-infusion) of the CAR-expressing cell or prior to apheresis of the cells. In one instance, the subject has cancer (e.g., a solid cancer or a hematological cancer such as ALL or CLL). In an instance, the subject has CLL. In instances, the subject has ALL. In instances, the subject has a solid cancer, e.g., a solid cancer described herein.

Exemplary GITR agonists include, e.g., GITR fusion proteins and anti-GITR antibodies (e.g., bivalent anti-GITR antibodies) such as, e.g., a GITR fusion protein described in U.S. Patent No.: 6,111,090, European Patent No.: 090505B1, U.S Patent No.: 8,586,023, PCT Publication Nos.: WO 2010/003118 and 2011/090754, or an anti-GITR antibody described, e.g., in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, PCT Publication No.:WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Patent No.: 7,618,632, and PCT Publication No.: WO 2011/051726.

In one instance, the combination of a CAR expressing cell described herein and a BTK inhibitor, e.g., a compound of formula (I), is administered to a subject in combination with a GITR agonist, e.g., a GITR agonist described herein. In one instance, the GITR agonist is administered prior to the CAR-expressing cell. For example, in one instance, the GITR agonist can be administered prior to apheresis of the cells. In one instance, the subject has CLL.

In one instance, a CAR expressing cell described herein, optionally in combination with a BTK inhibitor, e.g., a compound of formula (I), is administered to a subject in combination with a protein tyrosine phosphatase inhibitor, e.g., a protein tyrosine phosphatase inhibitor described herein. In one instance, the protein tyrosine phosphatase inhibitor is an SHP-1 inhibitor, e.g., an SHP-1 inhibitor described herein, such as, e.g., sodium stibogluconate. In one instance, the protein tyrosine phosphatase inhibitor is an SHP-2 inhibitor, e.g., an SHP-2 inhibitor described herein.

In one instance, a CD19 CAR-expressing cell described herein, optionally in combination with a BTK inhibitor, e.g., a compound of formula (I), can be used in combination with another kinase inhibitor. In one instance, the kinase inhibitor is a CDK4 inhibitor, e.g., a CDK4 inhibitor described herein, e.g., a CD4/6 inhibitor, such as, e.g., 6-Acetyl-8-cyclopentyl-5-methyl-2-(5-piperazin-1-yl-pyridin-2-ylamino)-8*H*-pyrido[2,3-*d*]pyrimidin-7-one, hydrochloride (also referred to as palbociclib or PD0332991). In one instance, the kinase inhibitor is a BTK inhibitor, e.g., a BTK inhibitor described herein, such as, e.g., ibrutinib. In one instance, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one instance, the kinase inhibitor is a MNK inhibitor, e.g., a MNK inhibitor described herein, such as, e.g., 4-amino-5-(4-fluoroanilino)-pyrazolo [3,4-d] pyrimidine. The MNK inhibitor can be, e.g., a MNK1a, MNK1b, MNK2a and/or MNK2b inhibitor.

In one instance, the kinase inhibitor is a CDK4 inhibitor selected from aloisine A; flavopiridol or HMR-1275, 2-(2-chlorophenyl)-5,7-dihydroxy-8-[(3S,4R)-3-hydroxy-1-methyl-4-piperidinyl]-4-chromenone; crizotinib (PF-02341066; 2-(2-Chlorophenyl)-5,7-dihydroxy-8-[(2*R*,3*S*)-2-(hydroxymethyl)-1-methyl-3-pyrrolidinyl]-4H-1-benzopyran-4-one, hydrochloride (P276-00); 1-methyl-5-[[2-[5-(trifluoromethyl)-1*H*-imidazol-2-yl]-4-pyridinyl]oxy]-*N*-[4-(trifluoromethyl)phenyl]-1*H*-benzimidazol-2-amine (RAF265); indisulam (E7070); roscovitine (CYC202); palbociclib (PD0332991); dinaciclib (SCH727965); N-[5-[[(5-*tert*-butyloxazol-2-yl)methyl]thio]thiazol-2-yl]piperidine-4-carboxamide (BMS 387032); 4-[[9-chloro-7-(2,6-difluorophenyl)-5*H*-pyrimido[5,4-*d*][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1H-imidazol-5-yl]-N-[4-(methylsulfonyl)phenyl]- 2-pyrimidinamine (AZD5438); and XL281 (BMS908662).

In one instance, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one instance, 1, 2, 3, 4, 5, 6, 7, 8, 9, 10, 11, 12 or more cycles of palbociclib are administered.

In instances, a CAR-expressing cell described herein, optionally in combination with a BTK inhibitor, e.g., a compound of formula (I), is administered to a subject in combination with a cyclin-dependent kinase (CDK) 4 or 6 inhibitor, e.g., a CDK4 inhibitor or a CDK6 inhibitor described herein. In instances, a CAR-expressing cell described herein is administered to a subject in combination with a CDK4/6 inhibitor (e.g., an inhibitor that targets both CDK4 and CDK6), e.g., a CDK4/6 inhibitor described herein. In an instance, the subject has MCL. MCL is an aggressive cancer that is poorly responsive to currently available therapies, i.e., essentially incurable. In many cases of MCL, cyclin D1 (a regulator of CDK4/6) is expressed (e.g., due to chromosomal translocation involving immunoglobulin and Cyclin D1 genes) in MCL cells. Thus, without being bound by theory, it is thought that MCL cells are highly sensitive to CDK4/6 inhibition with high specificity (i.e., minimal effect on normal immune cells). CDK4/6 inhibitors alone have had some efficacy in treating MCL, but have only achieved partial remission with a high relapse rate. An exemplary CDK4/6 inhibitor is LEE011 (also called ribociclib), the structure of which is shown below.

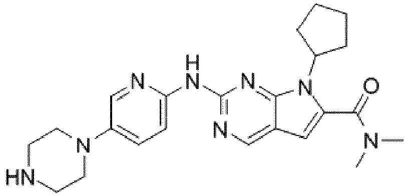

Without being bound by theory, it is believed that administration of a CAR-expressing cell described herein, optionally in combination with a BTK inhibitor, e.g., a compound of formula (I), with a CDK4/6 inhibitor (e.g., LEE011 or other CDK4/6 inhibitor described herein) can achieve higher responsiveness, e.g., with higher remission rates and/or lower relapse rates, e.g., compared to a CDK4/6 inhibitor alone.

In one instance, the kinase inhibitor is an mTOR inhibitor selected from temsirolimus; ridaforolimus (*1R,2R,4S*)-4-[(2*R*)*-*2[(1*R,*9*S,*12*S,*15*R,*16*E,*18*R,*19*R,*21*R,*23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3*S*)-3-methylmorpholin-4-yl]pyrido[2,3-*d*]pyrimidin-7-yl}-2-methoxyphenyl)methanol (AZD8055); 2-amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126) (SEQ ID NO: 134); and XL765.

In one instance, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle cycle, or daily for 28 day cycle. In one instance, 1, 2, 3, 4, 5, 6, 7, 8, 9, 10, 11, 12 or more cycles of rapamycin are administered. In one instance, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one instance, 1, 2, 3, 4, 5, 6, 7, 8, 9, 10, 11, 12 or more cycles of everolimus are administered.

In one instance, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4-*d*] pyrimidine.

In instances, a CAR-expressing cell described herein, optionally in combination with a BTK inhibitor, e.g., a compound of formula (I), is administered to a subject in combination with a phosphoinositide 3-kinase (PI3K) inhibitor (e.g., a PI3K inhibitor described herein, e.g., idelalisib or duvelisib) and/or rituximab. In instances, a CAR-expressing cell described herein is administered to a subject in combination with idelalisib and rituximab. In instances, a CAR-expressing cell described herein is administered to a subject in combination with duvelisib and rituximab. Idelalisib (also called GS-1101 or CAL-101; Gilead) is a small molecule that blocks the delta isoform of PI3K. The structure of idelalisib (5-Fluoro-3-phenyl-2-[(1*S*)-1-(7*H*-purin-6-ylamino)propyl]-4(3*H*)-quinazolinone) is shown below.

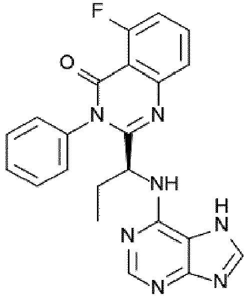

Duvelisib (also called IPI-145; Infinity Pharmaceuticals and Abbvie) is a small molecule that blocks PI3K-δ,γ. The structure of duvelisib (8-Chloro-2-phenyl-3-[(1S)-1-(9H-purin-6-ylamino)ethyl]-1(2H)-isoquinolinone) is shown below.

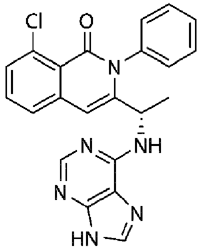

In instances, the subject has CLL. In instances, the subject has relapsed CLL, e.g., the subject has previously been administered a cancer therapy (e.g., previously been administered an anti-CD20 antibody or previously been administered ibrutinib). For example, the subject has a deletion in the short arm of chromosome 17 (del(17p), e.g., in a leukemic cell). In other examples, the subject does not have a del(17p). In instances, the subject comprises a leukemic cell comprising a mutation in the immunoglobulin heavy-chain variable-region (*IgV_{H}*) gene. In other instances, the subject does not comprise a leukemic cell comprising a mutation in the immunoglobulin heavy-chain variable-region (*IgV_{H}*) gene. In instances, the subject has a deletion in the long arm of chromosome 11 (del(11q)). In other instances, the subject does not have a del(11q). In instances, idelalisib is administered at a dosage of about 100-400 mg (e.g., 100-125, 125-150, 150-175, 175-200, 200-225, 225-250, 250-275, 275-300, 325-350, 350-375, or 375-400 mg), e.g., BID. In instances, duvelisib is administered at a dosage of about 15-100 mg (e.g., about 15-25, 25-50, 50-75, or 75-100 mg), e.g., twice a day. In instances, rituximab is administered at a dosage of about 350-550 mg/m² (e.g., 350-375, 375-400, 400-425, 425-450, 450-475, or 475-500 mg/m²), e.g., intravenously.

In one instance, the kinase inhibitor is a dual phosphatidylinositol 3-kinase (PI3K) and mTOR inhibitor selected from 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF-04691502); *N*-[4-[[4-(Dimethylamino)-1-piperidinyl]carbonyl]phenyl]-*N'*-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl}benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In instances, a CAR-expressing cell described herein, optionally in combination with a BTK inhibitor, e.g., a compound of formula (I), is administered to a subject in combination with an anaplastic lymphoma kinase (ALK) inhibitor. Exemplary ALK kinases include but are not limited to crizotinib (Pfizer), ceritinib (Novartis), alectinib (Chugai), brigatinib (also called *AP26113;* Ariad), entrectinib (Ignyta), PF-06463922 (Pfizer), TSR-011 (Tesaro) (see, e.g., Clinical Trial Identifier No. NCT02048488), CEP-37440 (Teva), and X-396 (Xcovery). In some instances, the subject has a solid cancer, e.g., a solid cancer described herein, e.g., lung cancer.

The chemical name of crizotinib is 3-[(1*R*)-1-(2,6-dichloro-3-fluorophenyl)ethoxy]-5-(1-piperidin-4-ylpyrazol-4-yl)pyridin-2-amine. The chemical name of ceritinib is 5-Chloro-*N*²-[2-isopropoxy-5-methyl-4-(4-piperidinyl)phenyl]-*N*⁴-[2-(isopropylsulfonyl)phenyl]-2,4-pyrimidinediamine. The chemical name of alectinib is 9-ethyl-6,6-dimethyl-8-(4-morpholinopiperidin-1-yl)-11-0x0-6,11-dihydro-5H-benzo[b]carbazole-3-carbonitrile. The chemical name of brigatinib is 5-Chloro-N²-{4-[4-(dimethylamino)-1-piperidinyl]-2-methoxyphenyl}-N⁴-[2-(dimethylphosphoryl)phenyl]-2,4-pyrimidinediamine. The chemical name of entrectinib is N-(5-(3,5-difluorobenzyl)-1H-indazol-3-yl)-4-(4-methylpiperazin-1-yl)-2-((tetrahydro-2H-pyran-4-yl)amino)benzamide. The chemical name of PF-06463922 is (10R)-7-Amino-12-fluoro-2,10,16-trimethyl-15-oxo-10,15,16,17-tetrahydro-2H-8,4-(metheno)pyrazolo[4,3-h][2,5,11]-benzoxadiazacyclotetradecine-3-carbonitrile. The chemical structure of CEP-37440 is (S)-2-((5-chloro-2-((6-(4-(2-hydroxyethyl)piperazin-1-yl)-1-methoxy-6,7,8,9-tetrahydro-5H-benzo[7]annulen-2-yl)amino)pyrimidin-4-yl)amino)-N-methylbenzamide. The chemical name of X-396 is (R)-6-amino-5-(1-(2,6-dichloro-3-fluorophenyl)ethoxy)-N-(4-(4-methylpiperazine-1-carbonyl)phenyl)pyridazine-3-carboxamide.

In one instance, the kinase inhibitor is an ITK inhibitor selected from ibrutinib; N-(5-(5-(4-Acetylpiperazine-1-carbonyl)-4-methoxy-2-methylphenylthio)thiazol-2-yl)-4-((3,3-dimethylbutan-2-ylamino)methyl)benzamide (BMS-509744); 7-benzyl-1-(3-(piperidin-1-yl)propyl)-2-(4-(pyridin-4-yl)phenyl)-1H-imidazo[4,5-g]quinoxalin-6(5H)-one (CTA056); *R*)-3-(1-(1-Acryloylpiperidin-3-yl)-4-amino-1*H*-pyrazolo[3,4-d]pyrimidin-3-yl)-*N*-(3-methyl-4-(1-methylethyl))benzamide (PF-06465469).

Drugs that inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993) can also be used. In a further aspect, the cell compositions of the present disclosure may be administered to a patient in conjunction with (e.g., before, simultaneously or following) bone marrow transplantation, T cell ablative therapy using chemotherapy agents such as, fludarabine, external-beam radiation therapy (XRT), cyclophosphamide, and/or antibodies such as OKT3 or CAMPATH. In one aspect, the cell compositions of the present disclosure are administered following B-cell ablative therapy such as agents that react with CD20, e.g., Rituxan. For example, in one instance, subjects may undergo standard treatment with high dose chemotherapy followed by peripheral blood stem cell transplantation. In certain instances, following the transplant, subjects receive an infusion of the expanded immune cells of the present disclosure. In an additional instance, expanded cells are administered before or following surgery.

In instances, a CAR-expressing cell described herein, optionally in combination with a BTK inhibitor, e.g., a compound of formula (I), is administered to a subject in combination with an indoleamine 2,3-dioxygenase (IDO) inhibitor. IDO is an enzyme that catalyzes the degradation of the amino acid, L-tryptophan, to kynurenine. Many cancers overexpress IDO, e.g., prostatic, colorectal, pancreatic, cervical, gastric, ovarian, head, and lung cancer. pDCs, macrophages, and dendritic cells (DCs) can express IDO. Without being bound by theory, it is thought that a decrease in L-tryptophan (e.g., catalyzed by IDO) results in an immunosuppressive milieu by inducing T-cell anergy and apoptosis. Thus, without being bound by theory, it is thought that an IDO inhibitor can enhance the efficacy of a CAR-expressing cell described herein, e.g., by decreasing the suppression or death of a CAR-expressing immune cell. In instances, the subject has a solid tumor, e.g., a solid tumor described herein, e.g., prostatic, colorectal, pancreatic, cervical, gastric, ovarian, head, or lung cancer. Exemplary inhibitors of IDO include but are not limited to 1-methyl-tryptophan, indoximod (NewLink Genetics) (see, e.g., Clinical Trial Identifier Nos. NCT01191216; NCT01792050), and INCB024360 (Incyte Corp.) (see, e.g., Clinical Trial Identifier Nos. NCT01604889; NCT01685255)

In instances, a CAR-expressing cell described herein, optionally in combination with a BTK inhibitor, e.g., a compound of formula (I), is administered to a subject in combination with a modulator of myeloid-derived suppressor cells (MDSCs). MDSCs accumulate in the periphery and at the tumor site of many solid tumors. These cells suppress T cell responses, thereby hindering the efficacy of CAR-expressing cell therapy. Without being bound by theory, it is thought that administration of a MDSC modulator enhances the efficacy of a CAR-expressing cell described herein. In an instance, the subject has a solid tumor, e.g., a solid tumor described herein, e.g., glioblastoma. Exemplary modulators of MDSCs include but are not limited to MCS110 and BLZ945. MCS110 is a monoclonal antibody (mAb) against macrophage colony-stimulating factor (M-CSF). See, e.g., Clinical Trial Identifier No. NCT00757757. BLZ945 is a small molecule inhibitor of colony stimulating factor 1 receptor (CSF1R). See, e.g., Pyonteck et al. Nat. Med. 19(2013):1264-72. The structure of BLZ945 is shown below.

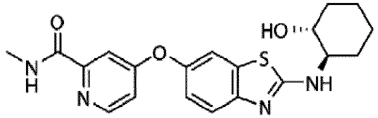

In some instances , a CAR-expressing cell described herein, optionally in combination with a BTK inhibitor, e.g., a compound of formula (I), is administered to a subject in combination with a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15 (Admune Therapeutics, LLC). hetIL-15 is a heterodimeric non-covalent complex of IL-15 and IL-15Ra. hetIL-15 is described in, e.g., U.S. 8,124,084, U.S. 2012/0177598, U.S. 2009/0082299, U.S. 2012/0141413, and U.S. 2011/0081311. In instances, het-IL-15 is administered subcutaneously. In instances, the subject has a cancer, e.g., solid cancer, e.g., melanoma or colon cancer. In instances, the subject has a metastatic cancer.

In instances, a subject having a disease described herein, e.g., a hematological disorder, e.g., AML or MDS, is administered a CAR-expressing cell described herein, optionally in combination with a BTK inhibitor, e.g., a compound of formula (I), in combination with an agent, e.g., cytotoxic or chemotherapy agent, a biologic therapy (e.g., antibody, e.g., monoclonal antibody, or cellular therapy), or an inhibitor (e.g., kinase inhibitor). In instances, the subject is administered a CAR-expressing cell described herein, optionally in combination with a BTK inhibitor, e.g., a compound of formula (I), in combination with a cytotoxic agent, e.g., CPX-351 (Celator Pharmaceuticals), cytarabine, daunorubicin, vosaroxin (Sunesis Pharmaceuticals), sapacitabine (Cyclacel Pharmaceuticals), idarubicin, or mitoxantrone. CPX-351 is a liposomal formulation comprising cytarabine and daunorubicin at a 5:1 molar ratio. In instances, the subject is administered a CAR-expressing cell described herein, optionally in combination with a BTK inhibitor, e.g., a compound of formula (I), in combination with a hypomethylating agent, e.g., a DNA methyltransferase inhibitor, e.g., azacitidine or decitabine. In instances, the subject is administered a CAR-expressing cell described herein, optionally in combination with a BTK inhibitor, e.g., a compound of formula (I), in combination with a biologic therapy, e.g., an antibody or cellular therapy, e.g., 225Ac-lintuzumab (Actimab-A; Actinium Pharmaceuticals), IPH2102 (Innate Pharma/Bristol Myers Squibb), SGN-CD33A (Seattle Genetics), or gemtuzumab ozogamicin (Mylotarg; Pfizer). SGN-CD33A is an antibody-drug conjugate (ADC) comprising a pyrrolobenzodiazepine dimer that is attached to an anti-CD33 antibody. Actimab-A is an anti-CD33 antibody (lintuzumab) labeled with actinium. IPH2102 is a monoclonal antibody that targets killer immunoglobulin-like receptors (KIRs). In instances, the subject is administered a CAR-expressing cell described herein, optionally in combination with a BTK inhibitor, e.g., a compound of formula (I), in combination a FLT3 inhibitor, e.g., sorafenib (Bayer), midostaurin (Novartis), quizartinib (Daiichi Sankyo), crenolanib (Arog Pharmaceuticals), PLX3397 (Daiichi Sankyo), AKN-028 (Akinion Pharmaceuticals), or ASP2215 (Astellas). In instances, the subject is administered a CAR-expressing cell described herein in combination with an isocitrate dehydrogenase (IDH) inhibitor, e.g., AG-221 (Celgene/Agios) or AG-120 (Agios/Celgene). In instances, the subject is administered a CAR-expressing cell described herein, optionally in combination with a BTK inhibitor, e.g., a compound of formula (I), in combination with a cell cycle regulator, e.g., inhibitor of polo-like kinase 1 (Plk1), e.g., volasertib (Boehringer Ingelheim); or an inhibitor of cyclin-dependent kinase 9 (Cdk9), e.g., alvocidib (Tolero Pharmaceuticals/Sanofi Aventis). In instances, the subject is administered a CAR-expressing cell described herein, optionally in combination with a BTK inhibitor, e.g., a compound of formula (I), in combination with a B cell receptor signaling network inhibitor, e.g., an inihibitor of B-cell lymphoma 2 (Bcl-2), e.g., venetoclax (Abbvie/Roche); or an inhibitor of Bruton's tyrosine kinase (Btk), e.g., ibrutinib (Pharmacyclics/Johnson & Johnson Janssen Pharmaceutical). In instances, the subject is administered a CAR-expressing cell described herein, optionally in combination with a BTK inhibitor, e.g., a compound of formula (I), in combination with an inhibitor of M1 aminopeptidase, e.g., tosedostat (CTI BioPharma/Vernalis); an inhibitor of histone deacetylase (HDAC), e.g., pracinostat (MEI Pharma); a multi-kinase inhibitor, e.g., rigosertib (Onconova Therapeutics/Baxter/SymBio); or a peptidic CXCR4 inverse agonist, e.g., BL-8040 (BioLineRx).

In another instance, the subjects receive an infusion of the CAR expressing cell, e.g., CD19 CAR-expressing cell, compositions of the present disclosure, optionally in combination with a BTK inhibitor, e.g., a compound of formula (I), prior to transplantation, e.g., allogeneic stem cell transplant, of cells. In a preferred instance, CAR expressing cells transiently express the CAR, e.g., by electroporation of an mRNA CAR, whereby the expression of the antigen targeted by the CAR, e.g., CD19 is terminated prior to infusion of donor stem cells to avoid engraftment failure.

In one instance, the subject can be administered an agent which reduces or ameliorates a side effect associated with the administration of a CAR-expressing cell. Side effects associated with the administration of a CAR-expressing cell include, but are not limited to CRS, and hemophagocytic lymphohistiocytosis (HLH), also termed Macrophage Activation Syndrome (MAS). Symptoms of CRS include high fevers, nausea, transient hypotension, hypoxia, and the like. Accordingly, the methods described herein can comprise administering a CAR-expressing cell described herein to a subject and further administering an agent to manage elevated levels of a soluble factor resulting from treatment with a CAR-expressing cell. In one instance, the soluble factor elevated in the subject is one or more of IFN-γ, TNFα, IL-2 receptor and IL-6. Therefore, an agent administered to treat this side effect can be an agent that neutralizes one or more of these soluble factors. Examples of such agents include, but are not limited to a steroid (e.g., corticosteroid), an inhibitor of TNFα, and an inhibitor of IL-6. An example of a TNFα inhibitor is an anti-TNFa antibody molecule such as, infliximab, adalimumab, certolizumab pegol, and golimumab. Another example of a TNFα inhibitor is a fusion protein such as entanercept. Small molecule inhibitor of TNFα include, but are not limited to, xanthine derivatives (e.g. pentoxifylline) and bupropion. An example of an IL-6 inhibitor is an anti-IL-6 antibody molecule or anti-IL-6 receptor antibody molecule such as tocilizumab (toc), sarilumab, elsilimomab, CNTO 328, ALD518/BMS-945429, CNTO 136, CPSI-2364, CDP6038, VX30, ARGX-109, FE301, and FM101. In one instance, the anti-IL-6 receptor antibody molecule is tocilizumab. An example of an IL-1R based inhibitor is anakinra.

In instances, lymphodepletion is performed on a subject, e.g., prior to administering one or more cells that express a CAR described herein, optionally in combination with a BTK inhibitor, e.g., a compound of formula (I). In instances, the lymphodepletion comprises administering one or more of melphalan, cytoxan, cyclophosphamide, and fludarabine.

In instances, a lymphodepleting chemotherapy is administered to the subject prior to, concurrently with, or after administration (e.g., infusion) of CAR cells, e.g., cells described herein, optionally in combination with a BTK inhibitor, e.g., a compound of formula (I). In an example, the lymphodepleting chemotherapy is administered to the subject prior to administration of CAR cells, optionally in combination with a BTK inhibitor, e.g., a compound of formula (I). For example, the lymphodepleting chemotherapy ends 1-4 days (e.g., 1, 2, 3, or 4 days) prior to CAR cell infusion, optionally in combination with a BTK inhibitor, e.g., a compound of formula (I). In instances, multiple doses of CAR cells are administered, e.g., as described herein. For example, a single dose comprises about 5 x 10⁸ CAR cells. In instances, a lymphodepleting chemotherapy is administered to the subject prior to, concurrently with, or after administration (e.g., infusion) of a CAR-expressing cell described herein, optionally in combination with a BTK inhibitor, e.g., a compound of formula (I).

### Inhibitory Molecule Inhibitors/Checkpoint Inhibitors

In one embodiment, the subject can be administered an agent which enhances the activity of a CAR-expressing cell. For example, in one embodiment, the agent can be an agent which inhibits an inhibitory molecule. Inhibitory molecules, e.g., Programmed Death 1 (PD1), can, in some embodiments, decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR (e.g., TGFR beta). In instances, the CAR-expressing cell described herein comprises a switch costimulatory receptor, e.g., as described in WO 2013/019615.

The methods described herein can include administration of a CAR-expressing cell, optionally in combination with a BTK inhibitor, e.g., a compound of formula (I), in combination with a checkpoint inhibitor. In one instance, the subject is a complete responder. In another instance, the subject is a partial responder or non-responder, and, e.g., in some instance, the checkpoint inhibitor is administered prior to the CAR-expressing cell, e.g., two weeks, 12 days, 10 days, 8 days, one week, 6 days, 5 days, 4 days, 3 days, 2 days or 1 day before administration of the CAR-expressing cell. In some instances, the checkpoint inhibitor is administered concurrently with the CAR-expressing cell.

Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), can be used to inhibit expression of an inhibitory molecule in the CAR-expressing cell. In an embodiment the inhibitor is an shRNA. In an embodiment, the inhibitory molecule is inhibited within a CAR-expressing cell. In these embodiments, a dsRNA molecule that inhibits expression of the inhibitory molecule is linked to the nucleic acid that encodes a component, e.g., all of the components, of the CAR.

In an embodiment, a nucleic acid molecule that encodes a dsRNA molecule that inhibits expression of the molecule that modulates or regulates, e.g., inhibits, T-cell function is operably linked to a promoter, e.g., a H1- or a U6-derived promoter such that the dsRNA molecule that inhibits expression of the molecule that modulates or regulates, e.g., inhibits, T-cell function is expressed, e.g., is expressed within a CAR-expressing cell. See e.g., Tiscornia G., "Development of Lentiviral Vectors Expressing siRNA," Chapter 3, in Gene Transfer: Delivery and Expression of DNA and RNA (eds. Friedmann and Rossi). Cold Spring Harbor Laboratory Press, Cold Spring Harbor, NY, USA, 2007; Brummelkamp TR, et al. (2002) Science 296: 550-553; Miyagishi M, et al. (2002) Nat. Biotechnol. 19: 497-500. In an embodiment the nucleic acid molecule that encodes a dsRNA molecule that inhibits expression of the molecule that modulates or regulates, e.g., inhibits, T-cell function is present on the same vector, e.g., a lentiviral vector, that comprises a nucleic acid molecule that encodes a component, e.g., all of the components, of the CAR. In such an embodiment, the nucleic acid molecule that encodes a dsRNA molecule that inhibits expression of the molecule that modulates or regulates, e.g., inhibits, T-cell function is located on the vector, e.g., the lentiviral vector, 5'- or 3'- to the nucleic acid that encodes a component, e.g., all of the components, of the CAR. The nucleic acid molecule that encodes a dsRNA molecule that inhibits expression of the molecule that modulates or regulates, e.g., inhibits, T-cell function can be transcribed in the same or different direction as the nucleic acid that encodes a component, e.g., all of the components, of the CAR. In an embodiment the nucleic acid molecule that encodes a dsRNA molecule that inhibits expression of the molecule that modulates or regulates, e.g., inhibits, T-cell function is present on a vector other than the vector that comprises a nucleic acid molecule that encodes a component, e.g., all of the components, of the CAR. In an embodiment, the nucleic acid molecule that encodes a dsRNA molecule that inhibits expression of the molecule that modulates or regulates, e.g., inhibits, T-cell function it transiently expressed within a CAR-expressing cell. In an embodiment, the nucleic acid molecule that encodes a dsRNA molecule that inhibits expression of the molecule that modulates or regulates, e.g., inhibits, T-cell function is stably integrated into the genome of a CAR-expressing cell. In an embodiment, the molecule that modulates or regulates, e.g., inhibits, T-cell function is PD-1.

In one embodiment, the inhibitor of an inhibitory signal can be, e.g., an antibody or antibody fragment that binds to an inhibitory molecule. For example, the agent can be an antibody or antibody fragment that binds to PD1, PD-L1, PD-L2 or CTLA4 (e.g., ipilimumab (also referred to as MDX-010 and MDX-101, and marketed as Yervoy®; Bristol-Myers Squibb; Tremelimumab (IgG2 monoclonal antibody available from Pfizer, formerly known as ticilimumab, CP-675,206).). In an embodiment, the agent is an antibody or antibody fragment that binds to TIM3. In an embodiment, the agent is an antibody or antibody fragment that binds to LAG3. In an embodiment, the agent is an antibody or antibody fragment that binds to CEACAM (e.g., CEACAM-1, CEACAM-3 and/or CEACAM-5). In instances, the agent that enhances the activity of a CAR-expressing cell, e.g., inhibitor of an inhibitory molecule, is administered in combination with an allogeneic CAR, e.g., an allogeneic CAR described herein (e.g., described in the Allogeneic CAR section herein).

PD1 is an inhibitory member of the CD28 family of receptors that also includes CD28, CTLA-4, ICOS, and BTLA. PD1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et al. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

Antibodies, antibody fragments, and other inhibitors of PD1, PD-L1 and PD-L2 are known and may be used combination with a CD19 CAR described herein. For example, nivolumab (also referred to as BMS-936558 or MDX1106; Bristol-Myers Squibb) is a fully human IgG4 monoclonal antibody which specifically blocks PD1. Nivolumab (clone 5C4) and other human monoclonal antibodies that specifically bind to PD1 are disclosed in US 8,008,449 and WO2006/121168. Pidilizumab (CT-011; Cure Tech) is a humanized IgGlk monoclonal antibody that binds to PD1. Pidilizumab and other humanized anti-PDl monoclonal antibodies are disclosed in WO2009/101611. Pembrolizumab (formerly known as lambrolizumab, and also referred to as Keytruda, MK03475; Merck) is a humanized IgG4 monoclonal antibody that binds to PD1. Pembrolizumab and other humanized anti-PDl antibodies are disclosed in US 8,354,509 and WO2009/114335. MEDI4736 (Medimmune) is a human monoclonal antibody that binds to PDL1, and inhibits interaction of the ligand with PD1. MDPL3280A (Genentech / Roche) is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906. Other anti-PD-Ll binding agents include YW243.55.S70 (heavy and light chain variable regions are shown in SEQ ID NOs 20 and 21 in WO2010/077634) and MDX-1 105 (also referred to as BMS-936559, and, e.g., anti-PD-Ll binding agents disclosed in WO2007/005874). AMP-224 (B7-DCIg; Amplimmune; e.g., disclosed in WO2010/027827 and WO2011/066342), is a PD-L2 Fc fusion soluble receptor that blocks the interaction between PD1 and B7-H1. Other anti-PDl antibodies include AMP 514 (Amplimmune), among others, e.g., anti-PDl antibodies disclosed in US 8,609,089, US 2010028330, and/or US 20120114649.

In some embodiments, a PD1 inhibitor described herein (e.g., a PD1 antibody, e.g., a PD1 antibody described herein) is used combination with a CD19 CAR described herein to treat a disease associated with expression of CD19. In some embodiments, a PD-L1 inhibitor described herein (e.g., a PD-L1 antibody, e.g., a PD-L1 antibody described herein) is used combination with a CD19 CAR described herein to treat a disease associated with expression of CD19. The disease may be, e.g., a lymphoma such as DLBCL including primary DLBCL or secondary DLBCL. In some embodiments, the subject has, or is identified as having, at least 5%, 6%, 7%, 8%, 9%, 10%, 20%, 30%, 40%, 50%, 60%, 70%, 80%, or 90% of cancer cells, e.g., DLBCL cells, which are CD3+/PD1+. In some embodiments, the subject has, or is identified as having, substantially non-overlapping populations of CD19+ cells and PD-L1+ cells in a cancer, e.g., the cancer microenvironment. For instance, in some embodiments, less than 20%, 10%, 9%, 8%, 7%, 6%, 5%, 4%, 3%, 2%, or 1% of cells in the cancer, e.g., cancer microenvironment, are double positive for CD19 and PD-L1.

In some embodiments, the subject is treated with a combination of a CD19 CAR, a PD1 inhibitor, and a PD-L1 inhibitor. In some embodiments, the subject is treated with a combination of a CD19 CAR, a PD1 inhibitor, and a CD3 inhibitor. In some embodiments, the subject is treated with a combination of a CD19 CAR, a PD1 inhibitor, a PD-L1 inhibitor, and a CD3 inhibitor.

In some instances, the methods herein include a step of assaying cells in a biological sample, e.g., a sample comprising DLBCL cells, for CD3 and/or PD-1 (e.g., CD3 and/or PD-1 expression). In some instances, the methods include a step of assaying cells in a biological sample, e.g., a sample comprising DLBCL cells, for CD19 and/or PD-L1 (e.g., CD19 and/or PD-L1 expression). In some instances, the methods include, e.g., providing a sample comprising cancer cells and performing a detection step, e.g., by immunohistochemistry, for one or more of CD3, PD-1, CD19, or PD-L1. The methods may comprise a further step of recommending or administering a treatment, e.g., a treatment comprising a CD19 CAR.

In one embodiment, the anti-PD-1 antibody or fragment thereof is an anti-PD-1 antibody molecule as described in US 2015/0210769, entitled "Antibody Molecules to PD-1 and Uses Thereof,". In one embodiment, the anti-PD-1 antibody molecule includes at least one, two, three, four, five or six CDRs (or collectively all of the CDRs) from a heavy and light chain variable region from an antibody chosen from any of BAP049-hum01, BAP049-hum02, BAP049-hum03, BAP049-hum04, BAP049-hum05, BAP049-hum06, BAP049-hum07, BAP049-hum08, BAP049-hum09, BAP049-hum10, BAP049-hum1 1, BAP049-hum12, BAP049-hum13, BAP049-hum14, BAP049-hum15, BAP049-hum16, BAP049-Clone-A, BAP049-Clone-B, BAP049-Clone-C, BAP049-Clone-D, or BAP049-Clone-E; or as described in Table 1 of US 2015/0210769, or encoded by the nucleotide sequence in Table 1, or a sequence substantially identical (e.g., at least 80%, 85%, 90%, 92%, 95%, 97%, 98%, 99% or higher identical) to any of the aforesaid sequences; or closely related CDRs, e.g., CDRs which are identical or which have at least one amino acid alteration, but not more than two, three or four alterations (e.g., substitutions, deletions, or insertions, e.g., conservative substitutions).

In yet another embodiment, the anti-PD-1 antibody molecule comprises at least one, two, three or four variable regions from an antibody described herein, e.g., an antibody chosen from any of BAP049-hum01, BAP049-hum02, BAP049-hum03, BAP049-hum04, BAP049-hum05, BAP049-hum06, BAP049-hum07, BAP049-hum08, BAP049-hum09, BAP049-hum10, BAP049-hum11, BAP049-hum12, BAP049-hum13, BAP049-hum14, BAP049-hum15, BAP049-hum16, BAP049-Clone-A, BAP049-Clone-B, BAP049-Clone-C, BAP049-Clone-D, or BAP049-Clone-E; or as described in Table 1 of US 2015/0210769, or encoded by the nucleotide sequence in Table 1; or a sequence substantially identical (e.g., at least 80%, 85%, 90%, 92%, 95%, 97%, 98%, 99% or higher identical) to any of the aforesaid sequences.

TIM3 (T cell immunoglobulin-3) also negatively regulates T cell function, particularly in IFN-g-secreting CD4+ T helper 1 and CD8+ T cytotoxic 1 cells, and plays a critical role in T cell exhaustion. Inhibition of the interaction between TIM3 and its ligands, e.g., galectin-9 (Gal9), phosphatidylserine (PS), and HMGB1, can increase immune response. Antibodies, antibody fragments, and other inhibitors of TIM3 and its ligands are available in the art and may be used combination with a CD19 CAR described herein. For example, antibodies, antibody fragments, small molecules, or peptide inhibitors that target TIM3 binds to the IgV domain of TIM3 to inhibit interaction with its ligands. Antibodies and peptides that inhibit TIM3 are disclosed in WO2013/006490 and US20100247521. Other anti-TIM3 antibodies include humanized versions of RMT3-23 (disclosed in Ngiow et al., 2011, Cancer Res, 71:3540-3551), and clone 8B.2C12 (disclosed in Monney et al., 2002, Nature, 415:536-541). Bi-specific antibodies that inhibit TIM3 and PD-1 are disclosed in US20130156774.

In one embodiment, the anti-TIM3 antibody or fragment thereof is an anti-TIM3 antibody molecule as described in US 2015/0218274, entitled "Antibody Molecules to TIM3 and Uses Thereof,". In one embodiment, the anti-TIM3 antibody molecule includes at least one, two, three, four, five or six CDRs (or collectively all of the CDRs) from a heavy and light chain variable region from an antibody chosen from any of ABTIM3, ABTIM3-hum01, ABTIM3-hum02, ABTIM3-humO3, ABTIM3-hum04, ABTIM3-hum05, ABTIM3-hum06, ABTIM3-hum07, ABTIM3-hum08, ABTIM3-hum09, ABTIM3-hum10, ABTIM3-hum11, ABTIM3-hum12, ABTIM3-hum13, ABTIM3-hum14, ABTIM3-hum15, ABTIM3-hum16, ABTIM3-hum17, ABTIM3-hum18, ABTIM3-hum19, ABTIM3-hum20, ABTIM3-hum21, ABTIM3-hum22, ABTIM3-hum23; or as described in Tables 1-4 of US 2015/0218274; or encoded by the nucleotide sequence in Tables 1-4; or a sequence substantially identical (e.g., at least 80%, 85%, 90%, 92%, 95%, 97%, 98%, 99% or higher identical) to any of the aforesaid sequences, or closely related CDRs, e.g., CDRs which are identical or which have at least one amino acid alteration, but not more than two, three or four alterations (e.g., substitutions, deletions, or insertions, e.g., conservative substitutions).

In yet another embodiment, the anti-TIM3 antibody molecule comprises at least one, two, three or four variable regions from an antibody described herein, e.g., an antibody chosen from any of ABTIM3, ABTIM3-hum01, ABTIM3-hum02, ABTIM3-hum03, ABTIM3-hum04, ABTIM3-hum05, ABTIM3-hum06, ABTIM3-hum07, ABTIM3-hum08, ABTIM3-hum09, ABTIM3-hum10, ABTIM3-hum11, ABTIM3-hum12, ABTIM3-hum13, ABTIM3-hum14, ABTIM3-hum15, ABTIM3-hum16, ABTIM3-hum17, ABTIM3-hum18, ABTIM3-hum19, ABTIM3-hum20, ABTIM3-hum21, ABTIM3-hum22, ABTIM3-hum23; or as described in Tables 1-4 of US 2015/0218274; or encoded by the nucleotide sequence in Tables 1-4; or a sequence substantially identical (e.g., at least 80%, 85%, 90%, 92%, 95%, 97%, 98%, 99% or higher identical) to any of the aforesaid sequences.

In other embodiments, the agent which enhances the activity of a CAR-expressing cell is a CEACAM inhibitor (e.g., CEACAM-1, CEACAM-3, and/or CEACAM-5 inhibitor). In one embodiment, the inhibitor of CEACAM is an anti-CEACAM antibody molecule. Exemplary anti-CEACAM-1 antibodies are described in WO 2010/125571, WO 2013/082366 WO 2014/059251 and WO 2014/022332, *e.g.,* a monoclonal antibody 34B1, 26H7, and 5F4; or a recombinant form thereof, as described in, *e.g.,* US 2004/0047858, US 7,132,255 and WO 99/052552. In other embodiments, the anti-CEACAM antibody binds to CEACAM-5 as described in, *e.g.,* Zheng et al. PLoS One. 2010 Sep 2;5(9). pii: e12529 (DOI:10:1371/journal.pone.0021146), or crossreacts with CEACAM-1 and CEACAM-5 as described in, *e.g.,* WO 2013/054331 and US 2014/0271618.

Without wishing to be bound by theory, carcinoembryonic antigen cell adhesion molecules (CEACAM), such as CEACAM-1 and CEACAM-5, are believed to mediate, at least in part, inhibition of an anti-tumor immune response (*see e.g.,* Markel et al. J Immunol. 2002 Mar 15;168(6):2803-10; Markel et al. J Immunol. 2006 Nov 1;177(9):6062-71; Markel et al. Immunology. 2009 Feb;126(2):186-200; Markel et al. Cancer Immunol Immunother. 2010 Feb;59(2):215-30; Ortenberg et al. Mol Cancer Ther. 2012 Jun;11(6):1300-10; Stern et al. J Immunol. 2005 Jun 1;174(11):6692-701; Zheng et al. PLoS One. 2010 Sep 2;5(9). pii: e12529). For example, CEACAM-1 has been described as a heterophilic ligand for TIM-3 and as playing a role in TIM-3-mediated T cell tolerance and exhaustion (*see e.g.,* WO 2014/022332; Huang, et al. (2014) Nature doi:10.1038/nature13848). In embodiments, co-blockade of CEACAM-1 and TIM-3 has been shown to enhance an anti-tumor immune response in xenograft colorectal cancer models (*see e.g.,* WO 2014/022332; Huang, *et al.* (2014), *supra*). In other embodiments, co-blockade of CEACAM-1 and PD-1 reduce T cell tolerance as described, *e.g*., in WO 2014/059251. Thus, CEACAM inhibitors can be used with the other immunomodulators described herein (*e.g*., anti-PD-1 and/or anti-TIM-3 inhibitors) to enhance an immune response against a cancer, *e.g.,* a melanoma, a lung cancer (e.g., NSCLC), a bladder cancer, a colon cancer an ovarian cancer, and other cancers as described herein.

LAG3 (lymphocyte activation gene-3 or CD223) is a cell surface molecule expressed on activated T cells and B cells that has been shown to play a role in CD8+ T cell exhaustion. Antibodies, antibody fragments, and other inhibitors of LAG3 and its ligands are available in the art and may be used combination with a CD19 CAR described herein. For example, BMS-986016 (Bristol-Myers Squib) is a monoclonal antibody that targets LAG3. IMP701 (Immutep) is an antagonist LAG3 antibody and IMP731 (Immutep and GlaxoSmithKline) is a depleting LAG3 antibody. Other LAG3 inhibitors include IMP321 (Immutep), which is a recombinant fusion protein of a soluble portion of LAG3 and Ig that binds to MHC class II molecules and activates antigen presenting cells (APC). Other antibodies are disclosed, e.g., in WO2010/019570.

In one embodiment, the anti-LAG3 antibody or fragment thereof is an anti-LAG3 antibody molecule as described in US 2015/0259420, entitled "Antibody Molecules to LAG3 and Uses Thereof,". In one embodiment, the anti- LAG3 antibody molecule includes at least one, two, three, four, five or six CDRs (or collectively all of the CDRs) from a heavy and light chain variable region from an antibody chosen from any of BAP050-hum01, BAP050-hum02, BAP050-hum03, BAP050-hum04, BAP050-hum05, BAP050-hum06, BAP050-hum07, BAP050-hum08, BAP050-hum09, BAP050-hum10, BAP050-hum11, BAP050-hum12, BAP050-hum13, BAP050-hum14, BAP050-hum15, BAP050-hum16, BAP050-hum17, BAP050-hum18, BAP050-hum19, BAP050-hum20, huBAP050(Ser) (e.g., BAP050-hum01-Ser, BAP050-hum02-Ser, BAP050-hum03-Ser, BAP050-hum04-Ser, BAP050-hum05-Ser, BAP050-hum06-Ser, BAP050-hum07-Ser, BAP050-hum08-Ser, BAP050-hum09-Ser, BAP050-hum10-Ser, BAP050-hum11-Ser, BAP050-hum12-Ser, BAP050-hum13-Ser, BAP050-hum14-Ser, BAP050-hum15-Ser, BAP050-hum18-Ser, BAP050-hum19-Ser, or BAP050-hum20-Ser), BAP050-Clone-F, BAP050-Clone-G, BAP050-Clone-H, BAP050-Clone-I, or BAP050-Clone-J; or as described in Table 1 of US 2015/0259420; or encoded by the nucleotide sequence in Table 1; or a sequence substantially identical (e.g., at least 80%, 85%, 90%, 92%, 95%, 97%, 98%, 99% or higher identical) to any of the aforesaid sequences, or closely related CDRs, e.g., CDRs which are identical or which have at least one amino acid alteration, but not more than two, three or four alterations (e.g., substitutions, deletions, or insertions, e.g., conservative substitutions).

In yet another embodiment, the anti- LAG3 antibody molecule comprises at least one, two, three or four variable regions from an antibody described herein, e.g., an antibody chosen from any of BAP050-hum01, BAP050-hum02, BAP050-hum03, BAP050-hum04, BAP050-hum05, BAP050-hum06, BAP050-hum07, BAP050-hum08, BAP050-hum09, BAP050-hum10, BAP050-hum11, BAP050-hum12, BAP050-hum13, BAP050-hum14, BAP050-hum15, BAP050-hum16, BAP050-hum17, BAP050-hum18, BAP050-hum19, BAP050-hum20, huBAP050(Ser) (e.g., BAP050-hum01-Ser, BAP050-hum02-Ser, BAP050-hum03-Ser, BAP050-hum04-Ser, BAP050-hum05-Ser, BAP050-hum06-Ser, BAP050-hum07-Ser, BAP050-hum08-Ser, BAP050-hum09-Ser, BAP050-hum10-Ser, BAP050-hum11-Ser, BAP050-hum12-Ser, BAP050-hum13-Ser, BAP050-hum14-Ser, BAP050-hum15-Ser, BAP050-hum18-Ser, BAP050-hum19-Ser, or BAP050-hum20-Ser), BAP050-Clone-F, BAP050-Clone-G, BAP050-Clone-H, BAP050-Clone-I, or BAP050-Clone-J; or as described in Table 1 of US 2015/0259420; or encoded by the nucleotide sequence in Tables 1; or a sequence substantially identical (e.g., at least 80%, 85%, 90%, 92%, 95%, 97%, 98%, 99% or higher identical) to any of the aforesaid sequences.

In some embodiments, the CAR therapy and BTK inhibitor are administered in combination with a toll like receptor (TLR) agonist. The TLR agonist can be a TLR9 agonist. In some embodiments, the TLR agonist is an oligodeoxynucleotide, e.g., a CG-enriched oligodeoxynucleotide, e.g., an unmethylated CG-enriched oligodeoxynucleotide. See, e.g., Sagiv-Barfi et al., "Ibrutinib enhances the antitumor immune response induced by intratumoral injection of a TLR9 ligand in syngeneic mouse lymphoma model." Blood. 2015 Feb 6. pii: blood-2014-08-593137. In some embodiments, the TLR agonist is administered in combination with a CAR-expressing NK cell. Without being bound by theory, the TLR agonist may promote activation of NK cells such as CAR-expressing NK cells. In some embodiments, the TLR agonist is administered by injection, e.g., intrarumoral injection.

In some embodiments, the agent which enhances the activity of a CAR-expressing cell can be, e.g., a fusion protein comprising a first domain and a second domain, wherein the first domain is an inhibitory molecule, or fragment thereof, and the second domain is a polypeptide that is associated with a positive signal, e.g., a polypeptide comrpsing an antracellular signaling domain as described herein. In some embodiments, the polypeptide that is associated with a positive signal can include a costimulatory domain of CD28, CD27, ICOS, e.g., an intracellular signaling domain of CD28, CD27 and/or ICOS, and/or a primary signaling domain, e.g., of CD3 zeta, e.g., described herein. In one embodiment, the fusion protein is expressed by the same cell that expressed the CAR. In another embodiment, the fusion protein is expressed by a cell, e.g., a T cell that does not express an anti-CD19 CAR.

In one embodiment, the agent which enhances activity of a CAR-expressing cell described herein is miR-17-92.

In one embodiment, the agent which enhances activity of a CAR-described herein is a cytokine. Cytokines have important functions related to T cell expansion, differentiation, survival, and homeostatis. Cytokines that can be administered to the subject receiving a CAR-expressing cell described herein include: IL-2, IL-4, IL-7, IL-9, IL-15, IL-18, and IL-21, or a combination thereof. In preferred embodiments, the cytokine administered is IL-7, IL-15, or IL-21, or a combination thereof. The cytokine can be administered once a day or more than once a day, e.g., twice a day, three times a day, or four times a day. The cytokine can be administered for more than one day, e.g. the cytokine is administered for 2 days, 3 days, 4 days, 5 days, 6 days, 1 week, 2 weeks, 3 weeks, or 4 weeks. For example, the cytokine is administered once a day for 7 days.

In embodiments, the cytokine is administered in combination with CAR-expressing cells. The cytokine can be administered simultaneously or concurrently with the CAR-expressing cells, e.g., administered on the same day. The cytokine may be prepared in the same pharmaceutical composition as the CAR-expressing cells, or may be prepared in a separate pharmaceutical composition. Alternatively, the cytokine can be administered shortly after administration of the CAR-expressing T cells, e.g., 1 day, 2 days, 3 days, 4 days, 5 days, 6 days, or 7 days after administration of the CAR-expressing cells. In embodiments where the cytokine is administered in a dosing regimen that occurs over more than one day, the first day of the cytokine dosing regimen can be on the same day as administration with the CAR-expressing cells, or the first day of the cytokine dosing regimen can be 1 day, 2 days, 3 days, 4 days, 5 days, 6 days, or 7 days after administration of the CAR-expressing T cells. In one embodiment, on the first day, the CAR-expressing cells are administered to the subject, and on the second day, a cytokine is administered once a day for the next 7 days. In a preferred embodiment, the cytokine to be administered in combination with the CAR-expressing cells is IL-7, IL-15, and/or IL-21.

In other embodiments, the cytokine is administered a sufficient period of time after administration of the CAR-expressing cells, e.g., at least 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells. In one embodiment, the cytokine is administered after assessment of the subject's response to the CAR-expressing cells. For example, the subject is administered CAR-expressing cells according to the dosage and regimens described herein. The response of the subject to CART therapy is assessed at 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells, using any of the methods described herein, including inhibition of tumor growth, reduction of circulating tumor cells, or tumor regression. Subjects that do not exhibit a sufficient response to CART therapy can be administered a cytokine. Administration of the cytokine to the subject that has sub-optimal response to the CART therapy improves CART efficacy and/or anti-tumor activity. In a preferred embodiment, the cytokine administered after administration of CAR-expressing cells is IL-7.

Further combination therapies may include anti-allergenic agents, anti-emetics, analgesics, adjunct therapies,

Some patients may experience allergic reactions to the therapeutics described herein and/or other anti-cancer agent(s) during or after administration; therefore, anti-allergic agents are often administered to minimize the risk of an allergic reaction. Suitable anti-allergic agents include corticosteroids, such as dexamethasone (e.g., Decadron®), beclomethasone (e.g., Beclovent®), hydrocortisone (also known as cortisone, hydrocortisone sodium succinate, hydrocortisone sodium phosphate, and sold under the tradenames Ala-Cort®, hydrocortisone phosphate, Solu-Cortef®, Hydrocort Acetate® and Lanacort®), prednisolone (sold under the tradenames Delta-Cortel®, Orapred®, Pediapred® and Prelone®), prednisone (sold under the tradenames Deltasone®, Liquid Red®, Meticorten® and Orasone®), methylprednisolone (also known as 6-methylprednisolone, methylprednisolone acetate, methylprednisolone sodium succinate, sold under the tradenames Duralone®, Medralone®, Medrol®, M-Prednisol® and Solu-Medrol®); antihistamines, such as diphenhydramine (e.g., Benadryl®), hydroxyzine, and cyproheptadine; and bronchodilators, such as the beta-adrenergic receptor agonists, albuterol (e.g., Proventil®), and terbutaline (Brethine®).

Some patients may experience nausea during and after administration of the therapeutics described herein and/or other anti-cancer agent(s); therefore, anti-emetics are used in preventing nausea (upper stomach) and vomiting. Suitable anti-emetics include aprepitant (Emend®), ondansetron (Zofran®), granisetron HC1 (Kytril®), lorazepam (Ativan®, dexamethasone (Decadron®), prochlorperazine (Compazine®), casopitant (Rezonic® and Zunrisa®), and combinations thereof.

Medication to alleviate the pain experienced during the treatment period is often prescribed to make the patient more comfortable. Common over-the-counter analgesics, such Tylenol®, are often used. However, opioid analgesic drugs such as hydrocodone/paracetamol or hydrocodone/acetaminophen (e.g., Vicodin®), morphine (e.g., Astramorph® or Avinza®), oxycodone (e.g., OxyContin® or Percocet®), oxymorphone hydrochloride (Opana®), and fentanyl (e.g., Duragesic®) are also useful for moderate or severe pain.

In an effort to protect normal cells from treatment toxicity and to limit organ toxicities, cytoprotective agents (such as neuroprotectants, free-radical scavengers, cardioprotectors, anthracycline extravasation neutralizers, nutrients and the like) may be used as an adjunct therapy. Suitable cytoprotective agents include Amifostine (Ethyol®), glutamine, dimesna (Tavocept®), mesna (Mesnex®), dexrazoxane (Zinecard® or Totect®), xaliproden (Xaprila®), and leucovorin (also known as calcium leucovorin, citrovorum factor and folinic acid).

The structure of the active compounds identified by code numbers, generic or trade names may be taken from the actual edition of the standard compendium "The Merck Index" or from databases, e.g. Patents International (e.g. IMS World Publications).

The above-mentioned compounds, which can be used in combination with a compound of the present disclosure, can be prepared and administered as described in the art, such as in the documents cited above.

In one embodiment, the present disclosure provides pharmaceutical compositions comprising at least one compound of the present disclosure (e.g., a compound of the present disclosure) or a pharmaceutically acceptable salt thereof together with a pharmaceutically acceptable carrier suitable for administration to a human or animal subject, either alone or together with other anti-cancer agents.

In one instance, the present disclosure provides methods of treating human or animal subjects suffering from a cellular proliferative disease, such as cancer. The present disclosure provides methods of treating a human or animal subject in need of such treatment, comprising administering to the subject a therapeutically effective amount of a compound of the present disclosure (e.g., a compound of the present disclosure) or a pharmaceutically acceptable salt thereof, either alone or in combination with other anti-cancer agents.

In particular, compositions will either be formulated together as a combination therapeutic or administered separately.

In combination therapy, the compound of the present disclosure and other anti-cancer agent(s) may be administered either simultaneously, concurrently or sequentially with no specific time limits, wherein such administration provides therapeutically effective levels of the two compounds in the body of the patient.

In a preferred instance, the compound of the present disclosure and the other anti-cancer agent(s) is generally administered sequentially in any order by infusion or orally. The dosing regimen may vary depending upon the stage of the disease, physical fitness of the patient, safety profiles of the individual drugs, and tolerance of the individual drugs, as well as other criteria well-known to the attending physician and medical practitioner(s) administering the combination. The compound of the present disclosure and other anti-cancer agent(s) may be administered within minutes of each other, hours, days, or even weeks apart depending upon the particular cycle being used for treatment. In addition, the cycle could include administration of one drug more often than the other during the treatment cycle and at different doses per administration of the drug.

In another aspect of the present disclosure, kits that include one or more compound of the present disclosure and a combination partner as disclosed herein are provided. Representative kits include (a) a compound of the present disclosure or a pharmaceutically acceptable salt thereof, (b) at least one combination partner, e.g., as indicated above, whereby such kit may comprise a package insert or other labeling including directions for administration.

A compound of the present disclosure may also be used to advantage in combination with known therapeutic processes, for example, the administration of hormones or especially radiation. A compound of the present disclosure may in particular be used as a radiosensitizer, especially for the treatment of tumors which exhibit poor sensitivity to radiotherapy.

### Combination with a low dose of an mTOR inhibitor

Methods described herein can use a low, immune enhancing, dose of an mTOR inhibitor e.g., an allosteric mTOR inhibitor, including rapalogs such as RAD001. Administration of a low, immune enhancing, dose of an mTOR inhibitor (e.g., a dose that is insufficient to completely suppress the immune system, but sufficient to improve immune function) can optimize the performance of immune effector cells, e.g., T cells or CAR-expressing cells, in the subject. Methods for measuring mTOR inhibition, dosages, treatment regimens, and suitable pharmaceutical compositions are described in U.S. Patent Application No. 2015/0140036, filed Nov. 13, 2014.

In an embodiment, administration of a low, immune enhancing, dose of an mTOR inhibitor can result in one or more of the following:
i) a decrease in the number of PD-1 positive immune effector cells;
ii) an increase in the number of PD-1 negative immune effector cells;
iii) an increase in the ratio of PD-1 negative immune effector cells / PD-1 positive immune effector cells;
iv) an increase in the number of naive T cells;
v) an increase in the expression of one or more of the following markers: CD62L^{high}, CD127^{high}, CD27⁺, and BCL2, e.g., on memory T cells, e.g., memory T cell precursors;
vi) a decrease in the expression of KLRG1, e.g., on memory T cells, e.g., memory T cell precursors; or
vii) an increase in the number of memory T cell precursors, e.g., cells with any one or combination of the following characteristics: increased CD62L^{high}, increased CD127^{high}, increased CD27⁺, decreased KLRG1, and increased BCL2;
and wherein any of the foregoing, e.g., i), ii), iii), iv), v), vi), or vii), occurs e.g., at least transiently, e.g., as compared to a non-treated subject.

In another embodiment, administration of a low, immune enhancing, dose of an mTOR inhibitor results in increased or prolonged proliferation of CAR-expressing cells, e.g., in culture or in a subject, e.g., as compared to non-treated CAR-expressing cells or a non-treated subject. In embodiments, increased proliferation is associated with in an increase in the number of CAR-expressing cells. In another embodiment, administration of a low, immune enhancing, dose of an mTOR inhibitor results in increased killing of cancer cells by CAR-expressing cells, e.g., in culture or in a subject, e.g., as compared to non-treated CAR-expressing cells or a non-treated subject. In embodiments, increased killing of cancer cells is associated with in a decrease in tumor volume.

In one instance, the cells expressing a CAR molecule, e.g., a CAR molecule described herein, are administered in combination with a low, immune enhancing dose of an mTOR inhibitor, e.g., an allosteric mTOR inhibitor, e.g., RAD001, or a catalytic mTOR inhibitor. For example, administration of the low, immune enhancing, dose of the mTOR inhibitor can be initiated prior to administration of a CAR-expressing cell described herein; completed prior to administration of a CAR-expressing cell described herein; initiated at the same time as administration of a CAR-expressing cell described herein; overlapping with administration of a CAR-expressing cell described herein; or continuing after administration of a CAR-expressing cell described herein.

Alternatively or in addition, administration of a low, immune enhancing, dose of an mTOR inhibitor can optimize immune effector cells to be engineered to express a CAR molecule described herein. In such instances, administration of a low, immune enhancing, dose of an mTOR inhibitor, e.g., an allosteric inhibitor, e.g., RAD001, or a catalytic inhibitor, is initiated or completed prior to harvest of immune effector cells, e.g., T cells or NK cells, to be engineered to express a CAR molecule described herein, from a subject.

In another instance, immune effector cells, e.g., T cells or NK cells, to be engineered to express a CAR molecule described herein, e.g., after harvest from a subject, or CAR-expressing immune effector cells, e.g., T cells or NK cells, e.g., prior to administration to a subject, can be cultured in the presence of a low, immune enhancing, dose of an mTOR inhibitor.

In an embodiment, administering to the subject a low, immune enhancing, dose of an mTOR inhibitor comprises administering, e.g., once per week, e.g., in an immediate release dosage form, 0.1 to 20, 0.5 to 10, 2.5 to 7.5, 3 to 6, or about 5, mgs of RAD001, or a bioequivalent dose thereof. In an embodiment, administering to the subject a low, immune enhancing, dose of an mTOR inhibitor comprises administering, e.g., once per week, e.g., in a sustained release dosage form, 0.3 to 60, 1.5 to 30, 7.5 to 22.5, 9 to 18, or about 15 mgs of RAD001, or a bioequivalent dose thereof.

In an embodiment, a dose of an mTOR inhibitor is associated with, or provides, mTOR inhibition of at least 5 but no more than 90%, at least 10 but no more than 90%, at least 15, but no more than 90%, at least 20 but no more than 90%, at least 30 but no more than 90%, at least 40 but no more than 90%, at least 50 but no more than 90%, at least 60 but no more than 90%, at least 70 but no more than 90%, at least 5 but no more than 80%, at least 10 but no more than 80%, at least 15, but no more than 80%, at least 20 but no more than 80%, at least 30 but no more than 80%, at least 40 but no more than 80%, at least 50 but no more than 80%, at least 60 but no more than 80%, at least 5 but no more than 70%, at least 10 but no more than 70%, at least 15, but no more than 70%, at least 20 but no more than 70%, at least 30 but no more than 70%, at least 40 but no more than 70%, at least 50 but no more than 70%, at least 5 but no more than 60%, at least 10 but no more than 60%, at least 15, but no more than 60%, at least 20 but no more than 60%, at least 30 but no more than 60%, at least 40 but no more than 60%, at least 5 but no more than 50%, at least 10 but no more than 50%, at least 15, but no more than 50%, at least 20 but no more than 50%, at least 30 but no more than 50%, at least 40 but no more than 50%, at least 5 but no more than 40%, at least 10 but no more than 40%, at least 15, but no more than 40%, at least 20 but no more than 40%, at least 30 but no more than 40%, at least 35 but no more than 40%, at least 5 but no more than 30%, at least 10 but no more than 30%, at least 15, but no more than 30%, at least 20 but no more than 30%, or at least 25 but no more than 30%.

The extent of mTOR inhibition can be conveyed as, or corresponds to, the extent of P70 S6 kinase inhibition, e.g., the extent of mTOR inhibition can be determined by the level of decrease in P70 S6 kinase activity, e.g., by the decrease in phosphorylation of a P70 S6 kinase substrate. The level of mTOR inhibition can be evaluated by various methods, such as measuring P70 S6 kinase activity by the Boulay assay, as described in U.S. Patent Application No. 2015/01240036, or as described in U.S. Patent No. 7,727,950; measuring the level of phosphorylated S6 by western blot; or evaluating a change in the ratio of PD1 negative immune effector cells to PD1 positive immune effector cells.

As used herein, the term "mTOR inhibitor" refers to a compound or ligand, or a pharmaceutically acceptable salt thereof, which inhibits the mTOR kinase in a cell. In an embodiment, an mTOR inhibitor is an allosteric inhibitor. Allosteric mTOR inhibitors include the neutral tricyclic compound rapamycin (sirolimus), rapamycin-related compounds, that is compounds having structural and functional similarity to rapamycin including, e.g., rapamycin derivatives, rapamycin analogs (also referred to as rapalogs) and other macrolide compounds that inhibit mTOR activity. In an embodiment, an mTOR inhibitor is a catalytic inhibitor.

Rapamycin is a known macrolide antibiotic produced by Streptomyces hygroscopicus having the structure shown in Formula A.

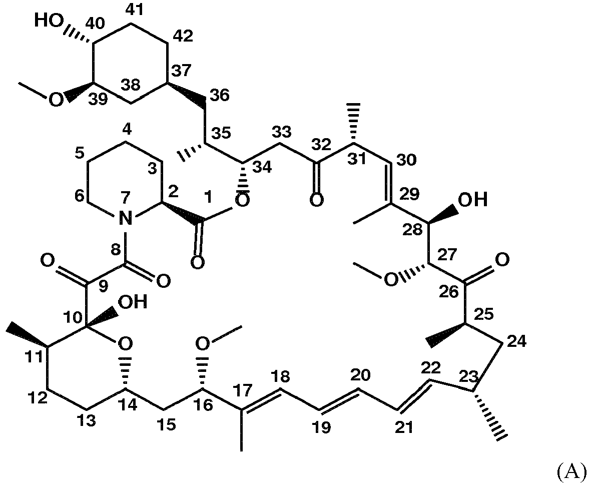

See, e.g., McAlpine, J.B., et al., J. Antibiotics (1991) 44: 688; Schreiber, S.L., et al., J. Am. Chem. Soc. (1991) 113: 7433; U.S. Patent No. 3,929,992. There are various numbering schemes proposed for rapamycin. To avoid confusion, when specific rapamycin analogs are named herein, the names are given with reference to rapamycin using the numbering scheme of formula A.

Rapamycin analogs useful in the disclosure are, for example, O-substituted analogs in which the hydroxyl group on the cyclohexyl ring of rapamycin is replaced by OR₁ in which R₁ is hydroxyalkyl, hydroxyalkoxyalkyl, acylaminoalkyl, or aminoalkyl; e.g. RAD001, also known as, everolimus as described in US 5,665,772 and WO94/09010. Other suitable rapamycin analogs include those substituted at the 26- or 28-position. The rapamycin analog may be an epimer of an analog mentioned above, particularly an epimer of an analog substituted in position 40, 28 or 26, and may optionally be further hydrogenated, e.g. as described in US 6,015,815, WO95/14023 and WO99/15530, e.g. ABT578 also known as zotarolimus or a rapamycin analog described in US 7,091,213, WO98/02441 and WO01/14387, e.g. AP23573 also known as ridaforolimus.

Examples of rapamycin analogs suitable for use in the present disclosure from US 5,665,772 include, but are not limited to, 40-O-benzyl-rapamycin, 40-0-(4'-hydroxymethyl)benzyl-rapamycin, 40-O-[4'-(1,2-dihydroxyethyl)]benzyl-rapamycin, 40-O-allyl-rapamycin, 40-O-[3'-(2,2-dimethyl-1,3-dioxolan-4(S)-yl)-prop-2'-en-1'-yl]-rapamycin, (2'E,4'S)-40-O-(4',5'-dihydroxypent-2'-en-1'-yl)-rapamycin, 40-O-(2-hydroxy)ethoxycarbonylmethyl-rapamycin, 40-O-(2-hydroxy)ethyl-rapamycin, 40-O-(3-hydroxy)propyl-rapamycin, 40-O-(6-hydroxy)hexyl-rapamycin, 40-O-[2-(2-hydroxy)ethoxy] ethyl-rapamycin, 40-O-[(3S)-2,2-dimethyldioxolan-3-yl]methyl-rapamycin, 40-O-[(2S)-2,3-dihydroxyprop-1-yl]-rapamycin, 40-O-(2-acetoxy)ethyl-rapamycin, 40-O-(2-nicotinoyloxy)ethyl-rapamycin, 40-O-[2-(N-morpholino)acetoxy]ethyl-rapamycin, 40-O-(2-N-imidazolylacetoxy)ethyl-rapamycin, 40-O-[2-(N-methyl-N'-piperazinyl)acetoxy]ethyl-rapamycin, 39-O-desmethyl-39,40-O,O-ethylene-rapamycin, (26R)-26-dihydro-40-O-(2-hydroxy)ethyl-rapamycin, 40-O-(2-aminoethyl)-rapamycin, 40-O-(2-acetaminoethyl)-rapamycin, 40-O-(2-nicotinamidoethyl)-rapamycin, 40-O-(2-(N-methyl-imidazo-2'-ylcarbethoxamido)ethyl)-rapamycin, 40-O-(2-ethoxycarbonylaminoethyl)-rapamycin, 40-O-(2-tolylsulfonamidoethyl)-rapamycin and 40-O-[2-(4',5'-dicarboethoxy-1',2',3'-triazol-1'-yl)-ethyl] - rapamycin.

Other rapamycin analogs useful in the present disclosure are analogs where the hydroxyl group on the cyclohexyl ring of rapamycin and/or the hydroxy group at the 28 position is replaced with an hydroxyester group are known, for example, rapamycin analogs found in US RE44,768, e.g. temsirolimus.

Other rapamycin analogs useful in the preset disclosure include those wherein the methoxy group at the 16 position is replaced with another substituent, preferably (optionally hydroxy-substituted) alkynyloxy, benzyl, orthomethoxybenzyl or chlorobenzyl and/or wherein the mexthoxy group at the 39 position is deleted together with the 39 carbon so that the cyclohexyl ring of rapamycin becomes a cyclopentyl ring lacking the 39 position methyoxy group; e.g. as described in WO95/16691 and WO96/41807 . The analogs can be further modified such that the hydroxy at the 40-position of rapamycin is alkylated and/or the 32-carbonyl is reduced.

Rapamycin analogs from WO95/16691 include, but are not limited to, 16-demthoxy-16-(pent-2-ynyl)oxy-rapamycin, 16-demthoxy-16-(but-2-ynyl)oxy-rapamycin, 16-demthoxy-16-(propargyl)oxy-rapamycin, 16-demethoxy-16-(4-hydroxy-but-2-ynyl)oxy-rapamycin, 16-demthoxy-16-benzyloxy-40-O-(2-hydroxyethyl)-rapamycin, 16-demthoxy-16-benzyloxy-rapamycin, 16-demethoxy-16-orthomethoxybenzyl-rapamycin, 16-demethoxy-40-O-(2-methoxyethyl)-16-pent-2-ynyl)oxy-rapamycin, 39-demethoxy-40-desoxy-39-formyl-42-nor-rapamycin, 39-demethoxy-40-desoxy-39-hydroxymethyl-42-nor-rapamycin, 39-demethoxy-40-desoxy-39-carboxy-42-nor-rapamycin, 39-demethoxy-40-desoxy-39-(4-methyl-piperazin-1-yl)carbonyl-42-nor-rapamycin, 39-demethoxy-40-desoxy-39-(morpholin-4-yl)carbonyl-42-nor-rapamycin, 39-demethoxy-40-desoxy-39-[N-methyl, N-(2-pyridin-2-yl-ethyl)]carbamoyl-42-nor-rapamycin and 39-demethoxy-40-desoxy-39-(p-toluenesulfonylhydrazonomethyl)-42-nor-rapamycin.

Rapamycin analogs from WO96/41807 include, but are not limited to, 32-deoxo-rapamycin, 16-O-pent-2-ynyl-32-deoxo-rapamycin, 16-O-pent-2-ynyl-32-deoxo-40-O-(2-hydroxy-ethyl)-rapamycin, 16-O-pent-2-ynyl-32-(S)-dihydro-40-O-(2-hydroxyethyl)-rapamycin, 32(S)-dihydro-40-O-(2-methoxy)ethyl-rapamycin and 32(S)-dihydro-40-O-(2-hydroxyethyl)-rapamycin.

Another suitable rapamycin analog is umirolimus as described in US2005/0101624.

RAD001, otherwise known as everolimus (Afinitor®), has the chemical name (1R,9S,12S,15R,16E,18R,19R,21R,23S,24E,26E,28E,30S,32S,35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone

Further examples of allosteric mTOR inhibitors include sirolimus (rapamycin, AY-22989), 40-[3-hydroxy-2-(hydroxymethyl)-2-methylpropanoate]-rapamycin (also called temsirolimus or CCI-779) and ridaforolimus (AP-23573/MK-8669). Other examples of allosteric mTor inhibtors include zotarolimus (ABT578) and umirolimus.

Alternatively or additionally, catalytic, ATP-competitive mTOR inhibitors have been found to target the mTOR kinase domain directly and target both mTORC1 and mTORC2. These are also more effective inhibitors of mTORC1 than such allosteric mTOR inhibitors as rapamycin, because they modulate rapamycin-resistant mTORC1 outputs such as 4EBP1-T37/46 phosphorylation and cap-dependent translation.

Catalytic inhibitors include: BEZ235 or 2-methyl-2-[4-(3-methyl-2-oxo-8-quinolin-3-yl-2,3-dihydro-imidazo[4,5-c]quinolin-1-yl)-phenyl]-propionitrile, or the monotosylate salt form. the synthesis of BEZ235 is described in WO2006/122806; CCG168 (otherwise known as AZD-8055, Chresta, C.M., et al., Cancer Res, 2010, 70(1), 288-298) which has the chemical name {5-[2,4-bis-((S)-3-methyl-morpholin-4-yl)-pyrido[2,3d]pyrimidin-7-yl]-2-methoxy-phenyl}-methanol; 3-[2,4-bis[(3S)-3-methylmorpholin-4-yl]pyrido[2,3-d]pyrimidin-7-yl]-N-methylbenzamide (WO09104019); 3-(2-aminobenzo[d]oxazol-5-yl)-1-isopropyl-1H-pyrazolo[3,4-d]pyrimidin-4-amine (WO10051043 and WO2013023184); A N-(3-(N-(3-((3,5-dimethoxyphenyl)amino)quinoxaline-2-yl)sulfamoyl)phenyl)-3-methoxy-4-methylbenzamide (WO07044729 and WO12006552); PKI-587 (Venkatesan, A.M., J. Med.Chem., 2010, 53, 2636-2645) which has the chemical name 1-[4-[4-(dimethylamino)piperidine-1-carbonyl]phenyl]-3-[4-(4,6-dimorpholino-1,3,5-triazin-2-yl)phenyl]urea; GSK-2126458 (ACS Med. Chem. Lett., 2010, 1, 39-43) which has the chemical name 2,4-difluoro-N-{2-methoxy-5-[4-(4-pyridazinyl)-6-quinolinyl]-3-pyridinyl}benzenesulfonamide;; 5-(9-isopropyl-8-methyl-2-morpholino-9H-purin-6-yl)pyrimidin-2-amine (WO10114484); (E)-N-(8-(6-amino-5-(trifluoromethyl)pyridin-3-yl)-1-(6-(2-cyanopropan-2-yl)pyridin-3-yl)-3-methyl-1H-imidazo[4,5-c]quinolin-2(3H)-ylidene)cyanamide (WO12007926).

Further examples of catalytic mTOR inhibitors include 8-(6-methoxy-pyridin-3-yl)-3-methyl-1-(4-piperazin-1-yl-3-trifluoromethyl-phenyl)-1,3-dihydro-imidazo[4,5-c]quinolin-2-one (WO2006/122806) and Ku-0063794 (Garcia-Martinez JM, et al.,Biochem J., 2009, 421(1), 29-42.. Ku-0063794 is a specific inhibitor of the mammalian target of rapamycin (mTOR).) WYE-354 is another example of a catalytic mTor inhibitor (Yu K, et al. (2009). Biochemical, Cellular, and In vivo Activity of Novel ATP-Competitive and Selective Inhibitors of the Mammalian Target of Rapamycin. Cancer Res. 69(15): 6232-6240).

mTOR inhibitors useful according to the present disclosure also include prodrugs, derivatives, pharmaceutically acceptable salts, or analogs thereof of any of the foregoing.

mTOR inhibitors, such as RAD001, may be formulated for delivery based on well-established methods in the art based on the particular dosages described herein. In particular, US Patent 6,004,973 provides examples of formulations useable with the mTOR inhibitors described herein.

### Pharmaceutical compositions and treatments

Pharmaceutical compositions of the present disclosure may comprise a CAR-expressing cell, e.g., a plurality of CAR-expressing cells, as described herein, or a BTK inhibitor, or both, and optionally one or more additional therapeutic agents, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are in one aspect formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

In an instance, cells expressing a CAR described herein, in combination with a BTK inhibitor described herein, are administered to a subject in combination with a molecule that decreases the Treg cell population. Methods that decrease the number of (e.g., deplete) Treg cells are known in the art and include, e.g., CD25 depletion, cyclophosphamide administration, modulating GITR function. Without wishing to be bound by theory, it is believed that reducing the number of Treg cells in a subject prior to apheresis or prior to administration of a CAR-expressing cell described herein reduces the number of unwanted immune cells (e.g., Tregs) in the tumor microenvironment and reduces the subject's risk of relapse.

In one instance, a therapy described herein, e.g., a CAR-expressing cell, in combination with a BTK inhibitor described herein, is administered to a subject in combination with a molecule targeting GITR and/or modulating GITR functions, such as a GITR agonist and/or a GITR antibody that depletes regulatory T cells (Tregs). In instances, cells expressing a CAR described herein are administered to a subject in combination with cyclophosphamide. In one instance, the GITR binding molecules and/or molecules modulating GITR functions (e.g., GITR agonist and/or Treg depleting GITR antibodies) are administered prior to the CAR-expressing cell. For example, in one instance, a GITR agonist can be administered prior to apheresis of the cells. In instances, cyclophosphamide is administered to the subject prior to administration (e.g., infusion or re-infusion) of the CAR-expressing cell or prior to apheresis of the cells. In instances, cyclophosphamide and an anti-GITR antibody are administered to the subject prior to administration (e.g., infusion or re-infusion) of the CAR-expressing cell or prior to apheresis of the cells. In one instance, the subject has cancer (e.g., a solid cancer or a hematological cancer such as ALL or CLL). In one instance, the subject has CLL. In instances, the subject has ALL. In instances, the subject has a solid cancer, e.g., a solid cancer described herein.

Exemplary GITR agonists include, e.g., GITR fusion proteins and anti-GITR antibodies (e.g., bivalent anti-GITR antibodies) such as, e.g., a GITR fusion protein described in U.S. Patent No.: 6,111,090, European Patent No.: 090505B1, U.S Patent No.: 8,586,023, PCT Publication Nos.: WO 2010/003118 and 2011/090754, or an anti-GITR antibody described, e.g., in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, PCT Publication No.:WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Patent No.: 7,618,632, and PCT Publication No.: WO 2011/051726.

In one instance, a CAR expressing cell described herein, in combination with a BTK inhibitor described herein, is administered to a subject in combination with a GITR agonist, e.g., a GITR agonist described herein. In one instance, the GITR agonist is administered prior to the CAR-expressing cell. For example, in one instance, the GITR agonist can be administered prior to apheresis of the cells. In one instance, the subject has CLL.

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-tumor effective amount," "a tumor-inhibiting effective amount," or "therapeutic amount" is indicated, the precise amount of the compositions of the present invention to be administered can be determined by a physician with consideration of individual differences in age, weight, tumor size, extent of infection or metastasis, and condition of the patient (subject). In some instances, a pharmaceutical composition comprising the T cells described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In some embodiments, a dose of CAR cells comprises about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, or 5 x 10⁸ cells/kg. In some embodiments, a dose of CAR cells (e.g., CD19 CAR cells) comprises at least about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, or 5 x 10⁸ cells/kg. In some embodiments, a dose of CAR cells (e.g., CD19 CAR cells) comprises up to about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, or 5 x 10⁸ cells/kg. In some embodiments, a dose of CAR cells (e.g., CD19 CAR cells) comprises about 1.1 x 10⁶ - 1.8 x 10⁷ cells/kg. In some embodiments, a dose of CAR cells (e.g., CD19 CAR cells) comprises about 1 x 10⁷, 2 x 10⁷, 5 x 10⁷, 1 x 10⁸, 2 x 10⁸, 5 x 10⁸, 1 x 10⁹, 2 x 10⁹, or 5 x 10⁹ cells. In some embodiments, a dose of CAR cells (e.g., CD19 CAR cells) comprises at least about 1 x 10⁷, 2 x 10⁷, 5 x 10⁷, 1 x 10⁸, 2 x 10⁸, 5 x 10⁸, 1 x 10⁹, 2 x 10⁹, or 5 x 10⁹ cells. In some embodiments, a dose of CAR cells (e.g., CD19 CAR cells) comprises up to about 1 x 10⁷, 2 x 10⁷, 5 x 10⁷, 1 x 10⁸, 2 x 10⁸, 5 x 10⁸, 1 x 10⁹, 2 x 10⁹, or 5 x 10⁹ cells.

In certain aspects, it may be desired to administer activated T cells to a subject and then subsequently redraw blood (or have an apheresis performed), activate T cells therefrom according to the present disclosure, and reinfuse the patient with these activated and expanded T cells. This process can be carried out multiple times every few weeks. In certain aspects, T cells can be activated from blood draws of from 10cc to 400cc. In certain aspects, T cells are activated from blood draws of 20cc, 30cc, 40cc, 50cc, 60cc, 70cc, 80cc, 90cc, or 100cc.

The administration of the subject compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection. In one aspect, the T cell compositions of the present invention are administered by i.v. injection. The compositions of T cells may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs of the disclosure may be introduced, thereby creating a CAR T cell of the disclosure. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present invention. In an additional aspect, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The suitable daily dose is 1 to 10 mg per day although in some instances larger doses of up to 40 mg per day may be used (described in U.S. Patent No. 6,120,766).

In one instance, the CAR is introduced into T cells, e.g., using in vitro transcription, and the subject (e.g., human) receives an initial administration of CAR T cells of the disclosure, and one or more subsequent administrations of the CAR T cells of the disclosure, wherein the one or more subsequent administrations are administered less than 15 days, e.g., 14, 13, 12, 11, 10, 9, 8, 7, 6, 5, 4, 3, or 2 days after the previous administration. In one instance, more than one administration of the CAR T cells of the disclosure are administered to the subject (e.g., human) per week, e.g., 2, 3, or 4 administrations of the CAR T cells of the disclosure are administered per week. In one instance, the subject (e.g., human subject) receives more than one administration of the CAR T cells per week (e.g., 2, 3 or 4 administrations per week) (also referred to herein as a cycle), followed by a week of no CAR T cells administrations, and then one or more additional administration of the CAR T cells (e.g., more than one administration of the CAR T cells per week) is administered to the subject. In another instance, the subject (e.g., human subject) receives more than one cycle of CAR T cells, and the time between each cycle is less than 10, 9, 8, 7, 6, 5, 4, or 3 days. In one instance, the CAR T cells are administered every other day for 3 administrations per week. In one instance, the CAR T cells of the disclosure are administered for at least two, three, four, five, six, seven, eight or more weeks.

In one aspect, CAR-expressing cells are generated using lentiviral viral vectors, such as lentivirus. Cells, e.g., CARTs generated that way will have stable CAR expression.

In one aspect, CAR-expressing cells, e.g., CARTs, are generated using a viral vector such as a gammaretroviral vector, e.g., a gammaretroviral vector described herein. CARTs generated using these vectors can have stable CAR expression.

In one aspect, CARTs transiently express CAR vectors for 4, 5, 6, 7, 8, 9, 10, 11, 12, 13, 14, 15 days after transduction. Transient expression of CARs can be effected by RNA CAR vector delivery. In one aspect, the CAR RNA is transduced into the T cell by electroporation.

A potential issue that can arise in patients being treated using transiently expressing CAR T cells (particularly with murine scFv bearing CARTs) is anaphylaxis after multiple treatments.

Without being bound by this theory, it is believed that such an anaphylactic response might be caused by a patient developing humoral anti-CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen day break in exposure to antigen.

If a patient is at high risk of generating an anti-CAR antibody response during the course of transient CAR therapy (such as those generated by RNA transductions), CART infusion breaks should not last more than ten to fourteen days.

### Exemplary CAR constructs

A CD19 antibody molecule can be, e.g., an antibody molecule (e.g., a humanized anti-CD19 antibody molecule) described in WO2014/153270. WO2014/153270 also describes methods of assaying the binding and efficacy of various CART constructs. Certain humanized CD19 variable domains are reproduced below in Table 1.

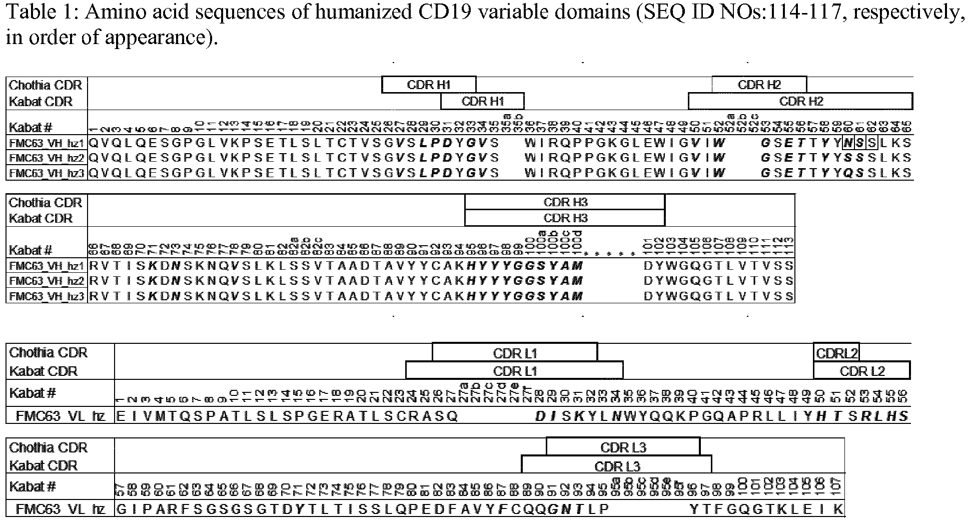

The order in which the VL and VH domains appear in the scFv can be varied (i.e., VL-VH, or VH-VL orientation), and (for example) three or four copies of the "G4S" (SEQ ID NO: 18) subunit can be used, in which each subunit comprises the sequence GGGGS (SEQ ID NO:18) (e.g., (G4S)₃ (SEQ ID NO:107) or (G4S)₄(SEQ ID NO:106)), connect the variable domains to create the entirety of the scFv domain, as shown in Table 2.

**Table 2. Humanized CD19 scFv constructs showing VH and VL orientation and linker length ("3G4S" is disclosed as SEQ ID NO: 107 and "4G4S" is disclosed as SEQ ID NO: 106).**

| construct ID | Length aa | annotation | Vh change |
|---|---|---|---|
| mscFvCTL019 | 486 | VL-VH, 3G4S | |
| 104879 | 491 | VL-VH, 4G4S | N/S |
| 104880 | 491 | VL-VH, 4G4S | N/Q |
| 104881 | 491 | VH-VL, 4G4S | N/S |
| 104882 | 491 | VH-VL, 4G4S | N/Q |
| 104875 | 486 | VL-VH, 3G4S | N/S |
| 104876 | 486 | VL-VH, 3G4S | N/Q |
| 104877 | 486 | VH-VL, 3G4S | N/S |
| 104878 | 486 | VH-VL, 3G4S | N/Q |
| 105974 | 491 | VL-VH, 4G4S | S/N |
| 105975 | 491 | VH-VL, 4G4S | S/N |
| 105976 | 486 | VL-VH, 3G4S | S/N |
| 105977 | 486 | VH-VL, 3G4S | S/N |

The sequences of humanized scFv fragments (SEQ ID NOS: 1-12) are provided below in Table 3. Full CAR constructs are provided as SEQ ID NOs: 1-12. Additional sequences, SEQ ID NOs: 13-17, shown below, can be used to generate full CAR constructs with SEQ ID NOs: 31-42.
- leader (amino acid sequence) (SEQ ID NO: 13) MALPVTALLLPLALLLHAARP
- **leader (nucleic acid sequence) (SEQ ID NO: 54)**
- CD8 hinge (amino acid sequence) (SEQ ID NO: 14) TTTPAPRPPTPAPTIASQPLSLRPEACRPAAGGAVHTRGLDFACD
- **CD8 hinge (nucleic acid sequence) (SEQ ID NO: 55)**
- CD8 transmembrane (amino acid sequence) (SEQ ID NO: 15) IYIWAPLAGTCGVLLLSLVITLYC
- **transmembrane (nucleic acid sequence) (SEQ ID NO: 56)**
- 4-1BB Intracellular domain (amino acid sequence) (SEQ ID NO: 16) KRGRKKLLYIFKQPFMRPVQTTQEEDGCSCRFPEEEEGGCEL
- **4-1BB Intracellular domain (nucleic acid sequence) (SEQ ID NO: 60)**
- ICOS Intracellular domain (amino acid sequence) (SEQ ID NO: 135) TKKKYSSSVHDPNGEYMFMRAVNTAKKSRLTDVTL
- **ICOS Intracellular domain (nucleic acid sequence) (SEQ ID NO: 136)**
- CD3 zeta domain (amino acid sequence) (SEQ ID NO: 17)
- **CD3 zeta (nucleic acid sequence) (SEQ ID NO: 101)**
- **CD3 zeta domain (amino acid sequence; NCBI Reference Sequence NM_000734.3) (SEQ ID NO:43)**
- **CD3 zeta (nucleic acid sequence; NCBI Reference Sequence NM_000734.3); (SEQ ID NO:44)** **IgG4 Hinge (amino acid sequence) (SEQ ID NO:102)** **IgG4 Hinge (nucleotide sequence) (SEQ ID NO:103)**

Clones optionally contain a Q/K residue change in the signal domain of the co-stimulatory domain derived from 4-1BB.

**Table 3: Humanized CD19 CAR Constructs**

| **Name** | **SEQ ID** | **Sequence** |
|---|---|---|
| **CAR 1** | | |
| **CAR1 scFv domain** | 1 | |
| **103101 CAR1 Soluble scFv - nt** | 61 | |
| | | |
| **103101 CAR1 Soluble scFv - aa** | 73 | |
| **104875 CAR1-Full** - **nt** | 85 | |
| | | |
| **104875 CAR1 - Full** - **aa** | 31 | |

| **CAR 2** | | |
|---|---|---|
| **CAR2 scFv domain** | 2 | |
| **103102 CAR2** - **Soluble scFv - nt** | 62 | |
| | | |
| **103102 CAR2 - Soluble scFv - aa** | 74 | |
| **104876 CAR 2** - **Full - nt** | 86 | |
| | | |
| **104876 CAR 2 - Full - aa** | 32 | |

| **CAR 3** | | |
|---|---|---|
| **CAR3 scFv domain** | 3 | |
| **103104 CAR 3** - **Soluble scFv** - **nt** | 63 | |
| | | |
| **103104 CAR 3** - **Soluble scFv** - **aa** | 75 | |
| **104877 CAR 3 - Full - nt** | 87 | |
| | | |
| **104877 CAR 3 - Full - aa** | 33 | |

| **CAR 4** | | |
|---|---|---|
| **CAR4 scFv domain** | 4 | |
| **103106 CAR4 - Soluble scFv** - **nt** | 64 | |
| | | |
| **103106 CAR4 - Soluble scFv -aa** | 76 | |
| 104878 CAR 4 - Full - nt | 88 | |
| | | |
| 104878 CAR 4 - Full - aa | 34 | |

| **CAR 5** | | |
|---|---|---|
| **CAR5 scFv domain** | 5 | |
| **99789 CAR5** - **Soluble scFv** - **nt** | 65 | |
| | | |
| **99789 CAR5** - **Soluble scFv -aa** | 77 | |
| **104879 CAR 5 - Full** - **nt** | 89 | |
| | | |
| **104879 CAR 5 - Full - aa** | 35 | |

| **CAR 6** | | |
|---|---|---|
| **CAR6 scFv domain** | 6 | |
| **99790 CAR6 - Soluble scFv - nt** | 66 | |
| | | |
| **99790 CAR6 - Soluble scFv - aa** | 78 | |
| **104880 CAR6 - Full - nt** | 90 | |
| | | |
| **104880 CAR6-Full - aa** | 36 | |

| **CAR 7** | | |
|---|---|---|
| **CAR7 scFv domain** | 7 | |
| **100796 CAR7 - Soluble scFv - nt** | 67 | |
| | | |
| **100796 CAR7 - Soluble scFv - aa** | 79 | |
| **104881 CAR 7 Full - nt** | 91 | |
| | | |
| **104881 CAR 7 Full - aa** | 37 | |

| **CAR 8** | | |
|---|---|---|
| **CAR8 scFv domain** | 8 | |
| **100798 CAR8 - Soluble scFv - nt** | 68 | |
| | | |
| **100798 CAR8 - Soluble scFv - aa** | 80 | |
| **104882 CAR 8 - Full - nt** | 92 | |
| | | |
| **104882 CAR 8 - Full - aa** | 38 | |

| **CAR 9** | | |
|---|---|---|
| **CAR9 scFv domain** | 9 | |
| **99789 CAR9 - Soluble scFv - nt** | 69 | |
| | | |
| **99789 CAR9 - Soluble scFv - aa** | 81 | |
| **105974 CAR 9 - Full - nt** | 93 | |
| | | |
| **105974 CAR 9 - Full - aa** | 39 | |

| **CAR10** | | |
|---|---|---|
| **CAR10 scFv domain** | 10 | |
| **100796 CAR10 - Soluble scFv - nt** | 70 | |
| | | |
| **100796 CAR10 - Soluble scFv - aa** | 82 | |
| **105975 CAR 10 Full - nt** | 94 | |
| | | |
| **105975 CAR 10 Full - aa** | 40 | |

| **CAR11** | | |
|---|---|---|
| **CAR11 scFv domain** | 11 | |
| **103101 CAR11** - **Soluble scFv** - **nt** | 71 | |
| | | |
| **103101 CAR11 - Soluble scFv - aa** | 83 | |
| **105976 CAR 11 Full - nt** | 95 | |
| | | |
| **105976 CAR 11 Full - aa** | 41 | |

| **CAR12** | | |
|---|---|---|
| **CAR12 scFv domain** | 12 | |
| **103104 CAR12 - Soluble scFv** - **nt** | 72 | |
| | | |
| **103104 CAR12 - Soluble scFv -aa** | 84 | |
| **105977 CAR 12 - Full - nt** | 96 | |
| | | |
| **105977 CAR 12 - Full - aa** | 42 | |

**Table 7: Murine CD19 CAR Constructs**

| **CTL019** | | |
|---|---|---|
| **CTL019 - Soluble scFv-Histag - nt** | 97 | |
| | | |
| **CTL019 - Soluble scFv-Histag - aa** | 98 | |
| **CTL019 Full - nt** | 99 | |
| | | |
| **CTL019 Full - aa** | 58 | |
| **CTL019 scFv domain** | 59 | |

The sequences of humanized CDR sequences of the scFv domains are shown in Table 4 for the heavy chain variable domains and in Table 5 for the light chain variable domains. "ID" stands for the respective SEQ ID NO for each CDR.

**Table 4. Heavy Chain Variable Domain CDRs (Kabat)**

| Candidate | FW | HCDR1 | ID | HCDR2 | ID | HCDR3 | ID |
|---|---|---|---|---|---|---|---|
| murine_CART19 | | IGVSLPDYGVS | 19 | VIWGSETTYYNSALKS | 20 | HYYYGGSYAMDY | 24 |
| humanized_CART19 a | VH4 | GVSLPDYGVS | 19 | VIWGSETTYY*S*S*S*LKS | 21 | HYYYGGSYAMDY | 24 |
| humanized_CART19 b | VH4 | GVSLPDYGVS | 19 | VIWGSETTYY*Q*S*S*LKS | 22 | HYYYGGSYAMDY | 24 |
| humanized_CART19 c | VH4 | GVSLPDYGVS | 19 | VIWGSETTYYNS*S*LKS | 23 | HYYYGGSYAMDY | 24 |

**Table 5. Light Chain Variable Domain CDRs**

| Candidate | FW | LCDR1 | ID | LCDR2 | ID | LCDR3 | ID |
|---|---|---|---|---|---|---|---|
| murine_CART19 | R1ASQDI | SKYLN | 25 | HTSRLHS | 26 QQ | GNTLPYT | 27 |
| humanized_CART19 a | VK3 | RASQDISKYLN | 25 | HTSRLHS | 26 | QQGNTLPYT | 27 |
| humanized_CART19 b | VK3 | RASQDISKYLN | 25 | HTSRLHS | 26 | QQGNTLPYT | 27 |
| humanized_CART19 c | VK3 | RASQDISKYLN | 25 | HTSRLHS | 26 | QQGNTLPYT | 27 |

Table 6 is an identification key correlating the CD19 constructs numerical names to the specific orientation of the light and heavy chains of the scFv, the number of linker units (i.e., (G4S)₃ (SEQ ID NO:107) or (G4S)₄(SEQ ID NO:106)), separating the heavy and light chains, and the distinguishing amino acid sequences in the heavy chain CDR2.

**Table 6: CD19 CAR designations.**

| **Clone ID/CAR#** | **Alt. Clone ID** | **Chain Orientation** | **Linkers** | **Site of Heavy CDR2 mutation** | **SEQ ID NO** |
|---|---|---|---|---|---|
| 104875 (CAR1) | C2136 | L2H | 3x | YSSSL | 28 |
| 104876 (CAR2) | C2137 | L2H | 3x | YQSSL | 29 |
| 104877 (CAR3) | C2138 | H2L | 3x | YSSSL | 28 |
| 104878 (CAR4) | C2139 | H2L | 3x | YQSSL | 29 |
| 104879 (CAR5) | C2140 | L2H | 4x | YSSSL | 28 |
| 104880 (CAR6) | C2141 | L2H | 4x | YQSSL | 29 |
| 104881 (CAR7) | C2142 | H2L | 4x | YSSSL | 28 |
| 104882 (CAR8) | C2143 | H2L | 4x | YQSSL | 29 |
| 105974 (CAR9) | C2144 | L2H | 4x | YNSSL | 30 |
| 105975 (CAR10) | C2145 | H2L | 4x | YNSSL | 30 |
| 105976 (CAR11) | C2146 | L2H | 3x | YNSSL | 30 |
| 105977 (CAR12) | C2147 | H2L | 3x | YNSSL | 30 |
| CTL019 | muCART19 | L2H | 3x | YNSAL | 57 |

The CAR scFv fragments can be cloned into lentiviral vectors to create a full length CAR construct in a single coding frame, using the EF1 alpha promoter for expression (SEQ ID NO: 100). EF1 alpha promoter

With reference to the heavy CDR2 site (Table 1), each of the three variations YSSSL, YQSSL and YNSSL (SEQ ID NOS:28, 29 and 30, respectively) can be present.

In addition, a G4S linker containing 3 copies of the subunit (3G4S) (SEQ ID NO: 107) and the G4S linker containing 4 copies of the subunit (4G4S) (SEQ ID NO: 106), can be used.

### EXAMPLES

The disclosure is further described in detail by reference to the following experimental examples. These examples are provided for purposes of illustration only, and are not intended to be limiting unless otherwise specified. Thus, the disclosure should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present disclosure and practice the claimed methods. The following working examples specifically point out various aspects of the present disclosure, and are not to be construed as limiting in any way the remainder of the disclosure.

### Example 1

### N-(3-(5-((1-Acryloylazetidin-3-yl)oxy)-6-aminopyrimidin-4-yl)-5-fluoro-2-methylphenyl)-4-cyclopropyl-2-fluorobenzamide

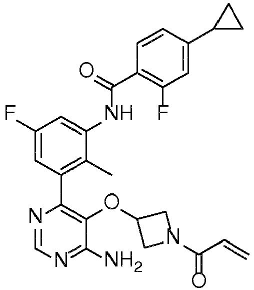

### (1) tert-Butyl 3-((4-amino-6-chloropyrimidin-5-yl)oxy)azetidine-1-carboxylate, INT 1

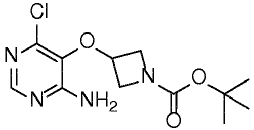

To a solution of *N*-Boc-3-iodoazetidine (6.84 g, 24.16 mmol) in DMF (37 mL) was added 4-amino-6-chloropyrimidin-5-ol (2.00 g, 13.74 mmol) followed by potassium carbonate (5.70 g, 41.24 mmol). The reaction mixture was stirred at 100 °C for 16 hr. The mixture was diluted with EtOAc and washed with saturated aqueous sodium hydrogen carbonate solution. The aqueous layer was back-extracted with EtOAc. The combined organic layers were washed with water (2x) and brine (2x), dried over magnesium sulfate, filtered and concentrated. The crude was dried in vacuum for 30 min. The residue was purified by flash chromatography (DCM/MeOH gradient, 0-5%). The isolated residue was triturated with cyclohexane. The resulting off-white solid was filtered off, rinsed with cyclohexane, and dried in vacuum to afford the title compound **INT 1** as an off-white solid.

UPLC-MS: MS (ESI): [M+H]⁺ 301.0, rt = 0.83 min. ¹H NMR (DMSO-*d*₆): δ (ppm) 7.98 (s, 1H), 7.34 (br s, 2H), 4.93-4.70 (m, 1H), 4.23-3.95 (m, 4H), 1.39 (s, 9H).

### (2) 2-(5-Fluoro-2-methyl-3-nitrophenyl)-4,4,5,5-tetramethyl-1,3,2-dioxaborolane, INT 2

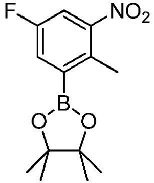

To a mixture of 1-bromo-5-fluoro-2-methyl-3-nitro-benzene (5.0 g, 21.37 mmol) and bis(diphenylphosphino)ferrocenedichloropalladium(II) (0.78 g, 1.06 mmol) in dioxane (200 mL) was added BISPIN (8.14 g, 32.05 mmol) followed by potassium acetate (7.34 g, 74.79 mmol). The reaction mixture was stirred at 100 °C for 6 hr. After cooling the brownish mixture was diluted with water (200 mL) and extracted with EtOAc. The organic layer was washed with saturated aqueous sodium hydrogen carbonate solution and brine (2x), dried over sodium sulfate, filtered and concentrated. The residue was purified by flash chromatography (silica; cyclohexane/EtOAc 9:1) to afford **INT 2** as a yellow oil. ¹H NMR (DMSO-*d*₆): δ (ppm) 7.79 (d, 1H), 7.55 (d, 1H), 2.48 (s, 3H), 1.31 (s, 12H).

### (3) 5-Fluoro-2-methyl-3-(4,4,5,5-tetramethyl-1,3,2-dioxaborolan-2-yl)aniline, INT 3

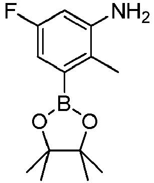

To a solution of **INT 2** (12.4 g, 44.1 mmol) in EtOAc (300 mL) was added Pd/C 10% (4.0 g). The reaction mixture was hydrogenated at room temperature and normal pressure for 18 hr. The mixture was filtered over Kieselgur (Supelco) and the filtrate was concentrated. The residue was purified by flash chromatography (silica, EtOAc) to afford **INT 3** as a beige solid.

MS (ESI): 252.2 [M+H]⁺, ¹H NMR (DMSO-*d*₆): δ (ppm) 6.52-6.46 (m, 2H), 5.13 (br s, 2H), 2.17 (s, 3H), 1.29 (s, 12H).

### (4) Methyl 4-cyclopropyl-2-fluorobenzoate, INT 4

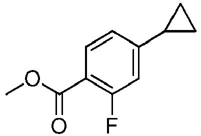

A mixture of methyl 4-bromo-2-fluorobenzoate (20.00 g, 85.82 mmol), cyclopropylboronic acid (9.68 g, 112.69 mmol) and potassium phosphate (35.70 g, 168.00 mmol) in toluene (250 mL) was degassed with argon for 5 min. Then, tricyclohexylphosphine (2.36 g, 8.41 mmol) and water (1.82 mL, 101.00 mmol) were added and the mixture was again degassed with argon for 5 min. Palladium(II) acetate (0.94 g, 4.21 mmol) was added and the reaction mixture was stirred at 100 °C overnight. The mixture was partitioned between EtOAc and water. The suspension was filtered through a pad of Celite. The phases of the filtrate were separated, the aqueous layer was back-extracted with EtOAc. The organic layers were combined, washed with saturated aqueous sodium hydrogen carbonate solution and brine, dried over magnesium sulfate, filtered and concentrated. The residue was purified by flash chromatography (cyclohexane/EtOAc gradient, 0-15%) to afford **INT 4** as an orange oil.

UPLC-MS: MS (ESI): [M+H]⁺ 195.0, rt = 1.11 min. ¹H NMR (CDCl₃): δ (ppm) 7.83 (t, 1H), 6.90 (d, 1H), 6.79 (d, 1H), 3.92 (s, 3H), 2.00-1.96 (m, 1H), 1.15-1.03 (m, 2H), 0.84-0.73 (m, 2H).

### (5) 4-Cyclopropyl-2-fluoro-N-(5-fluoro-2-methyl-3-(4,4,5,5-tetramethyl-1,3,2-dioxaborolan-2-yl)phenyl)benzamide, INT 5

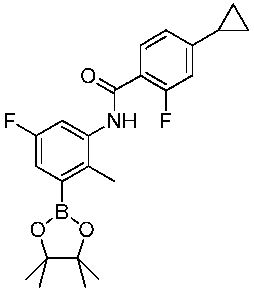

To a solution of **INT 3** (5.88 g, 23.41 mmol) and **INT 4** (5.00 g, 25.70 mmol) in THF (200 mL) at 0°C was added dropwise NaHMDS solution (1 M in THF, 35.1 mL, 35.10 mmol). The reaction mixture was stirred at RT for 2 hr, then additional NaHMDS solution (1 M in THF, 5.0 mL, 5.00 mmol) was added. After stirring for another hour more NaHMDS solution (1 M in THF, 5.0 mL, 5.00 mmol) was added and the mixture was stirred for an additional 2 hr. The mixture was diluted with EtOAc and washed with saturated aqueous sodium hydrogen carbonate solution and brine. The organic layer was dried over magnesium sulfate, filtered and concentrated. The crude was suspended in EtOAc and filtered. The collected solid was washed with EtOAc and dried in vacuum to afford compound **INT 5** as a beige solid.

UPLC-MS: MS (ESI): [M+H]⁺ 414.2, rt = 1.45 min. ¹H NMR (DMSO-*d*₆): δ (ppm) 9.70 (br s, 1H), 7.62 (t, 1H), 7.51 (d, 1H), 7.19 (dd, 1H), 7.10-7.00 (m, 2H), 2.37 (s, 3H), 2.06-1.96 (m, 1H), 1.31 (s, 12H), 1.08-0.99 (m, 2H), 0.82-0.73 (m, 2H).

### (6) tert-Butyl 3-((4-amino-6-(3-(4-cyclopropyl-2-fluorobenzamido)-5-fluoro-2-methylphenyl)pyrimidin-5-yl)oxy)azetidine-1-carboxylate, INT 6

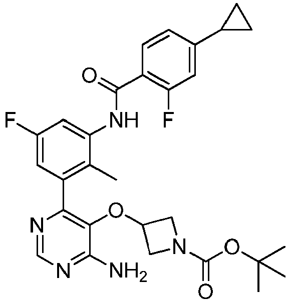

To a solution of **INT 1** (500 mg, 1.66 mmol) in DME (8.4 mL) and water (1.2 mL) was added **INT 5** (756 mg, 1.83 mmol) followed by aqueous sodium carbonate solution (1 M, 4.99 mL, 4.99 mmol). The mixture was degassed with argon for 10 min, then bis(triphenylphosphine)palladium(II) dichloride (58.3 mg, 0.083 mmol) was added. The reaction mixture was stirred for 10 min at 110 °C in a microwave reactor. More **INT 5** (137 mg, 0.33 mmol) was added. The reaction mixture was stirred at 110 °C for an additional 10 min in a microwave reactor. The mixture was partitioned between EtOAc and saturated aqueous sodium hydrogen carbonate solution. The solid was filtered off, washed with water and EtOAc, and dried in vacuum to afford compound **INT 6** as an off-white solid. The mother liquor of the filtration was transferred in an extraction funnel and the layers were separated. The aqueous layer was back-extracted with EtOAc. The combined organic layers were washed with brine, dried over magnesium sulfate, filtered and concentrated. The residue was purified by flash chromatography (silica; DCM/EtOAc gradient, 0-100%) to afford more **INT 6** as an off-white solid.

UPLC-MS: MS (ESI): [M+H]⁺ 552.3, rt = 1.15 min. ¹H NMR (DMSO-*d*₆): δ (ppm) 9.86 (s, 1H), 8.19 (s, 1H), 7.66 (t, 1H), 7.58 (d, 1H), 7.21-6.91 (m, 5H), 4.31-4.16 (m, 1H), 3.77-3.46 (m, 4H), 2.08-1.99 (overlapping s, 3H and m, 1H), 1.31 (s, 9H), 1.12-0.98 (m, 2H), 0.87-0.73 (m, 2H).

### (7) N-(3-(6-Amino-5-(azetidin-3-yloxy)pyrimidin-4-yl)-5-fluoro-2-methylphenyl)-4-cyclopropyl-2-fluorobenzamide, INT 7

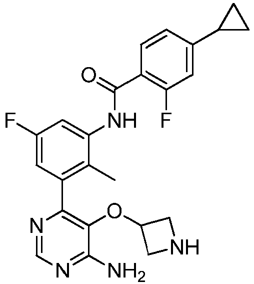

To a solution of **INT 6** (100 mg, 0.18 mmol) in DCM (2.0 mL) was added TFA (0.210 mL, 2.72 mmol) dropwise. The reaction mixture was stirred at RT overnight. The mixture was concentrated and the residue was dried in vacuum to afford crude **INT 7** as the TFA salt as a brown oil.

UPLC-MS: MS (ESI): [M+H]⁺ 452.3, rt = 0.73 min. ¹H NMR (DMSO-*d*₆): δ (ppm) 10.04 (s, 1H), 8.84 (s, br, 2H), 8.63 (s, 1H), 8.56 (s, br, 2H), 7.73-7.61 (m, 2H), 7.32-7.24 (m, 1H), 7.14-7.03 (m, 2H), 4.54-45 (m, 1H), 3.92-3.46 (m, br, 4H), 2.10-2.01 (overlapping s, 3H and m, 1H), 1.12-1.03 (m, 2H), 0.83-0.77 (m, 2H).

### (8) N-(3-(5-((1-Acryloylazetidin-3-yl)oxy)-6-aminopyrimidin-4-yl)-5-fluoro-2-methylphenyl)-4-cyclopropyl-2-fluorobenzamide

To a solution of acrylic acid (73 mg, 1.02 mmol) in DMF (1.5 mL) was added DIPEA (0.47 mL, 2.71 mmol) followed by T3P solution (50% in DMF) (0.51 mL, 0.88 mmol). The mixture was stirred at RT for 20 min. To a solution of **INT 7** (containing 2.5 eq TFA) (499 mg, 0.68 mmol) and DIPEA (0.36 mL, 2.03 mmol) in DMF (5.3 mL) at 0 °C was added dropwise the above solution. The reaction mixture was stirred at 0 °C for 90 min. The mixture was diluted with EtOAc and washed with saturated aqueous sodium hydrogen carbonate solution. The aqueous layer was back-extracted with EtOAc. The combined organic layers were washed with water and brine (2x), dried over magnesium sulfate, filtered and concentrated. The residue was purified by flash chromatography (silica; DCM/(MeOH with 2% aqueous ammonium hydroxide) gradient, 0-10%) to afford the title compound **Example 1** as a white solid after trituration with diethyl ether.

UPLC-MS: MS (ESI): [M+H]⁺ 506.2, rt = 0.93 min. ¹H NMR (DMSO-*d*₆): δ (ppm) 9.89 (s, 1H), 8.2 (s, 1H), 7.66 (t, 1H), 7.54 (d, 1H), 7.2-7.0 (m, 5H), 6.15 (dd, 1H), 6.02 (dd, 1H), 5.61 (dd, 1H), 4.37-4.29 (m, 1H), 4.11-3.95 (m, 2H), 3.8-3.66 (m, 2H), 2.08-1.99 (overlapping s, 3H and m, 1H), 1.08-1.02 (m, 2H), 0.83-0.76 (m, 2H).

### Example 2

### (E)-N-(3-(6-Amino-5-((1-(but-2-enoyl)azetidin-3-yl)oxy)pyrimidin-4-yl)-5-fluoro-2-methylphenyl)-4-cyclopropyl-2-fluorobenzamide

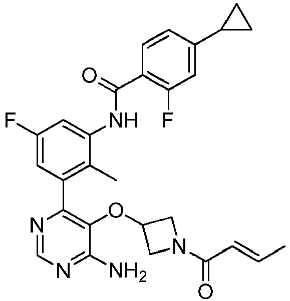

The title compound was prepared according to **Scheme 1** following a procedure analogous to **Example 1** replacing acrylic acid with (*E*)-but-2-enoic acid in step 8.

UPLC-MS: MS (ESI): [M+H]⁺ 520.2, rt = 0.97 min.

### Example 3

### N-(3-(6-Amino-5-((1-propioloylazetidin-3-yl)oxy)pyrimidin-4-yl)-5-fluoro-2-methylphenyl)-4-cyclopropyl-2-fluorobenzamide

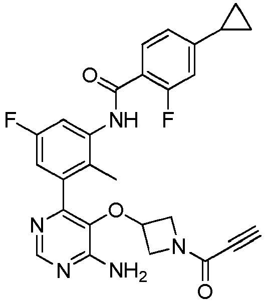

The title compound was prepared according to **Scheme 1** following a procedure analogous to **Example 1** replacing acrylic acid with propiolic acid in step 8.

UPLC-MS: MS (ESI): [M+H]⁺ 504.2, rt = 0.95 min.

### Example 4

### N-(3-(6-Amino-5-((1-(but-2-ynoyl)azetidin-3-yl)oxy)pyrimidin-4-yl)-5-fluoro-2-methylphenyl)-4-cyclopropyl-2-fluorobenzamide

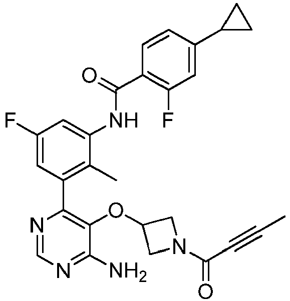

The title compound was prepared according to **Scheme 1** following a procedure analogous to **Example 1** replacing acrylic acid with 2-butynoic acid in step 8.

UPLC-MS: MS (ESI): [M+H]⁺ 518.2, rt = 0.97 min.

### Example 5

### N-(3-(5-((1-Acryloylpiperidin-4-yl)oxy)-6-aminopyrimidin-4-yl)-5-fluoro-2-methylphenyl)-4-cyclopropyl-2-fluorobenzamide

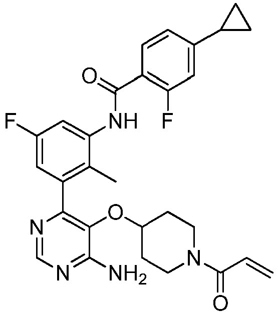

The title compound was prepared according to **Scheme 1** following a procedure analogous to **Example 1** replacing *N*-Boc-3-iodoazetidine with *N*-Boc-4-bromopiperidine in step 1.

UPLC-MS: MS (ESI): [M+H]⁺ 534.2, rt = 0.94 min.

Alternatively, agents of the disclosure may be prepared by a reaction sequence involving Mitsunobu reaction of 4-amino-6-chloropyrimidin-5-ol with an alcohol of formula 2' using an appropriate azodicarboxylate, such as DIAD, and Smopex-301 or triphenylphosphine; thereupon the reaction sequences of scheme 1 are being carried out, i.e. the Suzuki coupling with a boronic ester using an appropriate catalyst, such as bis(triphenyl-phosphine)-palladium(II) dichloride, deprotection using an appropriate acid, such as TFA or HCl, followed by amide formation of the ammonium salt or the free amine with an acid and using an appropriate coupling reagent, such as T3P, and an appropriate base, such as DIPEA, or with an acid chloride using an appropriate base, such as DIPEA, to yield a compound of the disclosure, i.e. a compound of formula 7, as shown in **Scheme 2** below:

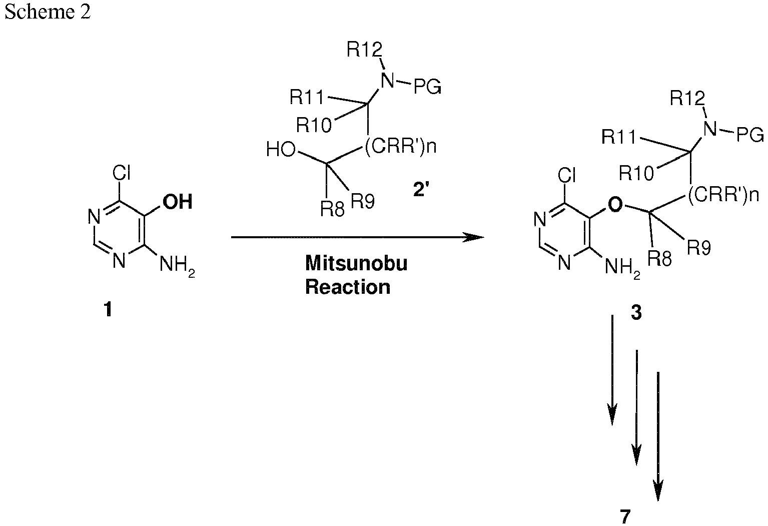

### Example 6

### N-(3-(6-Amino-5-(2-(N-methylacrylamido)ethoxy)pyrimidin-4-yl)-5-fluoro-2-methylphenyl)-4-cyclopropyl-2-fluorobenzamide

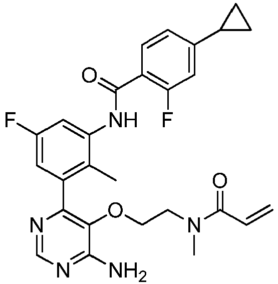

### (1) tert-Butyl (2-((4-amino-6-chloropyrimidin-5-yl)oxy)ethyl)(methyl)carbamate, INT 8

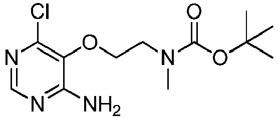

To a solution of 4-amino-6-chloropyrimidin-5-ol (content 90%, 2.00 g, 12.37 mmol) in THF (120 mL) was added *N*-Boc-*N*-methyl-2-hydroxyethylamine (6.07 g, 34.64 mmol) followed by SMOPEX-301 (1 mmol/g, 30.90 g, 30.90 mmol). Then, a solution of DIAD (6.01 mL, 30.52 mmol) in THF (20 mL) was added slowly. The reaction mixture was stirred at 60 °C for 3 hr. The mixture was filtered through a pad of Celite. The filtrate was concentrated to afford an oil which was triturated with EtOAc and a white precipitate was formed. The solid was filtered off to afford **INT 8.** The mother liquor was concentrated and the residue was purified by flash chromatography (silica; DCM/EtOAc gradient, 0-100%) to afford more **INT 8** as a beige solid.

UPLC-MS: MS (ESI): [M+H]⁺ 303.1, rt = 0.86 min. ¹H NMR (DMSO-*d*₆): δ (ppm) 7.97 (s, 1H), 7.26 (s, br, 2H), 4.02-3.93 (m, 2H), 3.54 (t, 2H), 2.89 (s, br, 3H), 1.39 (s, 9H).

### (2) tert-Butyl (2-((4-amino-6-(3-(4-cyclopropyl-2-fluorobenzamido)-5-fluoro-2-methylphenyl)pyrimidin-5-yl)oxy)ethyl)(methyl)carbamate, INT 9

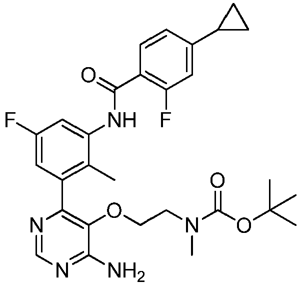

To a solution of **INT 8** (447 mg, 1.48 mmol) in DME (7.0 mL) and water (1.0 mL) was added **INT 5** (638 mg, 1.54 mmol) followed by aqueous sodium carbonate solution (1 M, 4.21 mL, 4.21 mmol). The mixture was degassed with argon for 10 min and bis(triphenylphosphine)palladium(II) dichloride (49.2 mg, 0.070 mmol) was added. The reaction mixture was stirred at 110 °C for 10 min in a microwave reactor. More **INT 5** (232 mg, 0.56 mmol) was added and the reaction mixture was stirred at 110 °C for an additional 15 min in a microwave reactor. The mixture was partitioned between saturated aqueous sodium hydrogen carbonate solution and EtOAc. The organic layer was washed with water and brine, dried over magnesium sulfate, filtered and concentrated. The residue was purified by flash chromatography (silica; DCM/EtOAc gradient, 0-100%) to afford **INT 9** as an off-white solid. UPLC-MS: MS (ESI): [M+H]⁺ 554.3, rt = 1.21 min. ¹H NMR (DMSO-*d*₆): δ (ppm) rotamers 9.76 (s, 1H), 8.19 (s, 1H), 7.74-7.53 (m, 2H) 7.20-6.85 (m, 5H), 3.57-3.48 (m, 2H), 3.29-3.15 (m, 2H), 2.58 (s, 3H), 2.08-1.99 (overlapping s, 3H and m, 1H), 1.34 and 1.28 (s, 9H), 1.10-1.02 (m, 2H), 0.84-0.77 (m, 2H).

### (3) N-(3-(6-Amino-5-(2-(methylamino)ethoxy)pyrimidin-4-yl)-5-fluoro-2-methylphenyl)-4-cyclopropyl-2-fluorobenzamide, INT 10

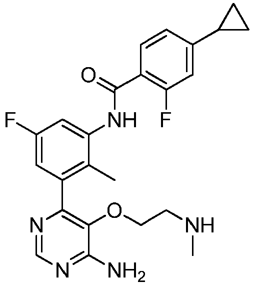

To a solution of **INT 9** (335 mg, 0.61 mmol) in DCM (5.0 mL) was added TFA (0.47 mL, 6.05 mmol). The reaction mixture was stirred at RT for 15 hr. The mixture was concentrated under reduced pressure. The residue was dried in vacuum to afford **INT 10** as theTFA salt as a brown oil.

UPLC-MS: MS (ESI): [M+H]⁺ 454.3, rt = 0.73 min. ¹H NMR (DMSO-*d*₆): δ (ppm) 10.02 (s, 1H), 9.07-8.13 (s, v br, number of H cannot be assigned), 8.58 (s, 1H), 8.51 (s, br, 2H), 7.71-7.61 (m, 2H), 7.29-7.22 (m, 1H), 7.14-7.05 (m, 2H), 3.75-3.65 (m, 2H), 3.16-3.07 (m, 2H), 2.48 (s, 3H, overlapping with solvent peak), 2.12 (s, 3H), 2.10-1.99 (m, 1H), 1.11-1.03 (m, 2H), 0.83-0.76 (m, 2H).

### (4) N-(3-(6-Amino-5-(2-(N-methylacrylamido)ethoxy)pyrimidin-4-yl)-5-fluoro-2-methylphenyl)-4-cyclopropyl-2-fluorobenzamide

To a solution of acrylic acid (62 mg, 0.87 mmol) in DMF (4.0 mL) was added DIPEA (0.302 mL, 1.73 mmol) followed by T3P solution (50% in DMF) (0.438 mL, 0.750 mmol). The mixture was stirred at RT for 30 min. To a solution of **INT 10** (containing 3.0 eq TFA, content 90%, 510 mg, 0.577 mmol) and DIPEA (0.302 mL, 1.731 mmol) in DMF (2.0 mL) at 0 °C was added dropwise the above solution. The reaction mixture was stirred at 0 °C for 30 min. The mixture was diluted with water and extracted with EtOAc. The organic layer was washed with water (2x) and brine (2x), dried over magnesium sulfate, filtered and concentrated. The residue was purified by flash chromatography (silica; DCM/(MeOH with 2% aqueous ammonium hydroxide) gradient, 0-9%) to afford the title compound **Example 6** as a white solid.

UPLC-MS: MS (ESI): [M+H]⁺ 508.3, rt = 0.95 min. ¹H NMR (DMSO-*d*₆): δ (ppm) rotamers 9.77 and 9.56 (s, total 1H), 8.25-8.14 (m, 1H), 7.79-7.50 (m, 2H), 7.17-6.93 (m, 5H), 6.70-6.55 (m, 1H), 6.06 (t, 1H), 5.59 (d, 1H), 3.63-3.40 (m, 4H), 2.80 and 2.49 (s, total 3H, peak at 2.49 overlapping with solvent peak), 2.09-1.93 (m, 4H), 1.11-1.00 (m, 2H), 0.85-0.76 (m, 2H).

### Example 7

### (E)-N-(3-(6-Amino-5-(2-(N-methylbut-2-enamido)ethoxy)pyrimidin-4-yl)-5-fluoro-2-methylphenyl)-4-cyclopropyl-2-fluorobenzamide

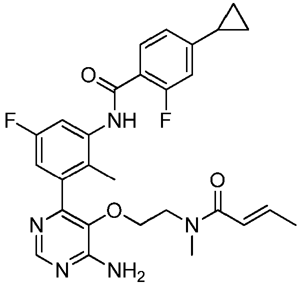

The title compound was prepared according to **Scheme 2** following a procedure analogous to **Example 6** replacing acrylic acid with (*E*)-but-2-enoic acid in step 4.

UPLC-MS: MS (ESI): [M+H]⁺ 522.2, rt = 0.97 min.

### Example 8

### N-(3-(6-Amino-5-(2-(N-methylpropiolamido)ethoxy)pyrimidin-4-yl)-5-fluoro-2-methylphenyl)-4-cyclopropyl-2-fluorobenzamide

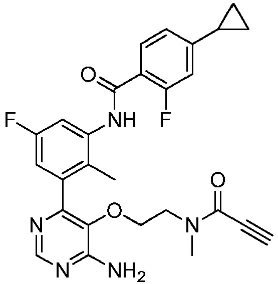

The title compound was prepared according to **Scheme 2** following a procedure analogous to **Example 6** replacing acrylic acid with propiolic acid in step 4.

UPLC-MS: MS (ESI): [M+H]⁺ 506.3, rt = 0.95 min.

### Example 9

### (E)-N-(3-(6-Amino-5-(2-(4-methoxy-N-methylbut-2-enamido)ethoxy)pyrimidin-4-yl)-5-fluoro-2-methylphenyl)-4-cyclopropyl-2-fluorobenzamide

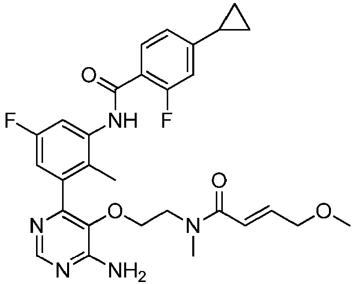

### (1) N-(3-(6-Amino-5-(2-(methylamino)ethoxy)pyrimidin-4-yl)-5-fluoro-2-methylphenyl)-4-cyclopropyl-2-fluorobenzamide, INT 11

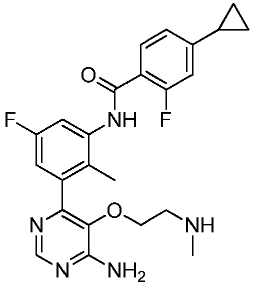

To a solution of **INT 9** (2.50 g, 4.52 mmol) in DCM (30 mL) was added HCl (2 M in diethyl ether, 20.0 mL, 40.00 mmol). The reaction mixture was stirred at RT for 4 hr. The mixture was concentrated under reduced pressure and the residue was dried in vacuum to afford **INT 11** as the hydrochloride salt as a white solid.

UPLC-MS: MS (ESI): [M+H]⁺ 454.2, rt = 0.70 min. ¹H NMR (MeOD-*d*₃): δ (ppm) 8.60 (s, 1H), 7.82 (t, 1H), 7.69-7.62 (m, 1H), 7.41-7.36 (m, 1H), 7.10 (d, 1H), 7.02 (d, 1H), 4.10-3.80 (m, br, 2H), 3.39-3.20 (m, 2H), 2.70 (s, 3H), 2.26 (s, 3H), 2.11-1.99 (m, 1H), 1.19-1.07 (m, 2H), 0.89-0.77 (m, 2H).

### (2) (E)-N-(3-(6-Amino-5-(2-(4-methoxy-N-methylbut-2-enamido)ethoxy)pyrimidin-4-yl)-5-fluoro-2-methylphenyl)-4-cyclopropyl-2-fluorobenzamide

The title compound was prepared following a procedure analogous to step 4 of **Example 6** replacing **INT 10** with **INT 11** (hydrochloride salt) and replacing acrylic acid with *(E)*-4-methoxy-but-2-enoic acid.

UPLC-MS: MS (ESI): [M+H]⁺ 552.2, rt = 0.93 min.

### Example 10

### N-(3-(6-Amino-5-(2-(N-methylbut-2-ynamido)ethoxy)pyrimidin-4-yl)-5-fluoro-2-methylphenyl)-4-cyclopropyl-2-fluorobenzamide

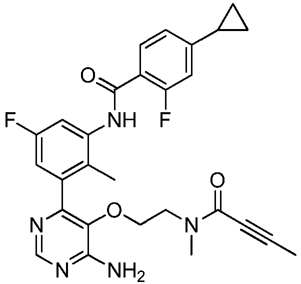

The title compound was prepared according to **Scheme 2** following a procedure analogous to **Example 6** replacing acrylic acid with 2-butynoic acid in step 4.

UPLC-MS: MS (ESI): [M+H]⁺ 520.2, rt = 0.96 min.

### Example 11

### N-(2-((4-Amino-6-(3-(4-cyclopropyl-2-fluorobenzamido)-5-fluoro-2-methylphenyl)pyrimidin-5-yl)oxy)ethyl)-N-methyloxirane-2-carboxamide

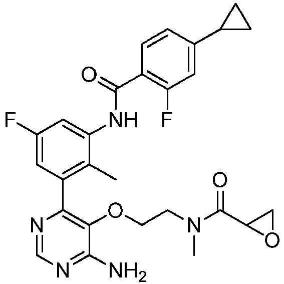

To a solution of TBHP (5.5 M in decane, 0.079 mL, 0.434 mmol) in THF (2.0 mL) at -78 °C was added *n*-butyl lithium (2.5 M in hexane, 0.145 mL, 0.362 mmol). The mixture was stirred at -78 °C for 10 min. Then, a solution of **Example 6** (147 mg, 0.290 mmol) in THF (1.0 mL) was added and the reaction mixture was stirred at RT for 5 hr. The mixture was diluted with water and extracted with EtOAc. The organic layer was washed with water and brine, dried over magnesium sulfate, filtered and concentrated. The residue was purified by preparative HPLC (Xterra 150, water/acetonitrile gradient) to afford **Example 11** as a white solid after lyophilization.

UPLC-MS: MS (ESI): [M+H]⁺ 524.4, rt = 0.88 min. ¹H NMR (DMSO-*d*₆): δ (ppm) rotamers 9.83 and 9.58 (s, total 1H), 8.26-8.15 (m, 1H), 7.78-7.61 (m, 1H), 7.61-7.48 (m, 1H), 7.22-6.90 (m, 5H), 3.84-3.39 (m, 5H), 2.89 (s, 1.2H), 2.87-2.76 (m, 1H), 2.71-2.61 (m, 1H), 2.44 (s, 1.8H, overlapping with solvent peak), 2.10-1.93 (m, 4H), 1.12-0.99 (m, 2H), 0.87-0.74 (m, 2H).

### Example 12

### N-(2-((4-Amino-6-(3-(6-cyclopropyl-8-fluoro-1-oxoisoquinolin-2(1H)-yl)phenyl)pyrimidin-5-yl)oxy)ethyl)-N-methylacrylamide

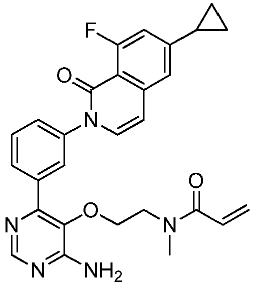

### (1) 2-(3-Chlorophenyl)-6-cyclopropyl-8-fluoroisoquinolin-1(2H)-one, INT 12

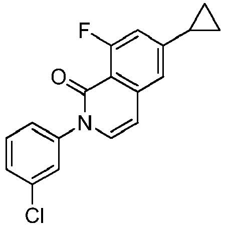

A mixture of 1-chloro-3-iodobenzene (0.439 ml, 3.54 mmol), 6-cyclopropyl-8-fluoro-isoquinolin-1(2*H*)-one (600 mg, 2.95 mmol), ethyl 2-oxocyclohexanecarboxylate (0.094 mL, 0.591 mmol) and cesium carbonate (2020 mg, 6.20 mmol) in DMSO (15 mL) was degassed with argon for 5 min. Copper(I) iodide (112 mg, 0.59 mmol) was added, the reaction flask was sealed, the mixture stirred at 120 °C for 16 hr. The mixture was cooled to RT and diluted with EtOAc (100 mL). The resulting slurry was filtered over Hyflo and the filter cake was washed with EtOAc. The filtrate was concentrated and the residue was purified by flash chromatography (silica; cyclohexane/EtOAc gradient, 5-40%) to afford **INT 12** as a yellow solid.

UPLC-MS: MS (ESI): [M+H]⁺ 314.1, rt = 1.25 min. ¹H NMR (DMSO-*d*₆): δ (ppm) 7.61 (s, 1H), 7.59-7.50 (m, 2H), 7.48-7.40 (m, 2H), 7.26 (s, 1H), 6.99 (d, 1H), 6.60 (d, 1H), 2.12-2.02 (m, 1H), 1.14-1.05 (m, 2H), 0.92-0.83 (m, 2H).

### (2) 6-Cyclopropyl-8-fluoro-2-(3-(4,4,5,5-tetramethyl-1,3,2-dioxaborolan-2-yl)phenyl)isoquinolin-1(2H)-one, INT 13

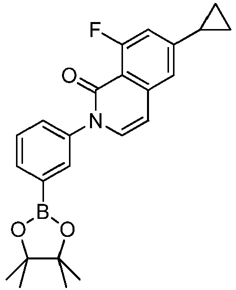

A mixture of **INT 12** (808 mg, 2.58 mmol), BISPIN (981 mg, 3.86 mmol), X-Phos (123 mg, 0.26 mmol) and potassium acetate (758 mg, 7.73 mmol) in dioxane (13 mL) was degassed under argon for 5 min. Tris(dibenzylideneacetone)dipalladium(0) (118 mg, 0.13 mmol) was added and the reaction flask was sealed. The reaction mixture was stirred at 105 °C for 2 hr. The mixture was cooled to RT, filtered over Hyflo and the filter cake was washed with EtOAc. Triphenylphosphine (169 mg, 0.64 mmol) was added to the filtrate. The filtrate was concentrated and the residue was purified by flash chromatography (silica;cyclohexane/EtOAc gradient, 5-40%). The residue was triturated with a mixture of diethyl ether and pentane (1:1) and filtered. The filter cake was washed with pentane and dried in vacuum to afford **INT 13** as a white solid.

UPLC-MS: MS (ESI): [M+H]⁺ 406.3, rt = 1.40 min. ¹H NMR (DMSO-*d*₆): δ (ppm) 7.75-7.70 (m, 1H), 7.64 (s, 1H), 7.59-7.54 (m, 2H), 7.44 (d, 1H), 7.25 (s, 1H), 6.98 (d, 1H), 6.59 (d, 1H), 2.11-2.02 (m, 1H), 1.31 (s, 12H), 1.13-1.06 (m, 2H), 0.91-0.84 (m, 2H).

### (3) 2-(3-(6-Amino-5-(2-(methylamino)ethoxy)pyrimidin-4-yl)phenyl)-6-cyclopropyl-8-fluoroisoquinolin-1(2H)-one, INT 14

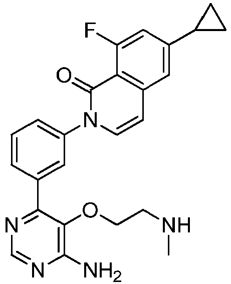

Intermediate **INT 14** was prepared according to **Scheme 2** following a procedure analogous to steps 2 and 3 of **Example 6** replacing **INT 5** with **INT 13** in step 2, and by doing a basic work-up in step 3 to afford **INT 14** as the free amine.

UPLC-MS: MS (ESI): [M+H]⁺ 446.3, rt = 0.71 min. ¹H NMR (DMSO-*d*₆): δ (ppm) 8.21 (s, 1H), 8.13-8.02 (m, 2H), 7.63 (t, 1H), 7.51 (t, 2H), 7.45-7.31 (m, 2H), 7.27 (s, 1H), 6.99 (d, 1H), 6.62 (d, 1H), 3.73-3.64 (m, 2H), 2.73-2.64 (m, 2H), 2.23 (s, 3H), 2.12-2.03 (m, 1H), 1.14-1.06 (m, 2H), 0.92-0.83 (m, 2H).

### (4) N-(2-((4-Amino-6-(3-(6-cyclopropyl-8-fluoro-1-oxoisoquinolin-2(1H)-yl)phenyl)pyrimidin-5-yl)oxy)ethyl)-N-methylacrylamide

To a solution of **INT 14** (73 mg, 0.16 mmol) and DIPEA (86 µl, 0.492 mmol) in THF (1.6 mL) at -20 °C was added acryloyl chloride (14 µl, 0.172 mmol). The reaction mixture was stirred at -20 °C for 10 min. The mixture was diluted with aqueous sodium carbonate solution (2 M) and water and extracted with DCM (3x). The combined organic layers were dried over sodium sulfate, filtered and concentrated. The residue was purified by SFC to afford **Example 12** as a white solid.

UPLC-MS: MS (ESI): [M+H]⁺ 500.4, rt = 0.93 min. ¹H NMR (DMSO-*d*₆): δ (ppm) rotamers 8.26-8.18 (m, 1H), 8.04-7.87 (m, 2H), 7.64-7.43 (m, 3H), 7.27 (s, 1H), 7.16-7.03 (m, 2H), 7.03-6.95 (m, 1H), 6.85 and 6.69 (dd, total 1H), 6.65-6.58 (m, 1H), 6.09 (d, 1H), 5.60 (t, 1H), 3.84-3.72 (m, 2H), 3.71-3.60 (m, 2H), 3.04 and 2.76 (s, total 3H), 2.13-2.02 (m, 1H), 1.16-1.05 (m, 2H), 0.93-0.83 (m, 2H).

### Example 13

### N-(3-(5-(2-Acrylamidoethoxy)-6-aminopyrimidin-4-yl)-5-fluoro-2-methylphenyl)-4-cyclopropyl-2-fluorobenzamide

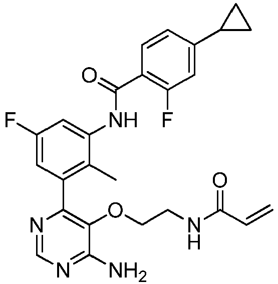

The title compound was prepared according to **Scheme 2** following a procedure analogous to **Example 6** replacing *N*-Boc-*N*-methyl-2-hydroxyethylamine with *N*-Boc-2-hydroxyethylamine in step 1. UPLC-MS: MS (ESI): [M+H]⁺ 494.2, rt = 0.91 min.

### Example 14

### N-(3-(6-Amino-5-(2-(N-ethylacrylamido)ethoxy)pyrimidin-4-yl)-5-fluoro-2-methylphenyl)-4-cyclopropyl-2-fluorobenzamide

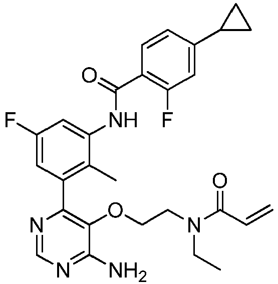

The title compound was prepared according to **Scheme 2** following a procedure analogous to **Example 6** replacing *N*-Boc-*N*-methyl-2-hydroxyethylamine with *N*-Boc-*N*-ethyl-2-hydroxyethylamine in step 1. UPLC-MS: MS (ESI): [M+H]⁺ 522.4, rt = 0.99 min.

### Example 15

### N-(3-(6-Amino-5-(2-(N-(2-fluoroethyl)acrylamido)ethoxy)pyrimidin-4-yl)-5-fluoro-2-methylphenyl)-4-cyclopropyl-2-fluorobenzamide

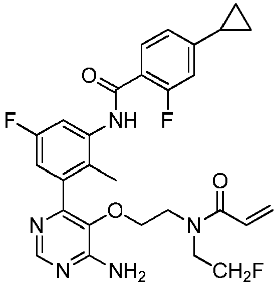

### (1) tert-Butyl (2-(benzyloxy)ethyl)(2-fluoroethyl)carbamate, INT 15

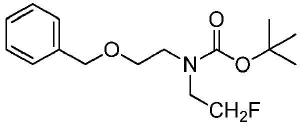

To a solution of 2-fluoroethanamine hydrochloride (4.35 g, 43.71 mmol) and 2-(benzyloxy)-acetaldehyde (6.04 g, 5.65 mL, 40.22 mmol) in MeOH (70 mL) was added sodium triacetoxyborohydride (10.44 g, 49.26 mmol). The reaction mixture was stirred at RT for 4 hr. The mixture was concentrated. The residue was taken up in EtOAc and washed with saturated aqueous sodium hydrogen carbonate solution, water and brine. The organic layer was dried over magnesium sulfate, filtered and concentrated. The residue was taken up in aqueous NaOH solution (2 M, 175 mL, 350 mmol) and di-*tert*-butyl dicarbonate (17.65 g, 80.87 mmol) was added. The reaction mixture was stirred at RT overnight. The mixture was diluted with water and EtOAc. The layers were separated. The aqueous layer was back-extracted with EtOAc. The combined organic layers were washed with water and brine, dried over magnesium sulfate, filtered and concentrated. The residue was purified by flash chromatography (silica, cyclohexane/EtOAc gradient, 0-10%) to afford **INT 15** as a pale colorless oil. MS (ESI): [M+H]⁺ 298.3. ¹H NMR (DMSO-*d*₆): δ (ppm) 7.41-7.24 (m, 5H), 4.59-4.39 (m, 4H), 3.59-3.45 (m, 4H), 3.44-3.36 (m, 2H), 1.46-1.31 (m, 9H).

### (2) N-Boc-N-(2-fluoroethyl)-2-hydroxyethylamine, INT 16

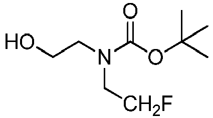

To a solution of **INT 15** (3.40 g, 11.43 mmol) in THF (115 mL) was added Pd-C 10% (340 mg). The reaction mixture was hydrogenated at RT and normal pressure for 7 hr. Pd-C 10% (340 mg) was added, and the reaction mixture was hydrogenated at RT and normal pressure overnight. More Pd-C 10% (340 mg) was added, and the reaction mixture was hydrogenated at RT and normal pressure for an additional 4 hr. The mixture was diluted with DCM, filtered over a pad of Celite and concentrated to afford crude **INT 16** as a colorless oil.

MS (ESI): [M+H]⁺ 208.2. ¹H NMR (DMSO-*d*₆): δ (ppm) 4.70-4.63 (m, 1H), 4.54 (t, 1H), 4.42 (t, 1H), 3.53 (t, 1H), 3.46 (t, 3H), 3.28-3.21 (m, 2H), 1.39 (s, 9H).

### (3) N-(3-(6-Amino-5-(2-(N-(2-fluoroethyl)acrylamido)ethoxy)pyrimidin-4-yl)-5-fluoro-2-methylphenyl)-4-cyclopropyl-2-fluorobenzamide

The title compound was prepared according to **Scheme 2** following a procedure analogous to **Example 6** replacing *N*-Boc-*N*-methyl-2-hydroxyethylamine with **INT 16** in step 1.

UPLC-MS: MS (ESI): [M+H]⁺ 540.3, rt = 0.96 min.

### Example 16

### N-(3-(5-((1-Acrylamidocyclopropyl)methoxy)-6-aminopyrimidin-4-yl)-5-fluoro-2-methylphenyl)-4-cyclopropyl-2-fluorobenzamide

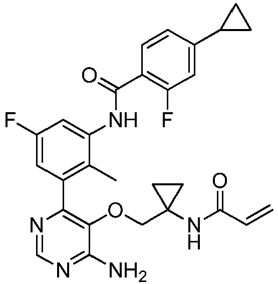

### (1) N-Boc-1-(hydroxymethyl)-cyclopropylamine, INT 17

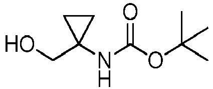

To a solution of methyl 1-((*tert*-butoxycarbonyl)amino)cyclopropanecarboxylate (9.30 g, 43.20 mmol) in THF (45 mL) was added lithium borohydride solution (2 M in THF, 40.0 mL, 80.00 mmol). The reaction mixture was stirred at RT overnight. The mixture was cooled to 0 °C and quenched carefully with water. The mixture was extracted with diethyl ether (2x). The combined organic layers were washed with water and brine, dried over magnesium sulfate, filtered and concentrated to afford crude **INT 17** as a white solid.

MS (ESI): [M+H]⁺ 188.2. ¹H NMR (DMSO-*d*₆): δ (ppm) 7.03 (s, 1H), 4.55 (t, 1H), 3.38 (d, 2H), 1.37 (s, 9H), 0.63-0.50 (m, 4H).

### (2) N-(3-(5-((1-Acrylamidocyclopropyl)methoxy)-6-aminopyrimidin-4-yl)-5-fluoro-2-methylphenyl)-4-cyclopropyl-2-fluorobenzamide

The title compound was prepared according to **Scheme 2** following a procedure analogous to **Example 6** replacing *N*-Boc-*N*-methyl-2-hydroxyethylamine with **INT 17** in step 1.

UPLC-MS: MS (ESI): [M+H]⁺ 520.4, rt = 0.95 min.

### Example 17

### (S)-N-(3-(5-(2-Acrylamidopropoxy)-6-aminopyrimidin-4-yl)-5-fluoro-2-methylphenyl)-4-cyclopropyl-2-fluorobenzamide

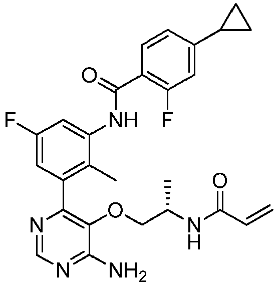

The title compound was prepared according to **Scheme 2** following a procedure analogous to **Example 6** replacing *N*-Boc-*N*-methyl-2-hydroxyethylamine with (*S*)-2-(Boc-amino)-1-propanol in step 1. UPLC-MS: MS (ESI): [M+H]⁺ 508.2, rt = 0.95 min.

### Example 18

### (S)-N-(3-(6-Amino-5-(2-(but-2-ynamido)propoxy)pyrimidin-4-yl)-5-fluoro-2-methylphenyl)-4-cyclopropyl-2-fluorobenzamide

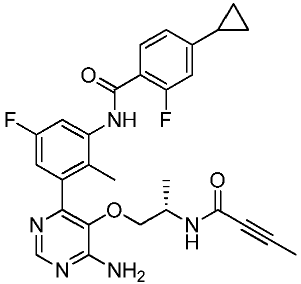

The title compound was prepared according to **Scheme 2** following a procedure analogous to **Example 6** replacing *N*-Boc-*N*-methyl-2-hydroxyethylamine with (*S*)-2-(Boc-amino)-1-propanol in step 1, and replacing acrylic acid with 2-butynoic acid in step 4.

UPLC-MS: MS (ESI): [M+H]⁺ 520.2, rt = 0.97 min.

### Example 19

### (S)-N-(3-(6-Amino-5-(2-(N-methylacrylamido)propoxy)pyrimidin-4-yl)-5-fluoro-2-methylphenyl)-4-cyclopropyl-2-fluorobenzamide

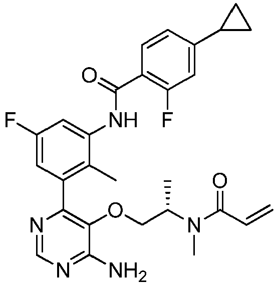

### (1) (S)-N-(3-(6-Amino-5-(2-aminopropoxy)pyrimidin-4-yl)-5-fluoro-2-methylphenyl)-4-cyclopropyl-2-fluorobenzamide, INT 18

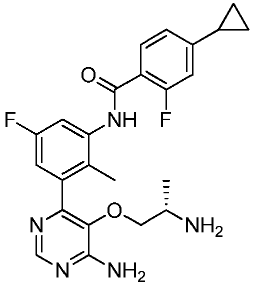

**INT 18** was prepared according to **Scheme 2** following a procedure analogous to **INT 10** replacing *N-*Boc-*N*-methyl-2-hydroxyethylamine with (*S*)-2-(Boc-amino)-1-propanol in step 1, and replacing TFA with HCl in step 3 to afford **INT 18** as the hydrochloride salt.

UPLC-MS: MS (ESI): [M+H]⁺ 454.3, rt = 0.73 min.

### (2) (S)-N-(3-(6-Amino-5-(2-(benzyl(methyl)amino)propoxy)pyrimidin-4-yl)-5-fluoro-2-methylphenyl)-4-cyclopropyl-2-fluorobenzamide, INT 19

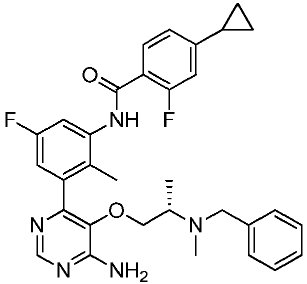

To a solution of **INT 18** (containing 2 eq of HCl, 590 mg, 1.12 mmol) in MeOH (30 mL) was added DIPEA (0.489 mL, 2.80 mmol), followed by acetic acid (0.321 mL, 5.60 mmol). Then a solution of benzaldehyde (131 mg, 1.23 mmol) in MeOH (3 mL) was added. The mixture was stirred at RT for 1 h, then sodium cyanoborohydride (77 mg, 1.23 mmol) was added. The reaction mixture was stirred at RT for 1 h. More sodium cyanoborohydride (35 mg, 0.561 mmol) was added and the mixture was stirred for an additional hour. Formaldehyde (37% in water, 1.00 mL, 13.45 mmol) was added, and stirring was continued for another hour. The mixture was diluted with DCM and washed with saturated aqueous sodium hydrogen carbonate solution. The organic layer was washed with brine, dried over magnesium sulfate, filtered and concentrated. The residue was purified by flash chromatography (silica; DCM/EtOAc gradient, 0-100%) to afford **INT 19** as a white solid.

UPLC-MS: MS (ESI): [M+H]⁺ 558.4, rt = 0.90 min. ¹H NMR (DMSO-*d*₆): δ (ppm) 9.79 (s, 1H), 8.20 (s, 1H), 7.63 (t, 1H), 7.55 (d, 1H), 7.34-7.14 (m, 7H), 7.12-6.95 (m, 3H), 3.65-3.56 (m, 1H), 3.48 (d, 1H), 3.39 (d, 1H), 3.34-3.27 (m, 2H), 2.99-2.86 (m, 1H), 2.03-1.99 (m, 4H), 1.94 (s, 3H), (1.11-0.99 (m, 2H), 0.83-0.70 (m, 2H).

### (3) (S)-N-(3-(6-Amino-5-(2-(methylamino)propoxy)pyrimidin-4-yl)-5-fluoro-2-methylphenyl)-4-cyclopropyl-2-fluorobenzamide, INT 20

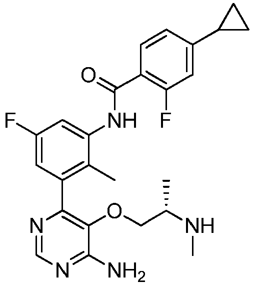

To a solution of **INT 19** (470 mg, 0.843 mmol) in MeOH (9 mL) was added Pd-C 10% (47 mg). The reaction mixture was hydrogenated at RT and normal pressure for 18 hr. More Pd-C 10% (47 mg) was added and the reaction was hydrogenated at RT and normal pressure overnight. The mixture was diluted with DCM and filtered over a pad of Celite. The filtrate was concentrated and the residue was dried in vacuum to afford crude **INT 20** as brown-gray solid.

UPLC-MS: MS (ESI): [M+H]⁺ 468.4, rt = 0.76 min. ¹H NMR (DMSO-*d*₆): δ (ppm) 9.84 (s, 1H), 8.18 (s, 1H), 7.65 (t, 1H), 7.58-7.49 (m, 1H), 7.28 (s, br, 1H), 7.09-7.00 (m, 3H), 3.34-3.25 (m, 3H), 3.17 (s, br, 1H), 2.17-1.98 (m, 7H), 1.67 (s, br, 1H), 1.08-1.01 (m, 2H), 0.81-0.77 (m, 2H).

### (4) (S)-N-(3-(6-Amino-5-(2-(N-methylacrylamido)propoxy)pyrimidin-4-yl)-5-fluoro-2-methylphenyl)-4-cyclopropyl-2-fluorobenzamide

The title compound was prepared according to **Scheme 2** following a procedure analogous to step 4 of **Example 6** replacing **INT 10** with **INT 20.**

UPLC-MS: MS (ESI): [M+H]⁺ 522.3, rt = 0.99 min.

### Example 20

### (S)-N-(3-(6-Amino-5-(2-(N-methylbut-2-ynamido)propoxy)pyrimidin-4-yl)-5-fluoro-2-methylphenyl)-4-cyclopropyl-2-fluorobenzamide

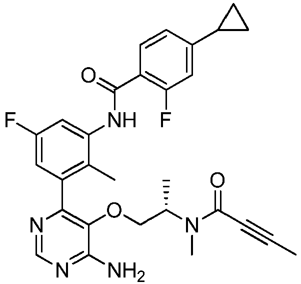

### (1) (S)-tert-Butyl (5-(2-(but-2-ynamido)propoxy)-6-(3-(N-(tert-butoxycarbonyl)-4-cyclopropylbenzamido)-5-fluoro-2-methylphenyl)pyrimidin-4-yl)(tert-butoxycarbonyl)carbamate, INT 21

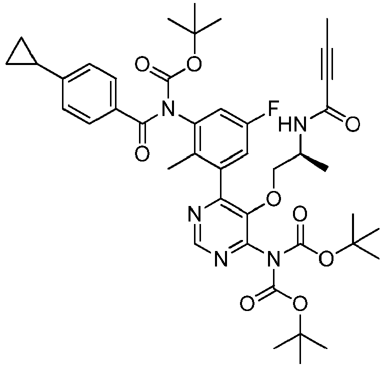

To a solution of **Example 18** (152 mg, 0.29 mmol) in THF (10 mL) was added DIPEA (0.200 mL, 1.15 mmol) followed by di-*tert*-butyl dicarbonate (233 mg, 1.07 mmol) and 4-(dimethylamino)pyridine (4 mg, 0.033 mmol). The reaction mixture was stirred at RT overnight. More di-*tert*-butyl dicarbonate (100 mg, 0.46 mmol) was added and the reaction mixture was stirred at RT for 1.5 hr. The mixture was concentrated under reduced pressure. The residue was purified by flash chromatography (silica; cyclohexane/EtOAc gradient, 0-100%) to afford **INT 21** as a yellow residue.

UPLC-MS: MS (ESI): [M+H]⁺ 820.4, rt = 1.48 min.

### (2) (S)-tert-butyl tert-butoxycarbonyl(6-(3-(N-(tert-butoxycarbonyl)-4-cyclopropyl-benzamido)-5-fluoro-2-methylphenyl)-5-(2-(N-methylbut-2-ynamido)propoxy)pyrimidin-4-yl)carbamate, INT 22

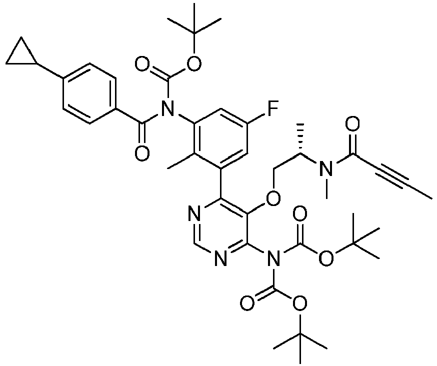

To a solution of **INT 21** (257 mg, 0.31 mmol) and iodomethane (0.040 mL, 0.64 mmol) in DMF (5.0 mL) at 0 °C was added NaH (60% in mineral oil, 26 mg, 0.65 mmol). The reaction mixture was stirred for 1.5 hr while allowing to warm to RT. The mixture was poured into aqueous HCl (0.5 M) and extracted with EtOAc (2x). The combined organic layers were washed with brine, dried over sodium sulfate, filtered and concentrated. The residue was purified by flash chromatography (silica; cyclohexane/EtOAc gradient, 0-100%) to afford **INT 22.**

UPLC-MS: MS (ESI): [M+H]⁺ 834.5, rt = 1.49 min.

### (3) (S)-N-(3-(6-Amino-5-(2-(N-methylbut-2-ynamido)propoxy)pyrimidin-4-yl)-5-fluoro-2-methylphenyl)-4-cyclopropyl-2-fluorobenzamide

To a solution of **INT 22** (117 mg, 0.14 mmol) in DCM (5.0 mL) was added TFA (0.200 mL, 2.60 mmol) followed by one drop of water. The reaction mixture was stirred at RT overnight. The mixture was concentrated. The residue was taken up in EtOAc and washed with saturated aqueous sodium hydrogen carbonate solution. The aqueous layer was back-extracted with EtOAc. The combined organic layers were washed with brine, dried over magnesium sulfate, filtered and concentrated. The residue was purified by flash chromatography (silica; EtOAc/MeOH gradient, 0-15%) followed by purification by SFC to afford **Example 20.**

UPLC-MS: MS (ESI): [M+H]⁺ 534.3, rt = 1.02 min. . ¹H NMR (CDCl₃): δ (ppm) rotamers 8.65-8.54 (m, 1H), 8.38 and 8.33 (s, total 1H), 8.19-8.05 (m, 2H), 7.07-6.95 (m, 2H), 6.90-6.82 (m, 1H), 5.76 and 5.23 (s, total 2H), 4.99-4.92 and 4.76-4.68 (m, total 1H), 3.54-3.45 (m, 1H), 3.43-3.37 and 3.28-3.21 (m, total 1H), 2.91 and 2.65 (s, total 3H), 2.16 (s, 3H), 2.03-1.92 (overlapping s and m, total 4H), 1.15-1.08 (m, 2H), 1.01 and 0.95 (d, total 3H), 0.83-0.77 (m, 2H).

### Example 21

### N-(3-(6-Amino-5-(3-(N-methylacrylamido)propoxy)pyrimidin-4-yl)-5-fluoro-2-methylphenyl)-4-cyclopropyl-2-fluorobenzamide

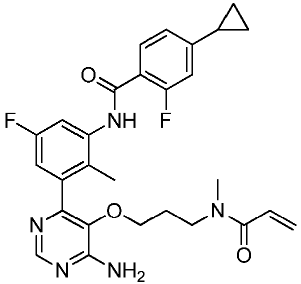

The title compound was prepared according to **Scheme 2** following a procedure analogous to **Example 6** replacing *N*-Boc-*N*-methyl-2-hydroxyethylamine with *N*-Boc-*N*-methyl-3-hydroxypropylamine in step 1.

UPLC-MS: MS (ESI): [M+H]⁺ 522.4, rt = 0.95 min.

### Example 22

### (S)-N-(3-(5-((1-Acryloylpyrrolidin-2-yl)methoxy)-6-aminopyrimidin-4-yl)-5-fluoro-2-methylphenyl)-4-cyclopropyl-2-fluorobenzamide

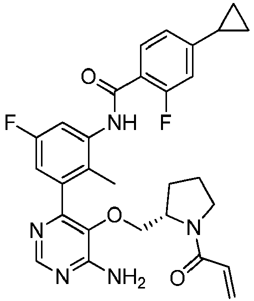

The title compound was prepared according to **Scheme 2** following a procedure analogous to **Example 6** replacing *N*-Boc-*N*-methyl-2-hydroxyethylamine with (S)-N-Boc-2-(hydroxymethyl)pyrrolidine in step 1.

UPLC-MS: MS (ESI): [M+H]⁺ 534.3, rt = 1.00 min.

### Example 23

### (S)-N-(3-(6-Amino-5-((1-(but-2-ynoyl)pyrrolidin-2-yl)methoxy)pyrimidin-4-yl)-5-fluoro-2-methylphenyl)-4-cyclopropyl-2-fluorobenzamide

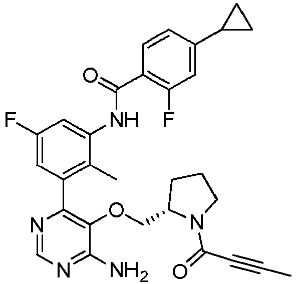

The title compound was prepared according to **Scheme 2** following a procedure analogous to **Example 6** replacing *N*-Boc-*N*-methyl-2-hydroxyethylamine with (*S*)-*N*-Boc-2-(hydroxymethyl)pyrrolidine in step 1, and replacing acrylic acid with 2-butynoic acid in step 4.

UPLC-MS: MS (ESI): [M+H]⁺ 546.3, rt = 1.02 min.

### Example 24

### (S)-2-(3-(5-((1-Acryloylpyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one

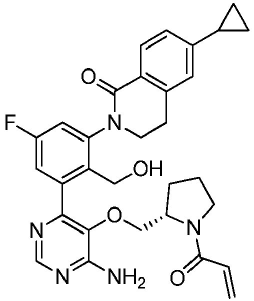

### (1) 2-(6-Cyclopropyl-1-oxo-3,4-dihydroisoquinolin-2(1H)-yl)-4-fluoro-6-(4,4,5,5-tetramethyl-1,3,2-dioxaborolan-2-yl)benzyl acetate, INT 23

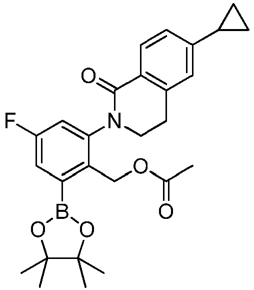

**INT 23** was prepared following a procedure analogous to **INT 2** replacing 1-bromo-5-fluoro-2-methyl-3-nitro-benzene with acetic acid 2-bromo-6-(6-cyclopropyl-1-oxo-3,4-dihydro-1*H*-isoquinolin-2-yl)-benzyl ester (WO2010/000633).

UPLC-MS: MS (ESI): [M+H]⁺ 480.4, rt = 1.36 min. ¹H NMR (DMSO-*d*₆): δ (ppm) 7.76 (s, 1H), 7.49-7.46 (m, 1H), 7.38-7.35 (m, 1H), 7.10 (d, 1H), 7.06 (s, 1H), 5.24 (d, 1H), 4.93 (d, 1H), 4.07-3.98 (m, 1H), 3.65-3.58 (m, 1H), 3.15-2.99 (m, 2H), 2.04-1.96 (m, 1H), 1.91 (s, 3H), 1.31 (s, 12H), 1.05-1.00 (m, 2H), 0.80-0.75 (m, 2H).

### (2) (S)-tert-Butyl 2-(((4-amino-6-chloropyrimidin-5-yl)oxy)methyl)pyrrolidine-1-carboxylate, INT 24

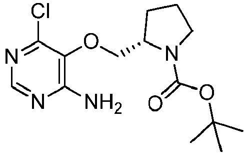

**INT 24** was prepared according to **Scheme 2** following a procedure analogous to step 1 of **Example 6** replacing *N*-Boc-*N*-methyl-2-hydroxyethylamine with (*S*)-*N*-Boc-2-(hydroxymethyl)pyrrolidine. UPLC-MS: MS (ESI): [M+H]⁺ 329.2, rt = 0.97 min.

### (3) (S)-tert-Butyl 2-(((4-amino-6-(3-(6-cyclopropyl-1-oxo-3,4-dihydroisoquinolin-2(1H)-yl)-5-fluoro-2-(hydroxymethyl)phenyl)pyrimidin-5-yl)oxy)methyl)pyrrolidine-1-carboxylate, INT 25

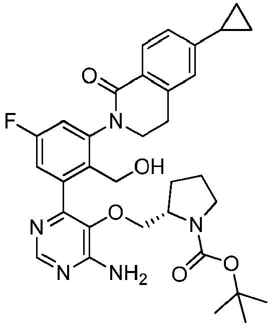

To a solution of **INT 24** (content 66%, 200 mg, 0.40 mmol) in DME (3.0 mL) and water (0.43 mL) was added **INT 23** (212 mg, 0.44 mmol) followed by aqueous sodium carbonate solution (1 M, 1.20 mL, 1.20 mmol). The mixture was degassed with argon for 10 min, then bis(triphenylphosphine)-palladium(II) dichloride (14 mg, 0.020 mmol) was added. The reaction mixture was stirred at 90 °C for 6 hr. After cooling to RT, aqueous NaOH solution (2 M, 2.0 mL, 4.00 mmol) was added and the mixture was stirred at RT for 20 min. The mixture was diluted with saturated aqueous sodium hydrogen carbonate solution and extracted with EtOAc. The organic layer was washed with water and brine, dried over magnesium sulfate, filtered and concentrated. The residue was purified by flash chromatography (silica; DCM/EtOAc gradient, 0-100%) to afford **INT 25** as a beige solid.

UPLC-MS: MS (ESI): [M+H]⁺ 604.5, rt = 1.20 min. ¹H NMR (DMSO-*d*₆): δ (ppm) 8.21 (s, 1H), 7.79 (d, 1H), 7.40 (d, 1H), 7.21 (d, 1H), 7.11 (d, 1H), 7.07 (s, 1H), 7.04-6.87 (s, br, 2H), 4.86-4.66 (m, 1H), 4.31 (m, 2H), 4.03-3.93 (m, 1H), 3.81-3.70 (m, 2H), 3.64-3.53 (m, 2H), 3.35-3.00 (m, 4H), 2.03-1.97 (m, 1H), 1.64-1.44 (m, 4H), 1.40-1.24 (m, 9H), 1.06-1.01 (m, 2H), 0.79-0.76 (m, 2H).

### (4) (S)-2-(3-(6-Amino-5-(pyrrolidin-2-ylmethoxy)pyrimidin-4-yl)-5-fluoro-2-(hydroxymethyl)phenyl)-6-cyclopropyl-3,4-dihydroisoquinolin-1(2H)-one, INT 26

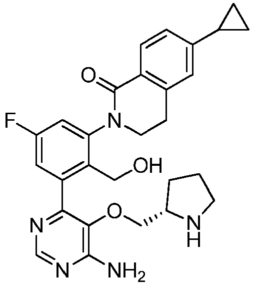

**INT 26** was prepared according to **Scheme 2** following a procedure analogous to step 3 of **Example 6** replacing **INT 9** with **INT 25.**

UPLC-MS: MS (ESI): [M+H]⁺ 504.4, rt = 0.75 min.

### (5) (S)-2-(3-(5-((1-Acryloylpyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one

The title compound was prepared according to **Scheme 2** following a procedure analogous to step 4 of **Example 6** replacing **INT 10** with **INT 26.**

UPLC-MS: MS (ESI): [M+H]⁺ 558.4, rt = 0.98 min.

### Example 25

### N-(2-((4-Amino-6-(3-(6-cyclopropyl-1-oxo-3,4-dihydroisoquinolin-2(1H)-yl)-5-fluoro-2-(hydroxymethyl)phenyl)pyrimidin-5-yl)oxy)ethyl)-N-methylacrylamide

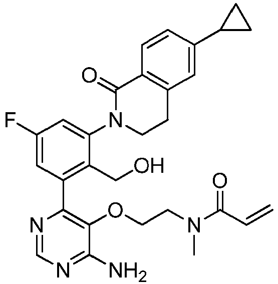

### (1) 2-(3-(6-Amino-5-(2-(methylamino)ethoxy)pyrimidin-4-yl)-5-fluoro-2-(hydroxymethyl)-phenyl)-6-cyclopropyl-3,4-dihydroisoquinolin-1(2H)-one, INT 27

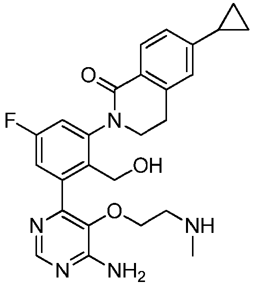

**INT 27** was prepared according to **Scheme 2** following a procedure analogous to **INT 26** replacing **INT 24** with **INT 8** in step 3, and purifying the TFA salt over a SPE cartridge (PL-HCO3 MP resin) to afford **INT 27** as the free amine in step 4.

UPLC-MS: MS (ESI): [M+H]⁺ 478.3, rt = 0.62 min.

### (2) N-(2-((4-Amino-6-(3-(6-cyclopropyl-1-oxo-3,4-dihydroisoquinolin-2(1H)-yl)-5-fluoro-2-(hydroxymethyl)phenyl)pyrimidin-5-yl)oxy)ethyl)-N-methylacrylamide

To a solution of **INT 27** (free amine, 130 mg, 0.272 mmol) and DIPEA (0.238 ml, 1.361 mmol) in DCM (9.0 mL) at -20 °C was added a solution of acryloyl chloride (24.64 mg, 0.272 mmol) in DCM (0.6 mL). The reaction mixture was stirred at -20 °C for 10 min. The mixture was diluted with DCM and poured into brine. The aqueous layer was back-extracted with DCM. The combined organic layers were dried over sodium sulfate and filtered. The filtrate was directly loaded onto a silica cartridge and purified by flash chromatography (silica; heptane/acetone gradient, 0-80%) to afford a white solid. The residue was triturated in acetonitrile, filtered off, and rinsed with acetonitrile. The solid was dried in vacuum to afford **Example 25** as a white solid.

UPLC-MS: MS (ESI): [M+H]⁺ 530.5, rt = 0.89 min. 1H NMR (DMSO-d6): δ (ppm) rotamers 8.23-8.16 (m, 1H), 7.83-7.77 (m, 1H), 7.43-7.32 (m, 1H), 7.20-7.04 (m, 5H), 6.70-6.60 (m, 1H), 6.11-6.00 (m, 1H), 5.69-5.53 (m, 1H), 4.77-4.61 (m, 1H), 4.37-4.24 (m, 2H), 4.05-3.93 (m, 1H), 3.83-3.73 (m, 1H), 3.68-3.55 (m, 2H), 3.54-3.44 (m, 1H), 3.27-3.15 (m, 2H), 3.09-2.99 (m, 1H), 2.89-2.55 (m, 3H), 2.05-1.95 (m, 1H), 1.08-0.99 (m, 2H), 0.81-0.74 (m, 2H).

### Example 26

### N-(3-(5-(((2S,4R)-1-Acryloyl-4-methoxypyrrolidin-2-yl)methoxy)-6-aminopyrimidin-4-yl)-5-fluoro-2-methylphenyl)-4-cyclopropyl-2-fluorobenzamide

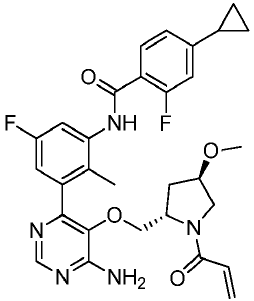

### (1) (2S,4R)-N-Boc-4-methoxypyrrolidine-2-carboxylic acid, INT 28

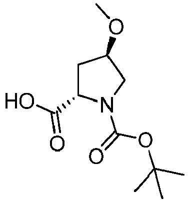

**INT 28** was prepared following a procedure analogous to WO2002/102790.

MS (ESI): [M-H]⁻ 244.2. ¹H NMR (DMSO-*d*₆): δ (ppm) rotamers 4.05-3.97 (m, 1H), 3.95-3.87 (m, 1H), 3.45-3.30 (m, 2H), 3.20 (s, 3H), 2.25-2.11 (m, 1H), 1.99-1.91 (m, 1H), 1.39 and 1.33 (s, total 9H).

### (2) (2S,4R)-N-Boc-2-(hydroxymethyl)-4-methoxypyrrolidine, INT 29

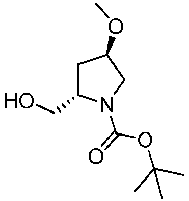

To solution of **INT 28** (5.00 g, 20.39 mmol) in THF (100 mL) at 0 °C was added borane tetrahydrofuran complex solution (1 M in THF, 30.6 mL, 30.6 mmol) dropwise. The reaction mixture was stirred at RT for 6 hr. The mixture was cooled to 0 °C and water (80 mL) was added dropwise. The resulting mixture was stirred at 0 °C for 1 hr, then diluted with EtOAc. The organic layer was washed with aqueous 10% citric acid solution, saturated aqueous sodium hydrogen carbonate solution and brine, dried over magnesium sulfate, filtered and concentrated. The residue was dried in vacuum to afford crude **INT 29** as a colorless liquid.

MS (ESI): [M+H-tBu]⁺ 176.1. ¹H NMR (DMSO-*d*₆): δ (ppm) 4.69 (t, 1H), 3.94-3.88 (m, 1H), 3.73 (s, v br, 1H), 3.48-3.36 (m, 3H), 3.31-3.22 (m, 1H), 3.20 (s, 3H), 2.08-1.87 (m, 2H), 1.40 (s, 9H).

### (3) (2S,4R)-tert-Butyl 2-(((4-amino-6-chloropyrimidin-5-yl)oxy)methyl)-4-methoxypyrrolidine-1-carboxylate, INT 30

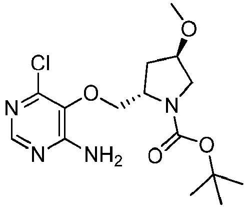

**INT 30** was prepared according to **Scheme 2** following a procedure analogous to step 1 of **Example 6** replacing *N*-Boc-*N*-methyl-2-hydroxyethylamine with **INT 29**.

UPLC-MS: MS (ESI): [M+H]⁺ 359.3, rt = 0.91 min.

### (4) N-(3-(5-(((2S,4R)-1-Acryloyl-4-methoxypyrrolidin-2-yl)methoxy)-6-aminopyrimidin-4-yl)-5-fluoro-2-methylphenyl)-4-cyclopropyl-2-fluorobenzamide

The title compound was prepared according to **Scheme 2** following a procedure analogous to **Example 6** replacing **INT 8** with **INT 30** in step 2.

UPLC-MS: MS (ESI): [M+H]⁺ 564.4, rt = 0.98 min.

### Example 27

### N-(3-(6-Amino-5-(((2S,4R)-1-(but-2-ynoyl)-4-methoxypyrrolidin-2-yl)methoxy)pyrimidin-4-yl)-5-fluoro-2-methylphenyl)-4-cyclopropyl-2-fluorobenzamide

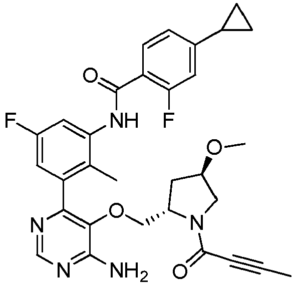

The title compound was prepared according to **Scheme 2** following a procedure analogous to **Example 6** replacing *N*-Boc-*N*-methyl-2-hydroxyethylamine with **INT 29** in step 1, and replacing acrylic acid with 2-butynoic acid in step 4.

UPLC-MS: MS (ESI): [M+H]⁺ 576.4, rt = 1.01 min.

### Example 28

### 2-(3-(5-(((2S,4R)-1-Acryloyl-4-methoxypyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one

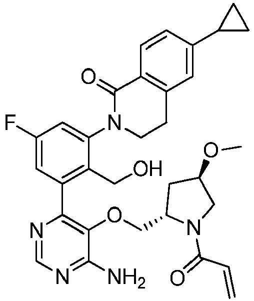

The title compound was prepared according to **Scheme 2** following a procedure analogous to **Example 24** replacing **INT 24** with **INT 30** in step 3.

UPLC-MS: MS (ESI): [M+H]⁺ 588.5, rt = 0.95 min.

### Example 29

### N-(3-(5-(((2S,4S)-1-Acryloyl-4-methoxypyrrolidin-2-yl)methoxy)-6-aminopyrimidin-4-yl)-5-fluoro-2-methylphenyl)-4-cyclopropyl-2-fluorobenzamide

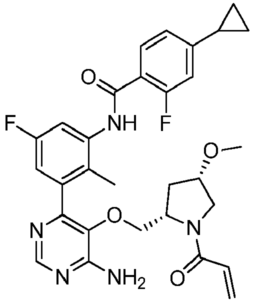

### (1) (2S,4S)-Methyl N-Boc-4-methoxypyrrolidine-2-carboxylate, INT 31

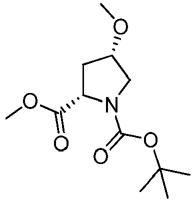

To a solution of (2*S*,4*S*)-methyl *N*-Boc-4-hydroxypyrrolidine-2-carboxylate (3.00 g, 12.23 mmol) in acetonitrile (60 mL) was added silver oxide (2.83 g, 12.23 mmol) followed by iodomethane (15.0 mL, 240.95 mmol). The reaction mixture was stirred at 85 °C for 4 hr. More iodomethane (5.0 mL, 80.32 mmol) was added and the mixture was stirred at 85 °C for an additional 5 hr. The mixture was filtered over a pad of Celite. The filtrated was diluted with diethyl ether and washed with saturated aqueous sodium hydrogen carbonate solution. The aqueous layer was back-extracted with diethyl ether. The combined organic layers were washed with brine, dried over magnesium sulfate, filtered and concentrated to afford crude **INT 31** as a pale yellow oil.

MS (ESI): [M+H]⁺ 260.3. ¹H NMR (DMSO-*d*₆): δ (ppm) rotamers 4.30-4.23 (m, 1H), 3.95-3.91 (m, 1H), 3.64 and 3.61 (s, total 3H), 3.55-3.50 (m, 1H), 3.27-3.21 (m, 1H), 3.17 and 3.16 (s, total 3H), 2.42-2.28 (m, 1H), 2.06-1.97 (m, 1H), 1.41 and 1.34 (s, total 9H).

### (2) (2S,4S)-N-Boc-2-(hydroxymethyl)-4-methoxypyrrolidine, INT 32

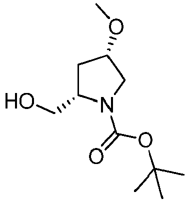

To a solution of **INT 31** (3.10 g, 11.96 mmol) in THF (120 mL) at 0 °C was added lithium borohydride solution (2 M in THF, 11.96 mL, 23.91 mmol). The reaction mixture was stirred at RT overnight. The mixture was cooled to 0 °C and poured onto ice water. The mixture was stirred for 15 min at RT, then extracted with diethyl ether. The aqueous layer was back-extracted with diethyl ether. The combined organic layers were washed with brine, dried over magnesium sulfate, filtered and concentrated to afford crude **INT 32** as a colorless oil.

MS (ESI): [M+H]⁺ 232.3. ¹H NMR (DMSO-*d*₆): δ (ppm) 4.64 (t, 1H), 3.87 (s, 1H), 3.68-3.44 (m, 3H), 3.32-3.26 (m, 1H), 3.21 (s, 3H), 3.18-3.15 (m, 1H), 2.04-1.97 (m, 1H), 1.42-1.34 (m, 1H), 1.40 (s, 9H).

### (3) N-(3-(5-(((2S,4S)-1-Acryloyl-4-methoxypyrrolidin-2-yl)methoxy)-6-aminopyrimidin-4-yl)-5-fluoro-2-methylphenyl)-4-cyclopropyl-2-fluorobenzamide

The title compound was prepared according to **Scheme 2** following a procedure analogous to **Example 6** replacing *N*-Boc-*N*-methyl-2-hydroxyethylamine with **INT 32** in step 1.

UPLC-MS: MS (ESI): [M+H]⁺ 564.4, rt = 0.99 min.

### Example 30

### N-(3-(6-Amino-5-(((2S,4S)-1-(but-2-ynoyl)-4-methoxypyrrolidin-2-yl)methoxy)pyrimidin-4-yl)-5-fluoro-2-methylphenyl)-4-cyclopropyl-2-fluorobenzamide

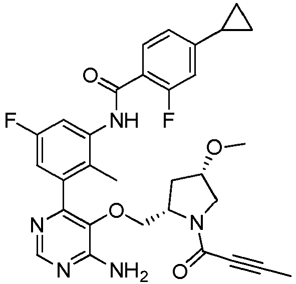

The title compound was prepared according to **Scheme 2** following a procedure analogous to **Example 6** replacing *N*-Boc-*N*-methyl-2-hydroxyethylamine with **INT 32** in step 1, and replacing acrylic acid with 2-butynoic acid in step 4.

UPLC-MS: MS (ESI): [M+H]⁺ 576.4, rt = 1.02 min.

### Example 31

### N-(3-(5-(((2S,4R)-1-Acryloyl-4-fluoropyrrolidin-2-yl)methoxy)-6-aminopyrimidin-4-yl)-5-fluoro-2-methylphenyl)-4-cyclopropyl-2-fluorobenzamide

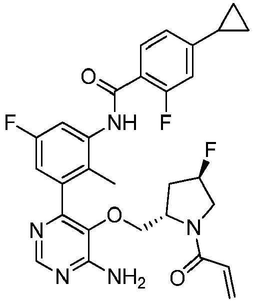

### (1) (2S,4R)-N-Boc-4-fluoropyrrolidine-2-carboxylic acid, INT 33

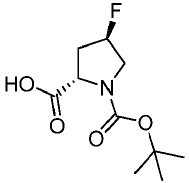

A solution of (2S,4*R*) methyl *N*-Boc-4-hydroxypyrrolidine-2-carboxylate (250 g, 1.02 mol), triphenylphosphine (401 g, 1.53 mmol) and benzoic acid (187 g, 1.53 mol) in THF (3.50 L) was cooled to reach an internal temperature of -4 °C, then a diethyl azodicarboxylate solution (40% in toluene, 625 mL, 1.43 mmol) in THF (1.50 L) was added within 1 hr. The reaction mixture was warmed to RT and stirred at RT overnight. The mixture was concentrated. The residue was taken up in diethyl ether (2.5 L) and the mixture was refluxed for 1 hr. The suspension was cooled to 0 °C, the white solid was filtered off, and washed with cold ethanol. The filtrate was concentrated. The residue was dissolved in a 4:1 mixture of warm hexane/EtOAc (1.5 L) and stirred at RT for 1 hr. The mixture was cooled to 10 °C and treated with hexane (250 mL). The mixture was stirred at RT for 30 min and a precipitate was formed. The solid was filtered off and washed with cold hexane (150 mL). The filtrate was concentrated. The residue was purified by flash chromatography (silica; hexane/EtOAc 4:1) to afford (2*S*,4*S*)-2-methyl N-Boc-4-(benzoyloxy)pyrrolidine-2-carboxylate as a white solid.

To a solution of (2*S*,4*S*)-2-methyl *N*-Boc-4-(benzoyloxy)pyrrolidine-2-carboxylate (248 g, 0.71 mol) in MeOH (4.5 L) was added sodium carbonate (98 g, 0.92 mol) followed by more MeOH (0.5 L). The reaction mixture was stirred at RT for 4 hr. The mixture was filtered, and the filtrated was concentrated to a volume of approximately 1 L. The solution was diluted with EtOAc (5.0 L), cooled to 5 °C and washed with water. The aqueous layer was back-extracted with EtOAc (2x). The combined organic layers were washed with brine and a 1:1 mixture of brine and water, dried over sodium sulfate, filtered and concentrated. The residue was crystallized from DCM/hexane to afford (2*S*,4*S*)-2-methyl *N*-Boc-4-hydroxy-pyrrolidine-2-carboxylate as a white solid.

To a solution of (2*S*,4*S*)-2-methyl *N*-Boc-4-hydroxy-pyrrolidine-2-carboxylate (270 g, 1.10 mol) in DCM (2.6 L) at -80 °C was added (diethylamino)sulfur trifluoride (567 mL, 4.29 mol) dropwise. The reaction mixture was stirred at RT overnight. The mixture was cooled to -78 °C and then added to a saturated aquous sodium hydrogen carbonate solution cooled to -10 °C. During the addition the inner temperature was kept below 5 °C. The mixture was then stirred at 0 °C for 30 min. The layers were separted, the aqueous layer was back-extracted with DCM. The combined organic layers were washed with saturated aqueous sodium hydrogen carbonate solution, dried over sodium sulfate, filtered and concentrated. The residue was purified by flash chromatography (silica; hexane/EtOAc gradient, 10-40%) to afford (2*S*,4*R*)-2-methyl *N*-Boc-4-fluoro-pyrrolidine-2-carboxylate as a yellow oil.

To a solution of (2*S*,4*R*)-2-methyl *N*-Boc-4-fluoro-pyrrolidine-2-carboxylate (13.0 g, 52.58 mmol) in dioxane (270 mL) at 15 °C was added a solution of sodium hydroxide (4.2 g, 105.00 mmol) in water (30 mL) dropwise. The mixture was cooled to 7 °C and the slurry was stirred at 7 °C overnight. Acetic acid (80 mL) was added and the mixture was diluted with DCM. The layers were separated, the aqueous layer was back-extracted with DCM. The combined organic layers were washed with brine, dried over sodium sulfate, filtered and concentrated. The residue was crystallized from diethyl ether/hexane to afford **INT 33** as a white solid.

MS (ESI): [M-H]⁻ 232.2. ¹H NMR (DMSO-*d*₆): δ (ppm) rotamers 12.72 (s, br, 1H), 5.40-5.21 (m, 1H), 4.22-4.13 (m, 1H), 3.72-3.58 (m, 1H), 3.58-3.36 (m, 1H), 2.60-2.44 (m, 1H, overlapping with solvent peak), 2.19-1.97 (m, 1H), 1.41 and 1.36 (s, total 9 H).

### (2) (2S,4R)-N-Boc-2-(hydroxymethyl)-4-fluoropyrrolidine, INT 34

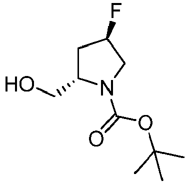

To a solution of **INT 33** (5.00 g, 21.44 mmol) in THF (105 mL) at 0 °C was added borane tetrahydrofuran complex solution (1 M in THF, 32.2 mL, 32.20 mmol). The reaction mixture was stirred at RT for 3 hr. The mixture was cooled to 0 °C and water (100 mL) was added dropwise. The resulting mixture was stirred at 0 °C for 1 hr, then extracted with EtOAc. The organic layer was washed with aqueous 10% citric acid solution, saturated aqueous sodium hydrogen carbonate solution and brine, dried over magnesium sulfate, filtered and concentrated to afford crude **INT 34** as a yellow oil.

MS (ESI): [M+H-tBu]⁺ 164.2. ¹H NMR (DMSO-*d*₆): δ (ppm) 5.23 (d, 1H), 4.74 (t, 1H), 3.84 (m, 1H), 3.74-3.62 (m, 1H), 3.57-3.44 (m, 2H), 3.41-3.23 (m, 1H), 2.22-2.05 (m, 2H), 1.41 (s, 9H).

### (3) N-(3-(5-(((2S,4R)-1-Acryloyl-4-fluoropyrrolidin-2-yl)methoxy)-6-aminopyrimidin-4-yl)-5-fluoro-2-methylphenyl)-4-cyclopropyl-2-fluorobenzamide

The title compound was prepared according to **Scheme 2** following a procedure analogous to **Example 6** replacing *N*-Boc-*N*-methyl-2-hydroxyethylamine with **INT 34** in step 1.

UPLC-MS: MS (ESI): [M+H]⁺ 552.5, rt = 1.00 min.

### Example 32

### N-(3-(6-Amino-5-(((2S,4R)-1-(but-2-ynoyl)-4-fluoropyrrolidin-2-yl)methoxy)pyrimidin-4-yl)-5-fluoro-2-methylphenyl)-4-cyclopropyl-2-fluorobenzamide

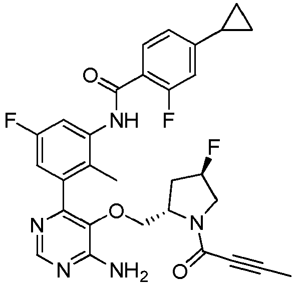

The title compound was prepared according to **Scheme 2** following a procedure analogous to **Example 6** replacing *N*-Boc-*N*-methyl-2-hydroxyethylamine with **INT 34** in step 1, and replacing acrylic acid with 2-butynoic acid in step 4.

UPLC-MS: MS (ESI): [M+H]⁺ 564.5, rt = 1.03 min.

### Example 33

### (S)-N-(3-(5-((1-Acryloylazetidin-2-yl)methoxy)-6-aminopyrimidin-4-yl)-5-fluoro-2-methylphenyl)-4-cyclopropyl-2-fluorobenzamide

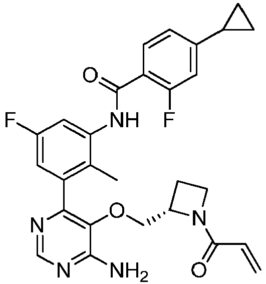

### (1) (S)-N-Boc-2-(hydroxymethyl)azetidine, INT 35

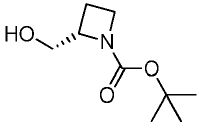

**INT 35** was prepared according to **Scheme 2** following a procedure analogous to step 2 of **Example 26** replacing **INT 28** with (*S*)-*N*-Boc-azetidine-2-carboxylic acid.

MS (ESI): [M+H]⁺ 188.1.

### (2) (S)-N-(3-(5-((1-Acryloylazetidin-2-yl)methoxy)-6-aminopyrimidin-4-yl)-5-fluoro-2-methylphenyl)-4-cyclopropyl-2-fluorobenzamide

The title compound was prepared according to **Scheme 2** following a procedure analogous to **Example 6** replacing *N*-Boc-*N*-methyl-2-hydroxyethylamine with **INT 35** in step 1.

UPLC-MS: MS (ESI): [M+H]⁺ 520.2, rt = 0.96 min.

### Example 34

### (S)-N-(3-(6-Amino-5-((1-propioloylazetidin-2-yl)methoxy)pyrimidin-4-yl)-5-fluoro-2-methylphenyl)-4-cyclopropyl-2-fluorobenzamide

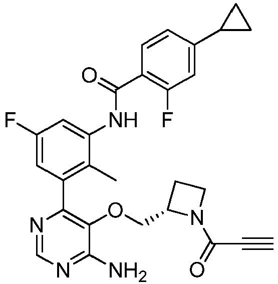

The title compound was prepared according to **Scheme 2** following a procedure analogous to **Example 6** replacing *N*-Boc-*N*-methyl-2-hydroxyethylamine with **INT 35** in step 1, and replacing acrylic acid with propiolic acid in step 4.

UPLC-MS (ESI): [M+H]⁺ 518.3, rt = 0.96 min.

### Example 35

### (S)-2-(3-(5-((1-Acryloylazetidin-2-yl)methoxy)-6-aminopyrimidin-4-yl)-5-fluoro-2-(hydroxymethyl)phenyl)-6-cyclopropyl-3,4-dihydroisoquinolin-1(2H)-one

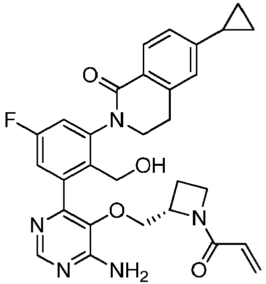

### (1) (S)-tert-Butyl 2-(((4-amino-6-chloropyrimidin-5-yl)oxy)methyl)azetidine-1-carboxylate, INT 36

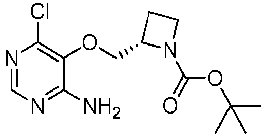

**INT 36** was prepared according to **Scheme 2** following a procedure analogous to step 1 of **Example 6** replacing *N*-Boc-*N*-methyl-hydroxyethylamine with **INT 35.**

UPLC-MS: MS (ESI): [M+H]⁺ 315.1, rt = 0.91 min.

### (2) (S)-2-(3-(5-((1-Acryloylazetidin-2-yl)methoxy)-6-aminopyrimidin-4-yl)-5-fluoro-2-(hydroxymethyl)phenyl)-6-cyclopropyl-3,4-dihydroisoquinolin-1(2H)-one

The title compound was prepared according to **Scheme 2** following a procedure analogous to **Example 24** replacing **INT 24** with **INT 36** in step 3.

UPLC-MS: MS (ESI): [M+H]⁺ 544.4, rt = 0.94 min.

### Example 36

### (R)-N-(3-(5-((1-Acryloylazetidin-2-yl)methoxy)-6-aminopyrimidin-4-yl)-5-fluoro-2-methylphenyl)-4-cyclopropyl-2-fluorobenzamide

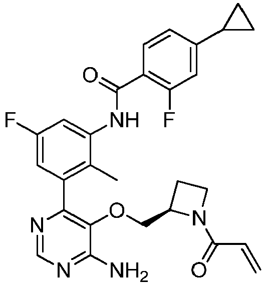

The title compound was prepared according to **Scheme 2** following a procedure analogous to **Example 33** replacing (*S*)-*N*-Boc-azetidine-2-carboxylic acid with (*R*)-*N*-Boc-azetidine-2-carboxylic acid in step 1.

UPLC-MS: MS (ESI): [M+H]⁺ 520.3, rt = 0.99 min.

### Example 37

### (R)-N-(3-(5-((1-Acryloylpiperidin-3-yl)methoxy)-6-aminopyrimidin-4-yl)-5-fluoro-2-methylphenyl)-4-cyclopropyl-2-fluorobenzamide

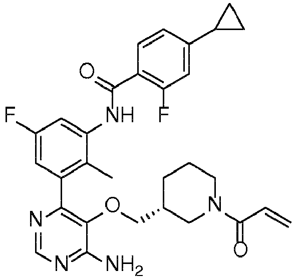

The title compound was prepared according to **Scheme 2** following a procedure analogous to **Example 6** replacing *N*-Boc-*N*-methyl-hydroxyethylamine with (*R*)-*N*-Boc-3-(hydroxylmethyl)piperidine in step 1. UPLC-MS: MS (ESI): [M+H]⁺ 548.5, rt = 1.02 min.

Alternatively, agents of the disclosure may be prepared by a reaction sequence involving deprotection e.g. with a Lewis acid of 4,6-dichloro-5-methoxypyrimidine 8 to yield 4,6-dichloro-5-hydroxyoxy-pyrimidine 9, followed by a Mitsunobu reaction of the pyrimidinol with an alcohol compound 2' using an appropriate azodicarboxylate, such as DIAD, and Smopex-301 or triphenylphosphine to yield intermediate 10, followed by a nucleophilic aromatic substitution e.g. with ammonia in water to yield the aminopyrimidine intermediate 3. Thereupon intermediate 3 is converted into a final compound of the disclosure, i.e. a compound 7, by the earlier described reaction sequences of scheme 1 and/or scheme 2, i.e. a Suzuki coupling with a boronic ester using an appropriate catalyst, such as bis(triphenylphosphine) palladium(II) dichloride, deprotection using an appropriate acid, such as TFA or HCl, followed by amide formation e.g. of the ammonium salt or the free amine with an acid and using an appropriate coupling reagent, such as T3P, and an appropriate base, such as DIPEA, or with an acid chloride using an appropriate base, such as DIPEA, as shown in **Scheme 3** below:

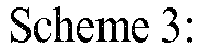

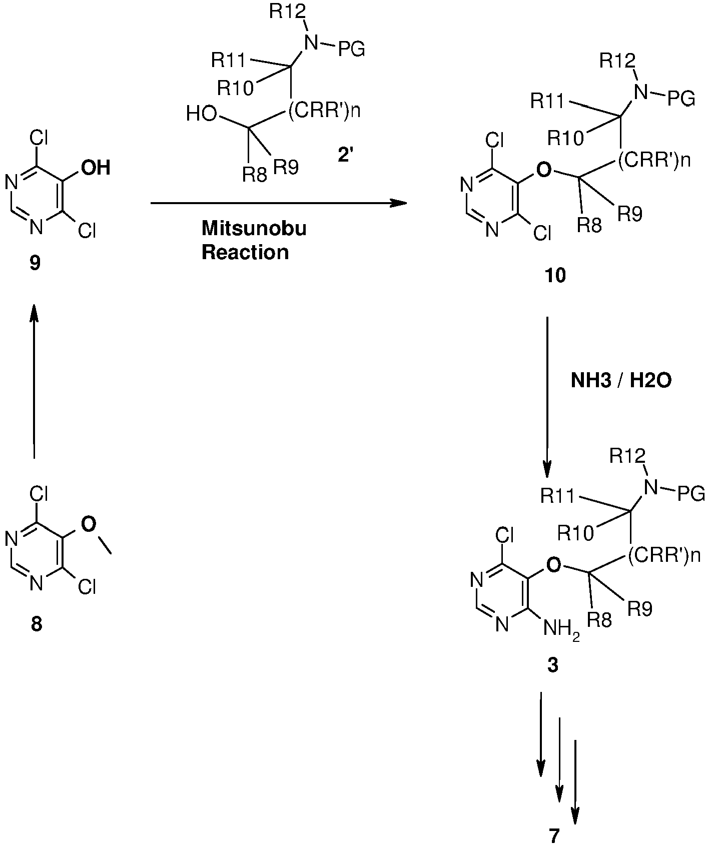

### Example 38

### N-(3-(5-(((2R,3S)-1-Acryloyl-3-methoxypyrrolidin-2-yl)methoxy)-6-aminopyrimidin-4-yl)-5-fluoro-2-methylphenyl)-4-cyclopropyl-2-fluorobenzamide

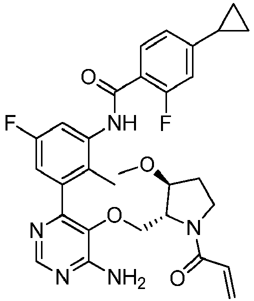

### (1) 4,6-Dichloropyrimidin-5-ol, INT 37

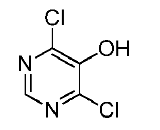

To a solution of 4,6-dichloro-5-methoxypyrimidine (5.00 g, 27.93 mmol) in DCE (80 mL) at 0 °C was added aluminum chloride (5.48 g, 41.10 mmol) in one portion. The reaction mixture was stirred vigorously at 50 °C for 6 hr. The mixture was cooled to 0 °C and aqueous HCl solution (1 M, 40 mL) followed by MeOH (10 mL) were added slowly. The mixture was stirred vigorously at RT for 10 min, then diluted with water and extracted with a mixture of DCM/MeOH (10:1, 2 x 100 mL) and EtOAc (1 x 100 mL). The combined organic layers were dried over sodium sulfate, filtered and concentrated to afford crude **INT 37** as beige solid.

UPLC-MS: MS (ESI): [M-H]⁻ 163.0, rt = 0.45 min. ¹H NMR (DMSO-*d*₆): δ (ppm) 11.71 (s, br, 1H), 8.39 (s, 1H).

### (2) (2S,3S) 2-Methyl N-Boc-3-hydroxypyrrolidine-2-carboxylate, INT 38

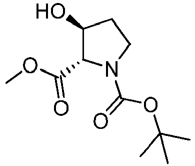

To a solution of (2*S*,3*S*)-N-Boc-3-hydroxypyrrolidine-2-carboxylic acid (4.10 g, 17.73 mmol) in DMF (100 mL) at 0 °C was added potassium carbonate (4.00 g, 28.94 mmol) followed by iodomethane (1.3 mL, 20.79 mmol). The reaction mixture was warmed to RT and stirred at RT for 4 hr, then at 90 °C for 1 hr. After cooling to RT iodomethane (0.70 mL, 11.19 mmol) was added and the reaction mixture was stirred at RT overnight. The mixture was diluted with brine and extracted with EtOAc (3x). The combined organic layers were dried over sodium sulfate, filtered and concentrated. The residue was purified by flash chromatography (silica; cyclohexane/EtOAc gradient, 0-50%) to afford **INT 38** as a colorless oil.

MS (ESI): [M+H]⁺ 246.2. ¹H NMR (CDCl₃): δ (ppm) rotamers 4.42 (s, br, 1H), 4.29 and 4.18 (s, total 1H), 3.74 (s, 3H), 3.66-3.53 (m, 3H), 2.13-2.03 (m, 1H), 1.97-1.88 (m, 1H), 1.46 and 1.41 (s, total 9H).

### (3) (2S,3S) 2-Methyl N-Boc-3-methoxypyrrolidine-2-carboxylate, INT 39

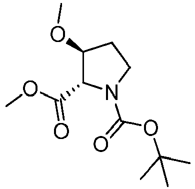

To a solution of **INT 38** (2.53 g, 10.33 mmol) in DMF (25.0 mL) was added iodomethane (3.2 mL, 51.60 mmol) followed by silver(I) oxide (7.18 g, 31.00 mmol). The reaction mixture was stirred at RT over the weekend. The mixture was diluted with EtOAc and filtered through a pad of Celite. The filtrate was washed with brine, aqueous 10% sodium thiosulfate solution and saturated aqueous sodium hydrogen carbonate solution. The organic layer was dried over sodium sulfate, filtered and concentrated to afford crude **INT 39** as a colorless oil.

¹H NMR (CDCl₃): δ (ppm) rotamers 4.41 and 4.26 (s, total 1H), 3.94-3.87 (m, br, 1H), 3.75 (s, 3H), 3.69-3.53 (m, 2H), 3.38 (s, 3H), 2.11-1.95 (m, 2H), 1.46 and 1.41 (s, total 9H).

### (4) (2R,3S)-N-Boc-2-hydroxymethyl-3-methoxy-pyrrolidine, INT 40

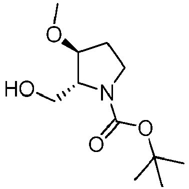

To a solution of **INT 39** (2.28 g, 8.81 mmol) in THF (25 mL) was added lithium chloride (1.12 g, 26.40 mmol) followed by sodium borohydride (1.00 g, 26.40 mmol). EtOH (50 mL) was added and the reaction mixture was stirred at RT for 4 hr. The mixture was cooled to 0 °C and water was added slowly. The mixture was extracted with EtOAc. The organic layer was washed with brine, dried over sodium sulfate, filtered and concentrated. The aqueous layer diluted with saturated aqueous ammonium chloride solution and back-extracted with EtOAc. The organic layer was washed with brine, dried over sodium sulfate, filtered and concentrated. The combined residues were purified by flash chromatography (silica; cyclohexane/EtOAc gradient, 15-100%) to afford **INT 40** as a colorless liquid. MS (ESI): [M+H]⁺ 232.2. ¹H NMR (CDCl₃): δ (ppm) rotamers 4.03-3.92 and 3.89-3.77 (m, br, total 2H), 3.72-3.55 (m, br, 2H), 3.52-3.30 (overlapping m, 2H and s, 3H), 2.01-1.92 (m, br, 2H), 1.47 (s, 9H).

### (5) (2R,3S)-tert-Butyl 2-(((4,6-dichloropyrimidin-5-yl)oxy)methyl)-3-methoxypyrrolidine-1-carboxylate, INT 41

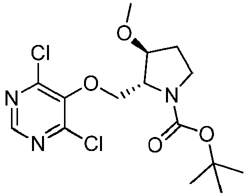

To a solution of **INT 37** (105 mg, 0.64 mmol) and **INT 40** (221 mg, 0.96 mmol) in THF (12 mL) was added triphenylphosphine (250 mg, 0.96 mmol) followed by the dropwise addition of DIAD (0.186 mL, 0.96 mmol). The reaction mixture was stirred at 60 °C overnight. The mixture was concentrated under reduced pressure. The residue was purified by flash chromatography (silica; cyclohexane/EtOAc gradient, 0-40%) to afford **INT 41** as a colorless residue.

UPLC-MS: MS (ESI): [M+H-tBu]⁺ 322.1, rt = 1.17 min. ¹H NMR (CDCl₃): δ (ppm) rotamers 8.57 and 8.54 (s, total 1H), 4.35-3.91 (m, 4H), 3.58-3.46 (m, 2H), 3.42 (s, 3H), 2.24-1.97 (m, 2H), 1.46 (s, 9H).

### (6) (2R,3S)-tert-Butyl 2-(((4-amino-6-chloropyrimidin-5-yl)oxy)methyl)-3-methoxypyrrolidine-1-carboxylate, INT 42

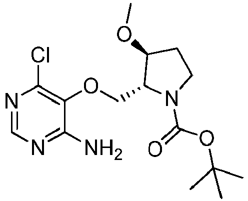

To a solution of **INT 41** (173 mg, 0.46 mmol) in 2-propanol (5.0 mL) was added aqueous 33% ammonium hydroxide solution (2.7 mL, 22.63 mmol). The reaction mixture was stirred in a scaled tube at 80 °C for 5 hr. The mixture was concentrated under reduced pressure. The residue was purified by flash chromatography (silica; DCM/EtOAc gradient, 0-50%) to afford **INT 42** as a colorless oil. UPLC-MS: MS (ESI): [M+H]⁺ 359.2, rt = 0.92 min. ¹H NMR (CDCl₃): δ (ppm) rotamers 8.08 (s, 1H), 6.22 and 5.78 (s, br, total 2H), 4.25-3.95 (m, br, 4H), 3.61-3.37 (m, 5H, including s, 3H, at δ 3.40), 2.18-1.95 (m, 2H), 1.46 (s, 9H).

### (7) N-(3-(5-(((2R,3S)-1-Acryloyl-3-methoxypyrrolidin-2-yl)methoxy)-6-aminopyrimidin-4-yl)-5 fluoro-2-methylphenyl)-4-cyclopropyl-2-fluorobenzamide, INT 43

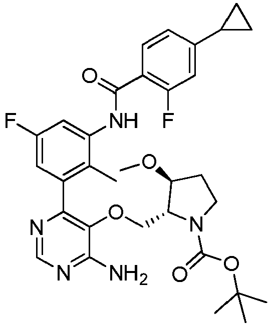

**INT 43** was prepared according to **Scheme 3** following a procedure analogous to step 2 of **Example 6** replacing **INT 8** with **INT 42.**

UPLC-MS: MS (ESI): [M+H]⁺ 610.5, rt = 1.21 min.

### (8) N-(3-(6-Amino-5-(((2R,3S)-3-methoxypyrrolidin-2-yl)methoxy)pyrimidin-4-yl)-5-fluoro-2-methylphenyl)-4-cyclopropyl-2-fluorobenzamide, INT 44

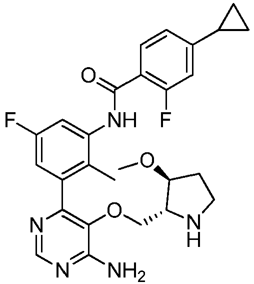

**INT 44** was prepared according to **Scheme 3** following a procedure analogous to step 3 of **Example 6** replacing **INT 9** with **INT 43** and purifying the crude by flash chromatography (silica; DCM/(MeOH with 2% aqueous ammonium hydroxide) gradient, 5-65%) to afford **INT 44** as the free amine. UPLC-MS: MS (ESI): [M+H]⁺ 510.3, rt = 0.77 min.

### (9) N-(3-(5-(((2R,3S)-1-Acryloyl-3-methoxypyrrolidin-2-yl)methoxy)-6-aminopyrimidin-4-yl)-5-fluoro-2-methylphenyl)-4-cyclopropyl-2-fluorobenzamide

The title compound was prepared according to **Scheme 3** following a procedure analogous to step 4 of **Example 6** replacing **INT 10** with **INT 44.**

UPLC-MS: MS (ESI): [M+H]⁺ 564.3, rt = 0.98 min. ¹H NMR (CDCl₃): δ (ppm) rotamers 8.60 and 8.55 (s, total 1H), 8.42 and 8.36 (s, total 1H), 8.20-8.13 (m, 1H), 8.13-8.04 (m, 1H), 7.07-7.01 (m, 1H), 6.96-6.83 (m, 2H), 6.47-6.32 (m, 2H), 5.79 (s, v br, 2H), 5.72-5.66 (m, 1H), 4.21-4.16 and 3.70-3.42 and 3.33-3.28 (m, total 6H), 3.26 and 3.20 (s, total 3H), 2.15 (s, 3H), 2.01-1.88 (m, 2H), 1.84-1.74 (m, 1H), 1.16-1.07 (m, 2H), 0.84-0.75 (m, 2H).

Alternatively, agents of the disclosure may be prepared by a reaction sequence involving alkylation of 4,6-dichloro-5-hydroxy-pyrimidine 9 with benzyl bromide using an appropriate base, such as potassium carbonate, followed by nucleophilic aromatic substitution with ammonium hydroxide to yield the aminopyrimidine 12, Suzuki coupling with a boronic ester 4 using an appropriate catalyst, such as bis(triphenylphosphine)-palladium(II) dichloride to yield the benzylated intermediate 13. Cleavage of the benzyl group, e.g. by hydrogenation, followed by a Mitsunobu reaction of the pyrimidinol with an alcohol of formula 2' using an appropriate azodicarboxylate, such as DIAD, and Smopex-301 or triphenylphosphine, deprotection using an appropriate acid, such as TFA or HCl, followed by amide formation of the ammonium salt or the free amine with an acid using an appropriate coupling reagent, such as T3P, and an appropriate base, such as DIPEA, or with an acid chloride using an appropriate base, such as DIPEA, to yield a final compound of the disclosure, i.e. a compound of formula 7, as shown in **Scheme 4** below:

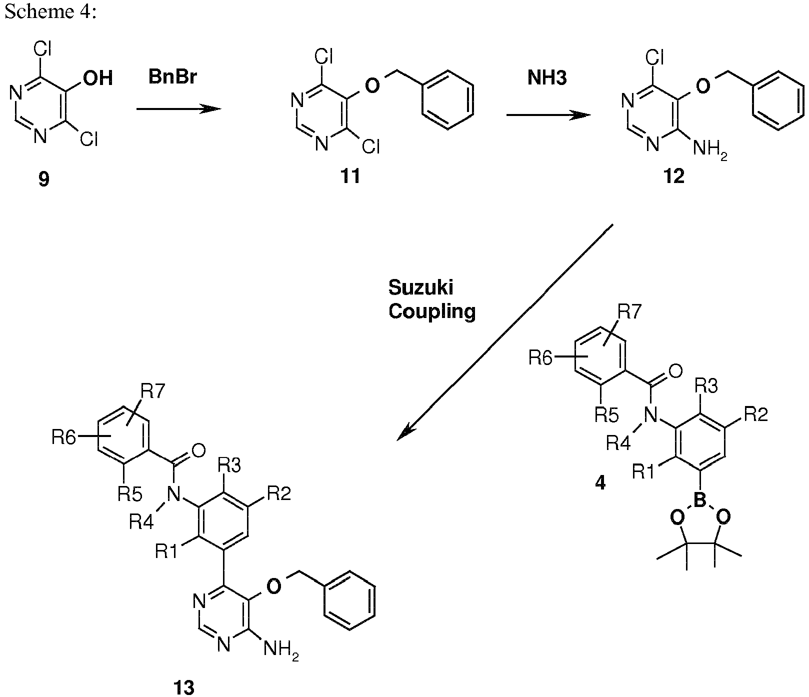

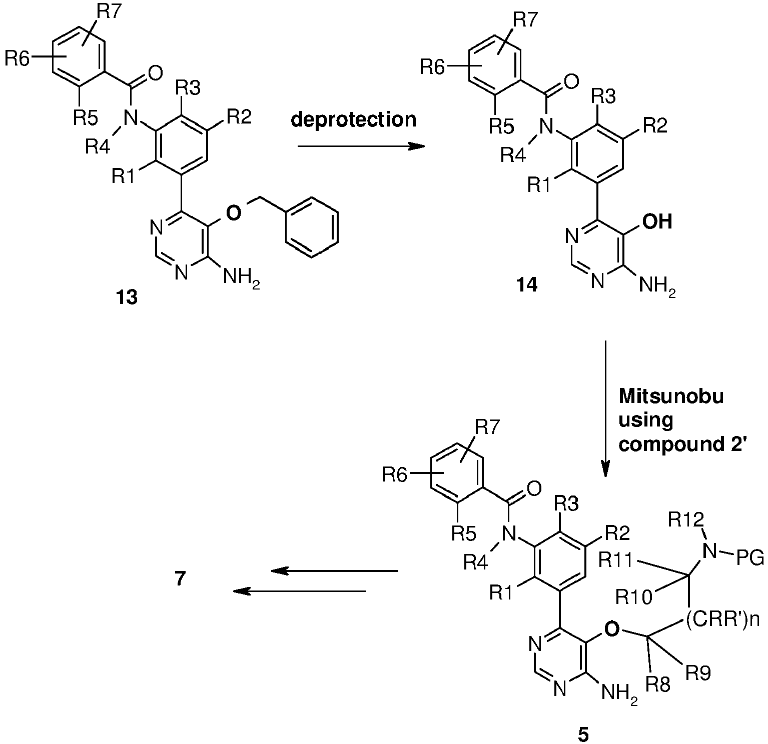

### Example 39

### N-(3-(5-(((2S,4R)-1-Acryloyl-4-cyanopyrrolidin-2-yl)methoxy)-6-aminopyrimidin-4-yl)-5-fluoro-2-methylphenyl)-4-cyclopropyl-2-fluorobenzamide

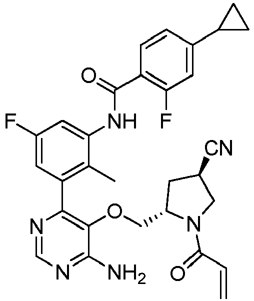

### (1) 5-(Benzyloxy)-4,6-dichloropyrimidine, INT 45

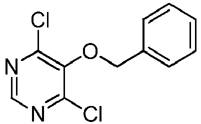

To a solution of **INT 37** (content 90%, 6.50 g, 35.50 mmol) in DMF (120 mL) was added benzyl bromide (8.42 mL, 70.90 mmol) followed by potassium carbonate (14.70 g, 106.36 mmol). The reaction mixture was stirred at 60 °C for 1 hr. The mixture was concentrated. The residue was partitioned between EtOAc and water. The organic layer was washed with water and brine, dried over magnesium sulfate, filtered and concentrated. The residue was purified by flash chromatography (silica; cyclohexane/EtOAc gradient, 0-10%) to afford **INT 45** as a colorless oil.

UPLC-MS: MS (ESI): [M+H]⁺ 255.1, rt = 1.15 min. ¹H NMR (DMSO-*d*₆): δ (ppm) 8.72 (s, 1H), 7.57-7.50 (m, 2H), 7.48-7.37 (m, 3H), 5.19 (s, 2H).

### (2) 5-(Benzyloxy)-6-chloropyrimidin-4-amine, INT 46

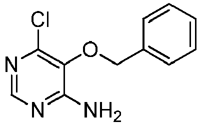

To a solution of **INT 45** (8.24 g, 32.30 mmol) in 2-propanol (100 mL) was added aqueous 26% ammonium hydroxide solution (93 mL, 614 mmol) in an autoclave. The reaction mixture was stirred at 80 °C for 12 hr. The mixture was concentrated. The residue was partitioned between EtOAc and water. The organic layer was washed with brine, dried over magnesium sulfate, filtered and concentrated to afford crude **INT 46** as a white solid.

UPLC-MS: MS (ESI): [M+H]⁺ 236.1, rt = 0.84 min. ¹H NMR (DMSO-*d*₆): δ (ppm) 7.98 (s, 1H), 7.58-7.51 (m, 2H), 7.43-7.32 (m, 3H), 7.25 (s, br, 2H), 4.95 (s, 2H).

### (3) N-(3-(6-Amino-5-(benzyloxy)pyrimidin-4-yl)-5-fluoro-2-methylphenyl)-4-cyclopropyl-2-fluorobenzamide, INT 47

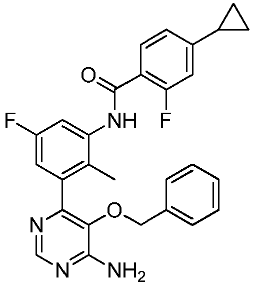

To a solution of **INT 46** (content 90%, 500 mg, 1.91 mmol) in DME (7.0 mL) and water (1.0 mL) was added **INT 5** (947 mg, 2.29 mmol) followed by aqueous sodium carbonate solution (2 M, 2.86 mL, 5.73 mmol). The mixture was degassed with argon for 10 min, then. bis(triphenyl-phosphine)palladium(II) dichloride (67.0 mg, 0.095 mmol) was added and the reaction mixture was stirred at 120 °C for 15 min in a microwave reactor. The mixture was partitioned between saturated aqueous sodium hydrogen carbonate solution and EtOAc. The organic layer was washed with water and brine, dried over magnesium sulfate, filtered and concentrated. The residue was purified by flash chromatography (silica; DCM/EtOAc gradient, 0-100%) to afford **INT 47** as a yellow solid.

UPLC-MS: MS (ESI): [M+H]⁺ 487.4, rt = 1.15 min. ¹H NMR (DMSO-*d*₆): δ (ppm) 9.80 (s, 1H), 8.20 (s, 1H), 7.66 (t, 1H), 7.54 (d, 1H), 7.26-7.18 (m, 3H), 7.11-6.91 (m, 7H), 4.55 (s, 2H), 2.08-1.95 (overlapping s and m, total 4H), 1.10-1.01 (m, 2H), 0.85-0.74 (m, 2H).

### (4) N-(3-(6-Amino-5-hydroxypyrimidin-4-yl)-5-fluoro-2-methylphenyl)-4-cyclopropyl-2-fluorobenzamide, INT 48

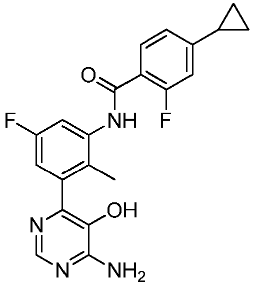

To a solution of **INT 47** (1.16 g, 2.38 mmol) in THF (20 mL) was added Pd-C (116 mg). The reaction mixture was hydrogenated at RT and normal pressure for 48 hr. The mixture was diluted with MeOH (10 mL) and filtered over a pad of Celite. The filtrate was concentrated. The residue was suspended in DCM (20 mL) and TFA (0.918 mL, 11.92 mmol) was added. The mixture was stirred at RT for 30 min, then poured into a mixture of saturated aqueous sodium hydrogen carbonate solution and EtOAc. The organic layer was washed with brine, dried over magnesium sulfate, filtered and concentrated to afford **INT 48** as a beige solid.

UPLC-MS: MS (ESI): [M+H]⁺ 397.2, rt = 0.80 min. ¹H NMR (DMSO-*d*₆): δ (ppm) 9.76 (s, 1H), 8.74 (s, 1H), 8.02 (s, 1H), 7.65 (t, 1H), 7.59-7.48 (m, 1H), 7.12-7.03 (m, 2H), 6.98-6.91 (m, 1H), 6.66 (s, br, 2H), 2.11-1.94 (overlapping s and m, total 4H), 1.14-0.98 (m, 2H), 0.87-0.71 (m, 2H).

### (5) (2S,4S)-2-Methyl N-Boc-4-((methylsulfonyl)oxy)pyrrolidine-2-carboxylate, INT 49

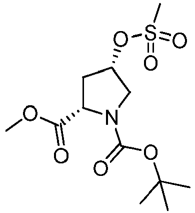

To a solution of (2*S*,4*S*)-methyl *N*-Boc-4-hydroxypyrrolidine-2-carboxylate (11.50 g, 46.88 mmol) in DCM (100 mL) was added DIPEA (9.70 mL, 55.54 mmol) followed by methanesulfonyl chloride (4.30 mL, 55.18 mmol). The reaction mixture was stirred at RT overnight. More DIPEA (1.50 mL, 8.59 mmol) and methanesulfonyl chloride (0.60 mL, 7.70 mmol) were added and the reaction mixture was stirred at RT for an additional hour. The mixture was concentrated. The residue was purified by flash chromatography (silica, DCM/EtOAc gradient, 5-15%) followed by a second purification by flash chromatography (silica; cyclohexane/EtOAc gradient, 0-100%) to afford **INT 49** as a yellow oil.

MS (ESI): [M+H]⁺ 324.2. ¹H NMR (CDCl₃): δ (ppm) rotamers 5.24 (m, br, 1H), 4.55-4.48 and 4.44-4.37 (m, total 1H), 3.84-3.70 (overlapping s and m, total 5H), 3.02 (s, 3H), 2.58-2.47 (m, br, 2H), 1.48 and 1.43 (s, total 9 H).

### (6) (2S,4S)-N-Boc-2-(hydroxymethyl)-4-((methylsulfonyl)oxy)pyrrolidine, INT 50

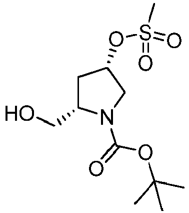

To a solution of **INT 49** (12.52 g, 38.72 mmol) in THF (100 mL) at 0 °C was added dropwise lithium borohydride solution (2 M in THF, 67.6 mL, 135.00 mmol). The reaction mixture was stirred overnight and allowed to warm up to RT. The mixture was cooled to 0 °C and water was added slowly. The mixture was extracted with EtOAc, the organic layer was washed with brine, dried over sodium sulfate, filtered and concentrated. The aqueous layer was diluted with saturated aqueous ammonium chloride solution and back-extracted with EtOAc. The organic layer was washed with brine, dried over sodium sulfate, filtered and concentrated. The two residues were combined and purified by flash chromatography (silica; cyclohexane/EtOAc gradient, 25-100%; followed by EtOAc/MeOH gradient, 0-10%) to afford **INT 50** as a colorless resin.

MS (ESI): [M+H-tBu]⁺ 240.1. ¹H NMR (CDCl₃): δ (ppm) rotamers 5.15-5.10 (m, br, 1H), 4.37-4.29 and 4.07-3.87 (m, total 2H), 3.81-3.62 (m, 2H), 3.59-3.47 (m, 2H), 3.00 (s, 3H), 2.37-2.25 and 2.11-2.02 (m, total 2H), 1.40 and 1.38 (s, total 9H).

### (7) (2S,4S)-N-Boc-2-((tert-butyldiphenylsilyl)oxymethyl)-4-((methylsulfonyl)oxy)-pyrrolidine, INT 51

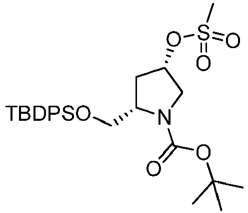

To a solution of **INT 50** (11.00 g, 37.24 mmol) in DCM (100 mL) was added imidazole (4.30 g, 63.16 mmol) followed by *tert*-butylchlorodiphenylsilane (11.0 mL, 42.82 mmol). The reaction mixture was stirred at RT for 3 hr. The suspension was filtered over a thin layer of Celite. The filtrate was washed with water and brine, dried over sodium sulfate, filtered and concentrated. The residue was purified by flash chromatography (silica; cyclohexane/EtOAc gradient, 0-50%) to afford **INT 51** as a colorless oil. UPLC-MS: MS (ESI): [M+H-tBu]⁺ 534.3, rt = 1.50 min. ¹H NMR (CDCl₃): δ (ppm) rotamers 7.69-7.62 (m, 4H), 7.45-7.35 (m, 6H), 5.28-5.16 (m, br, 1H), 4.17-4.07 and 4.05-3.97 (m, total 1H), 3.94-3.87 (m, 1H), 3.87-3.80 (m, 1H), 3.64-3.50 (m, 2H), 2.91 (s, br, 3H), 2.71-2.61 and 2.40-2.30 (m, total 2H), 1.43 and 1.33 (s, total 9H), 1.06 (s, 9H).

### (8) (2S,4R)-N-Boc-2-((tert-butyldiphenylsilyl)oxymethyl)-4-(cyano)pyrrolidine, INT 52

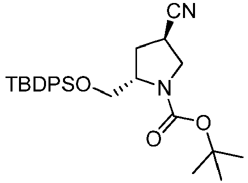

To a solution of **INT 51** (5.06 g, 9.48 mmol) in DMF (75 mL) was added sodium cyanide (1.39 g, 28.40 mmol). The reaction mixture was stirred at 100 °C for 3 hr. The mixture was partitioned between EtOAc and water. The organic layer was washed with brine, dried over sodium sulfate, filtered and concentrated. The residue was purified by flash chromatography (silica; cyclohexane/EtOAc gradient, 0-25%) to afford **INT 52** as a colorless resin.

¹H NMR (CDCl₃): δ (ppm) rotamers 7.65-7.55 (m, 4H), 7.47-7.31 (m, 6H), 4.13-4.05 and 4.02-3.91 and 3.78-3.57 (m, total 5H), 3.39-3.29 (m, 1H), 2.52-2.21 (m, 2H), 1.48 and 1.34 (s, total 9H), 1.05 (s, 9H).

### (9) (2S,4R)-N-Boc-2-(hydroxymethyl)-4-(cyano)pyrrolidine, INT 53

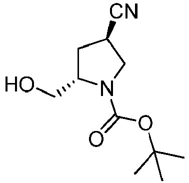

To a solution of **INT 52** (2.95 g, 6.35 mmol) in THF (30 mL) was added TBAF (1.0 M in THF, 7.5 mL, 7.50 mmol). The reaction mixture was stirred at RT for 2.5 hr. The mixture was concentrated and the residue was taken up in EtOAc. The organic phase was washed with water and brine, dried over sodium sulfate, filtered and concentrated. The residue was purified by flash chromatography (silica; cyclohexane/EtOAc gradient, 0-100%) to afford **INT 53** as a colorless residue.

MS (ESI): [M+H-tBu]⁺ 171.1. ¹H NMR (CDCl₃): δ (ppm) 4.14-3.83 (m, br, 2H), 3.75-3.53 (m, 4H), 3.35-3.19 (m, br, 1H), 2.40-2.26 and 2.23-2.10 (m, total 2H), 1.47 (s, 9H).

### (10) (2S,4R)-tert-Butyl 2-(((4-amino-6-(3-(4-cyclopropyl-2-fluorobenzamido)-5-fluoro-2-methylphenyl)pyrimidin-5-yl)oxy)methyl)-4-cyanopyrrolidine-1-carboxylate, INT 54

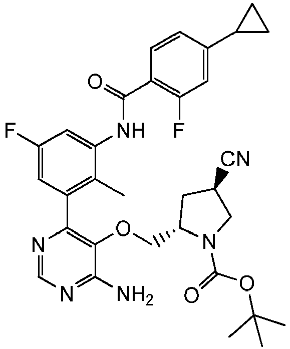

To a solution of **INT 48** (240 mg, 0.61 mmol) and **INT 53** (274 mg, 1.21 mmol) in THF (15 mL) was added SMOPEX-301 (1 mmol/g, 1.51 g, 1.51 mmol). The mixture was heated to 60 °C and DIAD was added dropwise at this temperature. The reaction mixture was stirred at 60 °C for 2 hr. The mixture was filtered through a pad of Celite, the filtrate was concentrated. The residue was purified by flash chromatography (silica; TBME/EtOAc gradient, 0-100%) to afford **INT 54** as a colorless oil. UPLC-MS: MS (ESI): [M+H]⁺ 605.3, rt = 1.14 min.

### (11) N-(3-(6-Amino-5-(((2S,4R)-4-cyanopyrrolidin-2-yl)methoxy)pyrimidin-4-yl)-5-fluoro-2-methylphenyl)-4-cyclopropyl-2-fluorobenzamide, INT 55

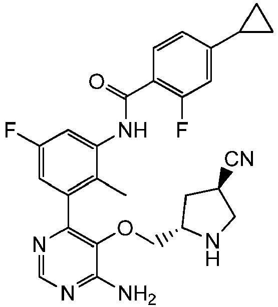

To a solution of **INT 54** (content 83%, 313 mg, 0.43 mmol) in DCM (10 mL) was added TFA (1.0 mL, 12.98 mmol) followed by one drop of water. The reaction mixture was stirred at RT for 1.5 hr. The mixture was concentrated. The residue was purified by flash chromatography (silica; DCM/(MeOH with 2% aqueous ammonium hydroxide) gradient, 0-40%) to afford **INT 55** as the free amine as a colorless residue.

UPLC-MS: MS (ESI): [M+H]⁺ 505.3, rt = 0.75 min.

### (12) N-(3-(5-(((2S,4R)-1-Acryloyl-4-cyanopyrrolidin-2-yl)methoxy)-6-aminopyrimidin-4-yl)-5-fluoro-2-methylphenyl)-4-cyclopropyl-2-fluorobenzamide

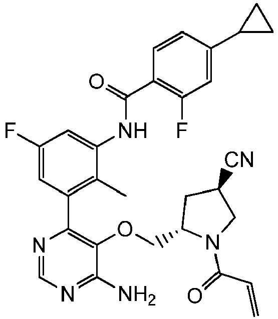

To a solution of **INT 55** (102 mg, 0.20 mmol) and DIPEA (0.200 mL, 1.15 mmol) in DCM (4.0 mL) at 0°C was slowly added dropwise acryloyl chloride (0.020 mL, 0.24 mmol). The reaction mixture was stirred at 0°C for 30 min. The mixture was concentrated. The residue was purified by flash chromatography (silica; EtOAc/MeOH gradient, 0-20%), followed by SFC purification to afford **Example 39** as white solid after lyophilization.

UPLC-MS: MS (ESI): [M+H]⁺ 559.4, rt = 0.96 min. ¹H NMR (DMSO-*d*₆): δ (ppm) rotamers 9.81 (s, 1H), 8.21 (d, 1H), 7.72-7.63 (m, 1H), 7.57-7.47 (m, 1H), 7.17-6.91 (m, 5H), 6.48-6.39 and 6.32-6.21 (m, total 1H), 6.15-6.05 (m, 1H), 5.68-5.56 (m, 1H), 4.29-4.22 and 4.18-4.12 (m, total 1H), 3.73-3.62 and 3.53-3.45 (m, total 3H), 3.35-3.25 and 3.17-3.08 (m, total 2H), 2.26-1.95 (overlapping m and s, total 6H), 1.10-1.01 (m, 2H), 0.85-0.75 (m, 2H).

### Example 40

### N-(3-(5-(((2S,4S)-1-Acryloyl-4-cyanopyrrolidin-2-yl)methoxy)-6-aminopyrimidin-4-yl)-5-fluoro-2-methylphenyl)-4-cyclopropyl-2-fluorobenzamide

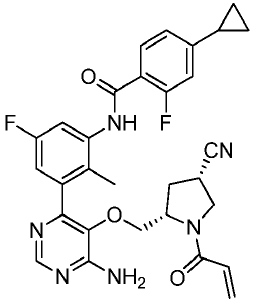

The title compound was prepared according to **Scheme** 4 following a procedure analogous to **Example 39** replacing (2S,4S)-methyl *N*-Boc-4-hydroxypyrrolidine-2-carboxylate with (2S,4R)-methyl *N*-Boc-4-hydroxypyrrolidine-2-carboxylate in step 5.

UPLC-MS: MS (ESI): [M+H]⁺ 559.4, rt = 0.94 min.

### Example 41: BTK inhibitor/CAR19 T-cell combined therapy for mantle cell lymphoma

Adoptive T-cell therapy holds considerable promise for the treatment of lymphoid malignancies. Mantle cell lymphoma (MCL), both before and after large cell transformation, will also likely to benefit from the CART19-based adoptive therapy, in particular when combined with BTK inhibitors such as those that directly affect MCL cells.

To further analyze the combination of CART19-based adoptive therapy in combination with BTK inhibitors, high throughput screens will be used to evaluate BTK inhibitors described herein in combination with CART19 cells. The most promising combinations will be evaluated in greater detail, both in vitro and in vivo, in MCL xenotransplant mouse model, which ultimately may guide the development of a clinical protocol to evaluate combination of BTK inhibitor and the CART cell immunotherapy in MCL patients.

In this study, preclinical studies will be performed to determine potential clinical efficacy of this approach in the various subtypes of MCL and to evaluate the ability to therapeutically target MCL cells using CART19 cells either alone or in will combination with small molecule BTK inhibitors.

### Research plan

In a pre-clinical setting, the ability to therapeutically target MCL cells, both cultured and primary-type cells, using inhibitors of BTK will be evaluated. A high throughput MTT assay will be used to determine the effect of these agents to identify potential optimal combinations, dosing and timing of the agent application. The most promising 2-3 combinations will be evaluated in the greater detail in regard to cell function, phosphorylation-based cell signaling, and gene expression first in vitro and later *in vivo* in the MCL xenotransplant model.

### In-vitro studies to characterize the ability of kinase inhibitor/CART-19 cell combinations to effectively target MCL cells

In this aim, detailed functional, phenotypic, biochemical, and molecular assays listed above to study in-vitro the impact of the BTK inhibitors on MCL cells as well as to examine interactions between CART19 cells and MCL cells and the impact of the inhibitors on these interactions will be examined.

The benchmarks for accomplishing this aim will be to generate a comprehensive data set to:
i. document that CART19 cells are activated by and lyse the cultured and primary MCL cells;
ii. demonstrate that the BTK inhibitors enhance the ability of each other and/or CART19 cells to eliminate MCL cells without negatively impacting CART19 cell function when appropriately applied in regard to the dose and, for some, timing of the inhibitor vs. CART19 cell administration; and
iii. establish a regimen for the schedule and dosing for the BTK inhibitor to be used in the in vivo MCL xenotransplant experiments using the NSG mice.

The goals of this study are, e.g., to evaluate whether identify the optimal therapeutic combinations of BTK inhibitors together with CART19 cells, monitor CART19 activity, and characterize the functional, biochemical, and molecular effects of the therapy on MCL.

These studies should to establish a rational schema for schedule for the timing and dose of BTK treatment kinase inhibitor in conjunction with CART19 therapy to be evaluated in vivo.

### In-vivo studies to evaluate the ability of CART19 cells to target follicular lymphoma, alone and in combination with BTK inhibitor

In this aim, we will test in animal models the ability of the selected inhibitor/CART19 cell combination(s) to affect growth of established and primary MCL cells.

The benchmarks for accomplishing this aim will be to generate a data set to:
i. demonstrate that the selected inhibitor/CART19 cell combination markedly enhances the survival of animals engrafted with MCL as compared to the controls (single and mock agent treated animals);
ii. establish a regimen for the schedule and dosing for the selected kinase inhibitor/CART19 combination to be used as the basis for a future clinical trial.

The goals of this study include evaluating the treatment and dose schedule defined in aim 1 for the identified kinase inhibitor/CART19 plus BTK inhibitor combination, and to test whether BTK treatment synergizes with CART19 to target MCL in NSG mice xenotransplanted with MCL cells, both cultured and primary.

The following cell types, compounds, animals and experimental methodologies will be used to accomplish the proposed aims:

### MCL cells:

Four MCL cell lines (Jeko-1, Mino, SP-49, and SP-53) and viably frozen samples from 15 primary MCL (12 typical and 3 blastoid). While the cell lines grow well spontaneously, the primary cells will be cultured alone as well as in the presence of conditioned medium collected from HS5 bone marrow stromal cells to improve their viability.

### CART19 cells:

Primary human T cells engineered to express CAR19 will be generated using lentivirus transduction and using the established protocols ((Kalos M, et al. (2011) Sci Transl Med. 3: 95ra73; and Porter DL, et al. (2011) N Engl J Med. 365: 725-733). Following a single transduction event T cells typically express CAR19 at frequencies exceeding 30%.

Our studies will use CART 19 populations from five SLL/CLL patients (50-100 vials/patient at with 1x10⁷ cells/vial are already available). CART19 cells will be identified using an anti-CAR19-specific idiotype antibody (STM). CART19 activity will be controlled both *in vitro* and *in vivo* in NSG mice in the standardized manner using CD19+ NALM-6, CD19-negative K562, and CD19-transduced K562 cell lines. Although CART19 cell function is not MHC restricted, CART19 cell from at least 5 MCL patients will also be used.

### BTK inhibitors

BTK inhibitors described herein, e.g., compounds of formula (I) can be tested using the methods described in this Example.

The compounds will be evaluated first at the pre-determined spectrum of effective doses, including the non-toxic concentrations reached in patients' sera, to assure optimal BTK inhibition.

### Animals:

The *in-vivo* experiments will be performed using NOD-SCID-IL-2Rgc null (NSG) mice which are bred and available from the Stem Cell and Xenograft Core using breeders obtained from Jackson Laboratory (Bar Harbor). Mice will be housed in sterile conditions using HEPAfiltered microisolators and fed with irradiated food and acidified water.

Transplanted mice are treated with antibiotics (neomycin and polymixin) for the duration of the experiment. Six to eight week-old animals, equal mixes of males and females, will be utilized for all studies in accordance with protocols approved by the Institutional Animal Care and Use Committee.

We have used NSG animals in previous T cell adoptive transfer studies specifically to evaluate differential activity of CART19 cells (Witzig TE, et al. (2010) Hematology Am Soc Hematol Educ Program. 2010:265-270, MTT assay). The high throughput MTT assay to evaluate MCL cell growth will be performed first in response to the BTK inhibitors applied either alone or in various combinations. This assay is able to simultaneously determine cell proliferation rate and viability, allowing efficient evaluation of many possible combinations of small molecule inhibitors in the presence or absence of CART19 cells. The key aspects of this analysis will be to characterize the drug effect in regard to potential synergistic, additive, or antagonistic effect. In addition, the effect of the BTK inhibitors on CART19 cells will be evaluated. Establishing the proper timing of the drug application to minimize or optimize their potential effect on CART19 cells will be one of the aims of these experiments.

To perform the test, MCL cells will be seeded in 96-well plates at 1x10⁴ cells/well, in triplicates, and exposed to medium or BTK inhibitors in various combinations and various concentrations of CART-19 cells. After 48 and 74 hrs, the relative number of metabolically active cells will be determined by the use of MTT reduction colorimetric assay (Promega).

The significance of difference between the mean values (+/- S.D.) of the controls and different treatment conditions will be evaluated using Student's t-test with the P value of <0.05 considered to be statistically significant.

### Cell proliferation and apoptosis assays:

The most promising drug combinations will be next evaluated in the CFSE labeling and terminal dUTP nick-end labeling (tunel) assays to determine both cytostatic and cytotoxic components of MCL cell growth inhibition, respectively. In the former assay, MCL cells will be labeled with CFSE addition of the BTK inhibitor and/or unlabeled CART-19 cells. After 48 hrs, the cultured cells will be the analyzed by FACS for the CFSE labeling pattern of the MCL-type cells. The tunel assay will be done using the ApoAlert DNA Fragmentation Assay Kit from BD Biosciences according to the manufacturer's protocol.

In brief, MCL cells will be cultured with the inhibitors and/or CART19 cells for 48 or 72 hours. After being washed, cells will be stained with labeled anti-CD20 antibody and permeabilized, washed, and incubated in TdT buffer for 1 hour at 37°C. The reaction will be stopped, the cells washed, resuspended, and analyzed by flow cytometry using the CellQuest PRO software.

### CART19 functional assays:

We will measure effector activity of CART19 cells against MCL cell lines using CD107 degranulation, Intracellular Cytokine Secretion (ICS) assays, proliferationcytolysis assays, and multiplex cytokine detection assays. For degranulation and ICS assays, effector (T cells) and Target (tumor cells) will be co-incubated in the presence of anti-CD107 antibody for 4 hours at E:T of 0.2:1 followed by staining for surface (CAR19, CD3, CD8, CD4) and intracellular cytokine markers as per established protocols. Cytolysis of MCL cells will be assessed using flow-cytometry-based cytolysis assays. For proliferation assays, effector cells will be pre-loaded with CFSE (Carboxyfluorescein succinimidyl estercarboxy-fluoroscein-succinil esterase), mixed with target cells at E:T of 0.2:1, co-incubated at 37°C for 4 days, stained for surface markers (CAR19, CD3, CD8, CD4) and analyzed for dilution of CFSE by flow-cytometry.

### Multiplex cytokine assays.

We will measure production of cytokines by CART19 cells in response to MCL targets using Luminex-based bead assays. For these analyses we will employ the Invitrogen 30-plex kit that simultaneously measures IL-1β, IL-1RA, IL-2, IL-2R, IL-4, IL-5, IL-6, IL-7, IL-8, IL-10, IL-12 (p40/p70), IL-13, IL-15, IL-17, TNF-α, IFN-α, IFN-γ, GM-CSF, MIP-1α, MIP-1β, IP-10, MIG, Eotaxin, RANTES, MCP-1, VEGF, G-CSF, EGF, FGF-basic, and HGF in serum, plasma, or tissue culture supernatant.

### Multiparametric flow cytometry analysis of CART19T cells:

We will measure the modulation of surface markers associated with functional activation and suppression on CART19 cells following co-incubation with tumor cells using four color flow cytometry and a custom BD LSR II equipped with 4 lasers (blue (488 nM), violet (405 nM), Green (532 nM), and Red (633 nM) available through the University of Pennsylvania Abramson Cancer Center Flow Cytometry Core. All flow cytometry data will be analyzed using FlowJo software (TreeStar, San Carlos, CA). These analyses can be performed using a dump channel to exclude dead cells and target cells (CD19+), and a CAR19 idiotype-specific reagent to detect CART19 cells. We will evaluate the following markers on CART19-positive and -negative cells (CD3+/CD8+ and CD3+/CD4+) post co-incubation with tumor cells, on either intact or permeabilized cells as needed. We have established multi-parametric panels for these markers:
- activation/effector function: CD25, CD154, CD134, CD137, CD69, CD57, CD28, T-bet
- inhibition: CD152 (CTLA4), PD1, LAG3, CD200
- suppression (Treg) CD4+/CD25++/CD127-, Fox-P3+

Simultaneously, MCL cells identified by CD19 and CD5 staining, will be examined for expression of the immunosuppressive proteins: CD174 (PD-L1) CD173 (PD-L2) and CD152.

### Inhibitor impact on cell signaling:

This part of the study will focus only on the selected compounds; the ones that proved to be the most effective in the functional assays (cell growth, proliferation, and apoptosis) described above. The effect will be studied separately for each drug and for the selected combinations and the studies will be adjusted to the specific compounds. For example, assays related to BTK inhibition will focus on the PI3K-AKT and MEK-ERK pathways. These studies will be performed by Western blotting using phospho-specific antibodies as described (Marzec M, et al. (2006) Blood. 108:1744-1750; Marzec M, et al. (2008) Blood 111: 2181-2189; Zhang Q, et al. (2011) Proc Natl Acad Sci USA 108: 11977-11982). In brief, the MCL cells will be lysed and the protein extracts will be assayed using the Lowry method (Bio-Rad) and loaded into the polyacrylamide gel. To examine protein phosphorylation, the blotted membranes will incubated with the phosphor-specific antibodies. Next, the membranes will be incubated with the appropriate secondary, peroxidase-conjugated antibodies. The blots will be developed using the ECL Plus System from Amersham.

### Genome-scale gene expression analysis:

Inhibition of cell signaling typically leads to changes in gene transcription. To determine the effects of the selected inhibitor, or a few inhibitors on gene transcription in MCL, a genome-scale gene expression analysis will be performed as done as described in Marzec M, et al. (2008) Blood 111: 2181-2189; Zhang Q, et al. (2011) Proc Natl Acad Sci USA 108: 11977-11982. In brief, the cells will be treated in triplicate cultures with the selected inhibitor or its diluent for 0, 4, and 8 hours. The total RNA will be further purified to enrich for mRNA which will be reverse transcribed, labeled and examined by hybridization to the Affimetrix microchip against all known gene exons. The microarray data will be normalized and summarized using RMA as implemented in GeneSpring and MAS5 algorithm. The resulting pvalues will be corrected for multiple testing using false discovery rate (FDR) by the Benjamini- Hochberg step-up method. Differential expression testing will be accomplished using a variety of tools including SAM and PartekPro. The emerging genes of interest will then be clustered based on expression patterns (GeneSpring or Spotfire), and clusters will be analyzed for functional groups and pathways in KEGG, Ingenuity Pathway Analysis, and Gene Ontology databases using the NIH-David as the search tool. For the genes identified based on the data, the independent expression conformation by the quantitative RTPCR will be performed on a larger pool of samples (at least 20) of various types of MCL (standard vs. blastoid and SOX11-positive vs. SOX11-negative).

### Whole-exome DNA sequence analysis:

To better characterize the MCL cases in regard to their pathogenesis and, to the extent possible, response to the proposed here combination therapies, the sequence of exomic DNA will be examined. Whole-exome capture and next generation sequencing of the MCL and normal peripheral blood DNA samples will performed using the NimbleGen Sequence Capture 2.1M Human Exome Array and the HiSeq 2000/1000 Illumina instrument.

### Evaluation of the treatment effect in the xenotransplanted tumors:

The NSG mice will carry the MCL tumors tumors (derived from both MCL cell lines: Jeko and Mino and primary cells implanted as either tissue fragments or, less preferably, cell suspensions). The tumors will be propagated by subcutaneous implantation of the small tumor fragments. The therapy will be initiated once the tumors reach 0.2-0.3 cm in the diameter. The BTK inhibitor(s) will administered by gavage at dose and timing preselected in vitro (for example, we expect to apply BTK inhibitor simultaneously with CART19 cells, given its B-cell specificity and expected lack of any inhibitory effect on CART19 cells). CART-19 cells will be injected into the tail vein of the tumor-bearing mice at a dose of 1x10⁷/animal, the BTK inhibitor(s) will administered by gavage at dose and timing preselected in vitro. a dose we have established to be sufficient to reproducibly eradicate malignant cells and, at the same time, not to induce xeno-graft versus host disease. A large master stock of CART19 cells (1x 10¹⁰) will be generated and frozen to minimize variability associated with effector cell differences. The primary measure from these experiments will be survival, which we will assess using Kaplan/Meier curves. As a secondary measure we will evaluate differential expansion of CART19 cells in animals following T cell infusion. This will be made possible by the fact that the infused T cell product will be composed of CART19-positive and -negative cells at a defined ratio. For these analyses, animals will be bled weekly by tail vein bleed (25 microliters each time), followed by red blood cell lysis and staining for human CD3, CD4, CD8, and CART19. Preferential expansion of CART19 cells (at least a 2-fold increase in the CART19+/CART19- ratio will be evidence for selective MCL-driven CART19 cell expansion. To assess the treatment results, volumes of the implanted subcutaneous tumors will be measured determined as follows, according to the formula: volume = 0.4ab2, where a and b designate respectively long and short diameters of the tumor. Tumor volumes differences between the treated and untreated groups of mice will be statistically analyzed using a standard t-test. Mice will be sacrificed at either the end-point of the experiments (>30 days), or if tumors reach > 1.2 cm in diameter, or when any evidence of the animal distress noted. Tumor volumes differences between the treated and untreated groups of mice will be statistically analyzed using a standard t-test. The tumors as well as the internal organs will be harvested, processed and analyzed by histology and, for selected tissues, by immunohistochemistry using the battery of antibodies against B cells (CD20, CD79a, Pax-5, CD10, BCL-6) and T cells (CD2, CD3, CD4, CD5, CD7, CD8, TIA-1), and the proliferation marker Ki-67.

### Statistical analysis:

In the *in vitro* functional studies, the significance of difference between the mean values (+/-S.D.) of the controls and different treatment conditions will be evaluated using Student's t-test with the P value of < 0.05 considered to be statistically significant. Based on our previous experiences, the differences between the experimental mouse groups are expected to be large. Thus, 10 NSG mice will be used for each treatment group, which will ensure at least 90% power at 0.05 type 1 error level with a two-sided two sample /-test, given the ratio between the difference in treatment means and the standard deviation is at least 3, which is expected. Data will be presented as mean ± SEM. Comparison among groups will be made using the two sample t-test. A value of p < 0.05 is considered to be significant. For tumor-free survival studies, groups of 10 mice will be used for survival comparison, and the disease status (tumor vs. no tumor) and tumor-free time for each mouse will be recorded. The Kaplan-Meier survival curve will be plotted and the log-rank test will be performed to compare the survival curves. The significance level is controlled at 0.05.

### Example 42: Ibrutinib/CAR19 T-cell combined therapy for mantle cell lymphoma

This example illustrates the efficacy of a CAR19 therapy in combination with the BTK inhibitor ibrutinib. The experiments in this example provide a guide for quantifying the efficacy of other BTK inhibitors in combination with CAR therapies, and also provide evidence for efficacy of a BTK inhibitor described herein in combination with a CAR therapy.

The experiments described in this example characterize CART19 activity in combination with ibrutinib treatment for treating mantle cell lymphoma *in vitro* and *in vivo.* Ibrutinib is a small molecule inhibitor of BTK often used for treatment of some hematological cancers. The *in vitro* experiments described herein include assessment of proliferation, cytokine production, CD107a degranulation, and cytotoxicity. Xenoplant mouse models were utilized to investigate the efficacy and optimal dosage of CART19 with ibrutinib treatment *in* vivo.Although ibrutinib displays considerable activity in MCL, about 30% of patients do not respond, and among the responders, only 21% to about one-third experience complete remission (Wang et al. NEJM 369.6(2013):507-16). Achievement of a complete remission is associated with improved progression-free survival. Furthermore, therapy can lead to drug resistance with the duration of median response of 17.5 months. In some settings, mutations in BTK binding sites or immediately downstream have been observed after ibrutinib therapy, highlighting a mechanism of drug resistance that may become increasingly frequent. See, e.g., Woyach et al. NEJM. 370.24(2014):2286-94. Also, blockade of BTK function leads to inhibition of B cell receptor (BCR) signaling and is not directly cytotoxic. See, e.g., Ponader et al. Blood. 119.5(2012):1182-89. Lack of cytotoxicity and failure to eradicate malignant clones predispose to clonal evolution under a selection pressure. Also, preliminary findings of increased transformation to aggressive disease in patients treated with ibrutinib for CLL are concerning. See, e.g., Byrd et al. NEJM. 369.1(2013):32-42; and Parikh et al. Blood. 123.11(2014):1647-57.

Infusion of autologous T cells transduced with chimeric antigen receptors (CAR) against the B-cell specific CD19 antigen (CTL019, CART19) leads to dramatic clinical responses in the majority of patients with various B-cell neoplasms, foremost acute lymphoblastic leukemia (ALL). See, e.g., Maude et al. NEJM. 371.16(2014):1507-17; and Ruella et al. Expert Opin. Biol. Ther. (2015):1-6. The presence of lymph node masses or bulky disease may lead to decreased T cell infiltration and consequent reduced anti-tumor activity. Bulky lymphadenopathy does not appear to impair the response to ibrutinib. Wang et al. NEJM. 369.6(2013):507-16. Also, ibrutinib has shown particular efficacy in reducing tumor masses and mobilizing neoplastic B cells in the peripheral blood.

### Methods

*Cell lines and primary samples.* MCL cell lines were obtained from ATCC (Mino, Jeko-1, SP-49) while MCL-RL was generated from a progressive pleural effusion of a MCL patient. For in vitro experiments, cell lines were maintained in culture with RPMI media supplemented with 10% fetal calf serum, penicillin, and streptomycin. For some experiments, MCL-RL and Jeko-1 cells were transduced with click beetle green luciferase/eGFP and then sorted to obtain a >99% positive population. The acute leukemia cell lines MOLM-14, K562 or NALM-6 and the T-ALL cell line JURKAT were used as controls. These cell lines were originally obtained from the ATCC. De-identified primary human MCL bone marrow (BM) and peripheral blood (PB) specimens were obtained from the clinical practices of University of Pennsylvania. For all functional studies, primary cells were thawed at least 12 hours before experiment and rested at 37° C.

*Generation of CAR constructs and CAR T cells.* The murine anti-CD 19 Chimeric antigen receptor (containing a CD8 hinge, 41BB costimulatory domain and CD3 zeta signaling domain) was generated as previously described. See, e.g., Milone et al. Molecular Therapy: the Journal of the American Society of Gene Therapy. 17.8(2009):1453-64. Production of CAR-expressing T cells was performed as previously described. See, e.g., Gill et al. Blood. 123.15(2014):2343-54. Normal donor CD4 and CD8 T cells or PB mononuclear cells (PBMC) were obtained from the Human Immunology Core of the University of Pennsylvania. T cells were plated at 1x10⁶/ml, with a CD4:CD8 ratio of 1:1 and expanded in X-vivo 15 media (Lonza, 04-418Q), human serum AB 5% (Gemini, 100-512), penicillin/streptomycin (Gibco, 15070063) and Glutamax (Gibco, 35050061) using anti-CD3/CD28 Dynabeads (Life Technologies, 11161D) added on the day 1 of culture and removed on day 6. T cells were transduced with lentivirus on day 2. T cells were expanded in culture for 8-15 days and harvested when the median cell volume was below 300 fl. T cells were then cryopreserved in FBS 10% DMSO for future experiments. Prior to all experiments, T cells were thawed and rested overnight at 37° C.

*Ibrutinib.* Ibrutinib (PCI-32765) was purchased from MedKoo (#202171) or Selleck Biochemicals (#S2680) as a powder or DMSO solution. For in vitro experiments, ibrutinib was diluted to the concentrations of 10, 100 and 1000 nM. For in vivo experiments, ibrutinib powder was dissolved in a 10% HP-beta-cyclodextrin solution (1.6 mg/ml) and administered to mice in the drinking water.

*Multiparametric flow cytometry analysis.* Anti-human antibodies were purchased from Biolegend, eBioscience, or Becton Dickinson. Cells were isolated from in vitro culture or from animals, washed once in PBS supplemented with 2% fetal calf serum, and stained for 15 minutes at room temperature. For cell number quantitation, Countbright (Invitrogen) beads were used according to the manufacturer's instructions. In all analyses, the population of interest was gated based on forward vs. side scatter characteristics followed by singlet gating, and live cells were gated using Live Dead Aqua (Invitrogen). Time gating was included for quality control. Surface expression of CAR19 was detected as previously described. See, e.g., Kalos et al. Science Translational Medicine. 3.95(2011):95ra73. Flow cytometry was performed on a four-laser Fortessa-LSR cytometer (Becton-Dickinson) and analyzed with FlowJo X 10.0.7r2 (Tree Star).

*Degranulation assay.* Degranulation assay was performed as previously described. See, e.g., Kalos et al. Science Translational Medicine. 3.95(2011):95ra73. T cells were incubated with target cells at a 1:5 ratio in T cell media. Anti-CD107a-PECY7 (Biolegend), anti-CD28 (BD Biosciences), anti-CD49d (BD Biosciences) antibodies and monensin (BD Biosciences) were added to the co-culture. After 4 hours, cells were harvested and stained for CAR expression, CD3, CD8 and Live Dead aqua staining (Invitrogen). Cells were fixed and permeabilized (Invitrogen Fix/Perm buffers) and intracellular staining was then performed to detect multiple cytokines (IFN, TNFa, IL-2, GM-CSF, MIP1b).

*Proliferation assay.* T cells were washed and resuspended at 1x10⁷/ml in 100 ul of PBS and stained with 100 ul of CFSE 2.5 uM (Invitrogen) for 5 minutes at 37° C. The reaction was then quenched with cold media, and cells were washed three times. Targets were irradiated at a dose of 100 Gy. T cells were incubated at a 1:1 ratio with irradiated target cells for 120 hours, adding media at 24 hours. Cells were then harvested, stained for CD3, CAR and Live Dead aqua (Invitrogen), and Countbright beads (Invitrogen) were added prior to flow cytometric analysis for absolute quantification.

*Cytotoxicity assays.* Luciferase/eGFP+ NALM-6 or RL cells were used for cytotoxicity assay as previously described. See, e.g., Gill et al. Blood. 123.15(2014):2343-54. Targets were incubated at the indicated ratios with effector T cells for 4 or 16 hours. Killing was calculated by bioluminescence imaging on a Xenogen IVIS-200 Spectrum camera.

*Cytokine measurements.* Effector and target cells were co-incubated at a 1:1 ratio in T cell media for 24 h. Supernatant was harvested and analyzed by 30-plex Luminex array (Luminex Corp, FLEXMAP 3D) according to the manufacturer's protocol (Invitrogen). See, e.g., Kalos et al. Science Translational Medicine. 3.95(2011):95ra73.

*In vivo experiments.* NOD-SCID-γ chain-/- (NSG) mice originally obtained from Jackson Laboratories were purchased from the Stem Cell and Xenograft Core of the University of Pennsylvania. All experiments were performed on protocols approved by the Institutional Animal Care and Use Committee (IACUC). Schematics of the utilized xenograft models are discussed herein. Cells (MCL cell lines or T cells) were injected in 200 ul of PBS at the indicated concentration into the tail veins of mice. Bioluminescent imaging was performed using a Xenogen IVIS-200 Spectrum camera and analyzed with LivingImage software v. 4.3.1 (Caliper LifeSciencies). Animals were euthanized at the end of the experiment or when they met pre-specified endpoints according to the IACUC protocols.

*Immunohistochemistry.* Immuno-histochemical (IHC) staining of formalin fixed paraffin embedded tissues was performed on a Leica Bond-III instrument using the Bond Polymer Refine Detection System. Antibodies against CD3, CD4, CD8, Pax5 and CyclinD1 were used undiluted. Heat-induced epitope retrieval was done for 20 minutes with ER2 solution (Leica Microsystems AR9640). Images were digitally acquired using the Aperio ScanScope™.

*Statistical Analysis.* All statistics were performed using GraphPad Prism 6 for windows, version 6.04.

### Mantle cell lymphoma cell lines

Most mantle cell lymphoma (MCL) lines in existence have been immortalized and propagated for many generations in vitro, thus losing their dependence on B cell receptor signaling. Consequently, they are poorly sensitive to ibrutinib. In vitro experiments were performed to determine the sensitivity of MCL cell lines on ibrutinib treatment. These cell lines were also used to assess the efficacy of ibrutinib/CART19 combination treatment in experiments discussed further in this example. MCL cells were harvested from the pleural effusion of a patient with multiply relapsed MCL. Both the original cells (RL^{primary}) and a cell line derived from them (RL) had a blastoid morphology, typical MCL immunophenotype and were positive for the classical t(11;14) translocation by fluorescence in-situ hybridization (FISH) (FIG.16A, 16B, 16C).

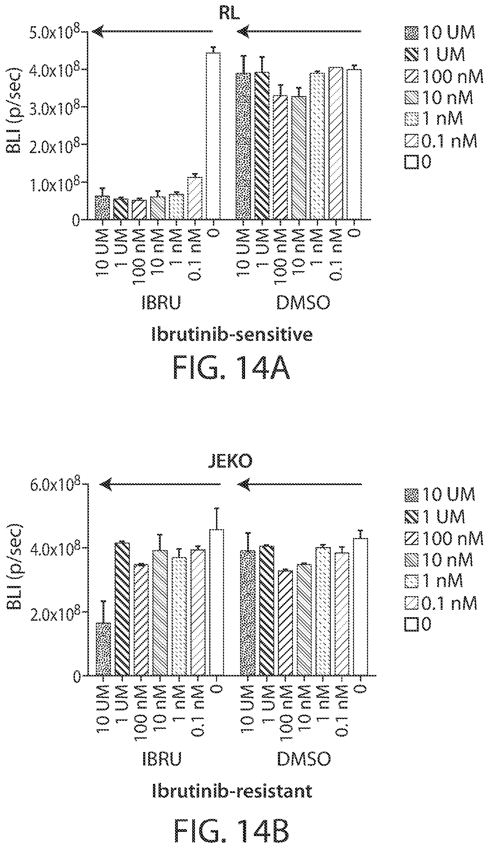

RL and JEKO-1 cells were cultured with different doses of ibrutinib (0.1 nM, 1nM, 10nM, 100nM, 1µM, and 10 µM) and sensitivity to ibrutinib was determined by measuring the reduction of bioluminescence (BLI). As shown in FIG. 14, RL cells were sensitive to ibrutinib treatment in a dose-dependent manner. However, JEKO-1 cells were resistant to ibrutinib treatment (no demonstration of reduced bioluminescence as a function of increased ibrutinib dosage).

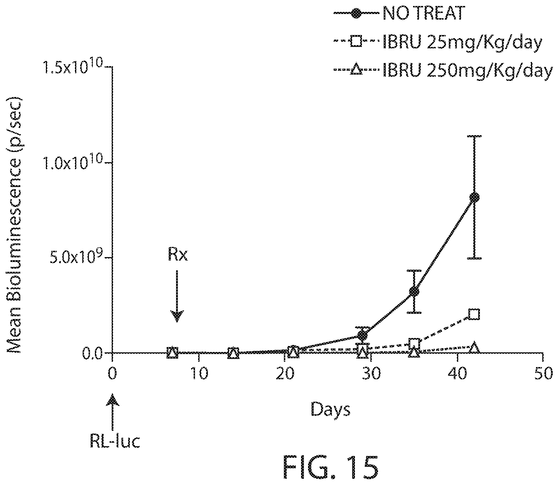

Sensitivity of RL, Jeko-1, and Mino cells to ibrutinib were also assayed using a MTT assay. Exposure of RL to increasing concentrations of ibrutinib in vitro led to a dose-dependent inhibition of proliferation and of downstream mediator phosphorylation, indicating an on-target effect of ibrutinib, with an IC50 of 10nM. In contrast, the established MCL cell lines Jeko-1 and Mino were relatively resistant to ibrutinib, with IC50 results up to 10 µM (FIG. 16D). To confirm RL as a viable model for in vivo experiments, immunodeficient NOD-SCID-γ chain knockout (NSG) mice were engrafted with 1x10⁶ luciferase-expressing RL cells (FIG. 16E). Their tumor burden and survival were assessed. After intravenous injection, MCL engrafted in all mice and localized to the spleen and liver, followed by dissemination to bone marrow, blood and lymph nodes (FIG. 15F). Histology of affected spleen and liver is consistent with parenchymal infiltration of MCL. (FIG. 16G).

These results showed that these different cell lines could therefore be used to model both ibrutinib-sensitive and ibrutinib-resistant MCL.

### Mantle cell lymphoma cells are sensitive to killing by CART19

Most preclinical work showing the efficacy of CART19 has been done using B-ALL cell lines, which are not sensitive to ibrutinib. Furthermore, the best clinical responses to date have been reported in patients with B-ALL, whereas patients with indolent B-cell malignancies have reportedly lower responses.

To show that MCL is sensitive to killing by CART19 in this model, healthy donor T cells were transduced with an anti-CD19 CAR construct that has been used in clinical trials. See, e.g., Porter, NEJM 2011. A series of in vitro experiments were performed to show that the ibrutinib-sensitive cell line RL and the ibrutinib-resistant cell line Jeko-1 lead to equivalent CART-19 degranulation, cytokine production, killing and proliferation (FIG. 17A, 17B, 17C, 17D). In addition, peripheral blood or bone marrow was obtained from two patients with MCL in leukemic phase, permitting the expansion and transduction of autologous T cells with anti-CD19 CAR. Autologous patient-derived CART19 cells were reactive to MCL whereas the untransduced T cells were unreactive to their respective autologous MCL (FIG. 17E, 17F). These results indicate that MCL are sensitive to the effector functions of CART19.

### Assessment of ibrutinib/CART19 treatment in vitro

An effect of ibrutinib on T cells was previously discounted based on short-term activity assays, as described in Honigberg et al. Proc. Natl. Acad. Sci. USA. 107(2010):13075-80. Later, an analysis of the effect of ibrutinib on the T cell kinase ITK was reported to support an immunomodulatory role of ibrutinib on CD4 T cells by inhibiting Th2-type polarization. See Dubovsky et al. Blood 122.15(2013):2539-49. Cytokine analysis of patients treated with CART19 by several groups indicates that CART19 therapy is associated with both Th1 (IL2, IFNγ, TNF), Th2 (IL-4, IL-5, IL-10) and other cytokines (see, e.g., Kalos et al. Science Translational Medicine 3.95(2011):95ra73). In this example, the effect of CART19 function was evaluated with ibrutinib at, above, and below the ibrutinib concentrations that would be expected in patients (mean peak concentration in serum 100-150ng/ml) See Advani et al. J. Clin. Oncol. 2013;31:88.

CART19 cells were found to contain ITK. Non-specific stimulation of CART19 cells via the TCR in the presence of ibrutinib led to a reduction in phosphorylated ITK (pITK-Y₁₈₀) as previously reported for CD4+ T cells. See Dubovsky et al. Blood 122.15(2013):2539-49. In contrast, specific stimulation of CART19 cells via the CAR did not lead to diminished ITK activation (FIG. 18A). This observation indicated that CART19 function would likely not be adversely affected by exposure to ibrutinib.

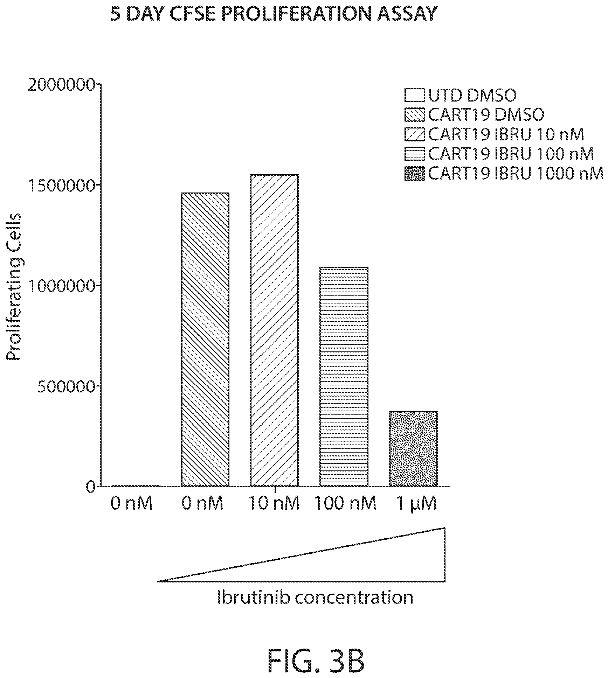

In addition, the short- and long-term in vitro function of CART19 cells in the presence of ibrutinib was determined. Ibrutinib at clinically relevant concentrations did not impair CART19 cell proliferation, degranulation or cytokine production; although at supra-physiologic concentrations, there was inhibition of CART19 cell functions, likely representing non-specific toxicity (FIG. 18B, 18C, 3B).

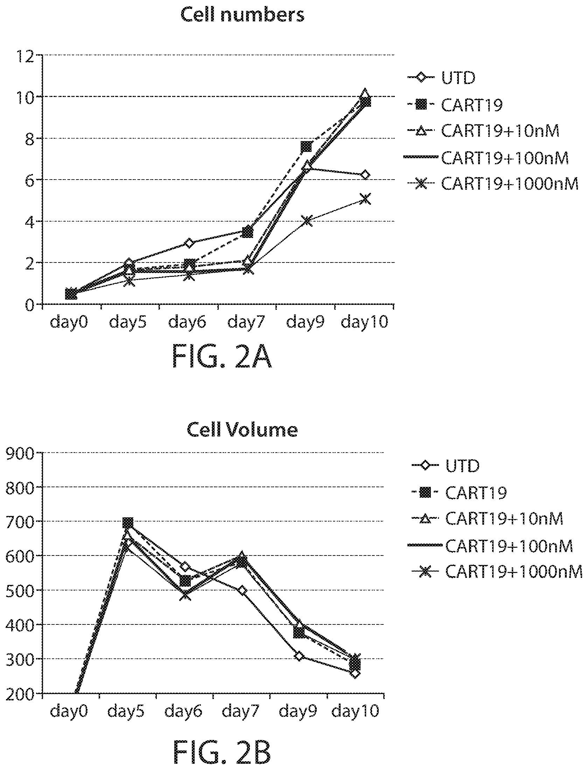

In particular, PBMCs isolated from a normal healthy donor was transduced with a lentiviral anti-CD19 CAR construct as described above. The resulting CAR19-expressing T cells (CART19) were cultured and passaged to determine proliferation and expansion capacity. A 1:1 ratio of CD4:CD8 expressing T cells were cultured culture with or without different concentrations of ibrutinib (10nM, 100nM, and 1000 nM ibrutinib). Ibrutinib was added at each cell passage. The number of cells was counted at day 0, day 5, day 6, day 7, day 9, and day 10 (FIG. 2A). Cell volume was also monitored (FIG. 2B).

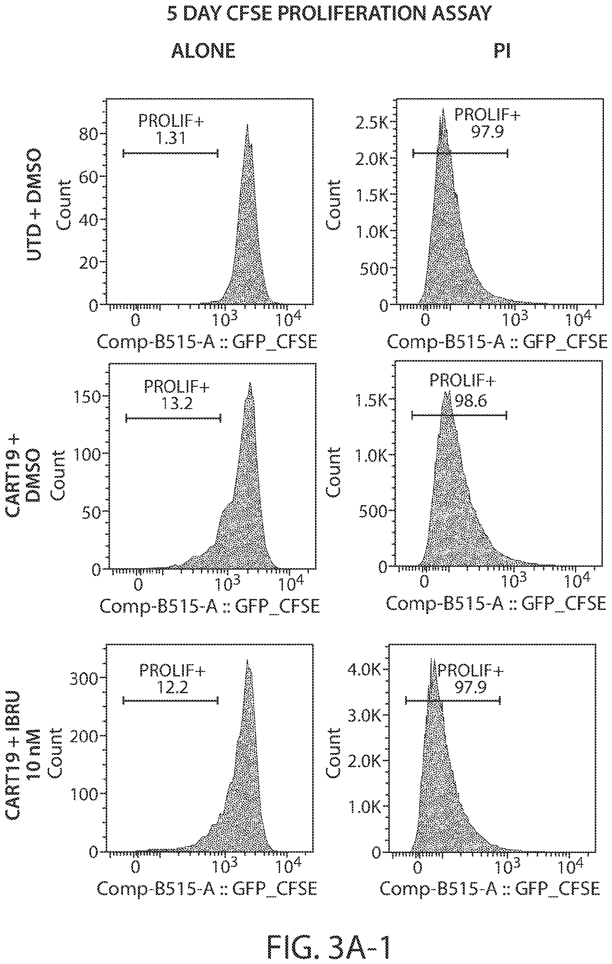

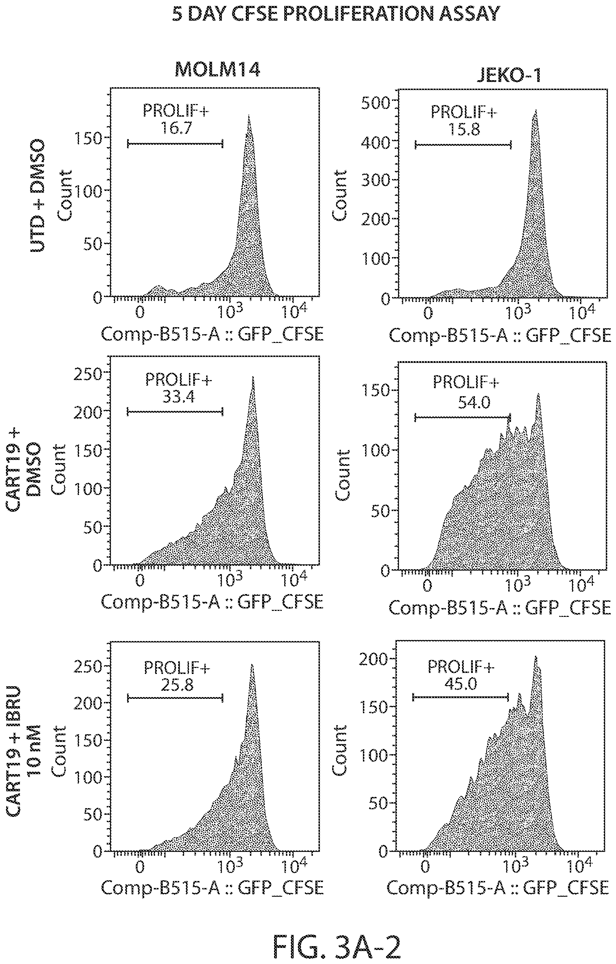

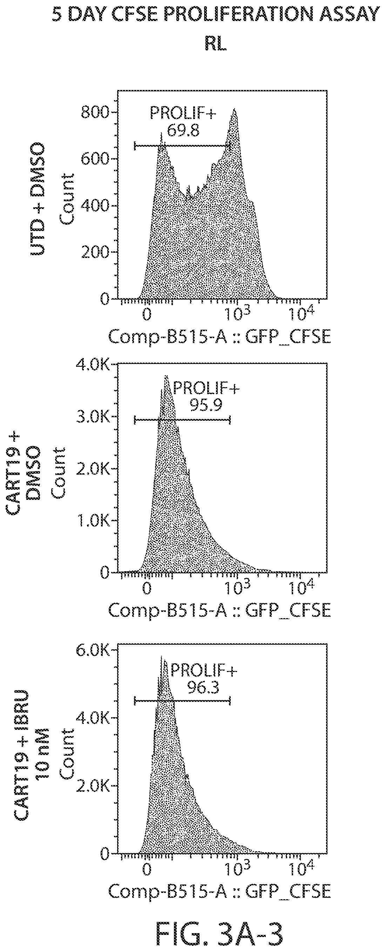

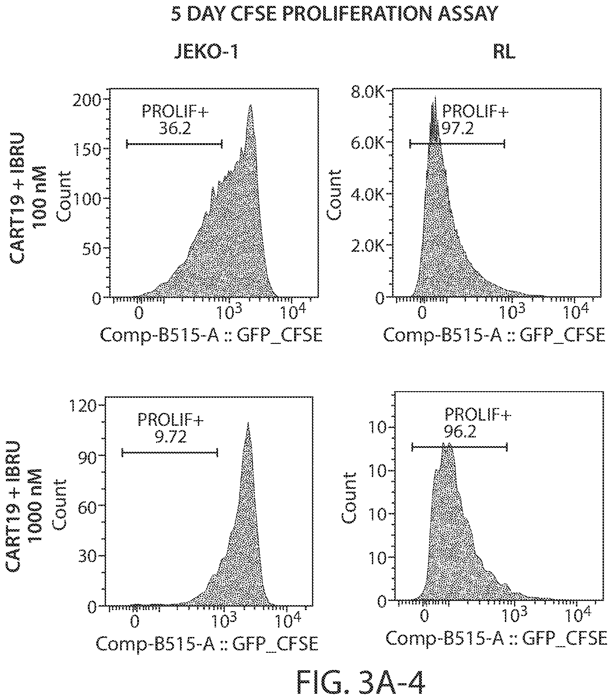

CFSE-staining and flow cytometry analysis was also used to assess proliferation of the CART19 cells in the presence of ibrutinib after stimulation by tumor cell lines MOLM14, JEKO-1, and RL. MOLM14 is an AML cell line, and JEKO-1 and RL are mantle cell lymphoma cell lines. Specifically, the RL cells are a novel MCL cell line derived from neoplastic B-cells obtained from a pleural effusion of a relapsed MCL patient. CART19 cells and tumor cells were mixed in a 1:1 ratio and proliferation was assessed over 5 days. Percentage of proliferating cells are designated in each histogram in FIG. 3A. Quantification of proliferation as shown in FIG. 3B shows that high doses of ibrutinib might inhibit CART 19 cell proliferation during co-culture with MCL cell lines.

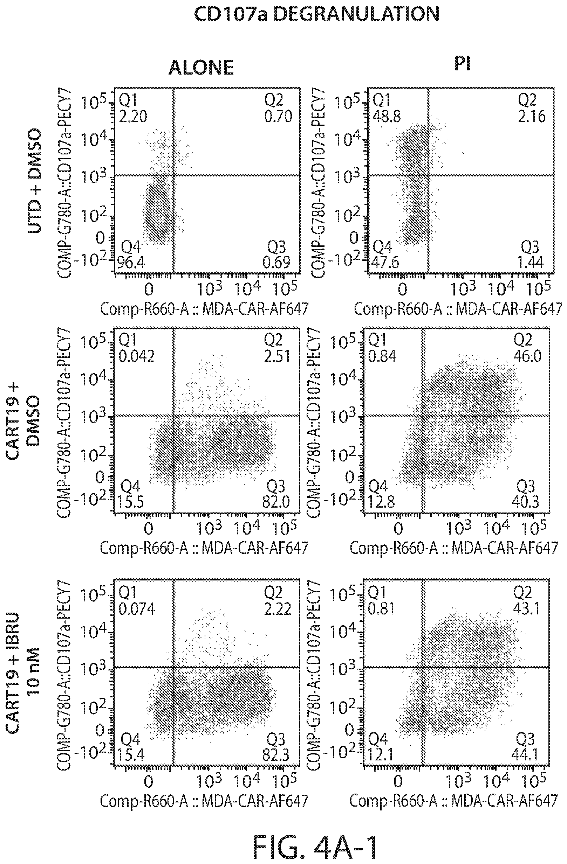

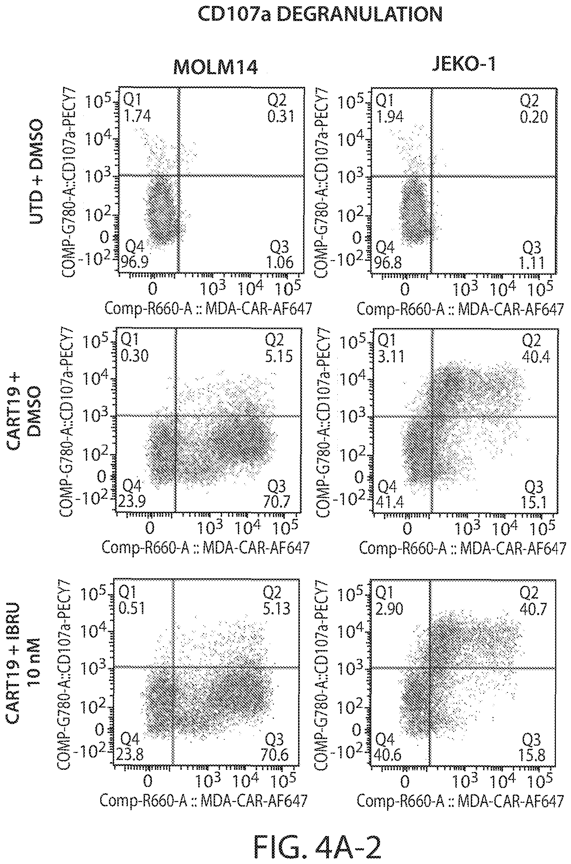

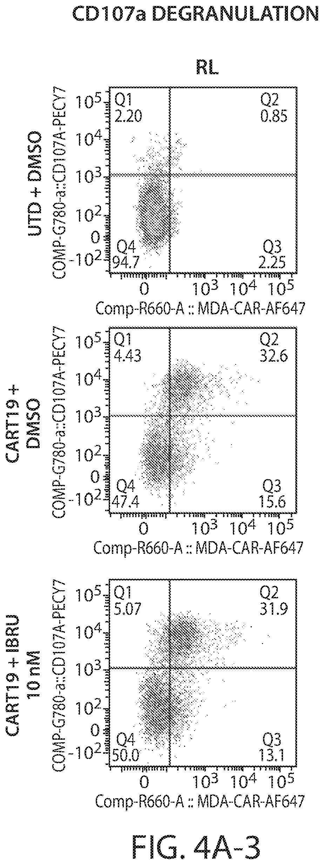

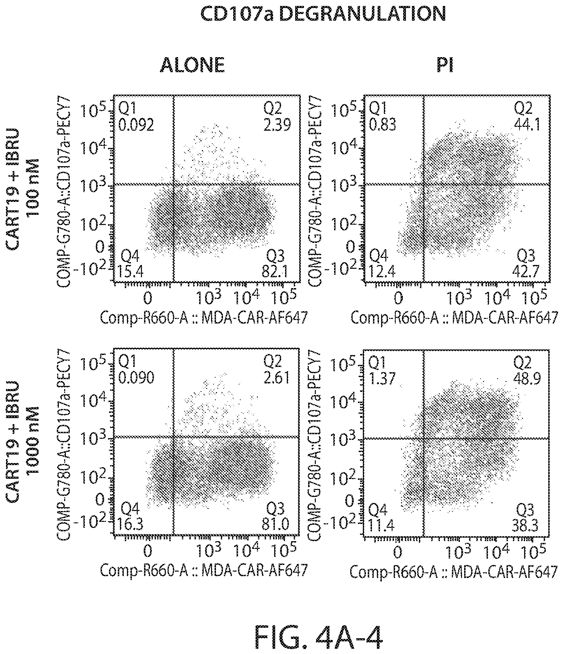

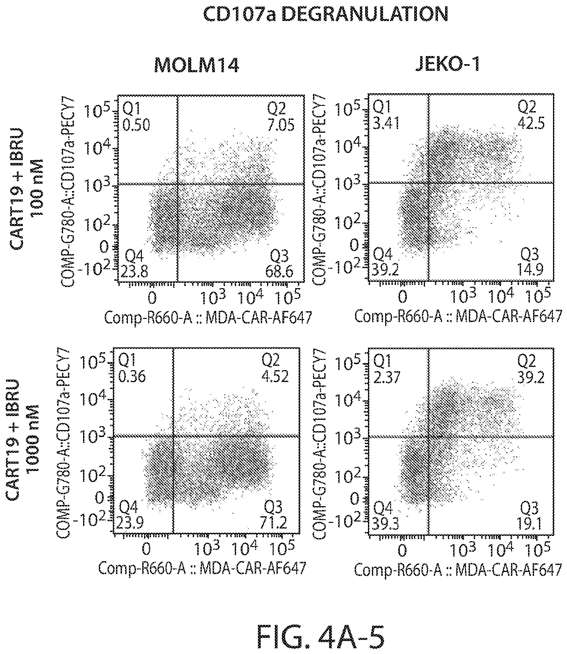

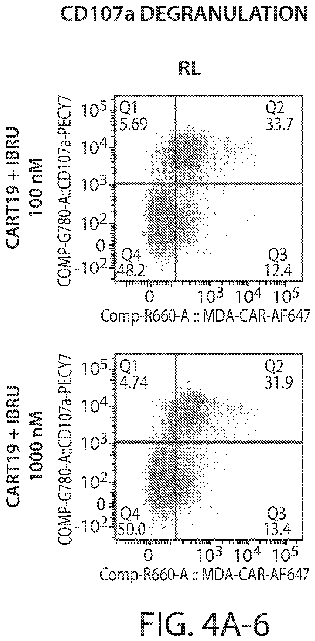

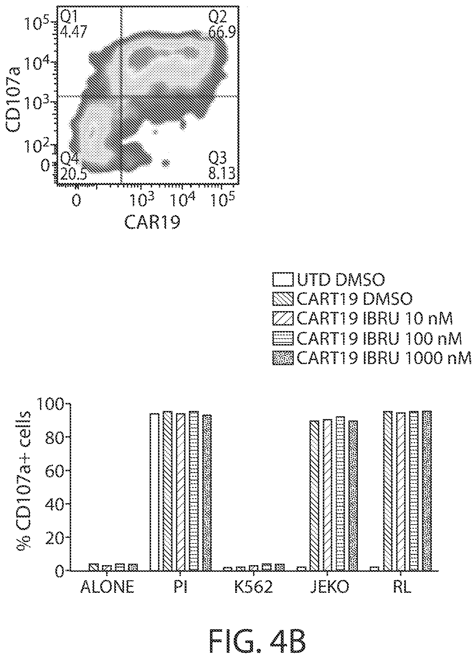

Degranulation of T cells indicates the activation of cytolytic T cells and the ability to initiate antigen-specific cytotoxicity. CD107a is a functional marker of degranulation of T cells transiently expressed on the cell surface after T cell stimulation. Flow cytometry analysis was used to quantify CD107a-expressing CART19 T cells after stimulation with tumor cell lines MOLM14, JEKO-1, and RL. CD107a-expressing cells were present in Q2 (quadrant 2) of the cell profiles shown in FIG. 4A. Quantification of the results obtained from the profiles of FIG. 4A is shown in FIG. 4B. These results indicate that co-culture of CART19 cells with either MCL-RL or JEKO-1 led to massive CAR-specific CD107a degranulation.

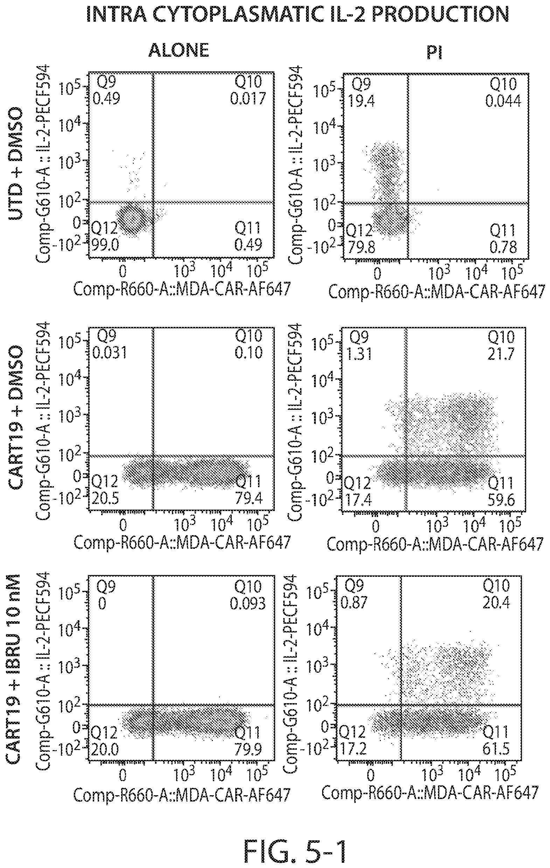

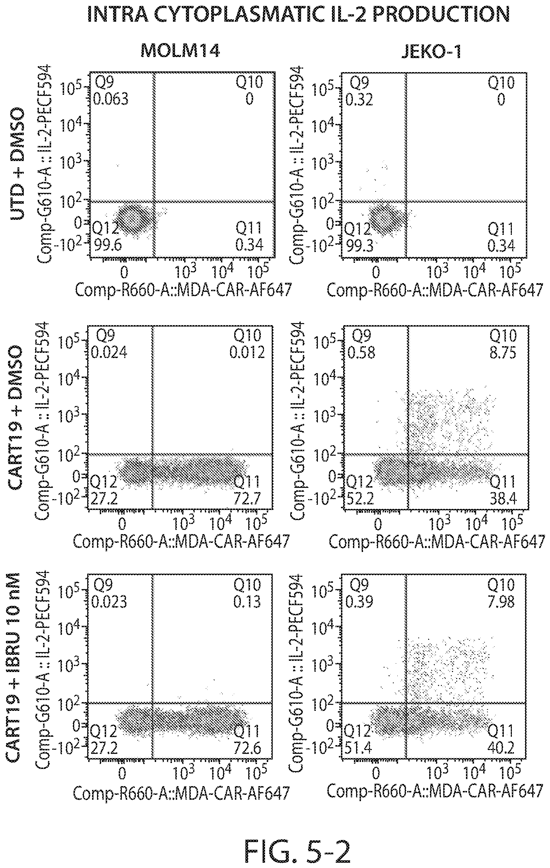

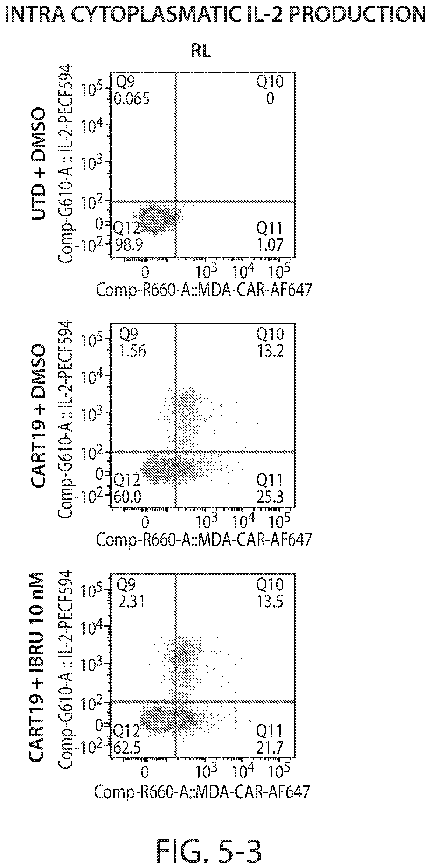

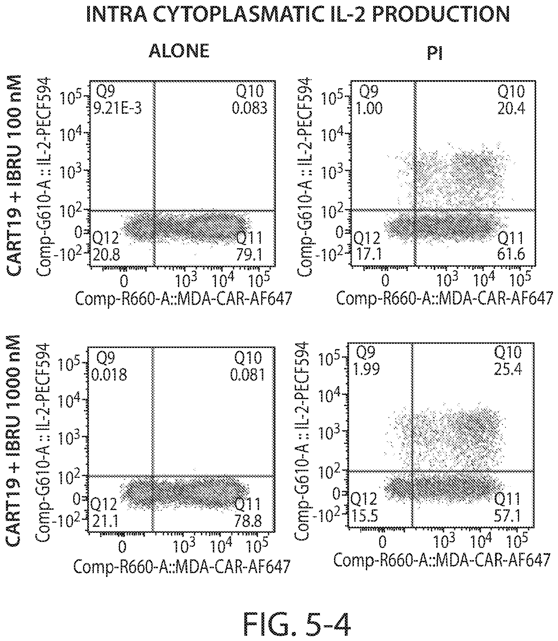

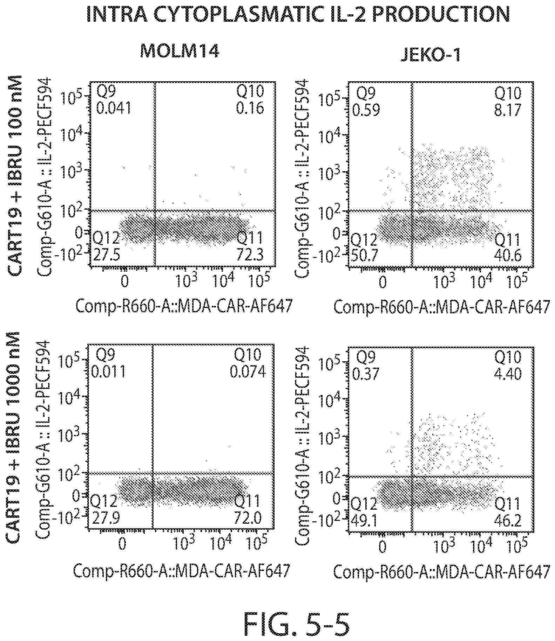

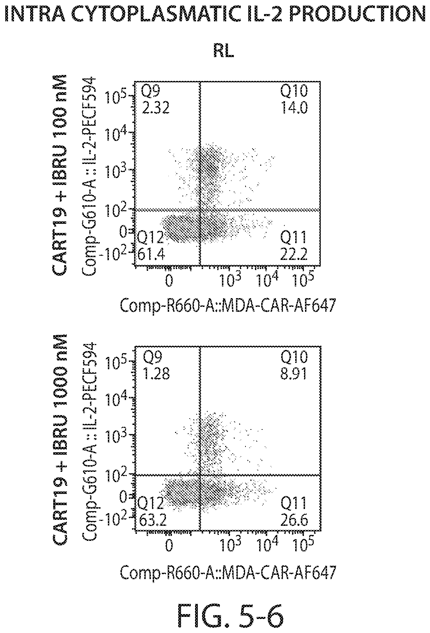

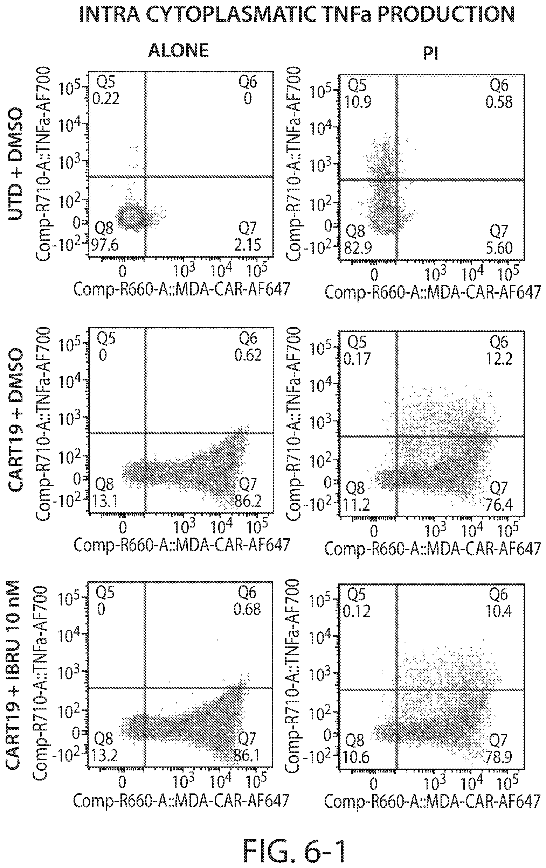

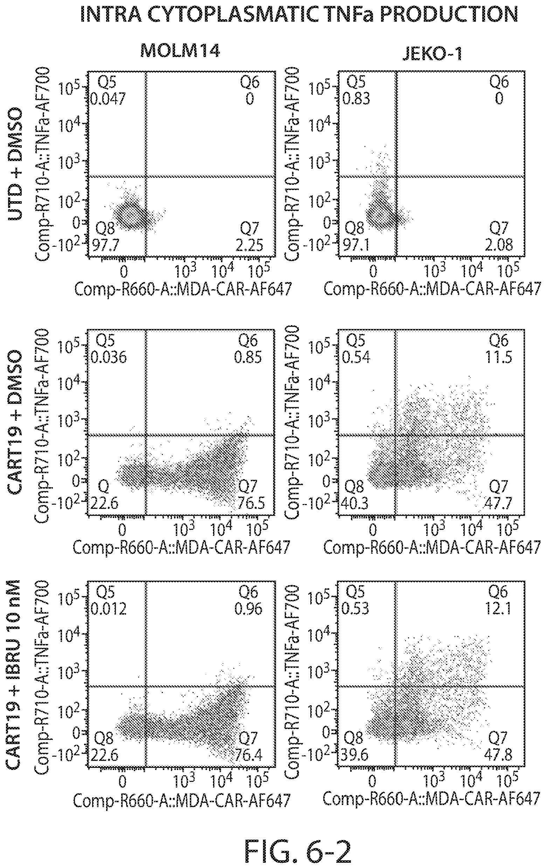

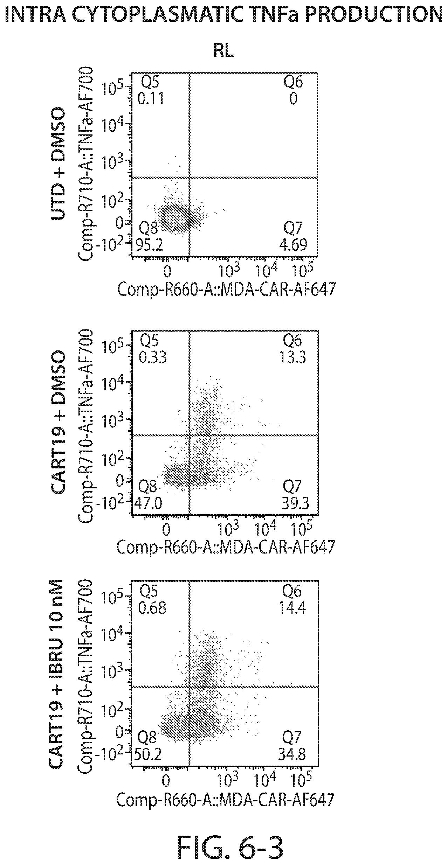

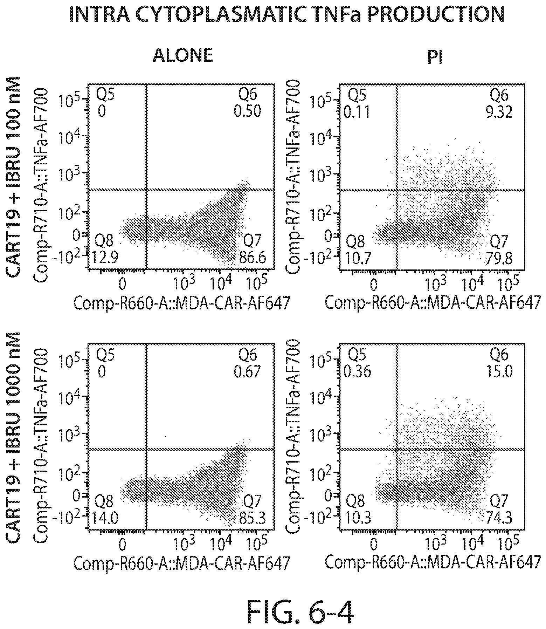

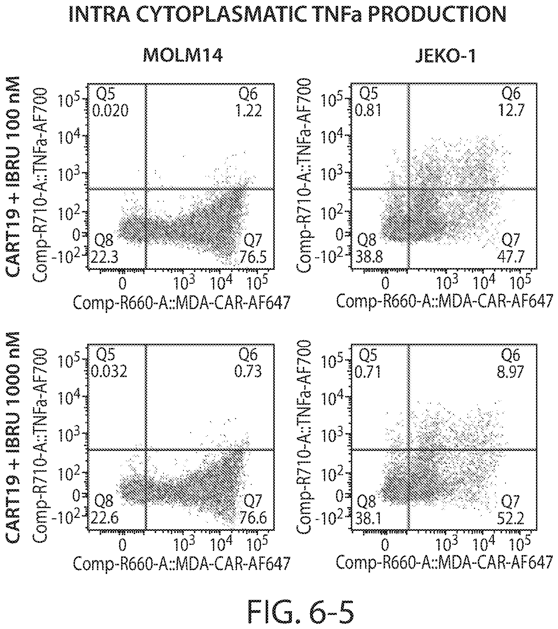

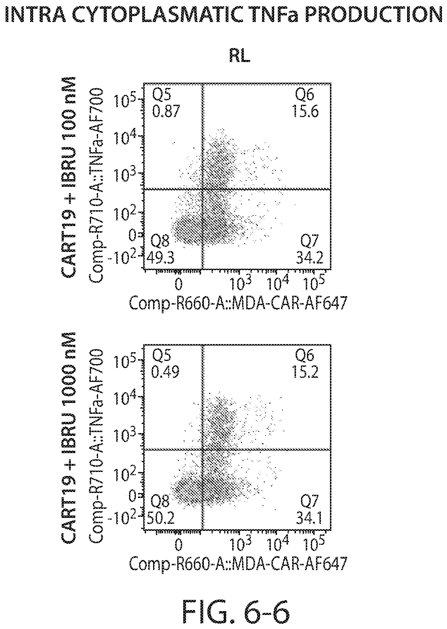

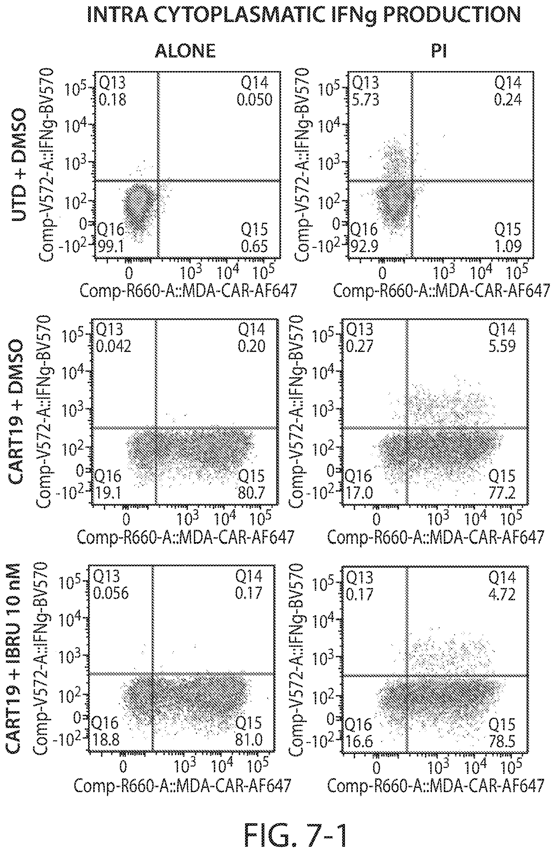

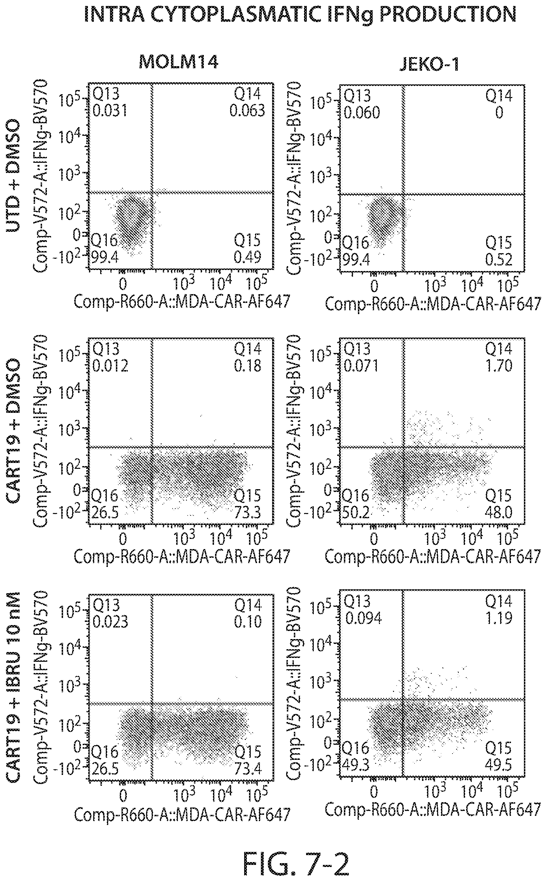

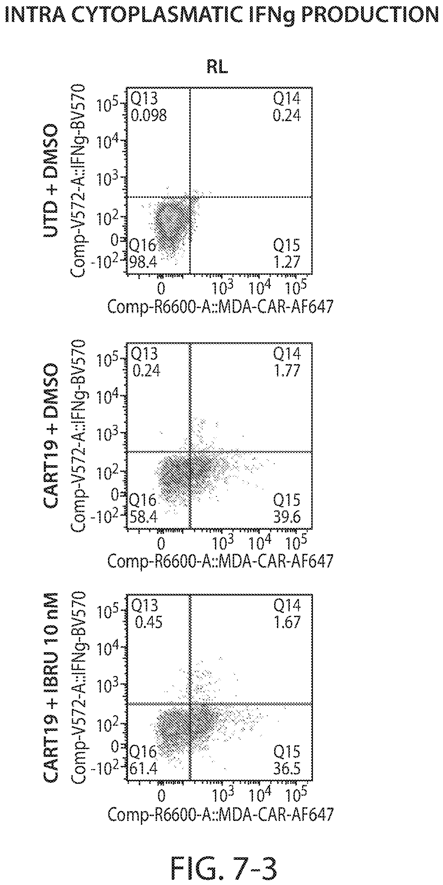

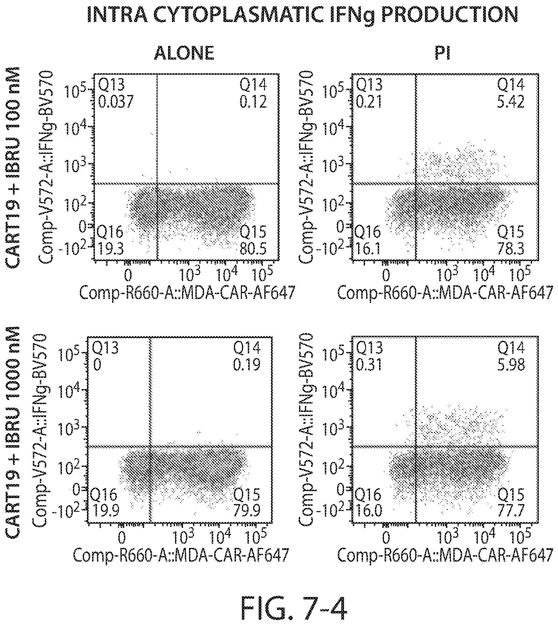

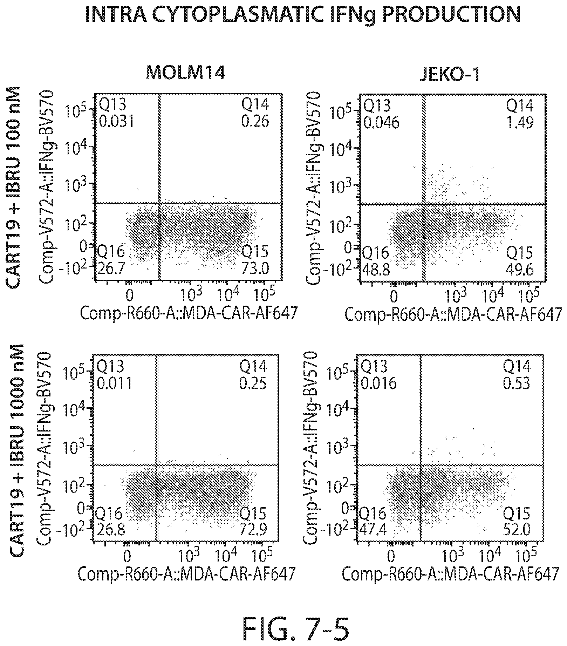

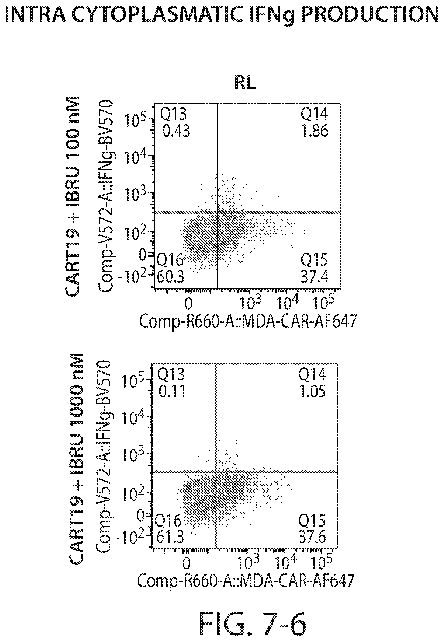

Cytokine production by CART19 T cells in the presence of ibrutinib was also quantified after stimulation by different tumor cell lines. IL-2, TNF-α, and IFN-γ production was assessed by flow cytometry. Cells producing the cytokines are present in quadrant 2 of the profiles shown in FIG. 5, FIG. 6, and FIG. 7. CART19 T cells stimulated by JEKO-1 and RL showed an increase in cytokine expression. Also, increasing concentrations of ibrutinib treatment did not affect the percentage of the CART19 cytokine-producing cells.

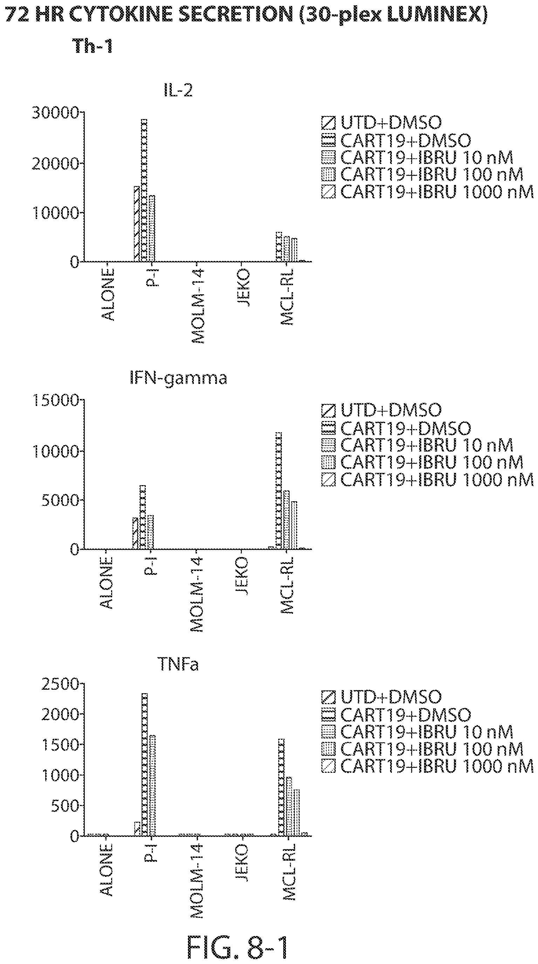

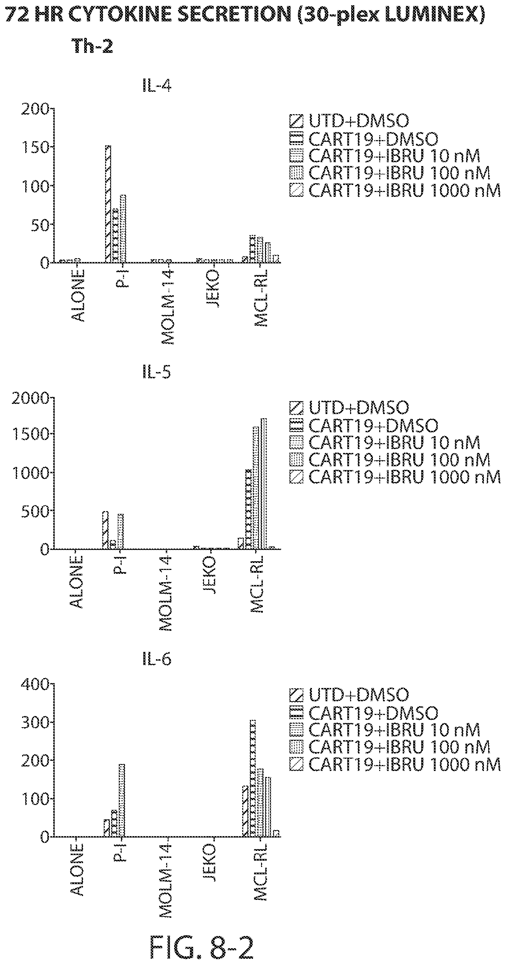

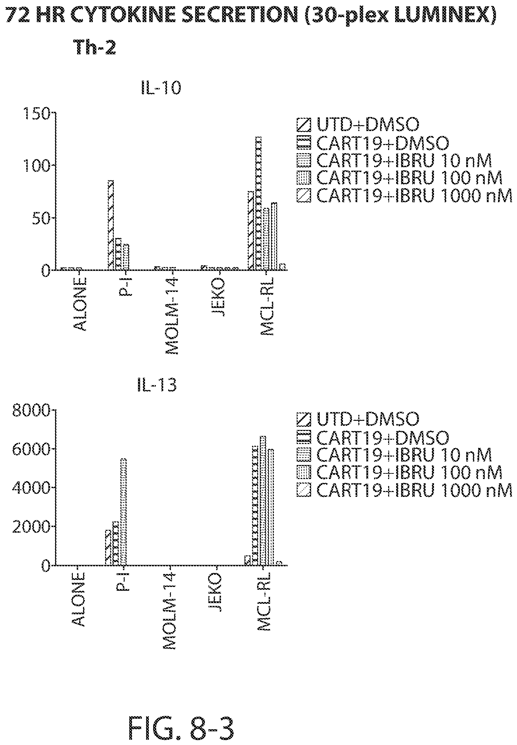

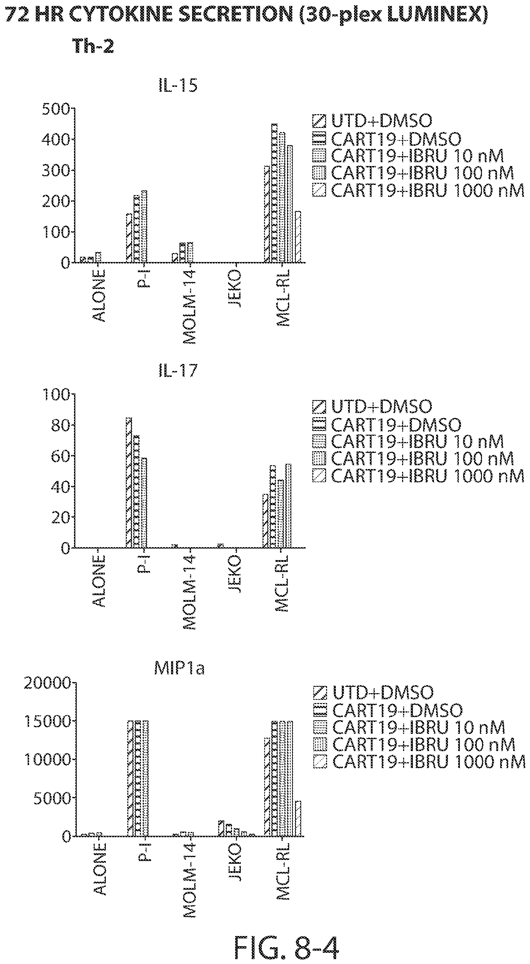

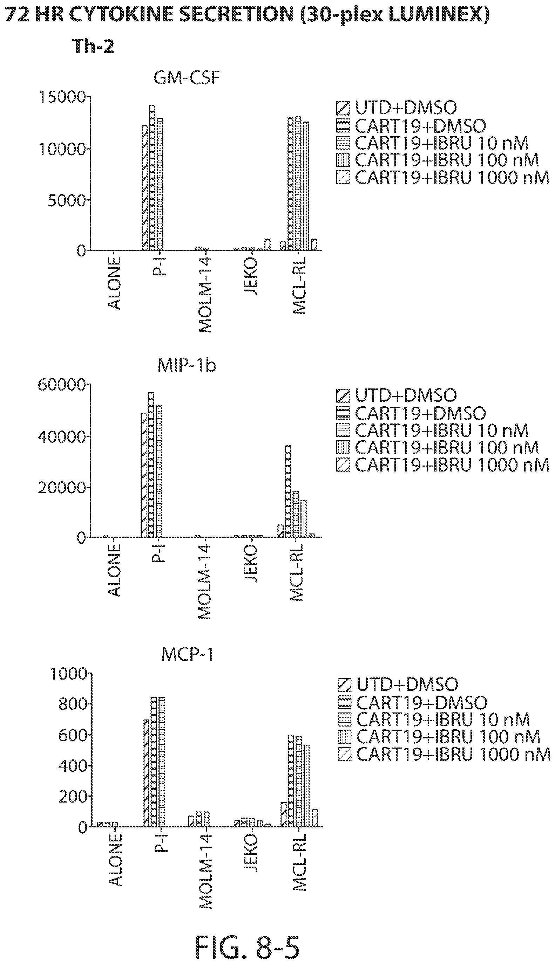

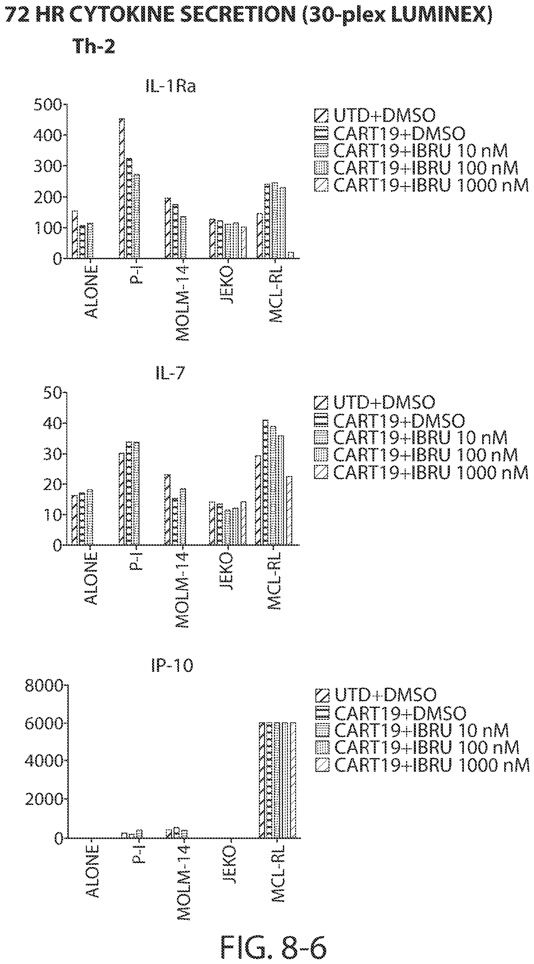

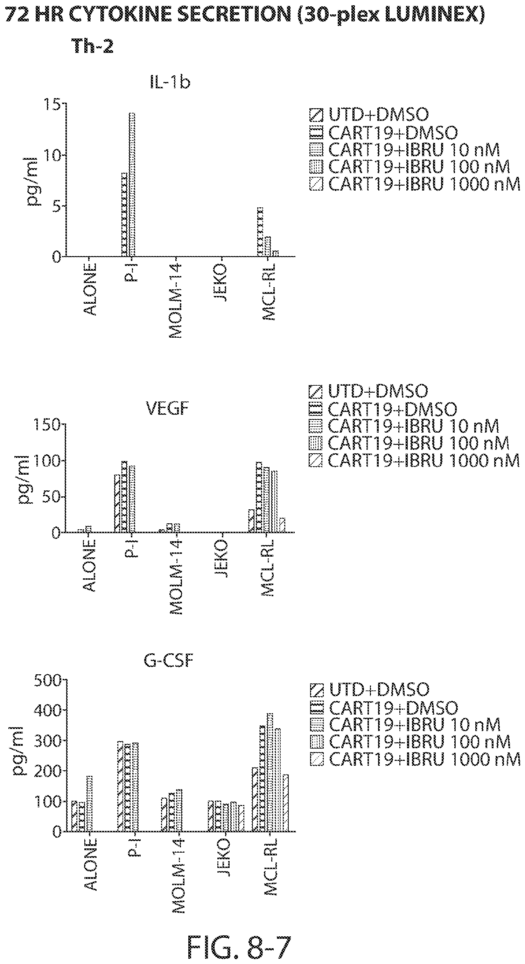

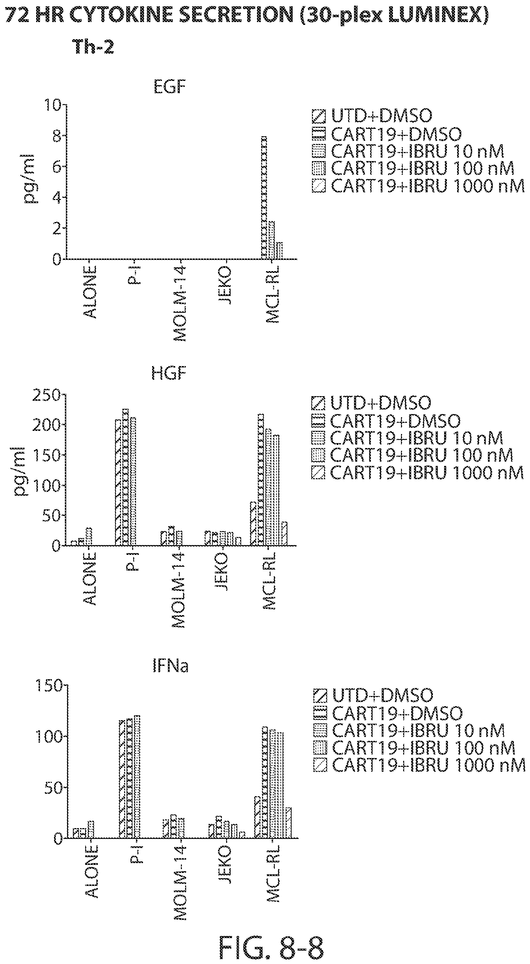

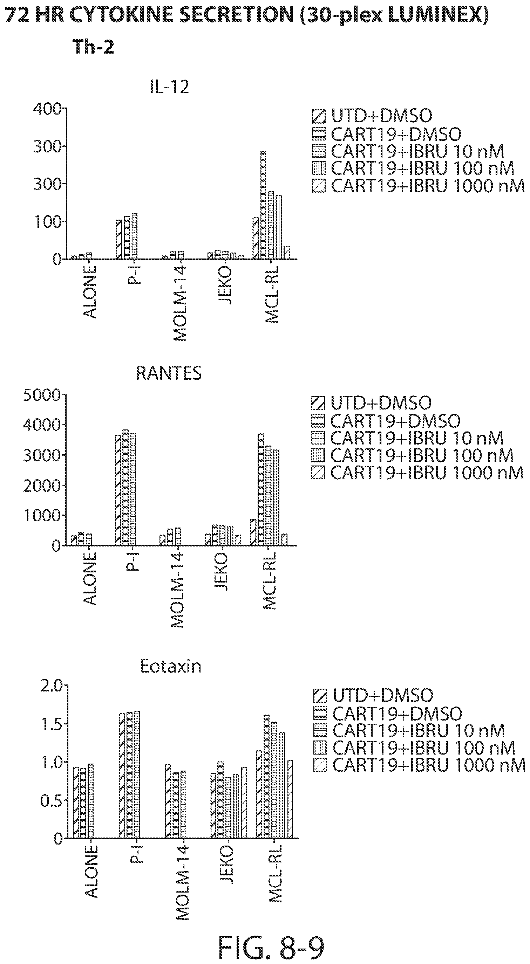

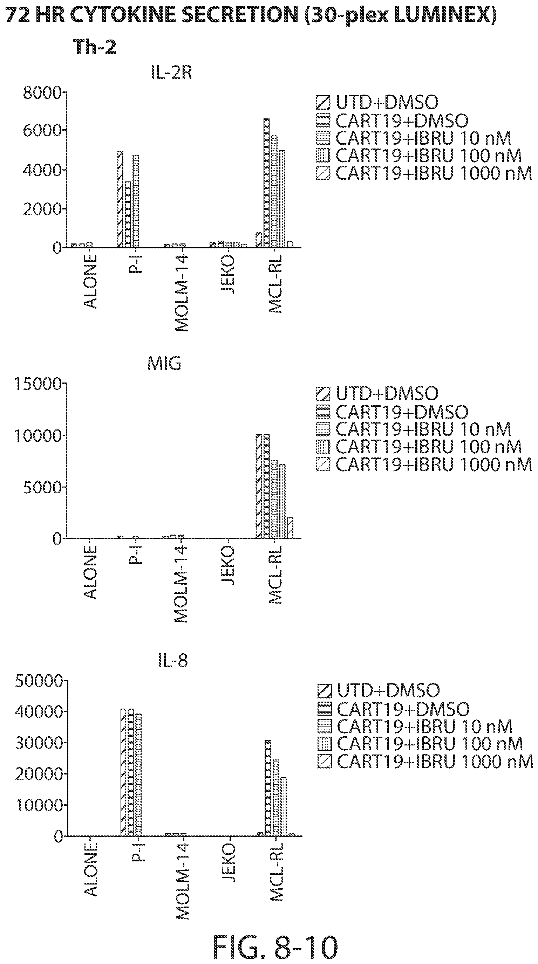

Cytokine secretion from CART19 cells after stimulation by tumor cells and in the presence of varying concentration of ibrutinib was analyzed by 30-plex LUMINEX assay. Cytokines secreted by T_{H}1 cells, such as IL-2, IFN-γ, and TNF-α, was assayed. Cytokines secreted by T_{H}2 cells, including IL-4, IL-5, IL-6, IL-10, IL-13, IL-15, IL-17, MIP1a, GM-CSF, MIP-1b, CP-1, IL-IRa, IL-7, IP-10, IL-1b, VEGF, G-CSF, EGF, HGF, IFNa, IL-12, RANTES, Eotaxin, IL-2R, MIG, and IL-8, was assayed. As shown in FIG. 8, T_{H}1 and T_{H}2 cytokines were secreted by the CART19 T cells.

Also, experiments using two different techniques indicated that there were no differences in Th1/Th2 polarization between ibrutinib-exposed and ibrutinib-unexposed CART19 cells (FIG. 18).

Killing of MCL cells by CART19 cells was augmented in the presence of ibrutinib, suggesting an at least additive effect from the combination (FIG. 18E). However, intrinsic cytotoxic function of CART19 cells was not augmented in the presence of ibrutinib. (FIG. 18F).

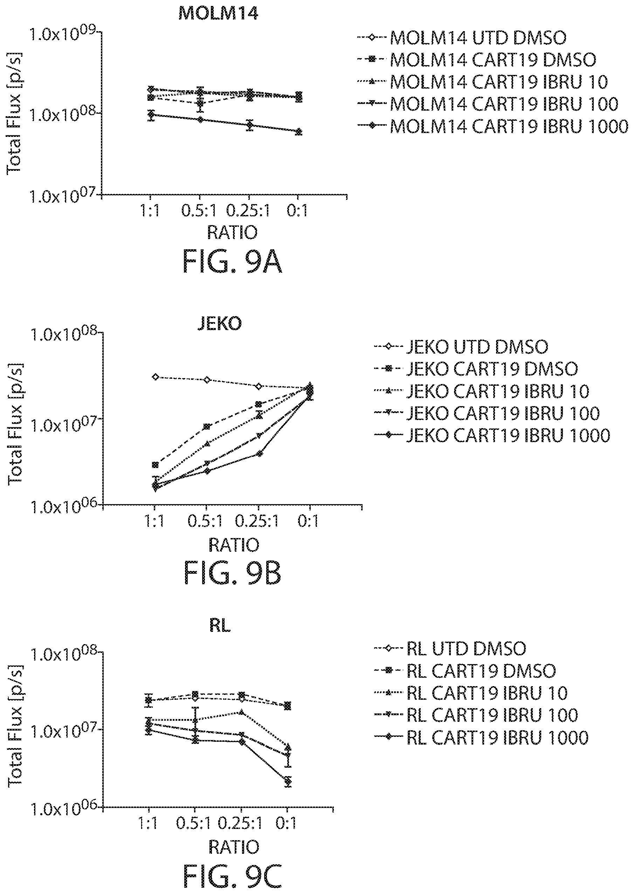

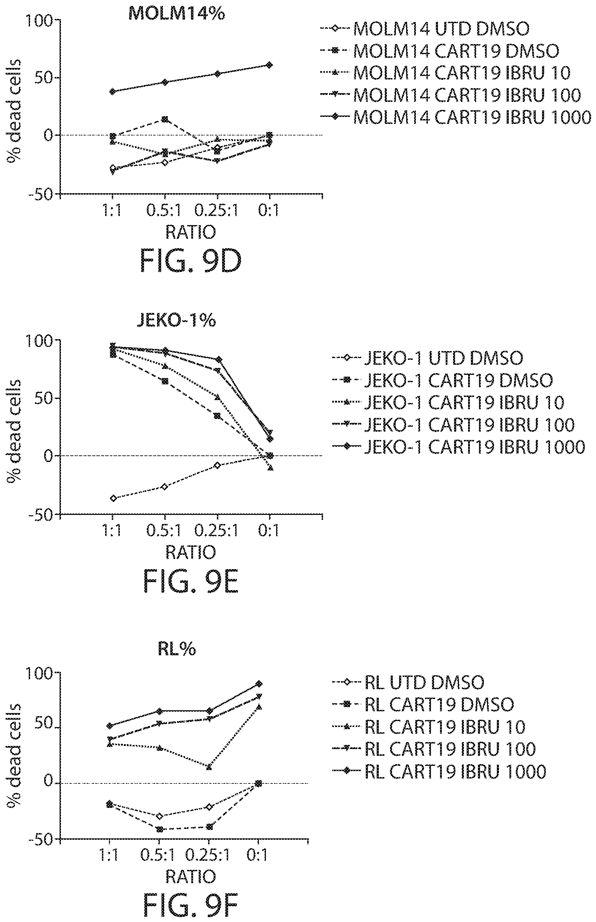

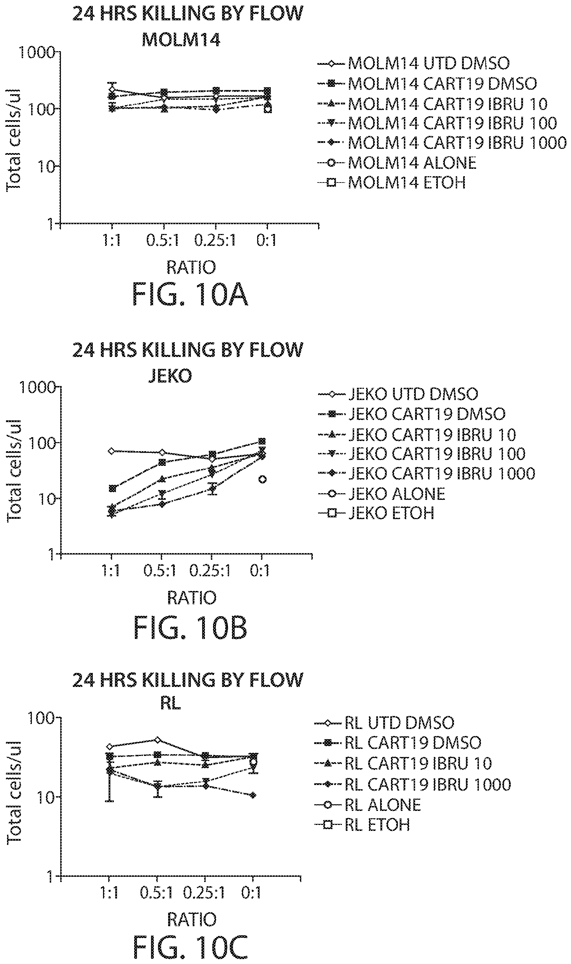

Additional bioluminescence assays were performed to assess CART19 cell killing of tumor cells. CART19 cells were plated with tumor cells MOLM14, JEKO-1, and RL carrying a luciferase reporter in varying ratios, such as 1:1; 1:0.5; 1:0.25; and 1:0 in a 96 well plate, in duplicate. After 24 hours, the bioluminescence was detected and quantified. Results indicated that bioluminescence in MOLM14 samples did not decrease after incubation with CART19 cells. However, for JEKO-1 and RL cells, the bioluminescence decreased in the presence of CART19 cells, indicating that CART19 cells mediated JEKO-1 and RL cell killing (FIG. 9A, 9B, 9C, 9D, 9E, and 9F). There was a further decrease in bioluminescence when treated with ibrutinib, indicating that the combination of ibrutinib and CART19 caused increased JEKO-1 and RL cell killing than with CART19 treatment alone. Consistent with these results, calculation of total cells after each treatment showed that CART19 treatment of JEKO-1 and RL cells caused reduction in cell number and a further reduction when treated with CART19 and ibrutinib (FIG. 10B and 10C), suggesting that the combination therapy was not only efficacious for killing MCL cells, but led to efficient killing of MCL cell lines.

### Assessment of ibrutinib/CART19 treatment in vivo

In these experiments, mouse models of mantle cell lymphoma were used to assess CART19 and ibrutinib combination therapy *in vivo.*

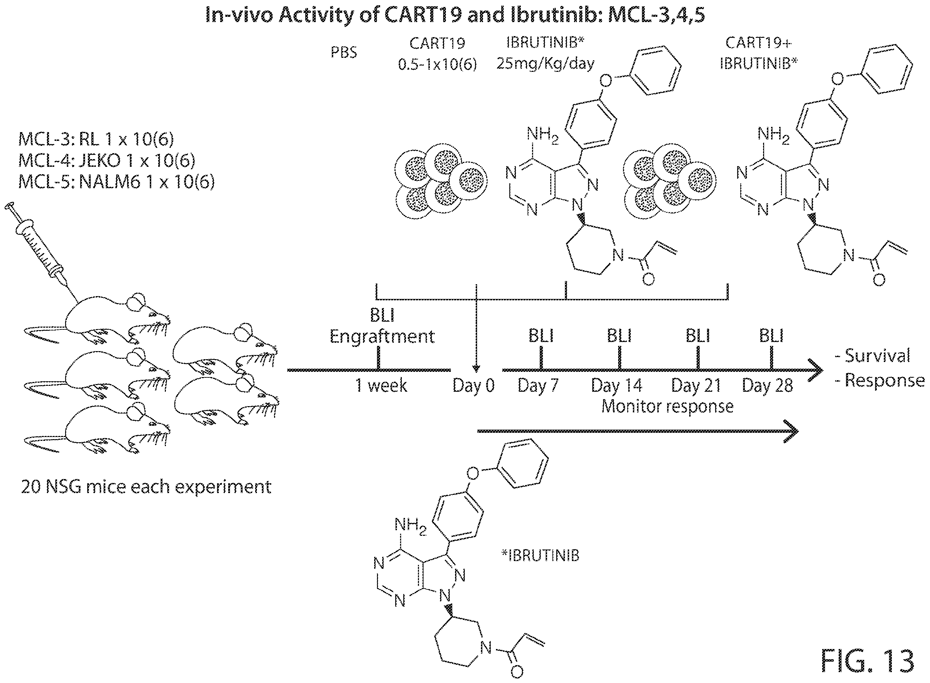

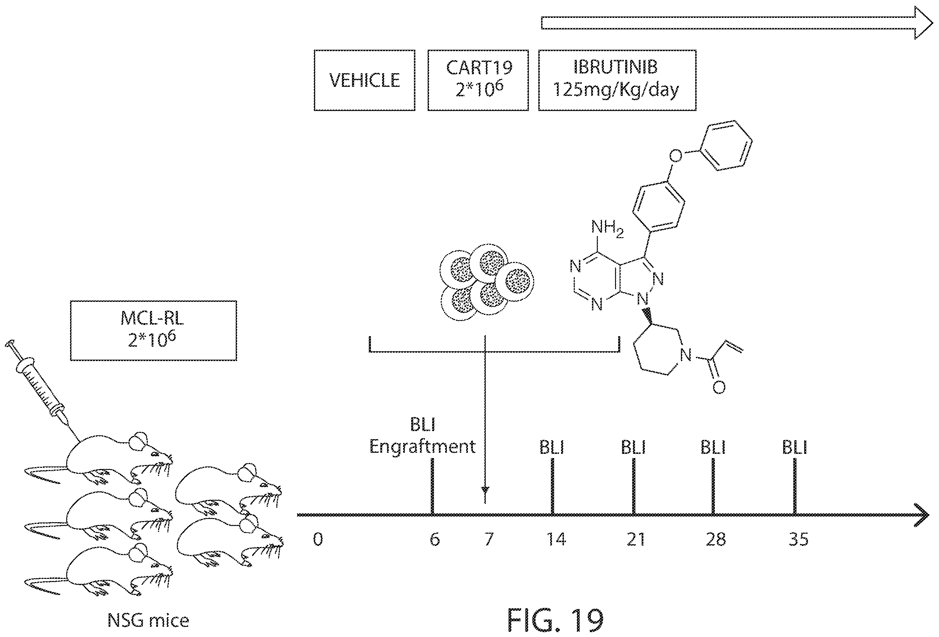

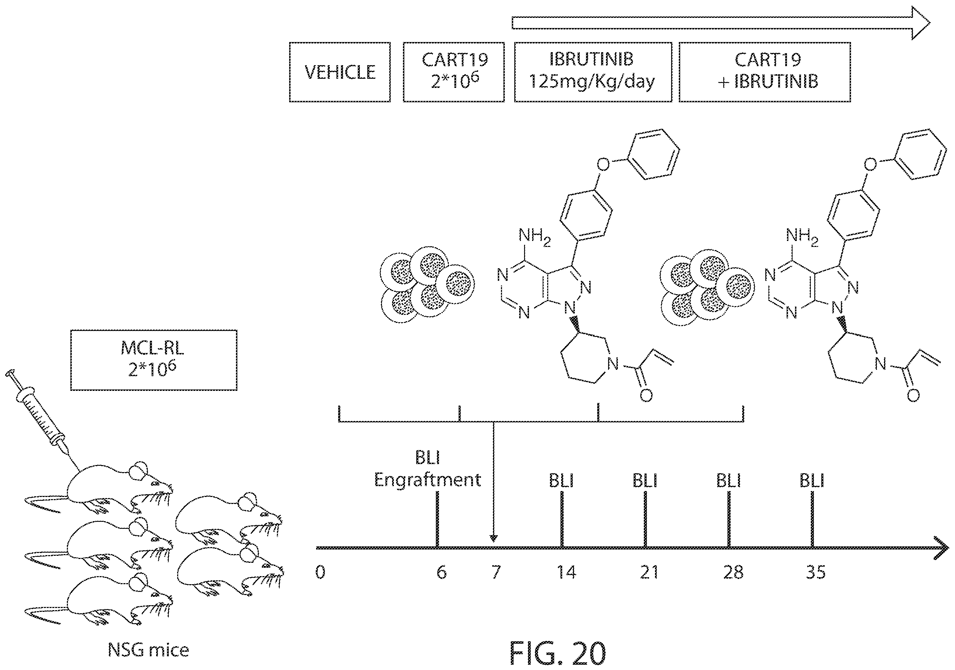

Schematics such as those shown in FIG.13, 19, and 20 were used in this example. FIG. 13 shows a schematic of testing CART19/ibrutinib combination therapy in the *in vivo* mouse models of MCL. 1x10⁶ cells from RL (MCL-3), JEKO-1 (MCL-4), and NALM6 (MCL-5) cell lines are injected in NSG mice (20 mice for each experiment). After 1 week to allow engraftment, treatment is initiated at Day 0, in which the treatment is: 0.5-1x10⁶ CART19 cells (via injection), 25 mg/kg/day ibrutinib (oral gavage), or CART19 + ibrutinib treatment. At Day 7, Day 14, Day 21, and Day 28, the bioluminescence is imaged to monitor tumor size. Mice that are receiving ibrutinib treatment are continuously treated with ibrutinib. Survival of the mice is also monitored. FIG. 19 and 20 show additional schematics of testing CART19/ibrutinib combination therapy in the in vivo mouse models of MCL. 2 x 106 MCL-RL cells are injected into NSG mice. After a week to allow engraftment (engraftment confirmed by bioluminescence imaging), treatment is initiated at Day 7 (after MCL-RL cell injection), in which treatment is: vehicle control, CART19 cells (2 x 10⁶ cells), and/or ibrutinib (125 mg/kg/day). At days 14, 21, 28, and 35 (after injection of MCL-RL cells), the bioluminescence is imaged to monitor tumor size.

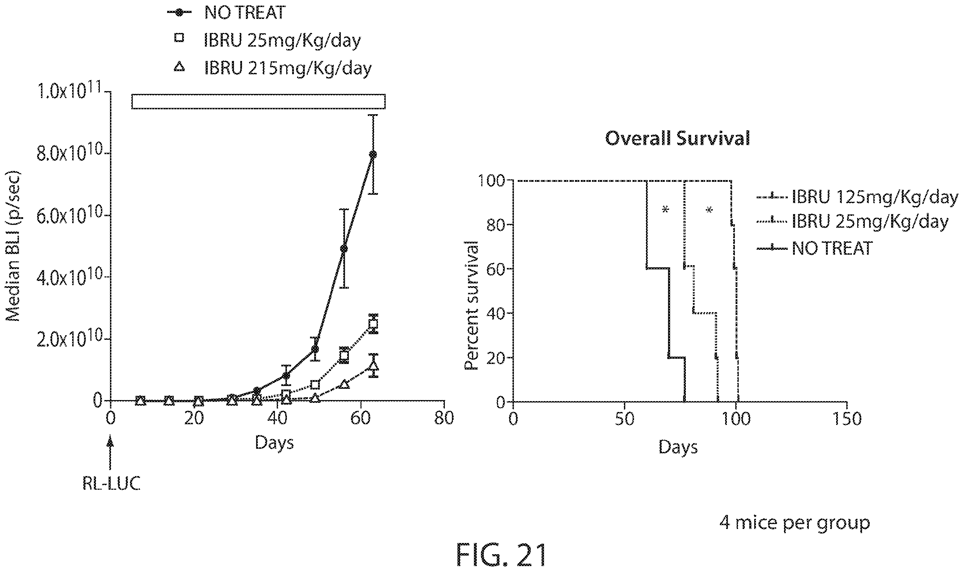

The effect of ibrutinib treatment alone on an *in vivo* mouse model of MCL was examined. RL cells transfected with the GFP/luciferase gene were intravenously injected into immunodeficient NSG mice, resulting in 100% MCL engraftment in liver and spleen, with eventual spread into lymph nodes and bone marrow. Mice were treated with varying doses of ibrutinib, 25 mg/kg/day and 250 mg/kg/day. Mean bioluminescence, representing tumor growth, was assessed at various timepoints. As shown in FIG. 15, RL-derived tumors demonstrated dose-related sensitivity. Additional experiments titrating ibrutinib doses in RL cell-containing mice were performed and are shown in FIG. 21. A higher dose led to a better antitumor activity without increasing toxicity, in line with the higher dose of ibrutinib used in the clinic for MCL (Wang et al. NEJM 369.6(2013):507-16).

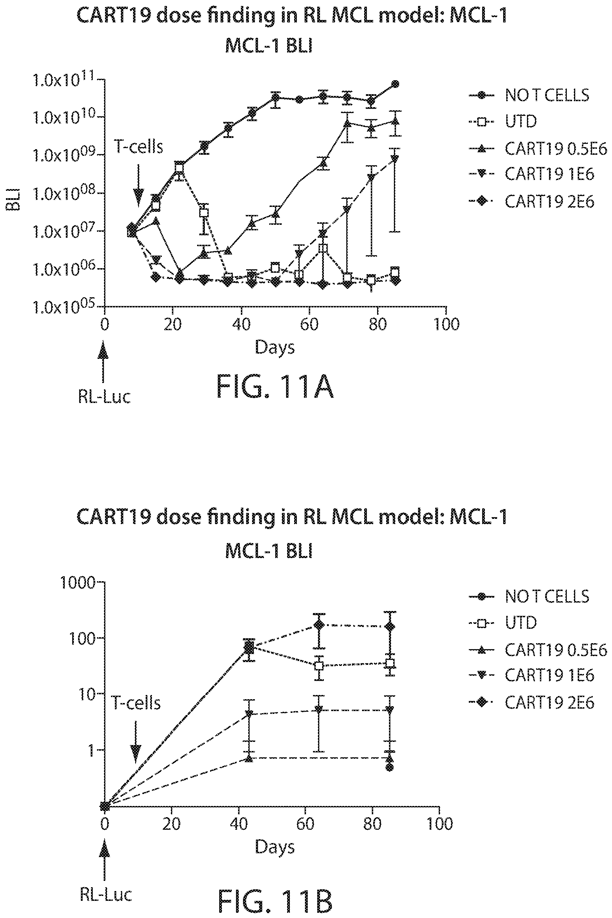

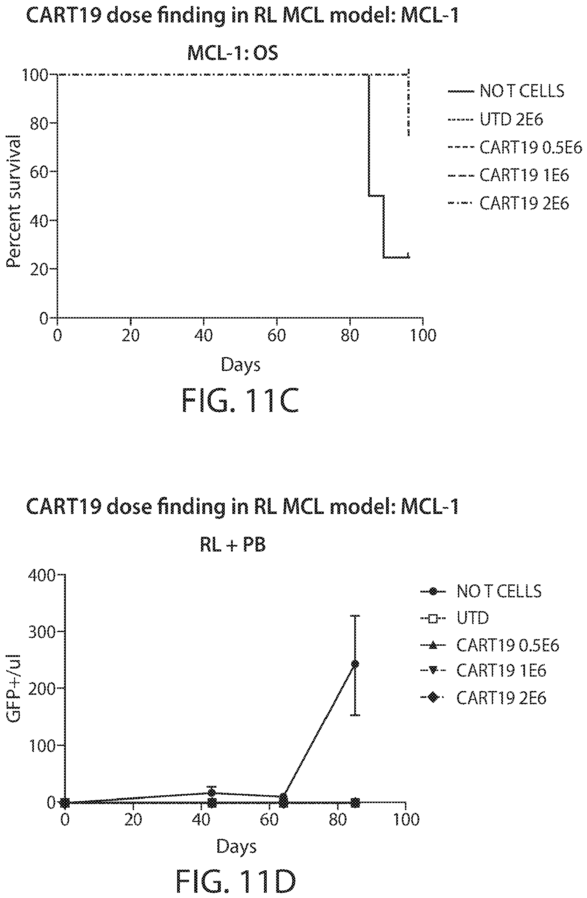

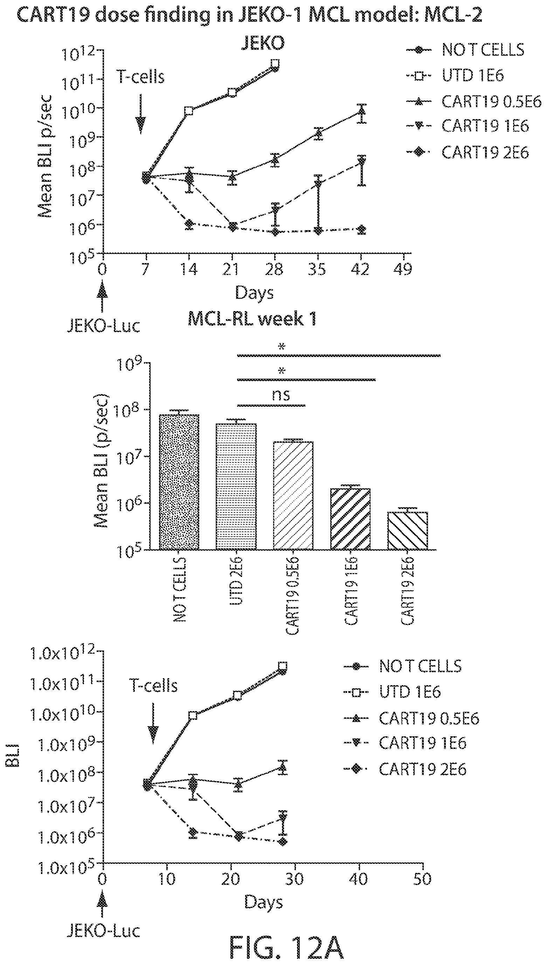

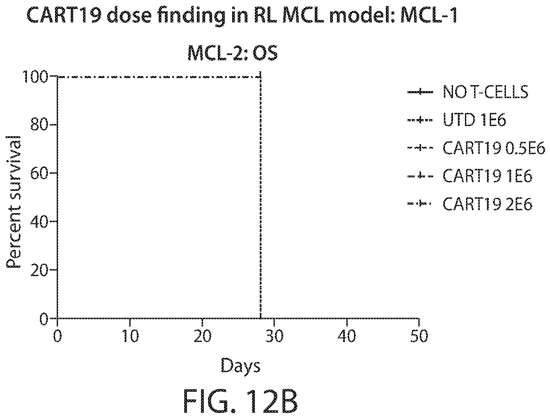

CART19 dose finding was also performed. Two MCL cell lines, RL (MCL-1) and JEKO-1 (MCL-2), carrying a GFP-luciferase reporter were injected into NSG mice at day 0. CART19 T cells were injected at day 7 at varying dosages, for example at 0.5 x e6 cells, 1 x e6 cells, or 2 x e6 cells. Mice were monitored, for example, for 100 days. At various timepoints, the mice were monitored for tumor size (e.g., bioluminescence imaging) (FIG. 11A and 11B, and FIG. 12A), and for overall survival (e.g., Kaplan-Meier survival curve) (FIG. 11C and FIG. 12B). Different doses of CART19 cells showing a dose-dependent anti-tumor efficacy, with 2 x 10⁶ CART19cells/mouse being the most effective dose (FIG. 12A).

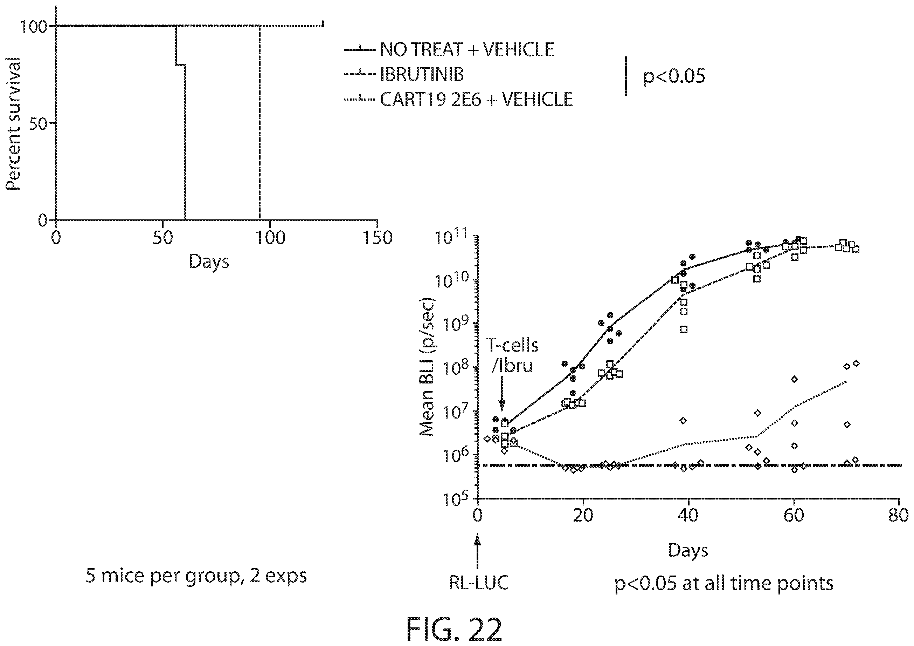

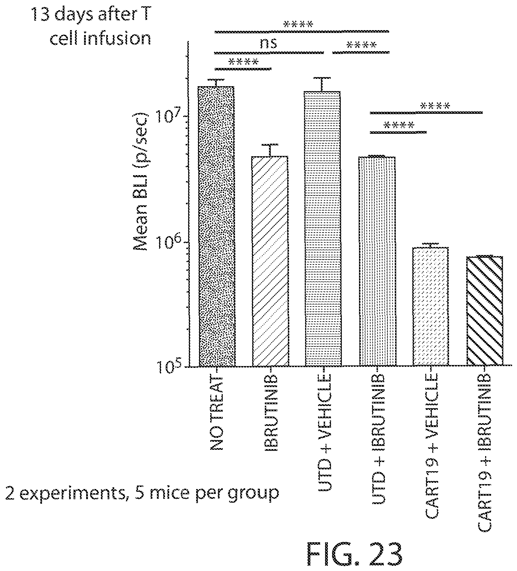

These studies provided an opportunity to conduct a head-to-head comparison of the two of therapies for MCL. As shown in FIG. 22, long-term survival was achieved only in mice treated with CART-19 cells. There was no difference in anti-tumor effect when comparing untransduced T cells plus ibrutinib with ibrutinib alone. Therefore, in all subsequent experiments, the control groups were vehicle and ibrutinib alone (FIG. 23).

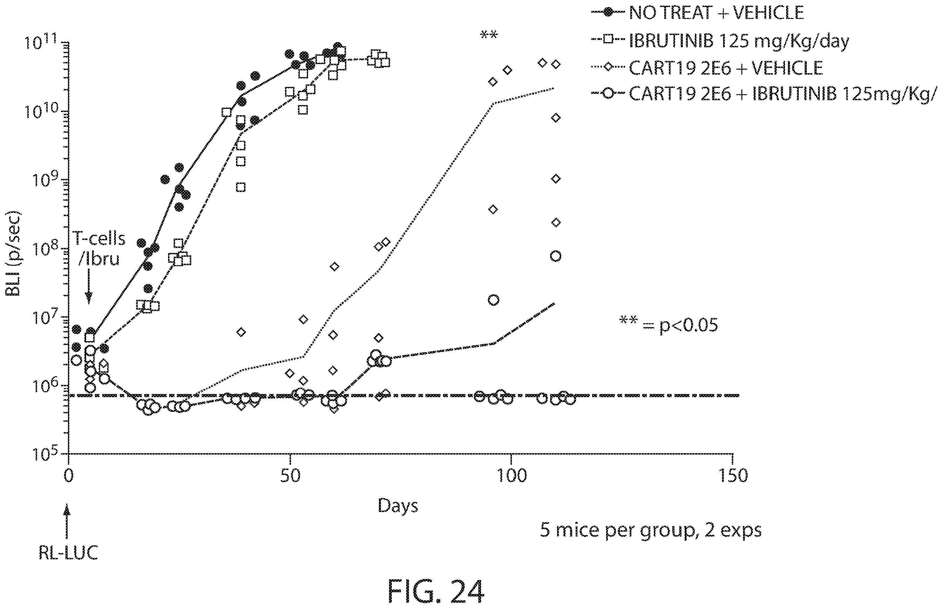

The addition of CART19 to ibrutinib was also tested, as detailed in the schematic in FIG. 20. Evaluation of the effect of ibrutinib on tumor burden indicated modestly delayed tumor growth at early time points. In contrast, CART19 cell therapy led to a clear decrease in tumor burden for several weeks. In mice receiving CART19 cells alone, this was followed by an indolent relapse beginning at Day 40, whereas mice that were treated with CART19 cells as well as ibrutinib had no detectable disease until Day 80 (FIG. 24). Histopathology of organs harvested at the end of the experiment showed persistence of disease in all control and ibrutinib treated mice with foci of tumor necrosis in the ibrutinib-treated. Most of the mice treated with CART19 alone showed indolent relapse at long term that was companied by the persistence of CART19 cells, while mice treated with CART19-ibrutinib showed clearance of the tumor and disappearance of CART19 from involved organs (data not shown).

### Mechanism of combined effect of CART19 and ibrutinib

The in vitro experiments herein indicated that ibrutinib neither impaired nor clearly augmented short-term CART19 effector functions. The in vivo studies showed that ibrutinib monotherapy had a modest anti-tumor effect. The results indicated that ibrutinib could significantly enhance the anti-tumor function of CART19 cells (FIG. 24). Therefore, experiments were performed to determine the mechanism for this effect.

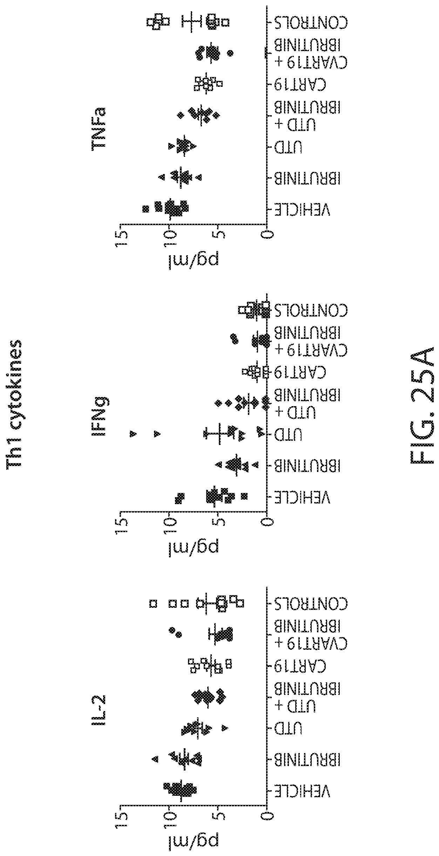

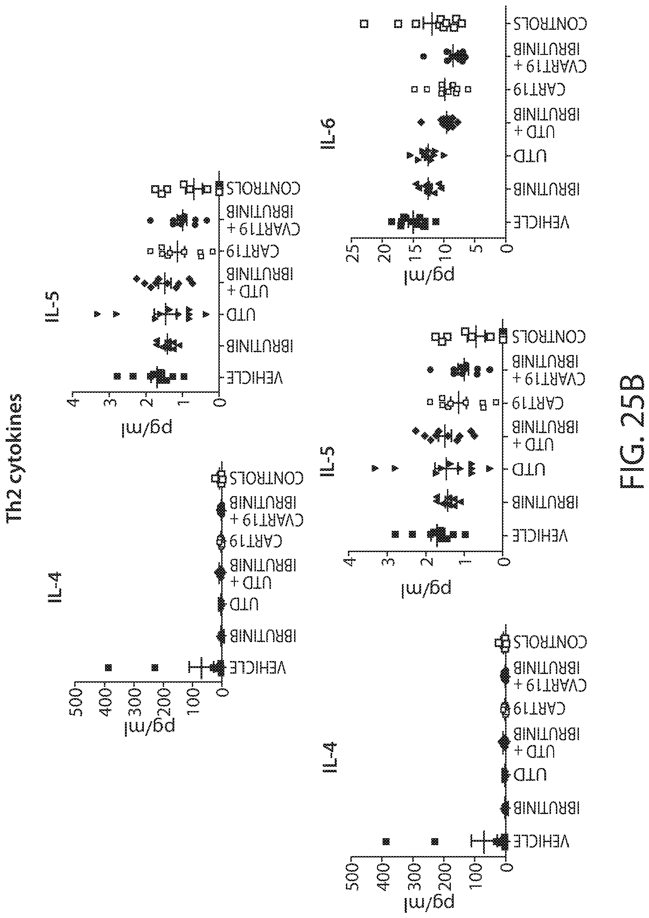

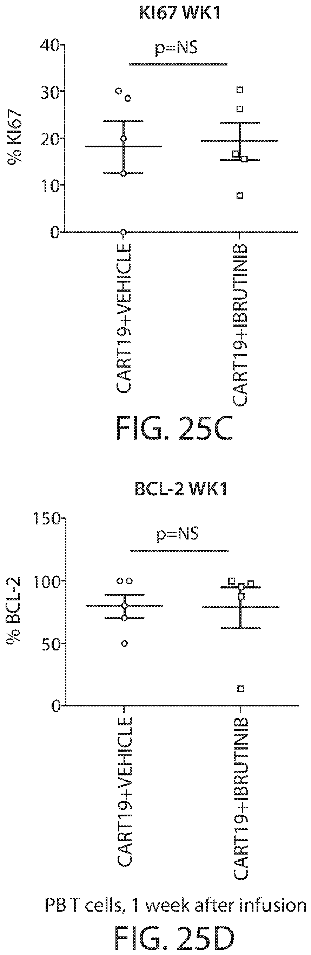

Inhibition of ITK has been shown to inhibit Th2 polarization and skew towards a Th1 phenotype (Dubovsky et al. Blood 122.15(2013):2539-49). In mice treated with CART19 cells and ibrutinib, an increase in Th1 cells when compared with CART19 cell monotherapy was not observed using this assay (FIG. 25A, 25B). However, exposure of mice to ibrutinib led to an increase in peripheral CART19 cells. There was no difference in the proliferation marker Ki67 between the treatment group and the control group (FIG. 25C), so this assay did not detect a difference in proliferation. Similarly, there was no difference in the anti-apoptotic marker Bcl2 or the apoptosis marker phosphotidyl serine, suggesting that the difference in CART cell numbers was not related to an impairment of apoptosis (FIG. 25D). As ibrutinib has been associated with peripheral lymphocytosis in patients, experiments were done to determine whether this was also found in mice treated with ibrutinib alone. One week after beginning ibrutinib, there were more circulating MCL cells and fewer nodal/organ MCL cells in ibrutinib-treated mice. Interestingly, this increase was observed in both ibrutinib-sensitive and -resistant in vivo model, as it was also observed in NSG mice engrafted with the acute leukemia cell line NALM-6 (data not shown).

In order to understand the role of ibrutinib in T cell expansion in vivo we engrafted NSG mice with MCL-RL WT cells and treated them with luciferase-positive T cells. Both CTL019 and CTL019-ibrutinib treated mice showed intense T cell expansion compared to UTD or UTD-ibrutinib (data not shown). We then investigated the frequency of different T cells subsets in vivo and did not see differences in PB T cells 1 week after T cells infusion, (data not shown). Since CXCR4 is involved in ibrutinib-driven B cell mobilization in humans, we checked the expression of CXCR4 in vivo in PB T cells of mice treated with CTL019 or CTL019-ibrutinib: CXCR4 level were similar in the 2 groups (data not shown). Lastly, expression of inhibitory/costimulatory receptor on PB of T cells of mice treated with CART19 and CART19-ibrutinib was analized. No difference in expression of TIM3, LAG3, CD137 or CTLA4 was evident, however a trend to a reduced PD-1 expression was noted in mice treated with CTL019 and UTD combined with ibrutinib.

### Conclusions

Therapies for B-cell malignancies include small molecule inhibitors of BCR signaling and CD19-directed T cell based therapies. In the setting of relapsed MCL, the BTK inhibitor ibrutinib is now approved by the FDA and engenders high initial response rates. Unfortunately, these responses tend to be transient and require higher drug doses than those used for CLL. CART-19 leads to durable responses in patients with high-risk B-ALL, and it may be efficacious in other B-cell malignancies as well. Preliminary data suggest that the responses of mature B-cell malignancies to CART-19 may be lower than those of B-ALL, but the mechanism of this disparity has not yet been ascertained. This example investigated the impact of adding ibrutinib to CART19 in the treatment of MCL.

Different MCL cell lines with variable sensitivities to ibrutinib (IC50 ranging from 10 nM to 10 µM) were used for in vitro experiments. These different cell lines were used to model both ibrutinib-sensitive and ibrutinib-resistant MCL. At all but the highest doses of ibrutinib, CART19 cell function was unimpaired, with intact T cell expansion kinetics, tumor recognition and killing, and cytokine production. Furthermore, the results did not reveal a T helper polarization upon ibrutinib exposure. This finding may be due to a combination of factors, including the use of a mixed culture of CD4 and CD8 cells, in contrast to the model of CD4-only experimentation performed by Dubovksy et al. Blood 122.15(2013):2539-49. Both ibrutinib-sensitive and ibrutinib-resistant cell lines strongly activated CART19 cells and induced killing, cytokine production and proliferation. Combination of CART19 and ibrutinib in vitro led to at least additive tumor killing. The results in this example show a superiority of CART19 over ibrutinib when each was used as monotherapy at clinically relevant doses and schedules of administration (single dose for CART19, continuous administration for ibrutinib).

A systemic xenograft MCL model was also generated in this example, using the MCL-RL cell line generated in a laboratory. Treatment of these mice with different doses of allogeneic CAR19 T cells led to a dose dependent anti-tumor effect. A similar dose response to CART-19 was also observed in the ibrutinib resistant JEKO-1 cell line. MCL-RL was treated in vivo with different doses (e.g., 0, 25 and 125 mg/kg/day) of Ibrutinib, leading to a median overall survival respectively of 70, 81 and 100 days (p<0.001). A direct in vivo comparison of the ibrutinib 125mg/kg and CART19 showed a significantly improved tumor control for CART19 treated mice. Also, MCL-RL engrafted mice were treated with vehicle, ibrutinib, CART19 or the combination of CART19 and ibrutinib (iCART19). At clinically relevant doses, monotherapy of MCL with CART19 was superior over monotherapy with ibrutinib, and the combination of ibrutinib with CART19 led to an augmented anti-tumor effect. In particular, the iCART19 combination in vivo led to initially higher circulating levels of CART19 cells, followed by deep tumor responses, and relapses were significantly delayed when ibrutinib was added to CART19. The iCART19 combination resulted in an improved tumor control with 80% of mice reaching complete remission and long-term disease-free survival. Mechanistically, mice treated with ibrutinib had higher numbers of circulating CART19 cells without changes in Th1/Th2 or memory phenotype. Thus, the results herein show that ibrutinib can be combined with CART19 in a rational manner and suggest that the properties of each of these therapies may compensate for deficiencies of the other, thus leading to enhanced long-term anti-tumor effect. The experiments and results of combining BCR signaling inhibition with anti-CD 19 directed T cell therapy pave the way to rational combinations of non-crossresistant therapies for B cell malignancies.

The kinetics of tumor response and relapse suggest that ibrutinib serves either to deepen the initial response achieved by CART19 alone, or to enhance the long-term immunosurveillance capacity of CART19 cells.

### Example 43: Ibrutinib/CAR19 T-cell combined therapy for hematologic cancers

This Example describes exemplary protocols for quantifying the efficacy of BTK inhibitors as described herein, e.g., compounds of formula (I), in combination with CAR therapy.

The therapies can be tested, for example, in a cell culture system or in an animal model. Numerous such systems and models are available. For instance, with respect to CLL, a cell culture system can be used such as primary CLL cells or an established cell line such as Hs 505.T (ATCC CRL-7306). Animal models for CLL are also known, including the Eµ-TCL1 transgenic mouse model described in Iacovelli et al., Blood. 2015 Mar 5;125(10):1578-88. With respect to cell culture systems for B-ALL, a primary cell line can be used, as can a cell line such as RCH-ACV. An exemplary animal model for B-ALL is described in Perova et al., Sci Transl Med 14 May 2014, Vol. 6, Issue 236, p. 236ra62. With respect to DLBCL, a primary cell line can be used, or an established line such as those described in Thompson et al. PLoS One. 2013 May 7;8(5):e62822. DLBCL animal models are described in, e.g., Donnou et al., Advances in Hematology, Volume 2012 (2012), Article ID 701704, 13 pages. With respect to multiple myeloma, one can use a primary cell line or a line described in Greenstein et al., Exp Hematol. 2003 Apr;31(4):271-82. Animal models for multiple myeloma include the Vk*MYC transgenic mouse model described in Chesi et al., Blood. 2012 Jul 12;120(2):376-85.

When using a cell culture system, the CAR-expressing cells and BTK inhibitor can be added to the test cells together or sequentially. Multiple doses can be tested. Vehicle-treated cancer cells can be used as a control, as can cancer cells treated with the CAR-expressing cells as a monotherapy or the BTK inhibitor as a monotherapy. Cancer cell death can be assayed by, e.g., cell counts. For instance, if the cancer cells are engineered to express renilla luciferase, bioluminescence can be detected and quantified. CART19 function can be assayed by determining, e.g., cell proliferation, degranulation, or cytokine production.

When using an animal model, the CAR-expressing cells and BTK inhibitor can be administered to the animal together or sequentially. Multiple doses can be tested. Controls can include vehicle-treated mice, as well as mice treated with the CAR-expressing cell as a monotherapy and mice treated with the BTK inhibitor as a monotherapy. Efficacy can be quantified by, e.g., measuring animal survival or cancer cell proliferation. Proliferation can be assayed, e.g., using bioluminescence if the cancer cells are engineered to express a bioluminescent marker.

### Example 44: Inhibition of Btk enzymatic activity

The inhibitory activity of the present compounds against BTK was assessed in a biochemical enzyme assay. Assay plates in 384 well format were prepared with 8-point serial dilutions for the test compounds on a Thermo CatX workstation equipped with a Innovadyne Nanodrop Express. The assay plates were prepared by addition of 50 nl per well of compound solution in 90 % DMSO. The kinase reactions were started by stepwise addition of 4.5 µl per well of peptide/ATP-solution (4 µM FITC-Ahx-TSELKKVVALYDYMPMNAND-NH2 (SEQ ID NO: 133), 164 µM ATP) in kinase buffer (50mM HEPES, pH 7.5, 1mM DTT, 0.02% Tween20, 0.02% BSA, 0.6% DMSO, 10 mM beta-glycerophosphate, and 10 µM sodium orthovanadate, 18 mM MgCl2, 1 mM MnCl2) and 4.5 µl per well of enzyme solution (6.4nM full-lenght human recombinant BTK) in kinase buffer. Kinase reactions were incubated at 30°C for 60 minutes and subsequently terminated by addition of 16 µl per well of stop solution (100 mM HEPES pH 7.5, 5 % DMSO, 0.1 % Caliper coating reagent, 10 mM EDTA, and 0.015 % Brij35). Kinase reactions were analyzed on a Caliper LC3000 workstation by separating phosphorylated and unphosphorylated peptides and kinase activities were calculated from the amounts of newly formed phospho-peptide. Inhibition data were calculated by comparison to control reactions without enzyme (100 % inhibition) and without inhibitors (0 % inhibition). The concentration of inhibitor required for 50 % inhibition (IC50) was calculated from the inhibition in response to inhibitor concentrations.

**Table 8.Inhibition of BTK enzymatic activity.**

| Example | Inhibition of BTK enzymatic activity IC₅₀ [uM] |
|---|---|
| Example 1 | 0.002 |
| Example 2 | 0.038 |
| Example 3 | 0.001 |
| Example 4 | 0.009 |
| Example 5 | 0.004 |
| Example 6 | 0.001 |
| Example 7 | 0.042 |
| Example 8 | 0.002 |
| Example 9 | 0.01 |
| Example 10 | 0.004 |
| Example 11 | 0.01 |
| Example 12 | 0.012 |
| Example 13 | 0.007 |
| Example 14 | 0.001 |
| Example 15 | 0.001 |
| Example 16 | 0.015 |
| Example 17 | 0.005 |
| Example 18 | 0.001 |
| Example 19 | 0.016 |
| Example 20 | 0.005 |
| Example 21 | 0.002 |
| Example 22 | <0.0001 |
| Example 23 | 0.001 |
| Example 24 | 0.0005 |
| Example 25 | 0.001 |
| Example 26 | 0.0004 |
| Example 27 | 0.003 |
| Example 28 | 0.001 |
| Example 29 | 0.004 |
| Example 30 | 0.006 |
| Example 31 | 0.002 |
| Example 32 | 0.004 |
| Example 33 | 0.001 |
| Example 34 | 0.002 |
| Example 35 | 0.002 |
| Example 36 | 0.017 |
| Example 37 | 0.032 |
| Example 38 | 0.002 |
| Example 39 | 0.001 |
| Example 40 | 0.002 |

### Example 45: Inhibition of BTK activity in blood

The inhibitory activity of the present compounds in human blood was assessed in the following in vitro B cell activation assay. Whole blood was collected with written consent from healthy volunteers by venipuncture into sodium heparin vials. Then, 90 (µl blood was mixed in 96 well U-bottomed microtiter plates (Thermo Scientific #163320) with 0.5 (µl of serial dilutions of test compounds in DMSO. Cultures were incubated at 37°C, 5% CO₂ for 1 hour. B cells were then stimulated by adding 10 µl of a dilution containing mouse anti-human IgM antibody (clone CW11) and recombinant human IL-4 (Immunotools) to final concentrations of 30 µg/ml and 5 ng/ml, respectively. The cultures were incubated for 20 hours and activation of B cells was measured by flow cytometry after staining for the B cell subset with APC-labeled anti-human CD19 (Beckton-Dickinson) and for the activation marker CD69 (PE-labeled anti-human CD69, Beckton-Dickinson). All staining procedures were performed at room temperature for 30 min in the dark in 96-deep well V-bottomed microtiter plates (Eppendorf) with FACS Lysing Solution (Beckton-Dickinson). Cytometric data was acquired on a CyAn cytometer (Beckman Coulter) and the subpopulation of lymphocytes were gated according to size and granularity, then further analyzed for expression of CD19 and the activation marker. Data for the inhibition of B cell activation were calculated from the percentage of cells positively stained for activation markers within the CD19 positive population. Inhibition data were calculated by comparison to control cultures without anti-IgM/IL-4 (100 % inhibition) and without inhibitors (0 % inhibition). The concentration of inhibitor required for 50 % inhibition (IC50) was calculated from the inhibition in response to inhibitor concentrations.

**Table 9. Inhibition of BTK activity in blood.**

| Example | Inhibition of BTK activity in blood IC₅₀ [uM] |
|---|---|
| 1 | 0.112 |
| 2 | 1.111 |
| 3 | 0.124 |
| 4 | 0.376 |
| 5 | 0.201 |
| 6 | 0.023 |
| 7 | 0.983 |
| 8 | 0.048 |
| 9 | 0.240 |
| 10 | 0.161 |
| 11 | 0.323 |
| 12 | 0.459 |
| 13 | 0.105 |
| 14 | 0.028 |
| 15 | 0.029 |
| 16 | 0.558 |
| 17 | 0.246 |
| 18 | 0.419 |
| 19 | 0.136 |
| 20 | 0.330 |
| 21 | 0.090 |
| 22 | 0.057 |
| 23 | 0.057 |
| 24 | 0.032 |
| 25 | 0.065 |
| 26 | 0.051 |
| 27 | 0.076 |
| 28 | 0.033 |
| 29 | 0.134 |
| 30 | 0.222 |
| 31 | 0.025 |
| 32 | 0.055 |
| 33 | 0.050 |
| 34 | 0.208 |
| 35 | 0.072 |
| 36 | 0.354 |
| 37 | 0.968 |
| 38 | 0.070 |
| 39 | 0.176 |
| 40 | 0.080 |

## Claims

1. A composition comprising a cell or a population of cells that expresses a CAR molecule that binds CD19 (a "CAR19-expressing cell") for use, in combination with a Bruton's tyrosine kinase (BTK) inhibitor, in the treatment of a disease associated with expression of CD19 in a mammal, wherein the disease is an autoimmune disease, an inflammatory disorder or a cancer, wherein the BTK inhibitor comprises a compound of formula (I) or a pharmaceutically acceptable salt thereof; 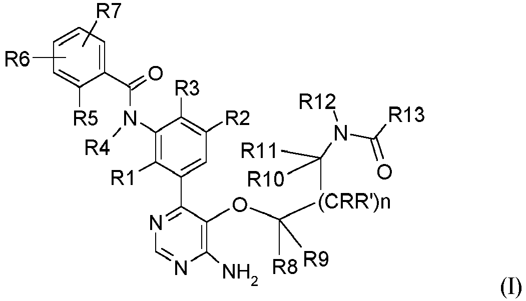 wherein,
R1 is hydrogen, C₁-C₆ alkyl optionally substituted by hydroxy;
R2 is hydrogen or halogen;
R3 is hydrogen or halogen;
R4 is hydrogen;
R5 is hydrogen or halogen;
or R4 and R5 are attached to each other and stand for a bond, -CH₂-, -CH₂-CH₂-, -CH=CH-, -CH=CH-CH₂-; -CH₂-CH=CH-; or -CH₂-CH₂-CH₂-;
R6 and R7 stand independently from each other for H, C₁-C₆ alkyl optionally substituted by hydroxyl, C₃-C₆ cycloalkyl optionally substituted by halogen or hydroxy, or halogen;
R8, R9, R, R', R10 and R11 independently from each other stand for H, or C₁-C₆ alkyl optionally substituted by C1-C6 alkoxy; or any two of R8, R9, R, R', R10 and R11 together with the carbon atom to which they are bound may form a 3 - 6 membered saturated carbocyclic ring;
R12 is hydrogen or C₁-C₆ alkyl optionally substituted by halogen or C₁-C₆ alkoxy;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C₁-C₆ alkyl or C₁-C₆ alkoxy;
n is 0 or 1; and
R13 is C₂-C₆ alkenyl optionally substituted by C₁-C₆ alkyl, C₁-C₆ alkoxy or N,N-di-C₁-C₆ alkyl amino; C₂-C₆ alkynyl optionally substituted by C₁-C₆ alkyl or C₁-C₆ alkoxy; or C₂-C₆ alkylenyl oxide optionally substituted by C₁-C₆ alkyl.

2. The composition for use of claim 1, wherein
R1 is hydrogen, or C₁-C₆ alkyl optionally substituted by hydroxy;
R2 is halogen;
R3 is hydrogen;
R4 is hydrogen;
R5 is halogen;
or R4 and R5 are attached to each other and stand for a bond, -CH2-, -CH2-CH2- , -CH=CH-, -CH=CH-CH2-; -CH2-CH=CH-; or -CH2-CH2-CH2-;
R6 and R7 stand independently from each other for H, C₁-C₆ alkyl optionally substituted by hydroxyl, C₃-C₆ cycloalkyl optionally substituted by halogen or hydroxy, or halogen;
R8, R9, R10 and R11 independently from each other stand for H, or C₁-C₆ alkyl; or any two of R8, R9, R10 and R11 together with the carbon atom to which they are bound may form a 3 - 6 membered saturated carbocyclic ring;
R and R' are hydrogen;
R12 is hydrogen or C₁-C₆ alkyl optionally substituted by halogen;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C₁-C₆ alkyl or C₁-C₆ alkoxy;
n is 0 or 1; and
R13 is C₂-C₆ alkenyl optionally substituted by C₁-C₆ alkyl or C₁-C₆ alkoxy; C₂-C₆ alkynyl optionally substituted by C₁-C₆ alkyl or C₁-C₆ alkoxy; or C₂-C₆ alkylenyl oxide optionally substituted by C₁-C₆ alkyl.

3. The composition for use of claim 1 or 2, wherein the compound of formula (I) is chosen from:
N-(3-(5-((1-Acryloylazetidin-3-yl)oxy)-6-aminopyrimidin-4-yl)-5-fluoro-2-methylphenyl)-4-cyclopropyl-2-fluorobenzamide;
(*E*)-*N*-(3-(6-Amino-5-((1-(but-2-enoyl)azetidin-3-yl)oxy)pyrimidin-4-yl)-5-fluoro-2-methylphenyl)-4-cyclopropyl-2-fluorobenzamide;
N-(3-(6-Amino-5-((1-propioloylazetidin-3-yl)oxy)pyrimidin-4-yl)-5-fluoro-2-methylphenyl)-4-cyclopropyl-2-fluorobenzamide;
*N*-(3-(6-Amino-5-((1-(but-2-ynoyl)azetidin-3-yl)oxy)pyrimidin-4-yl)-5-fluoro-2-methylphenyl)-4-cyclopropyl-2-fluorobenzamide;
*N*-(3-(5-((1-Acryloylpiperidin-4-yl)oxy)-6-aminopyrimidin-4-yl)-5-fluoro-2-methylphenyl)-4-cyclopropyl-2-fluorobenzamide;
*N*-(3-(6-Amino-5-(2-(*N*-methylacrylamido)ethoxy)pyrimidin-4-yl)-5-fluoro-2-methylphenyl)-4-cyclopropyl-2-fluorobenzamide;
*(E)-N-*(3-(6-Amino-5-(2-(*N-*methylbut-2-enamido)ethoxy)pyrimidin-4-yl)-5-fluoro-2-methylphenyl)-4-cyclopropyl-2-fluorobenzamide;
*N*-(3-(6-Amino-5-(2-(*N*-methylpropiolamido)ethoxy)pyrimidin-4-yl)-5-fluoro-2-methylphenyl)-4-cyclopropyl-2-fluorobenzamide;
*(E)*-*N*-(3-(6-Amino-5-(2-(4-methoxy-*N*-methylbut-2-enamido)ethoxy)pyrimidin-4-yl)-5-fluoro-2-methylphenyl)-4-cyclopropyl-2-fluorobenzamide;
*N*-(3-(6-Amino-5-(2-(*N*-methylbut-2-ynamido)ethoxy)pyrimidin-4-yl)-5-fluoro-2-methylphenyl)-4-cyclopropyl-2-fluorobenzamide;
*N*-(2-((4-Amino-6-(3-(4-cyclopropyl-2-fluorobenzamido)-5-fluoro-2-methylphenyl)pyrimidin-5-yl)oxy)ethyl)-*N*-methyloxirane-2-carboxamide;
*N*-(2-((4-Amino-6-(3-(6-cyclopropyl-8-fluoro-1-oxoisoquinolin-2(1*H*)-yl)phenyl)pyrimidin-5-yl)oxy)ethyl)-*N*-methylacrylamide;
*N*-(3-(5-(2-Acrylamidoethoxy)-6-aminopyrimidin-4-yl)-5-fluoro-2-methylphenyl)-4 cyclopropyl-2-fluorobenzamide;
*N*-(3-(6-Amino-5-(2-(*N*-ethylacrylamido)ethoxy)pyrimidin-4-y1)-5-fluoro-2-methylphenyl)-4-cyclopropyl-2-fluorobenzamide;
*N*-(3-(6-Amino-5-(2-(*N*-(2-fluoroethyl)acrylamido)ethoxy)pyrimidin-4-yl)-5-fluoro-2-methylphenyl)-4-cyclopropyl-2-fluorobenzamide;
*N-*(3-(5 -((1-Acrylamidocyclopropyl)methoxy)-6-aminopyrimidin-4-yl)-5 -fluoro-2-methylphenyl)-4-cyclopropyl-2-fluorobenzamide;
(*S*)-*N*-(3-(5-(2-Acrylamidopropoxy)-6-aminopyrimidin-4-yl)-5-fluoro-2-methylphenyl)-4-cyclopropyl-2-fluorobenzamide;
(*S*)-*N*-(3-(6-Amino-5-(2-(but-2-ynamido)propoxy)pyrimidin-4-yl)-5-fluoro-2-methylphenyl)-4-cyclopropyl-2-fluorobenzamide;
(*S*)-*N*-(3-(6-Amino-5-(2-(*N*-methylacrylamido)propoxy)pyrimidin-4-yl)-5-fluoro-2-methylphenyl)-4-cyclopropyl-2-fluorobenzamide;
(*S*)-*N*-(3-(6-Amino-5-(2-(*N*-methylbut-2-ynamido)propoxy)pyrimidin-4-yl)-5-fluoro-2-methylphenyl)-4-cyclopropyl-2-fluorobenzamide;
*N*-(3-(6-Amino-5-(3-(*N*-methylacrylamido)propoxy)pyrimidin-4-yl)-5-fluoro-2-methylphenyl)-4-cyclopropyl-2-fluorobenzamide;
(*S*)-*N*-(3-(5-((1-Acryloylpyrrolidin-2-yl)methoxy)-6-aminopyrimidin-4-yl)-5-fluoro-2-methylphenyl)-4-cyclopropyl-2-fluorobenzamide;
(*S*)-*N*-(3-(6-Amino-5-((1-(but-2-ynoyl)pyrrolidin-2-yl)methoxy)pyrimidin-4-yl)-5-fluoro-2-methylphenyl)-4-cyclopropyl-2-fluorobenzamide;
(*S*)-2-(3-(5-((1-Acryloylpyrrolidin-2-yl)methoxy)-6-aminopyrimidin-4-yl)-5-fluoro-2-(hydroxymethyl)phenyl)-6-cyclopropyl-3,4-dihydroisoquinolin-1(2*H*)-one;
*N*-(2-((4-Amino-6-(3-(6-cyclopropyl-1-oxo-3,4-dihydroisoquinolin-2(1*H*)-yl)-5-fluoro-2-(hydroxymethyl)phenyl)pyrimidin-5-yl)oxy)ethyl)-*N*-methylacrylamide;
*N*-(3-(5-(((2S,4R)-1-Acryloyl-4-methoxypyrrolidin-2-yl)methoxy)-6-aminopyrimidin-4-yl)-5-fluoro-2-methylphenyl)-4-cyclopropyl-2-fluorobenzamide;
*N*-(3-(6-Amino-5-(((2*S*,4*R*)-1-(but-2-ynoyl)-4-methoxypyrrolidin-2-yl)methoxy)pyrimidin-4-yl)-5-fluoro-2-methylphenyl)-4-cyclopropyl-2-fluorobenzamide;
2-(3-(5-(((2*S*,4*R*)-1-Acryloyl-4-methoxypyrrolidin-2-yl)methoxy)-6-aminopyrimidin-4-yl)-5-fluoro-2-(hydroxymethyl)phenyl)-6-cyclopropyl-3,4-dihydroisoquinolin-1(2*H*)-one;
*N-*(3-(5-(((2*S*,4*S*)-1-Acryloyl-4-methoxypyrrolidin-2-yl)methoxy)-6-aminopyrimidin-4-yl)-5 - fluoro-2-methylphenyl)-4-cyclopropyl-2-fluorobenzamide;
*N*-(3-(6-Amino-5-(((2*S*,4*S*)-1-(but-2-ynoyl)-4-methoxypyrrolidin-2-yl)methoxy)pyrimidin-4-yl)-5-fluoro-2-methylphenyl)-4-cyclopropyl-2-fluorobenzamide;
*N*-(3-(5-(((2*S*,4*R*)-1-Acryloyl-4-fluoropyrrolidin-2-yl)methoxy)-6-aminopyrimidin-4-yl)-5-fluoro-2-methylphenyl)-4-cyclopropyl-2-fluorobenzamide;
*N*-(3-(6-Amino-5-(((2*S*,4*R*)-1-(but-2-ynoyl)-4-fluoropyrrolidin-2-yl)methoxy)pyrimidin-4-yl)-5-fluoro-2-methylphenyl)-4-cyclopropyl-2-fluorobenzamide;
(*S*)-*N*-(3-(5-((1-Acryloylazetidin-2-yl)methoxy)-6-aminopyrimidin-4-yl)-5-fluoro-2-methylphenyl)-4-cyclopropyl-2-fluorobenzamide;
(*S*)-*N*-(3-(6-Amino-5-((1-propioloylazetidin-2-yl)methoxy)pyrimidin-4-yl)-5-fluoro-2-methylphenyl)-4-cyclopropyl-2-fluorobenzamide;
(*S*)-2-(3-(5-((1-Acryloylazetidin-2-yl)methoxy)-6-aminopyrimidin-4-yl)-5-fluoro-2-(hydroxymethyl)phenyl)-6-cyclopropyl-3,4-dihydroisoquinolin-1(2*H*)-one;
(*R*)-*N*-(3-(5-((1-Acryloylazetidin-2-yl)methoxy)-6-aminopyrimidin-4-yl)-5-fluoro-2-methylphenyl)-4-cyclopropyl-2-fluorobenzamide;
(*R*)-*N*-(3-(5-((1-Acryloylpiperidin-3-yl)methoxy)-6-aminopyrimidin-4-yl)-5-fluoro-2-methylphenyl)-4-cyclopropyl-2-fluorobenzamide;
*N-*(3-(5-(((2*R*,3*S*)-1-Acryloyl-3 -methoxypyrrolidin-2-yl)methoxy)-6-aminopyrimidin-4-yl)-5-fluoro-2-methylphenyl)-4-cyclopropyl-2-fluorobenzamide;
*N*-(3-(5-(((2*S*,4*R*)-1-Acryloyl-4-cyanopyrrolidin-2-yl)methoxy)-6-aminopyrimidin-4-yl)-5-fluoro-2-methylphenyl)-4-cyclopropyl-2-fluorobenzamide;
or
*N*-(3-(5-(((2*S*,4*S*)-1-Acryloyl-4-cyanopyrrolidin-2-yl)methoxy)-6-aminopyrimidin-4-yl)-5-fluoro-2-methylphenyl)-4-cyclopropyl-2-fluorobenzamide.

4. The composition for use of any one of claims 1-3, wherein the mammal is, or is identified as being, a complete or partial responder to a BTK inhibitor, e.g., a compound of formula (I) or ibrutinib, or a complete or partial responder to the CAR19-expressing cell.

5. The composition for use of any one of the preceding claims, wherein the cell expresses a CAR molecule comprising an anti-CD 19 binding domain, a transmembrane domain, and an intracellular signaling domain, wherein optionally the intracellular signaling domain comprises a costimulatory domain and a primary signaling domain.

6. The composition for use of claim 5, wherein:
(a) the CAR molecule comprises an anti-CD 19 binding domain comprising:
(i) a light chain complementary determining region 1 (LC CDR1), a light chain complementary determining region 2 (LC CDR2), a light chain complementary determining region 3 (LC CDR3), a heavy chain complementary determining region 1 (HC CDR1), a heavy chain complementary determining region 2 (HC CDR2), and a heavy chain complementary determining region 3 (HC CDR3) of an anti-CD 19 binding domain; or
(ii) a LC CDR1 of SEQ ID NO: 25, a LC CDR2 of SEQ ID NO: 26, and a LC CDR3 of SEQ ID NO: 27; and a HC CDR1 of SEQ ID NO: 19, a HC CDR2 of any of SEQ ID NOS: 20-23, and a HC CDR3 of SEQ ID NO: 24; and/or
(b) the anti-CD 19 binding domain:
(i) comprises a murine light chain variable region of SEQ ID NO: 59, a murine heavy chain variable region of SEQ ID NO: 59, or both;
(ii) comprises the amino acid sequence of SEQ ID NO:59, or an amino acid sequence with 95-99% identify thereto;
(iii) is a humanized anti-CD 19 binding domain, wherein optionally the humanized anti-CD19 binding domain comprises an amino acid sequence chosen from: SEQ ID NO:1, SEQ ID NO:2, SEQ ID NO:3, SEQ ID NO: 4, SEQ ID NO:5, SEQ ID NO:6, SEQ ID NO:7, SEQ ID NO:8, SEQ ID NO:9, SEQ ID NO: 10, SEQ ID NO:11 and SEQ ID NO: 12, or an amino acid sequence with 95-99% identity thereto;
(iv) is a humanized anti-CD 19 binding domain comprising the amino acid sequence of SEQ ID NO:2, or an amino acid sequence with 95-99% identity thereto; or
(v) is a humanized anti-CD 19 binding domain which is a scFv that comprises a light chain variable region attached to a heavy chain variable region via a linker, e.g., wherein the linker comprises the amino acid sequence of SEQ ID NO: 53.

7. The composition for use of any one of the preceding claims, wherein the CAR molecule comprises a transmembrane domain of a protein chosen from: the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 or CD154, wherein optionally:
(i) the transmembrane domain comprises the amino acid sequence of SEQ ID NO: 15; and/or
(ii) the anti-CD 19 binding domain is connected to the transmembrane domain by a hinge region, e.g., wherein the hinge region comprises the amino acid sequence of SEQ ID NO: 14 or SEQ ID NO:45.

8. The composition for use of any one of the preceding claims, wherein the CAR molecule comprises:
(a) a costimulatory domain, wherein the costimulatory domain comprises:
(i) a functional signaling domain of a protein chosen from: OX40, CD2, CD27, CD28, CDS, ICAM-1, LFA-1 (CD11a/CD18) or 4-1BB (CD137);
(ii) the amino acid sequence of SEQ ID NO: 16, or an amino acid sequence with 95-99% identity to the amino acid sequence of SEQ ID NO: 16; or
(iii) the amino acid sequence of SEQ ID NO:51, or an amino acid sequence with 95-99% identity to the amino acid sequence of SEQ ID NO:51; and/or
(b) an intracellular signaling domain, wherein the intracellular signaling domain comprises:
(i) a functional signaling domain of 4-1BB, a functional signaling domain of CD3 zeta, or both;
(ii) the amino acid sequence of CD27, a functional signaling domain of CD3 zeta, or both;
(iii) the amino acid sequence of SEQ ID NO: 16, the amino acid sequence of SEQ ID NO: 17, or both;
(iv) the amino acid sequence of SEQ ID NO: 16, the amino acid sequence of SEQ ID NO:43, or both;
(v) the amino acid sequence of SEQ ID NO: 51, the amino acid sequence of SEQ ID NO: 17, or both; or
(vi) the amino acid sequence of SEQ ID NO:51, the amino acid sequence of SEQ ID NO:43, or both.

9. The composition for use of any one of the preceding claims, wherein the CAR molecule:
(i) further comprises a leader sequence comprising the amino acid sequence of SEQ ID NO: 13; and/or
(ii) comprises the amino acid sequence of SEQ ID NO:58, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID N0:40, SEQ ID NO:41, or SEQ ID NO:42.

10. The composition for use of any one of the preceding claims, wherein:
(i) the composition is for use in combination with an agent which inhibits an immune inhibitory molecule chosen from: PD1, PD-L1, CTLA4, TIM3, CEACAM (e.g., CEACAM-1, CEACAM-3 and/or CEACAM-5), LAG3, VISTA, BTLA, TIGIT, LAIR1, CD160, or 2B4; and/or
(ii) the composition comprises 1-5 x 10⁸ CAR-expressing cells.

11. The composition for use of any one of the preceding claims, wherein the disease associated with expression of CD19 is a cancer, e.g., a hematological cancer, e.g., a hematological cancer chosen from a leukemia or lymphoma, optionally wherein the cancer is chosen from: chronic lymphocytic leukemia (CLL), mantle cell lymphoma (MCL), multiple myeloma, acute lymphoid leukemia (ALL), Hodgkin lymphoma, B-cell acute lymphoid leukemia (BALL), T-cell acute lymphoid leukemia (TALL), small lymphocytic lymphoma (SLL), B cell prolymphocytic leukemia, blastic plasmacytoid dendritic cell neoplasm, Burkitt's lymphoma, diffuse large B cell lymphoma (DLBCL), DLBCL associated with chronic inflammation, follicular lymphoma, pediatric follicular lymphoma, hairy cell leukemia, small cell- or a large cell-follicular lymphoma, malignant lymphoproliferative conditions, MALT lymphoma (extranodal marginal zone lymphoma of mucosa-associated lymphoid tissue), Marginal zone lymphoma, myelodysplasia and myelodysplastic syndrome, non-Hodgkin lymphoma, plasmablastic lymphoma, plasmacytoid dendritic cell neoplasm, Waldenstrom macroglobulinemia, splenic marginal zone lymphoma, splenic lymphoma/leukemia, splenic diffuse red pulp small B-cell lymphoma, hairy cell leukemia-variant, lymphoplasmacytic lymphoma, a heavy chain disease, plasma cell myeloma, solitary plasmocytoma of bone, extraosseous plasmocytoma, nodal marginal zone lymphoma, pediatric nodal marginal zone lymphoma, primary cutaneous follicle center lymphoma, lymphomatoid granulomatosis, primary mediastinal (thymic) large B-cell lymphoma, intravascular large B-cell lymphoma, ALK+ large B-cell lymphoma, large B-cell lymphoma arising in HHV8-associated multicentric Castleman disease, primary effusion lymphoma, B-cell lymphoma, or unclassifiable lymphoma.

12. The composition for use of any one of the preceding claims, wherein:
(a) the mammal has, or is identified as having, a BTK mutation;
(b) the disease associated with expression of CD19 is a hematological cancer, and wherein:
(i) resistance to the BTK inhibitor, the cell that expresses a CAR molecule, or both, is delayed or decreased; or
(ii) remission of the hematological cancer is prolonged or relapse of the hematological cancer is delayed;
(c) the mammal is, or is identified as being, a non-responder or relapser to a BTK inhibitor, e.g., ibrutinib, GDC-0834, RN-486, CGI-560, CGI-1764, HM-71224, CC-292, ONO-4059, CNX-774, or LFM-A13; and/or
(d) the mammal is, or is identified as being:
(i) a partial responder to the BTK inhibitor, and the mammal is administered the CAR19-expressing cell, alone or in combination with the BTK inhibitor, during the period of partial response; or
(ii) a non-responder having progressive or stable disease after treatment with ibrutinib, and the mammal is administered the CAR19-expressing cell, alone or in combination with the BTK inhibitor, during the period of progressive or stable disease

13. The composition for use of any one of the preceding claims, wherein:
(i) said use comprises performing a lymphocyte infusion with at least one CD19 CAR-expressing cell;
(ii) the cell and the BTK inhibitor are formulated for simultaneous administration or wherein the cell and the BTK inhibitor are formulated for sequential delivery; and/or
(iii) the mammal has undergone lymphodepletion, wherein optionally the lymphodepletion comprises administration of one or more of melphalan, cytoxan, cyclophosphamide, and fludarabine.

14. A composition comprising a cell that expresses a CAR molecule that binds CD19 (a "CAR19-expressing cell"), and a Bruton's tyrosine kinase (BTK) inhibitor,
wherein the BTK inhibitor comprises a compound of formula (I) or a pharmaceutically acceptable salt thereof; 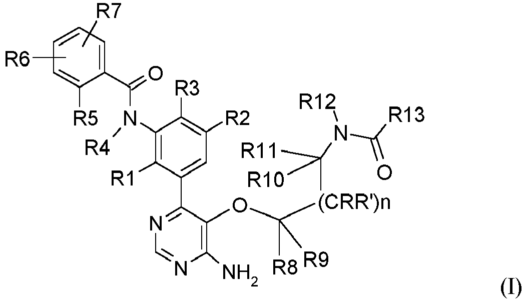 wherein,
R1 is hydrogen, C₁-C₆ alkyl optionally substituted by hydroxy;
R2 is hydrogen or halogen;
R3 is hydrogen or halogen;
R4 is hydrogen;
R5 is hydrogen or halogen;
or R4 and R5 are attached to each other and stand for a bond, -CH₂-, -CH₂-CH₂-, -CH=CH-, -CH=CH-CH₂-; -CH₂-CH=CH-; or -CH₂-CH₂-CH₂-;
R6 and R7 stand independently from each other for H, C₁-C₆ alkyl optionally substituted by hydroxyl, C₃-C₆ cycloalkyl optionally substituted by halogen or hydroxy, or halogen;
R8, R9, R, R', R10 and R11 independently from each other stand for H, or C₁-C₆ alkyl optionally substituted by C1-C6 alkoxy; or any two of R8, R9, R, R', R10 and R11 together with the carbon atom to which they are bound may form a 3 - 6 membered saturated carbocyclic ring;
R12 is hydrogen or C₁-C₆ alkyl optionally substituted by halogen or C₁-C₆ alkoxy;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C₁-C₆ alkyl or C₁-C₆ alkoxy;
n is 0 or 1; and
R13 is C₂-C₆ alkenyl optionally substituted by C₁-C₆ alkyl, C₁-C₆ alkoxy or N,N-di-C₁-C₆ alkyl amino; C₂-C₆ alkynyl optionally substituted by C₁-C₆ alkyl or C₁-C₆ alkoxy; or C₂-C₆ alkylenyl oxide optionally substituted by C₁-C₆ alkyl;
optionally wherein the CAR19-expressing cell and the BTK inhibitor are present in a single dose form, or as two or more dose forms.

15. The composition for use of any one of claims 1-13, wherein:
(a) the BTK inhibitor and the CAR19-expressing cell are administered to the mammal as a first line of therapy; or
(b) the CAR19-expressing cell is administered to the mammal after administration of the BTK inhibitor,
optionally wherein:
(i) the CAR19-expressing cell is administered after ceasing administration of the BTK inhibitor; or
(ii) administration of the BTK inhibitor is begun prior to administration of the CAR19-expressing cell, and the CAR19-expressing cell is administered in combination with continued administration of the BTK inhibitor.

16. The composition for use of any one of claims 1-13, or the composition of claim 14, wherein the CAR19-expressing cell is a human immune effector cell, e.g., a human T cell or a human NK cell, or a population of human immune effector cells.

## Patentansprüche

1. Zusammensetzung, umfassend eine Zelle oder eine Zellpopulation, die ein CAR-Molekül exprimiert, das CD19 (eine "CAR19-exprimierende Zelle") bindet, zur Verwendung, in Kombination mit einem Bruton-Tyrosinkinase-Inhibitor (BTK-Inhibitor), bei der Behandlung einer Erkrankung, die mit der Expression von CD19 bei einem Säuger assoziiert ist, wobei die Erkrankung eine Autoimmunerkrankung, eine Entzündungsstörung oder ein Krebs ist, wobei der BTK-Inhibitor eine Verbindung der Formel (I) oder ein pharmazeutisch verträgliches Salz davon umfasst; 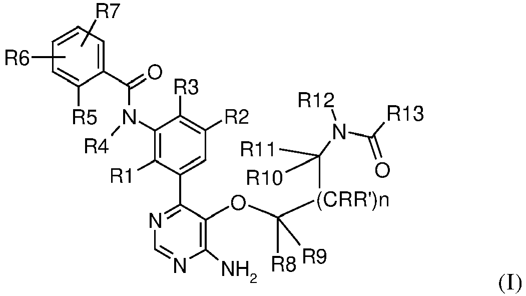 wobei,
R1 Wasserstoff, C₁-C₆-Alkyl, gegebenenfalls substituiert mit Hydroxy, ist;
R2 Wasserstoff oder Halogen ist;
R3 Wasserstoff oder Halogen ist;
R4 Wasserstoff ist;
R5 Wasserstoff oder Halogen ist;
oder R4 und R5 aneinander gebunden sind und für eine Bindung, -CH₂-, -CH₂-CH₂-, -CH=CH-, -CH=CH-CH₂-; -CH₂-CH=CH-; oder -CH₂-CH₂-CH₂-, stehen;
R6 und R7 unabhängig voneinander für H, C₁-C₆-Alkyl, gegebenenfalls substituiert mit Hydroxyl, C₃-C₆-Cycloalkyl, gegebenenfalls substituiert mit Halogen oder Hydroxy oder Halogen, stehen;
R8, R9, R, R', R10 und R11 unabhängig voneinander für H, oder C₁-C₆-Alkyl, gegebenenfalls substituiert mit C₁-C₆-Alkoxy stehen; oder jedwede zwei von R8, R9, R, R', R10 und R11 zusammen mit dem Kohlenstoffatom, an das sie gebunden sind, einen 3- bis 6-gliedrigen gesättigten carbocyclischen Ring bilden können;
R12 Wasserstoff oder C₁-C₆-Alkyl, gegebenenfalls substituiert mit Halogen oder C₁-C₆-Alkoxy, ist;
oder R12 und jedweder eine von R8, R9, R, R', R10 oder R11 zusammen mit den Atomen , an die sie gebunden sind, einen 4-, 5-, 6- oder 7-gliedrigen azacyclischen Ring bilden können, welcher Ring gegebenenfalls mit Halogen, Cyano, Hydroxyl, C₁-C₆-Alkyl oder C₁-C₆-Alkoxy substituiert sein kann;
n 0 oder 1 ist; und
R13 C₂-C₆-Alkenyl, gegebenenfalls substituiert mit C₁-C₆-Alkyl, C₁-C₆-Alkoxy oder N,N-Di-C₁-C₆-alkylamino; C₂-C₆-Alkinyl, gegebenenfalls substituiert mit C₁-C₆-Alkyl oder C₁-C₆-Alkoxy; oder C₂-C₆-Alkylenyloxid, gegebenenfalls substituiert mit C₁-C₆-Alkyl, ist.

2. Zusammensetzung zur Verwendung nach Anspruch 1, wobei
R1 Wasserstoff oder C₁-C₆-Alkyl, gegebenenfalls substituiert mit Hydroxy, ist;
R2 Halogen ist;
R3 Wasserstoff ist;
R4 Wasserstoff ist;
R5 Halogen ist;
oder R4 und R5 aneinander gebunden sind und für eine Bindung, -CH2-, -CH2-CH2-, -CH=CH-, -CH=CH-CH2-; -CH2-CH=CH-; oder -CH2-CH2-CH2-, stehen;
R6 und R7 unabhängig voneinander für H, C₁-C₆-Alkyl, gegebenenfalls substituiert mit Hydroxyl, C₃-C₆-Cycloalkyl, gegebenenfalls substituiert mit Halogen oder Hydroxy, oder Halogen, stehen;
R8, R9, R10 und R11 unabhängig voneinander für H, oder C₁-C₆-Alkyl stehen; oder jedwede zwei von R8, R9, R10 und R11 zusammen mit dem Kohlenstoffatom, an das sie gebunden sind, einen 3- bis 6-gliedrigen gesättigten carbocyclischen Ring bilden können;
R und R' Wasserstoff sind;
R12 Wasserstoff oder C₁-C₆-Alkyl, gegebenenfalls substituiert mit Halogen, ist;
oder R12 und jedweder eine von R8, R9, R, R', R10 oder R11 zusammen mit den Atomen, an die sie gebunden sind, einen 4-, 5-, 6- oder 7-gliedrigen azacyclischen Ring bilden können, welcher Ring gegebenenfalls mit Halogen, Cyano, Hydroxyl, C₁-C₆-Alkyl oder C₁-C₆-Alkoxy substituiert sein kann;
n 0 oder 1 ist; und
R13 C₂-C₆-Alkenyl, gegebenenfalls substituiert mit C₁-C₆-Alkyl oder C₁-C₆-Alkoxy; C₂-C₆-Alkinyl, gegebenenfalls substituiert mit C₁-C₆-Alkyl, oder C₁-C₆-Alkoxy; oder C₂-C₆-Alkylenyloxid, gegebenenfalls substituiert mit C₁ -C₆-Alkyl, ist.

3. Zusammensetzung zur Verwendung nach Anspruch 1 oder 2, wobei die Verbindung der Formel (I) ausgewählt ist aus:
*N*-(3-(5-((1-Acryloyl-azetidin-3-yl)oxy)-6-aminopyrimidin-4-yl)-5-fluor-2-methylphenyl)-4-cyclopropyl-2-fluorbenzamid;
(*E*)-*N*-(3-(6-Amino-5-((1-(but-2-enoyl)azetidin-3-yl)oxy)pyrimidin-4-yl)-5-fluor-2-methylphenyl)-4-cyclopropyl-2-fluorbenzamid;
N-(3-(6-Amino-5-((1-propioloyl-azetidin-3-yl)oxy)pyrimidin-4-yl)-5-fluor-2-methylphenyl)-4-cyclopropyl-2-fluorbenzamid;
*N*-(3-(6-Amino-5-((1-(but-2-inoyl)azetidin-3-yl)oxy)pyrimidin-4-yl)-5-fluor-2-methylphenyl)-4-cyclopropyl-2-fluorbenzamid;
*N*-(3-(5-((1-Acryloylpiperidin-4-yl)oxy)-6-aminopylimidin-4-yl)-5-fluor-2-methylphenyl)-4-cyclopropyl- 2-fluorbenzamid;
*N*-(3-(6-Amino-5-(2-(*N*-methylacrylamido)ethoxy)pyrimidin-4-yl)-5-fluor-2-methylphenyl)-4-cyclopropyl-2-fluorbenzamid;
(*E*)-*N*-(3-(6-Amino-5-(2-(*N*-methylbut-2-enamido)ethoxy)pyrimidin-4-yl)-5-fluor-2-methylphenyl)-4-cyclopropyl-2-fluorbenzamid;
*N*-(3-(6-Amino-5-(2-(*N*-methylpropiolamido)ethoxy)pyrimidin-4-yl)-5-fluor-2-methylphenyl)-4-cyclopropyl-2-fluorbenzamid;
*(E)*-*N*-(3-(6-Amino-5-(2-(4-methoxy-*N*-methylbut-2-enamido)ethoxy)pyrimidin-4-yl)-5-fluor-2-methylphenyl)-4-cyclopropyl-2-fluorbenzamid;
*N*-(3-(6-Amino-5-(2-(*N*-methylbut-2-inamido)ethoxy)pyrimidin-4-yl)-5-fluor-2-methylphenyl)-4-cyclopropyl-2-fluorbenzamid;
*N*-(2-((4-Amino-6-(3-(4-cyclopropyl-2-fluorbenzamido)-5-fluor-2-methylphenyl)pyrimidin-5-yl)oxy)ethyl)-*N*-methyloxiran-2-carboxamid;
*N*-(2-((4-Amino-6-(3-(6-cyclepropyl-8-fluor-1-oxoisochinolin-2(1H)-yl)phenyl)pyrimidin-5-yl)oxy)ethyl)-*N*-methylacrylamid;
*N*-(3-(5-(2-Acrylamidoethoxy)-6-aminopyrimidin-4-yl)-5-fluor-2-methylphenyl)-4-cyclopropyl-2-fluorbenzamid;
*N*-(3-(6-Amino-5-(2-(*N*-ethylacrylamido)ethoxy)pyrimidin-4-yl)-5-fluor-2-methylphenyl)-4-cyclopropyl-2-fluorbenzamid;
*N*-(3-(6-Amino-5-(2-(*N*-(2-fluorethyl)acrylamido)ethoxy)pyrimidin-4-yl)-5-fluor-2-methylphenyl)-4-cyclopropyl-2-fluorbenzamid;
*N*-(3-(5-((1-Acrylamidocyclopropyl)methoxy)-6-aminopyrimidin-4-yl)-5-fluor-2-methylphenyl)-4-cyclopropyl-2-fluorbenzamid;
(*S*)-*N*-(3-(5-(2-Acrylamidopropoxy)-6-aminopyrimidin-4-yl)-5-fluor-2-methylpheny1)-4-cyclopropyl-2-fluorbenzamid;
(*S*)-*N*-(3-(6-Amino-5-(2-(but-2-inamido)propoxy)pyrimidin-4-yl)-5-fluor-2-methylphenyl)-4-cyclopropyl-2-fluorbenzamid;
(*S*)-*N*-(3-(6-Amino-5-(2-(*N*-methylacrylamido)propoxy)pyrimidin-4-yl)-5-fluor-2-methylphenyl)-4-cyclopropyl-2-fluorbenzamid;
(*S*)-*N-*(3-(6-Amino-5-(2-(*N*-methylbut-2-inamido)propoxy)pyrimidin-4-yl)-5-fluor-2-methylphenyl)-4-cyclopropyl-2-fluorbenzamid;
*N*-(3-(6-Amino-5-(3-(*N*-methylacrylamido)propoxy)pyrimidin-4-yl)-5-fluor-2-methylphenyl)-4-cyclopropyl-2-fluorbenzamid;
(*S*)-*N*-(3-(5-((1-Acryloylpyrrolidin-2-yl)methoxy)-6-aminopyrimidin-4-yl)-5-fluor-2-methylphenyl)-4-cyclopropyl-2-fluorbenzamid;
(*S*)-*N*-(3-(6-Amino-5-((1-(but-2-inoyl)pyrrolidin-2-yl)methoxy)pyrimidin-4-yl)-5-fluor-2-methylphenyl)-4-cyclopropyl-2-fluorbenzamid;
(*S*)-2-(3-(5-((1-Acryloylpyrrolidin-2-yl)methoxy)-6-aminopyrimidin-4-yl)-5-fluor-2-(hydroxymethyl)phenyl)-6-cyclopropyl-3,4-dihydroisnochinolin-1(2*H*)-on;
*N*-(2-((4-Amino-6-(3-(6-cyclopropyl-1-oxo-3,4-dihydroisochinolin-2(1*H*)-yl)-5-fluor-2-(hydroxymethyl)phenyl)pyrimidin-5-yl)oxy)ethyl)-*N*-methylacrylamid;
*N*-(3-(5-«(2*S*,4*R*)-1-Acryloyl-4-methoxypyrrolidin-2-yl)methoxy)-6-aminopyrimidin-4-yl)-5-fluor-2-methylphenyl)-4-cyclopropyl-2-fluorbenzamid;
*N*-(3-(6-Amino-5-(((2*S*,4*R*)-1-(but-2-inoyl)-4-methoxypyrrolidin-2-yl)methoxy)pyrimidin-4-yl)-5-fluor-2-methylphenyl)-4-cyclopropyl-2-fluorbenzamid;
2-(3-(5-(((2*S*,4*R*)-1-Acryloyl-4-methoxypyrrolidin-2-yl)methoxy)-6-aminopyrimidin-4-yl)-5-fluor-2-(hydroxymethyl)phenyl)-6-cyclopropyl-3,4-dihydroisochinolin-1(2*H*)-on;
*N*-(3-(5-(((2*S*,4*S*)-1-Acryloyl-4-methoxypyrrolidin-2-yl)methoxy)-6-aminopyrimidin-4-yl)-5-fluor-2-methylphenyl)-4-cyclopropyl-2-fluorbenzamid;
*N*-(3-(6-Amino-5-(((2*S*,4*S*)-1-(but-2-inoyl)-4-methoxypyrrolidin-2-yl)methoxy)pyrimidin-4-yl)-5-fluor-2-methylphenyl)-4-cyclopropyl-2-fluorbenzamid;
*N-*(3-(5-(((2*S*,4*R*)-1-Acryloyl-4-fluorpyrrolidin-2-yl)methoxy)-6-aminopyrimidin-4-yl)-5-fluor-2-methylphenyl)-4-cyclopropyl-2-fluorbenzamid;
*N*-(3-(6-Amino-5-(((2*S*,4*R*)-1-(but-2-inoyl)-4-fluorpyrrolidin-2-yl)methoxy)pyrimidin-4-yl)-5-fluor-2-methylphenyl)-4-cyclopropyl-2-fluorbenzamid;
(*S*)-*N*-(3-(5-((1-Acryloyl-azetidin-2-yl)methoxy)-6-aminopyrimidin-4-yl)-5-fluor-2-methylphenyl)-4-cyclopropyl-2-fluorbenzamid;
(*S*)*-N-*(3-(6-Amino-5-((1-propioloyl-azetidin-2-yl)methoxy)pyrimidin-4-yl)-5-fluor-2-methylphenyl)-4-cyclopropyl-2-fluorbenzamid;
(*S*)-2-(3-(5-((1-Acryloyl-azetidin-2-yl)methoxy)-6-aminopyrimidin-4-yl)-5-fluor-2- (hydroxymethyl)phenyl)-6-cyclopropyl-3 ,4-dihydroisochinolin-1 (2*H*)-on;
(*R*)-*N*-(3-(5-((1-Acryloyl-azetidin-2-yl)methoxy)-6-aminopyrimidin-4-yl)-5-fluor-2-methylphenyl)-4-cyclopropyl-2-fluorbenzamid;
(*R*)-*N*-(3-(5-((1-Acryloylpiperidin-3-yl)methoxy)-6-aminopyrimidin-4-yl)-5-fluor-2-methylphenyl)-4-cyclopropyl-2-fluorbenzamid;
*N*-(3-(5-(((2*R*,3*S*)-1-Acryloyl-3-methoxypyrrolidin-2-yl)methoxy)-6-aminopyrimidin-4-yl)-5-fluor-2-methylphenyl)-4-cyclopropyl-2-fluorbenzamid;
*N*-(3-(5-(((2*S*,4*R*)-1-Acryloyl-4-cyanpyrrolidin-2-yl)methoxy)-6-aminopyrimidin-4-y1)-5-fluor-2-methylphenyl)-4-cyclopropyl-2-fluorbenzamid;
oder
*N*-3-(5-(((2*S*,4*S*)-1-Acryloyl-4-cyanpyrrolidin-2-yl)methoxy)-6-aminopyrimidin-4-yl)-5-fluor-2-methylphenyl)-4-cyclopropyl-2-fluorbenzamid.

4. Zusammensetzung zur Verwendung nach einem der Ansprüche 1 bis 3, wobei der Säuger ein kompletter oder partieller Responder auf einen BTK-Inhibitor, z. B. eine Verbindung der Formel (I) oder Ibrutinib, oder ein kompletter oder partieller Responder auf die CAR19-exprimierende Zelle ist oder als solcher identifiziert wird.

5. Zusammensetzung zur Verwendung nach einem der vorangehenden Ansprüche, wobei die Zelle ein CAR-Molekül exprimiert, umfassend eine Anti-CD19-Bindungsdomäne, eine Transmembrandomäne und eine intrazelluläre Signalisierungsdomäne, wobei die intrazelluläre Signalisierungsdomäne gegebenenfalls eine kostimulatorische Domäne und eine primäre Signalisierungsdomäne umfasst.

6. Zusammensetzung zur Verwendung nach Anspruch 5, wobei:
(a) das CAR-Molekül eine Anti-CD19-Bindungsdomäne umfasst, enthaltend:
(i) eine komplementäre Bestimmungsregion 1 der Leichtkette (LC CDR1), eine komplementäre Bestimmungsregion 2 der Leichtkette (LC CDR2), eine komplementäre Bestimmungsregion 3 der Leichtkette (LC CDR3), eine komplementäre Bestimmungsregion 1 der Schwerkette (HC CDR1), eine komplementäre Bestimmungsregion 2 der Schwerkette (HC CDR2) und eine komplementäre Bestimmungsregion 3 der Schwerkette (HC CDR3) von einer Anti-CD19-Bindungsdomäne; oder
(ii) eine LC CDR1 von SEQ ID NO: 25, eine LC CDR2 von SEQ ID NO: 26 und eine LC CDR3 von SEQ ID NO: 27; und eine HC CDR1 von SEQ ID NO: 19, eine HC CDR2 von jedweder einen der SEQ ID NOS: 20 bis 23, und eine HC CDR3 von SEQ ID NO: 24; und/oder
(b) die Anti-CD19-Bindungsdomäne:
(i) eine murine variable Leichtkettenregion von SEQ ID NO: 59, eine murine variable Schwerkettenregion von SEQ ID NO: 59, oder beide umfasst;
(ii) die Aminosäuresequenz von SEQ ID NO: 59, oder eine Aminosäuresequenz mit einer 95%igen bis 99%igen Identität damit umfasst;
(iii) eine humanisierte Anti-CD19-Bindungsdomäne ist, wobei die humanisierte Anti-CD19-Bindungsdomäne gegebenenfalls eine Aminosäuresequenz umfasst, ausgewählt aus: SEQ ID NO: 1, SEQ ID NO: 2, SEQ ID NO: 3, SEQ ID NO: 4, SEQID NO: 5, SEQ ID NO: 6, SEQ ID NO: 7, SEQ ID NO: 8, SEQ ID NO: 9, SEQ ID NO: 10, SEQ ID NO: 11 und SEQ ID NO: 12, oder eine Aminosäuresequenz mit einer 95%igen bis 99%igen Identität damit umfasst;
(iv) eine humanisierte Anti-CD19-Bindungsdomäne ist, umfassend die Aminosäuresequenz von SEQ ID NO: 2 oder eine Aminosäuresequenz mit einer 95%igen bis 99%igen Identität damit; oder
(v) eine humanisierte Anti-CD19-Bindungsdomäne ist, die ein scFv ist, das eine variable Leichtkettenregion umfasst, die über einen Linker an eine variable Schwerkettenregion gebunden ist, z. B. wobei der Linker die Aminosäuresequenz von SEQ ID NO: 53 umfasst.

7. Zusammensetzung zur Verwendung nach einem der vorangehenden Ansprüche, wobei das CAR-Molekül eine Transmembrandomäne von einem Protein umfasst, ausgewählt aus: der alpha-, beta- oder zeta-Kette des T-Zellrezeptors, CD28, CD3-epsilon, CD45, CD4, CD5, CD8, CD9, CD16, CD22, CD33, CD37, CD64, CD80, CD86, CD134, CD137 oder CD154, wobei gegebenenfalls:
(i) die Transmembrandomäne die Aminosäuresequenz von SEQ ID NO: 15 umfasst; und/oder
(ii) die Anti-CD19-Bindungsdomäne mit der Transmembrandomäne mittels einer Gelenkregion verbunden ist, z. B. wobei die Gelenkregion die Aminosäuresequenz von SEQ ID NO: 14 oder SEQ ID NO: 45 umfasst.

8. Zusammensetzung zur Verwendung nach einem der vorangehenden Ansprüche, wobei das CAR-Molekül Folgendes umfasst:
(a) eine kostimulatorische Domäne, wobei die kostimulatorische Domäne Folgendes enthält:
(i) eine funktionelle Signalisierungsdomäne von einem Protein, ausgewählt aus: OX40, CD2, CD27, CD28, CDS, ICAM-1, LFA-1 (CD11a/CD18) oder 4-1BB (CD137);
(ii) die Aminosäuresequenz von SEQ ID NO: 16, oder eine Aminosäuresequenz mit einer 95%igen bis 99%igen Identität mit der Aminosäuresequenz von SEQ ID NO: 16; oder
(iii) die Aminosäuresequenz von SEQ ID NO: 51, oder eine Aminosäuresequenz mit einer 95%igen bis 99%igen Identität mit der Aminosäuresequenz SEQ ID NO: 51; und/oder
(b) eine intrazelluläre Signalisierungsdomäne, wobei die intrazelluläre Signalisierungsdomäne Folgendes umfasst:
(i) eine funktionelle Signalisierungsdomäne von 4-1BB, eine funktionelle Signalisierungsdomäne von CD3-zeta, oder beide;
(ii) die Aminosäuresequenz von CD27, eine funktionelle Signalisierungsdomäne von CD3-zeta, oder beide;
(iii) die Aminosäuresequenz von SEQ ID NO: 16, die Aminosäuresequenz von SEQ ID NO: 17, oder beide;
(iv) die Aminosäuresequenz von SEQ ID NO: 16, die Aminosäuresequenz von SEQ ID NO: 43, oder beide;
(v) die Aminosäuresequenz von SEQ ID NO: 51, die Aminosäuresequenz von SEQ ID NO: 17, oder beide; oder
(vi) die Aminosäuresequenz von SEQ ID NO: 51, die Aminosäuresequenz von SEQ ID NO 43, oder beide.

9. Zusammensetzung zur Verwendung nach einem der vorangehenden Ansprüche, wobei das CAR-Molekül:
(i) weiter eine Leitsequenz umfasst, enthaltend die Aminosäuresequenz von SEQ ID NO: 13; und/oder
(ii) die Aminosäuresequenz von SEQ ID NO: 58, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41 oder SEQ ID NO: 42 umfasst.

10. Zusammensetzung zur Verwendung nach einem der vorangehenden Ansprüche, wobei:
(i) die Zusammensetzung zur Verwendung in Kombination mit einem Mittel vorgesehen ist, das ein immuninhibitorisches Molekül inhibiert, ausgewählt aus: PD1, PD-L1, CTLA4, TIM3, CEACAM (z. B. CEACAM-1, CEACAM-3 und/oder CEACAM-5), LAG3, VISTA, BTLA, TIGIT, LAIR1, CD160 oder 2B4; und/oder
(ii) die Zusammensetzung 1 bis 5 x 10⁸ CAR-exprimierende Zellen umfasst.

11. Zusammensetzung zur Verwendung nach einem der vorangehenden Ansprüche, wobei die mit der Expression von CD19 assoziierte Erkrankung ein Krebs ist, z. B. ein hämatologischer Krebs, z. B. ein hämatologischer Krebs, ausgewählt aus einer Leukämie oder einem Lymphom, gegebenenfalls wobei der Krebs ausgewählt ist aus: der chronischen lymphatischen Leukämie (CLL), dem Mantelzell-Lymphom (MCL), dem multiplen Myelom, der akuten lymphatischen Leukämie (ALL), dem Hodgkin-Lymphom, der B-Zell akuten lymphatischen Leukämie (BALL), der T-Zell akuten lymphatischen Leukämie (TALL), dem kleinzelligen lymphocytischen Lymphom (SLL), der Prolymphocytenleukämie vom B-Zell-Typ, der blastischen plasmacytoiden dendritischen Zellneoplasie, dem Burkitt-Lymphom, dem diffusen großzelligen B-Zell-Lymphom (DLBCL), dem mit einer chronischen Entzündung assoziierten DLBCL, dem follikulären Lymphom, dem pädiatrischen follikulären Lymphom, der Haarzellenleukämie, dem klein- oder großzelligen follikulären Lymphom, den malignen lymphoproliferativen Zuständen, dem MALT-Lymphom (dem extranodalen Marginalzonen-Lymphom des Mucosa-assoziierten Lymphgewebes), dem Marginalzonen-Lymphom, der Myelodysplasie und dem myelodysplastischen Syndrom, dem Non-Hodgkin-Lymphom, dem plasmablastischen Lymphom, der plasmacytoiden dendritischen Zellneoplasie, der Waldenströms Makroglobulinämie, dem splenischen Marginalzonen-Lymphom, dem splenischen Lymphom/der splenischen Leukämie, dem splenischen diffusen kleinzelligen B-Zell-Lymphom der roten Pulpa, einer Haarzellenleukämie-Varianten, dem lymphoplasmacytischen Lymphom, einer Schwerkettenerkrankung, dem Plasmazellmyelom, dem solitären Plasmocytom des Knochens, dem extraossären Plasmocytom, dem nodalen Marginalzonen-Lymphom, dem pädiatrischen nodalen Marginalzonen-Lymphom, dem primär kutanen Follikelzentrums-Lymphom, der lymphomatoiden Granulomatose, dem primär mediastinalen (thymischen) großzelligen B-Zell-Lymphom, dem intravaskulären großzelligen B-Zell-Lymphom, dem ALK+ großzelligen B-Zell-Lymphom, dem großzelligen B-Zell-Lymphom mit Entstehung aus der HHV8-assoziierten multizentrischen Castleman-Krankheit, dem primären Effusionslymphom, dem B-Zell-Lymphom oder einem unklassifizierbaren Lymphom.

12. Zusammensetzung zur Verwendung nach einem der vorangehenden Ansprüche, wobei:
(a) der Säuger eine BTK-Mutation aufweist oder identifiziert wird, dass er eine solche aufweist;
(b) die Erkrankung, assoziiert mit der Expression von CD19, ein hämatologischer Krebs ist, und wobei:
(i) die Resistenz gegen den BTK-Inhibitor, die Zelle, die ein CAR-Molekül exprimiert, oder beide, verzögert oder vermindert wird/werden; oder
(ii) die Remission des hämatologischen Krebses verlängert wird oder ein Rezidiv des hämatologischen Krebses verzögert wird;
(c) der Säuger ein Nichtresponder oder Relapser auf einen BTK-Inhibitor, z. B. Ibrutinib, GDC-0834, RN-486, CGI-560, CGI-1764, HM-71224, CC-292, ONO-4059, CNX-774 oder LFM-A13 ist oder als solcher identifiziert wird; und/oder
(d) der Säuger Folgendes ist oder identifiziert wird als:
(i) ein partieller Responder auf den BTK-Inhibitor, und dem Säuger die CAR19-exprimierende Zelle verabreicht wird, allein oder in Kombination mit dem BTK-Inhibitor, während der Dauer des partiellen Ansprechens; oder
(ii) ein Nichtresponder, der nach der Behandlung mit Ibrutinib eine progressive oder stabile Erkrankung aufweist, und dem Säuger die CAR19-exprimierende Zelle, allein oder in Kombination mit dem BTK-Inhibitor, während der Dauer der progressiven oder stabilen Erkrankung, verabreicht wird.

13. Zusammensetzung zur Verwendung nach einem der vorangehenden Ansprüche, wobei:
(i) die genannte Verwendung die Durchführung einer Lymphocyteninfusion mit mindestens einer CD19 CAR-exprimierenden Zelle umfasst;
(ii) die Zelle und der BTK-Inhibitor zur gleichzeitigen Verabreichung formuliert sind oder wobei die Zelle und der BTK-Inhibitor für die sequenzielle Abgabe formuliert sind; und/oder
(iii) der Säuger sich einer Lymphodepletion unterzogen hat, wobei die Lymphodepletion gegebenenfalls die Verabreichung von einem oder mehr von Melphalan, Cytoxan, Cyclophosphamid und Fludarabin umfasst.

14. Zusammensetzung, umfassend eine Zelle, die ein CAR-Molekül exprimiert, das CD19 (eine "CAR19-exprimierende Zelle") bindet, und einen Bruton-Tyrosinkinase-Inhibitor (BTK-Inhibitor),
wobei der BTK-Inhibitor eine Verbindung der Formel (I) oder ein pharmazeutisch verträgliches Salz davon umfasst; 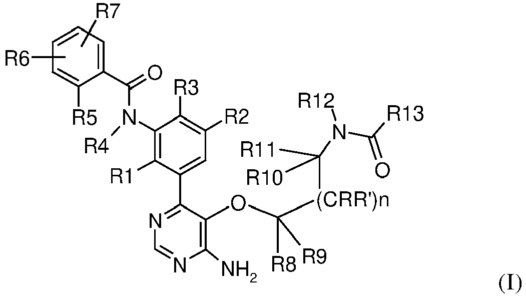 wobei,
R1 Wasserstoff, C₁-C₆-Alkyl, gegebenenfalls substituiert mit Hydroxy, ist;
R2 Wasserstoff oder Halogen ist;
R3 Wasserstoff oder Halogen ist;
R4 Wasserstoff ist;
R5 Wasserstoff oder Halogen ist;
oder R4 und R5 aneinander gebunden sind und für eine Bindung, -CH₂-, -CH₂-CH₂-, -CH=CH-, -CH=CH-CH₂-; -CH₂-CH=CH-; oder -CH₂-CH₂-CH₂-, stehen;
R6 and R7 unabhängig voneinander für H, C₁-C₆-Alkyl, gegebenenfalls substituiert mit Hydroxyl, C₃-C₆-Cycloalkyl, gegebenenfalls substituiert mit Halogen oder Hydroxy, oder Halogen, stehen;
R8, R9, R, R', R10 und R11 unabhängig voneinander für H, oder C₁-C₆-Alkyl, gegebenenfalls substituiert mit C₁-C₆-Alkoxy, stehen; oder jeweils zwei von R8, R9, R, R', R10 und R11 zusammen mit dem Kohlenstoffatom, an das sie gebunden sind, einen 3- bis 6-gliedrigen gesättigten carbocyclischen Ring bilden können;
R12 Wasserstoff oder C₁-C₆-Alkyl, gegebenenfalls substituiert mit Halogen oder C₁-C₆-Alkoxy, ist;
oder R12 und jedweder eine von R8, R9, R, R', R10 oder R11 zusammen mit den Atomen, an das sie gebunden sind, einen 4-, 5-, 6- oder 7-gliedrigen azacyclischen Ring bilden können, welcher Ring gegebenenfalls mit Halogen, Cyano, Hydroxyl, C₁-C₆-Alkyl oder C₁-C₆-Alkoxy substituiert sein kann;
n 0 oder 1 ist; und
R13 C₂-C₆-Alkenyl, gegebenenfalls substituiert mit C₁-C₆-Alkyl, C₁-C₆-Alkoxy oder N,N-Di-C₁-C₆-alkylamino; C₂-C₆-Alkinyl, gegebenenfalls substituiert mit C₁-C₆-Alkyl oder C₁-C₆-Alkoxy; oder C₂-C₆-Alkylenyloxid, gegebenenfalls substituiert mit C₁-C₆-Alkyl, ist;
gegebenenfalls wobei die CAR19-exprimierende Zelle und der BTK-Inhibitor in einer Einzeldosisform oder als zwei oder mehr Dosisformen vorliegen.

15. Zusammensetzung zur Verwendung nach einem der Ansprüche 1 bis 13, wobei:
(a) der BTK-Inhibitor und die CAR19-exprimierende Zelle an den Säuger als eine Erstlinientherapie verabreicht werden; oder
(b) die CAR19-exprimierende Zelle an den Säuger nach Verabreichung des BTK-Inhibitors verabreicht wird,
gegebenenfalls wobei:
(i) die CAR19-exprimierende Zelle nach Beendung der Verabreichung des BTK-Inhibitors verabreicht wird; oder
(ii) die Verabreichung des BTK-Inhibitors vor der Verabreichung der CAR19-exprimierenden Zelle begonnen wird, und die CAR19-exprimierende Zelle in Kombination mit der fortgesetzten Verabreichung des BTK-Inhibitors verabreicht wird.

16. Zusammensetzung zur Verwendung nach einem der Ansprüche 1 bis 13, oder Zusammensetzung nach Anspruch 14, wobei die CAR19-exprimierende Zelle eine humane Immuneffektorzelle, z. B. eine humane T-Zelle oder eine humane NK-Zelle oder eine Population von humanen Immuneffektorzellen ist.

## Revendications

1. Composition comprenant une cellule ou une population de cellules exprimant une molécule CAR qui se lie à CD19 (une « cellule exprimant CAR19 ») destinée à une utilisation, en association avec un inhibiteur de la tyrosine kinase de Bruton (BTK), dans le traitement d'une maladie associée à l'expression de CD19 chez un mammifère, la maladie étant une maladie auto-immune, une affection inflammatoire ou un cancer, l'inhibiteur de la BTK comprenant un composé selon la formule (I) ou un sel pharmaceutiquement acceptable de celui-ci ; 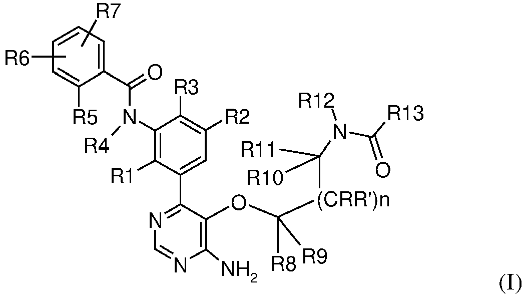 dans laquelle
R1 est un atome d'hydrogène ou un groupe alkyle C₁-C₆ optionnellement substitué par un groupe hydroxy ;
R2 est un atome d'hydrogène ou d'halogène ;
R3 est un atome d'hydrogène ou d'halogène ;
R4 est un atome d'hydrogène ;
R5 est un atome d'hydrogène ou d'halogène ;
ou R4 et R5 sont attachés l'un à l'autre et représentent une liaison, ou un groupe -CH₂-, -CH₂-CH₂- , -CH=CH-, -CH=CH-CH₂- ; -CH₂-CH=CH- ; ou -CH₂-CH₂-CH₂- ;
R6 et R7 représentent indépendamment l'un de l'autre un atome d'hydrogène, un groupe alkyle C₁-C₆ optionnellement substitué par un groupe hydroxyle, un groupe cycloalkyle C₃-C₆ optionnellement substitué par un atome d'halogène ou un groupe hydroxy, ou un atome d'halogène ;
R8, R9, R, R', R10 et R11 représentent indépendamment l'un de l'autre un atome d'hydrogène, ou un groupe alkyle C₁-C₆ optionnellement substitué par un groupe alcoxy C₁-C₆ ; ou deux quelconques de R8, R9, R, R', R10 et R11 conjointement avec l'atome de carbone auquel ils sont liés peuvent former un cycle carbocyclique saturé à 3 à 6 chaînons ;
R12 est un atome d'hydrogène ou un groupe alkyle C₁-C₆ optionnellement substitué par un atome d'halogène ou un groupe alcoxy C₁-C₆ ;
ou R12 et l'un quelconque de R8, R9, R', R', R10 ou R11 conjointement avec les atomes auxquels ils sont liés peuvent former un cycle azacyclique à 4, 5, 6 ou 7 chaînons, ledit cycle pouvant optionnellement être substitué par un atome d'halogène ou un groupe cyano, hydroxyle, alkyle C₁-C₆ ou alcoxy C₁-C₆;
n vaut 0 ou 1 ; et
R13 est un groupe alcényle C₂-C₆ optionnellement substitué par un groupe alkyle C₁-C₆, alcoxy C₁-C₆ ou N,N-di-alkylamino C₁-C₆ ; un groupe alkynyle C₂-C₆ optionnellement substitué par un groupe alkyle C₁-C₆ ou alcoxy C₁-C₆ ; ou un groupe oxyde d' alkylényle C₂-C₆ optionnellement substitué par un groupe alkyle C₁-C₆.

2. Composition destinée à une utilisation selon la revendication 1, dans laquelle
R1 est un atome d'hydrogène, ou un groupe alkyle C₁-C₆ optionnellement substitué par un groupe hydroxy ;
R2 est un atome d'halogène ;
R3 est un atome d'hydrogène ;
R 4 est un atome d'hydrogène ;
R5 est un atome d'halogène ;
ou R4 et R5 sont attachés l'un à l'autre et représentent une liaison, ou un groupe -CH₂-, -CH₂-CH₂- , -CH=CH-, -CH=CH-CH₂- ; -CH₂-CH=CH- ; ou -CH₂-CH₂-CH₂- ;
R6 et R7 représentent indépendamment l'un de l'autre un atome d'hydrogène, un groupe alkyle C₁-C₆ optionnellement substitué par un groupe hydroxyle, un groupe cycloalkyle C₃-C₆ optionnellement substitué par un atome d'halogène ou un groupe hydroxy, ou un atome d'halogène ;
R8, R9, R10 et R11 représentent indépendamment l'un de l'autre un atome d'hydrogène, ou un groupe alkyle C₁-C₆ ; ou deux quelconques de R8, R9, R10 et R11 conjointement avec l'atome de carbone auquel ils sont liés peuvent former un cycle carbocyclique saturé à 3 à 6 chaînons ;
R et R' sont un atome d'hydrogène ;
R12 est un atome d'hydrogène ou un groupe alkyle C₁-C₆ optionnellement substitué par un atome d'halogène ;
ou R12 et l'un quelconque de R8, R9, R', R', R10 ou R11 conjointement avec les atomes auxquels ils sont liés peuvent former un cycle azacyclique à 4, 5, 6 ou 7 chaînons, ledit cycle pouvant optionnellement être substitué par un atome d'halogène ou un groupe cyano, hydroxyle, alkyle C₁-C₆ ou alcoxy C₁-C₆ ;
n vaut 0 ou 1 ; et
R13 est un groupe alcényle C₂-C₆ optionnellement substitué par un groupe alkyle C₁-C₆ ou alcoxy C₁-C₆ ; un groupe alkynyle C₂-C₆ optionnellement substitué par un groupe alkyle C₁-C₆ ou alcoxy C₁-C₆ ; ou un groupe oxyde d'alkylényle C₂-C₆ optionnellement substitué par un groupe alkyle C₁-C₆.

3. Composition destinée à une utilisation selon la revendication 1 ou 2, dans laquelle le composé selon la formule (I) est choisi parmi :
N-(3-(5-((1-acryloylazétidin-3-yl)oxy)-6-aminopyrimidin-4-yl)-5-fluoro-2-méthylphényl)-4-cyclopropyl-2-fluorobenzamide ;
(*E*)-*N*-(3-(6-amino-5-((1-(but-2-enoyl)azétidin-3-yl)oxy)pyrimidin-4-yl)-5-fluoro-2-méthylphényl)-4-cyclopropyl-2-fluorobenzamide ;
N-(3-(6-amino-5-((1-propioloylazétidin-3-yl)oxy)pyrimidin-4-yl)-5-fluoro-2-méthylphényl)-4-cyclopropyl-2-fluorobenzamide ;
*N*-(3-(6-amino-5-((1-(but-2-ynoyl)azétidin-3-yl)oxy)pyrimidin-4-yl)-5-fluoro-2-méthylphényl)-4-cyclopropyl-2-fluorobenzamide ;
*N*-(3-(5-((1-acryloylpipéridin-4-yl)oxy)-6-aminopyrimidin-4-yl)-5-fluoro-2-méthylphényl)-4-cyclopropyl-2-fluorobenzamide ;
*N*-(3-(6-amino-5-(2-(*N*-méthylacrylamido)éthoxy)pyrimidin-4-yl)-5-fluoro-2-méthylphényl)-4-cyclopropyl-2-fluorobenzamide ;
(*E*)-*N*-(3-(6-amino-5-(2-(*N*-méthylbut-2-énamido)éthoxy)pyrimidin-4-yl)-5-fluoro-2-méthylphényl)-4-cyclopropyl-2-fluorobenzamide ;
*N*-(3-(6-amino-5-(2-(*N*-méthylpropiolamido)éthoxy)pyrimidin-4-yl)-5-fluoro-2-méthylphényl)-4-cyclopropyl-2-fluorobenzamide ;
(*E*)-*N*-(3-(6-amino-5-(2-(4-méthoxy-*N*-méthylbut-2-énamido)éthoxy)pyrimidin-4-yl)-5-fluoro-2-méthylphényl)-4-cyclopropyl-2-fluorobenzamide ;
*N*-(3-(6-amino-5-(2-(*N*-méthylbut-2-ynamido)éthoxy)pyrimidin-4-yl)-5-fluoro-2-méthylphényl)-4-cyclopropyl-2-fluorobenzamide ;
*N*-(2-((4-amino-6-(3-(4-cyclopropyl-2-fluorobenzamido)-5-fluoro-2-méthylphényl)pyrimidin-5-yl)oxy)éthyl)-*N*-méthyloxirane-2-carboxamide ;
*N*-(2-((4-amino-6-(3-(6-cyclopropyl-8-fluoro-1-oxoisoquinolin-2(1*H*)-yl)phényl)pyrimidin-5-yl)oxy)éthyl)-*N*-méthylacrylamide ;
*N*-(3-(5-(2-acrylamidoéthoxy)-6-aminopyrimidin-4-yl)-5-fluoro-2-méthylphényl)-4-cyclopropyl-2-fluorobenzamide ;
*N*-(3-(6-amino-5-(2-(*N*-éthylacrylamido)éthoxy)pyrimidin-4-yl)-5-fluoro-2-méthylphényl)-4-cyclopropyl-2-fluorobenzamide ;
*N*-(3-(6-amino-5-(2-(*N*-(2-fluoroéthyl)acrylamido)éthoxy)pyrimidin-4-yl)-5-fluoro-2-méthylphényl)-4-cyclopropyl-2-fluorobenzamide ;
*N*-(3-(5-((1-acrylamidocyclopropyl)méthoxy)-6-aminopyrimidin-4-yl)-5-fluoro-2-méthylphényl)-4-cyclopropyl-2-fluorobenzamide ;
(*S*)-*N*-(3-(5-(2-acrylamidopropoxy)-6-aminopyrimidin-4-yl)-5-fluoro-2-méthylphényl)-4-cyclopropyl-2-fluorobenzamide ;
(*S*)-*N*-(3-(6-amino-5-(2-(but-2-ynamido)propoxy)pyrimidin-4-yl)-5-fluoro-2-méthylphényl)-4-cyclopropyl-2-fluorobenzamide ;
(*S*)-*N*-(3-(6-amino-5-(2-(*N*-méthylacrylamido)propoxy)pyrimidin-4-yl)-5-fluoro-2-méthylphényl)-4-cyclopropyl-2-fluorobenzamide ;
(*S*)-*N*-(3-(6-amino-5-(2-(*N*-méthylbut-2-ynamido)propoxy)pyrimidin-4-yl)-5-fluoro-2-méthylphényl)-4-cyclopropyl-2-fluorobenzamide ;
*N*-(3-(6-amino-5-(3-(*N*-méthylacrylamido)propoxy)pyrimidin-4-yl)-5-fluoro-2-méthylphényl)-4-cyclopropyl-2-fluorobenzamide ;
(*S*)-*N*-(3-(5-((1-acryloylpyrrolidin-2-yl)méthoxy)-6-aminopyrimidin-4-yl)-5-fluoro-2-méthylphényl)-4-cyclopropyl-2-fluorobenzamide ;
(*S*)-*N*-(3-(6-amino-5-((1-(but-2-ynoyl)pyrrolidin-2-yl)méthoxy)pyrimidin-4-yl)-5-fluoro-2-méthylphényl)-4-cyclopropyl-2-fluorobenzamide ;
(*S*)-2-(3-(5-((1-acryloylpyrrolidin-2-yl)méthoxy)-6-aminopyrimidin-4-yl)-5-fluoro-2-(hydroxyméthyl)phényl)-6-cyclopropyl-3,4-dihydroisoquinolin-1(2*H*)-one;
*N*-(2-((4-amino-6-(3-(6-cyclopropyl-1-oxo-3,4-dihydroisoquinolin-2(1*H*)-yl)-5-fluoro-2-(hydroxyméthyl)phényl)pyrimidin-5-yl)oxy)éthyl)-*N*-méthylacrylamide;
*N*-(3-(5-(((2*S*,4*R*)-1-acryloyl-4-méthoxypyrrolidin-2-yl)méthoxy)-6-aminopyrimidin-4-yl)-5-fluoro-2-méthylphényl)-4-cyclopropyl-2-fluorobenzamide ;
*N*-(3-(6-amino-5-(((2*S*,4*R*)-1-(but-2-ynoyl)-4-méthoxypyrrolidin-2-yl)méthoxy)pyrimidin-4-yl)-5-fluoro-2-méthylphényl)-4-cyclopropyl-2-fluorobenzamide ;
2-(3-(5-(((2*S*,4*R*)-1-acryloyl-4-méthoxypyrrolidin-2-yl)méthoxy)-6-aminopyrimidin-4-yl)-5-fluoro-2-(hydroxyméthyl)phényl)-6-cyclopropyl-3,4-dihydroisoquinolin-1(2*H*)-one ;
*N*-(3-(5-(((2*S*,4*S*)-1-acryloyl-4-méthoxypyrrolidin-2-yl)méthoxy)-6-aminopyrimidin-4-yl)-5-fluoro-2-méthylphényl)-4-cyclopropyl-2-fluorobenzamide ;
*N*-(3-(6-amino-5-(((2*S*,4*S*)-1-(but-2-ynoyl)-4-méthoxypyrrolidin-2-yl)méthoxy)pyrimidin-4-yl)-5-fluoro-2-méthylphényl)-4-cyclopropyl-2-fluorobenzamide;
*N*-(3-(5-(((2*S*,4*R*)-1-acryloyl-4-fluoropyrrolidin-2-yl)méthoxy)-6-aminopyrimidin-4-yl)-5-fluoro-2-méthylphényl)-4-cyclopropyl-2-fluorobenzamide ;
*N*-(3-(6-amino-5-(((2*S*,4*R*)-1-(but-2-ynoyl)-4-fluoropyrrolidin-2-yl)méthoxy)pyrimidin-4-yl)-5-fluoro-2-méthylphényl)-4-cyclopropyl-2-fluorobenzamide;
(*S*)-*N*-(3-(5-((1-acryloylazétidin-2-yl)méthoxy)-6-aminopyrimidin-4-yl)-5-fluoro-2-méthylphényl)-4-cyclopropyl-2-fluorobenzamide ;
(*S*)-*N*-(3-(6-amino-5-((1-propioloylazétidin-2-yl)méthoxy)pyrimidin-4-yl)-5-fluoro-2-méthylphényl)-4-cyclopropyl-2-fluorobenzamide ;
(*S*)-2-(3-(5-((1-acryloylazétidin-2-yl)méthoxy)-6-aminopyrimidin-4-yl)-5-fluoro-2-(hydroxyméthyl)phényl)-6-cyclopropyl-3,4-dihydroisoquinolin-1(2*H*)-one ;
(*R*)-*N*-(3-(5-((1-acryloylazétidin-2-yl)méthoxy)-6-aminopyrimidin-4-yl)-5-fluoro-2-méthylphényl)-4-cyclopropyl-2-fluorobenzamide ;
(*R*)-*N*-(3-(5-((1-acryloylpipéridin-3-yl)méthoxy)-6-aminopyrimidin-4-y1)-5-fluoro-2-méthylphényl)-4-cyclopropyl-2-fluorobenzamide ;
*N*-(3-(5-(((2*R*,3*S*)-1-acryloyl-3-méthoxypyrrolidin-2-yl)méthoxy)-6-aminopyrimidin-4-yl)-5-fluoro-2-méthylphényl)-4-cyclopropyl-2-fluorobenzamide ;
*N*-(3-(5-(((2*S*,4*R*)-1-acryloyl-4-cyanopyrrolidin-2-yl)méthoxy)-6-aminopyrimidin-4-y1)-5-fluoro-2-méthylphényl)-4-cyclopropyl-2-fluorobenzamide ;
ou
*N*-(3-(5-(((2*S*,4*S*)-1-acryloyl-4-cyanopyrrolidin-2-yl)méthoxy)-6-aminopyrimidin-4-yl)-5-fluoro-2-méthylphényl)-4-cyclopropyl-2-fluorobenzamide.

4. Composition destinée à une utilisation selon l'une quelconque des revendications 1 à 3, dans laquelle le mammifère est, ou est identifié comme étant, un répondeur complet ou partiel à un inhibiteur de la BTK, par exemple un composé selon la formule (I) ou l'ibrutinib, ou un répondeur complet ou partiel à la cellule exprimant CAR19.

5. Composition destinée à une utilisation selon l'une quelconque des revendications précédentes, dans laquelle la cellule exprime une molécule CAR comprenant un domaine de liaison anti-CD19, un domaine transmembranaire, et un domaine de signalisation intracellulaire, dans laquelle, optionnellement, le domaine de signalisation intracellulaire comprend un domaine de costimulation et un domaine de signalisation primaire.

6. Composition destinée à une utilisation selon la revendication 5, dans laquelle :
a) la molécule CAR comprend un domaine de liaison anti-CD 19 comprenant :
i) une région 1 déterminant la complémentarité de chaîne légère (LC CDR1), une région 2 déterminant la complémentarité de chaîne légère (LC CDR2), une région 3 déterminant la complémentarité de chaîne légère (LC CDR3), une région 1 déterminant la complémentarité de chaîne lourde (HC CDR1), une région 2 déterminant la complémentarité de chaîne lourde (HC CDR2), et une région 3 déterminant la complémentarité de chaîne lourde (HC CDR3) d'un domaine de liaison anti-CD19 ; ou
ii) une région LC CDR1 de SÉQ. ID n° 25, une région LC CDR2 de SÉQ. ID n° 26, et une région LC CDR3 de SÉQ. ID n° 27 ; et une région HC CDR1 de SÉQ. ID n° 19, une région HC CDR2 de l'une quelconque des SÉQ. ID n^{os} 20 à 23, et une région HC CDR3 de SÉQ. ID n° 24 ; et/ou
b) le domaine de liaison anti-CD19 :
i) comprend une région variable de chaîne légère murine de SÉQ. ID n° 59, une région variable de chaîne lourde murine de SÉQ. ID n° 59, ou les deux ;
ii) comprend la séquence d'acides aminés SÉQ. ID n° 59, ou une séquence d'acides aminés ayant une identité de 95 à 99 % avec celle-ci ;
iii) est un domaine de liaison anti-CD19 humanisé, le domaine de liaison anti-CD19 humanisé comprenant optionnellement une séquence d'acides aminés choisie parmi : SÉQ. ID n° 1, SÉQ. ID n° 2, SÉQ. ID n° 3, SÉQ. ID n° 4, SÉQ. ID n° 5, SÉQ. ID n° 6, SÉQ. ID n° 7, SÉQ. ID n° 8, SÉQ. ID n° 9, SÉQ. ID n° 10, SÉQ. ID n° 11 et SÉQ. ID n° 12, ou une séquence d'acides aminés ayant une identité de 95 à 99 % avec celle-ci ;
iv) est un domaine de liaison anti-CD19 humanisé comprenant la séquence d'acides aminés SÉQ. ID n° 2, ou une séquence d'acides aminés ayant une identité de 95 à 99 % avec celle-ci ; ou
v) est un domaine de liaison anti-CD19 humanisé qui est un fragment scFv comprenant une région variable de chaîne légère attachée à une région variable de chaîne lourde via un lieur, le lieur comprenant par exemple la séquence d'acides aminés SÉQ. ID n° 53.

7. Composition destinée à une utilisation selon l'une quelconque des revendications précédentes, dans laquelle la molécule CAR comprend un domaine transmembranaire d'une protéine choisie parmi : la chaîne alpha, bêta ou zêta du récepteur des cellules T, CD28, CD3 epsilon, CD45, CD4, CD5, CD8, CD9, CD16, CD22, CD33, CD37, CD64, CD80, CD86, CD134, CD137 ou CD154, dans laquelle optionnellement :
i) le domaine transmembranaire comprend la séquence d'acides aminés SÉQ. ID n° 15 ; et/ou
ii) le domaine de liaison anti-CD19 est connecté au domaine transmembranaire par une région charnière, la région charnière comprenant par exemple la séquence d'acides aminés SÉQ. ID n° 14 ou SÉQ. ID n° 45.

8. Composition destinée à une utilisation selon l'une quelconque des revendications précédentes, dans laquelle la molécule CAR comprend :
a) un domaine de costimulation, le domaine de costimulation comprenant :
i) un domaine de signalisation fonctionnel d'une protéine choisie parmi : OX40, CD2, CD27, CD28, CDS, ICAM-1, LFA-1 (CD11a/CD18) ou 4-1BB (CD137) ;
ii) la séquence d'acides aminés SÉQ. ID n° 16, ou une séquence d'acides aminés ayant une identité de 95 à 99 % avec la séquence d'acides aminés SÉQ. ID n° 16 ; ou
iii) la séquence d'acides aminés SÉQ. ID n° 51, ou une séquence d'acides aminés ayant une identité de 95 à 99 % avec la séquence d'acides aminés SÉQ. ID n° 51 ; et/ou
b) un domaine de signalisation intracellulaire, le domaine de signalisation intracellulaire comprenant :
i) un domaine de signalisation fonctionnel de 4-1BB, un domaine de signalisation fonctionnel de CD3 zêta, ou les deux ;
ii) la séquence d'acides aminés de CD27, un domaine de signalisation fonctionnel de CD3 zêta, ou les deux ;
iii) la séquence d'acides aminés SÉQ. ID n° 16, la séquence d'acides aminés SÉQ. ID n° 17, ou les deux ;
iv) la séquence d'acides aminés SÉQ. ID n° 16, la séquence d'acides aminés SÉQ. ID n° 43, ou les deux ;
v) la séquence d'acides aminés SÉQ. ID n° 51, la séquence d'acides aminés SÉQ. ID n° 17, ou les deux ;
vi) la séquence d'acides aminés SÉQ. ID n° 51, la séquence d'acides aminés SÉQ. ID n° 43, ou les deux.

9. Composition destinée à une utilisation selon l'une quelconque des revendications précédentes, dans laquelle la molécule CAR :
i) comprend en outre une séquence de tête comprenant la séquence d'acides aminés SÉQ. ID n° 13 ; et/ou
ii) comprend la séquence d'acides aminés SÉQ. ID n° 58, SÉQ. ID n° 31, SÉQ. IDn° 32, SÉQ. ID n° 33, SÉQ. IDn° 34, SÉQ. ID n° 35, SÉQ. ID n° 36, SÉQ. ID n° 37, SÉQ. ID n° 38, SÉQ. ID n° 39, SÉQ. ID n° 40, SÉQ. ID n° 41, OU SÉQ. ID n° 42.

10. Composition destinée à une utilisation selon l'une quelconque des revendications précédentes, dans laquelle :
i) la composition est destinée à une utilisation en association avec un agent qui inhibe une molécule d'inhibition immunitaire choisie parmi : PD1, PD-L1, CTLA4, TIM3, CEACAM (par exemple CEACAM-1, CEACAM-3 et/ou CEACAM-5), LAG3, VISTA, BTLA, TIGIT, LAIR1, CD160, ou 2B4 ; et/ou
ii) la composition comprend 1-5 x 10⁸ cellules exprimant CAR.

11. Composition destinée à une utilisation selon l'une quelconque des revendications précédentes, la maladie associée à l'expression de CD19 étant un cancer, par exemple un cancer hématologique, par exemple un cancer hématologique choisi parmi une leucémie ou un lymphome, le cancer étant optionnellement choisi parmi : une leucémie lymphocytaire chronique (LLC), un lymphome à cellules du manteau (LCM), un myélome multiple, une leucémie lymphoïde aiguë (LLA), un lymphome hodgkinien, une leucémie lymphoïde aiguë à cellules B (LLA-B), une leucémie lymphoïde aiguë à cellules T (LLA-T), un lymphome à petits lymphocytes (LPL), une leucémie prolymphocytaire à cellules B, un néoplasme à cellules dendritiques plasmacytoïdes blastiques, un lymphome de Burkitt, un lymphome diffus à grandes cellules B (LDGCB), un LDGCB associé à une inflammation chronique, un lymphome folliculaire, un lymphome folliculaire pédiatrique, une leucémie à tricholeucocytes, un lymphome folliculaire à petites cellules ou à grandes cellules, des affections lymphoprolifératives malignes, un lymphome MALT (lymphome extraganglionnaire de la zone marginale du tissu lymphoïde associé aux muqueuses), un lymphome de la zone marginale, une myélodysplasie et un syndrome myélodysplasique, un lymphome non hodgkinien, un lymphome plasmoblastique, un néoplasme à cellules dendritiques plasmacytoïdes, une macroglobulinémie de Waldenström, un lymphome splénique de la zone marginale, un lymphome/une leucémie splénique, un lymphome splénique diffus à cellules B de la pulpe rouge, une variante de la leucémie à tricholeucocytes, un lymphome lymphoplasmacytique, une maladie des chaînes lourdes, un myélome à plasmocytes, un plasmocytome osseux solitaire, un plasmocytome extra-osseux, un lymphome nodulaire de la zone marginale, un lymphome nodulaire pédiatrique de la zone marginale, un lymphome centrofolliculaire cutané primitif, une granulomatose lymphomatoïde, un lymphome primaire médiastinal (thymique) à grandes cellules B, un lymphome à grandes cellules B intravasculaire, un lymphome à grandes cellules B ALK positif, un lymphome à grandes cellules B survenant dans une maladie de Castleman multicentrique associée à HHV8, un lymphome primitif des séreuses, un lymphome à cellules B, ou un lymphome inclassable.

12. Composition destinée à une utilisation selon l'une quelconque des revendications précédentes, dans laquelle :
a) le mammifère présente, ou est identifié comme présentant, une mutation BTK ;
b) la maladie associée à l'expression de CD19 est un cancer hématologique, et dans laquelle :
i) la résistance à l'inhibiteur de la BTK, à la cellule qui exprime une molécule CAR, ou aux deux, est retardée ou diminuée ; ou
ii) la rémission du cancer hématologique est prolongée ou la rechute du cancer hématologique est retardée ;
c) le mammifère est, ou est identifié comme étant, un non-répondeur ou un sujet qui a fait une rechute lors de l'utilisation d'un inhibiteur de la BTK, par exemple l'ibrutinib, GDC-0834, RN-486, CGI-560, CGI-1764, HM-71224, CC-292, ONO-4059, CNX-774, ou LFM-A13 ; et/ou
d) le mammifère est, ou est identifié comme étant :
i) un répondeur partiel à l'inhibiteur de la BTK, et on administre au mammifère la cellule exprimant CAR19, seule ou associée à l'inhibiteur de la BTK, pendant la période de réponse partielle ; ou
ii) un non-répondeur présentant une maladie progressive ou stable après un traitement par l'ibrutinib, et on administre au mammifère la cellule exprimant CAR19, seule ou associée à l'inhibiteur de la BTK, pendant la période de maladie progressive ou stable.

13. Composition destinée à une utilisation selon l'une quelconque des revendications précédentes, dans laquelle :
i) ladite utilisation comprend l'exécution d'une perfusion de lymphocytes comprenant au moins une cellule exprimant CAR ciblant CD 19 ;
ii) la cellule et l'inhibiteur de la BTK sont formulés pour une administration simultanée ou dans laquelle la cellule et l'inhibiteur de la BTK sont formulés pour une administration séquentielle ; et/ou
iii) le mammifère a subi une lymphodéplétion, la lymphodéplétion comprenant optionnellement l'administration d'un ou de plusieurs du melphalan, du cytoxan, du cyclophosphamide et de la fludarabine.

14. Composition comprenant une cellule qui exprime une molécule CAR qui se lie à CD19 (une « cellule exprimant CAR19 ») et un inhibiteur de la tyrosine kinase de Bruton (BTK),
dans laquelle l'inhibiteur de la BTK comprend un composé selon la formule (I) ou un sel pharmaceutiquement acceptable de celui-ci ; 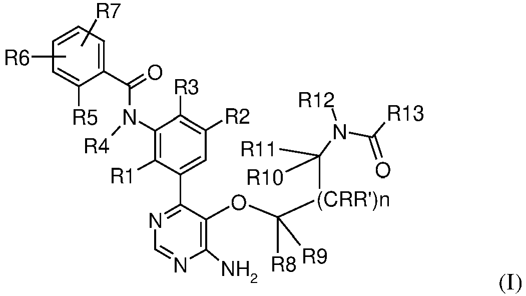 dans laquelle
R1 est un atome d'hydrogène ou un groupe alkyle C₁-C₆ optionnellement substitué par un groupe hydroxy ;
R2 est un atome d'hydrogène ou d'halogène ;
R3 est un atome d'hydrogène ou d'halogène ;
R4 est un atome d'hydrogène ;
R5 est un atome d'hydrogène ou d'halogène ;
ou R4 et R5 sont attachés l'un à l'autre et représentent une liaison, ou un groupe -CH₂-, -CH₂-CH₂- , -CH=CH-, -CH=CH-CH₂- ; -CH₂-CH=CH- ; ou -CH₂-CH₂-CH₂- ;
R6 et R7 représentent indépendamment l'un de l'autre un atome d'hydrogène, un groupe alkyle C₁-C₆ optionnellement substitué par un groupe hydroxyle, un groupe cycloalkyle C₃-C₆ optionnellement substitué par un atome d'halogène ou un groupe hydroxy, ou un atome d'halogène ;
R8, R9, R, R', R10 et R11 représentent indépendamment l'un de l'autre un atome d'hydrogène, ou un groupe alkyle C₁-C₆ optionnellement substitué par un groupe alcoxy C₁-C₆ ; ou deux quelconques de R8, R9, R, R', R10 et R11 conjointement avec l'atome de carbone auquel ils sont liés peuvent former un cycle carbocyclique saturé à 3 à 6 chaînons ;
R12 est un atome d'hydrogène ou un groupe alkyle C₁-C₆ optionnellement substitué par un atome d'halogène ou un groupe alcoxy C₁-C₆ ;
ou R12 et l'un quelconque de R8, R9, R', R', R10 ou R11 conjointement avec les atomes auxquels ils sont liés peuvent former un cycle azacyclique à 4, 5, 6 ou 7 chaînons, ledit cycle pouvant optionnellement être substitué par un atome d'halogène ou un groupe cyano, hydroxyle, alkyle C₁-C₆ ou alcoxy C₁-C₆, ;
n vaut 0 ou 1 ; et
R13 est un groupe alcényle C₂-C₆ optionnellement substitué par un groupe alkyle C₁-C₆, alcoxy C₁-C₆ ou N,N-di-alkylamino C₁-C₆, ; un groupe alkynyle C₂-C₆ optionnellement substitué par un groupe alkyle C₁-C₆ ou alcoxy C₁-C₆ ; ou un groupe oxyde d'alkylényle C₂-C₆ optionnellement substitué par un groupe alkyle C₁-C₆ ;
optionnellement dans laquelle la cellule exprimant CAR19 et l'inhibiteur de la BTK sont présents dans une seule forme galénique, ou dans deux formes galéniques ou plus.

15. Composition destinée à une utilisation selon l'une quelconque des revendications 1 à 13, dans laquelle :
a) l'inhibiteur de la BTK et la cellule exprimant CAR19 sont administrés au mammifère en tant que traitement de première ligne ; ou
b) la cellule exprimant CAR19 est administrée au mammifère après l'administration de l'inhibiteur de la BTK,
optionnellement :
i) la cellule exprimant CAR19 étant administrée après cessation de l'administration de l'inhibiteur de la BTK ; ou
ii) l'administration de l'inhibiteur de la BTK étant commencée avant l'administration de la cellule exprimant CAR19, et la cellule exprimant CAR19 étant administrée en association avec une administration continue de l'inhibiteur de la BTK.

16. Composition destinée à une utilisation selon l'une quelconque des revendications 1 à 13, ou composition selon la revendication 14, dans laquelle la cellule exprimant CAR19 est une cellule effectrice immunitaire humaine, par exemple une cellule T humaine ou une cellule NK humaine, ou une population de cellules effectrices immunitaires humaines.


### SEQUENCE LISTING

<110> NOVARTIS AG
   THE TRUSTEES OF THE UNIVERSITY OF PENNSYLVANIA
<120> COMBINATION OF CHIMERIC ANTIGEN RECEPTOR THERAPY AND AMINO PYRIMIDINE DERIVATIVES
<130> N2067-7092WO
<140>
   <141>
<150> 62/144,188
   <151> 2015-04-07
<160> 142
<170> PatentIn version 3.5
<210> 1
   <211> 242
   <212> PRT
   <213> Artificial Sequence
<220>
   <221> source
   <223> /note="Description of Artificial Sequence: Synthetic polypeptide"
<400> 1
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gln Val Gln Leu Gln Glu
           115                 120                 125
   Ser Gly Pro Gly Leu Val Lys Pro Ser Glu Thr Leu Ser Leu Thr Cys
       130                 135                 140
   Thr Val Ser Gly Val Ser Leu Pro Asp Tyr Gly Val Ser Trp Ile Arg
   145                 150                 155                 160
   Gln Pro Pro Gly Lys Gly Leu Glu Trp Ile Gly Val Ile Trp Gly Ser
                   165                 170                 175
   Glu Thr Thr Tyr Tyr Ser Ser Ser Leu Lys Ser Arg Val Thr Ile Ser
               180                 185                 190
   Lys Asp Asn Ser Lys Asn Gln Val Ser Leu Lys Leu Ser Ser Val Thr
           195                 200                 205
   Ala Ala Asp Thr Ala Val Tyr Tyr Cys Ala Lys His Tyr Tyr Tyr Gly
       210                 215                 220
   Gly Ser Tyr Ala Met Asp Tyr Trp Gly Gln Gly Thr Leu Val Thr Val
   225                 230                 235                 240
   Ser Ser
<210> 2
   <211> 242
   <212> PRT
   <213> Artificial Sequence
<220>
   <221> source
   <223> /note="Description of Artificial Sequence: Synthetic polypeptide"
<400> 2
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gln Val Gln Leu Gln Glu
           115                 120                 125
   Ser Gly Pro Gly Leu Val Lys Pro Ser Glu Thr Leu Ser Leu Thr Cys
       130                 135                 140
   Thr Val Ser Gly Val Ser Leu Pro Asp Tyr Gly Val Ser Trp Ile Arg
   145                 150                 155                 160
   Gln Pro Pro Gly Lys Gly Leu Glu Trp Ile Gly Val Ile Trp Gly Ser
                   165                 170                 175
   Glu Thr Thr Tyr Tyr Gln Ser Ser Leu Lys Ser Arg Val Thr Ile Ser
               180                 185                 190
   Lys Asp Asn Ser Lys Asn Gln Val Ser Leu Lys Leu Ser Ser Val Thr
           195                 200                 205
   Ala Ala Asp Thr Ala Val Tyr Tyr Cys Ala Lys His Tyr Tyr Tyr Gly
       210                 215                 220
   Gly Ser Tyr Ala Met Asp Tyr Trp Gly Gln Gly Thr Leu Val Thr Val
   225                 230                 235                 240
   Ser Ser
<210> 3
   <211> 242
   <212> PRT
   <213> Artificial Sequence
<220>
   <221> source
   <223> /note="Description of Artificial Sequence: Synthetic polypeptide"
<400> 3
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
   35 40 45
   Gly Val Ile Trp Gly Ser Glu Thr Thr Tyr Tyr Ser Ser Ser Leu Lys
   50 55 60
   Ser Arg Val Thr Ile Ser Lys Asp Asn Ser Lys Asn Gln Val Ser Leu
   65 70 75 80
   Lys Leu Ser Ser Val Thr Ala Ala Asp Thr Ala Val Tyr Tyr Cys Ala
   85 90 95
   Lys His Tyr Tyr Tyr Gly Gly Ser Tyr Ala Met Asp Tyr Trp Gly Gln
   100 105 110
   Gly Thr Leu Val Thr Val Ser Ser Gly Gly Gly Gly Ser Gly Gly Gly
   115 120 125
   Gly Ser Gly Gly Gly Gly Ser Glu Ile Val Met Thr Gln Ser Pro Ala
   130 135 140
   Thr Leu Ser Leu Ser Pro Gly Glu Arg Ala Thr Leu Ser Cys Arg Ala
   145 150 155 160
   Ser Gln Asp Ile Ser Lys Tyr Leu Asn Trp Tyr Gln Gln Lys Pro Gly
   165 170 175
   Gln Ala Pro Arg Leu Leu Ile Tyr His Thr Ser Arg Leu His Ser Gly
   180 185 190
   Ile Pro Ala Arg Phe Ser Gly Ser Gly Ser Gly Thr Asp Tyr Thr Leu
   195 200 205
   Thr Ile Ser Ser Leu Gln Pro Glu Asp Phe Ala Val Tyr Phe Cys Gln
   210 215 220
   Gln Gly Asn Thr Leu Pro Tyr Thr Phe Gly Gln Gly Thr Lys Leu Glu
   225 230 235 240
   Ile Lys
<210> 4
   <211> 242
   <212> PRT
   <213> Artificial Sequence
<220>
   <221> source
   <223> /note="Description of Artificial Sequence: Synthetic polypeptide"
<400> 4
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Gln Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Glu Ile Val Met Thr Gln Ser Pro Ala
       130                 135                 140
   Thr Leu Ser Leu Ser Pro Gly Glu Arg Ala Thr Leu Ser Cys Arg Ala
   145                 150                 155                 160
   Ser Gln Asp Ile Ser Lys Tyr Leu Asn Trp Tyr Gln Gln Lys Pro Gly
                   165                 170                 175
   Gln Ala Pro Arg Leu Leu Ile Tyr His Thr Ser Arg Leu His Ser Gly
               180                 185                 190
   Ile Pro Ala Arg Phe Ser Gly Ser Gly Ser Gly Thr Asp Tyr Thr Leu
           195                 200                 205
   Thr Ile Ser Ser Leu Gln Pro Glu Asp Phe Ala Val Tyr Phe Cys Gln
       210                 215                 220
   Gln Gly Asn Thr Leu Pro Tyr Thr Phe Gly Gln Gly Thr Lys Leu Glu
   225                 230                 235                 240
   Ile Lys
<210> 5
   <211> 247
   <212> PRT
   <213> Artificial Sequence
<220>
   <221> source
   <223> /note="Description of Artificial Sequence: Synthetic polypeptide"
<400> 5
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gly Gly Gly Gly Ser Gln
           115                 120                 125
   Val Gln Leu Gln Glu Ser Gly Pro Gly Leu Val Lys Pro Ser Glu Thr
       130                 135                 140
   Leu Ser Leu Thr Cys Thr Val Ser Gly Val Ser Leu Pro Asp Tyr Gly
   145                 150                 155                 160
   Val Ser Trp Ile Arg Gln Pro Pro Gly Lys Gly Leu Glu Trp Ile Gly
                   165                 170                 175
   Val Ile Trp Gly Ser Glu Thr Thr Tyr Tyr Ser Ser Ser Leu Lys Ser
               180                 185                 190
   Arg Val Thr Ile Ser Lys Asp Asn Ser Lys Asn Gln Val Ser Leu Lys
           195                 200                 205
   Leu Ser Ser Val Thr Ala Ala Asp Thr Ala Val Tyr Tyr Cys Ala Lys
       210                 215                 220
   His Tyr Tyr Tyr Gly Gly Ser Tyr Ala Met Asp Tyr Trp Gly Gln Gly
   225                 230                 235                 240
   Thr Leu Val Thr Val Ser Ser
                   245
<210> 6
   <211> 247
   <212> PRT
   <213> Artificial Sequence
<220>
   <221> source
   <223> /note="Description of Artificial Sequence: Synthetic polypeptide"
<400> 6
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gly Gly Gly Gly Ser Gln
           115                 120                 125
   Val Gln Leu Gln Glu Ser Gly Pro Gly Leu Val Lys Pro Ser Glu Thr
       130                 135                 140
   Leu Ser Leu Thr Cys Thr Val Ser Gly Val Ser Leu Pro Asp Tyr Gly
   145                 150                 155                 160
   Val Ser Trp Ile Arg Gln Pro Pro Gly Lys Gly Leu Glu Trp Ile Gly
                   165                 170                 175
   Val Ile Trp Gly Ser Glu Thr Thr Tyr Tyr Gln Ser Ser Leu Lys Ser
               180                 185                 190
   Arg Val Thr Ile Ser Lys Asp Asn Ser Lys Asn Gln Val Ser Leu Lys
           195                 200                 205
   Leu Ser Ser Val Thr Ala Ala Asp Thr Ala Val Tyr Tyr Cys Ala Lys
       210                 215                 220
   His Tyr Tyr Tyr Gly Gly Ser Tyr Ala Met Asp Tyr Trp Gly Gln Gly
   225                 230                 235                 240
   Thr Leu Val Thr Val Ser Ser
                   245
<210> 7
   <211> 247
   <212> PRT
   <213> Artificial Sequence
<220>
   <221> source
   <223> /note="Description of Artificial Sequence: Synthetic polypeptide"
<400> 7
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Ser Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Glu Ile Val Met
       130                 135                 140
   Thr Gln Ser Pro Ala Thr Leu Ser Leu Ser Pro Gly Glu Arg Ala Thr
   145                 150                 155                 160
   Leu Ser Cys Arg Ala Ser Gln Asp Ile Ser Lys Tyr Leu Asn Trp Tyr
                   165                 170                 175
   Gln Gln Lys Pro Gly Gln Ala Pro Arg Leu Leu Ile Tyr His Thr Ser
               180                 185                 190
   Arg Leu His Ser Gly Ile Pro Ala Arg Phe Ser Gly Ser Gly Ser Gly
           195                 200                 205
   Thr Asp Tyr Thr Leu Thr Ile Ser Ser Leu Gln Pro Glu Asp Phe Ala
       210                 215                 220
   Val Tyr Phe Cys Gln Gln Gly Asn Thr Leu Pro Tyr Thr Phe Gly Gln
   225                 230                 235                 240
   Gly Thr Lys Leu Glu Ile Lys
                   245
<210> 8
   <211> 247
   <212> PRT
   <213> Artificial Sequence
<220>
   <221> source
   <223> /note="Description of Artificial Sequence: Synthetic polypeptide"
<400> 8
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Gln Ser Ser Leu Lys
   50 55 60
   Ser Arg Val Thr Ile Ser Lys Asp Asn Ser Lys Asn Gln Val Ser Leu
   65 70 75 80
   Lys Leu Ser Ser Val Thr Ala Ala Asp Thr Ala Val Tyr Tyr Cys Ala
   85 90 95
   Lys His Tyr Tyr Tyr Gly Gly Ser Tyr Ala Met Asp Tyr Trp Gly Gln
   100 105 110
   Gly Thr Leu Val Thr Val Ser Ser Gly Gly Gly Gly Ser Gly Gly Gly
   115 120 125
   Gly Ser Gly Gly Gly Gly Ser Gly Gly Gly Gly Ser Glu Ile Val Met
   130 135 140
   Thr Gln Ser Pro Ala Thr Leu Ser Leu Ser Pro Gly Glu Arg Ala Thr
   145 150 155 160
   Leu Ser Cys Arg Ala Ser Gln Asp Ile Ser Lys Tyr Leu Asn Trp Tyr
   165 170 175
   Gln Gln Lys Pro Gly Gln Ala Pro Arg Leu Leu Ile Tyr His Thr Ser
   180 185 190
   Arg Leu His Ser Gly Ile Pro Ala Arg Phe Ser Gly Ser Gly Ser Gly
   195 200 205
   Thr Asp Tyr Thr Leu Thr Ile Ser Ser Leu Gln Pro Glu Asp Phe Ala
   210 215 220
   Val Tyr Phe Cys Gln Gln Gly Asn Thr Leu Pro Tyr Thr Phe Gly Gln
   225 230 235 240
   Gly Thr Lys Leu Glu Ile Lys
   245
<210> 9
   <211> 247
   <212> PRT
   <213> Artificial Sequence
<220>
   <221> source
   <223> /note="Description of Artificial Sequence: Synthetic polypeptide"
<400> 9
   Glu Ile Val Met Thr Gln Ser Pro Ala Thr Leu Ser Leu Ser Pro Gly
   1 5 10 15
   Glu Arg Ala Thr Leu Ser Cys Arg Ala Ser Gln Asp Ile Ser Lys Tyr
   20 25 30
   Leu Asn Trp Tyr Gln Gln Lys Pro Gly Gln Ala Pro Arg Leu Leu Ile
   35 40 45
   Tyr His Thr Ser Arg Leu His Ser Gly Ile Pro Ala Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Val Tyr Phe Cys Gln Gln Gly Asn Thr Leu Pro Tyr
   85 90 95
   Thr Phe Gly Gln Gly Thr Lys Leu Glu Ile Lys Gly Gly Gly Gly Ser
   100 105 110
   Gly Gly Gly Gly Ser Gly Gly Gly Gly Ser Gly Gly Gly Gly Ser Gln
   115 120 125
   Val Gln Leu Gln Glu Ser Gly Pro Gly Leu Val Lys Pro Ser Glu Thr
   130 135 140
   Leu Ser Leu Thr Cys Thr Val Ser Gly Val Ser Leu Pro Asp Tyr Gly
   145 150 155 160
   Val Ser Trp Ile Arg Gln Pro Pro Gly Lys Gly Leu Glu Trp Ile Gly
   165 170 175
   Val Ile Trp Gly Ser Glu Thr Thr Tyr Tyr Asn Ser Ser Leu Lys Ser
   180 185 190
   Arg Val Thr Ile Ser Lys Asp Asn Ser Lys Asn Gln Val Ser Leu Lys
   195 200 205
   Leu Ser Ser Val Thr Ala Ala Asp Thr Ala Val Tyr Tyr Cys Ala Lys
   210 215 220
   His Tyr Tyr Tyr Gly Gly Ser Tyr Ala Met Asp Tyr Trp Gly Gln Gly
   225 230 235 240
   Thr Leu Val Thr Val Ser Ser
   245
<210> 10
   <211> 247
   <212> PRT
   <213> Artificial Sequence
<220>
   <221> source
   <223> /note="Description of Artificial Sequence: Synthetic polypeptide"
<400> 10
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Asn Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Glu Ile Val Met
       130                 135                 140
   Thr Gln Ser Pro Ala Thr Leu Ser Leu Ser Pro Gly Glu Arg Ala Thr
   145                 150                 155                 160
   Leu Ser Cys Arg Ala Ser Gln Asp Ile Ser Lys Tyr Leu Asn Trp Tyr
                   165                 170                 175
   Gln Gln Lys Pro Gly Gln Ala Pro Arg Leu Leu Ile Tyr His Thr Ser
               180                 185                 190
   Arg Leu His Ser Gly Ile Pro Ala Arg Phe Ser Gly Ser Gly Ser Gly
           195                 200                 205
   Thr Asp Tyr Thr Leu Thr Ile Ser Ser Leu Gln Pro Glu Asp Phe Ala
       210                 215                 220
   Val Tyr Phe Cys Gln Gln Gly Asn Thr Leu Pro Tyr Thr Phe Gly Gln
   225                 230                 235                 240
   Gly Thr Lys Leu Glu Ile Lys
                   245
<210> 11
   <211> 242
   <212> PRT
   <213> Artificial Sequence
<220>
   <221> source
   <223> /note="Description of Artificial Sequence: Synthetic polypeptide"
<400> 11
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gln Val Gln Leu Gln Glu
           115                 120                 125
   Ser Gly Pro Gly Leu Val Lys Pro Ser Glu Thr Leu Ser Leu Thr Cys
       130                 135                 140
   Thr Val Ser Gly Val Ser Leu Pro Asp Tyr Gly Val Ser Trp Ile Arg
   145                 150                 155                 160
   Gln Pro Pro Gly Lys Gly Leu Glu Trp Ile Gly Val Ile Trp Gly Ser
   165 170 175
   Glu Thr Thr Tyr Tyr Asn Ser Ser Leu Lys Ser Arg Val Thr Ile Ser
   180 185 190
   Lys Asp Asn Ser Lys Asn Gln Val Ser Leu Lys Leu Ser Ser Val Thr
   195 200 205
   Ala Ala Asp Thr Ala Val Tyr Tyr Cys Ala Lys His Tyr Tyr Tyr Gly
   210 215 220
   Gly Ser Tyr Ala Met Asp Tyr Trp Gly Gln Gly Thr Leu Val Thr Val
   225 230 235 240
   Ser Ser
<210> 12
   <211> 242
   <212> PRT
   <213> Artificial Sequence
<220>
   <221> source
   <223> /note="Description of Artificial Sequence: Synthetic polypeptide"
<400> 12
   Gln Val Gln Leu Gln Glu Ser Gly Pro Gly Leu Val Lys Pro Ser Glu
   1 5 10 15
   Thr Leu Ser Leu Thr Cys Thr Val Ser Gly Val Ser Leu Pro Asp Tyr
   20 25 30
   Gly Val Ser Trp Ile Arg Gln Pro Pro Gly Lys Gly Leu Glu Trp Ile
   35 40 45
   Gly Val Ile Trp Gly Ser Glu Thr Thr Tyr Tyr Asn Ser Ser Leu Lys
   50 55 60
   Ser Arg Val Thr Ile Ser Lys Asp Asn Ser Lys Asn Gln Val Ser Leu
   65 70 75 80
   Lys Leu Ser Ser Val Thr Ala Ala Asp Thr Ala Val Tyr Tyr Cys Ala
   85 90 95
   Lys His Tyr Tyr Tyr Gly Gly Ser Tyr Ala Met Asp Tyr Trp Gly Gln
   100 105 110
   Gly Thr Leu Val Thr Val Ser Ser Gly Gly Gly Gly Ser Gly Gly Gly
           115                 120                 125
   Gly Ser Gly Gly Gly Gly Ser Glu Ile Val Met Thr Gln Ser Pro Ala
       130                 135                 140
   Thr Leu Ser Leu Ser Pro Gly Glu Arg Ala Thr Leu Ser Cys Arg Ala
   145                 150                 155                 160
   Ser Gln Asp Ile Ser Lys Tyr Leu Asn Trp Tyr Gln Gln Lys Pro Gly
                   165                 170                 175
   Gln Ala Pro Arg Leu Leu Ile Tyr His Thr Ser Arg Leu His Ser Gly
               180                 185                 190
   Ile Pro Ala Arg Phe Ser Gly Ser Gly Ser Gly Thr Asp Tyr Thr Leu
           195                 200                 205
   Thr Ile Ser Ser Leu Gln Pro Glu Asp Phe Ala Val Tyr Phe Cys Gln
       210                 215                 220
   Gln Gly Asn Thr Leu Pro Tyr Thr Phe Gly Gln Gly Thr Lys Leu Glu
   225                 230                 235                 240
   Ile Lys
<210> 13
   <211> 21
   <212> PRT
   <213> Artificial Sequence
<220>
   <221> source
   <223> /note="Description of Artificial Sequence: Synthetic peptide"
<400> 13
   Met Ala Leu Pro Val Thr Ala Leu Leu Leu Pro Leu Ala Leu Leu Leu
   1               5                   10                  15
   His Ala Ala Arg Pro
               20
<210> 14
   <211> 45
   <212> **PRT**
   <213> **Artificial** Sequence
<220>
   <221> source
   <223> /note="Description of Artificial Sequence: Synthetic polypeptide"
<400> 14
   Thr Thr Thr Pro Ala Pro Arg Pro Pro Thr Pro Ala Pro Thr Ile Ala
   1               5                   10                  15
   Ser Gln Pro Leu Ser Leu Arg Pro Glu Ala Cys Arg Pro Ala Ala Gly
               20                  25                  30
   Gly Ala Val His Thr Arg Gly Leu Asp Phe Ala Cys Asp
           35                  40                  45
<210> 15
   <211> 24
   <212> PRT
   <213> Artificial Sequence
<220>
   <221> source
   <223> /note="Description of Artificial Sequence: Synthetic peptide"
<400> 15
   Ile Tyr Ile Trp Ala Pro Leu Ala Gly Thr Cys Gly Val Leu Leu Leu
   1               5                   10                  15
   Ser Leu Val Ile Thr Leu Tyr Cys
               20
<210> 16
   <211> 42
   <212> PRT
   <213> Artificial Sequence
<220>
   <221> source
   <223> /note="Description of Artificial Sequence: Synthetic polypeptide"
<400> 16
   Lys Arg Gly Arg Lys Lys Leu Leu Tyr Ile Phe Lys Gln Pro Phe Met
   1               5                   10                  15
   Arg Pro Val Gln Thr Thr Gln Glu Glu Asp Gly Cys Ser Cys Arg Phe
               20                  25                  30
   Pro Glu Glu Glu Glu Gly Gly Cys Glu Leu
           35                  40
<210> 17
   <211> 112
   <212> PRT
   <213> Artificial Sequence
<220>
   <221> source
   <223> /note="Description of Artificial Sequence: Synthetic polypeptide"
<400> 17
   Arg Val Lys Phe Ser Arg Ser Ala Asp Ala Pro Ala Tyr Lys Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Leu Tyr Asn Glu Leu Gln Lys
       50                  55                  60
   Asp Lys Met Ala Glu Ala Tyr Ser Glu Ile Gly Met Lys Gly Glu Arg
   65                  70                  75                  80
   Arg Arg Gly Lys Gly His Asp Gly Leu Tyr Gln Gly Leu Ser Thr Ala
                   85                  90                  95
   Thr Lys Asp Thr Tyr Asp Ala Leu His Met Gln Ala Leu Pro Pro Arg
               100                 105                 110
<210> 18
   <211> 5
   <212> PRT
   <213> Artificial Sequence
<220>
   <221> source
   <223> /note="Description of Artificial Sequence: Synthetic peptide"
<400> 18
   Gly Gly Gly Gly Ser
   1               5
<210> 19
   <211> 10
   <212> PRT
   <213> Artificial Sequence
<220>
   <221> source
   <223> /note="Description of Artificial Sequence: Synthetic peptide"
<400> 19
   Gly Val Ser Leu Pro Asp Tyr Gly Val Ser
   1 5 10
<210> 20
   <211> 16
   <212> PRT
   <213> Artificial Sequence
<220>
   <221> source
   <223> /note="Description of Artificial Sequence: Synthetic peptide"
<400> 20
   Val Ile Trp Gly Ser Glu Thr Thr Tyr Tyr Asn Ser Ala Leu Lys Ser
   1 5 10 15
<210> 21
   <211> 16
   <212> PRT
   <213> Artificial Sequence
<220>
   <221> source
   <223> /note="Description of Artificial Sequence: Synthetic peptide"
<400> 21
   Val Ile Trp Gly Ser Glu Thr Thr Tyr Tyr Ser Ser Ser Leu Lys Ser
   1 5 10 15
<210> 22
   <211> 16
   <212> PRT
   <213> Artificial Sequence
<220>
   <221> source
   <223> /note="Description of Artificial Sequence: Synthetic peptide"
<400> 22
   Val Ile Trp Gly Ser Glu Thr Thr Tyr Tyr Gln Ser Ser Leu Lys Ser
   1 5 10 15
<210> 23
   <211> 16
   <212> PRT
   <213> Artificial Sequence
<220>
   <221> source
   <223> /note="Description of Artificial Sequence: Synthetic peptide"
<400> 23
   Val Ile Trp Gly Ser Glu Thr Thr Tyr Tyr Asn Ser Ser Leu Lys Ser
   1 5 10 15
<210> 24
   <211> 12
   <212> PRT
   <213> Artificial Sequence
<220>
   <221> source
   <223> /note="Description of Artificial Sequence: Synthetic peptide"
<400> 24
   His Tyr Tyr Tyr Gly Gly Ser Tyr Ala Met Asp Tyr
   1 5 10
<210> 25
   <211> 11
   <212> PRT
   <213> Artificial Sequence
<220>
   <221> source
   <223> /note="Description of Artificial Sequence: Synthetic peptide"
<400> 25
   Arg Ala Ser Gln Asp Ile Ser Lys Tyr Leu Asn
   1 5 10
<210> 26
   <211> 7
   <212> PRT
   <213> Artificial Sequence
<220>
   <221> source
   <223> /note="Description of Artificial Sequence: Synthetic peptide"
<400> 26
   His Thr Ser Arg Leu His Ser
   1 5
<210> 27
   <211> 9
   <212> PRT
   <213> Artificial Sequence
<220>
   <221> source
   <223> /note="Description of Artificial Sequence: Synthetic peptide"
<400> 27
   Gln Gln Gly Asn Thr Leu Pro Tyr Thr
   1 5
<210> 28
   <211> 5
   <212> PRT
   <213> Artificial Sequence
<220>
   <221> source
   <223> /note="Description of Artificial Sequence: Synthetic peptide"
<400> 28
   Tyr Ser Ser Ser Leu
   1               5
<210> 29
   <211> 5
   <212> PRT
   <213> Artificial Sequence
<220>
   <221> source
   <223> /note="Description of Artificial Sequence: Synthetic peptide"
<400> 29
   Tyr Gln Ser Ser Leu
   1               5
<210> 30
   <211> 5
   <212> PRT
   <213> Artificial Sequence
<220>
   <221> source
   <223> /note="Description of Artificial Sequence: Synthetic peptide"
<400> 30
   Tyr Asn Ser Ser Leu
   1               5
<210> 31
   <211> 486
   <212> PRT
   <213> Artificial Sequence
<220>
   <221> source
   <223> /note="Description of Artificial Sequence: Synthetic polypeptide"
<400> 31
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Ser Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Leu Val Thr Val Ser Ser Thr Thr Thr Pro Ala Pro Arg Pro Pro
               260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Lys Arg Gly Arg
                   325                 330                 335
   Lys Lys Leu Leu Tyr Ile Phe Lys Gln Pro Phe Met Arg Pro Val Gln
               340                 345                 350
   Thr Thr Gln Glu Glu Asp Gly Cys Ser Cys Arg Phe Pro Glu Glu Glu
           355                 360                 365
   Glu Gly Gly Cys Glu Leu Arg Val Lys Phe Ser Arg Ser Ala Asp Ala
       370                 375                 380
   Pro Ala Tyr Lys Gln Gly Gln Asn Gln Leu Tyr Asn Glu Leu Asn Leu
   385                 390                 395                 400
   Gly Arg Arg Glu Glu Tyr Asp Val Leu Asp Lys Arg Arg Gly Arg Asp
                   405                 410                 415
   Pro Glu Met Gly Gly Lys Pro Arg Arg Lys Asn Pro Gln Glu Gly Leu
               420                 425                 430
   Tyr Asn Glu Leu Gln Lys Asp Lys Met Ala Glu Ala Tyr Ser Glu Ile
           435                 440                 445
   Gly Met Lys Gly Glu Arg Arg Arg Gly Lys Gly His Asp Gly Leu Tyr
       450                 455                 460
   Gln Gly Leu Ser Thr Ala Thr Lys Asp Thr Tyr Asp Ala Leu His Met
   465                 470                 475                 480
   Gln Ala Leu Pro Pro Arg
                   485
<210> 32
   <211> 486
   <212> PRT
   <213> Artificial Sequence
<220>
   <221> source
   <223> /note="Description of Artificial Sequence: Synthetic polypeptide"
<400> 32
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Gln Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Leu Val Thr Val Ser Ser Thr Thr Thr Pro Ala Pro Arg Pro Pro
                   260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Lys Arg Gly Arg
                   325                 330                 335
   Lys Lys Leu Leu Tyr Ile Phe Lys Gln Pro Phe Met Arg Pro Val Gln
               340                 345                 350
   Thr Thr Gln Glu Glu Asp Gly Cys Ser Cys Arg Phe Pro Glu Glu Glu
           355                 360                 365
   Glu Gly Gly Cys Glu Leu Arg Val Lys Phe Ser Arg Ser Ala Asp Ala
       370                 375                 380
   Pro Ala Tyr Lys Gln Gly Gln Asn Gln Leu Tyr Asn Glu Leu Asn Leu
   385                 390                 395                 400
   Gly Arg Arg Glu Glu Tyr Asp Val Leu Asp Lys Arg Arg Gly Arg Asp
                   405                 410                 415
   Pro Glu Met Gly Gly Lys Pro Arg Arg Lys Asn Pro Gln Glu Gly Leu
               420                 425                 430
   Tyr Asn Glu Leu Gln Lys Asp Lys Met Ala Glu Ala Tyr Ser Glu Ile
           435                 440                 445
   Gly Met Lys Gly Glu Arg Arg Arg Gly Lys Gly His Asp Gly Leu Tyr
       450                 455                 460
   Gln Gly Leu Ser Thr Ala Thr Lys Asp Thr Tyr Asp Ala Leu His Met
   465                 470                 475                 480
   Gln Ala Leu Pro Pro Arg
                   485
<210> 33
   <211> 486
   <212> **PRT**
   <213> **Artificial** Sequence
<220>
   <221> source
   <223> /note="Description of Artificial Sequence: Synthetic polypeptide"
<400> 33
   Met Ala Leu Pro Val Thr Ala Leu Leu Leu Pro Leu Ala Leu Leu Leu
   1               5                   10                  15
   His Ala Ala Arg Pro Gln Val Gln Leu Gln Glu Ser Gly Pro Gly Leu
               20                  25                  30
   Val Lys Pro Ser Glu Thr Leu Ser Leu Thr Cys Thr Val Ser Gly Val
           35                  40                  45
   Ser Leu Pro Asp Tyr Gly Val Ser Trp Ile Arg Gln Pro Pro Gly Lys
       50                  55                  60
   Gly Leu Glu Trp Ile Gly Val Ile Trp Gly Ser Glu Thr Thr Tyr Tyr
   65                  70                  75                  80
   Ser Ser Ser Leu Lys Ser Arg Val Thr Ile Ser Lys Asp Asn Ser Lys
                   85                  90                  95
   Asn Gln Val Ser Leu Lys Leu Ser Ser Val Thr Ala Ala Asp Thr Ala
               100                 105                 110
   Val Tyr Tyr Cys Ala Lys His Tyr Tyr Tyr Gly Gly Ser Tyr Ala Met
           115                 120                 125
   Asp Tyr Trp Gly Gln Gly Thr Leu Val Thr Val Ser Ser Gly Gly Gly
       130                 135                 140
   Gly Ser Gly Gly Gly Gly Ser Gly Gly Gly Gly Ser Glu Ile Val Met
   145                 150                 155                 160
   Thr Gln Ser Pro Ala Thr Leu Ser Leu Ser Pro Gly Glu Arg Ala Thr
                   165                 170                 175
   Leu Ser Cys Arg Ala Ser Gln Asp Ile Ser Lys Tyr Leu Asn Trp Tyr
               180                 185                 190
   Gln Gln Lys Pro Gly Gln Ala Pro Arg Leu Leu Ile Tyr His Thr Ser
           195                 200                 205
   Arg Leu His Ser Gly Ile Pro Ala Arg Phe Ser Gly Ser Gly Ser Gly
       210                 215                 220
   Thr Asp Tyr Thr Leu Thr Ile Ser Ser Leu Gln Pro Glu Asp Phe Ala
   225                 230                 235                 240
   Val Tyr Phe Cys Gln Gln Gly Asn Thr Leu Pro Tyr Thr Phe Gly Gln
                   245                 250                 255
   Gly Thr Lys Leu Glu Ile Lys Thr Thr Thr Pro Ala Pro Arg Pro Pro
               260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Lys Arg Gly Arg
                   325                 330                 335
   Lys Lys Leu Leu Tyr Ile Phe Lys Gln Pro Phe Met Arg Pro Val Gln
               340                 345                 350
   Thr Thr Gln Glu Glu Asp Gly Cys Ser Cys Arg Phe Pro Glu Glu Glu
           355                 360                 365
   Glu Gly Gly Cys Glu Leu Arg Val Lys Phe Ser Arg Ser Ala Asp Ala
       370                 375                 380
   Pro Ala Tyr Lys Gln Gly Gln Asn Gln Leu Tyr Asn Glu Leu Asn Leu
   385                 390                 395                 400
   Gly Arg Arg Glu Glu Tyr Asp Val Leu Asp Lys Arg Arg Gly Arg Asp
                   405                 410                 415
   Pro Glu Met Gly Gly Lys Pro Arg Arg Lys Asn Pro Gln Glu Gly Leu
               420                 425                 430
   Tyr Asn Glu Leu Gln Lys Asp Lys Met Ala Glu Ala Tyr Ser Glu Ile
           435                 440                 445
   Gly Met Lys Gly Glu Arg Arg Arg Gly Lys Gly His Asp Gly Leu Tyr
       450                 455                 460
   Gln Gly Leu Ser Thr Ala Thr Lys Asp Thr Tyr Asp Ala Leu His Met
   465                 470                 475                 480
   Gln Ala Leu Pro Pro Arg
   485
<210> 34
   <211> 486
   <212> PRT
   <213> Artificial Sequence
<220>
   <221> source
   <223> /note="Description of Artificial Sequence: Synthetic polypeptide"
<400> 34
   Met Ala Leu Pro Val Thr Ala Leu Leu Leu Pro Leu Ala Leu Leu Leu
   1 5 10 15
   His Ala Ala Arg Pro Gln Val Gln Leu Gln Glu Ser Gly Pro Gly Leu
   20 25 30
   Val Lys Pro Ser Glu Thr Leu Ser Leu Thr Cys Thr Val Ser Gly Val
   35 40 45
   Ser Leu Pro Asp Tyr Gly Val Ser Trp Ile Arg Gln Pro Pro Gly Lys
   50 55 60
   Gly Leu Glu Trp Ile Gly Val Ile Trp Gly Ser Glu Thr Thr Tyr Tyr
   65 70 75 80
   Gln Ser Ser Leu Lys Ser Arg Val Thr Ile Ser Lys Asp Asn Ser Lys
   85 90 95
   Asn Gln Val Ser Leu Lys Leu Ser Ser Val Thr Ala Ala Asp Thr Ala
   100 105 110
   Val Tyr Tyr Cys Ala Lys His Tyr Tyr Tyr Gly Gly Ser Tyr Ala Met
   115 120 125
   Asp Tyr Trp Gly Gln Gly Thr Leu Val Thr Val Ser Ser Gly Gly Gly
   130 135 140
   Gly Ser Gly Gly Gly Gly Ser Gly Gly Gly Gly Ser Glu Ile Val Met
   145 150 155 160
   Thr Gln Ser Pro Ala Thr Leu Ser Leu Ser Pro Gly Glu Arg Ala Thr
   165 170 175
   Leu Ser Cys Arg Ala Ser Gln Asp Ile Ser Lys Tyr Leu Asn Trp Tyr
   180 185 190
   Gln Gln Lys Pro Gly Gln Ala Pro Arg Leu Leu Ile Tyr His Thr Ser
   195 200 205
   Arg Leu His Ser Gly Ile Pro Ala Arg Phe Ser Gly Ser Gly Ser Gly
   210 215 220
   Thr Asp Tyr Thr Leu Thr Ile Ser Ser Leu Gln Pro Glu Asp Phe Ala
   225 230 235 240
   Val Tyr Phe Cys Gln Gln Gly Asn Thr Leu Pro Tyr Thr Phe Gly Gln
   245 250 255
   Gly Thr Lys Leu Glu Ile Lys Thr Thr Thr Pro Ala Pro Arg Pro Pro
   260 265 270
   Thr Pro Ala Pro Thr Ile Ala Ser Gln Pro Leu Ser Leu Arg Pro Glu
   275 280 285
   Ala Cys Arg Pro Ala Ala Gly Gly Ala Val His Thr Arg Gly Leu Asp
   290 295 300
   Phe Ala Cys Asp Ile Tyr Ile Trp Ala Pro Leu Ala Gly Thr Cys Gly
   305 310 315 320
   Val Leu Leu Leu Ser Leu Val Ile Thr Leu Tyr Cys Lys Arg Gly Arg
   325 330 335
   Lys Lys Leu Leu Tyr Ile Phe Lys Gln Pro Phe Met Arg Pro Val Gln
   340 345 350
   Thr Thr Gln Glu Glu Asp Gly Cys Ser Cys Arg Phe Pro Glu Glu Glu
   355 360 365
   Glu Gly Gly Cys Glu Leu Arg Val Lys Phe Ser Arg Ser Ala Asp Ala
   370 375 380
   Pro Ala Tyr Lys Gln Gly Gln Asn Gln Leu Tyr Asn Glu Leu Asn Leu
   385 390 395 400
   Gly Arg Arg Glu Glu Tyr Asp Val Leu Asp Lys Arg Arg Gly Arg Asp
   405 410 415
   Pro Glu Met Gly Gly Lys Pro Arg Arg Lys Asn Pro Gln Glu Gly Leu
   420 425 430
   Tyr Asn Glu Leu Gln Lys Asp Lys Met Ala Glu Ala Tyr Ser Glu Ile
   435 440 445
   Gly Met Lys Gly Glu Arg Arg Arg Gly Lys Gly His Asp Gly Leu Tyr
   450 455 460
   Gln Gly Leu Ser Thr Ala Thr Lys Asp Thr Tyr Asp Ala Leu His Met
   465 470 475 480
   Gln Ala Leu Pro Pro Arg
   485
<210> 35
   <211> 491
   <212> PRT
   <213> Artificial Sequence
<220>
   <221> source
   <223> /note="Description of Artificial Sequence: Synthetic polypeptide"
<400> 35
   Met Ala Leu Pro Val Thr Ala Leu Leu Leu Pro Leu Ala Leu Leu Leu
   1 5 10 15
   His Ala Ala Arg Pro Glu Ile Val Met Thr Gln Ser Pro Ala Thr Leu
   20 25 30
   Ser Leu Ser Pro Gly Glu Arg Ala Thr Leu Ser Cys Arg Ala Ser Gln
   35 40 45
   Asp Ile Ser Lys Tyr Leu Asn Trp Tyr Gln Gln Lys Pro Gly Gln Ala
   50 55 60
   Pro Arg Leu Leu Ile Tyr His Thr Ser Arg Leu His Ser Gly Ile Pro
   65 70 75 80
   Ala Arg Phe Ser Gly Ser Gly Ser Gly Thr Asp Tyr Thr Leu Thr Ile
   85 90 95
   Ser Ser Leu Gln Pro Glu Asp Phe Ala Val Tyr Phe Cys Gln Gln Gly
   100 105 110
   Asn Thr Leu Pro Tyr Thr Phe Gly Gln Gly Thr Lys Leu Glu Ile Lys
   115 120 125
   Gly Gly Gly Gly Ser Gly Gly Gly Gly Ser Gly Gly Gly Gly Ser Gly
   130 135 140
   Gly Gly Gly Ser Gln Val Gln Leu Gln Glu Ser Gly Pro Gly Leu Val
   145 150 155 160
   Lys Pro Ser Glu Thr Leu Ser Leu Thr Cys Thr Val Ser Gly Val Ser
   165 170 175
   Leu Pro Asp Tyr Gly Val Ser Trp Ile Arg Gln Pro Pro Gly Lys Gly
   180 185 190
   Leu Glu Trp Ile Gly Val Ile Trp Gly Ser Glu Thr Thr Tyr Tyr Ser
   195 200 205
   Ser Ser Leu Lys Ser Arg Val Thr Ile Ser Lys Asp Asn Ser Lys Asn
   210 215 220
   Gln Val Ser Leu Lys Leu Ser Ser Val Thr Ala Ala Asp Thr Ala Val
   225 230 235 240
   Tyr Tyr Cys Ala Lys His Tyr Tyr Tyr Gly Gly Ser Tyr Ala Met Asp
   245 250 255
   Tyr Trp Gly Gln Gly Thr Leu Val Thr Val Ser Ser Thr Thr Thr Pro
   260 265 270
   Ala Pro Arg Pro Pro Thr Pro Ala Pro Thr Ile Ala Ser Gln Pro Leu
   275 280 285
   Ser Leu Arg Pro Glu Ala Cys Arg Pro Ala Ala Gly Gly Ala Val His
   290 295 300
   Thr Arg Gly Leu Asp Phe Ala Cys Asp Ile Tyr Ile Trp Ala Pro Leu
   305 310 315 320
   Ala Gly Thr Cys Gly Val Leu Leu Leu Ser Leu Val Ile Thr Leu Tyr
   325 330 335
   Cys Lys Arg Gly Arg Lys Lys Leu Leu Tyr Ile Phe Lys Gln Pro Phe
   340 345 350
   Met Arg Pro Val Gln Thr Thr Gln Glu Glu Asp Gly Cys Ser Cys Arg
   355 360 365
   Phe Pro Glu Glu Glu Glu Gly Gly Cys Glu Leu Arg Val Lys Phe Ser
   370 375 380
   Arg Ser Ala Asp Ala Pro Ala Tyr Lys Gln Gly Gln Asn Gln Leu Tyr
   385 390 395 400
   Asn Glu Leu Asn Leu Gly Arg Arg Glu Glu Tyr Asp Val Leu Asp Lys
   405 410 415
   Arg Arg Gly Arg Asp Pro Glu Met Gly Gly Lys Pro Arg Arg Lys Asn
   420 425 430
   Pro Gln Glu Gly Leu Tyr Asn Glu Leu Gln Lys Asp Lys Met Ala Glu
   435 440 445
   Ala Tyr Ser Glu Ile Gly Met Lys Gly Glu Arg Arg Arg Gly Lys Gly
   450 455 460
   His Asp Gly Leu Tyr Gln Gly Leu Ser Thr Ala Thr Lys Asp Thr Tyr
   465 470 475 480
   Asp Ala Leu His Met Gln Ala Leu Pro Pro Arg
   485 490
<210> 36
   <211> 491
   <212> PRT
   <213> Artificial Sequence
<220>
   <221> source
   <223> /note="Description of Artificial Sequence: Synthetic polypeptide"
<400> 36
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
   115 120 125
   Gly Gly Gly Gly Ser Gly Gly Gly Gly Ser Gly Gly Gly Gly Ser Gly
   130 135 140
   Gly Gly Gly Ser Gln Val Gln Leu Gln Glu Ser Gly Pro Gly Leu Val
   145 150 155 160
   Lys Pro Ser Glu Thr Leu Ser Leu Thr Cys Thr Val Ser Gly Val Ser
   165 170 175
   Leu Pro Asp Tyr Gly Val Ser Trp Ile Arg Gln Pro Pro Gly Lys Gly
   180 185 190
   Leu Glu Trp Ile Gly Val Ile Trp Gly Ser Glu Thr Thr Tyr Tyr Gln
   195 200 205
   Ser Ser Leu Lys Ser Arg Val Thr Ile Ser Lys Asp Asn Ser Lys Asn
   210 215 220
   Gln Val Ser Leu Lys Leu Ser Ser Val Thr Ala Ala Asp Thr Ala Val
   225 230 235 240
   Tyr Tyr Cys Ala Lys His Tyr Tyr Tyr Gly Gly Ser Tyr Ala Met Asp
   245 250 255
   Tyr Trp Gly Gln Gly Thr Leu Val Thr Val Ser Ser Thr Thr Thr Pro
   260 265 270
   Ala Pro Arg Pro Pro Thr Pro Ala Pro Thr Ile Ala Ser Gln Pro Leu
   275 280 285
   Ser Leu Arg Pro Glu Ala Cys Arg Pro Ala Ala Gly Gly Ala Val His
   290 295 300
   Thr Arg Gly Leu Asp Phe Ala Cys Asp Ile Tyr Ile Trp Ala Pro Leu
   305 310 315 320
   Ala Gly Thr Cys Gly Val Leu Leu Leu Ser Leu Val Ile Thr Leu Tyr
   325 330 335
   Cys Lys Arg Gly Arg Lys Lys Leu Leu Tyr Ile Phe Lys Gln Pro Phe
   340 345 350
   Met Arg Pro Val Gln Thr Thr Gln Glu Glu Asp Gly Cys Ser Cys Arg
   355 360 365
   Phe Pro Glu Glu Glu Glu Gly Gly Cys Glu Leu Arg Val Lys Phe Ser
       370                 375                 380
   Arg Ser Ala Asp Ala Pro Ala Tyr Lys Gln Gly Gln Asn Gln Leu Tyr
   385                 390                 395                 400
   Asn Glu Leu Asn Leu Gly Arg Arg Glu Glu Tyr Asp Val Leu Asp Lys
                   405                 410                 415
   Arg Arg Gly Arg Asp Pro Glu Met Gly Gly Lys Pro Arg Arg Lys Asn
               420                 425                 430
   Pro Gln Glu Gly Leu Tyr Asn Glu Leu Gln Lys Asp Lys Met Ala Glu
           435                 440                 445
   Ala Tyr Ser Glu Ile Gly Met Lys Gly Glu Arg Arg Arg Gly Lys Gly
       450                 455                 460
   His Asp Gly Leu Tyr Gln Gly Leu Ser Thr Ala Thr Lys Asp Thr Tyr
   465                 470                 475                 480
   Asp Ala Leu His Met Gln Ala Leu Pro Pro Arg
                   485                 490
<210> 37
   <211> 491
   <212> PRT
   <213> Artificial Sequence
<220>
   <221> source
   <223> /note="Description of Artificial Sequence: Synthetic polypeptide"
<400> 37
   Met Ala Leu Pro Val Thr Ala Leu Leu Leu Pro Leu Ala Leu Leu Leu
   1               5                   10                  15
   His Ala Ala Arg Pro Gln Val Gln Leu Gln Glu Ser Gly Pro Gly Leu
               20                  25                  30
   Val Lys Pro Ser Glu Thr Leu Ser Leu Thr Cys Thr Val Ser Gly Val
           35                  40                  45
   Ser Leu Pro Asp Tyr Gly Val Ser Trp Ile Arg Gln Pro Pro Gly Lys
       50                  55                  60
   Gly Leu Glu Trp Ile Gly Val Ile Trp Gly Ser Glu Thr Thr Tyr Tyr
   65                  70                  75                  80
   Ser Ser Ser Leu Lys Ser Arg Val Thr Ile Ser Lys Asp Asn Ser Lys
                   85                  90                  95
   Asn Gln Val Ser Leu Lys Leu Ser Ser Val Thr Ala Ala Asp Thr Ala
               100                 105                 110
   Val Tyr Tyr Cys Ala Lys His Tyr Tyr Tyr Gly Gly Ser Tyr Ala Met
           115                 120                 125
   Asp Tyr Trp Gly Gln Gly Thr Leu Val Thr Val Ser Ser Gly Gly Gly
       130                 135                 140
   Gly Ser Gly Gly Gly Gly Ser Gly Gly Gly Gly Ser Gly Gly Gly Gly
   145                 150                 155                 160
   Ser Glu Ile Val Met Thr Gln Ser Pro Ala Thr Leu Ser Leu Ser Pro
                   165                 170                 175
   Gly Glu Arg Ala Thr Leu Ser Cys Arg Ala Ser Gln Asp Ile Ser Lys
               180                 185                 190
   Tyr Leu Asn Trp Tyr Gln Gln Lys Pro Gly Gln Ala Pro Arg Leu Leu
           195                 200                 205
   Ile Tyr His Thr Ser Arg Leu His Ser Gly Ile Pro Ala Arg Phe Ser
       210                 215                 220
   Gly Ser Gly Ser Gly Thr Asp Tyr Thr Leu Thr Ile Ser Ser Leu Gln
   225                 230                 235                 240
   Pro Glu Asp Phe Ala Val Tyr Phe Cys Gln Gln Gly Asn Thr Leu Pro
                   245                 250                 255
   Tyr Thr Phe Gly Gln Gly Thr Lys Leu Glu Ile Lys Thr Thr Thr Pro
               260                 265                 270
   Ala Pro Arg Pro Pro Thr Pro Ala Pro Thr Ile Ala Ser Gln Pro Leu
           275                 280                 285
   Ser Leu Arg Pro Glu Ala Cys Arg Pro Ala Ala Gly Gly Ala Val His
       290                 295                 300
   Thr Arg Gly Leu Asp Phe Ala Cys Asp Ile Tyr Ile Trp Ala Pro Leu
   305                 310                 315                 320
   Ala Gly Thr Cys Gly Val Leu Leu Leu Ser Leu Val Ile Thr Leu Tyr
                   325                 330                 335
   Cys Lys Arg Gly Arg Lys Lys Leu Leu Tyr Ile Phe Lys Gln Pro Phe
   340 345 350
   Met Arg Pro Val Gln Thr Thr Gln Glu Glu Asp Gly Cys Ser Cys Arg
   355 360 365
   Phe Pro Glu Glu Glu Glu Gly Gly Cys Glu Leu Arg Val Lys Phe Ser
   370 375 380
   Arg Ser Ala Asp Ala Pro Ala Tyr Lys Gln Gly Gln Asn Gln Leu Tyr
   385 390 395 400
   Asn Glu Leu Asn Leu Gly Arg Arg Glu Glu Tyr Asp Val Leu Asp Lys
   405 410 415
   Arg Arg Gly Arg Asp Pro Glu Met Gly Gly Lys Pro Arg Arg Lys Asn
   420 425 430
   Pro Gln Glu Gly Leu Tyr Asn Glu Leu Gln Lys Asp Lys Met Ala Glu
   435 440 445
   Ala Tyr Ser Glu Ile Gly Met Lys Gly Glu Arg Arg Arg Gly Lys Gly
   450 455 460
   His Asp Gly Leu Tyr Gln Gly Leu Ser Thr Ala Thr Lys Asp Thr Tyr
   465 470 475 480
   Asp Ala Leu His Met Gln Ala Leu Pro Pro Arg
   485 490
<210> 38
   <211> 491
   <212> PRT
   <213> Artificial Sequence
<220>
   <221> source
   <223> /note="Description of Artificial Sequence: Synthetic polypeptide"
<400> 38
   Met Ala Leu Pro Val Thr Ala Leu Leu Leu Pro Leu Ala Leu Leu Leu
   1 5 10 15
   His Ala Ala Arg Pro Gln Val Gln Leu Gln Glu Ser Gly Pro Gly Leu
   20 25 30
   Val Lys Pro Ser Glu Thr Leu Ser Leu Thr Cys Thr Val Ser Gly Val
   35 40 45
   Ser Leu Pro Asp Tyr Gly Val Ser Trp Ile Arg Gln Pro Pro Gly Lys
       50                  55                  60
   Gly Leu Glu Trp Ile Gly Val Ile Trp Gly Ser Glu Thr Thr Tyr Tyr
   65                  70                  75                  80
   Gln Ser Ser Leu Lys Ser Arg Val Thr Ile Ser Lys Asp Asn Ser Lys
                   85                  90                  95
   Asn Gln Val Ser Leu Lys Leu Ser Ser Val Thr Ala Ala Asp Thr Ala
               100                 105                 110
   Val Tyr Tyr Cys Ala Lys His Tyr Tyr Tyr Gly Gly Ser Tyr Ala Met
           115                 120                 125
   Asp Tyr Trp Gly Gln Gly Thr Leu Val Thr Val Ser Ser Gly Gly Gly
       130                 135                 140
   Gly Ser Gly Gly Gly Gly Ser Gly Gly Gly Gly Ser Gly Gly Gly Gly
   145                 150                 155                 160
   Ser Glu Ile Val Met Thr Gln Ser Pro Ala Thr Leu Ser Leu Ser Pro
                   165                 170                 175
   Gly Glu Arg Ala Thr Leu Ser Cys Arg Ala Ser Gln Asp Ile Ser Lys
               180                 185                 190
   Tyr Leu Asn Trp Tyr Gln Gln Lys Pro Gly Gln Ala Pro Arg Leu Leu
           195                 200                 205
   Ile Tyr His Thr Ser Arg Leu His Ser Gly Ile Pro Ala Arg Phe Ser
       210                 215                 220
   Gly Ser Gly Ser Gly Thr Asp Tyr Thr Leu Thr Ile Ser Ser Leu Gln
   225                 230                 235                 240
   Pro Glu Asp Phe Ala Val Tyr Phe Cys Gln Gln Gly Asn Thr Leu Pro
                   245                 250                 255
   Tyr Thr Phe Gly Gln Gly Thr Lys Leu Glu Ile Lys Thr Thr Thr Pro
               260                 265                 270
   Ala Pro Arg Pro Pro Thr Pro Ala Pro Thr Ile Ala Ser Gln Pro Leu
           275                 280                 285
   Ser Leu Arg Pro Glu Ala Cys Arg Pro Ala Ala Gly Gly Ala Val His
       290                 295                 300
   Thr Arg Gly Leu Asp Phe Ala Cys Asp Ile Tyr Ile Trp Ala Pro Leu
   305 310 315 320
   Ala Gly Thr Cys Gly Val Leu Leu Leu Ser Leu Val Ile Thr Leu Tyr
   325 330 335
   Cys Lys Arg Gly Arg Lys Lys Leu Leu Tyr Ile Phe Lys Gln Pro Phe
   340 345 350
   Met Arg Pro Val Gln Thr Thr Gln Glu Glu Asp Gly Cys Ser Cys Arg
   355 360 365
   Phe Pro Glu Glu Glu Glu Gly Gly Cys Glu Leu Arg Val Lys Phe Ser
   370 375 380
   Arg Ser Ala Asp Ala Pro Ala Tyr Lys Gln Gly Gln Asn Gln Leu Tyr
   385 390 395 400
   Asn Glu Leu Asn Leu Gly Arg Arg Glu Glu Tyr Asp Val Leu Asp Lys
   405 410 415
   Arg Arg Gly Arg Asp Pro Glu Met Gly Gly Lys Pro Arg Arg Lys Asn
   420 425 430
   Pro Gln Glu Gly Leu Tyr Asn Glu Leu Gln Lys Asp Lys Met Ala Glu
   435 440 445
   Ala Tyr Ser Glu Ile Gly Met Lys Gly Glu Arg Arg Arg Gly Lys Gly
   450 455 460
   His Asp Gly Leu Tyr Gln Gly Leu Ser Thr Ala Thr Lys Asp Thr Tyr
   465 470 475 480
   Asp Ala Leu His Met Gln Ala Leu Pro Pro Arg
   485 490
<210> 39
   <211> 491
   <212> PRT
   <213> Artificial Sequence
<220>
   <221> source
   <223> /note="Description of Artificial Sequence: Synthetic polypeptide"
<400> 39
   Met Ala Leu Pro Val Thr Ala Leu Leu Leu Pro Leu Ala Leu Leu Leu
   1 5 10 15
   His Ala Ala Arg Pro Glu Ile Val Met Thr Gln Ser Pro Ala Thr Leu
   20 25 30
   Ser Leu Ser Pro Gly Glu Arg Ala Thr Leu Ser Cys Arg Ala Ser Gln
   35 40 45
   Asp Ile Ser Lys Tyr Leu Asn Trp Tyr Gln Gln Lys Pro Gly Gln Ala
   50 55 60
   Pro Arg Leu Leu Ile Tyr His Thr Ser Arg Leu His Ser Gly Ile Pro
   65 70 75 80
   Ala Arg Phe Ser Gly Ser Gly Ser Gly Thr Asp Tyr Thr Leu Thr Ile
   85 90 95
   Ser Ser Leu Gln Pro Glu Asp Phe Ala Val Tyr Phe Cys Gln Gln Gly
   100 105 110
   Asn Thr Leu Pro Tyr Thr Phe Gly Gln Gly Thr Lys Leu Glu Ile Lys
   115 120 125
   Gly Gly Gly Gly Ser Gly Gly Gly Gly Ser Gly Gly Gly Gly Ser Gly
   130 135 140
   Gly Gly Gly Ser Gln Val Gln Leu Gln Glu Ser Gly Pro Gly Leu Val
   145 150 155 160
   Lys Pro Ser Glu Thr Leu Ser Leu Thr Cys Thr Val Ser Gly Val Ser
   165 170 175
   Leu Pro Asp Tyr Gly Val Ser Trp Ile Arg Gln Pro Pro Gly Lys Gly
   180 185 190
   Leu Glu Trp Ile Gly Val Ile Trp Gly Ser Glu Thr Thr Tyr Tyr Asn
   195 200 205
   Ser Ser Leu Lys Ser Arg Val Thr Ile Ser Lys Asp Asn Ser Lys Asn
   210 215 220
   Gln Val Ser Leu Lys Leu Ser Ser Val Thr Ala Ala Asp Thr Ala Val
   225 230 235 240
   Tyr Tyr Cys Ala Lys His Tyr Tyr Tyr Gly Gly Ser Tyr Ala Met Asp
   245 250 255
   Tyr Trp Gly Gln Gly Thr Leu Val Thr Val Ser Ser Thr Thr Thr Pro
               260                 265                 270
   Ala Pro Arg Pro Pro Thr Pro Ala Pro Thr Ile Ala Ser Gln Pro Leu
           275                 280                 285
   Ser Leu Arg Pro Glu Ala Cys Arg Pro Ala Ala Gly Gly Ala Val His
       290                 295                 300
   Thr Arg Gly Leu Asp Phe Ala Cys Asp Ile Tyr Ile Trp Ala Pro Leu
   305                 310                 315                 320
   Ala Gly Thr Cys Gly Val Leu Leu Leu Ser Leu Val Ile Thr Leu Tyr
                   325                 330                 335
   Cys Lys Arg Gly Arg Lys Lys Leu Leu Tyr Ile Phe Lys Gln Pro Phe
               340                 345                 350
   Met Arg Pro Val Gln Thr Thr Gln Glu Glu Asp Gly Cys Ser Cys Arg
           355                 360                 365
   Phe Pro Glu Glu Glu Glu Gly Gly Cys Glu Leu Arg Val Lys Phe Ser
       370                 375                 380
   Arg Ser Ala Asp Ala Pro Ala Tyr Lys Gln Gly Gln Asn Gln Leu Tyr
   385                 390                 395                 400
   Asn Glu Leu Asn Leu Gly Arg Arg Glu Glu Tyr Asp Val Leu Asp Lys
                   405                 410                 415
   Arg Arg Gly Arg Asp Pro Glu Met Gly Gly Lys Pro Arg Arg Lys Asn
               420                 425                 430
   Pro Gln Glu Gly Leu Tyr Asn Glu Leu Gln Lys Asp Lys Met Ala Glu
           435                 440                 445
   Ala Tyr Ser Glu Ile Gly Met Lys Gly Glu Arg Arg Arg Gly Lys Gly
       450                 455                 460
   His Asp Gly Leu Tyr Gln Gly Leu Ser Thr Ala Thr Lys Asp Thr Tyr
   465                 470                 475                 480
   Asp Ala Leu His Met Gln Ala Leu Pro Pro Arg
                   485                 490
<210> 40
   <211> 491
   <212> PRT
   <213> Artificial Sequence
<220>
   <221> source
   <223> /note="Description of Artificial Sequence: Synthetic polypeptide"
<400> 40
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gly
       130                 135                 140
   Gly Gly Gly Ser Gln Val Gln Leu Gln Glu Ser Gly Pro Gly Leu Val
   145                 150                 155                 160
   Lys Pro Ser Glu Thr Leu Ser Leu Thr Cys Thr Val Ser Gly Val Ser
                   165                 170                 175
   Leu Pro Asp Tyr Gly Val Ser Trp Ile Arg Gln Pro Pro Gly Lys Gly
               180                 185                 190
   Leu Glu Trp Ile Gly Val Ile Trp Gly Ser Glu Thr Thr Tyr Tyr Asn
           195                 200                 205
   Ser Ser Leu Lys Ser Arg Val Thr Ile Ser Lys Asp Asn Ser Lys Asn
       210                 215                 220
   Gln Val Ser Leu Lys Leu Ser Ser Val Thr Ala Ala Asp Thr Ala Val
   225                 230                 235                 240
   Tyr Tyr Cys Ala Lys His Tyr Tyr Tyr Gly Gly Ser Tyr Ala Met Asp
                   245                 250                 255
   Tyr Trp Gly Gln Gly Thr Leu Val Thr Val Ser Ser Thr Thr Thr Pro
               260                 265                 270
   Ala Pro Arg Pro Pro Thr Pro Ala Pro Thr Ile Ala Ser Gln Pro Leu
           275                 280                 285
   Ser Leu Arg Pro Glu Ala Cys Arg Pro Ala Ala Gly Gly Ala Val His
       290                 295                 300
   Thr Arg Gly Leu Asp Phe Ala Cys Asp Ile Tyr Ile Trp Ala Pro Leu
   305                 310                 315                 320
   Ala Gly Thr Cys Gly Val Leu Leu Leu Ser Leu Val Ile Thr Leu Tyr
                   325                 330                 335
   Cys Lys Arg Gly Arg Lys Lys Leu Leu Tyr Ile Phe Lys Gln Pro Phe
               340                 345                 350
   Met Arg Pro Val Gln Thr Thr Gln Glu Glu Asp Gly Cys Ser Cys Arg
           355                 360                 365
   Phe Pro Glu Glu Glu Glu Gly Gly Cys Glu Leu Arg Val Lys Phe Ser
       370                 375                 380
   Arg Ser Ala Asp Ala Pro Ala Tyr Lys Gln Gly Gln Asn Gln Leu Tyr
   385                 390                 395                 400
   Asn Glu Leu Asn Leu Gly Arg Arg Glu Glu Tyr Asp Val Leu Asp Lys
                   405                 410                 415
   Arg Arg Gly Arg Asp Pro Glu Met Gly Gly Lys Pro Arg Arg Lys Asn
               420                 425                 430
   Pro Gln Glu Gly Leu Tyr Asn Glu Leu Gln Lys Asp Lys Met Ala Glu
           435                 440                 445
   Ala Tyr Ser Glu Ile Gly Met Lys Gly Glu Arg Arg Arg Gly Lys Gly
       450                 455                 460
   His Asp Gly Leu Tyr Gln Gly Leu Ser Thr Ala Thr Lys Asp Thr Tyr
   465                 470                 475                 480
   Asp Ala Leu His Met Gln Ala Leu Pro Pro Arg
   485 490
<210> 41
   <211> 491
   <212> PRT
   <213> Artificial Sequence
<220>
   <221> source
   <223> /note="Description of Artificial Sequence: Synthetic polypeptide"
<400> 41
   Met Ala Leu Pro Val Thr Ala Leu Leu Leu Pro Leu Ala Leu Leu Leu
   1 5 10 15
   His Ala Ala Arg Pro Gln Val Gln Leu Gln Glu Ser Gly Pro Gly Leu
   20 25 30
   Val Lys Pro Ser Glu Thr Leu Ser Leu Thr Cys Thr Val Ser Gly Val
   35 40 45
   Ser Leu Pro Asp Tyr Gly Val Ser Trp Ile Arg Gln Pro Pro Gly Lys
   50 55 60
   Gly Leu Glu Trp Ile Gly Val Ile Trp Gly Ser Glu Thr Thr Tyr Tyr
   65 70 75 80
   Asn Ser Ser Leu Lys Ser Arg Val Thr Ile Ser Lys Asp Asn Ser Lys
   85 90 95
   Asn Gln Val Ser Leu Lys Leu Ser Ser Val Thr Ala Ala Asp Thr Ala
   100 105 110
   Val Tyr Tyr Cys Ala Lys His Tyr Tyr Tyr Gly Gly Ser Tyr Ala Met
   115 120 125
   Asp Tyr Trp Gly Gln Gly Thr Leu Val Thr Val Ser Ser Gly Gly Gly
   130 135 140
   Gly Ser Gly Gly Gly Gly Ser Gly Gly Gly Gly Ser Gly Gly Gly Gly
   145 150 155 160
   Ser Glu Ile Val Met Thr Gln Ser Pro Ala Thr Leu Ser Leu Ser Pro
   165 170 175
   Gly Glu Arg Ala Thr Leu Ser Cys Arg Ala Ser Gln Asp Ile Ser Lys
   180 185 190
   Tyr Leu Asn Trp Tyr Gln Gln Lys Pro Gly Gln Ala Pro Arg Leu Leu
           195                 200                 205
   Ile Tyr His Thr Ser Arg Leu His Ser Gly Ile Pro Ala Arg Phe Ser
       210                 215                 220
   Gly Ser Gly Ser Gly Thr Asp Tyr Thr Leu Thr Ile Ser Ser Leu Gln
   225                 230                 235                 240
   Pro Glu Asp Phe Ala Val Tyr Phe Cys Gln Gln Gly Asn Thr Leu Pro
                   245                 250                 255
   Tyr Thr Phe Gly Gln Gly Thr Lys Leu Glu Ile Lys Thr Thr Thr Pro
               260                 265                 270
   Ala Pro Arg Pro Pro Thr Pro Ala Pro Thr Ile Ala Ser Gln Pro Leu
           275                 280                 285
   Ser Leu Arg Pro Glu Ala Cys Arg Pro Ala Ala Gly Gly Ala Val His
       290                 295                 300
   Thr Arg Gly Leu Asp Phe Ala Cys Asp Ile Tyr Ile Trp Ala Pro Leu
   305                 310                 315                 320
   Ala Gly Thr Cys Gly Val Leu Leu Leu Ser Leu Val Ile Thr Leu Tyr
                   325                 330                 335
   Cys Lys Arg Gly Arg Lys Lys Leu Leu Tyr Ile Phe Lys Gln Pro Phe
               340                 345                 350
   Met Arg Pro Val Gln Thr Thr Gln Glu Glu Asp Gly Cys Ser Cys Arg
           355                 360                 365
   Phe Pro Glu Glu Glu Glu Gly Gly Cys Glu Leu Arg Val Lys Phe Ser
       370                 375                 380
   Arg Ser Ala Asp Ala Pro Ala Tyr Lys Gln Gly Gln Asn Gln Leu Tyr
   385                 390                 395                 400
   Asn Glu Leu Asn Leu Gly Arg Arg Glu Glu Tyr Asp Val Leu Asp Lys
                   405                 410                 415
   Arg Arg Gly Arg Asp Pro Glu Met Gly Gly Lys Pro Arg Arg Lys Asn
               420                 425                 430
   Pro Gln Glu Gly Leu Tyr Asn Glu Leu Gln Lys Asp Lys Met Ala Glu
           435                 440                 445
   Ala Tyr Ser Glu Ile Gly Met Lys Gly Glu Arg Arg Arg Gly Lys Gly
       450                 455                 460
   His Asp Gly Leu Tyr Gln Gly Leu Ser Thr Ala Thr Lys Asp Thr Tyr
   465                 470                 475                 480
   Asp Ala Leu His Met Gln Ala Leu Pro Pro Arg
                   485                 490
<210> 42
   <211> 486
   <212> PRT
   <213> Artificial Sequence
<220>
   <221> source
   <223> /note="Description of Artificial Sequence: Synthetic polypeptide"
<400> 42
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Asn Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Leu Val Thr Val Ser Ser Thr Thr Thr Pro Ala Pro Arg Pro Pro
               260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Lys Arg Gly Arg
                   325                 330                 335
   Lys Lys Leu Leu Tyr Ile Phe Lys Gln Pro Phe Met Arg Pro Val Gln
               340                 345                 350
   Thr Thr Gln Glu Glu Asp Gly Cys Ser Cys Arg Phe Pro Glu Glu Glu
           355                 360                 365
   Glu Gly Gly Cys Glu Leu Arg Val Lys Phe Ser Arg Ser Ala Asp Ala
       370                 375                 380
   Pro Ala Tyr Lys Gln Gly Gln Asn Gln Leu Tyr Asn Glu Leu Asn Leu
   385                 390                 395                 400
   Gly Arg Arg Glu Glu Tyr Asp Val Leu Asp Lys Arg Arg Gly Arg Asp
   405 410 415
   Pro Glu Met Gly Gly Lys Pro Arg Arg Lys Asn Pro Gln Glu Gly Leu
   420 425 430
   Tyr Asn Glu Leu Gln Lys Asp Lys Met Ala Glu Ala Tyr Ser Glu Ile
   435 440 445
   Gly Met Lys Gly Glu Arg Arg Arg Gly Lys Gly His Asp Gly Leu Tyr
   450 455 460
   Gln Gly Leu Ser Thr Ala Thr Lys Asp Thr Tyr Asp Ala Leu His Met
   465 470 475 480
   Gln Ala Leu Pro Pro Arg
   485
<210> 43
   <211> 112
   <212> PRT
   <213> Homo sapiens
<400> 43
   Arg Val Lys Phe Ser Arg Ser Ala Asp Ala Pro Ala Tyr Gln Gln Gly
   1 5 10 15
   Gln Asn Gln Leu Tyr Asn Glu Leu Asn Leu Gly Arg Arg Glu Glu Tyr
   20 25 30
   Asp Val Leu Asp Lys Arg Arg Gly Arg Asp Pro Glu Met Gly Gly Lys
   35 40 45
   Pro Arg Arg Lys Asn Pro Gln Glu Gly Leu Tyr Asn Glu Leu Gln Lys
   50 55 60
   Asp Lys Met Ala Glu Ala Tyr Ser Glu Ile Gly Met Lys Gly Glu Arg
   65 70 75 80
   Arg Arg Gly Lys Gly His Asp Gly Leu Tyr Gln Gly Leu Ser Thr Ala
   85 90 95
   Thr Lys Asp Thr Tyr Asp Ala Leu His Met Gln Ala Leu Pro Pro Arg
   100 105 110
<210> 44
   <211> 336
   <212> DNA
   <213> Homo sapiens
<400> 44
   agagtgaagt tcagcaggag cgcagacgcc cccgcgtacc agcagggcca gaaccagctc     60
   tataacgagc tcaatctagg acgaagagag gagtacgatg ttttggacaa gagacgtggc    120
   cgggaccctg agatgggggg aaagccgaga aggaagaacc ctcaggaagg cctgtacaat    180
   gaactgcaga aagataagat ggcggaggcc tacagtgaga ttgggatgaa aggcgagcgc    240
   cggaggggca aggggcacga tggcctttac cagggtctca gtacagccac caaggacacc    300
   tacgacgccc ttcacatgca ggccctgccc cctcgc                              336
<210> 45
   <211> 230
   <212> PRT
   <213> Artificial Sequence
<220>
   <221> source
   <223> /note="Description of Artificial Sequence: Synthetic polypeptide"
<400> 45
   Glu Ser Lys Tyr Gly Pro Pro Cys Pro Pro Cys Pro Ala Pro Glu Phe
   1               5                   10                  15
   Leu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Pro Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Thr Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Tyr Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys Met
   225                 230
<210> 46
   <211> 690
   <212> DNA
   <213> Artificial Sequence
<220>
   <221> source
   <223> /note="Description of Artificial Sequence: Synthetic polynucleotide"
<400> 46
   gagagcaagt acggccctcc ctgcccccct tgccctgccc ccgagttcct gggcggaccc        60
   agcgtgttcc tgttcccccc caagcccaag gacaccctga tgatcagccg gacccccgag       120
   gtgacctgtg tggtggtgga cgtgtcccag gaggaccccg aggtccagtt caactggtac       180
   gtggacggcg tggaggtgca caacgccaag accaagcccc gggaggagca gttcaatagc       240
   acctaccggg tggtgtccgt gctgaccgtg ctgcaccagg actggctgaa cggcaaggaa       300
   tacaagtgta aggtgtccaa caagggcctg cccagcagca tcgagaaaac catcagcaag       360
   gccaagggcc agcctcggga gccccaggtg tacaccctgc cccctagcca agaggagatg       420
   accaagaacc aggtgtccct gacctgcctg gtgaagggct tctaccccag cgacatcgcc       480
   gtggagtggg agagcaacgg ccagcccgag aacaactaca agaccacccc ccctgtgctg       540
   gacagcgacg gcagcttctt cctgtacagc cggctgaccg tggacaagag ccggtggcag       600
   gagggcaacg tctttagctg ctccgtgatg cacgaggccc tgcacaacca ctacacccag       660
   aagagcctga gcctgtccct gggcaagatg                                        690
<210> 47
   <211> 282
   <212> PRT
   <213> Artificial Sequence
<220>
   <221> source
   <223> /note="Description of Artificial Sequence: Synthetic polypeptide"
<400> 47
   Arg Trp Pro Glu Ser Pro Lys Ala Gln Ala Ser Ser Val Pro Thr Ala
   1               5                   10                  15
   Gln Pro Gln Ala Glu Gly Ser Leu Ala Lys Ala Thr Thr Ala Pro Ala
               20                  25                  30
   Thr Thr Arg Asn Thr Gly Arg Gly Gly Glu Glu Lys Lys Lys Glu Lys
           35                  40                  45
   Glu Lys Glu Glu Gln Glu Glu Arg Glu Thr Lys Thr Pro Glu Cys Pro
       50                  55                  60
   Ser His Thr Gln Pro Leu Gly Val Tyr Leu Leu Thr Pro Ala Val Gln
   65                  70                  75                  80
   Asp Leu Trp Leu Arg Asp Lys Ala Thr Phe Thr Cys Phe Val Val Gly
                   85                  90                  95
   Ser Asp Leu Lys Asp Ala His Leu Thr Trp Glu Val Ala Gly Lys Val
               100                 105                 110
   Pro Thr Gly Gly Val Glu Glu Gly Leu Leu Glu Arg His Ser Asn Gly
           115                 120                 125
   Ser Gln Ser Gln His Ser Arg Leu Thr Leu Pro Arg Ser Leu Trp Asn
       130                 135                 140
   Ala Gly Thr Ser Val Thr Cys Thr Leu Asn His Pro Ser Leu Pro Pro
   145                 150                 155                 160
   Gln Arg Leu Met Ala Leu Arg Glu Pro Ala Ala Gln Ala Pro Val Lys
                   165                 170                 175
   Leu Ser Leu Asn Leu Leu Ala Ser Ser Asp Pro Pro Glu Ala Ala Ser
               180                 185                 190
   Trp Leu Leu Cys Glu Val Ser Gly Phe Ser Pro Pro Asn Ile Leu Leu
           195                 200                 205
   Met Trp Leu Glu Asp Gln Arg Glu Val Asn Thr Ser Gly Phe Ala Pro
       210                 215                 220
   Ala Arg Pro Pro Pro Gln Pro Gly Ser Thr Thr Phe Trp Ala Trp Ser
   225                 230                 235                 240
   Val Leu Arg Val Pro Ala Pro Pro Ser Pro Gln Pro Ala Thr Tyr Thr
   245 250 255
   Cys Val Val Ser His Glu Asp Ser Arg Thr Leu Leu Asn Ala Ser Arg
   260 265 270
   Ser Leu Glu Val Ser Tyr Val Thr Asp His
   275 280
<210> 48
   <211> 847
   <212> **DNA**
   <213> Artificial Sequence
<220>
   <221> source
   <223> /note="Description of Artificial Sequence: Synthetic polynucleotide"
<400> 48
   aggtggcccg aaagtcccaa ggcccaggca tctagtgttc ctactgcaca gccccaggca 60
   gaaggcagcc tagccaaagc tactactgca cctgccacta cgcgcaatac tggccgtggc 120
   ggggaggaga agaaaaagga gaaagagaaa gaagaacagg aagagaggga gaccaagacc 180
   cctgaatgtc catcccatac ccagccgctg ggcgtctatc tcttgactcc cgcagtacag 240
   gacttgtggc ttagagataa ggccaccttt acatgtttcg tcgtgggctc tgacctgaag 300
   gatgcccatt tgacttggga ggttgccgga aaggtaccca cagggggggt tgaggaaggg 360
   ttgctggagc gccattccaa tggctctcag agccagcact caagactcac ccttccgaga 420
   tccctgtgga acgccgggac ctctgtcaca tgtactctaa atcatcctag cctgccccca 480
   cagcgtctga tggcccttag agagccagcc gcccaggcac cagttaagct tagcctgaat 540
   ctgctcgcca gtagtgatcc cccagaggcc gccagctggc tcttatgcga agtgtccggc 600
   tttagcccgc ccaacatctt gctcatgtgg ctggaggacc agcgagaagt gaacaccagc 660
   ggcttcgctc cagcccggcc cccaccccag ccgggttcta ccacattctg ggcctggagt 720
   gtcttaaggg tcccagcacc acctagcccc cagccagcca catacacctg tgttgtgtcc 780
   catgaagata gcaggaccct gctaaatgct tctaggagtc tggaggtttc ctacgtgact 840
   gaccatt 847
<210> 49
   <211> 10
   <212> PRT
   <213> Artificial Sequence
<220>
   <221> source
   <223> /note="Description of Artificial Sequence: Synthetic peptide"
<400> 49
   Gly Gly Gly Gly Ser Gly Gly Gly Gly Ser
   1 5 10
<210> 50
   <211> 30
   <212> DNA
   <213> Artificial Sequence
<220>
   <221> source
   <223> /note="Description of Artificial Sequence: Synthetic oligonucleotide"
<400> 50
   ggtggcggag gttctggagg tggaggttcc 30
<210> 51
   <211> 48
   <212> PRT
   <213> Artificial Sequence
<220>
   <221> source
   <223> /note="Description of Artificial Sequence: Synthetic polypeptide"
<400> 51
   Gln Arg Arg Lys Tyr Arg Ser Asn Lys Gly Glu Ser Pro Val Glu Pro
   1 5 10 15
   Ala Glu Pro Cys Arg Tyr Ser Cys Pro Arg Glu Glu Glu Gly Ser Thr
   20 25 30
   Ile Pro Ile Gln Glu Asp Tyr Arg Lys Pro Glu Pro Ala Cys Ser Pro
   35 40 45
<210> 52
   <211> 123
   <212> **DNA**
   <213> Artificial Sequence
<220>
   <221> source
   <223> /note="Description of Artificial Sequence: Synthetic polynucleotide"
<400> 52
   aggagtaaga ggagcaggct cctgcacagt gactacatga acatgactcc ccgccgcccc 60
   gggcccaccc gcaagcatta ccagccctat gccccaccac gcgacttcgc agcctatcgc 120
   tcc 123
<210> 53
   <211> 30
   <212> PRT
   <213> Artificial Sequence
<220>
   <221> source
   <223> /note="Description of Artificial Sequence: Synthetic polypeptide"
<220>
   <221> misc_feature
   <222> (1) . . (30)
   <223> /note="This sequence may encompass 1-6 'Gly Gly Gly Gly Ser' repeating units"
<220>
   <221> source
   <223> /note="See specification as filed for detailed description of substitutions and preferred embodiments"
<400> 53
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser
               20                  25                  30
<210> 54
   <211> 63
   <212> DNA
   <213> Artificial Sequence
<220>
   <221> source
   <223> /note="Description of Artificial Sequence: Synthetic oligonucleotide"
<400> 54
   atggccctgc ctgtgacagc cctgctgctg cctctggctc tgctgctgca tgccgctaga       60
   ccc                                                                     63
<210> 55
   <211> 135
   <212> DNA
   <213> Artificial Sequence
<220>
   <221> source
   <223> /note="Description of Artificial Sequence: Synthetic polynucleotide"
<400> 55
   accacgacgc cagcgccgcg accaccaaca ccggcgccca ccatcgcgtc gcagcccctg       60
   tccctgcgcc cagaggcgtg ccggccagcg gcggggggcg cagtgcacac gagggggctg      120
   gacttcgcct gtgat                                                       135
<210> 56
   <211> 72
   <212> DNA
   <213> Artificial Sequence
<220>
   <221> source
   <223> /note="Description of Artificial Sequence: Synthetic oligonucleotide"
<400> 56
   atctacatct gggcgccctt ggccgggact tgtggggtcc ttctcctgtc actggttatc 60
   accctttact gc 72
<210> 57
   <211> 5
   <212> PRT
   <213> Artificial Sequence
<220>
   <221> source
   <223> /note="Description of Artificial Sequence: Synthetic peptide"
<400> 57
   Tyr Asn Ser Ala Leu
   1 5
<210> 58
   <211> 486
   <212> PRT
   <213> Artificial Sequence
<220>
   <221> source
   <223> /note="Description of Artificial Sequence: Synthetic polypeptide"
<400> 58
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Thr Thr Ser Ser Leu
   20 25 30
   Ser Ala Ser Leu Gly Asp Arg Val Thr Ile Ser Cys Arg Ala Ser Gln
   35 40 45
   Asp Ile Ser Lys Tyr Leu Asn Trp Tyr Gln Gln Lys Pro Asp Gly Thr
   50 55 60
   Val Lys Leu Leu Ile Tyr His Thr Ser Arg Leu His Ser Gly Val Pro
   65 70 75 80
   Ser Arg Phe Ser Gly Ser Gly Ser Gly Thr Asp Tyr Ser Leu Thr Ile
                   85                  90                  95
   Ser Asn Leu Glu Gln Glu Asp Ile Ala Thr Tyr Phe Cys Gln Gln Gly
               100                 105                 110
   Asn Thr Leu Pro Tyr Thr Phe Gly Gly Gly Thr Lys Leu Glu Ile Thr
           115                 120                 125
   Gly Gly Gly Gly Ser Gly Gly Gly Gly Ser Gly Gly Gly Gly Ser Glu
       130                 135                 140
   Val Lys Leu Gln Glu Ser Gly Pro Gly Leu Val Ala Pro Ser Gln Ser
   145                 150                 155                 160
   Leu Ser Val Thr Cys Thr Val Ser Gly Val Ser Leu Pro Asp Tyr Gly
                   165                 170                 175
   Val Ser Trp Ile Arg Gln Pro Pro Arg Lys Gly Leu Glu Trp Leu Gly
               180                 185                 190
   Val Ile Trp Gly Ser Glu Thr Thr Tyr Tyr Asn Ser Ala Leu Lys Ser
           195                 200                 205
   Arg Leu Thr Ile Ile Lys Asp Asn Ser Lys Ser Gln Val Phe Leu Lys
       210                 215                 220
   Met Asn Ser Leu Gln Thr Asp Asp Thr Ala Ile Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Ser Val Thr Val Ser Ser Thr Thr Thr Pro Ala Pro Arg Pro Pro
               260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Lys Arg Gly Arg
                   325                 330                 335
   Lys Lys Leu Leu Tyr Ile Phe Lys Gln Pro Phe Met Arg Pro Val Gln
   340 345 350
   Thr Thr Gln Glu Glu Asp Gly Cys Ser Cys Arg Phe Pro Glu Glu Glu
   355 360 365
   Glu Gly Gly Cys Glu Leu Arg Val Lys Phe Ser Arg Ser Ala Asp Ala
   370 375 380
   Pro Ala Tyr Lys Gln Gly Gln Asn Gln Leu Tyr Asn Glu Leu Asn Leu
   385 390 395 400
   Gly Arg Arg Glu Glu Tyr Asp Val Leu Asp Lys Arg Arg Gly Arg Asp
   405 410 415
   Pro Glu Met Gly Gly Lys Pro Arg Arg Lys Asn Pro Gln Glu Gly Leu
   420 425 430
   Tyr Asn Glu Leu Gln Lys Asp Lys Met Ala Glu Ala Tyr Ser Glu Ile
   435 440 445
   Gly Met Lys Gly Glu Arg Arg Arg Gly Lys Gly His Asp Gly Leu Tyr
   450 455 460
   Gln Gly Leu Ser Thr Ala Thr Lys Asp Thr Tyr Asp Ala Leu His Met
   465 470 475 480
   Gln Ala Leu Pro Pro Arg
   485
<210> 59
   <211> 242
   <212> PRT
   <213> Artificial Sequence
<220>
   <221> source
   <223> /note="Description of Artificial Sequence: Synthetic polypeptide"
<400> 59
   Asp Ile Gln Met Thr Gln Thr Thr Ser Ser Leu Ser Ala Ser Leu Gly
   1 5 10 15
   Asp Arg Val Thr Ile Ser Cys Arg Ala Ser Gln Asp Ile Ser Lys Tyr
   20 25 30
   Leu Asn Trp Tyr Gln Gln Lys Pro Asp Gly Thr Val Lys Leu Leu Ile
   35 40 45
   Tyr His Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Leu Pro Tyr
                   85                  90                  95
   Thr Phe Gly Gly Gly Thr Lys Leu Glu Ile Thr Gly Gly Gly Gly Ser
               100                 105                 110
   Gly Gly Gly Gly Ser Gly Gly Gly Gly Ser Glu Val Lys Leu Gln Glu
           115                 120                 125
   Ser Gly Pro Gly Leu Val Ala Pro Ser Gln Ser Leu Ser Val Thr Cys
       130                 135                 140
   Thr Val Ser Gly Val Ser Leu Pro Asp Tyr Gly Val Ser Trp Ile Arg
   145                 150                 155                 160
   Gln Pro Pro Arg Lys Gly Leu Glu Trp Leu Gly Val Ile Trp Gly Ser
                   165                 170                 175
   Glu Thr Thr Tyr Tyr Asn Ser Ala Leu Lys Ser Arg Leu Thr Ile Ile
               180                 185                 190
   Lys Asp Asn Ser Lys Ser Gln Val Phe Leu Lys Met Asn Ser Leu Gln
           195                 200                 205
   Thr Asp Asp Thr Ala Ile Tyr Tyr Cys Ala Lys His Tyr Tyr Tyr Gly
       210                 215                 220
   Gly Ser Tyr Ala Met Asp Tyr Trp Gly Gln Gly Thr Ser Val Thr Val
   225                 230                 235                 240
   Ser Ser
<210> 60
   <211> 126
   <212> DNA
   <213> Artificial Sequence
<220>
   <221> source
   <223> /note="Description of Artificial Sequence: Synthetic polynucleotide"
<400> 60
   aaacggggca gaaagaaact cctgtatata ttcaaacaac catttatgag accagtacaa 60
   actactcaag aggaagatgg ctgtagctgc cgatttccag aagaagaaga aggaggatgt 120
   gaactg 126
<210> 61
   <211> 813
   <212> DNA
   <213> Artificial Sequence
<220>
   <221> source
   <223> /note="Description of Artificial Sequence: Synthetic polynucleotide"
<400> 61
   atggccctcc ctgtcaccgc cctgctgctt ccgctggctc ttctgctcca cgccgctcgg 60
   cccgaaattg tgatgaccca gtcacccgcc actcttagcc tttcacccgg tgagcgcgca 120
   accctgtctt gcagagcctc ccaagacatc tcaaaatacc ttaattggta tcaacagaag 180
   cccggacagg ctcctcgcct tctgatctac cacaccagcc ggctccattc tggaatccct 240
   gccaggttca gcggtagcgg atctgggacc gactacaccc tcactatcag ctcactgcag 300
   ccagaggact tcgctgtcta tttctgtcag caagggaaca ccctgcccta cacctttgga 360
   cagggcacca agctcgagat taaaggtgga ggtggcagcg gaggaggtgg gtccggcggt 420
   ggaggaagcc aggtccaact ccaagaaagc ggaccgggtc ttgtgaagcc atcagaaact 480
   ctttcactga cttgtactgt gagcggagtg tctctccccg attacggggt gtcttggatc 540
   agacagccac cggggaaggg tctggaatgg attggagtga tttggggctc tgagactact 600
   tactactctt catccctcaa gtcacgcgtc accatctcaa aggacaactc taagaatcag 660
   gtgtcactga aactgtcatc tgtgaccgca gccgacaccg ccgtgtacta ttgcgctaag 720
   cattactatt atggcgggag ctacgcaatg gattactggg gacagggtac tctggtcacc 780
   gtgtccagcc accaccatca tcaccatcac cat 813
<210> 62
   <211> 813
   <212> DNA
   <213> Artificial Sequence
<220>
   <221> source
   <223> /note="Description of Artificial Sequence: Synthetic polynucleotide"
<400> 62
   atggccctcc ctgtcaccgc cctgctgctt ccgctggctc ttctgctcca cgccgctcgg 60
   cccgaaattg tgatgaccca gtcacccgcc actcttagcc tttcacccgg tgagcgcgca 120
   accctgtctt gcagagcctc ccaagacatc tcaaaatacc ttaattggta tcaacagaag 180
   cccggacagg ctcctcgcct tctgatctac cacaccagcc ggctccattc tggaatccct 240
   gccaggttca gcggtagcgg atctgggacc gactacaccc tcactatcag ctcactgcag 300
   ccagaggact tcgctgtcta tttctgtcag caagggaaca ccctgcccta cacctttgga 360
   cagggcacca agctcgagat taaaggtgga ggtggcagcg gaggaggtgg gtccggcggt 420
   ggaggaagcc aggtccaact ccaagaaagc ggaccgggtc ttgtgaagcc atcagaaact 480
   ctttcactga cttgtactgt gagcggagtg tctctccccg attacggggt gtcttggatc 540
   agacagccac cggggaaggg tctggaatgg attggagtga tttggggctc tgagactact 600
   tactaccaat catccctcaa gtcacgcgtc accatctcaa aggacaactc taagaatcag 660
   gtgtcactga aactgtcatc tgtgaccgca gccgacaccg ccgtgtacta ttgcgctaag 720
   cattactatt atggcgggag ctacgcaatg gattactggg gacagggtac tctggtcacc 780
   gtgtccagcc accaccatca tcaccatcac cat 813
<210> 63
   <211> 813
   <212> DNA
   <213> Artificial Sequence
<220>
   <221> source
   <223> /note="Description of Artificial Sequence: Synthetic polynucleotide"
<400> 63
   atggctctgc ccgtgaccgc actcctcctg ccactggctc tgctgcttca cgccgctcgc 60
   ccacaagtcc agcttcaaga atcagggcct ggtctggtga agccatctga gactctgtcc 120
   ctcacttgca ccgtgagcgg agtgtccctc ccagactacg gagtgagctg gattagacag 180
   cctcccggaa agggactgga gtggatcgga gtgatttggg gtagcgaaac cacttactat 240
   tcatcttccc tgaagtcacg ggtcaccatt tcaaaggata actcaaagaa tcaagtgagc 300
   ctcaagctct catcagtcac cgccgctgac accgccgtgt attactgtgc caagcattac 360
   tactatggag ggtcctacgc catggactac tggggccagg gaactctggt cactgtgtca 420
   tctggtggag gaggtagcgg aggaggcggg agcggtggag gtggctccga aatcgtgatg 480
   acccagagcc ctgcaaccct gtccctttct cccggggaac gggctaccct ttcttgtcgg 540
   gcatcacaag atatctcaaa atacctcaat tggtatcaac agaagccggg acaggcccct 600
   aggcttctta tctaccacac ctctcgcctg catagcggga ttcccgcacg ctttagcggg 660
   tctggaagcg ggaccgacta cactctgacc atctcatctc tccagcccga ggacttcgcc 720
   gtctacttct gccagcaggg taacaccctg ccgtacacct tcggccaggg caccaagctt 780
   gagatcaaac atcaccacca tcatcaccat cac 813
<210> 64
   <211> 813
   <212> DNA
   <213> Artificial Sequence
<220>
   <221> source
   <223> /note="Description of Artificial Sequence: Synthetic polynucleotide"
<400> 64
   atggctctgc ccgtgaccgc actcctcctg ccactggctc tgctgcttca cgccgctcgc      60
   ccacaagtcc agcttcaaga atcagggcct ggtctggtga agccatctga gactctgtcc     120
   ctcacttgca ccgtgagcgg agtgtccctc ccagactacg gagtgagctg gattagacag     180
   cctcccggaa agggactgga gtggatcgga gtgatttggg gtagcgaaac cacttactat     240
   caatcttccc tgaagtcacg ggtcaccatt tcaaaggata actcaaagaa tcaagtgagc     300
   ctcaagctct catcagtcac cgccgctgac accgccgtgt attactgtgc caagcattac     360
   tactatggag ggtcctacgc catggactac tggggccagg gaactctggt cactgtgtca     420
   tctggtggag gaggtagcgg aggaggcggg agcggtggag gtggctccga aatcgtgatg     480
   acccagagcc ctgcaaccct gtccctttct cccggggaac gggctaccct ttcttgtcgg     540
   gcatcacaag atatctcaaa atacctcaat tggtatcaac agaagccggg acaggcccct     600
   aggcttctta tctaccacac ctctcgcctg catagcggga ttcccgcacg ctttagcggg     660
   tctggaagcg ggaccgacta cactctgacc atctcatctc tccagcccga ggacttcgcc     720
   gtctacttct gccagcaggg taacaccctg ccgtacacct tcggccaggg caccaagctt     780
   gagatcaaac atcaccacca tcatcaccat cac                                  813
<210> 65
   <211> 828
   <212> DNA
   <213> Artificial Sequence
<220>
   <221> source
   <223> /note="Description of Artificial Sequence: Synthetic polynucleotide"
<400> 65
   atggccctcc cagtgaccgc tctgctgctg cctctcgcac ttcttctcca tgccgctcgg      60
   cctgagatcg tcatgaccca aagccccgct accctgtccc tgtcacccgg cgagagggca     120
   accctttcat gcagggccag ccaggacatt tctaagtacc tcaactggta tcagcagaag     180
   ccagggcagg ctcctcgcct gctgatctac cacaccagcc gcctccacag cggtatcccc     240
   gccagatttt ccgggagcgg gtctggaacc gactacaccc tcaccatctc ttctctgcag     300
   cccgaggatt tcgccgtcta tttctgccag caggggaata ctctgccgta caccttcggt 360
   caaggtacca agctggaaat caagggaggc ggaggatcag gcggtggcgg aagcggagga 420
   ggtggctccg gaggaggagg ttcccaagtg cagcttcaag aatcaggacc cggacttgtg 480
   aagccatcag aaaccctctc cctgacttgt accgtgtccg gtgtgagcct ccccgactac 540
   ggagtctctt ggattcgcca gcctccgggg aagggtcttg aatggattgg ggtgatttgg 600
   ggatcagaga ctacttacta ctcttcatca cttaagtcac gggtcaccat cagcaaagat 660
   aatagcaaga accaagtgtc acttaagctg tcatctgtga ccgccgctga caccgccgtg 720
   tactattgtg ccaaacatta ctattacgga gggtcttatg ctatggacta ctggggacag 780
   gggaccctgg tgactgtctc tagccatcac catcaccacc atcatcac 828
<210> 66
   <211> 828
   <212> DNA
   <213> Artificial Sequence
<220>
   <221> source
   <223> /note="Description of Artificial Sequence: Synthetic polynucleotide"
<400> 66
   atggccctcc cagtgaccgc tctgctgctg cctctcgcac ttcttctcca tgccgctcgg 60
   cctgagatcg tcatgaccca aagccccgct accctgtccc tgtcacccgg cgagagggca 120
   accctttcat gcagggccag ccaggacatt tctaagtacc tcaactggta tcagcagaag 180
   ccagggcagg ctcctcgcct gctgatctac cacaccagcc gcctccacag cggtatcccc 240
   gccagatttt ccgggagcgg gtctggaacc gactacaccc tcaccatctc ttctctgcag 300
   cccgaggatt tcgccgtcta tttctgccag caggggaata ctctgccgta caccttcggt 360
   caaggtacca agctggaaat caagggaggc ggaggatcag gcggtggcgg aagcggagga 420
   ggtggctccg gaggaggagg ttcccaagtg cagcttcaag aatcaggacc cggacttgtg 480
   aagccatcag aaaccctctc cctgacttgt accgtgtccg gtgtgagcct ccccgactac 540
   ggagtctctt ggattcgcca gcctccgggg aagggtcttg aatggattgg ggtgatttgg 600
   ggatcagaga ctacttacta ccagtcatca cttaagtcac gggtcaccat cagcaaagat 660
   aatagcaaga accaagtgtc acttaagctg tcatctgtga ccgccgctga caccgccgtg 720
   tactattgtg ccaaacatta ctattacgga gggtcttatg ctatggacta ctggggacag 780
   gggaccctgg tgactgtctc tagccatcac catcaccacc atcatcac 828
<210> 67
   <211> 828
   <212> **DNA**
   <213> Artificial Sequence
<220>
   <221> source
   <223> /note="Description of Artificial Sequence: Synthetic polynucleotide"
<400> 67
   atggcactgc ctgtcactgc cctcctgctg cctctggccc tccttctgca tgccgccagg      60
   ccccaagtcc agctgcaaga gtcaggaccc ggactggtga agccgtctga gactctctca     120
   ctgacttgta ccgtcagcgg cgtgtccctc cccgactacg gagtgtcatg gatccgccaa     180
   cctcccggga aagggcttga atggattggt gtcatctggg gttctgaaac cacctactac     240
   tcatcttccc tgaagtccag ggtgaccatc agcaaggata attccaagaa ccaggtcagc     300
   cttaagctgt catctgtgac cgctgctgac accgccgtgt attactgcgc caagcactac     360
   tattacggag gaagctacgc tatggactat tggggacagg gcactctcgt gactgtgagc     420
   agcggcggtg gagggtctgg aggtggagga tccggtggtg gtgggtcagg cggaggaggg     480
   agcgagattg tgatgactca gtcaccagcc accctttctc tttcacccgg cgagagagca     540
   accctgagct gtagagccag ccaggacatt tctaagtacc tcaactggta tcagcaaaaa     600
   ccggggcagg cccctcgcct cctgatctac catacctcac gccttcactc tggtatcccc     660
   gctcggttta gcggatcagg atctggtacc gactacactc tgaccatttc cagcctgcag     720
   ccagaagatt tcgcagtgta tttctgccag cagggcaata cccttcctta caccttcggt     780
   cagggaacca agctcgaaat caagcaccat caccatcatc accaccat                  828
<210> 68
   <211> 828
   <212> DNA
   <213> Artificial Sequence
<220>
   <221> source
   <223> /note="Description of Artificial Sequence: Synthetic polynucleotide"
<400> 68
   atggcactgc ctgtcactgc cctcctgctg cctctggccc tccttctgca tgccgccagg 60
   ccccaagtcc agctgcaaga gtcaggaccc ggactggtga agccgtctga gactctctca 120
   ctgacttgta ccgtcagcgg cgtgtccctc cccgactacg gagtgtcatg gatccgccaa 180
   cctcccggga aagggcttga atggattggt gtcatctggg gttctgaaac cacctactac 240
   cagtcttccc tgaagtccag ggtgaccatc agcaaggata attccaagaa ccaggtcagc 300
   cttaagctgt catctgtgac cgctgctgac accgccgtgt attactgcgc caagcactac 360
   tattacggag gaagctacgc tatggactat tggggacagg gcactctcgt gactgtgagc 420
   agcggcggtg gagggtctgg aggtggagga tccggtggtg gtgggtcagg cggaggaggg 480
   agcgagattg tgatgactca gtcaccagcc accctttctc tttcacccgg cgagagagca 540
   accctgagct gtagagccag ccaggacatt tctaagtacc tcaactggta tcagcaaaaa 600
   ccggggcagg cccctcgcct cctgatctac catacctcac gccttcactc tggtatcccc 660
   gctcggttta gcggatcagg atctggtacc gactacactc tgaccatttc cagcctgcag 720
   ccagaagatt tcgcagtgta tttctgccag cagggcaata cccttcctta caccttcggt 780
   cagggaacca agctcgaaat caagcaccat caccatcatc atcaccac 828
<210> 69
   <211> 828
   <212> DNA
   <213> Artificial Sequence
<220>
   <221> source
   <223> /note="Description of Artificial Sequence: Synthetic polynucleotide"
<400> 69
   atggccctcc cagtgaccgc tctgctgctg cctctcgcac ttcttctcca tgccgctcgg 60
   cctgagatcg tcatgaccca aagccccgct accctgtccc tgtcacccgg cgagagggca 120
   accctttcat gcagggccag ccaggacatt tctaagtacc tcaactggta tcagcagaag 180
   ccagggcagg ctcctcgcct gctgatctac cacaccagcc gcctccacag cggtatcccc 240
   gccagatttt ccgggagcgg gtctggaacc gactacaccc tcaccatctc ttctctgcag 300
   cccgaggatt tcgccgtcta tttctgccag caggggaata ctctgccgta caccttcggt 360
   caaggtacca agctggaaat caagggaggc ggaggatcag gcggtggcgg aagcggagga 420
   ggtggctccg gaggaggagg ttcccaagtg cagcttcaag aatcaggacc cggacttgtg 480
   aagccatcag aaaccctctc cctgacttgt accgtgtccg gtgtgagcct ccccgactac 540
   ggagtctctt ggattcgcca gcctccgggg aagggtcttg aatggattgg ggtgatttgg 600
   ggatcagaga ctacttacta caattcatca cttaagtcac gggtcaccat cagcaaagat 660
   aatagcaaga accaagtgtc acttaagctg tcatctgtga ccgccgctga caccgccgtg 720
   tactattgtg ccaaacatta ctattacgga gggtcttatg ctatggacta ctggggacag 780
   gggaccctgg tgactgtctc tagccatcac catcaccacc atcatcac 828
<210> 70
   <211> 828
   <212> DNA
   <213> Artificial Sequence
<220>
   <221> source
   <223> /note="Description of Artificial Sequence: Synthetic polynucleotide"
<400> 70
   atggcactgc ctgtcactgc cctcctgctg cctctggccc tccttctgca tgccgccagg 60
   ccccaagtcc agctgcaaga gtcaggaccc ggactggtga agccgtctga gactctctca 120
   ctgacttgta ccgtcagcgg cgtgtccctc cccgactacg gagtgtcatg gatccgccaa 180
   cctcccggga aagggcttga atggattggt gtcatctggg gttctgaaac cacctactac 240
   aactcttccc tgaagtccag ggtgaccatc agcaaggata attccaagaa ccaggtcagc 300
   cttaagctgt catctgtgac cgctgctgac accgccgtgt attactgcgc caagcactac 360
   tattacggag gaagctacgc tatggactat tggggacagg gcactctcgt gactgtgagc 420
   agcggcggtg gagggtctgg aggtggagga tccggtggtg gtgggtcagg cggaggaggg 480
   agcgagattg tgatgactca gtcaccagcc accctttctc tttcacccgg cgagagagca 540
   accctgagct gtagagccag ccaggacatt tctaagtacc tcaactggta tcagcaaaaa 600
   ccggggcagg cccctcgcct cctgatctac catacctcac gccttcactc tggtatcccc 660
   gctcggttta gcggatcagg atctggtacc gactacactc tgaccatttc cagcctgcag 720
   ccagaagatt tcgcagtgta tttctgccag cagggcaata cccttcctta caccttcggt 780
   cagggaacca agctcgaaat caagcaccat caccatcatc accaccat 828
<210> 71
   <211> 813
   <212> DNA
   <213> Artificial Sequence
<220>
   <221> source
   <223> /note="Description of Artificial Sequence: Synthetic polynucleotide"
<400> 71
   atggccctcc ctgtcaccgc cctgctgctt ccgctggctc ttctgctcca cgccgctcgg 60
   cccgaaattg tgatgaccca gtcacccgcc actcttagcc tttcacccgg tgagcgcgca 120
   accctgtctt gcagagcctc ccaagacatc tcaaaatacc ttaattggta tcaacagaag 180
   cccggacagg ctcctcgcct tctgatctac cacaccagcc ggctccattc tggaatccct 240
   gccaggttca gcggtagcgg atctgggacc gactacaccc tcactatcag ctcactgcag 300
   ccagaggact tcgctgtcta tttctgtcag caagggaaca ccctgcccta cacctttgga 360
   cagggcacca agctcgagat taaaggtgga ggtggcagcg gaggaggtgg gtccggcggt 420
   ggaggaagcc aggtccaact ccaagaaagc ggaccgggtc ttgtgaagcc atcagaaact 480
   ctttcactga cttgtactgt gagcggagtg tctctccccg attacggggt gtcttggatc 540
   agacagccac cggggaaggg tctggaatgg attggagtga tttggggctc tgagactact 600
   tactacaatt catccctcaa gtcacgcgtc accatctcaa aggacaactc taagaatcag 660
   gtgtcactga aactgtcatc tgtgaccgca gccgacaccg ccgtgtacta ttgcgctaag 720
   cattactatt atggcgggag ctacgcaatg gattactggg gacagggtac tctggtcacc 780
   gtgtccagcc accaccatca tcaccatcac cat 813
<210> 72
   <211> 813
   <212> DNA
   <213> Artificial Sequence
<220>
   <221> source
   <223> /note="Description of Artificial Sequence: Synthetic polynucleotide"
<400> 72
   atggctctgc ccgtgaccgc actcctcctg ccactggctc tgctgcttca cgccgctcgc 60
   ccacaagtcc agcttcaaga atcagggcct ggtctggtga agccatctga gactctgtcc 120
   ctcacttgca ccgtgagcgg agtgtccctc ccagactacg gagtgagctg gattagacag 180
   cctcccggaa agggactgga gtggatcgga gtgatttggg gtagcgaaac cacttactat 240
   aactcttccc tgaagtcacg ggtcaccatt tcaaaggata actcaaagaa tcaagtgagc 300
   ctcaagctct catcagtcac cgccgctgac accgccgtgt attactgtgc caagcattac 360
   tactatggag ggtcctacgc catggactac tggggccagg gaactctggt cactgtgtca 420
   tctggtggag gaggtagcgg aggaggcggg agcggtggag gtggctccga aatcgtgatg 480
   acccagagcc ctgcaaccct gtccctttct cccggggaac gggctaccct ttcttgtcgg 540
   gcatcacaag atatctcaaa atacctcaat tggtatcaac agaagccggg acaggcccct 600
   aggcttctta tctaccacac ctctcgcctg catagcggga ttcccgcacg ctttagcggg 660
   tctggaagcg ggaccgacta cactctgacc atctcatctc tccagcccga ggacttcgcc 720
   gtctacttct gccagcaggg taacaccctg ccgtacacct tcggccaggg caccaagctt 780
   gagatcaaac atcaccacca tcatcaccat cac 813
<210> 73
   <211> 271
   <212> PRT
   <213> Artificial Sequence
<220>
   <221> source
   <223> /note="Description of Artificial Sequence: Synthetic polypeptide"
<400> 73
   Met Ala Leu Pro Val Thr Ala Leu Leu Leu Pro Leu Ala Leu Leu Leu
   1 5 10 15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Ser Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Leu Val Thr Val Ser Ser His His His His His His His His
               260                 265                 270
<210> 74
   <211> 271
   <212> PRT
   <213> Artificial Sequence
<220>
   <221> source
   <223> /note="Description of Artificial Sequence: Synthetic polypeptide"
<400> 74
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Gln Ser Ser Leu Lys Ser
           195                 200                 205
   Arg Val Thr Ile Ser Lys Asp Asn Ser Lys Asn Gln Val Ser Leu Lys
   210 215 220
   Leu Ser Ser Val Thr Ala Ala Asp Thr Ala Val Tyr Tyr Cys Ala Lys
   225 230 235 240
   His Tyr Tyr Tyr Gly Gly Ser Tyr Ala Met Asp Tyr Trp Gly Gln Gly
   245 250 255
   Thr Leu Val Thr Val Ser Ser His His His His His His His His
   260 265 270
<210> 75
   <211> 271
   <212> PRT
   <213> Artificial Sequence
<220>
   <221> source
   <223> /note="Description of Artificial Sequence: Synthetic polypeptide"
<400> 75
   Met Ala Leu Pro Val Thr Ala Leu Leu Leu Pro Leu Ala Leu Leu Leu
   1 5 10 15
   His Ala Ala Arg Pro Gln Val Gln Leu Gln Glu Ser Gly Pro Gly Leu
   20 25 30
   Val Lys Pro Ser Glu Thr Leu Ser Leu Thr Cys Thr Val Ser Gly Val
   35 40 45
   Ser Leu Pro Asp Tyr Gly Val Ser Trp Ile Arg Gln Pro Pro Gly Lys
   50 55 60
   Gly Leu Glu Trp Ile Gly Val Ile Trp Gly Ser Glu Thr Thr Tyr Tyr
   65 70 75 80
   Ser Ser Ser Leu Lys Ser Arg Val Thr Ile Ser Lys Asp Asn Ser Lys
   85 90 95
   Asn Gln Val Ser Leu Lys Leu Ser Ser Val Thr Ala Ala Asp Thr Ala
   100 105 110
   Val Tyr Tyr Cys Ala Lys His Tyr Tyr Tyr Gly Gly Ser Tyr Ala Met
   115 120 125
   Asp Tyr Trp Gly Gln Gly Thr Leu Val Thr Val Ser Ser Gly Gly Gly
   130 135 140
   Gly Ser Gly Gly Gly Gly Ser Gly Gly Gly Gly Ser Glu Ile Val Met
   145 150 155 160
   Thr Gln Ser Pro Ala Thr Leu Ser Leu Ser Pro Gly Glu Arg Ala Thr
   165 170 175
   Leu Ser Cys Arg Ala Ser Gln Asp Ile Ser Lys Tyr Leu Asn Trp Tyr
   180 185 190
   Gln Gln Lys Pro Gly Gln Ala Pro Arg Leu Leu Ile Tyr His Thr Ser
   195 200 205
   Arg Leu His Ser Gly Ile Pro Ala Arg Phe Ser Gly Ser Gly Ser Gly
   210 215 220
   Thr Asp Tyr Thr Leu Thr Ile Ser Ser Leu Gln Pro Glu Asp Phe Ala
   225 230 235 240
   Val Tyr Phe Cys Gln Gln Gly Asn Thr Leu Pro Tyr Thr Phe Gly Gln
   245 250 255
   Gly Thr Lys Leu Glu Ile Lys His His His His His His His His
   260 265 270
<210> 76
   <211> 271
   <212> PRT
   <213> Artificial Sequence
<220>
   <221> source
   <223> /note="Description of Artificial Sequence: Synthetic polypeptide"
<400> 76
   Met Ala Leu Pro Val Thr Ala Leu Leu Leu Pro Leu Ala Leu Leu Leu
   1 5 10 15
   His Ala Ala Arg Pro Gln Val Gln Leu Gln Glu Ser Gly Pro Gly Leu
   20 25 30
   Val Lys Pro Ser Glu Thr Leu Ser Leu Thr Cys Thr Val Ser Gly Val
   35 40 45
   Ser Leu Pro Asp Tyr Gly Val Ser Trp Ile Arg Gln Pro Pro Gly Lys
   50 55 60
   Gly Leu Glu Trp Ile Gly Val Ile Trp Gly Ser Glu Thr Thr Tyr Tyr
   65 70 75 80
   Gln Ser Ser Leu Lys Ser Arg Val Thr Ile Ser Lys Asp Asn Ser Lys
                   85                  90                  95
   Asn Gln Val Ser Leu Lys Leu Ser Ser Val Thr Ala Ala Asp Thr Ala
               100                 105                 110
   Val Tyr Tyr Cys Ala Lys His Tyr Tyr Tyr Gly Gly Ser Tyr Ala Met
           115                 120                 125
   Asp Tyr Trp Gly Gln Gly Thr Leu Val Thr Val Ser Ser Gly Gly Gly
       130                 135                 140
   Gly Ser Gly Gly Gly Gly Ser Gly Gly Gly Gly Ser Glu Ile Val Met
   145                 150                 155                 160
   Thr Gln Ser Pro Ala Thr Leu Ser Leu Ser Pro Gly Glu Arg Ala Thr
                   165                 170                 175
   Leu Ser Cys Arg Ala Ser Gln Asp Ile Ser Lys Tyr Leu Asn Trp Tyr
               180                 185                 190
   Gln Gln Lys Pro Gly Gln Ala Pro Arg Leu Leu Ile Tyr His Thr Ser
           195                 200                 205
   Arg Leu His Ser Gly Ile Pro Ala Arg Phe Ser Gly Ser Gly Ser Gly
       210                 215                 220
   Thr Asp Tyr Thr Leu Thr Ile Ser Ser Leu Gln Pro Glu Asp Phe Ala
   225                 230                 235                 240
   Val Tyr Phe Cys Gln Gln Gly Asn Thr Leu Pro Tyr Thr Phe Gly Gln
                   245                 250                 255
   Gly Thr Lys Leu Glu Ile Lys His His His His His His His His
               260                 265                 270
<210> 77
   <211> 276
   <212> PRT
   <213> Artificial Sequence
<220>
   <221> source
   <223> /note="Description of Artificial Sequence: Synthetic polypeptide"
<400> 77
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gly
       130                 135                 140
   Gly Gly Gly Ser Gln Val Gln Leu Gln Glu Ser Gly Pro Gly Leu Val
   145                 150                 155                 160
   Lys Pro Ser Glu Thr Leu Ser Leu Thr Cys Thr Val Ser Gly Val Ser
                   165                 170                 175
   Leu Pro Asp Tyr Gly Val Ser Trp Ile Arg Gln Pro Pro Gly Lys Gly
               180                 185                 190
   Leu Glu Trp Ile Gly Val Ile Trp Gly Ser Glu Thr Thr Tyr Tyr Ser
           195                 200                 205
   Ser Ser Leu Lys Ser Arg Val Thr Ile Ser Lys Asp Asn Ser Lys Asn
       210                 215                 220
   Gln Val Ser Leu Lys Leu Ser Ser Val Thr Ala Ala Asp Thr Ala Val
   225                 230                 235                 240
   Tyr Tyr Cys Ala Lys His Tyr Tyr Tyr Gly Gly Ser Tyr Ala Met Asp
                   245                 250                 255
   Tyr Trp Gly Gln Gly Thr Leu Val Thr Val Ser Ser His His His His
               260                 265                 270
   His His His His
   275
<210> 78
   <211> 276
   <212> PRT
   <213> Artificial Sequence
<220>
   <221> source
   <223> /note="Description of Artificial Sequence: Synthetic polypeptide"
<400> 78
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gly
       130                 135                 140
   Gly Gly Gly Ser Gln Val Gln Leu Gln Glu Ser Gly Pro Gly Leu Val
   145                 150                 155                 160
   Lys Pro Ser Glu Thr Leu Ser Leu Thr Cys Thr Val Ser Gly Val Ser
                   165                 170                 175
   Leu Pro Asp Tyr Gly Val Ser Trp Ile Arg Gln Pro Pro Gly Lys Gly
               180                 185                 190
   Leu Glu Trp Ile Gly Val Ile Trp Gly Ser Glu Thr Thr Tyr Tyr Gln
           195                 200                 205
   Ser Ser Leu Lys Ser Arg Val Thr Ile Ser Lys Asp Asn Ser Lys Asn
       210                 215                 220
   Gln Val Ser Leu Lys Leu Ser Ser Val Thr Ala Ala Asp Thr Ala Val
   225                 230                 235                 240
   Tyr Tyr Cys Ala Lys His Tyr Tyr Tyr Gly Gly Ser Tyr Ala Met Asp
                   245                 250                 255
   Tyr Trp Gly Gln Gly Thr Leu Val Thr Val Ser Ser His His His His
               260                 265                 270
   His His His His
           275
<210> 79
   <211> 276
   <212> PRT
   <213> Artificial Sequence
<220>
   <221> source
   <223> /note="Description of Artificial Sequence: Synthetic polypeptide"
<400> 79
   Met Ala Leu Pro Val Thr Ala Leu Leu Leu Pro Leu Ala Leu Leu Leu
   1               5                   10                  15
   His Ala Ala Arg Pro Gln Val Gln Leu Gln Glu Ser Gly Pro Gly Leu
               20                  25                  30
   Val Lys Pro Ser Glu Thr Leu Ser Leu Thr Cys Thr Val Ser Gly Val
           35                  40                  45
   Ser Leu Pro Asp Tyr Gly Val Ser Trp Ile Arg Gln Pro Pro Gly Lys
       50                  55                  60
   Gly Leu Glu Trp Ile Gly Val Ile Trp Gly Ser Glu Thr Thr Tyr Tyr
   65                  70                  75                  80
   Ser Ser Ser Leu Lys Ser Arg Val Thr Ile Ser Lys Asp Asn Ser Lys
                   85                  90                  95
   Asn Gln Val Ser Leu Lys Leu Ser Ser Val Thr Ala Ala Asp Thr Ala
               100                 105                 110
   Val Tyr Tyr Cys Ala Lys His Tyr Tyr Tyr Gly Gly Ser Tyr Ala Met
   115 120 125
   Asp Tyr Trp Gly Gln Gly Thr Leu Val Thr Val Ser Ser Gly Gly Gly
   130 135 140
   Gly Ser Gly Gly Gly Gly Ser Gly Gly Gly Gly Ser Gly Gly Gly Gly
   145 150 155 160
   Ser Glu Ile Val Met Thr Gln Ser Pro Ala Thr Leu Ser Leu Ser Pro
   165 170 175
   Gly Glu Arg Ala Thr Leu Ser Cys Arg Ala Ser Gln Asp Ile Ser Lys
   180 185 190
   Tyr Leu Asn Trp Tyr Gln Gln Lys Pro Gly Gln Ala Pro Arg Leu Leu
   195 200 205
   Ile Tyr His Thr Ser Arg Leu His Ser Gly Ile Pro Ala Arg Phe Ser
   210 215 220
   Gly Ser Gly Ser Gly Thr Asp Tyr Thr Leu Thr Ile Ser Ser Leu Gln
   225 230 235 240
   Pro Glu Asp Phe Ala Val Tyr Phe Cys Gln Gln Gly Asn Thr Leu Pro
   245 250 255
   Tyr Thr Phe Gly Gln Gly Thr Lys Leu Glu Ile Lys His His His His
   260 265 270
   His His His His
   275
<210> 80
   <211> 276
   <212> PRT
   <213> Artificial Sequence
<220>
   <221> source
   <223> /note="Description of Artificial Sequence: Synthetic polypeptide"
<400> 80
   Met Ala Leu Pro Val Thr Ala Leu Leu Leu Pro Leu Ala Leu Leu Leu
   1 5 10 15
   His Ala Ala Arg Pro Gln Val Gln Leu Gln Glu Ser Gly Pro Gly Leu
   20 25 30
   Val Lys Pro Ser Glu Thr Leu Ser Leu Thr Cys Thr Val Ser Gly Val
   35 40 45
   Ser Leu Pro Asp Tyr Gly Val Ser Trp Ile Arg Gln Pro Pro Gly Lys
   50 55 60
   Gly Leu Glu Trp Ile Gly Val Ile Trp Gly Ser Glu Thr Thr Tyr Tyr
   65 70 75 80
   Gln Ser Ser Leu Lys Ser Arg Val Thr Ile Ser Lys Asp Asn Ser Lys
   85 90 95
   Asn Gln Val Ser Leu Lys Leu Ser Ser Val Thr Ala Ala Asp Thr Ala
   100 105 110
   Val Tyr Tyr Cys Ala Lys His Tyr Tyr Tyr Gly Gly Ser Tyr Ala Met
   115 120 125
   Asp Tyr Trp Gly Gln Gly Thr Leu Val Thr Val Ser Ser Gly Gly Gly
   130 135 140
   Gly Ser Gly Gly Gly Gly Ser Gly Gly Gly Gly Ser Gly Gly Gly Gly
   145 150 155 160
   Ser Glu Ile Val Met Thr Gln Ser Pro Ala Thr Leu Ser Leu Ser Pro
   165 170 175
   Gly Glu Arg Ala Thr Leu Ser Cys Arg Ala Ser Gln Asp Ile Ser Lys
   180 185 190
   Tyr Leu Asn Trp Tyr Gln Gln Lys Pro Gly Gln Ala Pro Arg Leu Leu
   195 200 205
   Ile Tyr His Thr Ser Arg Leu His Ser Gly Ile Pro Ala Arg Phe Ser
   210 215 220
   Gly Ser Gly Ser Gly Thr Asp Tyr Thr Leu Thr Ile Ser Ser Leu Gln
   225 230 235 240
   Pro Glu Asp Phe Ala Val Tyr Phe Cys Gln Gln Gly Asn Thr Leu Pro
   245 250 255
   Tyr Thr Phe Gly Gln Gly Thr Lys Leu Glu Ile Lys His His His His
   260 265 270
   His His His His
   275
<210> 81
   <211> 276
   <212> PRT
   <213> Artificial Sequence
<220>
   <221> source
   <223> /note="Description of Artificial Sequence: Synthetic polypeptide"
<400> 81
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gly
       130                 135                 140
   Gly Gly Gly Ser Gln Val Gln Leu Gln Glu Ser Gly Pro Gly Leu Val
   145                 150                 155                 160
   Lys Pro Ser Glu Thr Leu Ser Leu Thr Cys Thr Val Ser Gly Val Ser
                   165                 170                 175
   Leu Pro Asp Tyr Gly Val Ser Trp Ile Arg Gln Pro Pro Gly Lys Gly
               180                 185                 190
   Leu Glu Trp Ile Gly Val Ile Trp Gly Ser Glu Thr Thr Tyr Tyr Asn
   195 200 205
   Ser Ser Leu Lys Ser Arg Val Thr Ile Ser Lys Asp Asn Ser Lys Asn
   210 215 220
   Gln Val Ser Leu Lys Leu Ser Ser Val Thr Ala Ala Asp Thr Ala Val
   225 230 235 240
   Tyr Tyr Cys Ala Lys His Tyr Tyr Tyr Gly Gly Ser Tyr Ala Met Asp
   245 250 255
   Tyr Trp Gly Gln Gly Thr Leu Val Thr Val Ser Ser His His His His
   260 265 270
   His His His His
   275
<210> 82
   <211> 276
   <212> PRT
   <213> Artificial Sequence
<220>
   <221> source
   <223> /note="Description of Artificial Sequence: Synthetic polypeptide"
<400> 82
   Met Ala Leu Pro Val Thr Ala Leu Leu Leu Pro Leu Ala Leu Leu Leu
   1               5                   10                  15
   His Ala Ala Arg Pro Gln Val Gln Leu Gln Glu Ser Gly Pro Gly Leu
               20                  25                  30
   Val Lys Pro Ser Glu Thr Leu Ser Leu Thr Cys Thr Val Ser Gly Val
           35                  40                  45
   Ser Leu Pro Asp Tyr Gly Val Ser Trp Ile Arg Gln Pro Pro Gly Lys
       50                  55                  60
   Gly Leu Glu Trp Ile Gly Val Ile Trp Gly Ser Glu Thr Thr Tyr Tyr
   65                  70                  75                  80
   Asn Ser Ser Leu Lys Ser Arg Val Thr Ile Ser Lys Asp Asn Ser Lys
                   85                  90                  95
   Asn Gln Val Ser Leu Lys Leu Ser Ser Val Thr Ala Ala Asp Thr Ala
               100                 105                 110
   Val Tyr Tyr Cys Ala Lys His Tyr Tyr Tyr Gly Gly Ser Tyr Ala Met
   115 120 125
   Asp Tyr Trp Gly Gln Gly Thr Leu Val Thr Val Ser Ser Gly Gly Gly
   130 135 140
   Gly Ser Gly Gly Gly Gly Ser Gly Gly Gly Gly Ser Gly Gly Gly Gly
   145 150 155 160
   Ser Glu Ile Val Met Thr Gln Ser Pro Ala Thr Leu Ser Leu Ser Pro
   165 170 175
   Gly Glu Arg Ala Thr Leu Ser Cys Arg Ala Ser Gln Asp Ile Ser Lys
   180 185 190
   Tyr Leu Asn Trp Tyr Gln Gln Lys Pro Gly Gln Ala Pro Arg Leu Leu
   195 200 205
   Ile Tyr His Thr Ser Arg Leu His Ser Gly Ile Pro Ala Arg Phe Ser
   210 215 220
   Gly Ser Gly Ser Gly Thr Asp Tyr Thr Leu Thr Ile Ser Ser Leu Gln
   225 230 235 240
   Pro Glu Asp Phe Ala Val Tyr Phe Cys Gln Gln Gly Asn Thr Leu Pro
   245 250 255
   Tyr Thr Phe Gly Gln Gly Thr Lys Leu Glu Ile Lys His His His His
   260 265 270
   His His His His
   275
<210> 83
   <211> 271
   <212> PRT
   <213> Artificial Sequence
<220>
   <221> source
   <223> /note="Description of Artificial Sequence: Synthetic polypeptide"
<400> 83
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
               35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Asn Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Leu Val Thr Val Ser Ser His His His His His His His His
               260                 265                 270
<210> 84
   <211> 271
   <212> PRT
   <213> Artificial Sequence
<220>
   <221> source
   <223> /note="Description of Artificial Sequence: Synthetic polypeptide"
<400> 84
   Met Ala Leu Pro Val Thr Ala Leu Leu Leu Pro Leu Ala Leu Leu Leu
   1 5 10 15
   His Ala Ala Arg Pro Gln Val Gln Leu Gln Glu Ser Gly Pro Gly Leu
   20 25 30
   Val Lys Pro Ser Glu Thr Leu Ser Leu Thr Cys Thr Val Ser Gly Val
   35 40 45
   Ser Leu Pro Asp Tyr Gly Val Ser Trp Ile Arg Gln Pro Pro Gly Lys
   50 55 60
   Gly Leu Glu Trp Ile Gly Val Ile Trp Gly Ser Glu Thr Thr Tyr Tyr
   65 70 75 80
   Asn Ser Ser Leu Lys Ser Arg Val Thr Ile Ser Lys Asp Asn Ser Lys
   85 90 95
   Asn Gln Val Ser Leu Lys Leu Ser Ser Val Thr Ala Ala Asp Thr Ala
   100 105 110
   Val Tyr Tyr Cys Ala Lys His Tyr Tyr Tyr Gly Gly Ser Tyr Ala Met
   115 120 125
   Asp Tyr Trp Gly Gln Gly Thr Leu Val Thr Val Ser Ser Gly Gly Gly
   130 135 140
   Gly Ser Gly Gly Gly Gly Ser Gly Gly Gly Gly Ser Glu Ile Val Met
   145 150 155 160
   Thr Gln Ser Pro Ala Thr Leu Ser Leu Ser Pro Gly Glu Arg Ala Thr
   165 170 175
   Leu Ser Cys Arg Ala Ser Gln Asp Ile Ser Lys Tyr Leu Asn Trp Tyr
   180 185 190
   Gln Gln Lys Pro Gly Gln Ala Pro Arg Leu Leu Ile Tyr His Thr Ser
   195 200 205
   Arg Leu His Ser Gly Ile Pro Ala Arg Phe Ser Gly Ser Gly Ser Gly
   210 215 220
   Thr Asp Tyr Thr Leu Thr Ile Ser Ser Leu Gln Pro Glu Asp Phe Ala
   225                 230                 235                 240
   Val Tyr Phe Cys Gln Gln Gly Asn Thr Leu Pro Tyr Thr Phe Gly Gln
                   245                 250                 255
   Gly Thr Lys Leu Glu Ile Lys His His His His His His His His
               260                 265                 270
<210> 85
   <211> 1458
   <212> DNA
   <213> Artificial Sequence
<220>
   <221> source
   <223> /note="Description of Artificial Sequence: Synthetic polynucleotide"
<400> 85
   atggccctcc ctgtcaccgc cctgctgctt ccgctggctc ttctgctcca cgccgctcgg      60
   cccgaaattg tgatgaccca gtcacccgcc actcttagcc tttcacccgg tgagcgcgca     120
   accctgtctt gcagagcctc ccaagacatc tcaaaatacc ttaattggta tcaacagaag     180
   cccggacagg ctcctcgcct tctgatctac cacaccagcc ggctccattc tggaatccct     240
   gccaggttca gcggtagcgg atctgggacc gactacaccc tcactatcag ctcactgcag     300
   ccagaggact tcgctgtcta tttctgtcag caagggaaca ccctgcccta cacctttgga     360
   cagggcacca agctcgagat taaaggtgga ggtggcagcg gaggaggtgg gtccggcggt     420
   ggaggaagcc aggtccaact ccaagaaagc ggaccgggtc ttgtgaagcc atcagaaact     480
   ctttcactga cttgtactgt gagcggagtg tctctccccg attacggggt gtcttggatc     540
   agacagccac cggggaaggg tctggaatgg attggagtga tttggggctc tgagactact     600
   tactactctt catccctcaa gtcacgcgtc accatctcaa aggacaactc taagaatcag     660
   gtgtcactga aactgtcatc tgtgaccgca gccgacaccg ccgtgtacta ttgcgctaag     720
   cattactatt atggcgggag ctacgcaatg gattactggg gacagggtac tctggtcacc     780
   gtgtccagca ccactacccc agcaccgagg ccacccaccc cggctcctac catcgcctcc     840
   cagcctctgt ccctgcgtcc ggaggcatgt agacccgcag ctggtggggc cgtgcatacc     900
   cggggtcttg acttcgcctg cgatatctac atttgggccc ctctggctgg tacttgcggg     960
   gtcctgctgc tttcactcgt gatcactctt tactgtaagc gcggtcggaa gaagctgctg    1020
   tacatcttta agcaaccctt catgaggcct gtgcagacta ctcaagagga ggacggctgt    1080
   tcatgccggt tcccagagga ggaggaaggc ggctgcgaac tgcgcgtgaa attcagccgc    1140
   agcgcagatg ctccagccta caagcagggg cagaaccagc tctacaacga actcaatctt    1200
   ggtcggagag aggagtacga cgtgctggac aagcggagag gacgggaccc agaaatgggc    1260
   gggaagccgc gcagaaagaa tccccaagag ggcctgtaca acgagctcca aaaggataag    1320
   atggcagaag cctatagcga gattggtatg aaaggggaac gcagaagagg caaaggccac    1380
   gacggactgt accagggact cagcaccgcc accaaggaca cctatgacgc tcttcacatg    1440
   caggccctgc cgcctcgg                                                   1458
<210> 86
   <211> 1458
   <212> DNA
   <213> Artificial Sequence
<220>
   <221> source
   <223> /note="Description of Artificial Sequence: Synthetic polynucleotide"
<400> 86
   atggccctcc ctgtcaccgc cctgctgctt ccgctggctc ttctgctcca cgccgctcgg 60
   cccgaaattg tgatgaccca gtcacccgcc actcttagcc tttcacccgg tgagcgcgca 120
   accctgtctt gcagagcctc ccaagacatc tcaaaatacc ttaattggta tcaacagaag 180
   cccggacagg ctcctcgcct tctgatctac cacaccagcc ggctccattc tggaatccct 240
   gccaggttca gcggtagcgg atctgggacc gactacaccc tcactatcag ctcactgcag 300
   ccagaggact tcgctgtcta tttctgtcag caagggaaca ccctgcccta cacctttgga 360
   cagggcacca agctcgagat taaaggtgga ggtggcagcg gaggaggtgg gtccggcggt 420
   ggaggaagcc aggtccaact ccaagaaagc ggaccgggtc ttgtgaagcc atcagaaact 480
   ctttcactga cttgtactgt gagcggagtg tctctccccg attacggggt gtcttggatc 540
   agacagccac cggggaaggg tctggaatgg attggagtga tttggggctc tgagactact 600
   tactaccaat catccctcaa gtcacgcgtc accatctcaa aggacaactc taagaatcag 660
   gtgtcactga aactgtcatc tgtgaccgca gccgacaccg ccgtgtacta ttgcgctaag 720
   cattactatt atggcgggag ctacgcaatg gattactggg gacagggtac tctggtcacc 780
   gtgtccagca ccactacccc agcaccgagg ccacccaccc cggctcctac catcgcctcc 840
   cagcctctgt ccctgcgtcc ggaggcatgt agacccgcag ctggtggggc cgtgcatacc 900
   cggggtcttg acttcgcctg cgatatctac atttgggccc ctctggctgg tacttgcggg 960
   gtcctgctgc tttcactcgt gatcactctt tactgtaagc gcggtcggaa gaagctgctg 1020
   tacatcttta agcaaccctt catgaggcct gtgcagacta ctcaagagga ggacggctgt 1080
   tcatgccggt tcccagagga ggaggaaggc ggctgcgaac tgcgcgtgaa attcagccgc 1140
   agcgcagatg ctccagccta caagcagggg cagaaccagc tctacaacga actcaatctt 1200
   ggtcggagag aggagtacga cgtgctggac aagcggagag gacgggaccc agaaatgggc 1260
   gggaagccgc gcagaaagaa tccccaagag ggcctgtaca acgagctcca aaaggataag 1320
   atggcagaag cctatagcga gattggtatg aaaggggaac gcagaagagg caaaggccac 1380
   gacggactgt accagggact cagcaccgcc accaaggaca cctatgacgc tcttcacatg 1440
   caggccctgc cgcctcgg 1458
<210> 87
   <211> 1458
   <212> DNA
   <213> Artificial Sequence
<220>
   <221> source
   <223> /note="Description of Artificial Sequence: Synthetic polynucleotide"
<400> 87
   atggctctgc ccgtgaccgc actcctcctg ccactggctc tgctgcttca cgccgctcgc      60
   ccacaagtcc agcttcaaga atcagggcct ggtctggtga agccatctga gactctgtcc     120
   ctcacttgca ccgtgagcgg agtgtccctc ccagactacg gagtgagctg gattagacag     180
   cctcccggaa agggactgga gtggatcgga gtgatttggg gtagcgaaac cacttactat     240
   tcatcttccc tgaagtcacg ggtcaccatt tcaaaggata actcaaagaa tcaagtgagc     300
   ctcaagctct catcagtcac cgccgctgac accgccgtgt attactgtgc caagcattac     360
   tactatggag ggtcctacgc catggactac tggggccagg gaactctggt cactgtgtca     420
   tctggtggag gaggtagcgg aggaggcggg agcggtggag gtggctccga aatcgtgatg     480
   acccagagcc ctgcaaccct gtccctttct cccggggaac gggctaccct ttcttgtcgg     540
   gcatcacaag atatctcaaa atacctcaat tggtatcaac agaagccggg acaggcccct     600
   aggcttctta tctaccacac ctctcgcctg catagcggga ttcccgcacg ctttagcggg     660
   tctggaagcg ggaccgacta cactctgacc atctcatctc tccagcccga ggacttcgcc     720
   gtctacttct gccagcaggg taacaccctg ccgtacacct tcggccaggg caccaagctt     780
   gagatcaaaa ccactactcc cgctccaagg ccacccaccc ctgccccgac catcgcctct     840
   cagccgcttt ccctgcgtcc ggaggcatgt agacccgcag ctggtggggc cgtgcatacc     900
   cggggtcttg acttcgcctg cgatatctac atttgggccc ctctggctgg tacttgcggg     960
   gtcctgctgc tttcactcgt gatcactctt tactgtaagc gcggtcggaa gaagctgctg    1020
   tacatcttta agcaaccctt catgaggcct gtgcagacta ctcaagagga ggacggctgt    1080
   tcatgccggt tcccagagga ggaggaaggc ggctgcgaac tgcgcgtgaa attcagccgc    1140
   agcgcagatg ctccagccta caagcagggg cagaaccagc tctacaacga actcaatctt    1200
   ggtcggagag aggagtacga cgtgctggac aagcggagag gacgggaccc agaaatgggc    1260
   gggaagccgc gcagaaagaa tccccaagag ggcctgtaca acgagctcca aaaggataag    1320
   atggcagaag cctatagcga gattggtatg aaaggggaac gcagaagagg caaaggccac    1380
   gacggactgt accagggact cagcaccgcc accaaggaca cctatgacgc tcttcacatg    1440
   caggccctgc cgcctcgg                                                   1458
<210> 88
   <211> 1458
   <212> DNA
   <213> Artificial Sequence
<220>
   <221> source
   <223> /note="Description of Artificial Sequence: Synthetic polynucleotide"
<400> 88
   atggctctgc ccgtgaccgc actcctcctg ccactggctc tgctgcttca cgccgctcgc      60
   ccacaagtcc agcttcaaga atcagggcct ggtctggtga agccatctga gactctgtcc     120
   ctcacttgca ccgtgagcgg agtgtccctc ccagactacg gagtgagctg gattagacag     180
   cctcccggaa agggactgga gtggatcgga gtgatttggg gtagcgaaac cacttactat     240
   caatcttccc tgaagtcacg ggtcaccatt tcaaaggata actcaaagaa tcaagtgagc     300
   ctcaagctct catcagtcac cgccgctgac accgccgtgt attactgtgc caagcattac     360
   tactatggag ggtcctacgc catggactac tggggccagg gaactctggt cactgtgtca     420
   tctggtggag gaggtagcgg aggaggcggg agcggtggag gtggctccga aatcgtgatg     480
   acccagagcc ctgcaaccct gtccctttct cccggggaac gggctaccct ttcttgtcgg     540
   gcatcacaag atatctcaaa atacctcaat tggtatcaac agaagccggg acaggcccct     600
   aggcttctta tctaccacac ctctcgcctg catagcggga ttcccgcacg ctttagcggg     660
   tctggaagcg ggaccgacta cactctgacc atctcatctc tccagcccga ggacttcgcc     720
   gtctacttct gccagcaggg taacaccctg ccgtacacct tcggccaggg caccaagctt     780
   gagatcaaaa ccactactcc cgctccaagg ccacccaccc ctgccccgac catcgcctct     840
   cagccgcttt ccctgcgtcc ggaggcatgt agacccgcag ctggtggggc cgtgcatacc     900
   cggggtcttg acttcgcctg cgatatctac atttgggccc ctctggctgg tacttgcggg     960
   gtcctgctgc tttcactcgt gatcactctt tactgtaagc gcggtcggaa gaagctgctg    1020
   tacatcttta agcaaccctt catgaggcct gtgcagacta ctcaagagga ggacggctgt    1080
   tcatgccggt tcccagagga ggaggaaggc ggctgcgaac tgcgcgtgaa attcagccgc    1140
   agcgcagatg ctccagccta caagcagggg cagaaccagc tctacaacga actcaatctt    1200
   ggtcggagag aggagtacga cgtgctggac aagcggagag gacgggaccc agaaatgggc    1260
   gggaagccgc gcagaaagaa tccccaagag ggcctgtaca acgagctcca aaaggataag    1320
   atggcagaag cctatagcga gattggtatg aaaggggaac gcagaagagg caaaggccac    1380
   gacggactgt accagggact cagcaccgcc accaaggaca cctatgacgc tcttcacatg    1440
   caggccctgc cgcctcgg                                                  1458
<210> 89
   <211> 1473
   <212> DNA
   <213> Artificial Sequence
<220>
   <221> source
   <223> /note="Description of Artificial Sequence: Synthetic polynucleotide"
<400> 89
   atggccctcc ctgtcaccgc cctgctgctt ccgctggctc ttctgctcca cgccgctcgg 60
   cccgaaattg tgatgaccca gtcacccgcc actcttagcc tttcacccgg tgagcgcgca 120
   accctgtctt gcagagcctc ccaagacatc tcaaaatacc ttaattggta tcaacagaag 180
   cccggacagg ctcctcgcct tctgatctac cacaccagcc ggctccattc tggaatccct 240
   gccaggttca gcggtagcgg atctgggacc gactacaccc tcactatcag ctcactgcag 300
   ccagaggact tcgctgtcta tttctgtcag caagggaaca ccctgcccta cacctttgga 360
   cagggcacca agctcgagat taaaggtgga ggtggcagcg gaggaggtgg gtccggcggt 420
   ggaggaagcg gcggaggcgg gagccaggtc caactccaag aaagcggacc gggtcttgtg 480
   aagccatcag aaactctttc actgacttgt actgtgagcg gagtgtctct ccccgattac 540
   ggggtgtctt ggatcagaca gccaccgggg aagggtctgg aatggattgg agtgatttgg 600
   ggctctgaga ctacttacta ctcttcatcc ctcaagtcac gcgtcaccat ctcaaaggac 660
   aactctaaga atcaggtgtc actgaaactg tcatctgtga ccgcagccga caccgccgtg 720
   tactattgcg ctaagcatta ctattatggc gggagctacg caatggatta ctggggacag 780
   ggtactctgg tcaccgtgtc cagcaccact accccagcac cgaggccacc caccccggct 840
   cctaccatcg cctcccagcc tctgtccctg cgtccggagg catgtagacc cgcagctggt 900
   ggggccgtgc atacccgggg tcttgacttc gcctgcgata tctacatttg ggcccctctg 960
   gctggtactt gcggggtcct gctgctttca ctcgtgatca ctctttactg taagcgcggt 1020
   cggaagaagc tgctgtacat ctttaagcaa cccttcatga ggcctgtgca gactactcaa 1080
   gaggaggacg gctgttcatg ccggttccca gaggaggagg aaggcggctg cgaactgcgc 1140
   gtgaaattca gccgcagcgc agatgctcca gcctacaagc aggggcagaa ccagctctac 1200
   aacgaactca atcttggtcg gagagaggag tacgacgtgc tggacaagcg gagaggacgg 1260
   gacccagaaa tgggcgggaa gccgcgcaga aagaatcccc aagagggcct gtacaacgag 1320
   ctccaaaagg ataagatggc agaagcctat agcgagattg gtatgaaagg ggaacgcaga 1380
   agaggcaaag gccacgacgg actgtaccag ggactcagca ccgccaccaa ggacacctat 1440
   gacgctcttc acatgcaggc cctgccgcct cgg 1473
<210> 90
   <211> 1473
   <212> DNA
   <213> Artificial Sequence
<220>
   <221> source
   <223> /note="Description of Artificial Sequence: Synthetic polynucleotide"
<400> 90
   atggccctcc ctgtcaccgc cctgctgctt ccgctggctc ttctgctcca cgccgctcgg 60
   cccgaaattg tgatgaccca gtcacccgcc actcttagcc tttcacccgg tgagcgcgca 120
   accctgtctt gcagagcctc ccaagacatc tcaaaatacc ttaattggta tcaacagaag 180
   cccggacagg ctcctcgcct tctgatctac cacaccagcc ggctccattc tggaatccct 240
   gccaggttca gcggtagcgg atctgggacc gactacaccc tcactatcag ctcactgcag 300
   ccagaggact tcgctgtcta tttctgtcag caagggaaca ccctgcccta cacctttgga 360
   cagggcacca agctcgagat taaaggtgga ggtggcagcg gaggaggtgg gtccggcggt 420
   ggaggaagcg gaggcggagg gagccaggtc caactccaag aaagcggacc gggtcttgtg 480
   aagccatcag aaactctttc actgacttgt actgtgagcg gagtgtctct ccccgattac 540
   ggggtgtctt ggatcagaca gccaccgggg aagggtctgg aatggattgg agtgatttgg 600
   ggctctgaga ctacttacta ccaatcatcc ctcaagtcac gcgtcaccat ctcaaaggac 660
   aactctaaga atcaggtgtc actgaaactg tcatctgtga ccgcagccga caccgccgtg 720
   tactattgcg ctaagcatta ctattatggc gggagctacg caatggatta ctggggacag 780
   ggtactctgg tcaccgtgtc cagcaccact accccagcac cgaggccacc caccccggct 840
   cctaccatcg cctcccagcc tctgtccctg cgtccggagg catgtagacc cgcagctggt 900
   ggggccgtgc atacccgggg tcttgacttc gcctgcgata tctacatttg ggcccctctg 960
   gctggtactt gcggggtcct gctgctttca ctcgtgatca ctctttactg taagcgcggt 1020
   cggaagaagc tgctgtacat ctttaagcaa cccttcatga ggcctgtgca gactactcaa 1080
   gaggaggacg gctgttcatg ccggttccca gaggaggagg aaggcggctg cgaactgcgc 1140
   gtgaaattca gccgcagcgc agatgctcca gcctacaagc aggggcagaa ccagctctac 1200
   aacgaactca atcttggtcg gagagaggag tacgacgtgc tggacaagcg gagaggacgg 1260
   gacccagaaa tgggcgggaa gccgcgcaga aagaatcccc aagagggcct gtacaacgag 1320
   ctccaaaagg ataagatggc agaagcctat agcgagattg gtatgaaagg ggaacgcaga 1380
   agaggcaaag gccacgacgg actgtaccag ggactcagca ccgccaccaa ggacacctat 1440
   gacgctcttc acatgcaggc cctgccgcct cgg 1473
<210> 91
   <211> 1473
   <212> DNA
   <213> Artificial Sequence
<220>
   <221> source
   <223> /note="Description of Artificial Sequence: Synthetic polynucleotide"
<400> 91
   atggctctgc ccgtgaccgc actcctcctg ccactggctc tgctgcttca cgccgctcgc      60
   ccacaagtcc agcttcaaga atcagggcct ggtctggtga agccatctga gactctgtcc     120
   ctcacttgca ccgtgagcgg agtgtccctc ccagactacg gagtgagctg gattagacag     180
   cctcccggaa agggactgga gtggatcgga gtgatttggg gtagcgaaac cacttactat     240
   tcatcttccc tgaagtcacg ggtcaccatt tcaaaggata actcaaagaa tcaagtgagc     300
   ctcaagctct catcagtcac cgccgctgac accgccgtgt attactgtgc caagcattac     360
   tactatggag ggtcctacgc catggactac tggggccagg gaactctggt cactgtgtca     420
   tctggtggag gaggtagcgg aggaggcggg agcggtggag gtggctccgg aggtggcgga     480
   agcgaaatcg tgatgaccca gagccctgca accctgtccc tttctcccgg ggaacgggct     540
   accctttctt gtcgggcatc acaagatatc tcaaaatacc tcaattggta tcaacagaag     600
   ccgggacagg cccctaggct tcttatctac cacacctctc gcctgcatag cgggattccc     660
   gcacgcttta gcgggtctgg aagcgggacc gactacactc tgaccatctc atctctccag     720
   cccgaggact tcgccgtcta cttctgccag cagggtaaca ccctgccgta caccttcggc     780
   cagggcacca agcttgagat caaaaccact actcccgctc caaggccacc cacccctgcc     840
   ccgaccatcg cctctcagcc gctttccctg cgtccggagg catgtagacc cgcagctggt     900
   ggggccgtgc atacccgggg tcttgacttc gcctgcgata tctacatttg ggcccctctg     960
   gctggtactt gcggggtcct gctgctttca ctcgtgatca ctctttactg taagcgcggt    1020
   cggaagaagc tgctgtacat ctttaagcaa cccttcatga ggcctgtgca gactactcaa    1080
   gaggaggacg gctgttcatg ccggttccca gaggaggagg aaggcggctg cgaactgcgc    1140
   gtgaaattca gccgcagcgc agatgctcca gcctacaagc aggggcagaa ccagctctac    1200
   aacgaactca atcttggtcg gagagaggag tacgacgtgc tggacaagcg gagaggacgg    1260
   gacccagaaa tgggcgggaa gccgcgcaga aagaatcccc aagagggcct gtacaacgag    1320
   ctccaaaagg ataagatggc agaagcctat agcgagattg gtatgaaagg ggaacgcaga    1380
   agaggcaaag gccacgacgg actgtaccag ggactcagca ccgccaccaa ggacacctat    1440
   gacgctcttc acatgcaggc cctgccgcct cgg                                 1473
<210> 92
   <211> 1473
   <212> DNA
   <213> Artificial Sequence
<220>
   <221> source
   <223> /note="Description of Artificial Sequence: Synthetic polynucleotide"
<400> 92
   atggctctgc ccgtgaccgc actcctcctg ccactggctc tgctgcttca cgccgctcgc      60
   ccacaagtcc agcttcaaga atcagggcct ggtctggtga agccatctga gactctgtcc     120
   ctcacttgca ccgtgagcgg agtgtccctc ccagactacg gagtgagctg gattagacag     180
   cctcccggaa agggactgga gtggatcgga gtgatttggg gtagcgaaac cacttactat     240
   caatcttccc tgaagtcacg ggtcaccatt tcaaaggata actcaaagaa tcaagtgagc     300
   ctcaagctct catcagtcac cgccgctgac accgccgtgt attactgtgc caagcattac     360
   tactatggag ggtcctacgc catggactac tggggccagg gaactctggt cactgtgtca     420
   tctggtggag gaggtagcgg aggaggcggg agcggtggag gtggctccgg aggcggtggg     480
   tcagaaatcg tgatgaccca gagccctgca accctgtccc tttctcccgg ggaacgggct     540
   accctttctt gtcgggcatc acaagatatc tcaaaatacc tcaattggta tcaacagaag     600
   ccgggacagg cccctaggct tcttatctac cacacctctc gcctgcatag cgggattccc     660
   gcacgcttta gcgggtctgg aagcgggacc gactacactc tgaccatctc atctctccag     720
   cccgaggact tcgccgtcta cttctgccag cagggtaaca ccctgccgta caccttcggc     780
   cagggcacca agcttgagat caaaaccact actcccgctc caaggccacc cacccctgcc     840
   ccgaccatcg cctctcagcc gctttccctg cgtccggagg catgtagacc cgcagctggt     900
   ggggccgtgc atacccgggg tcttgacttc gcctgcgata tctacatttg ggcccctctg     960
   gctggtactt gcggggtcct gctgctttca ctcgtgatca ctctttactg taagcgcggt    1020
   cggaagaagc tgctgtacat ctttaagcaa cccttcatga ggcctgtgca gactactcaa    1080
   gaggaggacg gctgttcatg ccggttccca gaggaggagg aaggcggctg cgaactgcgc    1140
   gtgaaattca gccgcagcgc agatgctcca gcctacaagc aggggcagaa ccagctctac    1200
   aacgaactca atcttggtcg gagagaggag tacgacgtgc tggacaagcg gagaggacgg    1260
   gacccagaaa tgggcgggaa gccgcgcaga aagaatcccc aagagggcct gtacaacgag    1320
   ctccaaaagg ataagatggc agaagcctat agcgagattg gtatgaaagg ggaacgcaga    1380
   agaggcaaag gccacgacgg actgtaccag ggactcagca ccgccaccaa ggacacctat    1440
   gacgctcttc acatgcaggc cctgccgcct cgg                                 1473
<210> 93
   <211> 1473
   <212> DNA
   <213> Artificial Sequence
<220>
   <221> source
   <223> /note="Description of Artificial Sequence: Synthetic polynucleotide"
<400> 93
   atggccctcc ctgtcaccgc cctgctgctt ccgctggctc ttctgctcca cgccgctcgg      60
   cccgaaattg tgatgaccca gtcacccgcc actcttagcc tttcacccgg tgagcgcgca     120
   accctgtctt gcagagcctc ccaagacatc tcaaaatacc ttaattggta tcaacagaag     180
   cccggacagg ctcctcgcct tctgatctac cacaccagcc ggctccattc tggaatccct     240
   gccaggttca gcggtagcgg atctgggacc gactacaccc tcactatcag ctcactgcag     300
   ccagaggact tcgctgtcta tttctgtcag caagggaaca ccctgcccta cacctttgga     360
   cagggcacca agctcgagat taaaggtgga ggtggcagcg gaggaggtgg gtccggcggt     420
   ggaggaagcg gaggcggtgg gagccaggtc caactccaag aaagcggacc gggtcttgtg     480
   aagccatcag aaactctttc actgacttgt actgtgagcg gagtgtctct ccccgattac     540
   ggggtgtctt ggatcagaca gccaccgggg aagggtctgg aatggattgg agtgatttgg     600
   ggctctgaga ctacttacta caactcatcc ctcaagtcac gcgtcaccat ctcaaaggac     660
   aactctaaga atcaggtgtc actgaaactg tcatctgtga ccgcagccga caccgccgtg     720
   tactattgcg ctaagcatta ctattatggc gggagctacg caatggatta ctggggacag     780
   ggtactctgg tcaccgtgtc cagcaccact accccagcac cgaggccacc caccccggct     840
   cctaccatcg cctcccagcc tctgtccctg cgtccggagg catgtagacc cgcagctggt     900
   ggggccgtgc atacccgggg tcttgacttc gcctgcgata tctacatttg ggcccctctg     960
   gctggtactt gcggggtcct gctgctttca ctcgtgatca ctctttactg taagcgcggt    1020
   cggaagaagc tgctgtacat ctttaagcaa cccttcatga ggcctgtgca gactactcaa    1080
   gaggaggacg gctgttcatg ccggttccca gaggaggagg aaggcggctg cgaactgcgc    1140
   gtgaaattca gccgcagcgc agatgctcca gcctacaagc aggggcagaa ccagctctac    1200
   aacgaactca atcttggtcg gagagaggag tacgacgtgc tggacaagcg gagaggacgg    1260
   gacccagaaa tgggcgggaa gccgcgcaga aagaatcccc aagagggcct gtacaacgag    1320
   ctccaaaagg ataagatggc agaagcctat agcgagattg gtatgaaagg ggaacgcaga    1380
   agaggcaaag gccacgacgg actgtaccag ggactcagca ccgccaccaa ggacacctat    1440
   gacgctcttc acatgcaggc cctgccgcct cgg                                1473
<210> 94
   <211> 1473
   <212> DNA
   <213> Artificial Sequence
<220>
   <221> source
   <223> /note="Description of Artificial Sequence: Synthetic polynucleotide"
<400> 94
   atggccctcc ctgtcaccgc cctgctgctt ccgctggctc ttctgctcca cgccgctcgg      60
   cccgaaattg tgatgaccca gtcacccgcc actcttagcc tttcacccgg tgagcgcgca     120
   accctgtctt gcagagcctc ccaagacatc tcaaaatacc ttaattggta tcaacagaag     180
   cccggacagg ctcctcgcct tctgatctac cacaccagcc ggctccattc tggaatccct     240
   gccaggttca gcggtagcgg atctgggacc gactacaccc tcactatcag ctcactgcag     300
   ccagaggact tcgctgtcta tttctgtcag caagggaaca ccctgcccta cacctttgga     360
   cagggcacca agctcgagat taaaggtgga ggtggcagcg gaggaggtgg gtccggcggt     420
   ggaggaagcg gaggcggtgg gagccaggtc caactccaag aaagcggacc gggtcttgtg     480
   aagccatcag aaactctttc actgacttgt actgtgagcg gagtgtctct ccccgattac     540
   ggggtgtctt ggatcagaca gccaccgggg aagggtctgg aatggattgg agtgatttgg     600
   ggctctgaga ctacttacta caactcatcc ctcaagtcac gcgtcaccat ctcaaaggac     660
   aactctaaga atcaggtgtc actgaaactg tcatctgtga ccgcagccga caccgccgtg     720
   tactattgcg ctaagcatta ctattatggc gggagctacg caatggatta ctggggacag     780
   ggtactctgg tcaccgtgtc cagcaccact accccagcac cgaggccacc caccccggct     840
   cctaccatcg cctcccagcc tctgtccctg cgtccggagg catgtagacc cgcagctggt     900
   ggggccgtgc atacccgggg tcttgacttc gcctgcgata tctacatttg ggcccctctg     960
   gctggtactt gcggggtcct gctgctttca ctcgtgatca ctctttactg taagcgcggt    1020
   cggaagaagc tgctgtacat ctttaagcaa cccttcatga ggcctgtgca gactactcaa    1080
   gaggaggacg gctgttcatg ccggttccca gaggaggagg aaggcggctg cgaactgcgc    1140
   gtgaaattca gccgcagcgc agatgctcca gcctacaagc aggggcagaa ccagctctac    1200
   aacgaactca atcttggtcg gagagaggag tacgacgtgc tggacaagcg gagaggacgg    1260
   gacccagaaa tgggcgggaa gccgcgcaga aagaatcccc aagagggcct gtacaacgag    1320
   ctccaaaagg ataagatggc agaagcctat agcgagattg gtatgaaagg ggaacgcaga    1380
   agaggcaaag gccacgacgg actgtaccag ggactcagca ccgccaccaa ggacacctat    1440
   gacgctcttc acatgcaggc cctgccgcct cgg                                 1473
<210> 95
   <211> 1473
   <212> DNA
   <213> Artificial Sequence
<220>
   <221> source
   <223> /note="Description of Artificial Sequence: Synthetic polynucleotide"
<400> 95
   atggctctgc ccgtgaccgc actcctcctg ccactggctc tgctgcttca cgccgctcgc      60
   ccacaagtcc agcttcaaga atcagggcct ggtctggtga agccatctga gactctgtcc     120
   ctcacttgca ccgtgagcgg agtgtccctc ccagactacg gagtgagctg gattagacag     180
   cctcccggaa agggactgga gtggatcgga gtgatttggg gtagcgaaac cacttactat     240
   aactcttccc tgaagtcacg ggtcaccatt tcaaaggata actcaaagaa tcaagtgagc     300
   ctcaagctct catcagtcac cgccgctgac accgccgtgt attactgtgc caagcattac     360
   tactatggag ggtcctacgc catggactac tggggccagg gaactctggt cactgtgtca     420
   tctggtggag gaggtagcgg aggaggcggg agcggtggag gtggctccgg aggtggcgga     480
   agcgaaatcg tgatgaccca gagccctgca accctgtccc tttctcccgg ggaacgggct     540
   accctttctt gtcgggcatc acaagatatc tcaaaatacc tcaattggta tcaacagaag     600
   ccgggacagg cccctaggct tcttatctac cacacctctc gcctgcatag cgggattccc     660
   gcacgcttta gcgggtctgg aagcgggacc gactacactc tgaccatctc atctctccag     720
   cccgaggact tcgccgtcta cttctgccag cagggtaaca ccctgccgta caccttcggc     780
   cagggcacca agcttgagat caaaaccact actcccgctc caaggccacc cacccctgcc     840
   ccgaccatcg cctctcagcc gctttccctg cgtccggagg catgtagacc cgcagctggt     900
   ggggccgtgc atacccgggg tcttgacttc gcctgcgata tctacatttg ggcccctctg     960
   gctggtactt gcggggtcct gctgctttca ctcgtgatca ctctttactg taagcgcggt    1020
   cggaagaagc tgctgtacat ctttaagcaa cccttcatga ggcctgtgca gactactcaa    1080
   gaggaggacg gctgttcatg ccggttccca gaggaggagg aaggcggctg cgaactgcgc    1140
   gtgaaattca gccgcagcgc agatgctcca gcctacaagc aggggcagaa ccagctctac    1200
   aacgaactca atcttggtcg gagagaggag tacgacgtgc tggacaagcg gagaggacgg    1260
   gacccagaaa tgggcgggaa gccgcgcaga aagaatcccc aagagggcct gtacaacgag    1320
   ctccaaaagg ataagatggc agaagcctat agcgagattg gtatgaaagg ggaacgcaga    1380
   agaggcaaag gccacgacgg actgtaccag ggactcagca ccgccaccaa ggacacctat    1440
   gacgctcttc acatgcaggc cctgccgcct cgg                                 1473
<210> 96
   <211> 1458
   <212> DNA
   <213> Artificial Sequence
<220>
   <221> source
   <223> /note="Description of Artificial Sequence: Synthetic polynucleotide"
<400> 96
   atggccctcc ctgtcaccgc cctgctgctt ccgctggctc ttctgctcca cgccgctcgg 60
   cccgaaattg tgatgaccca gtcacccgcc actcttagcc tttcacccgg tgagcgcgca 120
   accctgtctt gcagagcctc ccaagacatc tcaaaatacc ttaattggta tcaacagaag 180
   cccggacagg ctcctcgcct tctgatctac cacaccagcc ggctccattc tggaatccct 240
   gccaggttca gcggtagcgg atctgggacc gactacaccc tcactatcag ctcactgcag 300
   ccagaggact tcgctgtcta tttctgtcag caagggaaca ccctgcccta cacctttgga 360
   cagggcacca agctcgagat taaaggtgga ggtggcagcg gaggaggtgg gtccggcggt 420
   ggaggaagcc aggtccaact ccaagaaagc ggaccgggtc ttgtgaagcc atcagaaact 480
   ctttcactga cttgtactgt gagcggagtg tctctccccg attacggggt gtcttggatc 540
   agacagccac cggggaaggg tctggaatgg attggagtga tttggggctc tgagactact 600
   tactacaact catccctcaa gtcacgcgtc accatctcaa aggacaactc taagaatcag 660
   gtgtcactga aactgtcatc tgtgaccgca gccgacaccg ccgtgtacta ttgcgctaag 720
   cattactatt atggcgggag ctacgcaatg gattactggg gacagggtac tctggtcacc 780
   gtgtccagca ccactacccc agcaccgagg ccacccaccc cggctcctac catcgcctcc 840
   cagcctctgt ccctgcgtcc ggaggcatgt agacccgcag ctggtggggc cgtgcatacc 900
   cggggtcttg acttcgcctg cgatatctac atttgggccc ctctggctgg tacttgcggg 960
   gtcctgctgc tttcactcgt gatcactctt tactgtaagc gcggtcggaa gaagctgctg 1020
   tacatcttta agcaaccctt catgaggcct gtgcagacta ctcaagagga ggacggctgt 1080
   tcatgccggt tcccagagga ggaggaaggc ggctgcgaac tgcgcgtgaa attcagccgc 1140
   agcgcagatg ctccagccta caagcagggg cagaaccagc tctacaacga actcaatctt 1200
   ggtcggagag aggagtacga cgtgctggac aagcggagag gacgggaccc agaaatgggc 1260
   gggaagccgc gcagaaagaa tccccaagag ggcctgtaca acgagctcca aaaggataag 1320
   atggcagaag cctatagcga gattggtatg aaaggggaac gcagaagagg caaaggccac 1380
   gacggactgt accagggact cagcaccgcc accaaggaca cctatgacgc tcttcacatg 1440
   caggccctgc cgcctcgg 1458
<210> 97
   <211> 813
   <212> DNA
   <213> Artificial Sequence
<220>
   <221> source
   <223> /note="Description of Artificial Sequence: Synthetic polynucleotide"
<400> 97
   atggccctgc ccgtcaccgc tctgctgctg ccccttgctc tgcttcttca tgcagcaagg       60
   ccggacatcc agatgaccca aaccacctca tccctctctg cctctcttgg agacagggtg      120
   accatttctt gtcgcgccag ccaggacatc agcaagtatc tgaactggta tcagcagaag      180
   ccggacggaa ccgtgaagct cctgatctac catacctctc gcctgcatag cggcgtgccc      240
   tcacgcttct ctggaagcgg atcaggaacc gattattctc tcactatttc aaatcttgag      300
   caggaagata ttgccaccta tttctgccag cagggtaata ccctgcccta caccttcgga      360
   ggagggacca agctcgaaat caccggtgga ggaggcagcg gcggtggagg gtctggtgga      420
   ggtggttctg aggtgaagct gcaagaatca ggccctggac ttgtggcccc ttcacagtcc      480
   ctgagcgtga cttgcaccgt gtccggagtc tccctgcccg actacggagt gtcatggatc      540
   agacaacctc cacggaaagg actggaatgg ctcggtgtca tctggggtag cgaaactact      600
   tactacaatt cagccctcaa aagcaggctg actattatca aggacaacag caagtcccaa      660
   gtctttctta agatgaactc actccagact gacgacaccg caatctacta ttgtgctaag      720
   cactactact acggaggatc ctacgctatg gattactggg gacaaggtac ttccgtcact      780
   gtctcttcac accatcatca ccatcaccat cac                                   813
<210> 98
   <211> 271
   <212> PRT
   <213> Artificial Sequence
<220>
   <221> source
   <223> /note="Description of Artificial Sequence: Synthetic polypeptide"
<400> 98
   Met Ala Leu Pro Val Thr Ala Leu Leu Leu Pro Leu Ala Leu Leu Leu
   1               5                   10                  15
   His Ala Ala Arg Pro Asp Ile Gln Met Thr Gln Thr Thr Ser Ser Leu
               20                  25                  30
   Ser Ala Ser Leu Gly Asp Arg Val Thr Ile Ser Cys Arg Ala Ser Gln
           35                  40                  45
   Asp Ile Ser Lys Tyr Leu Asn Trp Tyr Gln Gln Lys Pro Asp Gly Thr
       50                  55                  60
   Val Lys Leu Leu Ile Tyr His Thr Ser Arg Leu His Ser Gly Val Pro
   65                  70                  75                  80
   Ser Arg Phe Ser Gly Ser Gly Ser Gly Thr Asp Tyr Ser Leu Thr Ile
                   85                  90                  95
   Ser Asn Leu Glu Gln Glu Asp Ile Ala Thr Tyr Phe Cys Gln Gln Gly
               100                 105                 110
   Asn Thr Leu Pro Tyr Thr Phe Gly Gly Gly Thr Lys Leu Glu Ile Thr
           115                 120                 125
   Gly Gly Gly Gly Ser Gly Gly Gly Gly Ser Gly Gly Gly Gly Ser Glu
       130                 135                 140
   Val Lys Leu Gln Glu Ser Gly Pro Gly Leu Val Ala Pro Ser Gln Ser
   145                 150                 155                 160
   Leu Ser Val Thr Cys Thr Val Ser Gly Val Ser Leu Pro Asp Tyr Gly
                   165                 170                 175
   Val Ser Trp Ile Arg Gln Pro Pro Arg Lys Gly Leu Glu Trp Leu Gly
               180                 185                 190
   Val Ile Trp Gly Ser Glu Thr Thr Tyr Tyr Asn Ser Ala Leu Lys Ser
           195                 200                 205
   Arg Leu Thr Ile Ile Lys Asp Asn Ser Lys Ser Gln Val Phe Leu Lys
       210                 215                 220
   Met Asn Ser Leu Gln Thr Asp Asp Thr Ala Ile Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Ser Val Thr Val Ser Ser His His His His His His His His
               260                 265                 270
<210> 99
   <211> 1458
   <212> DNA
   <213> Artificial Sequence
<220>
   <221> source
   <223> /note="Description of Artificial Sequence: Synthetic polynucleotide"
<400> 99
   atggccttac cagtgaccgc cttgctcctg ccgctggcct tgctgctcca cgccgccagg      60
   ccggacatcc agatgacaca gactacatcc tccctgtctg cctctctggg agacagagtc     120
   accatcagtt gcagggcaag tcaggacatt agtaaatatt taaattggta tcagcagaaa     180
   ccagatggaa ctgttaaact cctgatctac catacatcaa gattacactc aggagtccca     240
   tcaaggttca gtggcagtgg gtctggaaca gattattctc tcaccattag caacctggag     300
   caagaagata ttgccactta cttttgccaa cagggtaata cgcttccgta cacgttcgga     360
   ggggggacca agctggagat cacaggtggc ggtggctcgg gcggtggtgg gtcgggtggc     420
   ggcggatctg aggtgaaact gcaggagtca ggacctggcc tggtggcgcc ctcacagagc     480
   ctgtccgtca catgcactgt ctcaggggtc tcattacccg actatggtgt aagctggatt     540
   cgccagcctc cacgaaaggg tctggagtgg ctgggagtaa tatggggtag tgaaaccaca     600
   tactataatt cagctctcaa atccagactg accatcatca aggacaactc caagagccaa     660
   gttttcttaa aaatgaacag tctgcaaact gatgacacag ccatttacta ctgtgccaaa     720
   cattattact acggtggtag ctatgctatg gactactggg gccaaggaac ctcagtcacc     780
   gtctcctcaa ccacgacgcc agcgccgcga ccaccaacac cggcgcccac catcgcgtcg     840
   cagcccctgt ccctgcgccc agaggcgtgc cggccagcgg cggggggcgc agtgcacacg     900
   agggggctgg acttcgcctg tgatatctac atctgggcgc ccttggccgg gacttgtggg     960
   gtccttctcc tgtcactggt tatcaccctt tactgcaaac ggggcagaaa gaaactcctg    1020
   tatatattca aacaaccatt tatgagacca gtacaaacta ctcaagagga agatggctgt    1080
   agctgccgat ttccagaaga agaagaagga ggatgtgaac tgagagtgaa gttcagcagg    1140
   agcgcagacg cccccgcgta caagcagggc cagaaccagc tctataacga gctcaatcta    1200
   ggacgaagag aggagtacga tgttttggac aagagacgtg gccgggaccc tgagatgggg    1260
   ggaaagccga gaaggaagaa ccctcaggaa ggcctgtaca atgaactgca gaaagataag    1320
   atggcggagg cctacagtga gattgggatg aaaggcgagc gccggagggg caaggggcac    1380
   gatggccttt accagggtct cagtacagcc accaaggaca cctacgacgc ccttcacatg    1440
   caggccctgc cccctcgc                                                   1458
<210> 100
   <211> 1184
   <212> DNA
   <213> Artificial Sequence
<220>
   <221> source
   <223> /note="Description of Artificial Sequence: Synthetic polynucleotide"
<400> 100
   cgtgaggctc cggtgcccgt cagtgggcag agcgcacatc gcccacagtc cccgagaagt      60
   tggggggagg ggtcggcaat tgaaccggtg cctagagaag gtggcgcggg gtaaactggg    120
   aaagtgatgt cgtgtactgg ctccgccttt ttcccgaggg tgggggagaa ccgtatataa     180
   gtgcagtagt cgccgtgaac gttctttttc gcaacgggtt tgccgccaga acacaggtaa     240
   gtgccgtgtg tggttcccgc gggcctggcc tctttacggg ttatggccct tgcgtgcctt     300
   gaattacttc cacctggctg cagtacgtga ttcttgatcc cgagcttcgg gttggaagtg     360
   ggtgggagag ttcgaggcct tgcgcttaag gagccccttc gcctcgtgct tgagttgagg     420
   cctggcctgg gcgctggggc cgccgcgtgc gaatctggtg gcaccttcgc gcctgtctcg     480
   ctgctttcga taagtctcta gccatttaaa atttttgatg acctgctgcg acgctttttt     540
   tctggcaaga tagtcttgta aatgcgggcc aagatctgca cactggtatt tcggtttttg     600
   gggccgcggg cggcgacggg gcccgtgcgt cccagcgcac atgttcggcg aggcggggcc     660
   tgcgagcgcg gccaccgaga atcggacggg ggtagtctca agctggccgg cctgctctgg     720
   tgcctggcct cgcgccgccg tgtatcgccc cgccctgggc ggcaaggctg gcccggtcgg     780
   caccagttgc gtgagcggaa agatggccgc ttcccggccc tgctgcaggg agctcaaaat     840
   ggaggacgcg gcgctcggga gagcgggcgg gtgagtcacc cacacaaagg aaaagggcct     900
   ttccgtcctc agccgtcgct tcatgtgact ccacggagta ccgggcgccg tccaggcacc     960
   tcgattagtt ctcgagcttt tggagtacgt cgtctttagg ttggggggag gggttttatg    1020
   cgatggagtt tccccacact gagtgggtgg agactgaagt taggccagct tggcacttga    1080
   tgtaattctc cttggaattt gccctttttg agtttggatc ttggttcatt ctcaagcctc    1140
   agacagtggt tcaaagtttt tttcttccat ttcaggtgtc gtga                     1184
<210> 101
   <211> 336
   <212> DNA
   <213> Artificial Sequence
<220>
   <221> source
   <223> /note="Description of Artificial Sequence: Synthetic polynucleotide"
<400> 101
   agagtgaagt tcagcaggag cgcagacgcc cccgcgtaca agcagggcca gaaccagctc        60
   tataacgagc tcaatctagg acgaagagag gagtacgatg ttttggacaa gagacgtggc       120
   cgggaccctg agatgggggg aaagccgaga aggaagaacc ctcaggaagg cctgtacaat       180
   gaactgcaga aagataagat ggcggaggcc tacagtgaga ttgggatgaa aggcgagcgc       240
   cggaggggca aggggcacga tggcctttac cagggtctca gtacagccac caaggacacc       300
   tacgacgccc ttcacatgca ggccctgccc cctcgc                                 336
<210> 102
   <211> 230
   <212> PRT
   <213> Artificial Sequence
<220>
   <221> source
   <223> /note="Description of Artificial Sequence: Synthetic polypeptide"
<400> 102
   Glu Ser Lys Tyr Gly Pro Pro Cys Pro Pro Cys Pro Ala Pro Glu Phe
   1               5                   10                  15
   Leu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Pro Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Thr Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Tyr Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys Met
   225                 230
<210> 103
   <211> 690
   <212> DNA
   <213> Artificial Sequence
<220>
   <221> source
   <223> /note="Description of Artificial Sequence: Synthetic polynucleotide"
<400> 103
   gagagcaagt acggccctcc ctgcccccct tgccctgccc ccgagttcct gggcggaccc        60
   agcgtgttcc tgttcccccc caagcccaag gacaccctga tgatcagccg gacccccgag       120
   gtgacctgtg tggtggtgga cgtgtcccag gaggaccccg aggtccagtt caactggtac       180
   gtggacggcg tggaggtgca caacgccaag accaagcccc gggaggagca gttcaatagc       240
   acctaccggg tggtgtccgt gctgaccgtg ctgcaccagg actggctgaa cggcaaggaa       300
   tacaagtgta aggtgtccaa caagggcctg cccagcagca tcgagaaaac catcagcaag       360
   gccaagggcc agcctcggga gccccaggtg tacaccctgc cccctagcca agaggagatg       420
   accaagaacc aggtgtccct gacctgcctg gtgaagggct tctaccccag cgacatcgcc       480
   gtggagtggg agagcaacgg ccagcccgag aacaactaca agaccacccc ccctgtgctg       540
   gacagcgacg gcagcttctt cctgtacagc cggctgaccg tggacaagag ccggtggcag       600
   gagggcaacg tctttagctg ctccgtgatg cacgaggccc tgcacaacca ctacacccag       660
   aagagcctga gcctgtccct gggcaagatg                                        690
<210> 104
   <211> 150
   <212> DNA
   <213> Artificial Sequence
<220>
   <221> source
   <223> /note="Description of Artificial Sequence: Synthetic polynucleotide"
<400> 104
   aaaaaaaaaa aaaaaaaaaa aaaaaaaaaa aaaaaaaaaa aaaaaaaaaa aaaaaaaaaa        60
   aaaaaaaaaa aaaaaaaaaa aaaaaaaaaa aaaaaaaaaa aaaaaaaaaa aaaaaaaaaa       120
   aaaaaaaaaa aaaaaaaaaa aaaaaaaaaa                                        150
<210> 105
   <211> 40
   <212> PRT
   <213> Artificial Sequence
<220>
   <221> source
   <223> /note="Description of Artificial Sequence: Synthetic polypeptide"
<220>
   <221> misc_feature
   <222> (1)..(40)
   <223> /note="This sequence may encompass 1-10 'Gly Gly Gly Ser' repeating units"
<220>
   <221> source
   <223> /note="See specification as filed for detailed description of substitutions and preferred embodiments"
<400> 105
   Gly Gly Gly Ser Gly Gly Gly Ser Gly Gly Gly Ser Gly Gly Gly Ser
   1               5                   10                  15
   Gly Gly Gly Ser Gly Gly Gly Ser Gly Gly Gly Ser Gly Gly Gly Ser
               20                  25                  30
   Gly Gly Gly Ser Gly Gly Gly Ser
           35                  40
<210> 106
   <211> 20
   <212> PRT
   <213> Artificial Sequence
<220>
   <221> source
   <223> /note="Description of Artificial Sequence: Synthetic peptide"
<400> 106
   Gly Gly Gly Gly Ser Gly Gly Gly Gly Ser Gly Gly Gly Gly Ser Gly
   1               5                   10                  15
   Gly Gly Gly Ser
               20
<210> 107
   <211> 15
   <212> PRT
   <213> Artificial Sequence
<220>
   <221> source
   <223> /note="Description of Artificial Sequence: Synthetic peptide"
<400> 107
   Gly Gly Gly Gly Ser Gly Gly Gly Gly Ser Gly Gly Gly Gly Ser
   1               5                   10                  15
<210> 108
   <211> 4
   <212> PRT
   <213> Artificial Sequence
<220>
   <221> source
   <223> /note="Description of Artificial Sequence: Synthetic peptide"
<400> 108
   Gly Gly Gly Ser
   1
<210> 109
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 50-5000 nucleotides"
<220>
   <221> source
   <223> /note="See specification as filed for detailed description of substitutions and preferred embodiments"
<400> 109
   aaaaaaaaaa aaaaaaaaaa aaaaaaaaaa aaaaaaaaaa aaaaaaaaaa aaaaaaaaaa      60
   aaaaaaaaaa aaaaaaaaaa aaaaaaaaaa aaaaaaaaaa aaaaaaaaaa aaaaaaaaaa     120
   aaaaaaaaaa aaaaaaaaaa aaaaaaaaaa aaaaaaaaaa aaaaaaaaaa aaaaaaaaaa     180
   aaaaaaaaaa aaaaaaaaaa aaaaaaaaaa aaaaaaaaaa aaaaaaaaaa aaaaaaaaaa     240
   aaaaaaaaaa aaaaaaaaaa aaaaaaaaaa aaaaaaaaaa aaaaaaaaaa aaaaaaaaaa     300
   aaaaaaaaaa aaaaaaaaaa aaaaaaaaaa aaaaaaaaaa aaaaaaaaaa aaaaaaaaaa     360
   aaaaaaaaaa aaaaaaaaaa aaaaaaaaaa aaaaaaaaaa aaaaaaaaaa aaaaaaaaaa     420
   aaaaaaaaaa aaaaaaaaaa aaaaaaaaaa aaaaaaaaaa aaaaaaaaaa aaaaaaaaaa     480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa aaaaaaaaaa aaaaaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa aaaaaaaaaa aaaaaaaaaa aaaaaaaaaa 2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa 3960
   aaaaaaaaaa aaaaaaaaaa aaaaaaaaaa aaaaaaaaaa aaaaaaaaaa aaaaaaaaaa 4020
   aaaaaaaaaa aaaaaaaaaa aaaaaaaaaa aaaaaaaaaa aaaaaaaaaa aaaaaaaaaa 4080
   aaaaaaaaaa aaaaaaaaaa aaaaaaaaaa aaaaaaaaaa aaaaaaaaaa aaaaaaaaaa 4140
   aaaaaaaaaa aaaaaaaaaa aaaaaaaaaa aaaaaaaaaa aaaaaaaaaa aaaaaaaaaa 4200
   aaaaaaaaaa aaaaaaaaaa aaaaaaaaaa aaaaaaaaaa aaaaaaaaaa aaaaaaaaaa 4260
   aaaaaaaaaa aaaaaaaaaa aaaaaaaaaa aaaaaaaaaa aaaaaaaaaa aaaaaaaaaa 4320
   aaaaaaaaaa aaaaaaaaaa aaaaaaaaaa aaaaaaaaaa aaaaaaaaaa aaaaaaaaaa 4380
   aaaaaaaaaa aaaaaaaaaa aaaaaaaaaa aaaaaaaaaa aaaaaaaaaa aaaaaaaaaa 4440
   aaaaaaaaaa aaaaaaaaaa aaaaaaaaaa aaaaaaaaaa aaaaaaaaaa aaaaaaaaaa 4500
   aaaaaaaaaa aaaaaaaaaa aaaaaaaaaa aaaaaaaaaa aaaaaaaaaa aaaaaaaaaa 4560
   aaaaaaaaaa aaaaaaaaaa aaaaaaaaaa aaaaaaaaaa aaaaaaaaaa aaaaaaaaaa 4620
   aaaaaaaaaa aaaaaaaaaa aaaaaaaaaa aaaaaaaaaa aaaaaaaaaa aaaaaaaaaa 4680
   aaaaaaaaaa aaaaaaaaaa aaaaaaaaaa aaaaaaaaaa aaaaaaaaaa aaaaaaaaaa 4740
   aaaaaaaaaa aaaaaaaaaa aaaaaaaaaa aaaaaaaaaa aaaaaaaaaa aaaaaaaaaa 4800
   aaaaaaaaaa aaaaaaaaaa aaaaaaaaaa aaaaaaaaaa aaaaaaaaaa aaaaaaaaaa 4860
   aaaaaaaaaa aaaaaaaaaa aaaaaaaaaa aaaaaaaaaa aaaaaaaaaa aaaaaaaaaa 4920
   aaaaaaaaaa aaaaaaaaaa aaaaaaaaaa aaaaaaaaaa aaaaaaaaaa aaaaaaaaaa 4980
   aaaaaaaaaa aaaaaaaaaa 5000
<210> 110
   <211> 100
   <212> DNA
   <213> Artificial Sequence
<220>
   <221> source
   <223> /note="Description of Artificial Sequence: Synthetic polynucleotide"
<400> 110
   tttttttttt tttttttttt tttttttttt tttttttttt tttttttttt tttttttttt 60
   tttttttttt tttttttttt tttttttttt tttttttttt 100
<210> 111
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 50-5000 nucleotides"
<220>
   <221> source
   <223> /note="See specification as filed for detailed description of substitutions and preferred embodiments"
<400> 111
   tttttttttt tttttttttt tttttttttt tttttttttt tttttttttt tttttttttt 60
   tttttttttt tttttttttt tttttttttt tttttttttt tttttttttt tttttttttt 120
   tttttttttt tttttttttt tttttttttt tttttttttt tttttttttt tttttttttt 180
   tttttttttt tttttttttt tttttttttt tttttttttt tttttttttt tttttttttt 240
   tttttttttt tttttttttt tttttttttt tttttttttt tttttttttt tttttttttt 300
   tttttttttt tttttttttt tttttttttt tttttttttt tttttttttt tttttttttt 360
   tttttttttt tttttttttt tttttttttt tttttttttt tttttttttt tttttttttt 420
   tttttttttt tttttttttt tttttttttt tttttttttt tttttttttt tttttttttt 480
   tttttttttt tttttttttt tttttttttt tttttttttt tttttttttt tttttttttt 540
   tttttttttt tttttttttt tttttttttt tttttttttt tttttttttt tttttttttt 600
   tttttttttt tttttttttt tttttttttt tttttttttt tttttttttt tttttttttt 660
   tttttttttt tttttttttt tttttttttt tttttttttt tttttttttt tttttttttt 720
   tttttttttt tttttttttt tttttttttt tttttttttt tttttttttt tttttttttt 780
   tttttttttt tttttttttt tttttttttt tttttttttt tttttttttt tttttttttt 840
   tttttttttt tttttttttt tttttttttt tttttttttt tttttttttt tttttttttt 900
   tttttttttt tttttttttt tttttttttt tttttttttt tttttttttt tttttttttt 960
   tttttttttt tttttttttt tttttttttt tttttttttt tttttttttt tttttttttt 1020
   tttttttttt tttttttttt tttttttttt tttttttttt tttttttttt tttttttttt 1080
   tttttttttt tttttttttt tttttttttt tttttttttt tttttttttt tttttttttt 1140
   tttttttttt tttttttttt tttttttttt tttttttttt tttttttttt tttttttttt 1200
   tttttttttt tttttttttt tttttttttt tttttttttt tttttttttt tttttttttt 1260
   tttttttttt tttttttttt tttttttttt tttttttttt tttttttttt tttttttttt 1320
   tttttttttt tttttttttt tttttttttt tttttttttt tttttttttt tttttttttt 1380
   tttttttttt tttttttttt tttttttttt tttttttttt tttttttttt tttttttttt 1440
   tttttttttt tttttttttt tttttttttt tttttttttt tttttttttt tttttttttt 1500
   tttttttttt tttttttttt tttttttttt tttttttttt tttttttttt tttttttttt 1560
   tttttttttt tttttttttt tttttttttt tttttttttt tttttttttt tttttttttt 1620
   tttttttttt tttttttttt tttttttttt tttttttttt tttttttttt tttttttttt 1680
   tttttttttt tttttttttt tttttttttt tttttttttt tttttttttt tttttttttt 1740
   tttttttttt tttttttttt tttttttttt tttttttttt tttttttttt tttttttttt 1800
   tttttttttt tttttttttt tttttttttt tttttttttt tttttttttt tttttttttt 1860
   tttttttttt tttttttttt tttttttttt tttttttttt tttttttttt tttttttttt 1920
   tttttttttt tttttttttt tttttttttt tttttttttt tttttttttt tttttttttt 1980
   tttttttttt tttttttttt tttttttttt tttttttttt tttttttttt tttttttttt 2040
   tttttttttt tttttttttt tttttttttt tttttttttt tttttttttt tttttttttt 2100
   tttttttttt tttttttttt tttttttttt tttttttttt tttttttttt tttttttttt 2160
   tttttttttt tttttttttt tttttttttt tttttttttt tttttttttt tttttttttt 2220
   tttttttttt tttttttttt tttttttttt tttttttttt tttttttttt tttttttttt 2280
   tttttttttt tttttttttt tttttttttt tttttttttt tttttttttt tttttttttt 2340
   tttttttttt tttttttttt tttttttttt tttttttttt tttttttttt tttttttttt 2400
   tttttttttt tttttttttt tttttttttt tttttttttt tttttttttt tttttttttt 2460
   tttttttttt tttttttttt tttttttttt tttttttttt tttttttttt tttttttttt 2520
   tttttttttt tttttttttt tttttttttt tttttttttt tttttttttt tttttttttt 2580
   tttttttttt tttttttttt tttttttttt tttttttttt tttttttttt tttttttttt 2640
   tttttttttt tttttttttt tttttttttt tttttttttt tttttttttt tttttttttt 2700
   tttttttttt tttttttttt tttttttttt tttttttttt tttttttttt tttttttttt 2760
   tttttttttt tttttttttt tttttttttt tttttttttt tttttttttt tttttttttt 2820
   tttttttttt tttttttttt tttttttttt tttttttttt tttttttttt tttttttttt 2880
   tttttttttt tttttttttt tttttttttt tttttttttt tttttttttt tttttttttt 2940
   tttttttttt tttttttttt tttttttttt tttttttttt tttttttttt tttttttttt 3000
   tttttttttt tttttttttt tttttttttt tttttttttt tttttttttt tttttttttt 3060
   tttttttttt tttttttttt tttttttttt tttttttttt tttttttttt tttttttttt 3120
   tttttttttt tttttttttt tttttttttt tttttttttt tttttttttt tttttttttt 3180
   tttttttttt tttttttttt tttttttttt tttttttttt tttttttttt tttttttttt 3240
   tttttttttt tttttttttt tttttttttt tttttttttt tttttttttt tttttttttt 3300
   tttttttttt tttttttttt tttttttttt tttttttttt tttttttttt tttttttttt 3360
   tttttttttt tttttttttt tttttttttt tttttttttt tttttttttt tttttttttt 3420
   tttttttttt tttttttttt tttttttttt tttttttttt tttttttttt tttttttttt 3480
   tttttttttt tttttttttt tttttttttt tttttttttt tttttttttt tttttttttt 3540
   tttttttttt tttttttttt tttttttttt tttttttttt tttttttttt tttttttttt 3600
   tttttttttt tttttttttt tttttttttt tttttttttt tttttttttt tttttttttt 3660
   tttttttttt tttttttttt tttttttttt tttttttttt tttttttttt tttttttttt 3720
   tttttttttt tttttttttt tttttttttt tttttttttt tttttttttt tttttttttt 3780
   tttttttttt tttttttttt tttttttttt tttttttttt tttttttttt tttttttttt 3840
   tttttttttt tttttttttt tttttttttt tttttttttt tttttttttt tttttttttt 3900
   tttttttttt tttttttttt tttttttttt tttttttttt tttttttttt tttttttttt 3960
   tttttttttt tttttttttt tttttttttt tttttttttt tttttttttt tttttttttt 4020
   tttttttttt tttttttttt tttttttttt tttttttttt tttttttttt tttttttttt 4080
   tttttttttt tttttttttt tttttttttt tttttttttt tttttttttt tttttttttt 4140
   tttttttttt tttttttttt tttttttttt tttttttttt tttttttttt tttttttttt 4200
   tttttttttt tttttttttt tttttttttt tttttttttt tttttttttt tttttttttt 4260
   tttttttttt tttttttttt tttttttttt tttttttttt tttttttttt tttttttttt 4320
   tttttttttt tttttttttt tttttttttt tttttttttt tttttttttt tttttttttt 4380
   tttttttttt tttttttttt tttttttttt tttttttttt tttttttttt tttttttttt 4440
   tttttttttt tttttttttt tttttttttt tttttttttt tttttttttt tttttttttt 4500
   tttttttttt tttttttttt tttttttttt tttttttttt tttttttttt tttttttttt 4560
   tttttttttt tttttttttt tttttttttt tttttttttt tttttttttt tttttttttt 4620
   tttttttttt tttttttttt tttttttttt tttttttttt tttttttttt tttttttttt 4680
   tttttttttt tttttttttt tttttttttt tttttttttt tttttttttt tttttttttt 4740
   tttttttttt tttttttttt tttttttttt tttttttttt tttttttttt tttttttttt 4800
   tttttttttt tttttttttt tttttttttt tttttttttt tttttttttt tttttttttt 4860
   tttttttttt tttttttttt tttttttttt tttttttttt tttttttttt tttttttttt 4920
   tttttttttt tttttttttt tttttttttt tttttttttt tttttttttt tttttttttt 4980
   tttttttttt tttttttttt 5000
<210> 112
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 100-5000 nucleotides"
<220>
   <221> source
   <223> /note="See specification as filed for detailed description of substitutions and preferred embodiments"
<400> 112
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa aaaaaaaaaa aaaaaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa aaaaaaaaaa aaaaaaaaaa aaaaaaaaaa 2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa 3960
   aaaaaaaaaa aaaaaaaaaa aaaaaaaaaa aaaaaaaaaa aaaaaaaaaa aaaaaaaaaa 4020
   aaaaaaaaaa aaaaaaaaaa aaaaaaaaaa aaaaaaaaaa aaaaaaaaaa aaaaaaaaaa 4080
   aaaaaaaaaa aaaaaaaaaa aaaaaaaaaa aaaaaaaaaa aaaaaaaaaa aaaaaaaaaa 4140
   aaaaaaaaaa aaaaaaaaaa aaaaaaaaaa aaaaaaaaaa aaaaaaaaaa aaaaaaaaaa 4200
   aaaaaaaaaa aaaaaaaaaa aaaaaaaaaa aaaaaaaaaa aaaaaaaaaa aaaaaaaaaa 4260
   aaaaaaaaaa aaaaaaaaaa aaaaaaaaaa aaaaaaaaaa aaaaaaaaaa aaaaaaaaaa 4320
   aaaaaaaaaa aaaaaaaaaa aaaaaaaaaa aaaaaaaaaa aaaaaaaaaa aaaaaaaaaa 4380
   aaaaaaaaaa aaaaaaaaaa aaaaaaaaaa aaaaaaaaaa aaaaaaaaaa aaaaaaaaaa 4440
   aaaaaaaaaa aaaaaaaaaa aaaaaaaaaa aaaaaaaaaa aaaaaaaaaa aaaaaaaaaa 4500
   aaaaaaaaaa aaaaaaaaaa aaaaaaaaaa aaaaaaaaaa aaaaaaaaaa aaaaaaaaaa 4560
   aaaaaaaaaa aaaaaaaaaa aaaaaaaaaa aaaaaaaaaa aaaaaaaaaa aaaaaaaaaa 4620
   aaaaaaaaaa aaaaaaaaaa aaaaaaaaaa aaaaaaaaaa aaaaaaaaaa aaaaaaaaaa 4680
   aaaaaaaaaa aaaaaaaaaa aaaaaaaaaa aaaaaaaaaa aaaaaaaaaa aaaaaaaaaa 4740
   aaaaaaaaaa aaaaaaaaaa aaaaaaaaaa aaaaaaaaaa aaaaaaaaaa aaaaaaaaaa 4800
   aaaaaaaaaa aaaaaaaaaa aaaaaaaaaa aaaaaaaaaa aaaaaaaaaa aaaaaaaaaa 4860
   aaaaaaaaaa aaaaaaaaaa aaaaaaaaaa aaaaaaaaaa aaaaaaaaaa aaaaaaaaaa 4920
   aaaaaaaaaa aaaaaaaaaa aaaaaaaaaa aaaaaaaaaa aaaaaaaaaa aaaaaaaaaa 4980
   aaaaaaaaaa aaaaaaaaaa 5000
<210> 113
   <211> 400
   <212> DNA
   <213> Artificial Sequence
<220>
   <221> source
   <223> /note="Description of Artificial Sequence: Synthetic polynucleotide"
<220>
   <221> misc_feature
   <222> (1)..(400)
   <223> /note="This sequence may encompass 100-400 nucleotides"
<220>
   <221> source
   <223> /note="See specification as filed for detailed description of substitutions and preferred embodiments"
<400> 113
   aaaaaaaaaa aaaaaaaaaa aaaaaaaaaa aaaaaaaaaa aaaaaaaaaa aaaaaaaaaa      60
   aaaaaaaaaa aaaaaaaaaa aaaaaaaaaa aaaaaaaaaa aaaaaaaaaa aaaaaaaaaa     120
   aaaaaaaaaa aaaaaaaaaa aaaaaaaaaa aaaaaaaaaa aaaaaaaaaa aaaaaaaaaa     180
   aaaaaaaaaa aaaaaaaaaa aaaaaaaaaa aaaaaaaaaa aaaaaaaaaa aaaaaaaaaa     240
   aaaaaaaaaa aaaaaaaaaa aaaaaaaaaa aaaaaaaaaa aaaaaaaaaa aaaaaaaaaa     300
   aaaaaaaaaa aaaaaaaaaa aaaaaaaaaa aaaaaaaaaa aaaaaaaaaa aaaaaaaaaa     360
   aaaaaaaaaa aaaaaaaaaa aaaaaaaaaa aaaaaaaaaa                           400
<210> 114
   <211> 120
   <212> PRT
   <213> Artificial Sequence
<220>
   <221> source
   <223> /note="Description of Artificial Sequence: Synthetic polypeptide"
<400> 114
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Asn Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser
           115                 120
<210> 115
   <211> 120
   <212> PRT
   <213> Artificial Sequence
<220>
   <221> source
   <223> /note="Description of Artificial Sequence: Synthetic polypeptide"
<400> 115
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Ser Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser
           115                 120
<210> 116
   <211> 120
   <212> PRT
   <213> Artificial Sequence
<220>
   <221> source
   <223> /note="Description of Artificial Sequence: Synthetic polypeptide"
<400> 116
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
   35 40 45
   Gly Val Ile Trp Gly Ser Glu Thr Thr Tyr Tyr Gln Ser Ser Leu Lys
   50 55 60
   Ser Arg Val Thr Ile Ser Lys Asp Asn Ser Lys Asn Gln Val Ser Leu
   65 70 75 80
   Lys Leu Ser Ser Val Thr Ala Ala Asp Thr Ala Val Tyr Tyr Cys Ala
   85 90 95
   Lys His Tyr Tyr Tyr Gly Gly Ser Tyr Ala Met Asp Tyr Trp Gly Gln
   100 105 110
   Gly Thr Leu Val Thr Val Ser Ser
   115 120
<210> 117
   <211> 107
   <212> PRT
   <213> Artificial Sequence
<220>
   <221> source
   <223> /note="Description of Artificial Sequence: Synthetic polypeptide"
<400> 117
   Glu Ile Val Met Thr Gln Ser Pro Ala Thr Leu Ser Leu Ser Pro Gly
   1 5 10 15
   Glu Arg Ala Thr Leu Ser Cys Arg Ala Ser Gln Asp Ile Ser Lys Tyr
   20 25 30
   Leu Asn Trp Tyr Gln Gln Lys Pro Gly Gln Ala Pro Arg Leu Leu Ile
   35 40 45
   Tyr His Thr Ser Arg Leu His Ser Gly Ile Pro Ala Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Val Tyr Phe Cys Gln Gln Gly Asn Thr Leu Pro Tyr
   85 90 95
   Thr Phe Gly Gln Gly Thr Lys Leu Glu Ile Lys
   100 105
<210> 118
   <211> 1132
   <212> PRT
   <213> Homo sapiens
<400> 118
<210> 119
   <211> 4027
   <212> DNA
   <213> Homo sapiens
<400> 119
   caggcagcgt ggtcctgctg cgcacgtggg aagccctggc cccggccacc cccgcgatgc        60
   cgcgcgctcc ccgctgccga gccgtgcgct ccctgctgcg cagccactac cgcgaggtgc       120
   tgccgctggc cacgttcgtg cggcgcctgg ggccccaggg ctggcggctg gtgcagcgcg       180
   gggacccggc ggctttccgc gcgctggtgg cccagtgcct ggtgtgcgtg ccctgggacg       240
   cacggccgcc ccccgccgcc ccctccttcc gccaggtgtc ctgcctgaag gagctggtgg       300
   cccgagtgct gcagaggctg tgcgagcgcg gcgcgaagaa cgtgctggcc ttcggcttcg       360
   cgctgctgga cggggcccgc gggggccccc ccgaggcctt caccaccagc gtgcgcagct       420
   acctgcccaa cacggtgacc gacgcactgc gggggagcgg ggcgtggggg ctgctgttgc       480
   gccgcgtggg cgacgacgtg ctggttcacc tgctggcacg ctgcgcgctc tttgtgctgg 540
   tggctcccag ctgcgcctac caggtgtgcg ggccgccgct gtaccagctc ggcgctgcca 600
   ctcaggcccg gcccccgcca cacgctagtg gaccccgaag gcgtctggga tgcgaacggg 660
   cctggaacca tagcgtcagg gaggccgggg tccccctggg cctgccagcc ccgggtgcga 720
   ggaggcgcgg gggcagtgcc agccgaagtc tgccgttgcc caagaggccc aggcgtggcg 780
   ctgcccctga gccggagcgg acgcccgttg ggcaggggtc ctgggcccac ccgggcagga 840
   cgcgtggacc gagtgaccgt ggtttctgtg tggtgtcacc tgccagaccc gccgaagaag 900
   ccacctcttt ggagggtgcg ctctctggca cgcgccactc ccacccatcc gtgggccgcc 960
   agcaccacgc gggcccccca tccacatcgc ggccaccacg tccctgggac acgccttgtc 1020
   ccccggtgta cgccgagacc aagcacttcc tctactcctc aggcgacaag gagcagctgc 1080
   ggccctcctt cctactcagc tctctgaggc ccagcctgac tggcgctcgg aggctcgtgg 1140
   agaccatctt tctgggttcc aggccctgga tgccagggac tccccgcagg ttgccccgcc 1200
   tgccccagcg ctactggcaa atgcggcccc tgtttctgga gctgcttggg aaccacgcgc 1260
   agtgccccta cggggtgctc ctcaagacgc actgcccgct gcgagctgcg gtcaccccag 1320
   cagccggtgt ctgtgcccgg gagaagcccc agggctctgt ggcggccccc gaggaggagg 1380
   acacagaccc ccgtcgcctg gtgcagctgc tccgccagca cagcagcccc tggcaggtgt 1440
   acggcttcgt gcgggcctgc ctgcgccggc tggtgccccc aggcctctgg ggctccaggc 1500
   acaacgaacg ccgcttcctc aggaacacca agaagttcat ctccctgggg aagcatgcca 1560
   agctctcgct gcaggagctg acgtggaaga tgagcgtgcg gggctgcgct tggctgcgca 1620
   ggagcccagg ggttggctgt gttccggccg cagagcaccg tctgcgtgag gagatcctgg 1680
   ccaagttcct gcactggctg atgagtgtgt acgtcgtcga gctgctcagg tctttctttt 1740
   atgtcacgga gaccacgttt caaaagaaca ggctcttttt ctaccggaag agtgtctgga 1800
   gcaagttgca aagcattgga atcagacagc acttgaagag ggtgcagctg cgggagctgt 1860
   cggaagcaga ggtcaggcag catcgggaag ccaggcccgc cctgctgacg tccagactcc 1920
   gcttcatccc caagcctgac gggctgcggc cgattgtgaa catggactac gtcgtgggag 1980
   ccagaacgtt ccgcagagaa aagagggccg agcgtctcac ctcgagggtg aaggcactgt 2040
   tcagcgtgct caactacgag cgggcgcggc gccccggcct cctgggcgcc tctgtgctgg 2100
   gcctggacga tatccacagg gcctggcgca ccttcgtgct gcgtgtgcgg gcccaggacc 2160
   cgccgcctga gctgtacttt gtcaaggtgg atgtgacggg cgcgtacgac accatccccc 2220
   aggacaggct cacggaggtc atcgccagca tcatcaaacc ccagaacacg tactgcgtgc 2280
   gtcggtatgc cgtggtccag aaggccgccc atgggcacgt ccgcaaggcc ttcaagagcc 2340
   acgtctctac cttgacagac ctccagccgt acatgcgaca gttcgtggct cacctgcagg 2400
   agaccagccc gctgagggat gccgtcgtca tcgagcagag ctcctccctg aatgaggcca 2460
   gcagtggcct cttcgacgtc ttcctacgct tcatgtgcca ccacgccgtg cgcatcaggg 2520
   gcaagtccta cgtccagtgc caggggatcc cgcagggctc catcctctcc acgctgctct 2580
   gcagcctgtg ctacggcgac atggagaaca agctgtttgc ggggattcgg cgggacgggc 2640
   tgctcctgcg tttggtggat gatttcttgt tggtgacacc tcacctcacc cacgcgaaaa 2700
   ccttcctcag gaccctggtc cgaggtgtcc ctgagtatgg ctgcgtggtg aacttgcgga 2760
   agacagtggt gaacttccct gtagaagacg aggccctggg tggcacggct tttgttcaga 2820
   tgccggccca cggcctattc ccctggtgcg gcctgctgct ggatacccgg accctggagg 2880
   tgcagagcga ctactccagc tatgcccgga cctccatcag agccagtctc accttcaacc 2940
   gcggcttcaa ggctgggagg aacatgcgtc gcaaactctt tggggtcttg cggctgaagt 3000
   gtcacagcct gtttctggat ttgcaggtga acagcctcca gacggtgtgc accaacatct 3060
   acaagatcct cctgctgcag gcgtacaggt ttcacgcatg tgtgctgcag ctcccatttc 3120
   atcagcaagt ttggaagaac cccacatttt tcctgcgcgt catctctgac acggcctccc 3180
   tctgctactc catcctgaaa gccaagaacg cagggatgtc gctgggggcc aagggcgccg 3240
   ccggccctct gccctccgag gccgtgcagt ggctgtgcca ccaagcattc ctgctcaagc 3300
   tgactcgaca ccgtgtcacc tacgtgccac tcctggggtc actcaggaca gcccagacgc 3360
   agctgagtcg gaagctcccg gggacgacgc tgactgccct ggaggccgca gccaacccgg 3420
   cactgccctc agacttcaag accatcctgg actgatggcc acccgcccac agccaggccg 3480
   agagcagaca ccagcagccc tgtcacgccg ggctctacgt cccagggagg gaggggcggc 3540
   ccacacccag gcccgcaccg ctgggagtct gaggcctgag tgagtgtttg gccgaggcct 3600
   gcatgtccgg ctgaaggctg agtgtccggc tgaggcctga gcgagtgtcc agccaagggc 3660
   tgagtgtcca gcacacctgc cgtcttcact tccccacagg ctggcgctcg gctccacccc 3720
   agggccagct tttcctcacc aggagcccgg cttccactcc ccacatagga atagtccatc 3780
   cccagattcg ccattgttca cccctcgccc tgccctcctt tgccttccac ccccaccatc 3840
   caggtggaga ccctgagaag gaccctggga gctctgggaa tttggagtga ccaaaggtgt 3900
   gccctgtaca caggcgagga ccctgcacct ggatgggggt ccctgtgggt caaattgggg 3960
   ggaggtgctg tgggagtaaa atactgaata tatgagtttt tcagttttga aaaaaaaaaa 4020
   aaaaaaa 4027
<210> 120
   <211> 1182
   <212> DNA
   <213> Artificial Sequence
<220>
   <221> source
   <223> /note="Description of Artificial Sequence: Synthetic polynucleotide"
<400> 120
   atggccctcc ctgtcactgc cctgcttctc cccctcgcac tcctgctcca cgccgctaga        60
   ccacccggat ggtttctgga ctctccggat cgcccgtgga atcccccaac cttctcaccg       120
   gcactcttgg ttgtgactga gggcgataat gcgaccttca cgtgctcgtt ctccaacacc       180
   tccgaatcat tcgtgctgaa ctggtaccgc atgagcccgt caaaccagac cgacaagctc       240
   gccgcgtttc cggaagatcg gtcgcaaccg ggacaggatt gtcggttccg cgtgactcaa       300
   ctgccgaatg gcagagactt ccacatgagc gtggtccgcg ctaggcgaaa cgactccggg       360
   acctacctgt gcggagccat ctcgctggcg cctaaggccc aaatcaaaga gagcttgagg       420
   gccgaactga gagtgaccga gcgcagagct gaggtgccaa ctgcacatcc atccccatcg       480
   cctcggcctg cggggcagtt tcagaccctg gtcacgacca ctccggcgcc gcgcccaccg       540
   actccggccc caactatcgc gagccagccc ctgtcgctga ggccggaagc atgccgccct       600
   gccgccggag gtgctgtgca tacccgggga ttggacttcg catgcgacat ctacatttgg       660
   gctcctctcg ccggaacttg tggcgtgctc cttctgtccc tggtcatcac cctgtactgc       720
   aagcggggtc ggaaaaagct tctgtacatt ttcaagcagc ccttcatgag gcccgtgcaa       780
   accacccagg aggaggacgg ttgctcctgc cggttccccg aagaggaaga aggaggttgc       840
   gagctgcgcg tgaagttctc ccggagcgcc gacgcccccg cctataagca gggccagaac       900
   cagctgtaca acgaactgaa cctgggacgg cgggaagagt acgatgtgct ggacaagcgg       960
   cgcggccggg accccgaaat gggcgggaag cctagaagaa agaaccctca ggaaggcctg      1020
   tataacgagc tgcagaagga caagatggcc gaggcctact ccgaaattgg gatgaaggga      1080
   gagcggcgga ggggaaaggg gcacgacggc ctgtaccaag gactgtccac cgccaccaag      1140
   gacacatacg atgccctgca catgcaggcc cttccccctc gc                         1182
<210> 121
   <211> 394
   <212> PRT
   <213> Artificial Sequence
<220>
   <221> source
   <223> /note="Description of Artificial Sequence: Synthetic polypeptide"
<400> 121
   Met Ala Leu Pro Val Thr Ala Leu Leu Leu Pro Leu Ala Leu Leu Leu
   1 5 10 15
   His Ala Ala Arg Pro Pro Gly Trp Phe Leu Asp Ser Pro Asp Arg Pro
               20                  25                  30
   Trp Asn Pro Pro Thr Phe Ser Pro Ala Leu Leu Val Val Thr Glu Gly
           35                  40                  45
   Asp Asn Ala Thr Phe Thr Cys Ser Phe Ser Asn Thr Ser Glu Ser Phe
       50                  55                  60
   Val Leu Asn Trp Tyr Arg Met Ser Pro Ser Asn Gln Thr Asp Lys Leu
   65                  70                  75                  80
   Ala Ala Phe Pro Glu Asp Arg Ser Gln Pro Gly Gln Asp Cys Arg Phe
                   85                  90                  95
   Arg Val Thr Gln Leu Pro Asn Gly Arg Asp Phe His Met Ser Val Val
               100                 105                 110
   Arg Ala Arg Arg Asn Asp Ser Gly Thr Tyr Leu Cys Gly Ala Ile Ser
           115                 120                 125
   Leu Ala Pro Lys Ala Gln Ile Lys Glu Ser Leu Arg Ala Glu Leu Arg
       130                 135                 140
   Val Thr Glu Arg Arg Ala Glu Val Pro Thr Ala His Pro Ser Pro Ser
   145                 150                 155                 160
   Pro Arg Pro Ala Gly Gln Phe Gln Thr Leu Val Thr Thr Thr Pro Ala
                   165                 170                 175
   Pro Arg Pro Pro Thr Pro Ala Pro Thr Ile Ala Ser Gln Pro Leu Ser
               180                 185                 190
   Leu Arg Pro Glu Ala Cys Arg Pro Ala Ala Gly Gly Ala Val His Thr
           195                 200                 205
   Arg Gly Leu Asp Phe Ala Cys Asp Ile Tyr Ile Trp Ala Pro Leu Ala
       210                 215                 220
   Gly Thr Cys Gly Val Leu Leu Leu Ser Leu Val Ile Thr Leu Tyr Cys
   225                 230                 235                 240
   Lys Arg Gly Arg Lys Lys Leu Leu Tyr Ile Phe Lys Gln Pro Phe Met
                   245                 250                 255
   Arg Pro Val Gln Thr Thr Gln Glu Glu Asp Gly Cys Ser Cys Arg Phe
               260                 265                 270
   Pro Glu Glu Glu Glu Gly Gly Cys Glu Leu Arg Val Lys Phe Ser Arg
   275 280 285
   Ser Ala Asp Ala Pro Ala Tyr Lys Gln Gly Gln Asn Gln Leu Tyr Asn
   290 295 300
   Glu Leu Asn Leu Gly Arg Arg Glu Glu Tyr Asp Val Leu Asp Lys Arg
   305 310 315 320
   Arg Gly Arg Asp Pro Glu Met Gly Gly Lys Pro Arg Arg Lys Asn Pro
   325 330 335
   Gln Glu Gly Leu Tyr Asn Glu Leu Gln Lys Asp Lys Met Ala Glu Ala
   340 345 350
   Tyr Ser Glu Ile Gly Met Lys Gly Glu Arg Arg Arg Gly Lys Gly His
   355 360 365
   Asp Gly Leu Tyr Gln Gly Leu Ser Thr Ala Thr Lys Asp Thr Tyr Asp
   370 375 380
   Ala Leu His Met Gln Ala Leu Pro Pro Arg
   385 390
<210> 122
   <211> 132
   <212> PRT
   <213> Artificial Sequence
<220>
   <221> source
   <223> /note="Description of Artificial Sequence: Synthetic polypeptide"
<400> 122
   Asp Val Pro Asp Tyr Ala Ser Leu Gly Gly Pro Ser Ser Pro Lys Lys
   1 5 10 15
   Lys Arg Lys Val Ser Arg Gly Val Gln Val Glu Thr Ile Ser Pro Gly
   20 25 30
   Asp Gly Arg Thr Phe Pro Lys Arg Gly Gln Thr Cys Val Val His Tyr
   35 40 45
   Thr Gly Met Leu Glu Asp Gly Lys Lys Phe Asp Ser Ser Arg Asp Arg
   50 55 60
   Asn Lys Pro Phe Lys Phe Met Leu Gly Lys Gln Glu Val Ile Arg Gly
   65 70 75 80
   Trp Glu Glu Gly Val Ala Gln Met Ser Val Gly Gln Arg Ala Lys Leu
   85 90 95
   Thr Ile Ser Pro Asp Tyr Ala Tyr Gly Ala Thr Gly His Pro Gly Ile
   100 105 110
   Ile Pro Pro His Ala Thr Leu Val Phe Asp Val Glu Leu Leu Lys Leu
   115 120 125
   Glu Thr Ser Tyr
   130
<210> 123
   <211> 108
   <212> PRT
   <213> Artificial Sequence
<220>
   <221> source
   <223> /note="Description of Artificial Sequence: Synthetic polypeptide"
<400> 123
   Val Gln Val Glu Thr Ile Ser Pro Gly Asp Gly Arg Thr Phe Pro Lys
   1 5 10 15
   Arg Gly Gln Thr Cys Val Val His Tyr Thr Gly Met Leu Glu Asp Gly
   20 25 30
   Lys Lys Phe Asp Ser Ser Arg Asp Arg Asn Lys Pro Phe Lys Phe Met
   35 40 45
   Leu Gly Lys Gln Glu Val Ile Arg Gly Trp Glu Glu Gly Val Ala Gln
   50 55 60
   Met Ser Val Gly Gln Arg Ala Lys Leu Thr Ile Ser Pro Asp Tyr Ala
   65 70 75 80
   Tyr Gly Ala Thr Gly His Pro Gly Ile Ile Pro Pro His Ala Thr Leu
   85 90 95
   Val Phe Asp Val Glu Leu Leu Lys Leu Glu Thr Ser
   100 105
<210> 124
   <211> 93
   <212> PRT
   <213> Artificial Sequence
<220>
   <221> source
   <223> /note="Description of Artificial Sequence: Synthetic polypeptide"
<400> 124
<210> 125
   <211> 95
   <212> PRT
   <213> Artificial Sequence
<220>
   <221> source
   <223> /note="Description of Artificial Sequence: Synthetic polypeptide"
<400> 125
   Ile Leu Trp His Glu Met Trp His Glu Gly Leu Ile Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Thr Gln Ala
   65 70 75 80
   Trp Asp Leu Tyr Tyr His Val Phe Arg Arg Ile Ser Lys Thr Ser
   85 90 95
<210> 126
   <211> 95
   <212> PRT
   <213> Artificial Sequence
<220>
   <221> source
   <223> /note="Description of Artificial Sequence: Synthetic polypeptide"
<400> 126
   Ile Leu Trp His Glu Met Trp His Glu Gly Leu Leu Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Thr Gln Ala
   65 70 75 80
   Trp Asp Leu Tyr Tyr His Val Phe Arg Arg Ile Ser Lys Thr Ser
   85 90 95
<210> 127
   <211> 95
   <212> PRT
   <213> Artificial Sequence
<220>
   <221> source
   <223> /note="Description of Artificial Sequence: Synthetic polypeptide"
<400> 127
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Leu Gln Ala
   65                  70                  75                  80
   Trp Asp Leu Tyr Tyr His Val Phe Arg Arg Ile Ser Lys Thr Ser
                   85                  90                  95
<210> 128
   <211> 95
   <212> PRT
   <213> Artificial Sequence
<220>
   <221> source
   <223> /note="Description of Artificial Sequence: Synthetic polypeptide"
<220>
   <221> MOD_RES
   <222> (12)..(12)
   <223> Any amino acid
<220>
   <221> MOD_RES
   <222> (78)..(78)
   <223> Any amino acid
<400> 128
   Ile Leu Trp His Glu Met Trp His Glu Gly Leu Xaa Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Xaa Gln Ala
   65 70 75 80
   Trp Asp Leu Tyr Tyr His Val Phe Arg Arg Ile Ser Lys Thr Ser
   85 90 95
<210> 129
   <211> 95
   <212> PRT
   <213> Artificial Sequence
<220>
   <221> source
   <223> /note="Description of Artificial Sequence: Synthetic polypeptide"
<400> 129
   Ile Leu Trp His Glu Met Trp His Glu Gly Leu Ile Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Leu Gln Ala
   65 70 75 80
   Trp Asp Leu Tyr Tyr His Val Phe Arg Arg Ile Ser Lys Thr Ser
   85 90 95
<210> 130
   <211> 95
   <212> PRT
   <213> Artificial Sequence
<220>
   <221> source
   <223> /note="Description of Artificial Sequence: Synthetic polypeptide"
<400> 130
   Ile Leu Trp His Glu Met Trp His Glu Gly Leu Leu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Leu Gln Ala
   65                  70                  75                  80
   Trp Asp Leu Tyr Tyr His Val Phe Arg Arg Ile Ser Lys Thr Ser
                   85                  90                  95
<210> 131
   <211> 2000
   <212> DNA
   <213> Artificial Sequence
<220>
   <221> source
   <223> /note="Description of Artificial Sequence: Synthetic polynucleotide"
<220>
   <221> misc_feature
   <222> (1)..(2000)
   <223> /note="This sequence may encompass 50-2000 nucleotides"
<220>
   <221> source
   <223> /note="See specification as filed for detailed description of substitutions and preferred embodiments"
<400> 131
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa 2000
<210> 132
   <211> 373
   <212> PRT
   <213> Artificial Sequence
<220>
   <221> source
   <223> /note="Description of Artificial Sequence: Synthetic polypeptide"
<400> 132
   Pro Gly Trp Phe Leu Asp Ser Pro Asp Arg Pro Trp Asn Pro Pro Thr
   1               5                   10                  15
   Phe Ser Pro Ala Leu Leu Val Val Thr Glu Gly Asp Asn Ala Thr Phe
               20                  25                  30
   Thr Cys Ser Phe Ser Asn Thr Ser Glu Ser Phe Val Leu Asn Trp Tyr
           35                  40                  45
   Arg Met Ser Pro Ser Asn Gln Thr Asp Lys Leu Ala Ala Phe Pro Glu
       50                  55                  60
   Asp Arg Ser Gln Pro Gly Gln Asp Cys Arg Phe Arg Val Thr Gln Leu
   65                  70                  75                  80
   Pro Asn Gly Arg Asp Phe His Met Ser Val Val Arg Ala Arg Arg Asn
                   85                  90                  95
   Asp Ser Gly Thr Tyr Leu Cys Gly Ala Ile Ser Leu Ala Pro Lys Ala
               100                 105                 110
   Gln Ile Lys Glu Ser Leu Arg Ala Glu Leu Arg Val Thr Glu Arg Arg
           115                 120                 125
   Ala Glu Val Pro Thr Ala His Pro Ser Pro Ser Pro Arg Pro Ala Gly
       130                 135                 140
   Gln Phe Gln Thr Leu Val Thr Thr Thr Pro Ala Pro Arg Pro Pro Thr
   145                 150                 155                 160
   Pro Ala Pro Thr Ile Ala Ser Gln Pro Leu Ser Leu Arg Pro Glu Ala
                   165                 170                 175
   Cys Arg Pro Ala Ala Gly Gly Ala Val His Thr Arg Gly Leu Asp Phe
               180                 185                 190
   Ala Cys Asp Ile Tyr Ile Trp Ala Pro Leu Ala Gly Thr Cys Gly Val
           195                 200                 205
   Leu Leu Leu Ser Leu Val Ile Thr Leu Tyr Cys Lys Arg Gly Arg Lys
       210                 215                 220
   Lys Leu Leu Tyr Ile Phe Lys Gln Pro Phe Met Arg Pro Val Gln Thr
   225                 230                 235                 240
   Thr Gln Glu Glu Asp Gly Cys Ser Cys Arg Phe Pro Glu Glu Glu Glu
                   245                 250                 255
   Gly Gly Cys Glu Leu Arg Val Lys Phe Ser Arg Ser Ala Asp Ala Pro
               260                 265                 270
   Ala Tyr Lys Gln Gly Gln Asn Gln Leu Tyr Asn Glu Leu Asn Leu Gly
           275                 280                 285
   Arg Arg Glu Glu Tyr Asp Val Leu Asp Lys Arg Arg Gly Arg Asp Pro
       290                 295                 300
   Glu Met Gly Gly Lys Pro Arg Arg Lys Asn Pro Gln Glu Gly Leu Tyr
   305                 310                 315                 320
   Asn Glu Leu Gln Lys Asp Lys Met Ala Glu Ala Tyr Ser Glu Ile Gly
                   325                 330                 335
   Met Lys Gly Glu Arg Arg Arg Gly Lys Gly His Asp Gly Leu Tyr Gln
               340                 345                 350
   Gly Leu Ser Thr Ala Thr Lys Asp Thr Tyr Asp Ala Leu His Met Gln
           355                 360                 365
   Ala Leu Pro Pro Arg
   370
<210> 133
   <211> 21
   <212> PRT
   <213> Artificial Sequence
<220>
   <221> source
   <223> /note="Description of Artificial Sequence: Synthetic peptide"
<220>
   <221> MOD_RES
   <222> (1)..(1)
   <223> Ahx
<400> 133
   Xaa Thr Ser Glu Leu Lys Lys Val Val Ala Leu Tyr Asp Tyr Met Pro
   1 5 10 15
   Met Asn Ala Asn Asp
   20
<210> 134
   <211> 4
   <212> PRT
   <213> Artificial Sequence
<220>
   <221> source
   <223> /note="Description of Artificial Sequence: Synthetic peptide"
<400> 134
   Arg Gly Asp Ser
   1
<210> 135
   <211> 35
   <212> PRT
   <213> Artificial Sequence
<220>
   <221> source
   <223> /note="Description of Artificial Sequence: Synthetic polypeptide"
<400> 135
   Thr Lys Lys Lys Tyr Ser Ser Ser Val His Asp Pro Asn Gly Glu Tyr
   1 5 10 15
   Met Phe Met Arg Ala Val Asn Thr Ala Lys Lys Ser Arg Leu Thr Asp
   20 25 30
   Val Thr Leu
   35
<210> 136
   <211> 105
   <212> DNA
   <213> Artificial Sequence
<220>
   <221> source
   <223> /note="Description of Artificial Sequence: Synthetic polynucleotide"
<400> 136
   acaaaaaaga agtattcatc cagtgtgcac gaccctaacg gtgaatacat gttcatgaga 60
   gcagtgaaca cagccaaaaa atccagactc acagatgtga cccta 105
<210> 137
   <211> 521
   <212> DNA
   <213> Unknown
<220>
   <221> source
   <223> /note="Description of Unknown: Phosphoglycerate kinase (PGK) promoter polynucleotide"
<400> 137
   acccctctct ccagccacta agccagttgc tccctcggct gacggctgca cgcgaggcct 60
   ccgaacgtct tacgccttgt ggcgcgcccg tccttgtccc gggtgtgatg gcggggtgtg 120
   gggcggaggg cgtggcgggg aagggccggc gacgagagcc gcgcgggacg actcgtcggc 180
   gataaccggt gtcgggtagc gccagccgcg cgacggtaac gagggaccgc gacaggcaga 240
   cgctcccatg atcactctgc acgccgaagg caaatagtgc aggccgtgcg gcgcttggcg 300
   ttccttggaa gggctgaatc cccgcctcgt ccttcgcagc ggccccccgg gtgttcccat 360
   cgccgcttct aggcccactg cgacgcttgc ctgcacttct tacacgctct gggtcccagc 420
   cgcggcgacg caaagggcct tggtgcgggt ctcgtcggcg cagggacgcg tttgggtccc 480
   gacggaacct tttccgcgtt ggggttgggg caccataagc t 521
<210> 138
   <211> 118
   <212> DNA
   <213> Unknown
<220>
   <221> source
   <223> /note="Description of Unknown: Phosphoglycerate kinase (PGK) promoter polynucleotide"
<400> 138
   acccctctct ccagccacta agccagttgc tccctcggct gacggctgca cgcgaggcct 60
   ccgaacgtct tacgccttgt ggcgcgcccg tccttgtccc gggtgtgatg gcggggtg 118
<210> 139
   <211> 221
   <212> DNA
   <213> Unknown
<220>
   <221> source
   <223> /note="Description of Unknown: Phosphoglycerate kinase (PGK) promoter polynucleotide"
<400> 139
   acccctctct ccagccacta agccagttgc tccctcggct gacggctgca cgcgaggcct 60
   ccgaacgtct tacgccttgt ggcgcgcccg tccttgtccc gggtgtgatg gcggggtgtg 120
   gggcggaggg cgtggcgggg aagggccggc gacgagagcc gcgcgggacg actcgtcggc 180
   gataaccggt gtcgggtagc gccagccgcg cgacggtaac g 221
<210> 140
   <211> 324
   <212> DNA
   <213> Unknown
<220>
   <221> source
   <223> /note="Description of Unknown: Phosphoglycerate kinase (PGK) promoter polynucleotide"
<400> 140
   acccctctct ccagccacta agccagttgc tccctcggct gacggctgca cgcgaggcct 60
   ccgaacgtct tacgccttgt ggcgcgcccg tccttgtccc gggtgtgatg gcggggtgtg 120
   gggcggaggg cgtggcgggg aagggccggc gacgagagcc gcgcgggacg actcgtcggc 180
   gataaccggt gtcgggtagc gccagccgcg cgacggtaac gagggaccgc gacaggcaga 240
   cgctcccatg atcactctgc acgccgaagg caaatagtgc aggccgtgcg gcgcttggcg 300
   ttccttggaa gggctgaatc cccg 324
<210> 141
   <211> 422
   <212> DNA
   <213> Unknown
<220>
   <221> source
   <223> /note="Description of Unknown: Phosphoglycerate kinase (PGK) promoter polynucleotide"
<400> 141
   acccctctct ccagccacta agccagttgc tccctcggct gacggctgca cgcgaggcct      60
   ccgaacgtct tacgccttgt ggcgcgcccg tccttgtccc gggtgtgatg gcggggtgtg     120
   gggcggaggg cgtggcgggg aagggccggc gacgagagcc gcgcgggacg actcgtcggc     180
   gataaccggt gtcgggtagc gccagccgcg cgacggtaac gagggaccgc gacaggcaga     240
   cgctcccatg atcactctgc acgccgaagg caaatagtgc aggccgtgcg gcgcttggcg     300
   ttccttggaa gggctgaatc cccgcctcgt ccttcgcagc ggccccccgg gtgttcccat    360
   cgccgcttct aggcccactg cgacgcttgc ctgcacttct tacacgctct gggtcccagc     420
   cg                                                                     422
<210> 142
   <211> 18
   <212> PRT
   <213> Artificial Sequence
<220>
   <221> source
   <223> /note="Description of Artificial Sequence: Synthetic peptide"
<400> 142
   Gly Ser Thr Ser Gly Ser Gly Lys Pro Gly Ser Gly Glu Gly Ser Thr
   1               5                   10                  15
   Lys Gly